# EUROPEAN PATENT APPLICATION

(11) **EP 1 229 046 A2**
(43) Date of publication of application: **07.08.2002**
(21) Application number: 02001167.2
(22) Date of filing: 28.01.2002
(51) Int. Cl.: C07K 14/47

(54) **Human testis expressed patched like protein**

(30) Priority: 30.01.2001 WO PCT/US01/00663; 30.01.2001 WO PCT/US01/00664; 30.01.2001 WO PCT/US01/00665; 30.01.2001 WO PCT/US01/00667; 30.01.2001 WO PCT/US01/00668; 30.01.2001 WO PCT/US01/00669; 23.05.2001 US 864761; 09.10.2001 US 327898
(71) Applicant: Aeomica, Inc., Sunnyvale, CA 94086-4520 (US)
(72) Inventor: Zhan, Jian, San Mateo, California 94403 (US)
(74) Representative: Rollins, Anthony John

(57) **Abstract**

The invention provides isolated nucleic acids that encode HTPL, including two isoforms, and fragments thereof, vectors for propagating and expressing HTPL nucleic acids, host cells comprising the nucleic acids and vectors of the present invention, proteins, protein fragments, and protein fusions of the novel HTPL isoforms, and antibodies thereto. The invention further provides transgenic cells and non-human organisms comprising human HTPL nucleic acids, and transgenic cells and non-human organisms with targeted disruption of the endogenous orthologue of the human HTPL gene. The invention further provides pharmaceutical formulations of the nucleic acids, proteins, and antibodies of the present invention, and diagnostic, investigational, and therapeutic methods based on the HTPL nucleic acids, proteins, and antibodies of the present invention.

## Description

### FIELD OF THE INVENTION

The present invention relates to novel human testis expressed patched like protein, including two isoforms. More specifically, the invention provides isolated nucleic acid molecules encoding human testis expressed patched like protein, fragments thereof, vectors and host cells comprising isolated nucleic acid molecules encoding human testis expressed patched like protein, human testis expressed patched like protein polypeptides, antibodies, transgenic cells and non-human organisms, and diagnostic, therapeutic, and investigational methods of using the same.

### BACKGROUND OF THE INVENTION

Sterols are key components of all cell membranes in eukaryotes. Cholesterol, in particular, plays an important role in regulating the properties of cell membranes. It rigidifies the fluid membrane by occupying the spaces between the saturated hydrocarbon chains of the sphingolipids, thus reducing passive permeability and increasing the mechanical durability of the lipid bilayer. Simons and Ikonens, *Science* 290:1721-1726 (2000). Cholesterol is also thought to increase post-Golgi protein sorting by regulating the thickness of the lipid bilayer. Simons and Ikonens, *Science* 290:1721-1726 (2000). It also functions in lipid raft assembly and function. These dynamic assemblies are involved in sorting and distributing lipids and proteins to the cell surface, where they play an important role in signal transduction and in generating cell surface polarity. Simons and Ikones, *Nature* 387:569-572 (1997). It is now widely accepted that "not only the total cellular level of cholesterol but also its distribution between membranes and within a given membrane are tightly regulated". Simons and Ikonens, *Science* 290:1721-1726 (2000).

The dynamic cholesterol balance between different cellular compartments is achieved through several important proteins. These include the sterol-regulated enzyme 3-hydroxy-3-methylglutaryl (HMG) CoA reductase, as well as SREBP cleavage activating protein (SCAP), a membrane-bound glycoprotein that regulates the proteolytic activation of sterol regulatory element binding proteins (SREBPs). Hua *et al., Cell* 87:415-426 (1996); Nohturfft *et al., Proc. Natl. Acad. Sci. USA* 95:12848-12853 (1998). A common domain has been identified in these two proteins and named sterol-sensing domain (SSD). Similar sequences have been found in several other proteins, suggesting that these proteins also play a role in cholesterol-related cellular functions. These proteins include the Niemann Pick-type C1 protein necessary for export of cholesterol from lysosomes (Stacie *et al., Science* 277:232-235 (1997)); Patched, a receptor for Hedgehog (Tabin and McMahon, *Trends Cell Biol.* 7:442-446 (1997)); a Patched-related protein implicated in the hereditary renal cell carcinoma (TRC8; Gemmill *et al., Proc. Natl. Acad. Sci. USA* 95:9572-9577 (1998)); and Dispatched, which is critical to the release of hedgehog (Burke, *et al., Cell* 99:803-815 (1999)). Sterol-sensing domain is indispensable for functions of the above proteins. For example, mutations in SSD of SCAP result in the disruption of cholesterol homeostasis. Nohturfft *et al., Proc. Natl. Acad. Sci. USA* 95:12848-12853 (1998). Recent evidence suggests that the sterol-sensing domain in Patched is critical in regulating vesicular trafficking of its receptor partner Smoothened. Strutt *et al., Curr. Biol.* 11:608-613 (2001); Martin *et al., Curr. Biol.* 11:601-607 (2001).

Hedgehog, the Patched ligand, is the only protein known to have a covalently attached cholesterol moiety. Porter *et al., Science* 274:255-259 (1996). Three hedgehog homologues have been identified in mammals: sonic hedgehog, Indian hedgehog, and desert hedgehog. Tabin and McMahon, *Trends Cell Biol.* 7:442-446 (1997). Function of the hedgehog signaling pathway has been studied extensively. It has been shown that Patched is a twelve transmembrane protein and forms complex with Smoothened, a seven transmembrane protein, which acts as the signaling component of the pathway. Without hedgehog binding, Patched inhibits Smoothened signaling thus ensures that the signaling is tightly controlled by ligand regulation. Kalderon, *Cell* 103:371-374 (2000). Mutations in sonic hedgehog, Patched, and smoothened have all been shown to cause tumors in skin and brain, among other tissues. Wicking *et al., Oncogene* 18:7844-7851 (1999). For example, mutations in the Patched gene result in basal cell carcinoma thus Patched is a tumor suppressor gene. Wicking *et al., Oncogene* 18:7844-7851 (1999). Desert hedgehog is expressed in testis and has been suggested to be the ligand of Patched-2. Carpenter *et al., Proc. Natl. Acad. Sci. USA* 95:13630-13634 (1998). Although Patched-2 has yet to be demonstrated as a tumor suppressor gene, its involvement in germ cell development suggests that its dysfunction may cause malformation of germ cells.

Given the fact that Patched is a transmembrane protein functioning in the hedgehog signaling pathway, mutations of which cause a variety of tumors, it has potential therapeutic as well as diagnostic roles in cancer. There is a need to identify and to characterize additional Patched like protein.

### SUMMARY OF THE INVENTION

The present invention solves these and other needs in the art by providing isolated nucleic acids that encode human testis expressed Patched like protein (HTPL), including two isoforms (HTPL-L for the long form and HTPL-S for the short form), and fragments thereof.

In other aspects, the invention provides vectors for propagating and expressing the nucleic acids of the present invention, host cells comprising the nucleic acids and vectors of the present invention, proteins, protein fragments, and protein fusions of HTPL, and antibodies thereto.

The invention further provides pharmaceutical formulations of the nucleic acids, proteins, and antibodies of the present invention.

In other aspects, the invention provides transgenic cells and non-human organisms comprising HTPL nucleic acids, and transgenic cells and non-human organisms with targeted disruption of the endogenous orthologue of the HTPL.

The invention additionally provides diagnostic, investigational, and therapeutic methods based on the HTPL nucleic acids, proteins, and antibodies of the present invention.

The invention further provides medical and diagnostic uses of the HTPL nucleic acids, proteins and antibodies.

### BRIEF DESCRIPTION OF THE DRAWINGS

The above and other objects and advantages of the present invention will be apparent upon consideration of the following detailed description taken in conjunction with the accompanying drawings, in which like characters refer to like parts throughout, and in which:
FIG. 1 (A) schematizes the protein domain structure of HTPL-L and HTPL-S, and FIG. 1 (B) shows the alignment of the Patched motif of HTPL-L with that of other Patched domain containing proteins;
FIG. 2 is a map showing the genomic structure of HTPL encoded at chromosome 10p12.1;
FIG. 3 presents the nucleotide and predicted amino acid sequences of HTPL-L;
FIG. 4 presents the nucleotide and predicted amino acid sequences of HTPL-S; and
FIG. 5 presents the expression analysis of HTPL by RT_PCR.

### DETAILED DESCRIPTION OF THE INVENTION

Mining the sequence of the human genome for novel human genes, the present inventors have identified HTPL, including two isoforms, a testis expressed Patched like transmembrane tumor suppressor involved in germ cell development.

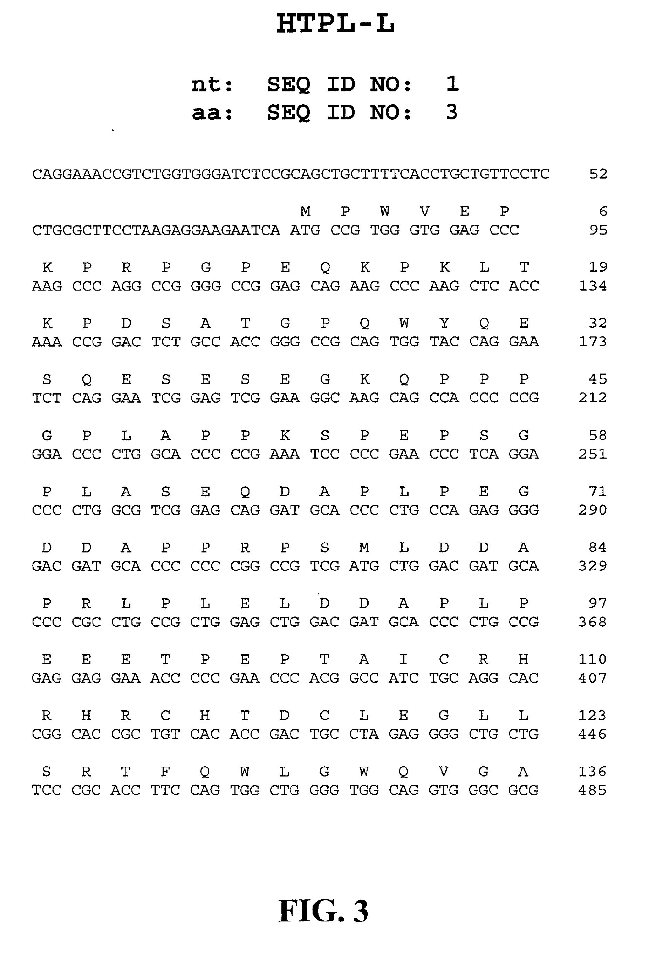

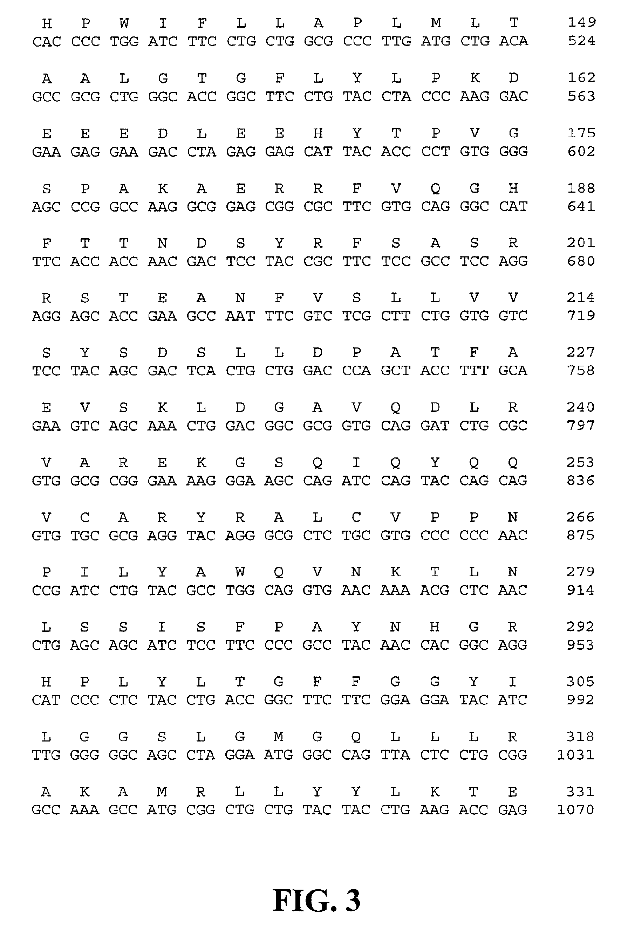

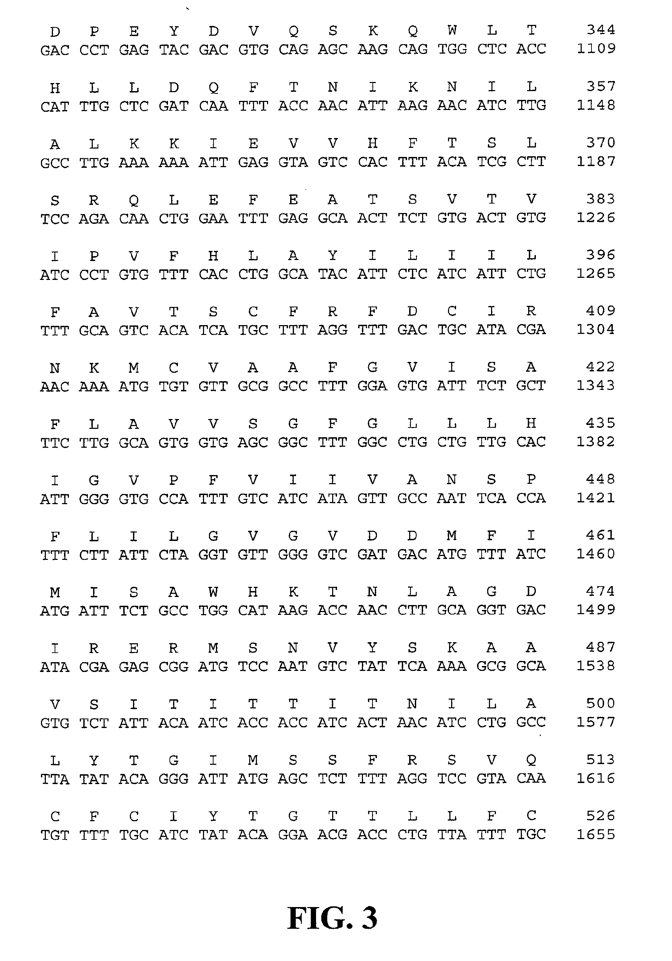

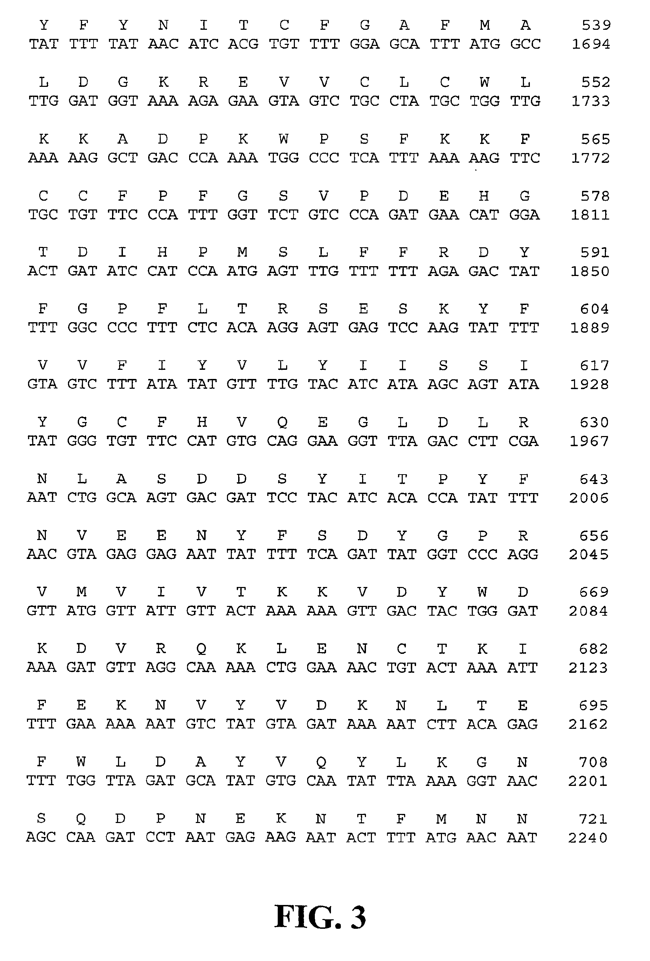

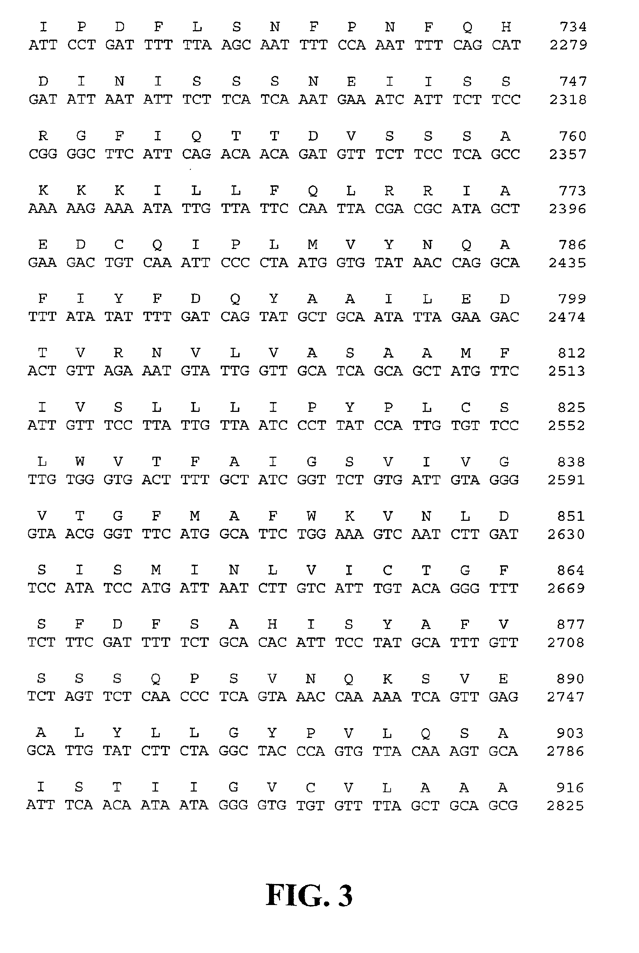

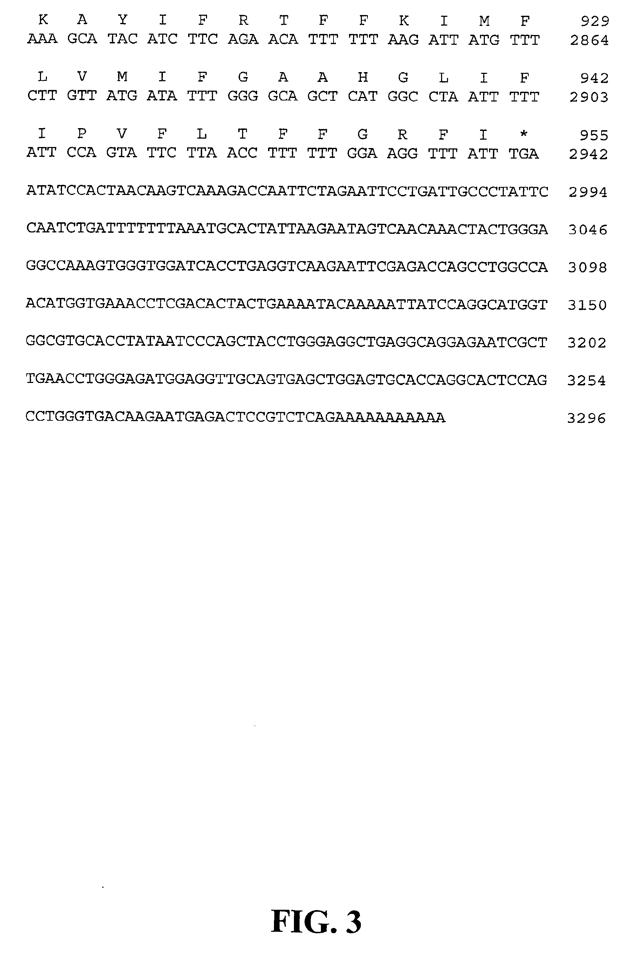

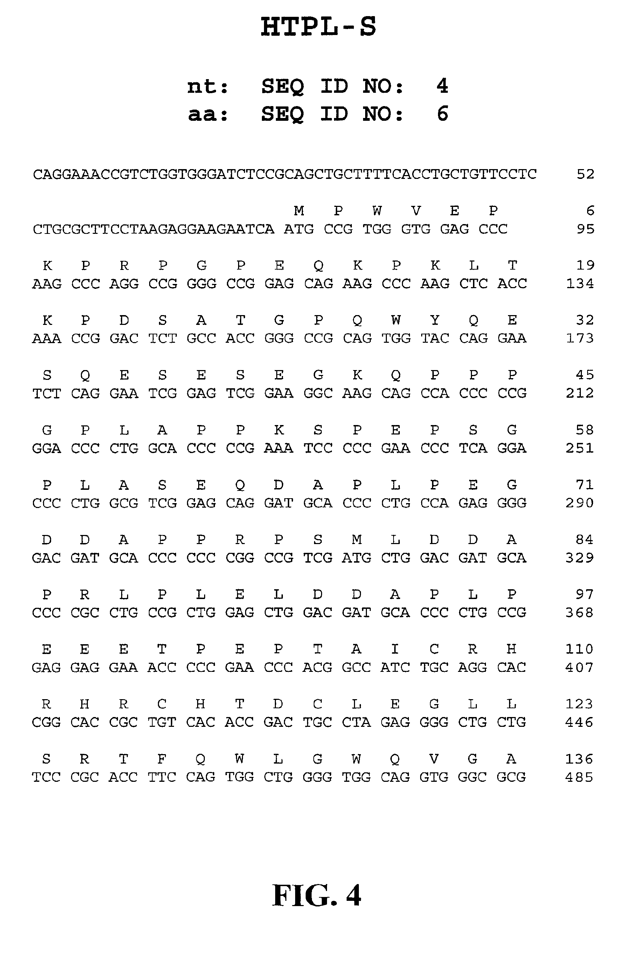

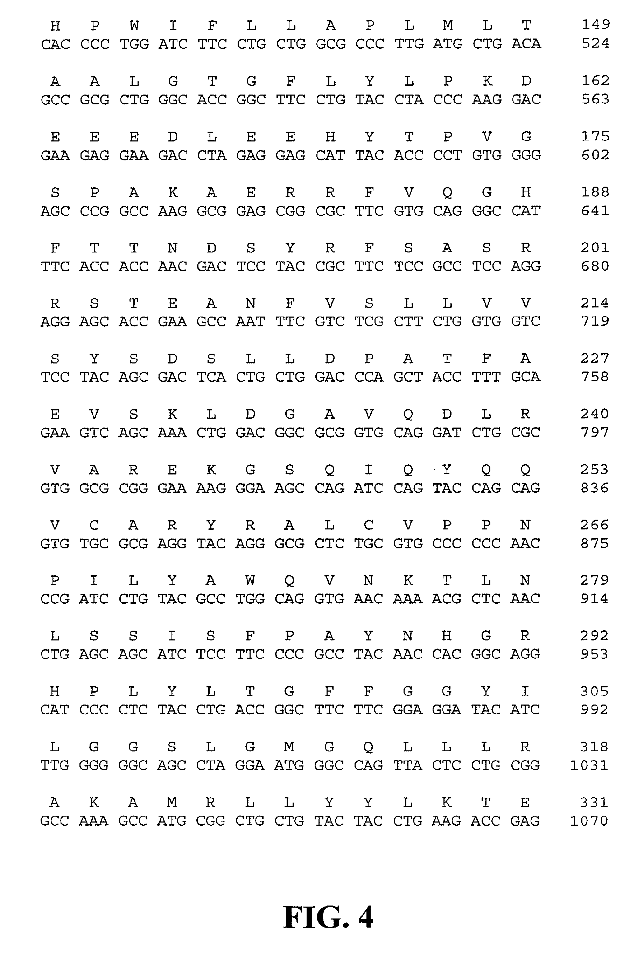

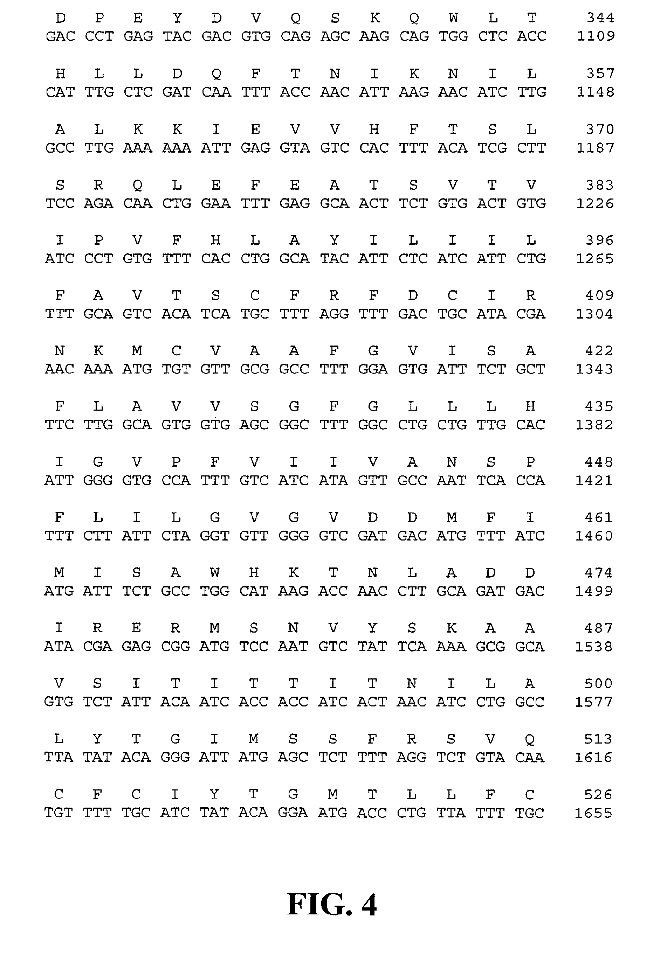

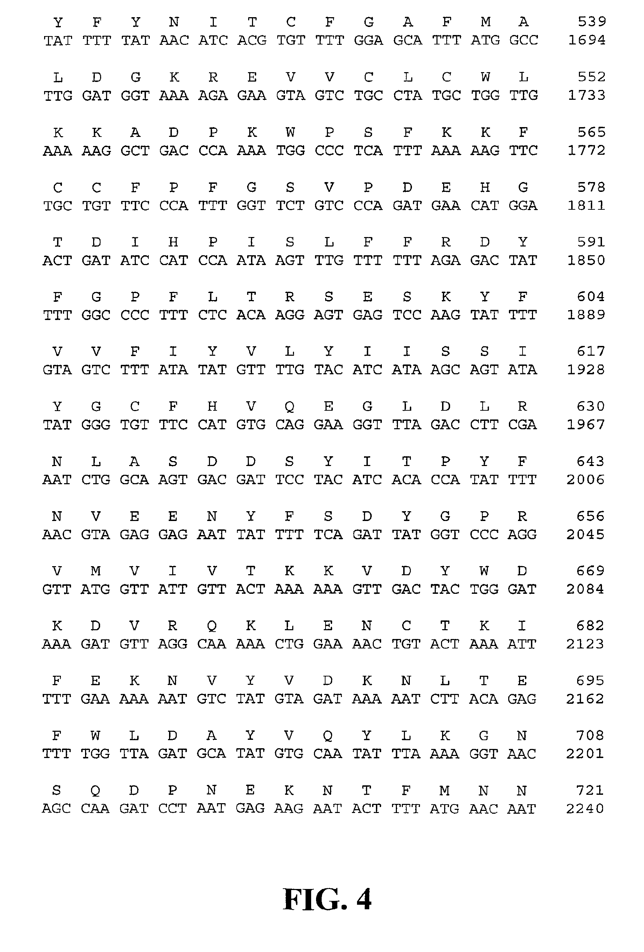

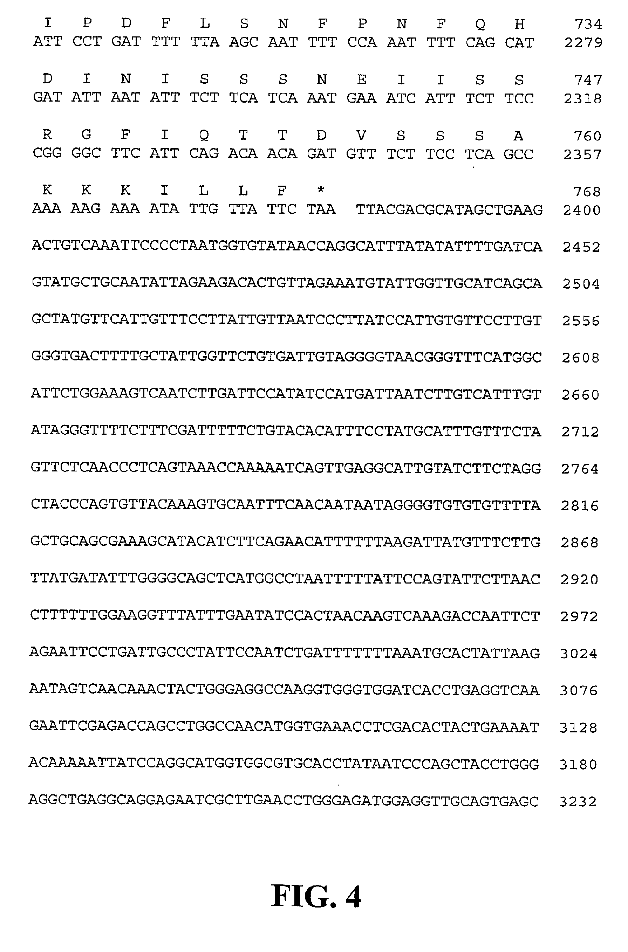

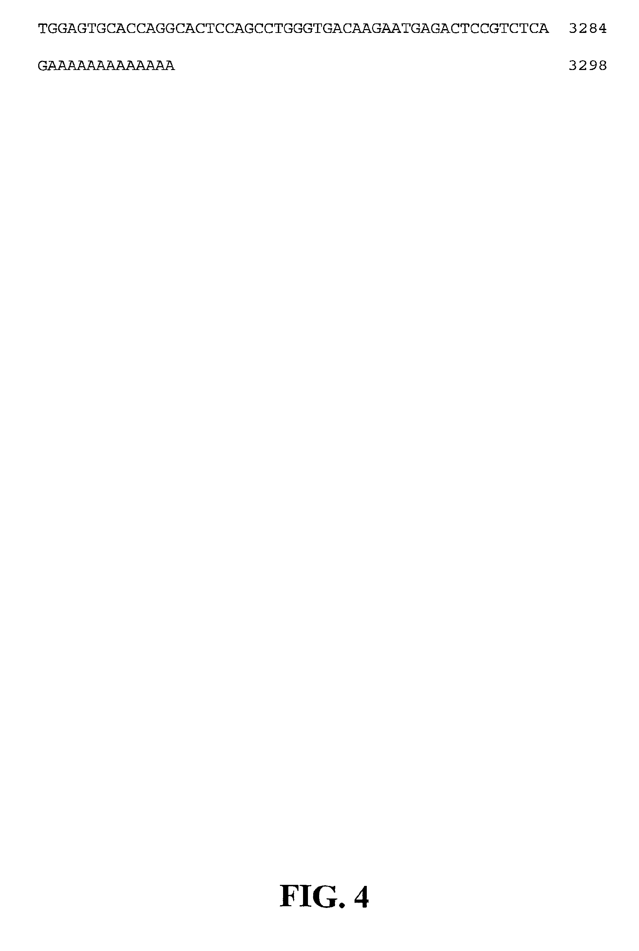

The newly isolated HTPL gene has two isoforms, with a few single base pair differences between the two (FIG. 3 and FIG. 4). One of the single base pair changes in HTPL-S introduces a premature stop codon in HTPL-S (S for short) compared to HTPL-L (L for long). HTPL shares an overall structural organization with the Patched protein. The shared structural features strongly imply that HTPL plays a role similar to that of Patched, in the hedgehog signaling pathway, and is a potential tumor suppressor.

Like Patched, HTPL-L contains a Patched domain (http://pfam.wustl.edu/hmmsearch.shtml), a Sterol-sensing domain (SSD, http://motif.genome.ad.jp/) and twelve transmembrane domains (http://smart .embl-heidelberg.de/smart/show_motifs.pl). The Patched domain in HTPL-L covers amino acid sequences 166 - 952 of HTPL-L. The SSD domain in HTPL-L covers amino acid sequences 383 - 540 of HTPL-L. The presence of these domains in HTPL-L suggest that HTPL-L, like Patched and other Patched domain containing proteins, is involved in the Hedgehog signaling pathway (see background section). Because of the presence of the premature stop codon, HTPL-S contains a partial Patched domain, a complete Sterol-sensing motif and seven transmembrane domains. The presence of these domains in HTPL-S suggests that HTPL-S is also involved in the Hedgehog signaling pathway.

Other signatures of the newly isolated HTPL proteins were identified by searching the PROSITE database (http://www.expasy.ch/tools/scnpsitl.html), and the list below is for both HTPL-L and HTPL-S unless specified otherwise. These include seven N-glycosylation sites (192 - 195, 275 - 278, 279 - 282, 530 - 533, 678 - 681, 692 - 695 and 737 - 740), one cAMP- and cGMP-dependent protein kinase phosphorylation site (201 - 204), seven protein kinase C phosphorylation sites (194 - 196, 200 - 202, 508 - 510, 561 - 563, 662 - 664, 746 - 748, and 759 - 761; plus one for HTPL-L at 800 - 802), twelve Casein kinase II phosphorylation sites (19 - 22, 36 - 39, 62 - 65, 79 - 82, 190 - 193, 215 - 218, 219 - 222, 225 - 228, 230 - 233, 572 - 575, 597 - 600, and 740 - 743), two tyrosine kinase phosphorylation site (329 - 335, and 681 - 688; plus one for HTPL-L at 887 - 893), four N-myristoylation sites (307 - 312, 418 - 4223, 504 - 509, and 535 - 540; plus one for HTPL-L at 935 - 940), and a single amidation site at 541 -544.

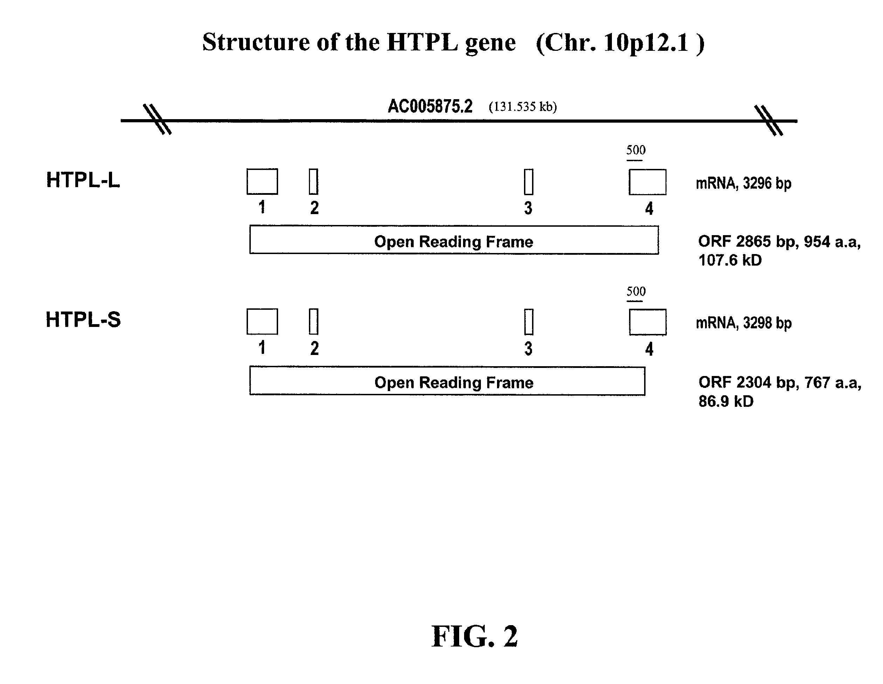

FIG. 2 shows the genomic organization of HTPL.

At the top is shown the bacterial artificial chromosome (BAC), with GenBank accession number (AC005875.2), that span the HTPL locus. The genome-derived single-exon probe first used to demonstrate expression from this locus is shown below the BACs and labeled "500". The 500 bp probe includes sequence drawn solely from exon four.

As shown in FIG. 2, two HTPL isoforms have been identified. Both isoforms contain four exons, with a few single base pair differences between them (FIG. 3 and FIG. 4). The longer form, HTPL-L, encodes a protein of 954 amino acids that has a predicted molecular weight, prior to any post-translational modification, of 107.6 kD. One of the single base pair changes in HTPL-S introduces a premature stop codon at position 2379 of the HTPL-S cDNA. HTPL-S, therefore, encodes a shorter protein of 767 amino acids that has a predicted molecular weight, prior to any post-translational modification, of 86.9 kD. Both cDNA clones appear full lengths, with the open reading frame starting with a methionine and terminating with a stop codon.

As further discussed in the examples herein, expression of HTPL was assessed using hybridization to genome-derived single exon microarrays. Microarray analysis of the fourth exon showed low level expression in all tissues tested, including adrenal, adult liver, bone marrow, brain, fetal liver, kidney, lung, placenta and prostate. This was confirmed by RT_PCR. RT_PCR also detected strong expression in testis and weak expression in skeletal muscle and colon (see Example 3).

As more fully described below, the present invention provides isolated nucleic acids that encode HTPL, including two isoforms, and fragments thereof. The invention further provides vectors for propagation and expression of the nucleic acids of the present invention, host cells comprising the nucleic acids and vectors of the present invention, proteins, protein fragments, and protein fusions of the present invention, and antibodies specific for all or any one of the isoforms. The invention provides pharmaceutical formulations of the nucleic acids, proteins, and antibodies of the present invention. The invention further provides transgenic cells and non-human organisms comprising human HTPL nucleic acids, and transgenic cells and non-human organisms with targeted disruption of the endogenous orthologue of the human HTPL. The invention additionally provides diagnostic, investigational, and therapeutic methods based on the HTPL nucleic acids, proteins, and antibodies of the present invention.

### DEFINITIONS

Unless defined otherwise, all technical and scientific terms used herein have the meaning commonly understood by one of ordinary skill in the art to which this invention belongs.

As used herein, "nucleic acid" (synonymously, "polynucleotide") includes polynucleotides having natural nucleotides in native 5'-3' phosphodiester linkage ― *e.g.*, DNA or RNA ― as well as polynucleotides that have nonnatural nucleotide analogues, nonnative internucleoside bonds, or both, so long as the nonnatural polynucleotide is capable of sequence-discriminating basepairing under experimentally desired conditions. Unless otherwise specified, the term "nucleic acid" includes any topological conformation; the term thus explicitly comprehends single-stranded, double-stranded, partially duplexed, triplexed, hairpinned, circular, and padlocked conformations.

As used herein, an "isolated nucleic acid" is a nucleic acid molecule that exists in a physical form that is nonidentical to any nucleic acid molecule of identical sequence as found in nature; "isolated" does not require, although it does not prohibit, that the nucleic acid so described has itself been physically removed from its native environment.

For example, a nucleic acid can be said to be "isolated" when it includes nucleotides and/or internucleoside bonds not found in nature. When instead composed of natural nucleosides in phosphodiester linkage, a nucleic acid can be said to be "isolated" when it exists at a purity not found in nature, where purity can be adjudged with respect to the presence of nucleic acids of other sequence, with respect to the presence of proteins, with respect to the presence of lipids, or with respect the presence of any other component of a biological cell, or when the nucleic acid lacks sequence that flanks an otherwise identical sequence in an organism's genome, or when the nucleic acid possesses sequence not identically present in nature.

As so defined, "isolated nucleic acid" includes nucleic acids integrated into a host cell chromosome at a heterologous site, recombinant fusions of a native fragment to a heterologous sequence, recombinant vectors present as episomes or as integrated into a host cell chromosome.

As used herein, an isolated nucleic acid "encodes" a reference polypeptide when at least a portion of the nucleic acid, or its complement, can be directly translated to provide the amino acid sequence of the reference polypeptide, or when the isolated nucleic acid can be used, alone or as part of an expression vector, to express the reference polypeptide *in vitro*, in a prokaryotic host cell, or in a eukaryotic host cell.

As used herein, the term "exon" refers to a nucleic acid sequence found in genomic DNA that is bioinformatically predicted and/or experimentally confirmed to contribute contiguous sequence to a mature mRNA transcript.

As used herein, the phrase "open reading frame" and the equivalent acronym "ORF" refer to that portion of a transcript-derived nucleic acid that can be translated in its entirety into a sequence of contiguous amino acids. As so defined, an ORF has length, measured in nucleotides, exactly divisible by 3. As so defined, an ORF need not encode the entirety of a natural protein.

As used herein, the phrase "ORF-encoded peptide" refers to the predicted or actual translation of an ORF.

As used herein, the phrase "degenerate variant" of a reference nucleic acid sequence intends all nucleic acid sequences that can be directly translated, using the standard genetic code, to provide an amino acid sequence identical to that translated from the reference nucleic acid sequence.

As used herein, the term "microarray" and the equivalent phrase "nucleic acid microarray" refer to a substrate-bound collection of plural nucleic acids, hybridization to each of the plurality of bound nucleic acids being separately detectable. The substrate can be solid or porous, planar or non-planar, unitary or distributed.

As so defined, the term "microarray" and phrase "nucleic acid microarray" include all the devices so called in Schena (ed.), DNA Microarrays: A Practical Approach (Practical Approach Series), Oxford University Press (1999) (ISBN: 0199637768); *Nature Genet.* 21(1)(suppl):1 - 60 (1999); and Schena (ed.), Microarray Biochip: Tools and Technology, Eaton Publishing Company/BioTechniques Books Division (2000) (ISBN: 1881299376), the disclosures of which are incorporated herein by reference in their entireties.

As so defined, the term "microarray" and phrase "nucleic acid microarray" also include substrate-bound collections of plural nucleic acids in which the plurality of nucleic acids are distributably disposed on a plurality of beads, rather than on a unitary planar substrate, as is described, *inter alia,* in Brenner *et al., Proc. Natl. Acad. Sci. USA* 97(4):166501670 (2000), the disclosure of which is incorporated herein by reference in its entirety; in such case, the term "microarray" and phrase "nucleic acid microarray" refer to the plurality of beads in aggregate.

As used herein with respect to solution phase hybridization, the term "probe", or equivalently, "nucleic acid probe" or "hybridization probe", refers to an isolated nucleic acid of known sequence that is, or is intended to be, detectably labeled. As used herein with respect to a nucleic acid microarray, the term "probe" (or equivalently "nucleic acid probe" or "hybridization probe") refers to the isolated nucleic acid that is, or is intended to be, bound to the substrate. In either such context, the term "target" refers to nucleic acid intended to be bound to probe by sequence complementarity.

As used herein, the expression "probe comprising SEQ ID NO:X", and variants thereof, intends a nucleic acid probe, at least a portion of which probe has either (i) the sequence directly as given in the referenced SEQ ID NO:X, or (ii) a sequence complementary to the sequence as given in the referenced SEQ ID NO:X, the choice as between sequence directly as given and complement thereof dictated by the requirement that the probe be complementary to the desired target.

As used herein, the phrases "expression of a probe" and "expression of an isolated nucleic acid" and their linguistic equivalents intend that the probe or, (respectively, the isolated nucleic acid), or a probe (or, respectively, isolated nucleic acid) complementary in sequence thereto,can hybridize detectably under high stringency conditions to a sample of nucleic acids that derive from mRNA transcripts from a given source. For example, and by way of illustration only, expression of a probe in "liver" means that the probe can hybridize detectably under high stringency conditions to a sample of nucleic acids that derive from mRNA obtained from liver.

As used herein, "a single exon probe" comprises at least part of an exon ("reference exon") and can hybridize detectably under high stringency conditions to transcript-derived nucleic acids that include the reference exon. The single exon probe will not, however, hybridize detectably under high stringency conditions to nucleic acids that lack the reference exon and that consist of one or more exons that are found adjacent to the reference exon in the genome.

For purposes herein, "high stringency conditions" are defined for solution phase hybridization as aqueous hybridization (*i.e*., free of formamide) in 6X SSC (where 20X SSC contains 3.0 M NaCl and 0.3 M sodium citrate), 1% SDS at 65°C for at least 8 hours, followed by one or more washes in 0.2X SSC, 0.1% SDS at 65°C. "Moderate stringency conditions" are defined for solution phase hybridization as aqueous hybridization (i.e., free of formamide) in 6X SSC, 1% SDS at 65°C for at least 8 hours, followed by one or more washes in 2x SSC, 0.1% SDS at room temperature.

For microarray-based hybridization, standard "high stringency conditions" are defined as hybridization in 50% formamide, 5X SSC, 0.2 µg/µl poly(dA), 0.2 µg/µl human cot1 DNA, and 0.5% SDS, in a humid oven at 42°C overnight, followed by successive washes of the microarray in 1X SSC, 0.2% SDS at 55°C for 5 minutes, and then 0.1X SSC, 0.2% SDS, at 55°C for 20 minutes. For microarray-based hybridization, "moderate stringency conditions", suitable for cross-hybridization to mRNA encoding structurally- and functionally-related proteins, are defined to be the same as those for high stringency conditions but with reduction in temperature for hybridization and washing to room temperature (approximately 25°C).

As used herein, the terms "protein", "polypeptide", and "peptide" are used interchangeably to refer to a naturally-occurring or synthetic polymer of amino acid monomers (residues), irrespective of length, where amino acid monomer here includes naturally-occurring amino acids, naturally-occurring amino acid structural variants, and synthetic non-naturally occurring analogs that are capable of participating in peptide bonds. The terms "protein", "polypeptide", and "peptide" explicitly permits of post-translational and post-synthetic modifications, such as glycosylation.

The term "oligopeptide" herein denotes a protein, polypeptide, or peptide having 25 or fewer monomeric subunits.

The phrases "isolated protein", "isolated polypeptide", "isolated peptide" and "isolated oligopeptide" refer to a protein (or respectively to a polypeptide, peptide, or oligopeptide) that is nonidentical to any protein molecule of identical amino acid sequence as found in nature; "isolated" does not require, although it does not prohibit, that the protein so described has itself been physically removed from its native environment.

For example, a protein can be said to be "isolated" when it includes amino acid analogues or derivatives not found in nature, or includes linkages other than standard peptide bonds.

When instead composed entirely of natural amino acids linked by peptide bonds, a protein can be said to be "isolated" when it exists at a purity not found in nature - where purity can be adjudged with respect to the presence of proteins of other sequence, with respect to the presence of non-protein compounds, such as nucleic acids, lipids, or other components of a biological cell, or when it exists in a composition not found in nature, such as in a host cell that does not naturally express that protein.

A "purified protein" (equally, a purified polypeptide, peptide, or oligopeptide) is an isolated protein, as above described, present at a concentration of at least 95%, as measured on a weight basis with respect to total protein in a composition. A "substantially purified protein" (equally, a substantially purified polypeptide, peptide, or oligopeptide) is an isolated protein, as above described, present at a concentration of at least 70%, as measured on a weight basis with respect to total protein in a composition.

As used herein, the phrase "protein isoforms" refers to a plurality of proteins having nonidentical primary amino acid sequence but that share amino acid sequence encoded by at least one common exon.

As used herein, the phrase "alternative splicing" and its linguistic equivalents includes all types of RNA processing that lead to expression of plural protein isoforms from a single gene; accordingly, the phrase "splice variant(s)" and its linguistic equivalents embraces mRNAs transcribed from a given gene that, however processed, collectively encode plural protein isoforms. For example, and by way of illustration only, splice variants can include exon insertions, exon extensions, exon truncations, exon deletions, alternatives in the 5' untranslated region ("5' UT") and alternatives in the 3' untranslated region ("3' UT"). Such 3' alternatives include, for example, differences in the site of RNA transcript cleavage and site of poly(A) addition. See, *e.g.,* Gautheret *et al., Genome Res.* 8:524-530 (1998).

As used herein, "orthologues" are separate occurrences of the same gene in multiple species. The separate occurrences have similar, albeit nonidentical, amino acid sequences, the degree of sequence similarity depending, in part, upon the evolutionary distance of the species from a common ancestor having the same gene.

As used herein, the term "paralogues" indicates separate occurrences of a gene in one species. The separate occurrences have similar, albeit nonidentical, amino acid sequences, the degree of sequence similarity depending, in part, upon the evolutionary distance from the gene duplication event giving rise to the separate occurrences.

As used herein, the term "homologues" is generic to "orthologues" and "paralogues".

As used herein, the term "antibody" refers to a polypeptide, at least a portion of which is encoded by at least one immunoglobulin gene, or fragment thereof, and that can bind specifically to a desired target molecule. The term includes naturally-occurring forms, as well as fragments and derivatives.

Fragments within the scope of the term "antibody" include those produced by digestion with various proteases, those produced by chemical cleavage and/or chemical dissociation, and those produced recombinantly, so long as the fragment remains capable of specific binding to a target molecule. Among such fragments are Fab, Fab', Fv, F(ab)'₂, and single chain Fv (scFv) fragments.

Derivatives within the scope of the term include antibodies (or fragments thereof) that have been modified in sequence, but remain capable of specific binding to a target molecule, including: interspecies chimeric and humanized antibodies; antibody fusions; heteromeric antibody complexes and antibody fusions, such as diabodies (bispecific antibodies), single-chain diabodies, and intrabodies (see, *e.g.*, Marasco (ed.), Intracellular Antibodies: Research and Disease Applications, Springer-Verlag New York, Inc. (1998) (ISBN: 3540641513), the disclosure of which is incorporated herein by reference in its entirety).

As used herein, antibodies can be produced by any known technique, including harvest from cell culture of native B lymphocytes, harvest from culture of hybridomas, recombinant expression systems, and phage display.

As used herein, "antigen" refers to a ligand that can be bound by an antibody; an antigen need not itself be immunogenic. The portions of the antigen that make contact with the antibody are denominated "epitopes".

"Specific binding" refers to the ability of two molecular species concurrently present in a heterogeneous (inhomogeneous) sample to bind to one another in preference to binding to other molecular species in the sample. Typically, a specific binding interaction will discriminate over adventitious binding interactions in the reaction by at least two-fold, more typically by at least 10-fold, often at least 100-fold; when used to detect analyte, specific binding is sufficiently discriminatory when determinative of the presence of the analyte in a heterogeneous (inhomogeneous) sample. Typically, the affinity or avidity of a specific binding reaction is least about 10⁻⁷ M, with specific binding reactions of greater specificity typically having affinity or avidity of at least 10⁻⁸ M to at least about 10⁻⁹ M.

As used herein, "molecular binding partners" ― and equivalently, "specific binding partners" ― refer to pairs of molecules, typically pairs of biomolecules, that exhibit specific binding. Nonlimiting examples are receptor and ligand, antibody and antigen, and biotin to any of avidin, streptavidin, neutrAvidin and captAvidin.

The term "antisense", as used herein, refers to a nucleic acid molecule sufficiently complementary in sequence, and sufficiently long in that complementary sequence, as to hybridize under intracellular conditions to (i) a target mRNA transcript or (ii) the genomic DNA strand complementary to that transcribed to produce the target mRNA transcript.

The term "portion", as used with respect to nucleic acids, proteins, and antibodies, is synonymous with "fragment".

### NUCLEIC ACID MOLECULES

In a first aspect, the invention provides isolated nucleic acids that encode HTPL, including two isoforms, variants having at least 65% sequence identity thereto, degenerate variants thereof, variants that encode HTPL proteins having conservative or moderately conservative substitutions, cross-hybridizing nucleic acids, and fragments thereof.

FIG. 3 and FIG. 4 presents the nucleotide sequence of the HTPL-L and HTPL-S cDNA clones, with predicted amino acid translation; the sequences are further presented in the Sequence Listing, incorporated herein by reference in its entirety, in SEQ ID NOs: 1 (full length nucleotide sequence of human HTPL-L cDNA), 3 (full length amino acid coding sequence of human HTPL-L), 4 (full length nucleotide sequence of human HTPL-S cDNA), and 6 (full length amino acid coding sequence of human HTPL-S).

Unless otherwise indicated, each nucleotide sequence is set forth herein as a sequence of deoxyribonucleotides. It is intended, however, that the given sequence be interpreted as would be appropriate to the polynucleotide composition: for example, if the isolated nucleic acid is composed of RNA, the given sequence intends ribonucleotides, with uridine substituted for thymidine.

Unless otherwise indicated, nucleotide sequences of the isolated nucleic acids of the present invention were determined by sequencing a DNA molecule that had resulted, directly or indirectly, from at least one enzymatic polymerization reaction (*e.g.*, reverse transcription and/or polymerase chain reaction) using an automated sequencer (such as the MegaBACE™ 1000, Molecular Dynamics, Sunnyvale, CA, USA), or by reliance upon such sequence or upon genomic sequence prior-accessioned into a public database. Unless otherwise indicated, all amino acid sequences of the polypeptides of the present invention were predicted by translation from the nucleic acid sequences so determined.

As a consequence, any nucleic acid sequence presented herein may contain errors introduced by erroneous incorporation of nucleotides during polymerization, by erroneous base calling by the automated sequencer (although such sequencing errors have been minimized for the nucleic acids directly determined herein, unless otherwise indicated, by the sequencing of each of the complementary strands of a duplex DNA), or by similar errors accessioned into the public database. Such errors can readily be identified and corrected by resequencing of the genomic locus using standard techniques.

Single nucleotide polymorphisms (SNPs) occur frequently in eukaryotic genomes - more than 1.4 million SNPs have already identified in the human genome, International Human Genome Sequencing Consortium, *Nature* 409:860 - 921 (2001) - and the sequence determined from one individual of a species may differ from other allelic forms present within the population. Additionally, small deletions and insertions, rather than single nucleotide polymorphisms, are not uncommon in the general population, and often do not alter the function of the protein.

Accordingly, it is an aspect of the present invention to provide nucleic acids not only identical in sequence to those described with particularity herein, but also to provide isolated nucleic acids at least about 65% identical in sequence to those described with particularity herein, typically at least about 70%, 75%, 80%, 85%, or 90% identical in sequence to those described with particularity herein, usefully at least about 91%, 92%, 93%, 94%, or 95% identical in sequence to those described with particularity herein, usefully at least about 96%, 97%, 98%, or 99% identical in sequence to those described with particularity herein, and, most conservatively, at least about 99.5%, 99.6%, 99.7%, 99.8% and 99.9% identical in sequence to those described with particularity herein. These sequence variants can be naturally occurring or can result from human intervention, as by random or directed mutagenesis.

For purposes herein, percent identity of two nucleic acid sequences is determined using the procedure of Tatiana *et al.*, "Blast 2 sequences - a new tool for comparing protein and nucleotide sequences", *FEMS Microbiol Lett.* 174:247-250 (1999), which procedure is effectuated by the computer program BLAST 2 SEQUENCES, available online at
http://www.ncbi.nlm.nih.gov/blast/bl2seq/bl2.html.
To assess percent identity of nucleic acids, the BLASTN module of BLAST 2 SEQUENCES is used with default values of (i) reward for a match: 1; (ii) penalty for a mismatch: -2; (iii) open gap 5 and extension gap 2 penalties; (iv) gap X_dropoff 50 expect 10 word size 11 filter, and both sequences are entered in their entireties.

As is well known, the genetic code is degenerate, with each amino acid except methionine translated from a plurality of codons, thus permitting a plurality of nucleic acids of disparate sequence to encode the identical protein. As is also well known, codon choice for optimal expression varies from species to species. The isolated nucleic acids of the present invention being useful for expression of HTPL proteins and protein fragments, it is, therefore, another aspect of the present invention to provide isolated nucleic acids that encode HTPL proteins and portions thereof not only identical in sequence to those described with particularity herein, but degenerate variants thereof as well.

As is also well known, amino acid substitutions occur frequently among natural allelic variants, with conservative substitutions often occasioning only *de minimis* change in protein function.

Accordingly, it is an aspect of the present invention to provide nucleic acids not only identical in sequence to those described with particularity herein, but also to provide isolated nucleic acids that encode HTPL, and portions thereof, having conservative amino acid substitutions, and also to provide isolated nucleic acids that encode HTPL, and portions thereof, having moderately conservative amino acid substitutions.

Although there are a variety of metrics for calling conservative amino acid substitutions, based primarily on either observed changes among evolutionarily related proteins or on predicted chemical similarity, for purposes herein a conservative replacement is any change having a positive value in the PAM250 log-likelihood matrix reproduced herein below (*see* Gonnet *et al.*, *Science* 256(5062):1443-5 (1992)):

| | **A** | **R** | **N** | **D** | **C** | **Q** | **E** | **G** | **H** | **I** | **L** | **K** | **M** | **F** | **P** | **S** | **T** | **W** | **Y** | **V** |
|---|---|---|---|---|---|---|---|---|---|---|---|---|---|---|---|---|---|---|---|---|
| **A** | 2 | -1 | 0 | 0 | 0 | 0 | 0 | 0 | -1 | -1 | -1 | 0 | -1 | -2 | 0 | 1 | 1 | -4 | -2 | 0 |
| **R** | -1 | 5 | 0 | 0 | -2 | 2 | 0 | -1 | 1 | -2 | -2 | 3 | -2 | -3 | -1 | 0 | 0 | -2 | -2 | -2 |
| **N** | 0 | 0 | 4 | 2 | -2 | 1 | 1 | 0 | 1 | -3 | -3 | 1 | -2 | -3 | -1 | 1 | 0 | -4 | -1 | -2 |
| **D** | 0 | 0 | 2 | 5 | -3 | 1 | 3 | 0 | 0 | -4 | -4 | 0 | -3 | -4 | -1 | 0 | 0 | -5 | -3 | -3 |
| **C** | 0 | -2 | -2 | -3 | 12 | -2 | -3 | -2 | -1 | -1 | -2 | -3 | -1 | -1 | -3 | 0 | 0 | -1 | 0 | 0 |
| **Q** | 0 | 2 | 1 | 1 | -2 | 3 | 2 | -1 | 1 | -2 | -2 | 2 | -1 | -3 | 0 | 0 | 0 | -3 | -2 | -2 |
| **E** | 0 | 0 | 1 | 3 | -3 | 2 | 4 | -1 | 0 | -3 | -3 | 1 | -2 | -4 | 0 | 0 | 0 | -4 | -3 | -2 |
| **G** | 0 | -1 | 0 | 0 | -2 | -1 | -1 | 7 | -1 | -4 | -4 | -1 | -4 | -5 | -2 | 0 | -1 | -4 | -4 | -3 |
| **H** | -1 | 1 | 1 | 0 | -1 | 1 | 0 | -1 | 6 | -2 | -2 | 1 | -1 | 0 | -1 | 0 | 0 | -1 | 2 | -2 |
| **I** | -1 | -2 | -3 | -4 | -1 | -2 | -3 | -4 | -2 | 4 | 3 | -2 | 2 | 1 | -3 | -2 | -1 | -2 | -1 | 3 |
| **L** | -1 | -2 | -3 | -4 | -2 | -2 | -3 | -4 | -2 | 3 | 4 | -2 | 3 | 2 | -2 | -2 | -1 | -1 | 0 | 2 |
| **K** | 0 | 3 | 1 | 0 | -3 | 2 | 1 | -1 | 1 | -2 | -2 | 3 | -1 | -3 | -1 | 0 | 0 | -4 | -2 | -2 |
| **M** | -1 | -2 | -2 | -3 | -1 | -1 | -2 | -4 | -1 | 2 | 3 | -1 | 4 | 2 | -2 | -1 | -1 | -1 | 0 | 2 |
| **F** | -2 | -3 | -3 | -4 | -1 | -3 | -4 | -5 | 0 | 1 | 2 - | 3 | 2 | 7 | -4 | -3 | -2 | 4 | 5 | 0 |
| **P** | 0 | -1 | -1 | -1 | -3 | 0 | 0 | -2 | -1 | -3 | -2 | -1 | -2 | -4 | 8 | 0 | 0 | -5 | -3 | -2 |
| **S** | 1 | 0 | 1 | 0 | 0 | 0 | 0 | 0 | 0 | -2 | -2 | 0 | -1 | -3 | 0 | 2 | 2 | -3 | -2 | -1 |
| **T** | 1 | 0 | 0 | 0 | 0 | 0 | 0 | -1 | 0 | -1 | -1 | 0 | -1 | -2 | 0 | 2 | 2 | -4 | -2 | 0 |
| **W** | -4 | -2 | -4 | -5 | -1 | -3 | -4 | -4 | -1 | -2 | -1 | -4 | -1 | 4 | -5 | -3 | -4 | 14 | 4 | -3 |
| **Y** | -2 | -2 | -1 | -3 | 0 | -2 | -3 | -4 | 2 | -1 | 0 | -2 | 0 | 5 | -3 | -2 | -2 | 4 | 8 | -1 |
| **V** | 0 | -2 | -2 | -3 | 0 | -2 | -2 | -3 | -2 | 3 | 2 | -2 | 2 | 0 | -2 | -1 | 0 | -3 | -1 | 3 |

For purposes herein, a "moderately conservative" replacement is any change having a nonnegative value in the PAM250 log-likelihood matrix reproduced herein above.

As is also well known in the art, relatedness of nucleic acids can also be characterized using a functional test, the ability of the two nucleic acids to base-pair to one another at defined hybridization stringencies.

It is, therefore, another aspect of the invention to provide isolated nucleic acids not only identical in sequence to those described with particularity herein, but also to provide isolated nucleic acids ("cross-hybridizing nucleic acids") that hybridize under high stringency conditions (as defined herein below) to all or to a portion of various of the isolated HTPL nucleic acids of the present invention ("reference nucleic acids"), as well as cross-hybridizing nucleic acids that hybridize under moderate stringency conditions to all or to a portion of various of the isolated HTPL nucleic acids of the present invention.

Such cross-hybridizing nucleic acids are useful, *inter alia,* as probes for, and to drive expression of, proteins related to the proteins of the present invention as alternative isoforms, homologues, paralogues, and orthologues. Particularly useful orthologues are those from other primate species, such as chimpanzee, rhesus macaque, monkey, baboon, orangutan, and gorilla; from rodents, such as rats, mice, guinea pigs; from lagomorphs, such as rabbits; and from domestic livestock, such as cow, pig, sheep, horse, goat and chicken.

For purposes herein, high stringency conditions are defined as aqueous hybridization *(i.e.,* free of formamide) in 6X SSC (where 20X SSC contains 3.0 M NaCl and 0.3 M sodium citrate), 1% SDS at 65°C for at least 8 hours, followed by one or more washes in 0.2X SSC, 0.1% SDS at 65°C. For purposes herein, moderate stringency conditions are defined as aqueous hybridization *(i.e.,* free of formamide) in 6X SSC, 1% SDS at 65°C for at least 8 hours, followed by one or more washes in 2x SSC, 0.1% SDS at room temperature.

The hybridizing portion of the reference nucleic acid is typically at least 15 nucleotides in length, often at least 17 nucleotides in length. Often, however, the hybridizing portion of the reference nucleic acid is at least 20 nucleotides in length, 25 nucleotides in length, and even 30 nucleotides, 35 nucleotides, 40 nucleotides, and 50 nucleotides in length. Of course, cross-hybridizing nucleic acids that hybridize to a larger portion of the reference nucleic acid - for example, to a portion of at least 50 nt, at least 100 nt, at least 150 nt, 200 nt, 250 nt, 300 nt, 350 nt, 400 nt, 450 nt, or 500 nt or more - or even to the entire length of the reference nucleic acid, are also useful.

The hybridizing portion of the cross-hybridizing nucleic acid is at least 75% identical in sequence to at least a portion of the reference nucleic acid. Typically, the hybridizing portion of the cross-hybridizing nucleic acid is at least 80%, often at least 85%, 86%, 87%, 88%, 89% or even at least 90% identical in sequence to at least a portion of the reference nucleic acid. Often, the hybridizing portion of the cross-hybridizing nucleic acid will be at least 91%, 92%, 93%, 94%, 95%, 96%, 97%, 98%, or 99% identical in sequence to at least a portion of the reference nucleic acid sequence. At times, the hybridizing portion of the cross-hybridizing nucleic acid will be at least 99.5% identical in sequence to at least a portion of the reference nucleic acid.

The invention also provides fragments of various of the isolated nucleic acids of the present invention.

By "fragments" of a reference nucleic acid is here intended isolated nucleic acids, however obtained, that have a nucleotide sequence identical to a portion of the reference nucleic acid sequence, which portion is at least 17 nucleotides and less than the entirety of the reference nucleic acid. As so defined, "fragments" need not be obtained by physical fragmentation of the reference nucleic acid, although such provenance is not thereby precluded.

In theory, an oligonucleotide of 17 nucleotides is of sufficient length as to occur at random less frequently than once in the three gigabase human genome, and thus to provide a nucleic acid probe that can uniquely identify the reference sequence in a nucleic acid mixture of genomic complexity. As is well known, further specificity can be obtained by probing nucleic acid samples of subgenomic complexity, and/or by using plural fragments as short as 17 nucleotides in length collectively to prime amplification of nucleic acids, as, *e.g.*, by polymerase chain reaction (PCR).

As further described herein below, nucleic acid fragments that encode at least 6 contiguous amino acids *(i.e.,* fragments of 18 nucleotides or more) are useful in directing the expression or the synthesis of peptides that have utility in mapping the epitopes of the protein encoded by the reference nucleic acid. *See, e.g.,* Geysen *et al.,* "Use of peptide synthesis to probe viral antigens for epitopes to a resolution of a single amino acid," *Proc. Natl. Acad. Sci. USA* 81:3998-4002 (1984); and U.S. Pat. Nos. 4,708,871 and 5,595,915, the disclosures of which are incorporated herein by reference in their entireties.

As further described herein below, fragments that encode at least 8 contiguous amino acids *(i.e.,* fragments of 24 nucleotides or more) are useful in directing the expression or the synthesis of peptides that have utility as immunogens. *See, e.g.*, Lerner, "Tapping the immunological repertoire to produce antibodies of predetermined specificity," Nature 299:592-596 (1982); Shinnick *et al.,* "Synthetic peptide immunogens as vaccines," *Annu. Rev. Microbiol.* 37:425-46 (1983); Sutcliffe *et al.*, "Antibodies that react with predetermined sites on proteins," *Science* 219:660-6 (1983), the disclosures of which are incorporated herein by reference in their entireties.

The nucleic acid fragment of the present invention is thus at least 17 nucleotides in length, typically at least 18 nucleotides in length, and often at least 24 nucleotides in length. Often, the nucleic acid of the present invention is at least 25 nucleotides in length, and even 30 nucleotides, 35 nucleotides, 40 nucleotides, or 45 nucleotides in length. Of course, larger fragments having at least 50 nt, at least 100 nt, at least 150 nt, 200 nt, 250 nt, 300 nt, 350 nt, 400 nt, 450 nt, or 500 nt or more are also useful, and at times preferred.

Having been based upon the mining of genomic sequence, rather than upon surveillance of expressed message, the present invention further provides isolated genome-derived nucleic acids that include portions of the HTPL gene.

The invention particularly provides genome-derived single exon probes.

As further described in commonly owned and copending U.S. patent application serial nos. 09/864,761, filed May 23, 2001; 09/774,203, filed January 29, 2001; and 09/632,366, filed August 3, 2000, the disclosures of which are incorporated herein by reference in their entireties, "a single exon probe" comprises at least part of an exon ("reference exon") and can hybridize detectably under high stringency conditions to transcript-derived nucleic acids that include the reference exon. The single exon probe will not, however, hybridize detectably under high stringency conditions to nucleic acids that lack the reference exon and instead consist of one or more exons that are found adjacent to the reference exon in the genome.

Genome-derived single exon probes typically further comprise, contiguous to a first end of the exon portion, a first intronic and/or intergenic sequence that is identically contiguous to the exon in the genome. Often, the genome-derived single exon probe further comprises, contiguous to a second end of the exonic portion, a second intronic and/or intergenic sequence that is identically contiguous to the exon in the genome.

The minimum length of genome-derived single exon probes is defined by the requirement that the exonic portion be of sufficient length to hybridize under high stringency conditions to transcript-derived nucleic acids. Accordingly, the exon portion is at least 17 nucleotides, typically at least 18 nucleotides, 20 nucleotides, 24 nucleotides, 25 nucleotides or even 30, 35, 40, 45, or 50 nucleotides in length, and can usefully include the entirety of the exon, up to 100 nt, 150 nt, 200 nt, 250 nt, 300 nt, 350 nt, 400 nt or even 500 nt or more in length.

The maximum length of genome-derived single exon probes is defined by the requirement that the probes contain portions of no more than one exon, that is, be unable to hybridize detectably under high stringency conditions to nucleic acids that lack the reference exon but include one or more exons that are found adjacent to the reference exon the genome.

Given variable spacing of exons through eukaryotic genomes, the maximum length of single exon probes of the present invention is typically no more than 25 kb, often no more than 20 kb, 15 kb, 10 kb or 7.5 kb, or even no more than 5 kb, 4 kb, 3 kb, or even no more than about 2.5 kb in length.

The genome-derived single exon probes of the present invention can usefully include at least a first terminal priming sequence not found in contiguity with the rest of the probe sequence in the genome, and often will contain a second terminal priming sequence not found in contiguity with the rest of the probe sequence in the genome.

The present invention also provides isolated genome-derived nucleic acids that include nucleic acid sequence elements that control transcription of the HTPL gene.

With a complete draft of the human genome now available, genomic sequences that are within the vicinity of the HTPL coding region (and that are additional to those described with particularity herein) can readily be obtained by PCR amplification.

The isolated nucleic acids of the present invention can be composed of natural nucleotides in native 5'-3' phosphodiester internucleoside linkage - *e.g.*, DNA or RNA - or can contain any or all of nonnatural nucleotide analogues, nonnative internucleoside bonds, or post-synthesis modifications, either throughout the length of the nucleic acid or localized to one or more portions thereof.

As is well known in the art, when the isolated nucleic acid is used as a hybridization probe, the range of such nonnatural analogues, nonnative internucleoside bonds, or post-synthesis modifications will be limited to those that permit sequence-discriminating basepairing of the resulting nucleic acid. When used to direct expression or RNA or protein *in vitro* or *in vivo,* the range of such nonnatural analogues, nonnative internucleoside bonds, or post-synthesis modifications will be limited to those that permit the nucleic acid to function properly as a polymerization substrate. When the isolated nucleic acid is used as a therapeutic agent, the range of such changes will be limited to those that do not confer toxicity upon the isolated nucleic acid.

For example, when desired to be used as probes, the isolated nucleic acids of the present invention can usefully include nucleotide analogues that incorporate labels that are directly detectable, such as radiolabels or fluorophores, or nucleotide analogues that incorporate labels that can be visualized in a subsequent reaction, such as biotin or various haptens.

Common radiolabeled analogues include those labeled with ³³P, ³²P, and ³⁵S, such as α-³²P-dATP, α-³²P-dCTP, α-³²P-dGTP, α-³²P-dTTP, α-³²P-3'dATP, α-³²P-ATP, α-³²P-CTP, α-³²P-GTP, α-³²P-UTP, α-³⁵S-dATP, γ-³⁵S-GTP, γ-³³P-dATP, and the like.

Commercially available fluorescent nucleotide analogues readily incorporated into the nucleic acids of the present invention include Cy3-dCTP, Cy3-dUTP, Cy5-dCTP, Cy3-dUTP (Amersham Pharmacia Biotech, Piscataway, New Jersey, USA), fluorescein-12-dUTP, tetramethylrhodamine-6-dUTP, Texas Red®-5-dUTP, Cascade Blue®-7-dUTP, BODIPY® FL-14-dUTP, BODIPY® TMR-14-dUTP, BODIPY® TR-14-dUTP, Rhodamine Green™-5-dUTP, Oregon Green® 488-5-dUTP, Texas Red®-12-dUTP, BODIPY® 630/650-14-dUTP, BODIPY® 650/665-14-dUTP, Alexa Fluor® 488-5-dUTP, Alexa Fluor® 532-5-dUTP, Alexa Fluor® 568-5-dUTP, Alexa Fluor® 594-5-dUTP, Alexa Fluor® 546-14-dUTP, fluorescein-12-UTP, tetramethylrhodamine-6-UTP, Texas Red®-5-UTP, Cascade Blue®-7-UTP, BODIPY® FL-14-UTP, BODIPY® TMR-14-UTP, BODIPY® TR-14-UTP, Rhodamine Green™-5-UTP, Alexa Fluor® 488-5-UTP, Alexa Fluor® 546-14-UTP (Molecular Probes, Inc. Eugene, OR, USA).

Protocols are available for custom synthesis of nucleotides having other fluorophores. Henegariu *et al.,* "Custom Fluorescent-Nucleotide Synthesis as an Alternative Method for Nucleic Acid Labeling," *Nature Biotechnol.* 18:345 - 348 (2000), the disclosure of which is incorporated herein by reference in its entirety.

Haptens that are commonly conjugated to nucleotides for subsequent labeling include biotin (biotin-11-dUTP, Molecular Probes, Inc., Eugene, OR, USA; biotin-21-UTP, biotin-21-dUTP, Clontech Laboratories, Inc., Palo Alto, CA, USA), digoxigenin (DIG-11-dUTP, alkali labile, DIG-11-UTP, Roche Diagnostics Corp., Indianapolis, IN, USA), and dinitrophenyl (dinitrophenyl-11-dUTP, Molecular Probes, Inc., Eugene, OR, USA).

As another example, when desired to be used for antisense inhibition of transcription or translation, the isolated nucleic acids of the present invention can usefully include altered, often nuclease-resistant, internucleoside bonds. See Hartmann *et al.* (eds.), Manual of Antisense Methodology (Perspectives in Antisense Science), Kluwer Law International (1999) (ISBN:079238539X); Stein *et al.* (eds.), Applied Antisense Oligonucleotide Technology, Wiley-Liss (cover (1998) (ISBN: 0471172790); Chadwick *et al.* (eds.), Oligonucleotides as Therapeutic Agents - Symposium No. 209, John Wiley & Son Ltd (1997) (ISBN: 0471972797), the disclosures of which are incorporated herein by reference in their entireties. Such altered internucloside bonds are often desired also when the isolated nucleic acid of the present invention is to be used for targeted gene correction, Gamper *et al., Nucl. Acids Res.* 28(21):4332-4339 (2000), the disclosures of which are incorporated herein by reference in its entirety.

Modified oligonucleotide backbones often preferred when the nucleic acid is to be used for antisense purposes are, for example, phosphorothioates, chiral phosphorothioates, phosphorodithioates, phosphotriesters, aminoalkylphosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those having inverted polarity wherein the adjacent pairs of nucleoside units are linked 3'-5' to 5'-3' or 2'-5' to 5'-2'. Representative U.S. patents that teach the preparation of the above phosphorus-containing linkages include, but are not limited to, U.S. Pat. Nos. 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; and 5,625,050, the disclosures of which are incorporated herein by reference in their entireties.

Preferred modified oligonucleotide backbones for antisense use that do not include a phosphorus atom have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH₂ component parts. Representative U.S. patents that teach the preparation of the above backbones include, but are not limited to, U.S. Pat. Nos. 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; and 5,677,439, the disclosures of which are incorporated herein by reference in their entireties.

In other preferred oligonucleotide mimetics, both the sugar and the internucleoside linkage are replaced with novel groups, such as peptide nucleic acids (PNA).

In PNA compounds, the phosphodiester backbone of the nucleic acid is replaced with an amide-containing backbone, in particular by repeating N-(2-aminoethyl) glycine units linked by amide bonds. Nucleobases are bound directly or indirectly to aza nitrogen atoms of the amide portion of the backbone, typically by methylene carbonyl linkages.

The uncharged nature of the PNA backbone provides PNA/DNA and PNA/RNA duplexes with a higher thermal stability than is found in DNA/DNA and DNA/RNA duplexes, resulting from the lack of charge repulsion between the PNA and DNA or RNA strand. In general, the Tm of a PNA/DNA or PNA/RNA duplex is 1°C higher per base pair than the Tm of the corresponding DNA/DNA or DNA/RNA duplex (in 100 mM NaCl).

The neutral backbone also allows PNA to form stable DNA duplexes largely independent of salt concentration. At low ionic strength, PNA can be hybridized to a target sequence at temperatures that make DNA hybridization problematic or impossible. And unlike DNA/DNA duplex formation, PNA hybridization is possible in the absence of magnesium. Adjusting the ionic strength, therefore, is useful if competing DNA or RNA is present in the sample, or if the nucleic acid being probed contains a high level of secondary structure.

PNA also demonstrates greater specificity in binding to complementary DNA. A PNA/DNA mismatch is more destabilizing than DNA/DNA mismatch. A single mismatch in mixed a PNA/DNA 15-mer lowers the Tm by 8-20°C (15°C on average). In the corresponding DNA/DNA duplexes, a single mismatch lowers the Tm by 4-16°C (11°C on average). Because PNA probes can be significantly shorter than DNA probes, their specificity is greater.

Additionally, nucleases and proteases do not recognize the PNA polyamide backbone with nucleobase sidechains. As a result, PNA oligomers are resistant to degradation by enzymes, and the lifetime of these compounds is extended both *in vivo* and *in vitro.* In addition, PNA is stable over a wide pH range.

Because its backbone is formed from amide bonds, PNA can be synthesized using a modified peptide synthesis protocol. PNA oligomers can be synthesized by both Fmoc and tBoc methods. Representative U.S. patents that teach the preparation of PNA compounds include, but are not limited to, U.S. Pat. Nos. 5,539,082; 5,714,331; and 5,719,262, each of which is herein incorporated by reference; automated PNA synthesis is readily achievable on commercial synthesizers (see, *e.g.,* "PNA User's Guide," Rev. 2, February 1998, Perseptive Biosystems Part No. 60138, Applied Biosystems, Inc., Foster City, CA).

PNA chemistry and applications are reviewed, *inter alia,* in Ray *et al., FASEB J.* 14(9):1041-60 (2000); Nielsen *et al., Pharmacol Toxicol.* 86(1):3-7 (2000); Larsen *et al., Biochim Biophys Acta.* 1489(1):159-66 (1999); Nielsen, *Curr. Opin. Struct. Biol.* 9(3):353-7 (1999), and Nielsen, *Curr. Opin. Biotechnol.* 10(1):71-5 (1999), the disclosures of which are incorporated herein by reference in their entireties.

Differences from nucleic acid compositions found in nature - *e.g.*, nonnative bases, altered internucleoside linkages, post-synthesis modification - can be present throughout the length of the nucleic acid or can, instead, usefully be localized to discrete portions thereof. As an example of the latter, chimeric nucleic acids can be synthesized that have discrete DNA and RNA domains and demonstrated utility for targeted gene repair, as further described in U.S. Pat. Nos. 5,760,012 and 5,731,181, the disclosures of which are incorporated herein by reference in their entireties. As another example, chimeric nucleic acids comprising both DNA and PNA have been demonstrated to have utility in modified PCR reactions. See Misra *et al., Biochem.* 37: 1917-1925 (1998); *see also* Finn *et al., Nucl. Acids* Res. 24: 3357-3363 (1996), incorporated herein by reference.

Unless otherwise specified, nucleic acids of the present invention can include any topological conformation appropriate to the desired use; the term thus explicitly comprehends, among others, single-stranded, double-stranded, triplexed, quadruplexed, partially double-stranded, partially-triplexed, partially-quadruplexed, branched, hairpinned, circular, and padlocked conformations. Padlock conformations and their utilities are further described in Baner *et al., Curr. Opin. Biotechnol.* 12:11-15 (2001); Escude *et al.,* Proc. Natl. Acad. Sci. USA 14;96(19):10603-7 (1999); Nilsson *et al., Science* 265(5181):2085-8 (1994), the disclosures of which are incorporated herein by reference in their entireties. Triplex and quadruplex conformations, and their utilities, are reviewed in Praseuth *et al., Biochim. Biophys. Acta*. 1489(1):181-206 (1999); Fox, *Curr. Med. Chem.* 7(1):17-37 (2000); Kochetkova *et al., Methods Mol. Biol*. 130:189-201 (2000); Chan *et al., J. Mol.* *Med.* 75(4):267-82 (1997), the disclosures of which are incorporated herein by reference in their entireties.

The nucleic acids of the present invention can be detectably labeled.

Commonly-used labels include radionuclides, such as ³²P, ³³P, ³⁵S, ³H (and for NMR detection, ¹³C and ¹⁵N), haptens that can be detected by specific antibody or high affinity binding partner (such as avidin), and fluorophores.

As noted above, detectable labels can be incorporated by inclusion of labeled nucleotide analogues in the nucleic acid.

Such analogues can be incorporated by enzymatic polymerization, such as by nick translation, random priming, polymerase chain reaction (PCR), terminal transferase tailing, and end-filling of overhangs, for DNA molecules, and *in vitro* transcription driven, *e.g.*, from phage promoters, such as T7, T3, and SP6, for RNA molecules. Commercial kits are readily available for each such labeling approach.

Analogues can also be incorporated during automated solid phase chemical synthesis.

As is well known, labels can also be incorporated after nucleic acid synthesis, with the 5' phosphate and 3' hydroxyl providing convenient sites for post-synthetic covalent attachment of detectable labels.

Various other post-synthetic approaches permit internal labeling of nucleic acids.

For example, fluorophores can be attached using a cisplatin reagent that reacts with the N7 of guanine residues (and, to a lesser extent, adenine bases) in DNA, RNA, and PNA to provide a stable coordination complex between the nucleic acid and fluorophore label (Universal Linkage System) (available from Molecular Probes, Inc., Eugene, OR, USA and Amersham Pharmacia Biotech, Piscataway, NJ, USA); see Alers *et al., Genes, Chromosomes & Cancer,* Vol. 25, pp. 301 - 305 (1999); Jelsma *et al., J. NIH Res.* 5:82 (1994); Van Belkum *et al., BioTechniques* 16:148-153 (1994), incorporated herein by reference. As another example, nucleic acids can be labeled using a disulfide-containing linker (FastTag™ Reagent, Vector Laboratories, Inc., Burlingame, CA, USA) that is photo- or thermally coupled to the target nucleic acid using aryl azide chemistry; after reduction, a free thiol is available for coupling to a hapten, fluorophore, sugar, affinity ligand, or other marker.

Multiple independent or interacting labels can be incorporated into the nucleic acids of the present invention.

For example, both a fluorophore and a moiety that in proximity thereto acts to quench fluorescence can be included to report specific hybridization through release of fluorescence quenching, Tyagi *et al., Nature Biotechnol*. 14: 303-308 (1996); Tyagi *et al., Nature Biotechnol*. 16, 49-53 (1998); Sokol *et al.*, *Proc. Natl. Acad. Sci. USA* 95: 11538-11543 (1998); Kostrikis *et al., Science* 279:1228-1229 (1998); Marras *et al., Genet.* Anal. 14: 151-156 (1999); U.S. Pat. Nos. 5,846,726, 5,925,517, 5925517, or to report exonucleotidic excision, U.S. Pat. No. 5,538,848; Holland *et al., Proc. Natl. Acad. Sci. USA* 88:7276-7280 (1991); Heid *et al., Genome Res.* 6(10):986-94 (1996); Kuimelis *et al., Nucleic Acids Symp Ser.* (37):255-6 (1997); U.S. Patent No. 5,723,591, the disclosures of which are incorporated herein by reference in their entireties.

So labeled, the isolated nucleic acids of the present invention can be used as probes, as further described below.

Nucleic acids of the present invention can also usefully be bound to a substrate. The substrate can porous or solid, planar or non-planar, unitary or distributed; the bond can be covalent or noncovalent. Bound to a substrate, nucleic acids of the present invention can be used as probes in their unlabeled state.

For example, the nucleic acids of the present invention can usefully be bound to a porous substrate, commonly a membrane, typically comprising nitrocellulose, nylon, or positively-charged derivatized nylon; so attached, the nucleic acids of the present invention can be used to detect HTPL nucleic acids present within a labeled nucleic acid sample, either a sample of genomic nucleic acids or a sample of transcript-derived nucleic acids, *e.g.* by reverse dot blot.

The nucleic acids of the present invention can also usefully be bound to a solid substrate, such as glass, although other solid materials, such as amorphous silicon, crystalline silicon, or plastics, can also be used. Such plastics include polymethylacrylic, polyethylene, polypropylene, polyacrylate, polymethylmethacrylate, polyvinylchloride, polytetrafluoroethylene, polystyrene, polycarbonate, polyacetal, polysulfone, celluloseacetate, cellulosenitrate, nitrocellulose, or mixtures thereof.

Typically, the solid substrate will be rectangular, although other shapes, particularly disks and even spheres, present certain advantages. Particularly advantageous alternatives to glass slides as support substrates for array of nucleic acids are optical discs, as described in Demers, "Spatially Addressable Combinatorial Chemical Arrays in CD-ROM Format," international patent publication WO 98/12559, incorporated herein by reference in its entirety.

The nucleic acids of the present invention can be attached covalently to a surface of the support substrate or applied to a derivatized surface in a chaotropic agent that facilitates denaturation and adherence by presumed noncovalent interactions, or some combination thereof.

The nucleic acids of the present invention can be bound to a substrate to which a plurality of other nucleic acids are concurrently bound, hybridization to each of the plurality of bound nucleic acids being separately detectable. At low density, *e.g.* on a porous membrane, these substrate-bound collections are typically denominated macroarrays; at higher density, typically on a solid support, such as glass, these substrate bound collections of plural nucleic acids are colloquially termed microarrays. As used herein, the term microarray includes arrays of all densities. It is, therefore, another aspect of the invention to provide microarrays that include the nucleic acids of the present invention.

The isolated nucleic acids of the present invention can be used as hybridization probes to detect, characterize, and quantify HTPL nucleic acids in, and isolate HTPL nucleic acids from, both genomic and transcript-derived nucleic acid samples. When free in solution, such probes are typically, but not invariably, detectably labeled; bound to a substrate, as in a microarray, such probes are typically, but not invariably unlabeled.

For example, the isolated nucleic acids of the present invention can be used as probes to detect and characterize gross alterations in the HTPL genomic locus, such as deletions, insertions, translocations, and duplications of the HTPL genomic locus through fluorescence *in situ* hybridization (FISH) to chromosome spreads. *See, e.g.,* Andreeff *et al.* (eds.), Introduction to Fluorescence *In Situ* Hybridization: Principles and Clinical Applications, John Wiley & Sons (1999) (ISBN: 0471013455), the disclosure of which is incorporated herein by reference in its entirety. The isolated nucleic acids of the present invention can be used as probes to assess smaller genomic alterations using, *e.g.*, Southern blot detection of restriction fragment length polymorphisms. The isolated nucleic acids of the present invention can be used as probes to isolate genomic clones that include the nucleic acids of the present invention, which thereafter can be restriction mapped and sequenced to identify deletions, insertions, translocations, and substitutions (single nucleotide polymorphisms, SNPs) at the sequence level.

The isolated nucleic acids of the present invention can also be used as probes to detect, characterize, and quantify HTPL nucleic acids in, and isolate HTPL nucleic acids from, transcript-derived nucleic acid samples.

For example, the isolated nucleic acids of the present invention can be used as hybridization probes to detect, characterize by length, and quantify HTPL mRNA by northern blot of total or poly-A⁺- selected RNA samples. For example, the isolated nucleic acids of the present invention can be used as hybridization probes to detect, characterize by location, and quantify HTPL message by *in situ* hybridization to tissue sections (see, *e.g.,* Schwarchzacher *et al.,* In Situ Hybridization, Springer-Verlag New York (2000) (ISBN: 0387915966), the disclosure of which is incorporated herein by reference in its entirety). For example, the isolated nucleic acids of the present invention can be used as hybridization probes to measure the representation of HTPL clones in a cDNA library. For example, the isolated nucleic acids of the present invention can be used as hybridization probes to isolate HTPL nucleic acids from cDNA libraries, permitting sequence level characterization of HTPL messages, including identification of deletions, insertions, truncations - including deletions, insertions, and truncations of exons in alternatively spliced forms - and single nucleotide polymorphisms.

All of the aforementioned probe techniques are well within the skill in the art, and are described at greater length in standard texts such as Sambrook *et al.,* Molecular Cloning: A Laboratory Manual (3^{rd} ed.), Cold Spring Harbor Laboratory Press (2001) (ISBN: 0879695773); Ausubel *et al.* (eds.), Short Protocols in Molecular Biology: A Compendium of Methods from Current Protocols in Molecular Biology (4^{th} ed.), John Wiley & Sons, 1999 (ISBN: 047132938X); and Walker *et al.* (eds.), The Nucleic Acids Protocols Handbook, Humana Press (2000) (ISBN: 0896034593), the disclosures of which are incorporated herein by reference in their entirety.

As described in the Examples herein below, the nucleic acids of the present invention can also be used to detect and quantify HTPL nucleic acids in transcript-derived samples - that is, to measure expression of the HTPL gene - when included in a microarray. Measurement of HTPL expression has particular utility in diagnosis and treatment of male infertility and tumor, as further described in the Examples herein below.

As would be readily apparent to one of skill in the art, each HTPL nucleic acid probe - whether labeled, substrate-bound, or both - is thus currently available for use as a tool for measuring the level of HTPL expression in the tissue in which expression has already been confirmed, notably testis, also adrenal, adult and fetal liver, bone marrow, brain, kidney, lung, placenta, prostate, skeletal muscle and colon. The utility is specific to the probe: under high stringency conditions, the probe reports the level of expression of message specifically containing that portion of the HTPL gene included within the probe.

Measuring tools are well known in many arts, not just in molecular biology, and are known to possess credible, specific, and substantial utility. For example, U.S. Patent No. 6,016,191 describes and claims a tool for measuring characteristics of fluid flow in a hydrocarbon well; U.S. Patent No. 6,042,549 describes and claims a device for measuring exercise intensity; U.S. Patent No. 5,889,351 describes and claims a device for measuring viscosity and for measuring characteristics of a fluid; U.S. Patent No. 5,570,694 describes and claims a device for measuring blood pressure; U.S. Patent No. 5,930,143 describes and claims a device for measuring the dimensions of machine tools; U.S. Patent No. 5,279,044 describes and claims a measuring device for determining an absolute position of a movable element; U.S. Patent No. 5,186,042 describes and claims a device for measuring action force of a wheel; and U.S. Patent No. 4,246,774 describes and claims a device for measuring the draft of smoking articles such as cigarettes.

As for tissues not yet demonstrated to express HTPL, the HTPL nucleic acid probes of the present invention are currently available as tools for surveying such tissues to detect the presence of HTPL nucleic acids.

Survey tools - *i.e.,* tools for determining the presence and/or location of a desired object by search of an area - are well known in many arts, not just in molecular biology, and are known to possess credible, specific, and substantial utility. For example, U.S. Patent No. 6,046,800 describes and claims a device for surveying an area for objects that move; U.S. Patent No. 6,025,201 describes and claims an apparatus for locating and discriminating platelets from non-platelet particles or cells on a cell-by-cell basis in a whole blood sample; U.S. Patent No. 5,990,689 describes and claims a device for detecting and locating anomalies in the electromagnetic protection of a system; U.S. Patent No. 5,984,175 describes and claims a device for detecting and identifying wearable user identification units; U.S. Patent No. 3,980,986 ("Oil well survey tool"), describes and claims a tool for finding the position of a drill bit working at the bottom of a borehole.

As noted above, the nucleic acid probes of the present invention are useful in constructing microarrays; the microarrays, in turn, are products of manufacture that are useful for measuring and for surveying gene expression.

When included on a microarray, each HTPL nucleic acid probe makes the microarray specifically useful for detecting that portion of the HTPL gene included within the probe, thus imparting upon the microarray device the ability to detect a signal where, absent such probe, it would have reported no signal. This utility makes each individual probe on such microarray akin to an antenna, circuit, firmware or software element included in an electronic apparatus, where the antenna, circuit, firmware or software element imparts upon the apparatus the ability newly and additionally to detect signal in a portion of the radio-frequency spectrum where previously it could not; such devices are known to have specific, substantial, and credible utility.

Changes in the level of expression need not be observed for the measurement of expression to have utility.

For example, where gene expression analysis is used to assess toxicity of chemical agents on cells, the failure of the agent to change a gene's expression level is evidence that the drug likely does not affect the pathway of which the gene's expressed protein is a part. Analogously, where gene expression analysis is used to assess side effects of pharmacologic agents - whether in lead compound discovery or in subsequent screening of lead compound derivatives - the inability of the agent to alter a gene's expression level is evidence that the drug does not affect the pathway of which the gene's expressed protein is a part.

WO 99/58720, incorporated herein by reference in its entirety, provides methods for quantifying the relatedness of a first and second gene expression profile and for ordering the relatedness of a plurality of gene expression profiles, without regard to the identity or function of the genes whose expression is used in the calculation.

Gene expression analysis, including gene expression analysis by microarray hybridization, is, of course, principally a laboratory-based art. Devices and apparatus used principally in laboratories to facilitate laboratory research are well-established to possess specific, substantial, and credible utility. For example, U.S. Patent No. 6,001,233 describes and claims a gel electrophoresis apparatus having a cam-activated clamp; for example, U.S. Patent No. 6,051,831 describes and claims a high mass detector for use in time-of-flight mass spectrometers; for example, U.S. Patent NO. 5,824,269 describes and claims a flow cytometer-as is well known, few gel electrophoresis apparatuses, TOF-MS devices, or flow cytometers are sold for consumer use.

Indeed, and in particular, nucleic acid microarrays, as devices intended for laboratory use in measuring gene expression, are well-established to have specific, substantial and credible utility. Thus, the microarrays of the present invention have at least the specific, substantial and credible utilities of the microarrays claimed as devices and articles of manufacture in the following U.S. patents, the disclosures of each of which is incorporated herein by reference: U.S. Patent Nos. 5,445,934 ("Array of oligonucleotides on a solid substrate"); 5,744,305 ("Arrays of materials attached to a substrate"); and 6,004,752 ("Solid support with attached molecules").

Genome-derived single exon probes and genome-derived single exon probe microarrays have the additional utility, *inter alia,* of permitting high-throughput detection of splice variants of the nucleic acids of the present invention, as further described in copending and commonly owned U.S. Patent application no. 09/632,366, filed August 3, 2000, the disclosure of which is incorporated herein by reference in its entirety.

The isolated nucleic acids of the present invention can also be used to prime synthesis of nucleic acid, for purpose of either analysis or isolation, using mRNA, cDNA, or genomic DNA as template.

For use as primers, at least 17 contiguous nucleotides of the isolated nucleic acids of the present invention will be used. Often, at least 18, 19, or 20 contiguous nucleotides of the nucleic acids of the present invention will be used, and on occasion at least 20, 22, 24, or 25 contiguous nucleotides of the nucleic acids of the present invention will be used, and even 30 nucleotides or more of the nucleic acids of the present invention can be used to prime specific synthesis.

The nucleic acid primers of the present invention can be used, for example, to prime first strand cDNA synthesis on an mRNA template.

Such primer extension can be done directly to analyze the message. Alternatively, synthesis on an mRNA template can be done to produce first strand cDNA. The first strand cDNA can thereafter be used, *inter alia,* directly as a single-stranded probe, as above-described, as a template for sequencing - permitting identification of alterations, including deletions, insertions, and substitutions, both normal allelic variants and mutations associated with abnormal phenotypes- or as a template, either for second strand cDNA synthesis *(e.g.,* as an antecedent to insertion into a cloning or expression vector), or for amplification.

The nucleic acid primers of the present invention can also be used, for example, to prime single base extension (SBE) for SNP detection *(see, e.g.,* U.S. Pat. No. 6,004,744, the disclosure of which is incorporated herein by reference in its entirety).

As another example, the nucleic acid primers of the present invention can be used to prime amplification of HTPL nucleic acids, using transcript-derived or genomic DNA as template.

Primer-directed amplification methods are now well-established in the art. Methods for performing the polymerase chain reaction (PCR) are compiled, *inter alia,* in McPherson, PCR (Basics: From Background to Bench), Springer Verlag (2000) (ISBN: 0387916008); Innis *et al.* (eds.), PCR Applications: Protocols for Functional Genomics, Academic Press (1999) (ISBN: 0123721857); Gelfand *et al.* (eds.), PCR Strategies, Academic Press (1998) (ISBN: 0123721822); Newton *et al.,* PCR, Springer-Verlag New York (1997) (ISBN: 0387915060); Burke (ed.), PCR: Essential Techniques, John Wiley & Son Ltd (1996) (ISBN: 047195697X); White (ed.), PCR Cloning Protocols: From Molecular Cloning to Genetic Engineering, Vol. 67, Humana Press (1996) (ISBN: 0896033430); McPherson et al. (eds.), PCR 2: A Practical Approach, Oxford University Press, Inc. (1995) (ISBN: 0199634254), the disclosures of which are incorporated herein by reference in their entireties. Methods for performing RT-PCR are collected, *e.g.,* in Siebert *et al*. (eds.), Gene Cloning and Analysis by RT-PCR, Eaton Publishing Company/Bio Techniques Books Division, 1998 (ISBN: 1881299147); Siebert (ed.), PCR Technique:RT-PCR, Eaton Publishing Company/BioTechniques Books (1995) (ISBN:1881299139), the disclosure of which is incorporated herein by reference in its entirety.

Isothermal amplification approaches, such as rolling circle amplification, are also now well-described. *See, e.g.,* Schweitzer *et al., Curr. Opin. Biotechnol.* 12(1):21-7 (2001); U.S. Patent Nos. 6,235,502, 6,221,603, 6,210,884, 6,183,960, 5,854,033, 5,714,320, 5,648,245, and international patent publications WO 97/19193 and WO 00/15779, the disclosures of which are incorporated herein by reference in their entireties. Rolling circle amplification can be combined with other techniques to facilitate SNP detection. See, *e.g.,* Lizardi *et al., Mature Genet.* 19(3):225-32 (1998).

As further described below, nucleic acids of the present invention, inserted into vectors that flank the nucleic acid insert with a phage promoter, such as T7, T3, or SP6 promoter, can be used to drive *in vitro* expression of RNA complementary to either strand of the nucleic acid of the present invention. The RNA can be used, *inter alia,* as a single-stranded probe, in cDNA-mRNA subtraction, or for *in vitro* translation.

As will be further discussed herein below, nucleic acids of the present invention that encode HTPL protein or portions thereof can be used, *inter alia,* to express the HTPL proteins or protein fragments, either alone, or as part of fusion proteins.

Expression can be from genomic nucleic acids of the present invention, or from transcript-derived nucleic acids of the present invention.

Where protein expression is effected from genomic DNA, expression will typically be effected in eukaryotic, typically mammalian, cells capable of splicing introns from the initial RNA transcript. Expression can be driven from episomal vectors, such as EBV-based vectors, or can be effected from genomic DNA integrated into a host cell chromosome. As will be more fully described below, where expression is from transcript-derived (or otherwise intron-less) nucleic acids of the present invention, expression can be effected in wide variety of prokaryotic or eukaryotic cells.

Expressed *in vitro,* the protein, protein fragment, or protein fusion can thereafter be isolated, to be used, *inter alia,* as a standard in immunoassays specific for the proteins, or protein isoforms, of the present invention; to be used as a therapeutic agent, *e.g.,* to be administered as passive replacement therapy in individuals deficient in the proteins of the present invention, or to be administered as a vaccine; to be used for *in vitro* production of specific antibody, the antibody thereafter to be used, *e.g.,* as an analytical reagent for detection and quantitation of the proteins of the present invention or to be used as an immunotherapeutic agent.

The isolated nucleic acids of the present invention can also be used to drive *in vivo* expression of the proteins of the present invention. *In vivo* expression can be driven from a vector - typically a viral vector, often a vector based upon a replication incompetent retrovirus, an adenovirus, or an adeno-associated virus (AAV) - for purpose of gene therapy. *In vivo* expression can also be driven from signals endogenous to the nucleic acid or from a vector, often a plasmid vector, such as pVAX1 (Invitrogen, Carlsbad CA, USA), for purpose of "naked" nucleic acid vaccination, as further described in U.S. Pat. Nos. 5,589,466; 5,679,647; 5,804,566; 5,830,877; 5,843,913; 5,880,104; 5,958,891; 5,985,847; 6,017,897; 6,110,898; 6,204,250, the disclosures of which are incorporated herein by reference in their entireties.

The nucleic acids of the present invention can also be used for antisense inhibition of transcription or translation. See Phillips (ed.), Antisense Technology, Part B, Methods in Enzymology Vol. 314, Academic Press, Inc. (1999) (ISBN: 012182215X); Phillips (ed.), Antisense Technology, Part A, Methods in Enzymology Vol. 313, Academic Press, Inc. (1999) (ISBN: 0121822141); Hartmann *et al.* (eds.), Manual of Antisense Methodology (Perspectives in Antisense Science), Kluwer Law International (1999) (ISBN:079238539X); Stein *et al.* (eds.), Applied Antisense Oligonucleotide Technology, Wiley-Liss (cover (1998) (ISBN: 0471172790); Agrawal *et al.* (eds.), Antisense Research and Application, Springer-Verlag New York, Inc. (1998) (ISBN: 3540638334); Lichtenstein *et al*. (eds.), Antisense Technology: A Practical Approach, Vol. 185, Oxford University Press, INC. (1998) (ISBN: 0199635838); Gibson (ed.), Antisense and Ribozyme Methodology: Laboratory Companion, Chapman & Hall (1997) (ISBN: 3826100794); Chadwick *et al.* (eds.), Oligonucleotides as Therapeutic Agents - Symposium No. 209, John Wiley & Son Ltd (1997) (ISBN: 0471972797), the disclosures of which are incorporated herein by reference in their entireties.

Nucleic acids of the present invention, particularly cDNAs of the present invention, that encode full-length human HTPL protein isoforms, have additional, well-recognized, immediate, real world utility as commercial products of manufacture suitable for sale.

For example, Invitrogen Corp. (Carlsbad, CA, USA), through its Research Genetics subsidiary, sells full length human cDNAs cloned into one of a selection of expression vectors as GeneStorm® expression-ready clones; utility is specific for the gene, since each gene is capable of being ordered separately and has a distinct catalogue number, and utility is substantial, each clone selling for $650.00 US. Similarly, Incyte Genomics (Palo Alto, CA, USA) sells clones from public and proprietary sources in multi-well plates or individual tubes.

Nucleic acids of the present invention that include genomic regions encoding the human HTPL protein, or portions thereof, have yet further utilities.

For example, genomic nucleic acids of the present invention can be used as amplification substrates, e.g. for preparation of genome-derived single exon probes of the present invention, as described above and in copending and commonly-owned U.S. patent application nos. 09/864,761, filed May 23, 2001, 09/774,203, filed January 29, 2001, and 09/632,366, filed August 3, 2000, the disclosures of which are incorporated herein by reference in their entireties.

As another example, genomic nucleic acids of the present invention can be integrated non-homologously into the genome of somatic cells, *e.g.* CHO cells, COS cells, or 293 cells, with or without amplification of the insertional locus, in order, *e.g.,* to create stable cell lines capable of producing the proteins of the present invention.

As another example, more fully described herein below, genomic nucleic acids of the present invention can be integrated nonhomologously into embryonic stem (ES) cells to create transgenic non-human animals capable of producing the proteins of the present invention.

Genomic nucleic acids of the present invention can also be used to target homologous recombination to the human HTPL locus. *See, e.g.,* U.S. Patent Nos. 6,187,305; 6,204,061; 5,631,153; 5,627,059; 5,487,992; 5,464,764; 5,614,396; 5,527,695 and 6,063,630; and Kmiec *et al.* (eds.), Gene Targeting Protocols, Vol. 133, Humana Press (2000) (ISBN: 0896033600); Joyner (ed.), Gene Targeting: A Practical Approach, Oxford University Press, Inc. (2000) (ISBN: 0199637938); Sedivy *et al.,* Gene Targeting, Oxford University Press (1998) (ISBN: 071677013X); Tymms *et al.* (eds.), Gene Knockout Protocols, Humana Press (2000) (ISBN: 0896035727); Mak *et al.* (eds.), The Gene Knockout FactsBook, Vol. 2, Academic Press, Inc. (1998) (ISBN: 0124660444); Torres *et al.,* Laboratory Protocols for Conditional Gene Targeting, Oxford University Press (1997) (ISBN: 019963677X); Vega (ed.), Gene Targeting, CRC Press, LLC (1994) (ISBN: 084938950X), the disclosures of which are incorporated herein by reference in their entireties.

Where the genomic region includes transcription regulatory elements, homologous recombination can be used to alter the expression of HTPL, both for purpose of *in vitro* production of HTPL protein from human cells, and for purpose of gene therapy. *See, e.g.,* U.S. Pat. Nos. 5,981,214, 6,048,524; 5,272,071.

Fragments of the nucleic acids of the present invention smaller than those typically used for homologous recombination can also be used for targeted gene correction or alteration, possibly by cellular mechanisms different from those engaged during homologous recombination.

For example, partially duplexed RNA/DNA chimeras have been shown to have utility in targeted gene correction, U.S. Pat. Nos. 5,945,339, 5,888,983, 5,871,984, 5,795,972, 5,780,296, 5,760,012, 5,756,325, 5,731,181, the disclosures of which are incorporated herein by reference in their entireties. So too have small oligonucleotides fused to triplexing domains have been shown to have utility in targeted gene correction, Culver *et al.,* "Correction of chromosomal point mutations in human cells with bifunctional oligonucleotides," *Nature Biotechnol*. 17(10):989-93 (1999), as have oligonucleotides having modified terminal bases or modified terminal internucleoside bonds, Gamper *et al., Nucl. Acids Res.* 28(21):4332-9 (2000), the disclosures of which are incorporated herein by reference.

The isolated nucleic acids of the present invention can also be used to provide the initial substrate for recombinant engineering of HTPL protein variants having desired phenotypic improvements. Such engineering includes, for example, site-directed mutagenesis, random mutagenesis with subsequent functional screening, and more elegant schemes for recombinant evolution of proteins, as are described, *inter alia,* in U.S. Pat. Nos. 6,180,406; 6,165,793; 6,117,679; and 6,096,548, the disclosures of which are incorporated herein by reference in their entireties.

Nucleic acids of the present invention can be obtained by using the labeled probes of the present invention to probe nucleic acid samples, such as genomic libraries, cDNA libraries, and mRNA samples, by standard techniques. Nucleic acids of the present invention can also be obtained by amplification, using the nucleic acid primers of the present invention, as further demonstrated in Example 1, herein below. Nucleic acids of the present invention of fewer than about 100 nt can also be synthesized chemically, typically by solid phase synthesis using commercially available automated synthesizers.

### "Full Length" HTPL Nucleic Acids

In a first series of nucleic acid embodiments, the invention provides isolated nucleic acids that encode the entirety of the HTPL protein. As discussed above, the "full-length" nucleic acids of the present invention can be used, *inter alia,* to express full length HTPL protein. The full-length nucleic acids can also be used as nucleic acid probes; used as probes, the isolated nucleic acids of these embodiments will hybridize to HTPL.

In a first such embodiment, the invention provides an isolated nucleic acid comprising (i) the nucleotide sequence of SEQ ID NO: 1, or (ii) the complement of (i). SEQ ID NO: 1 presents the entire cDNA of HTPL-L, including the 5' untranslated (UT) region and 3' UT.

In a second embodiment, the invention provides an isolated nucleic acid comprising (i) the nucleotide sequence of SEQ ID NO: 2, (ii) a degenerate variant of the nucleotide sequence of SEQ ID NO: 2, or (iii) the complement of (i) or (ii). SEQ ID NO: 2 presents the open reading frame (ORF) from SEQ ID NO: 1.

In a third embodiment, the invention provides an isolated nucleic acid comprising (i) a nucleotide sequence that encodes a polypeptide with the amino acid sequence of SEQ ID NO: 3 or (ii) the complement of a nucleotide sequence that encodes a polypeptide with the amino acid sequence of SEQ ID NO: 3. SEQ ID NO: 3 provides the amino acid sequence of HTPL-L.

In a fourth embodiment, the invention provides an isolated nucleic acid having a nucleotide sequence that (i) encodes a polypeptide having the sequence of SEQ ID NO: 3, (ii) encodes a polypeptide having the sequence of SEQ ID NO: 3 with conservative amino acid substitutions, or (iii) that is the complement of (i) or (ii), where SEQ ID NO: 3 provides the amino acid sequence of HTPL-L.

In another such embodiment, the invention provides an isolated nucleic acid comprising (i) the nucleotide sequence of SEQ ID NO: 4, or (ii) the complement of (i). SEQ ID NO: 4 presents the entire cDNA of HTPL-S, including the 5' untranslated (UT) region and 3' UT.

In another embodiment, the invention provides an isolated nucleic acid comprising (i) the nucleotide sequence of SEQ ID NO: 5, (ii) a degenerate variant of the nucleotide sequence of SEQ ID NO: 5, or (iii) the complement of (i) or (ii). SEQ ID NO: 5 presents the open reading frame (ORF) from SEQ ID NO: 4.

In yet another embodiment, the invention provides an isolated nucleic acid comprising (i) a nucleotide sequence that encodes a polypeptide with the amino acid sequence of SEQ ID NO: 6 or (ii) the complement of a nucleotide sequence that encodes a polypeptide with the amino acid sequence of SEQ ID NO: 6. SEQ ID NO: 6 provides the amino acid sequence of HTPL-S.

In still another embodiment, the invention provides an isolated nucleic acid having a nucleotide sequence that (i) encodes a polypeptide having the sequence of SEQ ID NO: 6, (ii) encodes a polypeptide having the sequence of SEQ ID NO: 6 with conservative amino acid substitutions, or (iii) that is the complement of (i) or (ii), where SEQ ID NO: 6 provides the amino acid sequence of HTPL-S.

### Selected Partial Nucleic Acids

In a second series of nucleic acid embodiments, the invention provides isolated nucleic acids that encode select portions of HTPL. As will be further discussed herein below, these "partial" nucleic acids can be used, *inter alia,* to express specific portions of the HTPL. These "partial" nucleic acids can also be used, *inter alia,* as nucleic probes.

In a first such embodiment, the invention provides an isolated nucleic acid comprising (i) the nucleotide sequence of SEQ ID NO: 7, (ii) a degenerate variant of SEQ ID NO: 9, or (iii) the complement of (i) or (ii), wherein the isolated nucleic acid is no more than about 100 kb in length, typically no more than about 75 kb in length, more typically no more than about 50 kb length. SEQ ID NO: 7 encodes a novel portion of HTPL-L. Often, the isolated nucleic acids of this embodiment are no more than about 25 kb in length, often no more than about 15 kb in length, and frequently no more than about 10 kb in length.

In another embodiment, the invention provides an isolated nucleic acid comprising (i) a nucleotide sequence that encodes SEQ ID NO: 10 or (ii) the complement of a nucleotide sequence that encodes SEQ ID NO: 10, wherein the isolated nucleic acid is no more than about 100 kb in length, typically no more than about 75 kb in length, frequently no more than about 50 kb in length. SEQ ID NO: 10 is the amino acid sequence encoded by the portion of HTPL-L not found in any EST fragments. Often, the isolated nucleic acids of this embodiment are no more than about 25 kb in length, often no more than about 15 kb in length, and frequently no more than about 10 kb in length.

In another embodiment, the invention provides an isolated nucleic acid comprising (i) a nucleotide sequence that encodes SEQ ID NO: 10, (ii) a nucleotide sequence that encodes SEQ ID NO: 10 with conservative substitutions, or (iii) the complement of (i) or (ii), wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

In another such embodiment, the invention provides an isolated nucleic acid comprising (i) the nucleotide sequence of SEQ ID NO: 11, (ii) a degenerate variant of SEQ ID NO: 12, or (iii) the complement of (i) or (ii), wherein the isolated nucleic acid is no more than about 100 kb in length, typically no more than about 75 kb in length, more typically no more than about 50 kb length. SEQ ID NO: 11 encodes a novel portion of HTPL-L. Often, the isolated nucleic acids of this embodiment are no more than about 25 kb in length, often no more than about 15 kb in length, and frequently no more than about 10 kb in length.

In another embodiment, the invention provides an isolated nucleic acid comprising (i) a nucleotide sequence that encodes SEQ ID NO: 14 or (ii) the complement of a nucleotide sequence that encodes SEQ ID NO: 14, wherein the isolated nucleic acid is no more than about 100 kb in length, typically no more than about 75 kb in length, frequently no more than about 50 kb in length. SEQ ID NO: 14 is the amino acid sequence encoded by the portion of HTPL-L not found in any EST fragments. Often, the isolated nucleic acids of this embodiment are no more than about 25 kb in length, often no more than about 15 kb in length, and frequently no more than about 10 kb in length.

In another embodiment, the invention provides an isolated nucleic acid comprising (i) a nucleotide sequence that encodes SEQ ID NO: 14, (ii) a nucleotide sequence that encodes SEQ ID NO: 14 with conservative substitutions, or (iii) the complement of (i) or (ii), wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

In another such embodiment, the invention provides an isolated nucleic acid comprising (i) the nucleotide sequence of SEQ ID NO: 4796, (ii) a degenerate variant of SEQ ID NO: 4798, or (iii) the complement of (i) or (ii), wherein the isolated nucleic acid is no more than about 100 kb in length, typically no more than about 75 kb in length, more typically no more than about 50 kb length. SEQ ID NO: 4796 encodes a novel portion of HTPL-S. Often, the isolated nucleic acids of this embodiment are no more than about 25 kb in length, often no more than about 15 kb in length, and frequently no more than about 10 kb in length.

In another embodiment, the invention provides an isolated nucleic acid comprising (i) a nucleotide sequence that encodes SEQ ID NO: 4799 or (ii) the complement of a nucleotide sequence that encodes SEQ ID NO: 4799, wherein the isolated nucleic acid is no more than about 100 kb in length, typically no more than about 75 kb in length, frequently no more than about 50 kb in length. SEQ ID NO: 4799 is the amino acid sequence encoded by the portion of HTPL-S not found in any EST fragments. Often, the isolated nucleic acids of this embodiment are no more than about 25 kb in length, often no more than about 15 kb in length, and frequently no more than about 10 kb in length.

In another embodiment, the invention provides an isolated nucleic acid comprising (i) a nucleotide sequence that encodes SEQ ID NO: 4799, (ii) a nucleotide sequence that encodes SEQ ID NO: 4799 with conservative substitutions, or (iii) the complement of (i) or (ii), wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

In another such embodiment, the invention provides an isolated nucleic acid comprising (i) the nucleotide sequence of SEQ ID NO: 4800, or (ii) the complement of (i), wherein the isolated nucleic acid is no more than about 100 kb in length, typically no more than about 75 kb in length, more typically no more than about 50 kb length. SEQ ID NO: 4800 encodes a novel portion of HTPL-S. Often, the isolated nucleic acids of this embodiment are no more than about 25 kb in length, often no more than about 15 kb in length, and frequently no more than about 10 kb in length.

### Cross-Hybridizing Nucleic Acids

In another series of nucleic acid embodiments, the invention provides isolated nucleic acids that hybridize to various of the HTPL nucleic acids of the present invention. These cross-hybridizing nucleic acids can be used, *inter alia,* as probes for, and to drive expression of, proteins that are related to HTPL of the present invention as further isoforms, homologues, paralogues, or orthologues.

In a first embodiment, the invention provides an isolated nucleic acid comprising a sequence that hybridizes under high stringency conditions to a probe the nucleotide sequence of which consists of at least 17 nt, 18, 19, 20, 21, 22, 23, 24, 25, 30, 40, or 50 nt of SEQ ID NO: 7 or the complement of SEQ ID NO: 7, wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

In a further embodiment, the invention provides an isolated nucleic acid comprising a sequence that hybridizes under moderate stringency conditions to a probe the nucleotide sequence of which consists of at least 17 nt, 18, 19, 20, 21, 22, 23, 24, 25, 30, 40, or 50 nt of SEQ ID NO: 7 or the complement of SEQ ID NO: 7, wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

In a further embodiment, the invention provides an isolated nucleic acid comprising a sequence that hybridizes under high stringency conditions to a hybridization probe the nucleotide sequence of which (i) encodes a polypeptide having the sequence of SEQ ID NO: 10, (ii) encodes a polypeptide having the sequence of SEQ ID NO: 10 with conservative amino acid substitutions, or (iii) is the complement of (i) or (ii), wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

In another embodiment, the invention provides an isolated nucleic acid comprising a sequence that hybridizes under high stringency conditions to a probe the nucleotide sequence of which consists of at least 17 nt, 18, 19, 20, 21, 22, 23, 24, 25, 30, 40, or 50 nt of SEQ ID NO: 11 or the complement of SEQ ID NO: 11, wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.
In a further embodiment, the invention provides an isolated nucleic acid comprising a sequence that hybridizes under moderate stringency conditions to a probe the nucleotide sequence of which consists of at least 17 nt, 18, 19, 20, 21, 22, 23, 24, 25, 30, 40, or 50 nt of SEQ ID NO: 11 or the complement of SEQ ID NO: 11, wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

In a further embodiment, the invention provides an isolated nucleic acid comprising a sequence that hybridizes under high stringency conditions to a hybridization probe the nucleotide sequence of which (i) encodes a polypeptide having the sequence of SEQ ID NO: 14, (ii) encodes a polypeptide having the sequence of SEQ ID NO: 14 with conservative amino acid substitutions, or (iii) is the complement of (i) or (ii), wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

In another embodiment, the invention provides an isolated nucleic acid comprising a sequence that hybridizes under high stringency conditions to a probe the nucleotide sequence of which consists of at least 17 nt, 18, 19, 20, 21, 22, 23, 24, 25, 30, 40, or 50 nt of SEQ ID NO: 4796 or the complement of SEQ ID NO: 4796, wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.
In a further embodiment, the invention provides an isolated nucleic acid comprising a sequence that hybridizes under moderate stringency conditions to a probe the nucleotide sequence of which consists of at least 17 nt, 18, 19, 20, 21, 22, 23, 24, 25, 30, 40, or 50 nt of SEQ ID NO: 4796 or the complement of SEQ ID NO: 4796, wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

In a further embodiment, the invention provides an isolated nucleic acid comprising a sequence that hybridizes under high stringency conditions to a hybridization probe the nucleotide sequence of which (i) encodes a polypeptide having the sequence of SEQ ID NO: 4799, (ii) encodes a polypeptide having the sequence of SEQ ID NO: 4799 with conservative amino acid substitutions, or (iii) is the complement of (i) or (ii), wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

In another embodiment, the invention provides an isolated nucleic acid comprising a sequence that hybridizes under high stringency conditions to a probe the nucleotide sequence of which consists of at least 17 nt, 18, 19, 20, 21, 22, 23, 24, 25, 30, 40, or 50 nt of SEQ ID NO: 4800 or the complement of SEQ ID NO: 4800, wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.
In a further embodiment, the invention provides an isolated nucleic acid comprising a sequence that hybridizes under moderate stringency conditions to a probe the nucleotide sequence of which consists of at least 17 nt, 18, 19, 20, 21, 22, 23, 24, 25, 30, 40, or 50 nt of SEQ ID NO: 4800 or the complement of SEQ ID NO: 4800, wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

### Particularly Useful Nucleic Acids

Particularly useful among the above-described nucleic acids are those that are expressed, or the complement of which are expressed, in testis, as well as adrenal, adult and fetal liver, bone marrow, brain, kidney, lung, placenta, prostate, skeletal muscle and colon.

Also particularly useful among the above-described nucleic acids are those that encode, or the complement of which encode, a membrane protein functioning in the hedgehog signaling pathway, mutations of which cause male sterility or cancer.

Other particularly useful embodiments of the nucleic acids above-described are those that encode, or the complement of which encode, a polypeptide having a Patched motif, or having a Sterol-sensing domain, or having at least one transmembrane domain, especially those having a plurality of transmembrane domains, particularly those having twelve (for HTPL-L) or seven (for HTPL-S) transmembrane domains in tandem.

### Nucleic Acid Fragments

In another series of nucleic acid embodiments, the invention provides fragments of various of the isolated nucleic acids of the present invention which prove useful, *inter alia,* as nucleic acid probes, as amplification primers, and to direct expression or synthesis of epitopic or immunogenic protein fragments.

In a first embodiment, the invention provides an isolated nucleic acid comprising at least 17 nucleotides, 18 nucleotides, 20 nucleotides, 24 nucleotides, or 25 nucleotides of (i) SEQ ID NO: 7, (ii) a degenerate variant of SEQ ID NO: 9, or (iii) the complement of (i) or (ii), wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

The invention also provides an isolated nucleic acid comprising (i) a nucleotide sequence that encodes a peptide of at least 8 contiguous amino acids of SEQ ID NO: 10, (ii) a nucleotide sequence that encodes a peptide of at least 15 contiguous amino acids of SEQ ID NO: 10, or (iii) the complement of (i) or (ii), wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

The invention also provides an isolated nucleic acid comprising a nucleotide sequence that encodes (i) a polypeptide having the sequence of at least 8 contiguous amino acids of SEQ ID NO: 10 with conservative amino acid substitutions, (ii) a polypeptide having the sequence of at least 15 contiguous amino acids of SEQ ID NO: 10 with conservative amino acid substitutions, (iii) a polypeptide having the sequence of at least 8 contiguous amino acids of SEQ ID NO: 10 with moderately conservative substitutions, (iv) a polypeptide having the sequence of at least 15 congiuous amino acids of SEQ ID NO: 10 with moderately conservative substitutions, or (v) the complement of any of (i) - (iv), wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

In another embodiment, the invention provides an isolated nucleic acid comprising at least 17 nucleotides, 18 nucleotides, 20 nucleotides, 24 nucleotides, or 25 nucleotides of (i) SEQ ID NO: 11, (ii) a degenerate variant of SEQ ID NO: 12, or (iii) the complement of (i) or (ii), wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

The invention also provides an isolated nucleic acid comprising (i) a nucleotide sequence that encodes a peptide of at least 8 contiguous amino acids of SEQ ID NO: 14, (ii) a nucleotide sequence that encodes a peptide of at least 15 contiguous amino acids of SEQ ID NO: 14, or (iii) the complement of (i) or (ii), wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

The invention also provides an isolated nucleic acid comprising a nucleotide sequence that encodes (i) a polypeptide having the sequence of at least 8 contiguous amino acids of SEQ ID NO: 14 with conservative amino acid substitutions, (ii) a polypeptide having the sequence of at least 15 contiguous amino acids of SEQ ID NO: 14 with conservative amino acid substitutions, (iii) a polypeptide having the sequence of at least 8 contiguous amino acids of SEQ ID NO: 14 with moderately conservative substitutions, (iv) a polypeptide having the sequence of at least 15 contiguous amino acids of SEQ ID NO: 14 with moderately conservative substitutions, or (v) the complement of any of (i) - (iv), wherein the isolated nucleic acid is no more than about 100 kb in length, typically no more than about 75 kb in length, more typically no more than about 50 kb in length.

Often, the isolated nucleic acids of this embodiment are no more than about 25 kb in length, often no more than about 15 kb in length, and frequently no more than about 10 kb in length.

In another embodiment, the invention provides an isolated nucleic acid comprising at least 17 nucleotides, 18 nucleotides, 20 nucleotides, 24 nucleotides, or 25 nucleotides of (i) SEQ ID NO: 4796, (ii) a degenerate variant of SEQ ID NO: 4798, or (iii) the complement of (i) or (ii), wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

The invention also provides an isolated nucleic acid comprising (i) a nucleotide sequence that encodes a peptide of at least 8 contiguous amino acids of SEQ ID NO: 4799, (ii) a nucleotide sequence that encodes a peptide of at least 15 contiguous amino acids of SEQ ID NO: 4799, or (iii) the complement of (i) or (ii), wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

The invention also provides an isolated nucleic acid comprising a nucleotide sequence that encodes (i) a polypeptide having the sequence of at least 8 contiguous amino acids of SEQ ID NO: 4799 with conservative amino acid substitutions, (ii) a polypeptide having the sequence of at least 15 contiguous amino acids of SEQ ID NO: 4799 with conservative amino acid substitutions, (iii) a polypeptide having the sequence of at least 8 contiguous amino acids of SEQ ID NO: 4799 with moderately conservative substitutions, (iv) a polypeptide having the sequence of at least 15 contiguous amino acids of SEQ ID NO: 4799 with moderately conservative substitutions, or (v) the complement of any of (i) - (iv), wherein the isolated nucleic acid is no more than about 100 kb in length, typically no more than about 75 kb in length, more typically no more than about 50 kb in length.

Often, the isolated nucleic acids of this embodiment are no more than about 25 kb in length, often no more than about 15 kb in length, and frequently no more than about 10 kb in length.

In another embodiment, the invention provides an isolated nucleic acid comprising at least 17 nucleotides, 18 nucleotides, 20 nucleotides, 24 nucleotides, or 25 nucleotides of (i) SEQ ID NO: 4800, or (ii) the complement of (i), wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

### Single Exon Probes

The invention further provides genome-derived single exon probes having portions of no more than one exon of the HTPL gene. As further described in commonly owned and copending U.S. patent application serial no. 09/632,366, filed August 3, 2000 ("Methods and Apparatus for High Throughput Detection and Characterization of alternatively Spliced Genes"), the disclosure of which is incorporated herein by reference in its entirety, such single exon probes have particular utility in identifying and characterizing splice variants. In particular, such single exon probes are useful for identifying and discriminating the expression of distinct isoforms of HTPL.

In a first embodiment, the invention provides an isolated nucleic acid comprising a nucleotide sequence of no more than one portion of SEQ ID NOs: 15 - 18 or the complement of SEQ ID NOs: 15 - 18, wherein the portion comprises at least 17 contiguous nucleotides, 18 contiguous nucleotides, 20 contiguous nucleotides, 24 contiguous nucleotides, 25 contiguous nucleotides, or 50 contiguous nucleotides of any one of SEQ ID NOs: 15 - 18, or their complement. In a further embodiment, the exonic portion comprises the entirety of the referenced SEQ ID NO: or its complement.

In other embodiments, the invention provides isolated single exon probes having the nucleotide sequence of any one of SEQ ID NOs: 19 - 22.

### Transcription Control Nucleic Acids

In another aspect, the present invention provides genome-derived isolated nucleic acids that include nucleic acid sequence elements that control transcription of the HTPL gene. These nucleic acids can be used, *inter alia,* to drive expression of heterologous coding regions in recombinant constructs, thus conferring upon such heterologous coding regions the expression pattern of the native HTPL gene. These nucleic acids can also be used, conversely, to target heterologous transcription control elements to the HTPL genomic locus, altering the expression pattern of the HTPL gene itself.

In a first such embodiment, the invention provides an isolated nucleic acid comprising the nucleotide sequence of SEQ ID NO: 23 or its complement, wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

In another embodiment, the invention provides an isolated nucleic acid comprising at least 17, 18, 20, 24, or 25 nucleotides of the sequence of SEQ ID NO: 23 or its complement, wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

### VECTORS AND HOST CELLS

In another aspect, the present invention provides vectors that comprise one or more of the isolated nucleic acids of the present invention, and host cells in which such vectors have been introduced.

The vectors can be used, *inker alia*, for propagating the nucleic acids of the present invention in host cells (cloning vectors), for shuttling the nucleic acids of the present invention between host cells derived from disparate organisms (shuttle vectors), for inserting the nucleic acids of the present invention into host cell chromosomes (insertion vectors), for expressing sense or antisense RNA transcripts of the nucleic acids of the present invention *in vitro* or within a host cell, and for expressing polypeptides encoded by the nucleic acids of the present invention, alone or as fusions to heterologous polypeptides. Vectors of the present invention will often be suitable for several such uses.

Vectors are by now well-known in the art, and are described, *inter alia,* in Jones *et al.* (eds.), Vectors: Cloning Applications : Essential Techniques (Essential Techniques Series), John Wiley & Son Ltd 1998 (ISBN: 047196266X); Jones *et al.* (eds.), Vectors: Expression Systems : Essential Techniques (Essential Techniques Series), John Wiley & Son Ltd, 1998 (ISBN:0471962678); Gacesa *et al.,* Vectors: Essential Data, John Wiley & Sons, 1995 (ISBN: 0471948411); Cid-Arregui (eds.), Viral Vectors: Basic Science and Gene Therapy, Eaton Publishing Co., 2000 (ISBN: 188129935X); Sambrook *et al.,* Molecular Cloning: A Laboratory Manual (3^{rd} ed.), Cold Spring Harbor Laboratory Press, 2001 (ISBN: 0879695773); Ausubel *et al.* (eds.), Short Protocols in Molecular Biology: A Compendium of Methods from Current Protocols in Molecular Biology (4^{th} ed.), John Wiley & Sons, 1999 (ISBN: 047132938X), the disclosures of which are incorporated herein by reference in their entireties. Furthermore, an enormous variety of vectors are available commercially. Use of existing vectors and modifications thereof being well within the skill in the art, only basic features need be described here.

Typically, vectors are derived from virus, plasmid, prokaryotic or eukaryotic chromosomal elements, or some combination thereof, and include at least one origin of replication, at least one site for insertion of heterologous nucleic acid, typically in the form of a polylinker with multiple, tightly clustered, single cutting restriction sites, and at least one selectable marker, although some integrative vectors will lack an origin that is functional in the host to be chromosomally modified, and some vectors will lack selectable markers. Vectors of the present invention will further include at least one nucleic acid of the present invention inserted into the vector in at least one location.

Where present, the origin of replication and selectable markers are chosen based upon the desired host cell or host cells; the host cells, in turn, are selected based upon the desired application.

For example, prokaryotic cells, typically E. *coli,* are typically chosen for cloning. In such case, vector replication is predicated on the replication strategies of coliform-infecting phage - such as phage lambda, M13, T7, T3 and P1 - or on the replication origin of autonomously replicating episomes, notably the ColE1 plasmid and later derivatives, including pBR322 and the pUC series plasmids. Where *E. coli* is used as host, selectable markers are, analogously, chosen for selectivity in gram negative bacteria: *e.g.,* typical markers confer resistance to antibiotics, such as ampicillin, tetracycline, chloramphenicol, kanamycin, streptomycin, zeocin; auxotrophic markers can also be used.

As another example, yeast cells, typically *S. cerevisiae,* are chosen, *inter alia,* for eukaryotic genetic studies, due to the ease of targeting genetic changes by homologous recombination and to the ready ability to complement genetic defects using recombinantly expressed proteins, for identification of interacting protein components, *e.g.* through use of a two-hybrid system, and for protein expression. Vectors of the present invention for use in yeast will typically, but not invariably, contain an origin of replication suitable for use in yeast and a selectable marker that is functional in yeast.

Integrative YIp vectors do not replicate autonomously, but integrate, typically in single copy, into the yeast genome at low frequencies and thus replicate as part of the host cell chromosome; these vectors lack an origin of replication that is functional in yeast, although they typically have at least one origin of replication suitable for propagation of the vector in bacterial cells. YEp vectors, in contrast, replicate episomally and autonomously due to presence of the yeast 2 micron plasmid origin (2 µm ori). The YCp yeast centromere plasmid vectors are autonomously replicating vectors containing centromere sequences, CEN, and autonomously replicating sequences, ARS; the ARS sequences are believed to correspond to the natural replication origins of yeast chromosomes. YACs are based on yeast linear plasmids, denoted YLp, containing homologous or heterologous DNA sequences that function as telomeres (TEL) *in vivo,* as well as containing yeast ARS (origins of replication) and CEN (centromeres) segments.

Selectable markers in yeast vectors include a variety of auxotrophic markers, the most common of which are (in *Saccharomyces cerevisiae)* URA3, HIS3, LEU2, TRP1 and LYS2, which complement specific auxotrophic mutations, such as ura3-52, his3-D1, leu2-D1, trpl-D1 and lys2-201. The URA3 and LYS2 yeast genes further permit negative selection based on specific inhibitors, 5-fluoro-orotic acid (FOA) and α-aminoadipic acid (αAA), respectively, that prevent growth of the prototrophic strains but allows growth of the ura3 and lys2 mutants, respectively. Other selectable markers confer resistance to, *e.g.,* zeocin.

As yet another example, insect cells are often chosen for high efficiency protein expression. Where the host cells are from *Spodoptera frugiperda - e.g.,* Sf9 and Sf21 cell lines, and expresSF™ cells (Protein Sciences Corp., Meriden, CT, USA) - the vector replicative strategy is typically based upon the baculovirus life cycle. Typically, baculovirus transfer vectors are used to replace the wild-type AcMNPV polyhedrin gene with a heterologous gene of interest. Sequences that flank the polyhedrin gene in the wild-type genome are positioned 5' and 3' of the expression cassette on the transfer vectors. Following cotransfection with AcMNPV DNA, a homologous recombination event occurs between these sequences resulting in a recombinant virus carrying the gene of interest and the polyhedrin or p10 promoter. Selection can be based upon visual screening for lacZ fusion activity.

As yet another example, mammalian cells are often chosen for expression of proteins intended as pharmaceutical agents, and are also chosen as host cells for screening of potential agonist and antagonists of a protein or a physiological pathway.

Where mammalian cells are chosen as host cells, vectors intended for autonomous extrachromosomal replication will typically include a viral origin, such as the SV40 origin (for replication in cell lines expressing the large T-antigen, such as COS1 and COS7 cells), the papillomavirus origin, or the EBV origin for long term episomal replication (for use, *e.g.,* in 293-EBNA cells, which constitutively express the EBV EBNA-1 gene product and adenovirus E1A). Vectors intended for integration, and thus replication as part of the mammalian chromosome, can, but need not, include an origin of replication functional in mammalian cells, such as the SV40 origin. Vectors based upon viruses, such as adenovirus, adeno-associated virus, vaccinia virus, and various mammalian retroviruses, will typically replicate according to the viral replicative strategy.

Selectable markers for use in mammalian cells include resistance to neomycin (G418), blasticidin, hygromycin and to zeocin, and selection based upon the purine salvage pathway using HAT medium.

Plant cells can also be used for expression, with the vector replicon typically derived from a plant virus *(e.g.,* cauliflower mosaic virus, CaMV; tobacco mosaic virus, TMV) and selectable markers chosen for suitability in plants.

For propagation of nucleic acids of the present invention that are larger than can readily be accomodated in vectors derived from plasmids or virus, the invention further provides artificial chromosomes - BACs, YACs, PACs, and HACs - that comprise HTPL nucleic acids, often genomic nucleic acids.

The BAC system is based on the well-characterized *E. coli* F-factor, a low copy plasmid that exists in a supercoiled circular form in host cells. The structural features of the F-factor allow stable maintenance of individual human DNA clones as well as easy manipulation of the cloned DNA. *See* Shizuya *et al., Keio J. Med.* 50(1):26-30 (2001); Shizuya *et al., Proc. Natl. Acad. Sci. USA* 89(18):8794-7 (1992).

YACs are based on yeast linear plasmids, denoted YLp, containing homologous or heterologous DNA sequences that function as telomeres (TEL) *in vivo,* as well as containing yeast ARS (origins of replication) and CEN (centromeres) segments.

HACs are human artifical chromosomes. Kuroiwa *et al., Nature Biotechnol*. 18(10):1086-90 (2000); Henning *et al., Proc. Natl. Acad. Sci. USA* 96(2):592-7 (1999); Harrington *et al., Nature Genet.* 15(4):345-55 (1997). In one version, long synthetic arrays of alpha satellite DNA are combined with telomeric DNA and genomic DNA to generate linear microchromosomes that are mitotically and cytogenetically stable in the absence of selection.

PACs are P1-derived artificial chromosomes. Sternberg, *Proc. Natl. Acad. Sci. USA* 87(1):103-7 (1990); Sternberg *et al., New Biol.* 2(2):151-62 (1990); Pierce *et al., Proc. Natl Acad. Sci. USA* 89(6):2056-60 (1992).

Vectors of the present invention will also often include elements that permit *in vitro* transcription of RNA from the inserted heterologous nucleic acid. Such vectors typically include a phage promoter, such as that from T7, T3, or SP6, flanking the nucleic acid insert. Often two different such promoters flank the inserted nucleic acid, permitting separate *in vitro* production of both sense and antisense strands.

Expression vectors of the present invention - that is, those vectors that will drive expression of polypeptides from the inserted heterologous nucleic acid - will often include a variety of other genetic elements operatively linked to the protein-encoding heterologous nucleic acid insert, typically genetic elements that drive transcription, such as promoters and enhancer elements, those that facilitate RNA processing, such as transcription termination and/or polyadenylation signals, and those that facilitate translation, such as ribosomal consensus sequences.

For example; vectors for expressing proteins of the present invention in prokaryotic cells, typically *E. coli,* will include a promoter, often a phage promoter, such as phage lambda pL promoter, the trc promoter, a hybrid derived from the trp and lac promoters, the bacteriophage T7 promoter (in *E. coli* cells engineered to express the T7 polymerase), or the araBAD operon. Often, such prokaryotic expression vectors will further include transcription terminators, such as the aspA terminator, and elements that facilitate translation, such as a consensus ribosome binding site and translation termination codon, Schomer *et al., Proc. Natl. Acad. Sci. USA* 83:8506-8510 (1986).

As another example, vectors for expressing proteins of the present invention in yeast cells, typically *S. cerevisiae,* will include a yeast promoter, such as the CYC1 promoter, the GAL1 promoter, ADH1 promoter, or the GPD promoter, and will typically have elements that facilitate transcription termination, such as the transcription termination signals from the CYC1 or ADH1 gene.

As another example, vectors for expressing proteins of the present invention in mammalian cells will include a promoter active in mammalian cells. Such promoters are often drawn from mammalian viruses - such as the enhancer-promoter sequences from the immediate early gene of the human cytomegalovirus (CMV), the enhancer-promoter sequences from the Rous sarcoma virus long terminal repeat (RSV LTR), and the enhancer-promoter from SV40. Often, expression is enhanced by incorporation of polyadenylation sites, such as the late SV40 polyadenylation site and the polyadenylation signal and transcription termination sequences from the bovine growth hormone (BGH) gene, and ribosome binding sites. Furthermore, vectors can include introns, such as intron II of rabbit β-globin gene and the SV40 splice elements.

Vector-drive protein expression can be constitutive or inducible.

Inducible vectors include either naturally inducible promoters, such as the trc promoter, which is regulated by the lac operon, and the pL promoter, which is regulated by tryptophan, the MMTV-LTR promoter, which is inducible by dexamethasone, or can contain synthetic promoters and/or additional elements that confer inducible control on adjacent promoters. Examples of inducible synthetic promoters are the hybrid Plac/ara-1 promoter and the PLtetO-1 promoter. The PltetO-1 promoter takes advantage of the high expression levels from the PL promoter of phage lambda, but replaces the lambda repressor sites with two copies of operator 2 of the Tn10 tetracycline resistance operon, causing this promoter to be tightly repressed by the Tet repressor protein and induced in response to tetracycline (Tc) and Tc derivatives such as anhydrotetracycline.

As another example of inducible elements, hormone response elements, such as the glucocorticoid response element (GRE) and the estrogen response element (ERE), can confer hormone inducibility where vectors are used for expression in cells having the respective hormone receptors. To reduce background levels of expression, elements responsive to ecdysone, an insect hormone, can be used instead, with coexpression of the ecdysone receptor.

Expression vectors can be designed to fuse the expressed polypeptide to small protein tags that facilitate purification and/or visualization.

For example, proteins of the present invention can be expressed with a polyhistidine tag that facilitates purification of the fusion protein by immobilized metal affinity chromatography, for example using NiNTA resin (Qiagen Inc., Valencia, CA, USA) or TALON™ resin (cobalt immobilized affinity chromatography medium, Clontech Labs, Palo Alto, CA, USA). As another example, the fusion protein can include a chitin-binding tag and self-excising intein, permitting chitin-based purification with self-removal of the fused tag (IMPACT™ system, New England Biolabs, Inc., Beverley, MA, USA). Alternatively, the fusion protein can include a calmodulin-binding peptide tag, permitting purification by calmodulin affinity resin (Stratagene, La Jolla, CA, USA), or a specifically excisable fragment of the biotin carboxylase carrier protein, permitting purification of *in vivo* biotinylated protein using an avidin resin and subsequent tag removal (Promega, Madison, WI, USA). As another useful alternative, the proteins of the present invention can be expressed as a fusion to glutathione-S-transferase, the affinity and specificity of binding to glutathione permitting purification using glutathione affinity resins, such as Glutathione-Superflow Resin (Clontech Laboratories, Palo Alto, CA, USA), with subsequent elution with free glutathione.

Other tags include, for example, the Xpress epitope, detectable by anti-Xpress antibody (Invitrogen, Carlsbad, CA, USA), a myc tag, detectable by anti-myc tag antibody, the V5 epitope, detectable by anti-V5 antibody (Invitrogen, Carlsbad, CA, USA), FLAG® epitope, detectable by anti-FLAG® antibody (Stratagene, La Jolla, CA, USA), and the HA epitope.

For secretion of expressed proteins, vectors can include appropriate sequences that encode secretion signals, such as leader peptides. For example, the pSecTag2 vectors (Invitrogen, Carlsbad, CA, USA) are 5.2 kb mammalian expression vectors that carry the secretion signal from the V-J2-C region of the mouse Ig kappa-chain for efficient secretion of recombinant proteins from a variety of mammalian cell lines.

Expression vectors can also be designed to fuse proteins encoded by the heterologous nucleic acid insert to polypeptides larger than purification and/or identification tags.

Useful protein fusions include those that permit display of the encoded protein on the surface of a phage or cell, fusions to intrinsically fluorescent proteins, such as those that have a green fluorescent protein (GFP)-like chromophore, fusions to the IgG Fc region, and fusions for use in two hybrid systems.

Vectors for phage display fuse the encoded polypeptide to, *e.g.*, the gene III protein (pIII) or gene VIII protein (pVIII) for display on the surface of filamentous phage, such as M13. *See* Barbas *et al.,* Phage Display: A Laboratory Manual, Cold Spring Harbor Laboratory Press (2001) (ISBN 0-87969-546-3); Kay *et al*. (eds.), Phage Display of Peptides and Proteins: A Laboratory Manual, San Diego: Academic Press, Inc., 1996; Abelson *et al*. (eds.), Combinatorial Chemistry, Methods in Enzymology vol. 267, Academic Press (May 1996).

Vectors for yeast display, *e.g*. the pYD1 yeast display vector (Invitrogen, Carlsbad, CA, USA), use the α-agglutinin yeast adhesion receptor to display recombinant protein on the surface of *S. cerevisiae.* Vectors for mammalian display, *e.g.,* the pDisplay™ vector (Invitrogen, Carlsbad, CA, USA), target recombinant proteins using an N-terminal cell surface targeting signal and a C-terminal transmembrane anchoring domain of platelet derived growth factor receptor.

A wide variety of vectors now exist that fuse proteins encoded by heterologous nucleic acids to the chromophore of the substrate-independent, intrinsically fluorescent green fluorescent protein from *Aequorea victoria* ("GFP") and its variants. These proteins are intrinsically fluorescent: the GFP-like chromophore is entirely encoded by its amino acid sequence and can fluoresce without requirement for cofactor or substrate.

Structurally, the GFP-like chromophore comprises an 11-stranded β-barrel (β-can) with a central α-helix, the central α-helix having a conjugated π-resonance system that includes two aromatic ring systems and the bridge between them. The π-resonance system is created by autocatalytic cyclization among amino acids; cyclization proceeds through an imidazolinone intermediate, with subsequent dehydrogenation by molecular oxygen at the Cα-Cβ bond of a participating tyrosine.

The GFP-like chromophore can be selected from GFP-like chromophores found in naturally occurring proteins, such as *A. victoria* GFP (GenBank accession number AAA27721), *Renilla reniformis* GFP, FP583 (GenBank accession no. AF168419) (DsRed), FP593 (AF272711), FP483 (AF168420), FP484 (AF168424), FP595 (AF246709), FP486 (AF168421), FP538 (AF168423), and FP506 (AF168422), and need include only so much of the native protein as is needed to retain the chromophore's intrinsic fluorescence. Methods for determining the minimal domain required for fluorescence are known in the art. Li *et al*., "Deletions of the *Aequorea victoria* Green Fluorescent Protein Define the Minimal Domain Required for Fluorescence," *J. Biol. Chem.* 272:28545-28549 (1997).

Alternatively, the GFP-like chromophore can be selected from GFP-like chromophores modified from those found in nature. Typically, such modifications are made to improve recombinant production in heterologous expression systems (with or without change in protein sequence), to alter the excitation and/or emission spectra of the native protein, to facilitate purification, to facilitate or as a consequence of cloning, or are a fortuitous consequence of research investigation.

The methods for engineering such modified GFP-like chromophores and testing them for fluorescence activity, both alone and as part of protein fusions, are well-known in the art. Early results of these efforts are reviewed in Heim *et al., Curr. Biol.* 6:178-182 (1996), incorporated herein by reference in its entirety; a more recent review, with tabulation of useful mutations, is found in Palm *et al.,* "Spectral Variants of Green Fluorescent Protein," in Green Fluorescent Proteins, Conn (ed.), *Methods Enzymol*. vol. 302, pp. 378 - 394 (1999), incorporated herein by reference in its entirety. A variety of such modified chromophores are now commercially available and can readily be used in the fusion proteins of the present invention.

For example, EGFP ("enhanced GFP"), Cormack *et al., Gene* 173:33-38 (1996); U.S. Pat. Nos. 6,090,919 and 5,804,387, is a red-shifted, human codon-optimized variant of GFP that has been engineered for brighter fluorescence, higher expression in mammalian cells, and for an excitation spectrum optimized for use in flow cytometers. EGFP can usefully contribute a GFP-like chromophore to the fusion proteins of the present invention. A variety of EGFP vectors, both plasmid and viral, are available commercially (Clontech Labs, Palo Alto, CA, USA), including vectors for bacterial expression, vectors for N-terminal protein fusion expression, vectors for expression of C-terminal protein fusions, and for bicistronic expression.

Toward the other end of the emission spectrum, EBFP ("enhanced blue fluorescent protein") and BFP2 contain four amino acid substitutions that shift the emission from green to blue, enhance the brightness of fluorescence and improve solubility of the protein, Heim *et al., Curr. Biol.* 6:178-182 (1996); Cormack *et al.,* Gene 173:33-38 (1996). EBFP is optimized for expression in mammalian cells whereas BFP2, which retains the original jellyfish codons, can be expressed in bacteria; as is further discussed below, the host cell of production does not affect the utility of the resulting fusion protein. The GFP-like chromophores from EBFP and BFP2 can usefully be included in the fusion proteins of the present invention, and vectors containing these blue-shifted variants are available from Clontech Labs (Palo Alto, CA, USA).

Analogously, EYFP ("enhanced yellow fluorescent protein"), also available from Clontech Labs, contains four amino acid substitutions, different from EBFP, Ormö *et al., Science* 273:1392-1395 (1996), that shift the emission from green to yellowish-green. Citrine, an improved yellow fluorescent protein mutant, is described in Heikal *et al., Proc. Natl. Acad. Sci. USA* 97:11996-12001 (2000). ECFP ("enhanced cyan fluorescent protein") (Clontech Labs, Palo Alto, CA, USA) contains six amino acid substitutions, one of which shifts the emission spectrum from green to cyan. Heim *et al., Curr. Biol.* 6:178-182 (1996); Miyawaki *et al.,* Nature 388:882-887 (1997). The GFP-like chromophore of each of these GFP variants can usefully be included in the fusion proteins of the present invention.

The GFP-like chromophore can also be drawn from other modified GFPs, including those described in U.S. Pat. Nos. 6,124,128; 6,096,865; 6,090,919; 6,066,476; 6,054,321; 6,027,881; 5,968,750; 5,874,304; 5,804,387; 5,777,079; 5,741,668; and 5,625,048, the disclosures of which are incorporated herein by reference in their entireties. See also Conn (ed.), Green Fluorescent Protein, *Methods in Enzymol*. Vol. 302, pp 378-394 (1999), incorporated herein by reference in its entirety. A variety of such modified chromophores are now commercially available and can readily be used in the fusion proteins of the present invention.

Fusions to the IgG Fc region increase serum half life of protein pharmaceutical products through interaction with the FcRn receptor (also denominated the FcRp receptor and the Brambell receptor, FcRb), further described in international patent application nos. WO 97/43316, WO 97/34631, WO 96/32478, WO 96/18412.

For long-term, high-yield recombinant production of the proteins, protein fusions, and protein fragments of the present invention, stable expression is particularly useful.

Stable expression is readily achieved by integration into the host cell genome of vectors having selectable markers, followed by selection for integrants.

For example, the pUB6/V5-His A, B, and C vectors (Invitrogen, Carlsbad, CA, USA) are designed for high-level stable expression of heterologous proteins in a wide range of mammalian tissue types and cell lines. pUB6/V5-His uses the promoter/enhancer sequence from the human ubiquitin C gene to drive expression of recombinant proteins: expression levels in 293, CHO, and NIH3T3 cells are comparable to levels from the CMV and human EF-1a promoters. The bsd gene permits rapid selection of stably transfected mammalian cells with the potent antibiotic blasticidin.

Replication incompetent retroviral vectors, typically derived from Moloney murine leukemia virus, prove particularly useful for creating stable transfectants having integrated provirus. The highly efficient transduction machinery of retroviruses, coupled with the availability of a variety of packaging cell lines - such as RetroPack™ PT 67, EcoPack2™-293, AmphoPack-293, GP2-293 cell lines (all available from Clontech Laboratories, Palo Alto, CA, USA) - allow a wide host range to be infected with high efficiency; varying the multiplicity of infection readily adjusts the copy number of the integrated provirus. Retroviral vectors are available with a variety of selectable markers, such as resistance to neomycin, hygromycin, and puromycin, permitting ready selection of stable integrants.

The present invention further includes host cells comprising the vectors of the present invention, either present episomally within the cell or integrated, in whole or in part, into the host cell chromosome.

Among other considerations, some of which are described above, a host cell strain may be chosen for its ability to process the expressed protein in the desired fashion. Such post-translational modifications of the polypeptide include, but are not limited to, acetylation, carboxylation, glycosylation, phosphorylation, lipidation, and acylation, and it is an aspect of the present invention to provide HTPL proteins with such post-translational modifications.

As noted earlier, host cells can be prokaryotic or eukaryotic. Representative examples of appropriate host cells include, but are not limited to, bacterial cells, such as E. *coli, Caulobacter crescentus, Streptomyces* species, and *Salmonella typhimurium;* yeast cells, such as *Saccharomyces cerevisiae, Schizosaccharomyces pombe, Pichia pastoris, Pichia methanolica;* insect cell lines, such as those from *Spodoptera frugiperda - e.g.,* Sf9 and Sf21 cell lines, and expresSF™ cells (Protein Sciences Corp., Meriden, CT, USA) - *Drosophila* S2 cells, and *Trichoplusia ni* High Five® Cells (Invitrogen, Carlsbad, CA, USA); and mammalian cells. Typical mammalian cells include COS1 and COS7 cells, chinese hamster ovary (CHO) cells, NIH 3T3 cells, 293 cells, HEPG2 cells, HeLa cells, L cells, murine ES cell lines (*e.g.*, from strains 129/SV, C57/BL6, DBA-1, 129/SVJ), K562, Jurkat cells, and BW5147. Other mammalian cell lines are well known and readily available from the American Type Culture Collection (ATCC) (Manassas, VA, USA) and the National Institute of General medical Sciences (NIGMS) Human Genetic Cell Repository at the Coriell Cell Repositories (Camden, NJ, USA).

Methods for introducing the vectors and nucleic acids of the present invention into the host cells are well known in the art; the choice of technique will depend primarily upon the specific vector to be introduced and the host cell chosen.

For example, phage lambda vectors will typically be packaged using a packaging extract (*e.g.*, Gigapack® packaging extract, Stratagene, La Jolla, CA, USA), and the packaged virus used to infect *E. coli.* Plasmid vectors will typically be introduced into chemically competent or electrocompetent bacterial cells.

*E. coli* cells can be rendered chemically competent by treatment, *e.g.*, with CaCl₂, or a solution of Mg²⁺, Mn²⁺, Ca²⁺, Rb⁺ or K⁺, dimethyl sulfoxide, dithiothreitol, and hexamine cobalt (III), Hanahan, *J. Mol. Biol.* 166(4):557-80 (1983), and vectors introduced by heat shock. A wide variety of chemically competent strains are also available commercially *(e.g.,* Epicurian Coli® XL10-Gold® Ultracompetent Cells (Stratagene, La Jolla, CA, USA); DH5α competent cells (Clontech Laboratories, Palo Alto, CA, USA); TOP10 Chemically Competent E. coli Kit (Invitrogen, Carlsbad, CA, USA)).

Bacterial cells can be rendered electrocompetent - that is, competent to take up exogenous DNA by electroporation - by various pre-pulse treatments; vectors are introduced by electroporation followed by subsequent outgrowth in selected media. An extensive series of protocols is provided online in Electroprotocols (BioRad, Richmond, CA, USA) (http://www.biorad.com/LifeScience/pdf/New_Gene_Pulser.pdf).

Vectors can be introduced into yeast cells by spheroplasting, treatment with lithium salts, electroporation, or protoplast fusion.

Spheroplasts are prepared by the action of hydrolytic enzymes - a snail-gut extract, usually denoted Glusulase, or Zymolyase, an enzyme from *Arthrobacter luteus -* to remove portions of the cell wall in the presence of osmotic stabilizers, typically 1 M sorbitol. DNA is added to the spheroplasts, and the mixture is co-precipitated with a solution of polyethylene glycol (PEG) and Ca²⁺. Subsequently, the cells are resuspended in a solution of sorbitol, mixed with molten agar and then layered on the surface of a selective plate containing sorbitol. For lithium-mediated transformation, yeast cells are treated with lithium acetate, which apparently permeabilizes the cell wall, DNA is added and the cells are co-precipitated with PEG. The cells are exposed to a brief heat shock, washed free of PEG and lithium acetate, and subsequently spread on plates containing ordinary selective medium. Increased frequencies of transformation are obtained by using specially-prepared single-stranded carrier DNA and certain organic solvents. Schiestl *et al., Curr. Genet.* 16(5-6):339-46 (1989). For electroporation, freshly-grown yeast cultures are typically washed, suspended in an osmotic protectant, such as sorbitol, mixed with DNA, and the cell suspension pulsed in an electroporation device. Subsequently, the cells are spread on the surface of plates containing selective media. Becker *et al., Methods Enzymol.* 194:182-7 (1991). The efficiency of transformation by electroporation can be increased over 100-fold by using PEG, single-stranded carrier DNA and cells that are in late log-phase of growth. Larger constructs, such as YACs, can be introduced by protoplast fusion.

Mammalian and insect cells can be directly infected by packaged viral vectors, or transfected by chemical or electrical means.

For chemical transfection, DNA can be coprecipitated with CaPO₄ or introduced using liposomal and nonliposomal lipid-based agents. Commercial kits are available for CaPO₄ transfection (CalPhos™ Mammalian Transfection Kit, Clontech Laboratories, Palo Alto, CA, USA), and lipid-mediated transfection can be practiced using commercial reagents, such as LIPOFECTAMINE™ 2000, LIPOFECTAMINE™ Reagent, CELLFECTIN® Reagent, and LIPOFECTIN® Reagent (Invitrogen, Carlsbad, CA, USA), DOTAP Liposomal Transfection Reagent, FuGENE 6, X-tremeGENE Q2, DOSPER, (Roche Molecular Biochemicals, Indianapolis, IN USA), Effectene™, PolyFect®, Superfect® (Qiagen, Inc., Valencia, CA, USA). Protocols for electroporating mammalian cells can be found online in Electroprotocols (Bio-Rad, Richmond, CA, USA) (http://www.bio-rad.com/LifeScience/pdf/New_Gene_Pulser.pdf). *See also,* Norton *et al.* (eds.), Gene Transfer Methods: Introducing DNA into Living Cells and Organisms, BioTechniques Books, Eaton Publishing Co. (2000) (ISBN 1-881299-34-1), incorporated herein by reference in its entirety.

Other transfection techniques include transfection by particle embardment. *See, e.g.,* Cheng *et al., Proc. Natl. Acad. Sci. USA* 90(10):4455-9 (1993); Yang *et al., Proc. Natl. Acad. Sci. USA* 87(24):9568-72 (1990).

### PROTEINS

In another aspect, the present invention provides HTPL proteins, various fragments thereof suitable for use as antigens (e.g., for epitope mapping) and for use as immunogens (e.g., for raising antibodies or as vaccines), fusions of HTPL polypeptides and fragments to heterologous polypeptides, and conjugates of the proteins, fragments, and fusions of the present invention to other moieties *(e.g.,* to carrier proteins, to fluorophores).

FIG. 3 and FIG. 4 presents the predicted amino acid sequences encoded by the HTPL cDNA clones. The amino acid sequence is further presented, respectively, in SEQ ID NO: 3 (full length amino acid coding sequence of human HTPL-L), and 6 (full length amino acid coding sequence of human HTPL-S).

Unless otherwise indicated, amino acid sequences of the proteins of the present invention were determined as a predicted translation from a nucleic acid sequence. Accordingly, any amino acid sequence presented herein may contain errors due to errors in the nucleic acid sequence, as described in detail above. Furthermore, single nucleotide polymorphisms (SNPs) occur frequently in eukaryotic genomes - more than 1.4 million SNPs have already identified in the human genome, International Human Genome Sequencing Consortium, *Nature* 409:860 - 921 (2001) - and the sequence determined from one individual of a species may differ from other allelic forms present within the population. Small deletions and insertions can often be found that does not alter the function of the protein.

Accordingly, it is an aspect of the present invention to provide proteins not only identical in sequence to those described with particularity herein, but also to provide isolated proteins at least about 65% identical in sequence to those described with particularity herein, typically at least about 70%, 75%, 80%, 85%, or 90% identical in sequence to those described with particularity herein, usefully at least about 91%, 92%, 93%, 94%, or 95% identical in sequence to those described with particularity herein, usefully at least about 96%, 97%, 98%, or 99% identical in sequence to those described with particularity herein, and, most conservatively, at least about 99.5%, 99.6%, 99.7%, 99.8% and 99.9% identical in sequence to those described with particularity herein. These sequence variants can be naturally occurring or can result from human intervention by way of random or directed mutagenesis.

For purposes herein, percent identity of two amino acid sequences is determined using the procedure of Tatiana *et al.,* "Blast 2 sequences - a new tool for comparing protein and nucleotide sequences", *FEMS Microbiol Lett.* 174:247-250 (1999), which procedure is effectuated by the computer program BLAST 2 SEQUENCES, available online at
http://www.ncbi.nlm.nih.gov/blast/bl2seq/bl2.html,
To assess percent identity of amino acid sequences, the BLASTP module of BLAST 2 SEQUENCES is used with default values of (i) BLOSUM62 matrix, Henikoff *et al., Proc. Natl. Acad. Sci USA* 89(22):10915-9 (1992); (ii) open gap 11 and extension gap 1 penalties; and (iii) gap x_dropoff 50 expect 10 word size 3 filter, and both sequences are entered in their entireties.

As is well known, amino acid substitutions occur frequently among natural allelic variants, with conservative substitutions often occasioning only *de minimis* change in protein function.

Accordingly, it is an aspect of the present invention to provide proteins not only identical in sequence to those described with particularity herein, but also to provide isolated proteins having the sequence of HTPL proteins, or portions thereof, with conservative amino acid substitutions. It is a further aspect to provide isolated proteins having the sequence of HTPL proteins, and portions thereof, with moderately conservative amino acid substitutions. These conservatively-substituted and moderately conservatively-substituted variants can be naturally occurring or can result from human intervention.

Although there are a variety of metrics for calling conservative amino acid substitutions, based primarily on either observed changes among evolutionarily related proteins or on predicted chemical similarity, for purposes herein a conservative replacement is any change having a positive value in the PAM250 log-likelihood matrix reproduced herein below (see Gonnet *et al., Science* 256(5062):1443-5 (1992)):

| | **A** | **R** | **N** | **D** | **C** | **Q** | **E** | **G** | **H** | **I** | **L** | **K** | **M** | **F** | **P** | **S** | **T** | **W** | **Y** | **V** |
|---|---|---|---|---|---|---|---|---|---|---|---|---|---|---|---|---|---|---|---|---|
| **A** | 2 | -1 | 0 | 0 | 0 | 0 | 0 | 0 | -1 | -1 | -1 | 0 | -1 | -2 | 0 | 1 | 1 | -4 | -2 | 0 |
| **R** | -1 | 5 | 0 | 0 | -2 | 2 | 0 | -1 | 1 | -2 | -2 | 3 | -2 | -3 | -1 | 0 | 0 | -2 | -2 | -2 |
| **N** | 0 | 0 | 4 | 2 | -2 | 1 | 1 | 0 | 1 | -3 | -3 | 1 | -2 | -3 | -1 | 1 | 0 | -4 | -1 | -2 |
| **D** | 0 | 0 | 2 | 5 | -3 | 1 | 3 | 0 | 0 | -4 | -4 | 0 | -3 | -4 | -1 | 0 | 0 | -5 | -3 | -3 |
| **C** | 0 | -2 | -2 | -3 | 12 | -2 | -3 | -2 | -1 | -1 | -2 | -3 | -1 | -1 | -3 | 0 | 0 | -1 | 0 | 0 |
| **Q** | 0 | 2 | 1 | 1 | -2 | 3 | 2 | -1 | 1 | -2 | -2 | 2 | -1 | -3 | 0 | 0 | 0 | -3 | -2 | -2 |
| **E** | 0 | 0 | 1 | 3 | -3 | 2 | 4 | -1 | 0 | -3 | -3 | 1 | -2 | -4 | 0 | 0 | 0 | -4 | -3 | -2 |
| **G** | 0 | -1 | 0 | 0 | -2 | -1 | -1 | 7 | -1 | -4 | -4 | -1 | -4 | -5 | -2 | 0 | -1 | -4 | -4 | -3 |
| **H** | -1 | 1 | 1 | 0 | -1 | 1 | 0 | -1 | 6 | -2 | -2 | 1 | -1 | 0 | -1 | 0 | 0 | -1 | 2 | -2 |
| **I** | -1 | -2 | -3 | -4 | -1 | -2 | -3 | -4 | -2 | 4 | 3 | -2 | 2 | 1 | -3 | -2 | -1 | -2 | -1 | 3 |
| **L** | -1 | -2 | -3 | -4 | -2 | -2 | -3 | -4 | -2 | 3 | 4 | -2 | 3 | 2 | -2 | -2 | -1 | -1 | 0 | 2 |
| **K** | 0 | 3 | 1 | 0 | -3 | 2 | 1 | -1 | 1 | -2 | -2 | 3 | -1 | -3 | -1 | 0 | 0 | -4 | -2 | -2 |
| **M** | -1 | -2 | -2 | -3 | -1 | -1 | -2 | -4 | -1 | 2 | 3 | -1 | 4 | 2 | -2 | -1 | -1 | -1 | 0 | 2 |
| **F** | -2 | -3 | -3 | -4 | -1 | -3 | -4 | -5 | 0 | 1 | 2 | -3 | 2 | 7 | -4 | -3 | -2 | 4 | 5 | 0 |
| **P** | 0 | -1 | -1 | -1 | -3 | 0 | 0 | -2 | -1 | -3 | -2 | -1 | -2 | -4 | 8 | 0 | 0 | -5 | -3 | -2 |
| **S** | 1 | 0 | 1 | 0 | 0 | 0 | 0 | 0 | 0 | -2 | -2 | 0 | -1 | -3 | 0 | 2 | 2 | -3 | -2 | -1 |
| **T** | 1 | 0 | 0 | 0 | 0 | 0 | 0 | -1 | 0 | -1 | -1 | 0 | -1 | -2 | 0 | 2 | 2 | -4 | -2 | 0 |
| **W** | -4 | -2 | -4 | -5 | -1 | -3 | -4 | -4 | -1 | -2 | -1 | -4 | -1 | 4 | -5 | -3 | -4 | 14 | 4 | -3 |
| **Y** | -2 | -2 | -1 | -3 | 0 | -2 | -3 | -4 | 2 | -1 | 0 | -2 | 0 | 5 | -3 | -2 | -2 | 4 | 8 | -1 |
| **V** | 0 | -2 | -2 | -3 | 0 | -2 | -2 | -3 | -2 | 3 | 2 | -2 | 2 | 0 | -2 | -1 | 0 | -3 | -1 | 3 |

For purposes herein, a "moderately conservative" replacement is any change having a nonnegative value in the PAM250 log-likelihood matrix reproduced herein above.

As is also well known in the art, relatedness of proteins can also be characterized using a functional test, the ability of the encoding nucleic acids to base-pair to one another at defined hybridization stringencies.

It is, therefore, another aspect of the invention to provide isolated proteins not only identical in sequence to those described with particularity herein, but also to provide isolated proteins ("hybridization related proteins") that are encoded by nucleic acids that hybridize under high stringency conditions (as defined herein above) to all or to a portion of various of the isolated nucleic acids of the present invention ("reference nucleic acids"). It is a further aspect of the invention to provide isolated proteins ("hybridization related proteins") that are encoded by nucleic acids that hybridize under moderate stringency conditions (as defined herein above) to all or to a portion of various of the isolated nucleic acids of the present invention ("reference nucleic acids").

The hybridization related proteins can be alternative isoforms, homologues, paralogues, and orthologues of the HTPL protein of the present invention. Particularly useful orthologues are those from other primate species, such as chimpanzee, rhesus macaque monkey, baboon, orangutan, and gorilla, from rodents, such as rats, mice, guinea pigs; from lagomorphs, such as rabbits, and from domestic livestock, such as cow, pig, sheep, horse, and goat.

Relatedness of proteins can also be characterized using a second functional test, the ability of a first protein competitively to inhibit the binding of a second protein to an antibody.

It is, therefore, another aspect of the present invention to provide isolated proteins not only identical in sequence to those described with particularity herein, but also to provide isolated proteins ("cross-reactive proteins") that competitively inhibit the binding of antibodies to all or to a portion of various of the isolated HTPL proteins of the present invention ("reference proteins"). Such competitive inhibition can readily be determined using immunoassays well known in the art.

Among the proteins of the present invention that differ in amino acid sequence from those described with particularity herein - including those that have deletions and insertions causing up to 10% non-identity, those having conservative or moderately conservative substitutions, hybridization related proteins, and cross-reactive proteins - those that substantially retain one or more HTPL activities are particularly useful. As described above, those activities include regulating male germ cell development and tumor suppression.

Residues that are tolerant of change while retaining function can be identified by altering the protein at known residues using methods known in the art, such as alanine scanning mutagenesis, Cunningham *et al., Science* 244(4908):1081-5 (1989); transposon linker scanning mutagenesis, Chen *et al., Gene* 263(1-2):39-48 (2001); combinations of homolog- and alanine-scanning mutagenesis, Jin *et al*., J. Mol. Biol. 226(3):851-65 (1992); combinatorial alanine scanning, Weiss *et al., Proc. Natl. Acad. Sci USA* 97(16):8950-4 (2000), followed by functional assay. Transposon linker scanning kits are available commercially (New England Biolabs, Beverly, MA, USA, catalog. no. E7-102S; EZ::TN™ In-Frame Linker Insertion Kit, catalogue no. EZI04KN, Epicentre Technologies Corporation, Madison, WI, USA).

As further described below, the isolated proteins of the present invention can readily be used as specific immunogens to raise antibodies that specifically recognize HTPL proteins, their isoforms, homologues, paralogues, and/or orthologues. The antibodies, in turn, can be used, *inter alia,* specifically to assay for the HTPL proteins of the present invention - e.g. by ELISA for detection of protein fluid samples, such as serum, by immunohistochemistry or laser scanning cytometry, for detection of protein in tissue samples, or by flow cytometry, for detection of intracellular protein in cell suspensions - for specific antibody-mediated isolation and/or purification of HTPL proteins, as for example by immunoprecipitation, and for use as specific agonists or antagonists of HTPL action.

The isolated proteins of the present invention are also immediately available for use as specific standards in assays used to determine the concentration and/or amount specifically of the HTPL proteins of the present invention. As is well known, ELISA kits for detection and quantitation of protein analytes typically include isolated and purified protein of known concentration for use as a measurement standard (*e.g.*, the human interferon-γ OptEIA kit, catalog no. 555142, Pharmingen, San Diego, CA, USA includes human recombinant gamma interferon, baculovirus produced).

The isolated proteins of the present invention are also immediately available for use as specific biomolecule capture probes for surface-enhanced laser desorption ionization (SELDI) detection of protein-protein interactions, WO 98/59362; WO 98/59360; WO 98/59361; and Merchant *et al., Electrophoresis* 21(6):1164-77 (2000), the disclosures of which are incorporated herein by reference in their entireties. Analogously, the isolated proteins of the present invention are also immediately available for use as specific biomolecule capture probes on BIACORE surface plasmon resonance probes. . *See* Weinberger *et al., Pharmacogenomics* 1(4):395-416 (2000); Malmqvist, *Biochem. Soc. Trans.* 27(2):335-40 (1999).

The isolated proteins of the present invention are also useful as a therapeutic supplement in patients having a specific deficiency in HTPL production.

In another aspect, the invention also provides fragments of various of the proteins of the present invention. The protein fragments are useful, *inter alia,* as antigenic and immunogenic fragments of HTPL.

By "fragments" of a protein is here intended isolated proteins (equally, polypeptides, peptides, oligopeptides), however obtained, that have an amino acid sequence identical to a portion of the reference amino acid sequence, which portion is at least 6 amino acids and less than the entirety of the reference nucleic acid. As so defined, "fragments" need not be obtained by physical fragmentation of the reference protein, although such provenance is not thereby precluded.

Fragments of at least 6 contiguous amino acids are useful in mapping B cell and T cell epitopes of the reference protein. *See, e.g.,* Geysen *et al.*, "Use of peptide synthesis to probe viral antigens for epitopes to a resolution of a single amino acid," *Proc. Natl. Acad. Sci. USA* 81:3998-4002 (1984) and U.S. Pat. Nos. 4,708,871 and 5,595,915, the disclosures of which are incorporated herein by reference in their entireties. Because the fragment need not itself be immunogenic, part of an immunodominant epitope, nor even recognized by native antibody, to be useful in such epitope mapping, all fragments of at least 6 amino acids of the proteins of the present invention have utility in such a study.

Fragments of at least 8 contiguous amino acids, often at least 15 contiguous amino acids, have utility as immunogens for raising antibodies that recognize the proteins of the present invention. See, *e.g.*, Lerner, "Tapping the immunological repertoire to produce antibodies of predetermined specificity," Nature 299:592-596 (1982); Shinnick *et al.,* "Synthetic peptide immunogens as vaccines," *Annu. Rev. Microbiol.* 37:425-46 (1983); Sutcliffe *et al.*, "Antibodies that react with predetermined sites on proteins," *Science* 219:660-6 (1983), the disclosures of which are incorporated herein by reference in their entireties. As further described in the above-cited references, virtually all 8-mers, conjugated to a carrier, such as a protein, prove immunogenic - that is, prove capable of eliciting antibody for the conjugated peptide; accordingly, all fragments of at least 8 amino acids of the proteins of the present invention have utility as immunogens.

Fragments of at least 8, 9, 10 or 12 contiguous amino acids are also useful as competitive inhibitors of binding of the entire protein, or a portion thereof, to antibodies (as in epitope mapping), and to natural binding partners, such as subunits in a multimeric complex or to receptors or ligands of the subject protein; this competitive inhibition permits identification and separation of molecules that bind specifically to the protein of interest, U.S. Pat. Nos. 5,539,084 and 5,783,674, incorporated herein by reference in their entireties.

The protein, or protein fragment, of the present invention is thus at least 6 amino acids in length, typically at least 8, 9, 10 or 12 amino acids in length, and often at least 15 amino acids in length. Often, the protein or the present invention, or fragment thereof, is at least 20 amino acids in length, even 25 amino acids, 30 amino acids, 35 amino acids, or 50 amino acids or more in length. Of course, larger fragments having at least 75 amino acids, 100 amino acids, or even 150 amino acids are also useful, and at times preferred.

The present invention further provides fusions of each of the proteins and protein fragments of the present invention to heterologous polypeptides.

By fusion is here intended that the protein or protein fragment of the present invention is linearly contiguous to the heterologous polypeptide in a peptide-bonded polymer of amino acids or amino acid analogues; by "heterologous polypeptide" is here intended a polypeptide that does not naturally occur in contiguity with the protein or protein fragment of the present invention. As so defined, the fusion can consist entirely of a plurality of fragments of the HTPL protein in altered arrangement; in such case, any of the HTPL fragments can be considered heterologous to the other HTPL fragments in the fusion protein. More typically, however, the heterologous polypeptide is not drawn from the HTPL protein itself.

The fusion proteins of the present invention will include at least one fragment of the protein of the present invention, which fragment is at least 6, typically at least 8, often at least 15, and usefully at least 16, 17, 18, 19, or 20 amino acids long. The fragment of the protein of the present to be included in the fusion can usefully be at least 25 amino acids long, at least 50 amino acids long, and can be at least 75, 100, or even 150 amino acids long. Fusions that include the entirety of the proteins of the present invention have particular utility.

The heterologous polypeptide included within the fusion protein of the present invention is at least 6 amino acids in length, often at least 8 amino acids in length, and usefully at least 15, 20, and 25 amino acids in length. Fusions that include larger polypeptides, such as the IgG Fc region, and even entire proteins (such as GFP chromophore-containing proteins), have particular utility.

As described above in the description of vectors and expression vectors of the present invention, which discussion is incorporated herein by reference in its entirety, heterologous polypeptides to be included in the fusion proteins of the present invention can usefully include those designed to facilitate purification and/or visualization of recombinantly-expressed proteins. Although purification tags can also be incorporated into fusions that are chemically synthesized, chemical synthesis typically provides sufficient purity that further purification by HPLC suffices; however, visualization tags as above described retain their utility even when the protein is produced by chemical synthesis, and when so included render the fusion proteins of the present invention useful as directly detectable markers of HTPL presence.

As also discussed above, heterologous polypeptides to be included in the fusion proteins of the present invention can usefully include those that facilitate secretion of recombinantly expressed proteins - into the periplasmic space or extracellular milieu for prokaryotic hosts, into the culture medium for eukaryotic cells - through incorporation of secretion signals and/or leader sequences.

Other useful protein fusions of the present invention include those that permit use of the protein of the present invention as bait in a yeast two-hybrid system. See Bartel *et al*. (eds.), The Yeast Two-Hybrid System, Oxford University Press (1997) (ISBN: 0195109384); Zhu *et al.,* Yeast Hybrid Technologies, Eaton Publishing, (2000) (ISBN 1-881299-15-5); Fields *et al., Trends Genet.* 10(8):286-92 (1994); Mendelsohn *et al., Curr. Opin. Biotechnol*. 5(5):482-6 (1994); Luban *et al., Curr. Opin. Biotechnol.* 6(1):59-64 (1995); Allen *et al.,* Trends Biochem. Sci. 20(12):511-6 (1995); Drees, *Curr. Opin. Chem. Biol.* 3(1):64-70 (1999); Topcu *et al., Pharm. Res.* 17(9):1049-55 (2000); Fashena et al., Gene 250(1-2):1-14 (2000), the disclosures of which are incorporated herein by reference in their entireties. Typically, such fusion is to either *E. coli* LexA or yeast GAL4 DNA binding domains. Related bait plasmids are available that express the bait fused to a nuclear localization signal.

Other useful protein fusions include those that permit display of the encoded protein on the surface of a phage or cell, fusions to intrinsically fluorescent proteins, such as green fluorescent protein (GFP), and fusions to the IgG Fc region, as described above, which discussion is incorporated here by reference in its entirety.

The proteins and protein fragments of the present invention can also usefully be fused to protein toxins, such as Pseudomonas exotoxin A, diphtheria toxin, shiga toxin A, anthrax toxin lethal factor, ricin, in order to effect ablation of cells that bind or take up the proteins of the present invention.

The isolated proteins, protein fragments, and protein fusions of the present invention can be composed of natural amino acids linked by native peptide bonds, or can contain any or all of nonnatural amino acid analogues, nonnative bonds, and post-synthetic (post translational) modifications, either throughout the length of the protein or localized to one or more portions thereof.

As is well known in the art, when the isolated protein is used, *e.g.*, for epitope mapping, the range of such nonnatural analogues, nonnative inter-residue bonds, or post-synthesis modifications will be limited to those that permit binding of the peptide to antibodies. When used as an immunogen for the preparation of antibodies in a non-human host, such as a mouse, the range of such nonnatural analogues, nonnative inter-residue bonds, or post-synthesis modifications will be limited to those that do not interfere with the immunogenicity of the protein. When the isolated protein is used as a therapeutic agent, such as a vaccine or for replacement therapy, the range of such changes will be limited to those that do not confer toxicity upon the isolated protein.

Non-natural amino acids can be incorporated during solid phase chemical synthesis or by recombinant techniques, although the former is typically more common.

Solid phase chemical synthesis of peptides is well established in the art. Procedures are described, inter alia, in Chan *et al*. (eds.), Fmoc Solid Phase Peptide Synthesis: A Practical Approach (Practical Approach Series), Oxford Univ. Press (March 2000) (ISBN: 0199637245); Jones, Amino Acid and Peptide Synthesis (Oxford Chemistry Primers, No 7), Oxford Univ. Press (August 1992) (ISBN: 0198556683); and Bodanszky, Principles of Peptide Synthesis (Springer Laboratory), Springer Verlag (December 1993) (ISBN: 0387564314), the disclosures of which are incorporated herein by reference in their entireties.

For example, D-enantiomers of natural amino acids can readily be incorporated during chemical peptide synthesis: peptides assembled from D-amino acids are more resistant to proteolytic attack; incorporation of D-enantiomers can also be used to confer specific three dimensional conformations on the peptide. Other amino acid analogues commonly added during chemical synthesis include ornithine, norleucine, phosphorylated amino acids (typically phosphoserine, phosphothreonine, phosphotyrosine), L-malonyltyrosine, a non-hydrolyzable analog of phosphotyrosine (Kole *et al., Biochem. Biophys. Res. Com.* 209:817-821 (1995)), and various halogenated phenylalanine derivatives.

Amino acid analogues having detectable labels are also usefully incorporated during synthesis to provide a labeled polypeptide.

Biotin, for example (indirectly detectable through interaction with avidin, streptavidin, neutravidin, captavidin, or anti-biotin antibody), can be added using biotinoyl--(9-fluorenylmethoxycarbonyl)-L-lysine (FMOC biocytin) (Molecular Probes, Eugene, OR, USA). (Biotin can also be added enzymatically by incorporation into a fusion protein of a *E. coli* BirA substrate peptide.)

The FMOC and tBOC derivatives of dabcyl-L-lysine (Molecular Probes, Inc., Eugene, OR, USA) can be used to incorporate the dabcyl chromophore at selected sites in the peptide sequence during synthesis. The aminonaphthalene derivative EDANS, the most common fluorophore for pairing with the dabcyl quencher in fluorescence resonance energy transfer (FRET) systems, can be introduced during automated synthesis of peptides by using EDANS--FMOC-L-glutamic acid or the corresponding tBOC derivative (both from Molecular Probes, Inc., Eugene, OR, USA). Tetramethylrhodamine fluorophores can be incorporated during automated FMOC synthesis of peptides using (FMOC)--TMR-L-lysine (Molecular Probes, Inc. Eugene, OR, USA).

Other useful amino acid analogues that can be incorporated during chemical synthesis include aspartic acid, glutamic acid, lysine, and tyrosine analogues having allyl side-chain protection (Applied Biosystems, Inc., Foster City, CA, USA); the allyl side chain permits synthesis of cyclic, branched-chain, sulfonated, glycosylated, and phosphorylated peptides.

A large number of other FMOC-protected non-natural amino acid analogues capable of incorporation during chemical synthesis are available commercially, including, *e.g.,* Fmoc-2-aminobicyclo[2.2.1]heptane-2-carboxylic acid, Fmoc-3-endo-aminobicyclo[2.2.1]heptane-2-endo-carboxylic acid, Fmoc-3-exo-aminobicyclo[2.2.1]heptane-2-exo-carboxylic acid, Fmoc-3-endo-amino-bicyclo[2.2.1]hept-5-ene-2-endo-carboxylic acid, Fmoc-3-exo-amino-bicyclo[2.2.1]hept-5-ene-2-exo-carboxylic acid, Fmoc-cis-2-amino-1-cyclohexanecarboxylic acid, Fmoc-trans-2-amino-1-cyclohexanecarboxylic acid, Fmoc-1-amino-1-cyclopentanecarboxylic acid, Fmoc-cis-2-amino-1-cyclopentanecarboxylic acid, Fmoc-1-amino-1-cyclopropanecarboxylic acid, Fmoc-D-2-amino-4-(ethylthio)butyric acid, Fmoc-L-2-amino-4-(ethylthio)butyric acid, Fmoc-L-buthionine, Fmoc-S-methyl-L-Cysteine, Fmoc-2-aminobenzoic acid (anthranillic acid), Fmoc-3-aminobenzoic acid, Fmoc-4-aminobenzoic acid, Fmoc-2-aminobenzophenone-2'-carboxylic acid, Fmoc-N-(4-aminobenzoyl)-b-alanine, Fmoc-2-amino-4,5-dimethoxybenzoic acid, Fmoc-4-aminohippuric acid, Fmoc-2-amino-3-hydroxybenzoic acid, Fmoc-2-amino-5-hydroxybenzoic acid, Fmoc-3-amino-4-hydroxybenzoic acid, Fmoc-4-amino-3-hydroxybenzoic acid, Fmoc-4-amino-2-hydroxybenzoic acid, Fmoc-5-amino-2-hydroxybenzoic acid, Fmoc-2-amino-3-methoxybenzoic acid, Fmoc-4-amino-3-methoxybenzoic acid, Fmoc-2-amino-3-methylbenzoic acid, Fmoc-2-amino-5-methylbenzoic acid, Fmoc-2-amino-6-methylbenzoic acid, Fmoc-3-amino-2-methylbenzoic acid, Fmoc-3-amino-4-methylbenzoic acid, Fmoc-4-amino-3-methylbenzoic acid, Fmoc-3-amino-2-naphtoic acid, Fmoc-D,L-3-amino-3-phenylpropionic acid, Fmoc-L-Methyldopa, Fmoc-2-amino-4,6-dimethyl-3-pyridinecarboxylic acid, Fmoc-D,L-?-amino-2-thiophenacetic acid, Fmoc-4-(carboxymethyl)piperazine, Fmoc-4-carboxypiperazine, Fmoc-4-(carboxymethyl)homopiperazine, Fmoc-4-phenyl-4-piperidinecarboxylic acid, Fmoc-L-1,2,3,4-tetrahydronorharman-3-carboxylic acid, Fmoc-L-thiazolidine-4-carboxylic acid, all available from The Peptide Laboratory (Richmond, CA, USA).

Non-natural residues can also be added biosynthetically by engineering a suppressor tRNA, typically one that recognizes the UAG stop codon, by chemical aminoacylation with the desired unnatural amino acid and. Conventional site-directed mutagenesis is used to introduce the chosen stop codon UAG at the site of interest in the protein gene. When the acylated suppressor tRNA and the mutant gene are combined in an *in vitro* transcription/translation system, the unnatural amino acid is incorporated in response to the UAG codon to give a protein containing that amino acid at the specified position. Liu *et al., Proc. Natl Acad. Sci. USA* 96(9):4780-5 (1999); Wang *et al., Science* 292(5516):498-500 (2001).

The isolated proteins, protein fragments and fusion proteins of the present invention can also include nonnative inter-residue bonds, including bonds that lead to circular and branched forms.

The isolated proteins and protein fragments of the present invention can also include post-translational and post-synthetic modifications, either throughout the length of the protein or localized to one or more portions thereof.

For example, when produced by recombinant expression in eukaryotic cells, the isolated proteins, fragments, and fusion proteins of the present invention will typically include N-linked and/or O-linked glycosylation, the pattern of which will reflect both the availability of glycosylation sites on the protein sequence and the identity of the host cell. Further modification of glycosylation pattern can be performed enzymatically.

As another example, recombinant polypeptides of the invention may also include an initial modified methionine residue, in some cases resulting from host-mediated processes.

When the proteins, protein fragments, and protein fusions of the present invention are produced by chemical synthesis, post-synthetic modification can be performed before deprotection and cleavage from the resin or after deprotection and cleavage. Modification before deprotection and cleavage of the synthesized protein often allows greater control, *e.g.* by allowing targeting of the modifying moiety to the N-terminus of a resin-bound synthetic peptide.

Useful post-synthetic (and post-translational) modifications include conjugation to detectable labels, such as fluorophores.

A wide variety of amine-reactive and thiol-reactive fluorophore derivatives have been synthesized that react under nondenaturing conditions with N-terminal amino groups and epsilon amino groups of lysine residues, on the one hand, and with free thiol groups of cysteine residues, on the other.

Kits are available commercially that permit conjugation of proteins to a variety of amine-reactive or thiol-reactive fluorophores: Molecular Probes, Inc. (Eugene, OR, USA), e.g., offers kits for conjugating proteins to Alexa Fluor 350, Alexa Fluor 430, Fluorescein-EX, Alexa Fluor 488, Oregon Green 488, Alexa Fluor 532, Alexa Fluor 546, Alexa Fluor 546, Alexa Fluor 568, Alexa Fluor 594, and Texas Red-X.

A wide variety of other amine-reactive and thiol-reactive fluorophores are available commercially (Molecular Probes, Inc., Eugene, OR, USA), including Alexa Fluor® 350, Alexa Fluor® 488, Alexa Fluor® 532, Alexa Fluor® 546, Alexa Fluor® 568, Alexa Fluor® 594, Alexa Fluor® 647 (monoclonal antibody labeling kits available from Molecular Probes, Inc., Eugene, OR, USA), BODIPY dyes, such as BODIPY 493/503, BODIPY FL, BODIPY R6G, BODIPY 530/550, BODIPY TMR, BODIPY 558/568, BODIPY 558/568, BODIPY 564/570, BODIPY 576/589, BODIPY 581/591, BODIPY TR, BODIPY 630/650, BODIPY 650/665, Cascade Blue, Cascade Yellow, Dansyl, lissamine rhodamine B, Marina Blue, Oregon Green 488, Oregon Green 514, Pacific Blue, rhodamine 6G, rhodamine green, rhodamine red, tetramethylrhodamine, Texas Red (available from Molecular Probes, Inc., Eugene, OR, USA).

The polypeptides of the present invention can also be conjugated to fluorophores, other proteins, and other macromolecules, using bifunctional linking reagents.

Common homobifunctional reagents include, *e.g.*, APG, AEDP, BASED, BMB, BMDB, BMH, BMOE, BM[PEO]3, BM[PEO]4, BS3, BSOCOES, DFDNB, DMA, DMP, DMS, DPDPB, DSG, DSP (Lomant's Reagent), DSS, DST, DTBP, DTME, DTSSP, EGS, HBVS, Sulfo-BSOCOES, Sulfo-DST, Sulfo-EGS (all available from Pierce, Rockford, IL, USA); common heterobifunctional cross-linkers include ABH, AMAS, ANB-NOS, APDP, ASBA, BMPA, BMPH, BMPS, EDC, EMCA, EMCH, EMCS, KMUA, KMUH, GMBS, LC-SMCC, LC-SPDP, MBS, M2C2H, MPBH, MSA, NHS-ASA, PDPH, PMPI, SADP, SAED, SAND, SANPAH, SASD, SATP, SBAP, SFAD, SIA, SIAB, SMCC, SMPB, SMPH, SMPT, SPDP, Sulfo-EMCS, Sulfo-GMBS, Sulfo-HSAB, Sulfo-KMUS, Sulfo-LC-SPDP, Sulfo-MBS, Sulfo-NHS-LC-ASA, Sulfo-SADP, Sulfo-SANPAH, Sulfo-SIAB, Sulfo-SMCC, Sulfo-SMPB, Sulfo-LC-SMPT, SVSB, TFCS (all available Pierce, Rockford, IL, USA).

The proteins, protein fragments, and protein fusions of the present invention can be conjugated, using such cross-linking reagents, to fluorophores that are not amine- or thiol-reactive.

Other labels that usefully can be conjugated to the proteins, protein fragments, and fusion proteins of the present invention include radioactive labels, echosonographic contrast reagents, and MRI contrast agents.

The proteins, protein fragments, and protein fusions of the present invention can also usefully be conjugated using cross-linking agents to carrier proteins, such as KLH, bovine thyroglobulin, and even bovine serum albumin (BSA), to increase immunogenicity for raising anti-HTPL antibodies.

The proteins, protein fragments, and protein fusions of the present invention can also usefully be conjugated to polyethylene glycol (PEG); PEGylation increases the serum half life of proteins administered intravenously for replacement therapy. Delgado *et al., Crit. Rev. Ther. Drug Carrier Syst.* 9(3-4):249-304 (1992); Scott *et al., Curr. Pharm. Des.* 4(6):423-38 (1998); DeSantis *et al., Curr. Opin. Biotechnol.* 10(4):324-30 (1999), incorporated herein by reference in their entireties. PEG monomers can be attached to the protein directly or through a linker, with PEGylation using PEG monomers activated with tresyl chloride (2,2,2-trifluoroethanesulphonyl chloride) permitting direct attachment under mild conditions.

The isolated proteins of the present invention, including fusions thereof, can be produced by recombinant expression, typically using the expression vectors of the present invention as above-described or, if fewer than about 100 amino acids, by chemical synthesis (typically, solid phase synthesis), and, on occasion, by *in vitro* translation.

Production of the isolated proteins of the present invention can optionally be followed by purification. Purification of recombinantly expressed proteins is now well within the skill in the art. *See, e.g.,* Thorner *et al.* (eds.), Applications of Chimeric Genes and Hybrid Proteins, Part A: Gene Expression and Protein Purification (Methods in Enzymology, Volume 326), Academic Press (2000), (ISBN: 0121822273); Harbin (ed.), Cloning, Gene Expression and Protein Purification : Experimental Procedures and Process Rationale, Oxford Univ. Press (2001) (ISBN: 0195132947); Marshak *et al.*, Strategies for Protein Purification and Characterization: A Laboratory Course Manual, Cold Spring Harbor Laboratory Press (1996) (ISBN: 0-87969-385-1); and Roe (ed.), Protein Purification Applications, Oxford University Press (2001), the disclosures of which are incorporated herein by reference in their entireties, and thus need not be detailed here.

Briefly, however, if purification tags have been fused through use of an expression vector that appends such tag, purification can be effected, at least in part, by means appropriate to the tag, such as use of immobilized metal affinity chromatography for polyhistidine tags. Other techniques common in the art include ammonium sulfate fractionation, immunoprecipitation, fast protein liquid chromatography (FPLC), high performance liquid chromatography (HPLC), and preparative gel electrophoresis.

Purification of chemically-synthesized peptides can readily be effected, *e.g.,* by HPLC.

Accordingly, it is an aspect of the present invention to provide the isolated proteins of the present invention in pure or substantially pure form.

A purified protein of the present invention is an isolated protein, as above described, that is present at a concentration of at least 95%, as measured on a weight basis (w/w) with respect to total protein in a composition. Such purities can often be obtained during chemical synthesis without further purification, as, *e.g.*, by HPLC. Purified proteins of the present invention can be present at a concentration (measured on a weight basis with respect to total protein in a composition) of 96%, 97%, 98%, and even 99%. The proteins of the present invention can even be present at levels of 99.5%, 99.6%, and even 99.7%, 99.8%, or even 99.9% following purification, as by HPLC.

Although high levels of purity are particularly useful when the isolated proteins of the present invention are used as therapeutic agents - such as vaccines, or for replacement therapy - the isolated proteins of the present invention are also useful at lower purity. For example, partially purified proteins of the present invention can be used as immunogens to raise antibodies in laboratory animals.

Thus, in another aspect, the present invention provides the isolated proteins of the present invention in substantially purified form. A "substantially purified protein" of the present invention is an isolated protein, as above described, present at a concentration of at least 70%, measured on a weight basis with respect to total protein in a composition. Usefully, the substantially purified protein is present at a concentration, measured on a weight basis with respect to total protein in a composition, of at least 75%, 80%, or even at least 85%, 90%, 91%, 92%, 93%, 94%, 94.5% or even at least 94.9%.

In preferred embodiments, the purified and substantially purified proteins of the present invention are in compositions that lack detectable ampholytes, acrylamide monomers, bis-acrylamide monomers, and polyacrylamide.

The proteins, fragments, and fusions of the present invention can usefully be attached to a substrate. The substrate can porous or solid, planar or non-planar; the bond can be covalent or noncovalent.

For example, the proteins, fragments, and fusions of the present invention can usefully be bound to a porous substrate, commonly a membrane, typically comprising nitrocellulose, polyvinylidene fluoride (PVDF), or cationically derivatized, hydrophilic PVDF; so bound, the proteins, fragments, and fusions of the present invention can be used to detect and quantify antibodies, *e.g.* in serum, that bind specifically to the immobilized protein of the present invention.

As another example, the proteins, fragments, and fusions of the present invention can usefully be bound to a substantially nonporous substrate, such as plastic, to detect and quantify antibodies, *e.g.* in serum, that bind specifically to the immobilized protein of the present invention. Such plastics include polymethylacrylic, polyethylene, polypropylene, polyacrylate, polymethylmethacrylate, polyvinylchloride, polytetrafluoroethylene, polystyrene, polycarbonate, polyacetal, polysulfone, celluloseacetate, cellulosenitrate, nitrocellulose, or mixtures thereof; when the assay is performed in standard microtiter dish, the plastic is typically polystyrene.

The proteins, fragments, and fusions of the present invention can also be attached to a substrate suitable for use as a surface enhanced laser desorption ionization source; so attached, the protein, fragment, or fusion of the present invention is useful for binding and then detecting secondary proteins that bind with sufficient affinity or avidity to the surface-bound protein to indicate biologic interaction therebetween. The proteins, fragments, and fusions of the present invention can also be attached to a substrate suitable for use in surface plasmon resonance detection; so attached, the protein, fragment, or fusion of the present invention is useful for binding and then detecting secondary proteins that bind with sufficient affinity or avidity to the surface-bound protein to indicate biological interaction therebetween.

### HTPL Proteins

In a first series of protein embodiments, the invention provides an isolated HTPL-L polypeptide having the amino acid sequence in SEQ ID NO: 3, which are full length HTPL-L proteins. When used as immunogens, the full length proteins of the present invention can be used, *inter alia,* to elicit antibodies that bind to a variety of epitopes of the HTPL-L protein.

The invention further provides fragments of the above-described polypeptides, particularly fragments having at least 6 amino acids, typically at least 8 amino acids, often at least 15 amino acids, and even the entirety of the sequence given in SEQ ID NO: 3.

In another series of protein embodiments, the invention provides an isolated HTPL-S polypeptide having the amino acid sequence in SEQ ID NO: 6, which are full length HTPL-S proteins. When used as immunogens, the full length proteins of the present invention can be used, *inter alia,* to elicit antibodies that bind to a variety of epitopes of the HTPL-S protein.

The invention further provides fragments of the above-described polypeptides, particularly fragments having at least 6 amino acids, typically at least 8 amino acids, often at least 15 amino acids, and even the entirety of the sequence given in SEQ ID NO: 6.

The invention further provides fragments of at least 6 amino acids, typically at least 8 amino acids, often at least 15 amino acids, and even the entirety of the sequence given in SEQ ID NO: 10, SEQ ID NO: 14, SEQ ID NO: 4799.

As described above, the invention further provides proteins that differ in sequence from those described with particularity in the above-referenced SEQ ID NOs., whether by way of insertion or deletion, by way of conservative or moderately conservative substitutions, as hybridization related proteins, or as cross-hybridizing proteins, with those that substantially retain a HTPL activity particularly useful.

The invention further provides fusions of the proteins and protein fragments herein described to heterologous polypeptides.

### ANTIBODIES AND ANTIBODY-PRODUCING CELLS

In another aspect, the invention provides antibodies, including fragments and derivatives thereof, that bind specifically to HTPL proteins and protein fragments of the present invention or to one or more of the proteins and protein fragments encoded by the isolated HTPL nucleic acids of the present invention. The antibodies of the present invention can be specific for all of linear epitopes, discontinuous epitopes, or conformational epitopes of such proteins or protein fragments, either as present on the protein in its native conformation or, in some cases, as present on the proteins as denatured, as, *e.g.*, by solubilization in SDS.

In other embodiments, the invention provides antibodies, including fragments and derivatives thereof, the binding of which can be competitively inhibited by one or more of the HTPL proteins and protein fragments of the present invention, or by one or more of the proteins and protein fragments encoded by the isolated HTPL nucleic acids of the present invention.

As used herein, the term "antibody" refers to a polypeptide, at least a portion of which is encoded by at least one immunoglobulin gene, which can bind specifically to a first molecular species, and to fragments or derivatives thereof that remain capable of such specific binding.

By "bind specifically" and "specific binding" is here intended the ability of the antibody to bind to a first molecular species in preference to binding to other molecular species with which the antibody and first molecular species are admixed. An antibody is said specifically to "recognize" a first molecular species when it can bind specifically to that first molecular species.

As is well known in the art, the degree to which an antibody can discriminate as among molecular species in a mixture will depend, in part, upon the conformational relatedness of the species in the mixture; typically, the antibodies of the present invention will discriminate over adventitious binding to non-HTPL proteins by at least two-fold, more typically by at least 5-fold, typically by more than 10-fold, 25-fold, 50-fold, 75-fold, and often by more than 100-fold, and on occasion by more than 500-fold or 1000-fold. When used to detect the proteins or protein fragments of the present invention, the antibody of the present invention is sufficiently specific when it can be used to determine the presence of the protein of the present invention in samples derived from human testis, as well as adrenal, adult and fetal liver, bone marrow, brain, kidney, lung, placenta, prostate, skeletal muscle and colon.

Typically, the affinity or avidity of an antibody (or antibody multimer, as in the case of an IgM pentamer) of the present invention for a protein or protein fragment of the present invention will be at least about 1 x 10⁻⁶ molar (M), typically at least about 5 x 10⁻⁷ M, usefully at least about 1 x 10⁻⁷ M, with affinities and avidities of at least 1 x 10⁻⁸ M, 5 x 10⁻⁹ M, and 1 x 10⁻¹⁰ M proving especially useful.

The antibodies of the present invention can be naturally-occurring forms, such as IgG, IgM, IgD, IgE, and IgA, from any mammalian species.

Human antibodies can, but will infrequently, be drawn directly from human donors or human cells. In such case, antibodies to the proteins of the present invention will typically have resulted from fortuitous immunization, such as autoimmune immunization, with the protein or protein fragments of the present invention. Such antibodies will typically, but will not invariably, be polyclonal.

Human antibodies are more frequently obtained using transgenic animals that express human immunoglobulin genes, which transgenic animals can be affirmatively immunized with the protein immunogen of the present invention. Human Ig-transgenic mice capable of producing human antibodies and methods of producing human antibodies therefrom upon specific immunization are described, *inter alia,* in U.S. Patent Nos. 6,162,963; 6,150,584; 6,114,598; 6,075,181; 5,939,598; 5,877,397; 5,874,299; 5,814,318; 5,789,650; 5,770,429; 5,661,016; 5,633,425; 5,625,126; 5,569,825; 5,545,807; 5,545,806, and 5,591,669, the disclosures of which are incorporated herein by reference in their entireties. Such antibodies are typically monoclonal, and are typically produced using techniques developed for production of murine antibodies.

Human antibodies are particularly useful, and often preferred, when the antibodies of the present invention are to be administered to human beings as *in vivo* diagnostic or therapeutic agents, since recipient immune response to the administered antibody will often be substantially less than that occasioned by administration of an antibody derived from another species, such as mouse.

IgG, IgM, IgD, IgE and IgA antibodies of the present invention are also usefully obtained from other mammalian species, including rodents - typically mouse, but also rat, guinea pig, and hamster - lagomorphs, typically rabbits, and also larger mammals, such as sheep, goats, cows, and horses. In such cases, as with the transgenic human-antibody-producing non-human mammals, fortuitous immunization is not required, and the non-human mammal is typically affirmatively immunized, according to standard immunization protocols, with the protein or protein fragment of the present invention.

As discussed above, virtually all fragments of 8 or more contiguous amino acids of the proteins of the present invention can be used effectively as immunogens when conjugated to a carrier, typically a protein such as bovine thyroglobulin, keyhole limpet hemocyanin, or bovine serum albumin, conveniently using a bifunctional linker such as those described elsewhere above, which discussion is incorporated by reference here.

Immunogenicity can also be conferred by fusion of the proteins and protein fragments of the present invention to other moieties.

For example, peptides of the present invention can be produced by solid phase synthesis on a branched polylysine core matrix; these multiple antigenic peptides (MAPs) provide high purity, increased avidity, accurate chemical definition and improved safety in vaccine development. Tam *et al.*, Proc. Natl. Acad. Sci. USA 85:5409-5413 (1988); Posnett *et* al., *J. Biol. Chem.* 263, 1719-1725 (1988).

Protocols for immunizing non-human mammals are well-established in the art, Harlow *et al*. (eds.), Antibodies: A Laboratory Manual, Cold Spring Harbor Laboratory (1998) (ISBN: 0879693142); Coligan *et al*. (eds.), Current Protocols in Immunology, John Wiley & Sons, Inc. (2001) (ISBN: 0-471-52276-7); Zola, Monoclonal Antibodies : Preparation and Use of Monoclonal Antibodies and Engineered Antibody Derivatives (Basics: From Background to Bench), Springer Verlag (2000) (ISBN: 0387915907), the disclosures of which are incorporated herein by reference, and often include multiple immunizations, either with or without adjuvants such as Freund's complete adjuvant and Freund's incomplete adjuvant.

Antibodies from nonhuman mammals can be polyclonal or monoclonal, with polyclonal antibodies having certain advantages in immunohistochemical detection of the proteins of the present invention and monoclonal antibodies having advantages in identifying and distinguishing particular epitopes of the proteins of the present invention.

Following immunization, the antibodies of the present invention can be produced using any art-accepted technique. Such techniques are well known in the art, Coligan *et al*. (eds.), Current Protocols in Immunology, John Wiley & Sons, Inc. (2001) (ISBN: 0-471-52276-7); Zola, Monoclonal Antibodies : Preparation and Use of Monoclonal Antibodies and Engineered Antibody Derivatives (Basics: From Background to Bench), Springer Verlag (2000) (ISBN: 0387915907); Howard *et al.* (eds.), Basic Methods in Antibody Production and Characterization, CRC Press (2000) (ISBN: 0849394457); Harlow *et al.* (eds.), Antibodies: A Laboratory Manual, Cold Spring Harbor Laboratory (1998) (ISBN: 0879693142); Davis (ed.), Monoclonal Antibody Protocols, Vol. 45, Humana Press (1995) (ISBN: 0896033082); Delves (ed.), Antibody Production: Essential Techniques, John Wiley & Son Ltd (1997) (ISBN: 0471970107); Kenney, Antibody Solution: An Antibody Methods Manual, Chapman & Hall (1997) (ISBN: 0412141914), incorporated herein by reference in their entireties, and thus need not be detailed here.

Briefly, however, such techniques include, *inter alia,* production of monoclonal antibodies by hybridomas and expression of antibodies or fragments or derivatives thereof from host cells engineered to express immunoglobulin genes or fragments thereof.

These two methods of production are not mutually exclusive: genes encoding antibodies specific for the proteins or protein fragments of the present invention can be cloned from hybridomas and thereafter expressed in other host cells. Nor need the two necessarily be performed together: *e.g.*, genes encoding antibodies specific for the proteins and protein fragments of the present invention can be cloned directly from B cells known to be specific for the desired protein, as further described in U.S. Pat. No. 5,627,052, the disclosure of which is incorporated herein by reference in its entirety, or from antibody-displaying phage.

Recombinant expression in host cells is particularly useful when fragments or derivatives of the antibodies of the present invention are desired.

Host cells for recombinant antibody production - either whole antibodies, antibody fragments, or antibody derivatives - can be prokaryotic or eukaryotic.

Prokaryotic hosts are particularly useful for producing phage displayed antibodies of the present invention.

The technology of phage-displayed antibodies, in which antibody variable region fragments are fused, for example, to the gene III protein (pIII) or gene VIII protein (pVIII) for display on the surface of filamentous phage, such as M13, is by now well-established, Sidhu, *Curr. Opin. Biotechnol.* 11(6):610-6 (2000); Griffiths *et al., Curr. Opin. Biotechnol*. 9(1):102-8 (1998); Hoogenboom *et al., Immunotechnology,* 4(1):1-20 (1998); Rader *et al., Current Opinion in Biotechnology* 8:503-508 (1997); Aujame *et al., Human Antibodies* 8:155-168 (1997); Hoogenboom, *Trends in Biotechnol.* 15:62-70 (1997); de Kruif *et al.,* 17:453-455 (1996); Barbas *et al., Trends in Biotechnol.* 14:230-234 (1996); Winter *et al., Ann. Rev. Immunol.* 433-455 (1994), and techniques and protocols required to generate, propagate, screen (pan), and use the antibody fragments from such libraries have recently been compiled, Barbas et *al.,* Phage Display: A Laboratory Manual, Cold Spring Harbor Laboratory Press (2001) (ISBN 0-87969-546-3); Kay *et al.* (eds.), Phage Display of Peptides and Proteins: A Laboratory Manual, Academic Press, Inc. (1996); Abelson *et al.* (eds.), Combinatorial Chemistry, Methods in Enzymology vol. 267, Academic Press (May 1996), the disclosures of which are incorporated herein by reference in their entireties.

Typically, phage-displayed antibody fragments are scFv fragments or Fab fragments; when desired, full length antibodies can be produced by cloning the variable regions from the displaying phage into a complete antibody and expressing the full length antibody in a further prokaryotic or a eukaryotic host cell.

Eukaryotic cells are also useful for expression of the antibodies, antibody fragments, and antibody derivatives of the present invention.

For example, antibody fragments of the present invention can be produced in *Pichia pastoris,* Takahashi *et al., Biosci. Biotechnol. Biochem.* 64(10):2138-44 (2000); Freyre *et al.,* J. Biotechnol. 76(2-3):157-63 (2000); Fischer *et al., Biotechnol. Appl. Biochem.* 30 (Pt 2):117-20 (1999); Pennell *et al., Res. Immunol.* 149(6):599-603 (1998); Eldin *et al., J. Immunol. Methods.* 201(1):67-75 (1997); and in *Saccharomyces cerevisiae,* Frenken *et al., Res. Immunol.* 149(6):589-99 (1998); Shusta et al., *Nature Biotechnol.* 16(8):773-7 (1998), the disclosures of which are incorporated herein by reference in their entireties.

Antibodies, including antibody fragments and derivatives, of the present invention can also be produced in insect cells, Li *et al., Protein Expr. Purif.* 21(1):121-8 (2001); Ailor *et al., Biotechnol. Bioeng*. 58(2-3):196-203 (1998); Hsu *et al., Biotechnol. Prog.* 13(1):96-104 (1997); Edelman *et al., Immunology* 91(1):13-9 (1997); and Nesbit *et al., J. Immunol. Methods.* 151(1-2):201-8 (1992), the disclosures of which are incorporated herein by reference in their entireties.

Antibodies and fragments and derivatives thereof of the present invention can also be produced in plant cells, Giddings *et al*., *Nature Biotechnol*. 18(11):1151-5 (2000); Gavilondo *et al., Biotechniques* 29(1):128-38 (2000); Fischer *et al., J. Biol. Regul. Homeost. Agents* 14(2):83-92 (2000); Fischer *et al., Biotechnol. Appl. Biochem.* 30 (Pt 2):113-6 (1999); Fischer *et al., Biol. Chem.* 380(7-8):825-39 (1999); Russell, *Curr. Top. Microbiol. Immunol.* 240:119-38 (1999); and Ma *et al., Plant Physiol.* 109(2):341-6 (1995), the disclosures of which are incorporated herein by reference in their entireties.

Mammalian cells useful for recombinant expression of antibodies, antibody fragments, and antibody derivatives of the present invention include CHO cells, COS cells, 293 cells, and myeloma cells.

Verma *et al., J. Immunol. Methods* 216(1-2):165-81 (1998), review and compare bacterial, yeast, insect and mammalian expression systems for expression of antibodies.

Antibodies of the present invention can also be prepared by cell free translation, as further described in Merk *et al.,* J. Biochem. (Tokyo). 125(2):328-33 (1999) and Ryabova *et al., Nature Biotechnol*. 15(1):79-84 (1997), and in the milk of transgenic animals, as further described in Pollock *et al., J. Immunol. Methods* 231(1-2):147-57 (1999), the disclosures of which are incorporated herein by reference in their entireties.

The invention further provides antibody fragments that bind specifically to one or more of the proteins and protein fragments of the present invention, to one or more of the proteins and protein fragments encoded by the isolated nucleic acids of the present invention, or the binding of which can be competitively inhibited by one or more of the proteins and protein fragments of the present invention or one or more of the proteins and protein fragments encoded by the isolated nucleic acids of the present invention.

Among such useful fragments are Fab, Fab', Fv, F(ab)'₂, and single chain Fv (scFv) fragments. Other useful fragments are described in Hudson, *Curr. Opin. Biotechnol*. 9(4):395-402 (1998).

It is also an aspect of the present invention to provide antibody derivatives that bind specifically to one or more of the proteins and protein fragments of the present invention, to one or more of the proteins and protein fragments encoded by the isolated nucleic acids of the present invention, or the binding of which can be competitively inhibited by one or more of the proteins and protein fragments of the present invention or one or more of the proteins and protein fragments encoded by the isolated nucleic acids of the present invention.

Among such useful derivatives are chimeric, primatized, and humanized antibodies; such derivatives are less immunogenic in human beings, and thus more suitable for in vivo administration, than are unmodified antibodies from non-human mammalian species.

Chimeric antibodies typically include heavy and/or light chain variable regions (including both CDR and framework residues) of immunoglobulins of one species, typically mouse, fused to constant regions of another species, typically human. *See, e.g.,* U.S. Pat. No. 5,807,715; Morrison *et al., Proc. Natl.* *Acad. Sci USA*.81(21):6851-5 (1984); Sharon *et al.*, *Nature* 309(5966):364-7 (1984); Takeda *et al., Nature* 314(6010):452-4 (1985), the disclosures of which are incorporated herein by reference in their entireties. Primatized and humanized antibodies typically include heavy and/or light chain CDRs from a murine antibody grafted into a non-human primate or human antibody V region framework, usually further comprising a human constant region, Riechmann *et al., Nature* 332(6162):323-7 (1988); Co *et al., Nature* 351(6326):501-2 (1991); U.S. Pat. Nos. 6,054,297; 5,821,337; 5,770,196; 5,766,886; 5,821,123; 5,869,619; 6,180,377; 6,013,256; 5,693,761; and 6,180,370, the disclosures of which are incorporated herein by reference in their entireties.

Other useful antibody derivatives of the invention include heteromeric antibody complexes and antibody fusions, such as diabodies (bispecific antibodies), single-chain diabodies, and intrabodies.

The antibodies of the present invention, including fragments and derivatives thereof, can usefully be labeled. It is, therefore, another aspect of the present invention to provide labeled antibodies that bind specifically to one or more of the proteins and protein fragments of the present invention, to one or more of the proteins and protein fragments encoded by the isolated nucleic acids of the present invention, or the binding of which can be competitively inhibited by one or more of the proteins and protein fragments of the present invention or one or more of the proteins and protein fragments encoded by the isolated nucleic acids of the present invention.

The choice of label depends, in part, upon the desired use.

For example, when the antibodies of the present invention are used for immunohistochemical staining of tissue samples, the label can usefully be an enzyme that catalyzes production and local deposition of a detectable product.

Enzymes typically conjugated to antibodies to permit their immunohistochemical visualization are well known, and include alkaline phosphatase, β-galactosidase, glucose oxidase, horseradish peroxidase (HRP), and urease. Typical substrates for production and deposition of visually detectable products include o-nitrophenyl-beta-D-galactopyranoside (ONPG); o-phenylenediamine dihydrochloride (OPD); p-nitrophenyl phosphate (PNPP); p-nitrophenyl-beta-D-galactopryanoside (PNPG); 3',3'-diaminobenzidine (DAB); 3-amino-9-ethylcarbazole (AEC); 4-chloro-1-naphthol (CN); 5-bromo-4-chloro-3-indolyl-phosphate (BCIP); ABTS®; BluoGal; iodonitrotetrazolium (INT); nitroblue tetrazolium chloride (NBT); phenazine methosulfate (PMS); phenolphthalein monophosphate (PMP); tetramethyl benzidine (TMB); tetranitroblue tetrazolium (TNBT); X-Gal; X-Gluc; and X-Glucoside.

Other substrates can be used to produce products for local deposition that are luminescent. For example, in the presence of hydrogen peroxide (H₂O₂), horseradish peroxidase (HRP) can catalyze the oxidation of cyclic diacylhydrazides, such as luminol. Immediately following the oxidation, the luminol is in an excited state (intermediate reaction product), which decays to the ground state by emitting light. Strong enhancement of the light emission is produced by enhancers, such as phenolic compounds. Advantages include high sensitivity, high resolution, and rapid detection without radioactivity and requiring only small amounts of antibody. *See, e.g.,* Thorpe *et al., Methods Enzymol.* 133:331-53 (1986); Kricka *et al., J. Immunoassay* 17(1):67-83 (1996); and Lundqvist *et al., J. Biolumin. Chemilumin*. 10(6):353-9 (1995), the disclosures of which are incorporated herein by reference in their entireties. Kits for such enhanced chemiluminescent detection (ECL) are available commercially.

The antibodies can also be labeled using colloidal gold.

As another example, when the antibodies of the present invention are used, *e.g.*, for flow cytometric detection, for scanning laser cytometric detection, or for fluorescent immunoassay, they can usefully be labeled with fluorophores.

There are a wide variety of fluorophore labels that can usefully be attached to the antibodies of the present invention.

For flow cytometric applications, both for extracellular detection and for intracellular detection, common useful fluorophores can be fluorescein isothiocyanate (FITC), allophycocyanin (APC), R-phycoerythrin (PE), peridinin chlorophyll protein (PerCP), Texas Red, Cy3, Cy5, fluorescence resonance energy tandem fluorophores such as PerCP-Cy5.5, PE-Cy5, PE-Cy5.5, PE-Cy7, PE-Texas Red, and APC-Cy7.

Other fluorophores include, *inter alia,* Alexa Fluor® 350, Alexa Fluor® 488, Alexa Fluor® 532, Alexa Fluor® 546, Alexa Fluor® 568, Alexa Fluor® 594, Alexa Fluor® 647 (monoclonal antibody labeling kits available from Molecular Probes, Inc., Eugene, OR, USA), BODIPY dyes, such as BODIPY 493/503, BODIPY FL, BODIPY R6G, BODIPY 530/550, BODIPY TMR, BODIPY 558/568, BODIPY 558/568, BODIPY 564/570, BODIPY 576/589, BODIPY 581/591, BODIPY TR, BODIPY 630/650, BODIPY 650/665, Cascade Blue, Cascade Yellow, Dansyl, lissamine rhodamine B, Marina Blue, Oregon Green 488, Oregon Green 514, Pacific Blue, rhodamine 6G, rhodamine green, rhodamine red, tetramethylrhodamine, Texas Red (available from Molecular Probes, Inc., Eugene, OR, USA), and Cy2, Cy3, Cy3.5, Cy5, Cy5.5, Cy7, all of which are also useful for fluorescently labeling the antibodies of the present invention.

For secondary detection using labeled avidin, streptavidin, captavidin or neutravidin, the antibodies of the present invention can usefully be labeled with biotin.

When the antibodies of the present invention are used, *e.g.*, for western blotting applications, they can usefully be labeled with radioisotopes, such as ³³P, ³²P, ³⁵S, ³H, and ¹²⁵I.

As another example, when the antibodies of the present invention are used for radioimmunotherapy, the label can usefully be ²²⁸Th, ²²⁷Ac, ²²⁵Ac, ²²³Ra, ²¹³Bi, ²¹²Pb, ²¹²Bi, ²¹¹At, ²⁰³Pb, ¹⁹⁴Os, ¹⁸⁸Re, ¹⁸⁶Re, ¹⁵³Sm, ¹⁴⁹Tb, ¹³¹I, , ¹²⁵I, ¹¹¹In, ¹⁰⁵Rh, ^{99m}Tc, ⁹⁷Ru, ⁹⁰Y, ⁹⁰Sr, ⁸⁸Y, ⁷²Se, ⁶⁷Cu, or ⁴⁷Sc.

As another example, when the antibodies of the present invention are to be used for *in vivo* diagnostic use, they can be rendered detectable by conjugation to MRI contrast agents, such as gadolinium diethylenetriaminepentaacetic acid (DTPA), Lauffer *et al., Radiology* 207(2):529-38 (1998), or by radioisotopic labeling

As would be understood, use of the labels described above is not restricted to the application as for which they were mentioned.

The antibodies of the present invention, including fragments and derivatives thereof, can also be conjugated to toxins, in order to target the toxin's ablative action to cells that display and/or express the proteins of the present invention. Commonly, the antibody in such immunotoxins is conjugated to Pseudomonas exotoxin A, diphtheria toxin, shiga toxin A, anthrax toxin lethal factor, or ricin. *See* Hall (ed.), Immunotoxin Methods and Protocols (Methods in Molecular Biology, Vol 166), Humana Press (2000) (ISBN:0896037754); and Frankel *et al*. (eds.), Clinical Applications of Immunotoxins, Springer-Verlag New York, Incorporated (1998) (ISBN:3540640975), the disclosures of which are incorporated herein by reference in their entireties, for review.

The antibodies of the present invention can usefully be attached to a substrate, and it is, therefore, another aspect of the invention to provide antibodies that bind specifically to one or more of the proteins and protein fragments of the present invention, to one or more of the proteins and protein fragments encoded by the isolated nucleic acids of the present invention, or the binding of which can be competitively inhibited by one or more of the proteins and protein fragments of the present invention or one or more of the proteins and protein fragments encoded by the isolated nucleic acids of the present invention, attached to a substrate.

Substrates can be porous or nonporous, planar or nonplanar.

For example, the antibodies of the present invention can usefully be conjugated to filtration media, such as NHS-activated Sepharose or CNBr-activated Sepharose for purposes of immunoaffinity chromatography.

For example, the antibodies of the present invention can usefully be attached to paramagnetic microspheres, typically by biotin-streptavidin interaction, which microsphere can then be used for isolation of cells that express or display the proteins of the present invention. As another example, the antibodies of the present invention can usefully be attached to the surface of a microtiter plate for ELISA.

As noted above, the antibodies of the present invention can be produced in prokaryotic and eukaryotic cells. It is, therefore, another aspect of the present invention to provide cells that express the antibodies of the present invention, including hybridoma cells, B cells, plasma cells, and host cells recombinantly modified to express the antibodies of the present invention.

In yet a further aspect, the present invention provides aptamers evolved to bind specifically to one or more of the proteins and protein fragments of the present invention, to one or more of the proteins and protein fragments encoded by the isolated nucleic acids of the present invention, or the binding of which can be competitively inhibited by one or more of the proteins and protein fragments of the present invention or one or more of the proteins and protein fragments encoded by the isolated nucleic acids of the present invention.

### HTPL Antibodies

In a first series of antibody embodiments, the invention provides antibodies, both polyclonal and monoclonal, and fragments and derivatives thereof, that bind specifically to a polypeptide having the amino acid sequence in SEQ ID NO: 3, which are full length HTPL-L proteins.

In a second series of antibody embodiments, the invention provides antibodies, both polyclonal and monoclonal, and fragments and derivatives thereof, that bind specifically to a polypeptide having the amino acid sequence in SEQ ID NO: 6, which are full length HTPL-S proteins.

Such antibodies are useful in *in vitro* immunoassays, such as ELISA, western blot or immunohistochemical assay of testis or other disease tissue or cells. Such antibodies are also useful in isolating and purifying HTPL proteins, including related cross-reactive proteins, by immunoprecipitation, immunoaffinity chromatography, or magnetic bead-mediated purification.

In another series of antibody embodiments, the invention provides antibodies, both polyclonal and monoclonal, and fragments and derivatives thereof, that bind specifically to a polypeptide having an amino acid sequence in SEQ ID NO: 14, which is unique to the HTPL-L isoform, and binding of which can be competitively inhibited by a polypeptide the sequence of which is given in SEQ ID NO: 3 and cannot be competitively inhibited by a polypeptide having the amino acid sequence of SEQ ID NO: 6 (the full length HTPL-S protein).

Such antibodies are useful in *in vitro* immunoassays, such as ELISA, western blot or immunohistochemical assay of testis or other disease tissue or cells. Such antibodies are also useful in isolating and purifying HTPL-L protein, including related cross-reactive proteins, by immunoprecipitation, immunoaffinity chromatography, or magnetic bead-mediated purification.

In another series of antibody embodiments, the invention provides antibodies, both polyclonal and monoclonal, and fragments and derivatives thereof, the specific binding of which can be competitively inhibited by the isolated proteins and polypeptides of the present invention.

In other embodiments, the invention further provides the above-described antibodies detectably labeled, and in yet other embodiments, provides the above-described antibodies attached to a substrate.

### PHARMACEUTICAL COMPOSITIONS

HTPL is important in regulating male germ cell development and as a tumor suppressor; defects in HTPL expression, activity, distribution, localization, and/or solubility are a cause of human disease, which disease can manifest as a disorder of testis, or adrenal, adult and fetal liver, bone marrow, brain, kidney, lung, placenta, prostate, skeletal muscle or colon function.

Accordingly, pharmaceutical compositions comprising nucleic acids, proteins, and antibodies of the present invention, as well as mimetics, agonists, antagonists, or inhibitors of HTPL activity, can be administered as therapeutics for treatment of HTPL defects.

Thus, in another aspect, the invention provides pharmaceutical compositions comprising the nucleic acids, nucleic acid fragments, proteins, protein fusions, protein fragments, antibodies, antibody derivatives, antibody fragments, mimetics, agonists, antagonists, and inhibitors of the present invention.

Such a composition typically contains from about 0.1 to 90% by weight of a therapeutic agent of the invention formulated in and/or with a pharmaceutically acceptable carrier or excipient.

Pharmaceutical formulation is a well-established art, and is further described in Gennaro (ed.), Remington: The Science and Practice of Pharmacy, 20^{th} ed., Lippincott, Williams & Wilkins (2000) (ISBN: 0683306472); Ansel *et al.,* Pharmaceutical Dosage Forms and Drug Delivery Systems, 7^{th} ed., Lippincott Williams & Wilkins Publishers (1999) (ISBN: 0683305727); and Kibbe (ed.), Handbook of Pharmaceutical Excipients American Pharmaceutical Association, 3^{rd} ed. (2000) (ISBN: 091733096X), the disclosures of which are incorporated herein by reference in their entireties, and thus need not be described in detail herein.

Briefly, however, formulation of the pharmaceutical compositions of the present invention will depend upon the route chosen for administration. The pharmaceutical compositions utilized in this invention can be administered by various routes including both enteral and parenteral routes, including oral, intravenous, intramuscular, subcutaneous, inhalation, topical, sublingual, rectal, intra-arterial, intramedullary, intrathecal, intraventricular, transmucosal, transdermal, intranasal, intraperitoneal, intrapulmonary, and intrauterine.

Oral dosage forms can be formulated as tablets, pills, dragees, capsules, liquids, gels, syrups, slurries, suspensions, and the like, for ingestion by the patient.

Solid formulations of the compositions for oral administration can contain suitable carriers or excipients, such as carbohydrate or protein fillers, such as sugars, including lactose, sucrose, mannitol, or sorbitol; starch from corn, wheat, rice, potato, or other plants; cellulose, such as methyl cellulose, hydroxypropylmethyl-cellulose, sodium carboxymethylcellulose, or microcrystalline cellulose; gums including arabic and tragacanth; proteins such as gelatin and collagen; inorganics, such as kaolin, calcium carbonate, dicalcium phosphate, sodium chloride; and other agents such as acacia and alginic acid.

Agents that facilitate disintegration and/or solubilization can be added, such as the cross-linked polyvinyl pyrrolidone, agar, alginic acid, or a salt thereof, such as sodium alginate, microcrystalline cellulose, corn starch, sodium starch glycolate, and alginic acid.

Tablet binders that can be used include acacia, methylcellulose, sodium carboxymethylcellulose, polyvinylpyrrolidone (Povidone™), hydroxypropyl methylcellulose, sucrose, starch and ethylcellulose.

Lubricants that can be used include magnesium stearates, stearic acid, silicone fluid, talc, waxes, oils, and colloidal silica.

Fillers, agents that facilitate disintegration and/or solubilization, tablet binders and lubricants, including the aforementioned, can be used singly or in combination.

Solid oral dosage forms need not be uniform throughout.

For example, dragee cores can be used in conjunction with suitable coatings, such as concentrated sugar solutions, which can also contain gum arabic, talc, polyvinylpyrrolidone, carbopol gel, polyethylene glycol, and/or titanium dioxide, lacquer solutions, and suitable organic solvents or solvent mixtures.

Oral dosage forms of the present invention include push-fit capsules made of gelatin, as well as soft, sealed capsules made of gelatin and a coating, such as glycerol or sorbitol. Push-fit capsules can contain active ingredients mixed with a filler or binders, such as lactose or starches, lubricants, such as talc or magnesium stearate, and, optionally, stabilizers. In soft capsules, the active compounds can be dissolved or suspended in suitable liquids, such as fatty oils, liquid, or liquid polyethylene glycol with or without stabilizers.

Additionally, dyestuffs or pigments can be added to the tablets or dragee coatings for product identification or to characterize the quantity of active compound, *i.e*., dosage.

Liquid formulations of the pharmaceutical compositions for oral (enteral) administration are prepared in water or other aqueous vehicles and can contain various suspending agents such as methylcellulose, alginates, tragacanth, pectin, kelgin, carrageenan, acacia, polyvinylpyrrolidone, and polyvinyl alcohol. The liquid formulations can also include solutions, emulsions, syrups and elixirs containing, together with the active compound(s), wetting agents, sweeteners, and coloring and flavoring agents.

The pharmaceutical compositions of the present invention can also be formulated for parenteral administration.

For intravenous injection, water soluble versions of the compounds of the present invention are formulated in, or if provided as a lyophilate, mixed with, a physiologically acceptable fluid vehicle, such as 5% dextrose ("D5"), physiologically buffered saline, 0.9% saline, Hanks' solution, or Ringer's solution.

Intramuscular preparations, *e.g.* a sterile formulation of a suitable soluble salt form of the compounds of the present invention, can be dissolved and administered in a pharmaceutical excipient such as Water-for-Injection, 0.9% saline, or 5% glucose solution. Alternatively, a suitable insoluble form of the compound can be prepared and administered as a suspension in an aqueous base or a pharmaceutically acceptable oil base, such as an ester of a long chain fatty acid (*e.g.*, ethyl oleate), fatty oils such as sesame oil, triglycerides, or liposomes.

Parenteral formulations of the compositions can contain various carriers such as vegetable oils, dimethylacetamide, dimethylformamide, ethyl lactate, ethyl carbonate, isopropyl myristate, ethanol, polyols (glycerol, propylene glycol, liquid polyethylene glycol, and the like).

Aqueous injection suspensions can also contain substances that increase the viscosity of the suspension, such as sodium carboxymethyl cellulose, sorbitol, or dextran. Non-lipid polycationic amino polymers can also be used for delivery.

Optionally, the suspension can also contain suitable stabilizers or agents that increase the solubility of the compounds to allow for the preparation of highly concentrated solutions.

Pharmaceutical compositions of the present invention can also be formulated to permit injectable, long-term, deposition.

The pharmaceutical compositions of the present invention can be administered topically.

A topical semi-solid ointment formulation typically contains a concentration of the active ingredient from about 1 to 20%, *e.g.*, 5 to 10%, in a carrier such as a pharmaceutical cream base. Various formulations for topical use include drops, tinctures, lotions, creams, solutions, and ointments containing the active ingredient and various supports and vehicles. In other transdermal formulations, typically in patch-delivered formulations, the pharmaceutically active compound is formulated with one or more skin penetrants, such as 2-N-methyl-pyrrolidone (NMP) or Azone.

Inhalation formulations can also readily be formulated. For inhalation, various powder and liquid formulations can be prepared.

The pharmaceutically active compound in the pharmaceutical compositions of the present inention can be provided as the salt of a variety of acids, including but not limited to hydrochloric, sulfuric, acetic, lactic, tartaric, malic, and succinic acid. Salts tend to be more soluble in aqueous or other protonic solvents than are the corresponding free base forms.

After pharmaceutical compositions have been prepared, they are packaged in an appropriate container and labeled for treatment of an indicated condition.

The active compound will be present in an amount effective to achieve the intended purpose. The determination of an effective dose is well within the capability of those skilled in the art.

A "therapeutically effective dose" refers to that amount of active ingredient - for example HTPL protein, fusion protein, or fragments thereof, antibodies specific for HTPL, agonists, antagonists or inhibitors of HTPL - which ameliorates the signs or symptoms of the disease or prevents progression thereof; as would be understood in the medical arts, cure, although desired, is not required.

The therapeutically effective dose of the pharmaceutical agents of the present invention can be estimated initially by *in vitro* tests, such as cell culture assays, followed by assay in model animals, usually mice, rats, rabbits, dogs, or pigs. The animal model can also be used to determine an initial useful concentration range and route of administration.

For example, the ED50 (the dose therapeutically effective in 50% of the population) and LD50 (the dose lethal to 50% of the population) can be determined in one or more cell culture of animal model systems. The dose ratio of toxic to therapeutic effects is the therapeutic index, which can be expressed as LD50/ED50. Pharmaceutical compositions that exhibit large therapeutic indices are particularly useful.

The data obtained from cell culture assays and animal studies is used in formulating an initial dosage range for human use, and preferably provides a range of circulating concentrations that includes the ED50 with little or no toxicity. After administration, or between successive administrations, the circulating concentration of active agent varies within this range depending upon pharmacokinetic factors well known in the art, such as the dosage form employed, sensitivity of the patient, and the route of administration.

The exact dosage will be determined by the practitioner, in light of factors specific to the subject requiring treatment. Factors that can be taken into account by the practitioner include the severity of the disease state, general health of the subject, age, weight, gender of the subject, diet, time and frequency of administration, drug combination(s), reaction sensitivities, and tolerance/response to therapy. Long-acting pharmaceutical compositions can be administered every 3 to 4 days, every week, or once every two weeks depending on half-life and clearance rate of the particular formulation.

Normal dosage amounts may vary from 0.1 to 100,000 micrograms, up to a total dose of about 1 g, depending upon the route of administration. Where the therapeutic agent is a protein or antibody of the present invention, the therapeutic protein or antibody agent typically is administered at a daily dosage of 0.01 mg to 30 mg/kg of body weight of the patient (e.g., lmg/kg to 5 mg/kg). The pharmaceutical formulation can be administered in multiple doses per day, if desired, to achieve the total desired daily dose.

Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc.

Conventional methods, known to those of ordinary skill in the art of medicine, can be used to administer the pharmaceutical formulation(s) of the present invention to the patient. The pharmaceutical compositions of the present invention can be administered alone, or in combination with other therapeutic agents or interventions.

### THERAPEUTIC METHODS

The present invention further provides methods of treating subjects having defects in HTPL - *e.g.*, in expression, activity, distribution, localization, and/or solubility of HTPL - which can manifest as a disorder of testis, or adrenal, adult and fetal liver, bone marrow, brain, kidney, lung, placenta, prostate, skeletal muscle or colon function. As used herein, "treating" includes all medically-acceptable types of therapeutic intervention, including palliation and prophylaxis (prevention) of disease.

In one embodiment of the therapeutic methods of the present invention, a therapeutically effective amount of a pharmaceutical composition comprising HTPL protein, fusion, fragment or derivative thereof is administered to a subject with a clinically-significant HTPL defect.

Protein compositions are administered, for example, to complement a deficiency in native HTPL. In other embodiments, protein compositions are administered as a vaccine to elicit a humoral and/or cellular immune response to HTPL. The immune response can be used to modulate activity of HTPL or, depending on the immunogen, to immunize against aberrant or aberrantly expressed forms, such as mutant or inappropriately expressed isoforms. In yet other embodiments, protein fusions having a toxic moiety are administered to ablate cells that aberrantly accumulate HTPL.

In another embodiment of the therapeutic methods of the present invention, a therapeutically effective amount of a pharmaceutical composition comprising nucleic acid of the present invention is administered. The nucleic acid can be delivered in a vector that drives expression of HTPL protein, fusion, or fragment thereof, or without such vector.

Nucleic acid compositions that can drive expression of HTPL are administered, for example, to complement a deficiency in native HTPL, or as DNA vaccines. Expression vectors derived from virus, replication deficient retroviruses, adenovirus, adeno-associated (AAV) virus, herpes virus, or vaccinia virus can be used - *see, e.g.,* Cid-Arregui (ed.), Viral Vectors: Basic Science and Gene Therapy, Eaton Publishing Co., 2000 (ISBN: 188129935X) - as can plasmids .

Antisense nucleic acid compositions, or vectors that drive expression of HTPL antisense nucleic acids, are administered to downregulate transcription and/or translation of HTPL in circumstances in which excessive production, or production of aberrant protein, is the pathophysiologic basis of disease.

Antisense compositions useful in therapy can have sequence that is complementary to coding or to noncoding regions of the HTPL gene. For example, oligonucleotides derived from the transcription initiation site, *e.g.,* between positions -10 and +10 from the start site, are particularly useful.

Catalytic antisense compositions, such as ribozymes, that are capable of sequence-specific hybridization to HTPL transcripts, are also useful in therapy. *See, e.g.,* Phylactou, *Adv. Drug Deliv. Rev.* 44(2-3):97-108 (2000); Phylactou *et al., Hum. Mol. Genet.* 7(10):1649-53 (1998); Rossi, *Ciba Found. Symp.* 209:195-204 (1997); and Sigurdsson *et al., Trends Biotechnol.* 13(8):286-9 (1995), the disclosures of which are incorporated herein by reference in their entireties.

Other nucleic acids useful in the therapeutic methods of the present invention are those that are capable of triplex helix formation in or near the HTPL genomic locus. Such triplexing oligonucleotides are able to inhibit transcription, Intody *et al.,* Nucleic Acids Res. 28(21):4283-90 (2000); McGuffie *et al., Cancer Res.* 60(14):3790-9 (2000), the disclosures of which are incorporated herein by reference, and pharmaceutical compositions comprising such triplex forming oligos (TFOs) are administered in circumstances in which excessive production, or production of aberrant protein, is a pathophysiologic basis of disease.

In another embodiment of the therapeutic methods of the present invention, a therapeutically effective amount of a pharmaceutical composition comprising an antibody (including fragment or derivative thereof) of the present invention is administered. As is well known, antibody compositions are administered, for example, to antagonize activity of HTPL, or to target therapeutic agents to sites of HTPL presence and/or accumulation.

In another embodiment of the therapeutic methods of the present invention, a pharmaceutical composition comprising a non-antibody antagonist of HTPL is administered. Antagonists of HTPL can be produced using methods generally known in the art. In particular, purified HTPL can be used to screen libraries of pharmaceutical agents, often combinatorial libraries of small molecules, to identify those that specifically bind and antagonize at least one activity of HTPL.

In other embodiments a pharmaceutical composition comprising an agonist of HTPL is administered. Agonists can be identified using methods analogous to those used to identify antagonists.

In still other therapeutic methods of the present invention, pharmaceutical compositions comprising host cells that express HTPL, fusions, or fragments thereof can be administered.

In such cases, the cells are typically autologous, so as to circumvent xenogeneic or allotypic rejection, and are administered to complement defects in HTPL production or activity.

In other embodiments, pharmaceutical compositions comprising the HTPL proteins, nucleic acids, antibodies, antagonists, and agonists of the present invention can be administered in combination with other appropriate therapeutic agents. Selection of the appropriate agents for use in combination therapy can be made by one of ordinary skill in the art according to conventional pharmaceutical principles. The combination of therapeutic agents or approaches can act additively or synergistically to effect the treatment or prevention of the various disorders described above, providing greater therapeutic efficacy and/or permitting use of the pharmaceutical compositions of the present invention using lower dosages, reducing the potential for adverse side effects.

### TRANSGENIC ANIMALS AND CELLS

In another aspect, the invention provides transgenic cells and non-human organisms comprising HTPL isoform nucleic acids, and transgenic cells and non-human organisms with targeted disruption of the endogenous orthologue of the human HTPL gene.

The cells can be embryonic stem cells or somatic cells. The transgenic non-human organisms can be chimeric, nonchimeric heterozygotes, and nonchimeric homozygotes.

### DIAGNOSTIC METHODS

The nucleic acids of the present invention can be used as nucleic acid probes to assess the levels of HTPL mRNA in testis or other disease tissue or cells, and antibodies of the present invention can be used to assess the expression levels of HTPL proteins in testis or other disease tissue or cells to diagnose male infertility or cancer.

The following examples are offered for purpose of illustration, not limitation.

### EXAMPLE 1

### Identification and Characterization of cDNAs Encoding HTPL Proteins

Predicating our gene discovery efforts on use of genome-derived single exon probes and hybridization to genome-derived single exon microarrays - an approach that we have previously demonstrated will readily identify novel genes that have proven refractory to mRNA-based identification efforts - we identified an exon in raw human genomic sequence that is particularly expressed in human adrenal, adult and fetal liver, bone marrow, brain, kidney, lung, placenta and prostate.

Briefly, bioinformatic algorithms were applied to human genomic sequence data to identify putative exons. Each of the predicted exons was amplified from genomic DNA, typically centering the putative coding sequence within a larger amplicon that included flanking noncoding sequence. These genome-derived single exon probes were arrayed on a support and expression of the bioinformatically predicted exons assessed through a series of simultaneous two-color hybridizations to the genome-derived single exon microarrays.

The approach and procedures are further described in detail in Penn *et al.*, "Mining the Human Genome using Microarrays of Open Reading Frames," *Nature Genetics* 26:315-318 (2000); commonly owned and copending U.S. patent application nos. 09/864,761, filed May 23, 2001, 09/774,203, filed January 29, 2001, and 09/632,366, filed August 3, 2000, the disclosures of which are incorporated herein by reference in their entireties.

Using a graphical display particularly designed to facilitate computerized query of the resulting exon-specific expression data, as further described in commonly owned and copending U.S. patent application no. 09/864,761, filed May 23, 2001, 09/774,203, filed January 29, 2001 and 09/632,366, filed August 3, 2000, the disclosures of which are incorporated herein by reference in their entireties, one exon was identified that is expressed in all the human tissues tested; subsequent analysis revealed that the exon represent a gene.

Table 1 summarizes the microarray expression data obtained using genome-derived single exon probe corresponding to exon four. The probe was completely sequenced on both strands prior to its use on a genome-derived single exon microarray; sequencing confirmed the exact chemical structure of the probe. An added benefit of sequencing is that it placed us in possession of a set of single base-incremented fragments of the sequenced nucleic acid, starting from the sequencing primer's 3' OH. (Since the single exon probe was first obtained by PCR amplification from genomic DNA, we were of course additionally in possession of an even larger set of single base incremented fragments of the single exon probe, each fragment corresponding to an extension product from one of the two amplification primers.)

Signals and expression ratios are normalized values measured and calculated as further described in commonly owned and copending U.S. patent application nos. 09/864,761, filed May 23, 2001, 09/774,203, filed January 29, 2001, 09/632,366, filed August 3, 2000, and U.S. provisional patent application no. 60/207,456, filed May 26, 2000, the disclosures of which are incorporated herein by reference in their entireties.

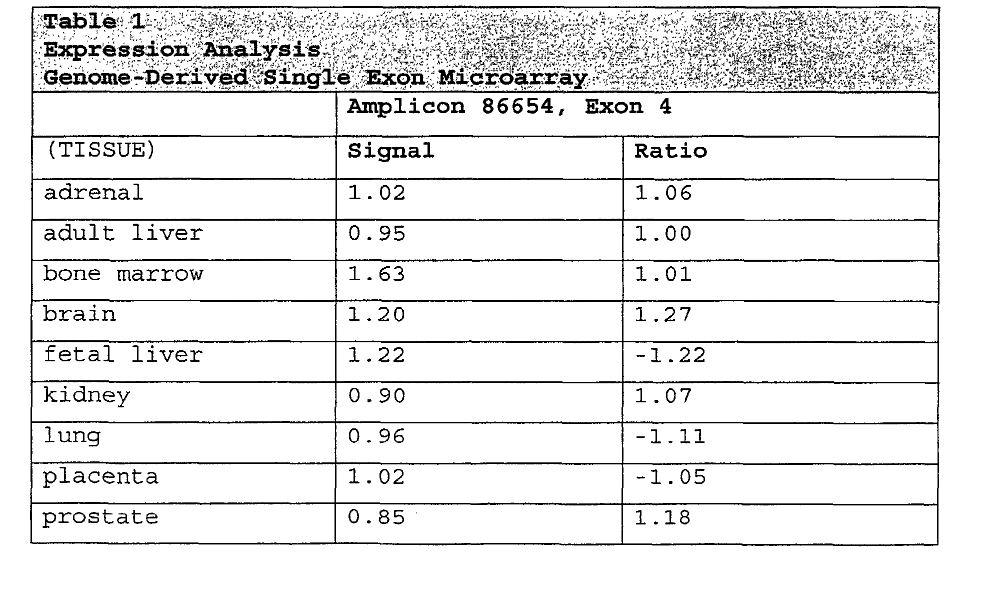

As shown in Table 1, low level expression of exon four was seen in adrenal, adult and fetal liver, bone marrow, brain, kidney, lung, placenta and prostate. Low level expression of HTPL in these tissues was further confirmed by RT_PCR analysis (see below).

Rapid Amplification of cDNA ends (RACE, Clontech Laboratories, Palo Alto, CA) and direct RT_PCR were used to obtain the coding region of the human cDNA sequence. The final pair of primers used to amplify the 3.3 kb transcript were: 62NF3 (5'-CAGGAAACCGTCTGGTGGGATCTC-3') and 62487Rend (5'-CTGAGACGGAGTCTCATTCTTGTCACC-3'). Human testis Marathon ready cDNA (Clontech Laboratories) was used to clone the entire coding region. The PCR parameters were as follows: 94°C 1 min; (94°C 10 seconds; 64°C 30 seconds; 72°C 3 min.) for 35 cycles. The PCR reaction composition was as follows: 5 ul of cDNA; 5 ul of 10X amplification buffer; 1 ul dNTP(10 M); 3 ul of primer pairs; 1 ul of Taq polymerase Mix and 35 ul double distilled water.

The PCR product was cloned into a pGEM-Teasy vector and inserts of multiple clones were sequenced on both strands using a MegaBACE™ automatic sequencer (Molecular Dynamics, Sunnyvale, CA). Single base pair sequence changes were identified between two groups of clones, and each group was recognized as one isoform. The presence of two isoforms was confirmed by the presence of two genomic clones (see below), in the public database, with the same single base pair sequence changes between them. For reasons described below, we named the two isoforms HTPL-L and HTPL-S. Sequencing both strands provided us with the exact chemical structure of the cDNA, which are shown in FIG. 3 and FIG. 4 and further presented in the SEQUENCE LISTING as SEQ ID NOs: 1 and 4, and placed us in actual physical possession of the entire set of single-base incremented fragments of the sequenced clones, starting at the 5' and 3' termini.

As shown in FIG. 3, the HTPL-L cDNA spans 3296 nucleotides and contains an open reading frame from nucleotide 78 through and including nt 2942 (inclusive of termination codon), predicting a protein of 954 amino acids with a (posttranslationally unmodified) molecular weight of 107.6 kD. The clone appears full length, with the reading frame opening starting with a methionine and terminating with a stop codon.

As shown in FIG. 4, the HTPL-S cDNA span 3298 nucleotides and contains an open reading frame from nucleotide 78 through and including nt 2381 (inclusive of termination codon), predicting a protein of 767 amino acids with a (posttranslationally unmodified) molecular weight of 86.9 kD. The clone appears full length, with the reading frame opening starting with a methionine and terminating with a stop codon.

BLAST query of genomic sequence identified one BAC, spanning 16.7 kb, which constitutes the minimum set of clones encompassing the cDNA sequences. Based upon the known origin of the BAC (GenBank accession number AC005875.2), the HTPL gene can be mapped to human chromosome 10p12.1.

Comparison of the cDNA and genomic sequences identified four exons for HTPL. Exon organization for HTPL is listed in Table 2.

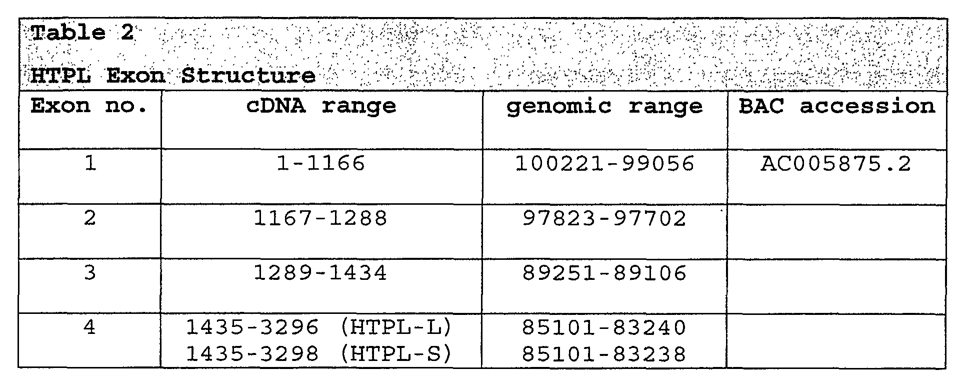

FIG. 2 schematizes the exon organization of the HTPL gene.

At the top is shown the bacterial artificial chromosome (BAC), with GenBank accession number (AC005875.2), that span the HTPL locus. The genome-derived single-exon probe first used to demonstrate expression from this locus is shown below the BAC and labeled "500". The 500 bp probe includes sequence drawn solely from exon four.

As shown in FIG. 2, two HTPL isoforms have been identified. Both isoforms contain four exons, with a few single base pair differences between them (FIG. 3 and FIG. 4). Indeed, BAC AC005875.2 contains the same exonic sequence as HTPL-L, while BAC AL355493.1 contains the same exonic sequence as HTPL-S. The longer isoform, HTPL-L, encodes a protein of 954 amino acids that has a predicted molecular weight, prior to any post-translational modification, of 107.6 kD. One of the single base pair changes in the shorter form (HTPL-S) introduces a premature stop codon at position 2379 of the HTPL-S cDNA. HTPL-S, therefore, encodes a shortened protein of 767 amino acids that has a predicted molecular weight, prior to any post-translational modification, of 86.9 kD. Both cDNA clones appear full length, with the open reading frame starting with a methionine and terminating with a stop codon.

As further discussed in the examples herein, expression of HTPL was assessed using hybridization to genome-derived single exon microarrays. Microarray analysis of the fourth exon showed low level expression in all tissues tested, including adrenal, adult liver, bone marrow, brain, fetal liver, kidney, lung, placenta and prostate. This was confirmed by RT_PCR. RT_PCR also detected strong expression in testis and weak expression in colon and skeletal muscle (see Example 3).

The sequence of the HTPL cDNA was used as a BLAST query into the GenBank nr and dbEst databases. The nr database includes all non-redundant GenBank coding sequence translations, sequences derived from the 3-dimensional structures in the Brookhaven Protein Data Bank (PDB), sequences from SwissProt, sequences from the protein information resource (PIR), and sequences from protein research foundation (PRF). The dbEst (database of expressed sequence tags) includes ESTs, short, single pass read cDNA (mRNA) sequences, and cDNA sequences from differential display experiments and RACE experiments.

BLAST search identified a single human EST (AW665031.1), a single mouse EST (AV280614.1), and a single pig EST (AW436721.1) as having sequence closely related to HTPL.

Globally, the human HTPL proteins resemble a putative mouse transcript (GenBank accession: BAB29848, the HTPL-L protein with 66 % amino acid identity and 78 % amino acid similarity over the entire open reading frame).

Motif searches using Pfam (http://pfam.wustl.edu), SMART (http://smart.embl- heidelberg.de), and PROSITE pattern and profile databases (http://www.expasy.ch/prosite), identified several known domains shared with Patched, including the Patched domain and the Sterol-sensing domain.

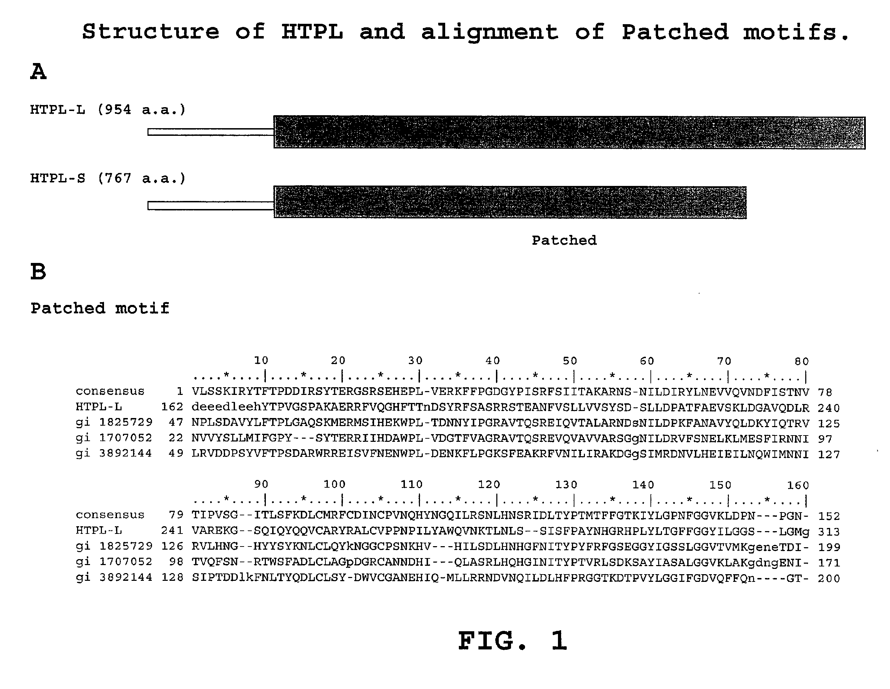

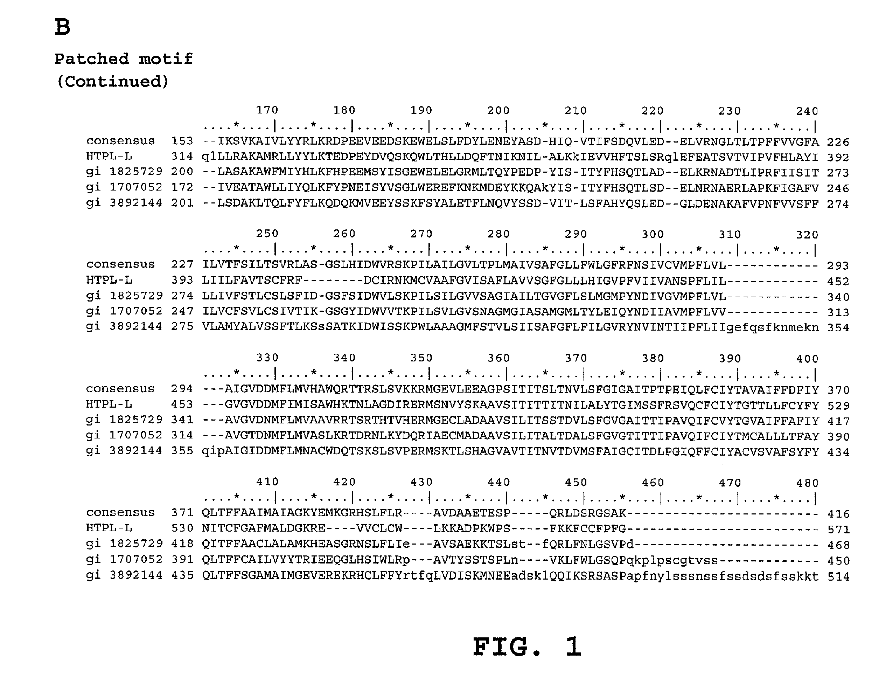

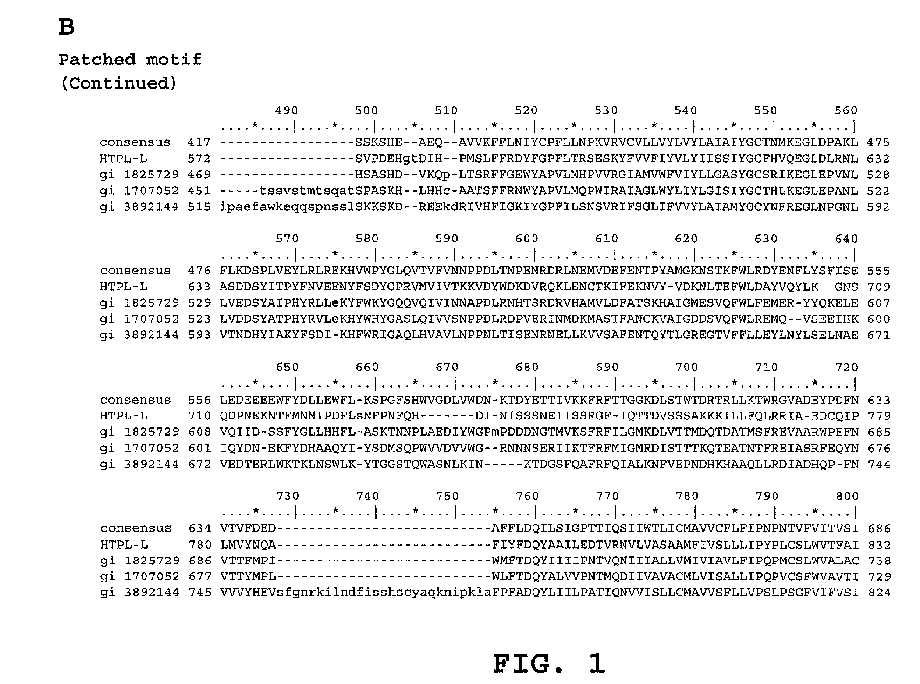

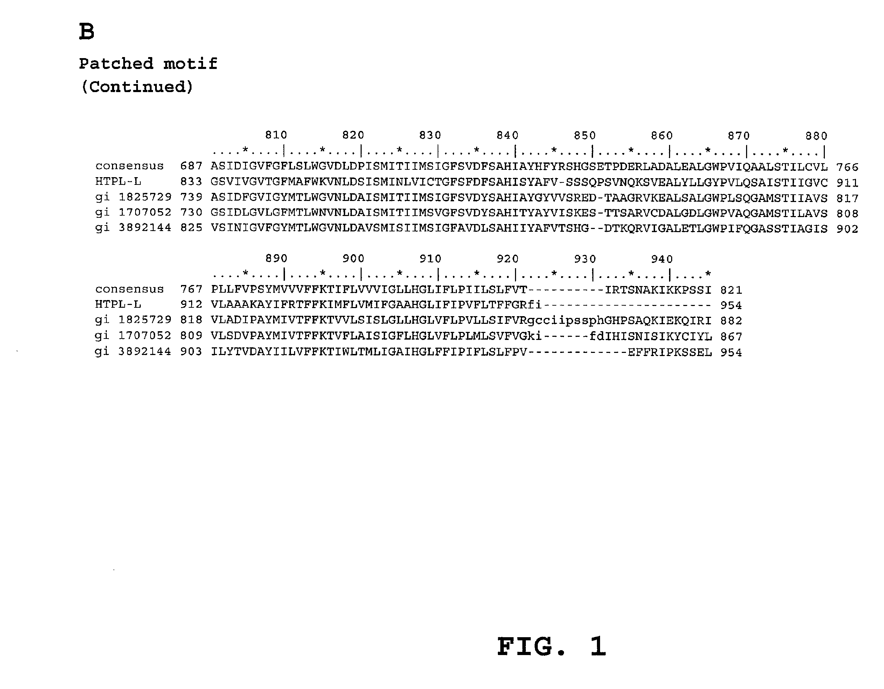

FIG. 1 shows the domain structure of HTPL-L and HTPL-S as well as the alignment of the Patched domain of HTPL-L with that of other protein.

As schematized in FIG. 1, HTPL shares an overall structural organization with the Patched protein. The shared structural features strongly imply that HTPL plays a role similar to that of Patched, in male germ cell development, and is a potential tumor suppressor.

Like Patched, HTPL-L contains a Patched domain (http://pfam.wustl.edu/hmmsearch.shtml), a Sterol-sensing domain (SSD, http://motif.genome.ad.jp/) and twelve transmembrane domains (http://smart.embl-heidelberg.de/smart/show_motifs.pl). The Patched domain in HTPL-L covers amino acid sequences 166 - 952 of HTPL-L. The SSD domain in HTPL-L covers amino acid sequences 383 - 540 of HTPL-L. The presence of these domains in HTPL-L suggest that HTPL-L, like Patched and other Patched domain containing proteins, is involved in the Hedgehog signaling pathway (see background section). Because of the premature stop of protein translation, HTPL-S contains a partial Patched domain, a complete Sterol-sensing motif and seven transmembrane domains. The presence of these domains in HTPL-S suggests that HTPL-S is also involved in the Hedgehog signaling pathway.

Other signatures of the newly isolated HTPL proteins were identified by searching the PROSITE database (http://www.expasy.ch/tools/scnpsitl.html), and the list below is for both HTPL-L and HTPL-S unless specified otherwise. These include seven N-glycosylation sites (192 - 195, 275 - 278, 279 - 282, 530 - 533, 678 - 681, 692 - 695 and 737 - 740), one cAMP- and cGMP-dependent protein kinase phosphorylation site (201 - 204), seven protein kinase C phosphorylation sites (194 - 196, 200 - 202, 508 - 510, 561 - 563, 662 - 664, 746 - 748, and 759 - 761; plus one for HTPL-L at 800 - 802), twelve Casein kinase II phosphorylation sites (19 - 22, 36 - 39, 62 - 65, 79 - 82, 190 - 193, 215 - 218, 219 - 222, 225 - 228, 230 - 233, 572 - 575, 597 - 600, and 740 - 743), two tyrosine kinase phosphorylation site (329 - 335, and 681 - 688; plus one for HTPL-L at 887 - 893), four N-myristoylation sites (307 - 312, 418 - 4223, 504 - 509, and 535 - 540; plus one for HTPL-L at 935 - 940), and a single amidation site at 541 -544.

Possession of the genomic sequence permitted search for promoter and other control sequences for the HTPL gene. A putative transcriptional control region, inclusive of promoter and downstream elements, was defined as 1 kb around the transcription start site, itself defined as the first nucleotide of the HTPL cDNA clone. The region, drawn from sequence of BAC AC005875.2, has the sequence given in SEQ ID NO: 23, which lists 1000 nucleotides before the transcription start site.

Transcription factor binding sites were identified using a web based program (http://motif.genome.ad.jp/), including binding sites for homeo domain factor Nkx-2.5/Csx (625 - 631 bp), for USF (891 - 898 bp) and for CdxA (399-405 and 612 - 618 bp, with numbering according to SEQ ID NO: 23), amongst others.

We have thus identified a newly described human gene, HTPL, which shares certain protein domains and an overall structural organization with Patched. The shared structural features strongly imply that the HTPL protein plays a role similar to Patched, in the hedgehog signaling pathway, functioning as a potential tumor suppressor and in germ cell development. This makes the HTPL proteins and nucleic acids clinically useful diagnostic markers and potential therapeutic agents for male infertility and cancer.

### EXAMPLE 2

### Preparation and Labeling of Useful Fragments of HTPL

Useful fragments of HTPL are produced by PCR, using standard techniques, or solid phase chemical synthesis using an automated nucleic acid synthesizer. Each fragment is sequenced, confirming the exact chemical structure thereof.

The exact chemical structure of preferred fragments is provided in the attached SEQUENCE LISTING, the disclosure of which is incorporated herein by reference in its entirety. The following summary identifies the fragments whose structures are more fully described in the SEQUENCE LISTING:
SEQ ID NO: 1 (nt, full length HTPL-L cDMA)
SEQ ID NO: 2 (nt, cDNA ORF of HTPL-L)
SEQ ID NO: 3 (aa, full length HTPL-L protein)
SEQ ID NO: 4 (nt, full length HTPL-S cDNA)
SEQ ID NO: 5 (nt, cDNA ORF of HTPL-S)
SEQ ID NO: 6 (aa, full length HTPL-S protein)
SEQ ID NO: 7 (nt, (nt 1 - 2021) portion of HTPL-L)
SEQ ID NO: 8 (nt, 5' UT portion of SEQ ID NO: 7)
SEQ ID NO: 9 (nt, coding region of SEQ ID NO: 7)
SEQ ID NO: 10 (aa, (aa 1 - 648) CDS entirely within SEQ ID NO: 9)
SEQ ID NO: 11 (nt, (nt 2637 - 3041) portion of HTPL-L)
SEQ ID NO: 12 (nt, coding region of SEQ ID NO: 11)
SEQ ID NO: 13 (nt, 3' UT portion of SEQ ID NO: 11)
SEQ ID NO: 14 (aa, (aa 854 - 954) CDS entirely within SEQ ID NO: 12)
SEQ ID NO: 15 - 18 (nt, exons 1 - 4 of HTPL-L)
SEQ ID NO: 19 - 22 (nt, 500 bp genomic amplicons centered about exons 1 - 4 of HTPL-L)
SEQ ID NO: 23 (nt, 1000 bp putative promoter of HTPL)
SEQ ID NOs: 24 - 2028 (nt, 17-mers scanning SEQ ID NO: 7)
SEQ ID NOs: 2029 - 4025 (nt, 25-mers scanning SEQ ID NO: 7)
SEQ ID NOs: 4026 - 4414 (nt, 17-mers scanning SEQ ID NO: 11)
SEQ ID NOs: 4415 - 4795 (nt, 25-mers scanning SEQ ID NO: 11)
SEQ ID NO: 4796 (nt, (nt 1 - 2021) portion of HTPL-S)
SEQ ID NO: 4797 (nt, 5' UT portion of SEQ ID NO: 4796)
SEQ ID NO: 4798 (nt, coding region of SEQ ID NO: 4796)
SEQ ID NO: 4799 (aa, (aa 1 - 648) CDS entirely within SEQ ID NO: 4798)
SEQ ID NO: 4800 (nt, (nt 2637 - 3041) portion of HTPL-S)
SEQ ID NO: 4801 (nt, primer 62NF3 for cloning of HTPL)
SEQ ID NO: 4802 (nt, primer 62487Rend for cloning of HTPL)
SEQ ID NO: 4803 (nt, primer 62487Pu for RT PCR analysis of HTPL)
SEQ ID NO: 4804 (nt, primer 62487Pd for RT PCR analysis of HTPL)

Upon confirmation of the exact structure, each of the above-described nucleic acids of confirmed structure is recognized to be immediately useful as a HTPL-specific probe.

For use as labeled nucleic acid probes, the above-described HTPL nucleic acids are separately labeled by random priming. As is well known in the art of molecular biology, random priming places the investigator in possession of a near-complete set of labeled fragments of the template of varying length and varying starting nucleotide.

The labeled probes are used to identify the HTPL gene on a Southern blot, and are used to measure expression of HTPL mRNA on a northern blot and by RT-PCR, using standard techniques.

### EXAMPLE 3

### Expression Analysis of HTPL by RT_PCR

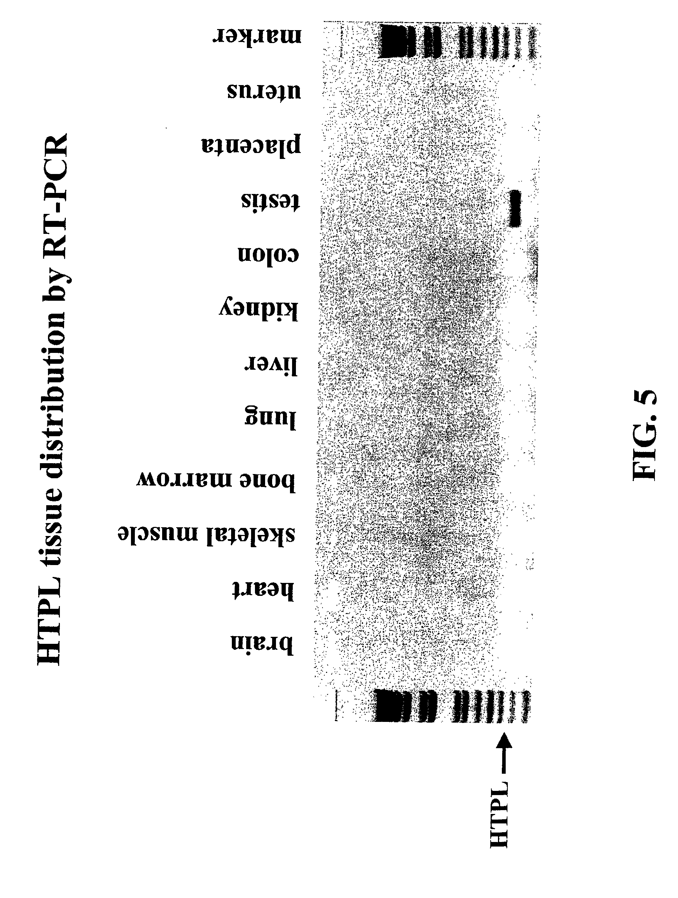

The Advantage 2 PCR amplification kit and PCR cDNA of different human tissues were obtained from Clontech Laboratories Inc. (Palo Alto, CA). The PCR parameters were set-up as follows, 94°C 15 seconds; 59°C 30 seconds; 72°C 40 seconds for 35 cycles. The PCR composition is as follows: 0.5 ul of cDNA; 2.5 ul of 10X amplification buffer; 0.5ul dNTP (10 M); 1 ul of primer pairs (10 M each; 62487Pu: 5'-ACATACGAGAGCGGATGTCCAA-3' and 62487Pd: 5'-GGGACAGAACCAAATGGGAAAC-3'); 0.5 ul of Advantage polymerase Mix in 25 ul reaction mixture. The amplified DNA products were resolved in 1.2 % agarose gel in TAE buffer. The gel was scanned using Typhoon™ Imaging System (Molecular Dynamics). HTPL is strongly expressed in testis, weakly expressed in skeletal muscle, bone marrow, lung, liver, kidney, colon and placenta, while hardly expressed in brain, heart and uterus (FIG. 5).

### EXAMPLE 4

### Production of HTPL Protein

The full length HTPL cDNA clone is cloned into the mammalian expression vector pcDNA3.1/HISA (Invitrogen, Carlsbad, CA, USA), transfected into COS7 cells, transfectants selected with G418, and protein expression in transfectants confirmed by detection of the anti-Xpress™ epitope according to manufacturer's instructions. Protein is purified using immobilized metal affinity chromatography and vector-encoded protein sequence is then removed with enterokinase, per manufacturer's instructions, followed by gel filtration and/or HPLC.

Following epitope tag removal, HTPL protein is present at a concentration of at least 70%, measured on a weight basis with respect to total protein (i.e., w/w), and is free of acrylamide monomers, bis acrylamide monomers, polyacrylamide and ampholytes. Further HPLC purification provides HTPL protein at a concentration of at least 95%, measured on a weight basis with respect to total protein (i.e., w/w).

### EXAMPLE 5

### Production of Anti-HTPL Antibody

Purified proteins prepared as in Example 4 are conjugated to carrier proteins and used to prepare murine monoclonal antibodies by standard techniques. Initial screening with the unconjugated purified proteins, followed by competitive inhibition screening using peptide fragments of the HTPL, identifies monoclonal antibodies with specificity for HTPL.

### EXAMPLE 6

### Use of HTPL Probes and Antibodies for Diagnosis of Male Infertility and Tumor

After informed consent is obtained, samples are drawn from testis or other disease tissue or cells and tested for HTPL mRNA levels by standard techniques and tested additionally for HTPL protein levels using anti- HTPL antibodies in a standard ELISA.

### EXAMPLE 7

### Use of HTPL Nucleic Acids, Proteins, and Antibodies in Therapy

Once over-expression of HTPL is detected in patients, HTPL antisense RNA or HTPL-specific antibody is introduced into disease cells to reduce the amount of the protein.

Once mutations of HTPL have been detected in patients, normal HTPL is reintroduced into the patient's disease cells by introduction of expression vectors that drive HTPL expression or by introducing HTPL proteins into cells. Antibodies for the mutated forms of HTPL are used to block the function of the abnormal forms of the protein.

### EXAMPLE 8

### HTPL Disease Associations

Diseases that map to the HTPL chromosomal region are shown in Table 3:

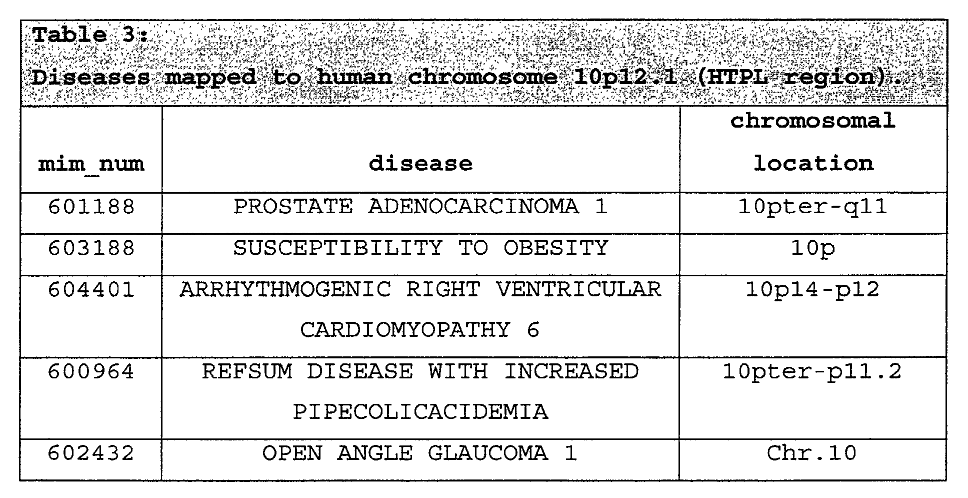

Prostate cancer is the second leading cause of male cancer deaths in the United States (http://www.ncbi.nlm.nih.gov/entrez/). A genetic locus, designated prostate adenocarcinoma 1 (PAC1), is involved in tumor suppression of human prostate carcinoma. Sanchez *et al., Proc. Nat. Acad. Sci.* 93:2551-2556 (1996). HTPL is a candidate gene for PAC1 as it maps to the same chromosomal region. Alternatively, mutations of HTPL could also lead to other diseases, such as those listed in Table 3.

All patents, patent publications, and other published references mentioned herein are hereby incorporated by reference in their entireties as if each had been individually and specifically incorporated by reference herein. While preferred illustrative embodiments of the present invention are described, one skilled in the art will appreciate that the present invention can be practiced by other than the described embodiments, which are presented for purposes of illustration only and not by way of limitation. The present invention is limited only by the claims that follow.

PB0177 – sequence listing

<110> Zhang, Jian

<120> HUMAN TESTIS EXPRESSED PATCHED LIKE PROTEIN

<130> AEOMICA-25

<160> 4804

<170> Aeomica Sequence Listing Engine

<210> 1
<211> 3296
<212> DNA
<213> Homo sapiens

<400> 1
caggaaaccg tctggtggga tctccgcagc tgcttttcac ctgctgttcc tcctgcgctt 60
cctaagagga agaatcaatg ccgtgggtgg agcccaagcc caggccgggg ccggagcaga 120
agcccaagct caccaaaccg gactctgcca ccgggccgca gtggtaccag gaatctcagg 180
aatcggagtc ggaaggcaag cagccacccc cgggacccct ggcacccccg aaatcccccg 240
aaccctcagg acccctggcg tcggagcagg atgcacccct gccagagggg gacgatgcac 300
ccccccggcc gtcgatgctg gacgatgcac cccgcctgcc gctggagctg gacgatgcac 360
ccctgccgga ggaggaaacc cccgaaccca cggccatctg caggcaccgg caccgctgtc 420
acaccgactg cctagagggg ctgctgtccc gcaccttcca gtggctgggg tggcaggtgg 480
gcgcgcaccc ctggatcttc ctgctggcgc ccttgatgct gacagccgcg ctgggcaccg 540
gcttcctgta cctacccaag gacgaagagg aagacctaga ggagcattac acccctgtgg 600
ggagcccggc caaggcggag cggcgcttcg tgcagggcca tttcaccacc aacgactcct 660
accgcttctc cgcctccagg aggagcaccg aagccaattt cgtctcgctt ctggtggtct 720
cctacagcga ctcactgctg gacccagcta cctttgcaga agtcagcaaa ctggacggcg 780
cggtgcagga tctgcgcgtg gcgcgggaaa agggaagcca gatccagtac cagcaggtgt 840
gcgcgaggta cagggcgctc tgcgtgcccc ccaacccgat cctgtacgcc tggcaggtga 900
acaaaacgct caacctgagc agcatctcct tccccgccta caaccacggc aggcatcccc 960
tctacctgac cggcttcttc ggaggataca tcttgggggg cagcctagga atgggccagt 1020
tactcctgcg ggccaaagcc atgcggctgc tgtactacct gaagaccgag gaccctgagt 1080
acgacgtgca gagcaagcag tggctcaccc atttgctcga tcaatttacc aacattaaga 1140
acatcttggc cttgaaaaaa attgaggtag tccactttac atcgctttcc agacaactgg 1200
aatttgaggc aacttctgtg actgtgatcc ctgtgtttca cctggcatac attctcatca 1260
ttctgtttgc agtcacatca tgctttaggt ttgactgcat acgaaacaaa atgtgtgttg 1320
cggcctttgg agtgatttct gctttcttgg cagtggtgag cggctttggc ctgctgttgc 1380
acattggggt gccatttgtc atcatagttg ccaattcacc atttcttatt ctaggtgttg 1440
gggtcgatga catgtttatc atgatttctg cctggcataa gaccaacctt gcaggtgaca 1500
tacgagagcg gatgtccaat gtctattcaa aagcggcagt gtctattaca atcaccacca 1560
tcactaacat cctggcctta tatacaggga ttatgagctc ttttaggtcc gtacaatgtt 1620
tttgcatcta tacaggaacg accctgttat tttgctattt ttataacatc acgtgttttg 1680
gagcatttat ggccttggat ggtaaaagag aagtagtctg cctatgctgg ttgaaaaagg 1740
ctgacccaaa atggccctca tttaaaaagt tctgctgttt cccatttggt tctgtcccag 1800
atgaacatgg aactgatatc catccaatga gtttgttttt tagagactat tttggcccct 1860
ttctcacaag gagtgagtcc aagtattttg tagtctttat atatgttttg tacatcataa 1920
gcagtatata tgggtgtttc catgtgcagg aaggtttaga ccttcgaaat ctggcaagtg 1980
acgattccta catcacacca tattttaacg tagaggagaa ttatttttca gattatggtc 2040
ccagggttat ggttattgtt actaaaaaag ttgactactg ggataaagat gttaggcaaa 2100
aactggaaaa ctgtactaaa atttttgaaa aaaatgtcta tgtagataaa aatcttacag 2160
agttttggtt agatgcatat gtgcaatatt taaaaggtaa cagccaagat cctaatgaga 2220
agaatacttt tatgaacaat attcctgatt ttttaagcaa ttttccaaat tttcagcatg 2280
atattaatat ttcttcatca aatgaaatca tttcttcccg gggcttcatt cagacaacag 2340
atgtttcttc ctcagccaaa aagaaaatat tgttattcca attacgacgc atagctgaag 2400
actgtcaaat tcccctaatg gtgtataacc aggcatttat atattttgat cagtatgctg 2460
caatattaga agacactgtt agaaatgtat tggttgcatc agcagctatg ttcattgttt 2520
ccttattgtt aatcccttat ccattgtgtt ccttgtgggt gacttttgct atcggttctg 2580
tgattgtagg ggtaacgggt ttcatggcat tctggaaagt caatcttgat tccatatcca 2640
tgattaatct tgtcatttgt acagggtttt ctttcgattt ttctgcacac atttcctatg 2700

catttgtttc tagttctcaa ccctcagtaa accaaaaatc agttgaggca ttgtatcttc 2760
taggctaccc agtgttacaa agtgcaattt caacaataat aggggtgtgt gttttagctg 2820
cagcgaaagc atacatcttc agaacatttt ttaagattat gtttcttgtt atgatatttg 2880
gggcagctca tggcctaatt tttattccag tattcttaac cttttttgga aggtttattt 2940
gaatatccac taacaagtca aagaccaatt ctagaattcc tgattgccct attccaatct 3000
gattttttta aatgcactat taagaatagt caacaaacta ctgggaggcc aaagtgggtg 3060
gatcacctga ggtcaagaat tcgagaccag cctggccaac atggtgaaac ctcgacacta 3120
ctgaaaatac aaaaattatc caggcatggt ggcgtgcacc tataatccca gctacctggg 3180
aggctgaggc aggagaatcg cttgaacctg ggagatggag gttgcagtga gctggagtgc 3240
accaggcact ccagcctggg tgacaagaat gagactccgt ctcagaaaaa aaaaaa 3296

<210> 2
<211> 2865
<212> DNA
<213> Homo sapiens

<400> 2
atgccgtggg tggagcccaa gcccaggccg gggccggagc agaagcccaa gctcaccaaa 60
ccggactctg ccaccgggcc gcagtggtac caggaatctc aggaatcgga gtcggaaggc 120
aagcagccac ccccgggacc cctggcaccc ccgaaatccc ccgaaccctc aggacccctg 180
gcgtcggagc aggatgcacc cctgccagag ggggacgatg cacccccccg gccgtcgatg 240
ctggacgatg caccccgcct gccgctggag ctggacgatg cacccctgcc ggaggaggaa 300
acccccgaac ccacggccat ctgcaggcac cggcaccgct gtcacaccga ctgcctagag 360
gggctgctgt cccgcacctt ccagtggctg gggtggcagg tgggcgcgca cccctggatc 420
ttcctgctgg cgcccttgat gctgacagcc gcgctgggca ccggcttcct gtacctaccc 480
aaggacgaag aggaagacct agaggagcat tacacccctg tggggagccc ggccaaggcg 540
gagcggcgct tcgtgcaggg ccatttcacc accaacgact cctaccgctt ctccgcctcc 600
aggaggagca ccgaagccaa tttcgtctcg cttctggtgg tctcctacag cgactcactg 660
ctggacccag ctacctttgc agaagtcagc aaactggacg gcgcggtgca ggatctgcgc 720
gtggcgcggg aaaagggaag ccagatccag taccagcagg tgtgcgcgag gtacagggcg 780
ctctgcgtgc cccccaaccc gatcctgtac gcctggcagg tgaacaaaac gctcaacctg 840
agcagcatct ccttccccgc ctacaaccac ggcaggcatc ccctctacct gaccggcttc 900
ttcggaggat acatcttggg gggcagccta ggaatgggcc agttactcct gcgggccaaa 960
gccatgcggc tgctgtacta cctgaagacc gaggaccctg agtacgacgt gcagagcaag 1020
cagtggctca cccatttgct cgatcaattt accaacatta agaacatctt ggccttgaaa 1080
aaaattgagg tagtccactt tacatcgctt tccagacaac tggaatttga ggcaacttct 1140
gtgactgtga tccctgtgtt tcacctggca tacattctca tcattctgtt tgcagtcaca 1200
tcatgcttta ggtttgactg catacgaaac aaaatgtgtg ttgcggcctt tggagtgatt 1260
tctgctttct tggcagtggt gagcggcttt ggcctgctgt tgcacattgg ggtgccattt 1320
gtcatcatag ttgccaattc accatttctt attctaggtg ttggggtcga tgacatgttt 1380
atcatgattt ctgcctggca taagaccaac cttgcaggtg acatacgaga gcggatgtcc 1440
aatgtctatt caaaagcggc agtgtctatt acaatcacca ccatcactaa catcctggcc 1500
ttatatacag ggattatgag ctcttttagg tccgtacaat gtttttgcat ctatacagga 1560
acgaccctgt tattttgcta tttttataac atcacgtgtt ttggagcatt tatggccttg 1620
gatggtaaaa gagaagtagt ctgcctatgc tggttgaaaa aggctgaccc aaaatggccc 1680
tcatttaaaa agttctgctg tttcccattt ggttctgtcc cagatgaaca tggaactgat 1740
atccatccaa tgagtttgtt ttttagagac tattttggcc cctttctcac aaggagtgag 1800
tccaagtatt ttgtagtctt tatatatgtt ttgtacatca taagcagtat atatgggtgt 1860
ttccatgtgc aggaaggttt agaccttcga aatctggcaa gtgacgattc ctacatcaca 1920
ccatatttta acgtagagga gaattatttt tcagattatg gtcccagggt tatggttatt 1980
gttactaaaa aagttgacta ctgggataaa gatgttaggc aaaaactgga aaactgtact 2040
aaaatttttg aaaaaaatgt ctatgtagat aaaaatctta cagagttttg gttagatgca 2100
tatgtgcaat atttaaaagg taacagccaa gatcctaatg agaagaatac ttttatgaac 2160
aatattcctg attttttaag caattttcca aattttcagc atgatattaa tatttcttca 2220
tcaaatgaaa tcatttcttc ccggggcttc attcagacaa cagatgtttc ttcctcagcc 2280
aaaaagaaaa tattgttatt ccaattacga cgcatagctg aagactgtca aattccccta 2340
atggtgtata accaggcatt tatatatttt gatcagtatg ctgcaatatt agaagacact 2400
gttagaaatg tattggttgc atcagcagct atgttcattg tttccttatt gttaatccct 2460
tatccattgt gttccttgtg ggtgactttt gctatcggtt ctgtgattgt aggggtaacg 2520
ggtttcatgg cattctggaa agtcaatctt gattccatat ccatgattaa tcttgtcatt 2580
tgtacagggt tttctttcga tttttctgca cacatttcct atgcatttgt ttctagttct 2640

caaccctcag taaaccaaaa atcagttgag gcattgtatc ttctaggcta cccagtgtta 2700
caaagtgcaa tttcaacaat aataggggtg tgtgttttag ctgcagcgaa agcatacatc 2760
ttcagaacat tttttaagat tatgtttctt gttatgatat ttggggcagc tcatggccta 2820
atttttattc cagtattctt aacctttttt ggaaggttta tttga 2865

<210> 3
<211> 954
<212> PRT
<213> Homo sapiens

<400> 3
Met Pro Trp Val Glu Pro Lys Pro Arg Pro Gly Pro Glu Gln Lys Pro
1               5                   10                  15

Lys Leu Thr Lys Pro Asp Ser Ala Thr Gly Pro Gln Trp Tyr Gln Glu
            20                  25                  30

Ser Gln Glu Ser Glu Ser Glu Gly Lys Gln Pro Pro Pro Gly Pro Leu
        35                  40                  45

Ala Pro Pro Lys Ser Pro Glu Pro Ser Gly Pro Leu Ala Ser Glu Gln
    50                  55                  60

Asp Ala Pro Leu Pro Glu Gly Asp Asp Ala Pro Pro Arg Pro Ser Met
65                  70                  75                  80

Leu Asp Asp Ala Pro Arg Leu Pro Leu Glu Leu Asp Asp Ala Pro Leu
                85                  90                  95

Pro Glu Glu Glu Thr Pro Glu Pro Thr Ala Ile Cys Arg His Arg His
            100                 105                 110

Arg Cys His Thr Asp Cys Leu Glu Gly Leu Leu Ser Arg Thr Phe Gln
        115                 120                 125

Trp Leu Gly Trp Gln Val Gly Ala His Pro Trp Ile Phe Leu Leu Ala
    130                 135                 140

Pro Leu Met Leu Thr Ala Ala Leu Gly Thr Gly Phe Leu Tyr Leu Pro
145                 150                 155                 160

Lys Asp Glu Glu Glu Asp Leu Glu Glu His Tyr Thr Pro Val Gly Ser
                165                 170                 175

Pro Ala Lys Ala Glu Arg Arg Phe Val Gln Gly His Phe Thr Thr Asn
            180                 185                 190

Asp Ser Tyr Arg Phe Ser Ala Ser Arg Arg Ser Thr Glu Ala Asn Phe
        195                 200                 205

Val Ser Leu Leu Val Val Ser Tyr Ser Asp Ser Leu Leu Asp Pro Ala
    210                 215                 220

Thr Phe Ala Glu Val Ser Lys Leu Asp Gly Ala Val Gln Asp Leu Arg
225                 230                 235                 240

Val Ala Arg Glu Lys Gly Ser Gln Ile Gln Tyr Gln Gln Val Cys Ala
                245                 250                 255

Arg Tyr Arg Ala Leu Cys Val Pro Pro Asn Pro Ile Leu Tyr Ala Trp
            260                 265                 270

Gln Val Asn Lys Thr Leu Asn Leu Ser Ser Ile Ser Phe Pro Ala Tyr
        275                 280                 285

Asn His Gly Arg His Pro Leu Tyr Leu Thr Gly Phe Phe Gly Gly Tyr
    290                 295                 300

Ile Leu Gly Gly Ser Leu Gly Met Gly Gln Leu Leu Leu Arg Ala Lys
305                 310                 315                 320

Ala Met Arg Leu Leu Tyr Tyr Leu Lys Thr Glu Asp Pro Glu Tyr Asp
                325                 330                 335

Val Gln Ser Lys Gln Trp Leu Thr His Leu Leu Asp Gln Phe Thr Asn
            340                 345                 350

Ile Lys Asn Ile Leu Ala Leu Lys Lys Ile Glu Val Val His Phe Thr
        355                 360                 365

Ser Leu Ser Arg Gln Leu Glu Phe Glu Ala Thr Ser Val Thr Val Ile
    370                 375                 380

Pro Val Phe His Leu Ala Tyr Ile Leu Ile Ile Leu Phe Ala Val Thr
385                 390                 395                 400

Ser Cys Phe Arg Phe Asp Cys Ile Arg Asn Lys Met Cys Val Ala Ala
                405                 410                 415

Phe Gly Val Ile Ser Ala Phe Leu Ala Val Val Ser Gly Phe Gly Leu
            420                 425                 430

Leu Leu His Ile Gly Val Pro Phe Val Ile Ile Val Ala Asn Ser Pro
        435                 440                 445

Phe Leu Ile Leu Gly Val Gly Val Asp Asp Met Phe Ile Met Ile Ser
    450                 455                 460

Ala Trp His Lys Thr Asn Leu Ala Gly Asp Ile Arg Glu Arg Met Ser
465                 470                 475                 480

Asn Val Tyr Ser Lys Ala Ala Val Ser Ile Thr Ile Thr Thr Ile Thr
                485                 490                 495

Asn Ile Leu Ala Leu Tyr Thr Gly Ile Met Ser Ser Phe Arg Ser Val
            500                 505                 510

Gln Cys Phe Cys Ile Tyr Thr Gly Thr Thr Leu Leu Phe Cys Tyr Phe
        515                 520                 525

Tyr Asn Ile Thr Cys Phe Gly Ala Phe Met Ala Leu Asp Gly Lys Arg
    530                 535                 540

Glu Val Val Cys Leu Cys Trp Leu Lys Lys Ala Asp Pro Lys Trp Pro
545                 550                 555                 560

Ser Phe Lys Lys Phe Cys Cys Phe Pro Phe Gly Ser Val Pro Asp Glu
                565                 570                 575

His Gly Thr Asp Ile His Pro Met Ser Leu Phe Phe Arg Asp Tyr Phe
            580                 585                 590

Gly Pro Phe Leu Thr Arg Ser Glu Ser Lys Tyr Phe Val Val Phe Ile
        595                 600                 605

Tyr Val Leu Tyr Ile Ile Ser Ser Ile Tyr Gly Cys Phe His Val Gln
    610                 615                 620

Glu Gly Leu Asp Leu Arg Asn Leu Ala Ser Asp Asp Ser Tyr Ile Thr
625                 630                 635                 640

Pro Tyr Phe Asn Val Glu Glu Asn Tyr Phe Ser Asp Tyr Gly Pro Arg
                645                 650                 655

Val Met Val Ile Val Thr Lys Lys Val Asp Tyr Trp Asp Lys Asp Val
            660                 665                 670

Arg Gln Lys Leu Glu Asn Cys Thr Lys Ile Phe Glu Lys Asn Val Tyr
        675                 680                 685

Val Asp Lys Asn Leu Thr Glu Phe Trp Leu Asp Ala Tyr Val Gln Tyr
    690                 695                 700

Leu Lys Gly Asn Ser Gln Asp Pro Asn Glu Lys Asn Thr Phe Met Asn
705                 710                 715                 720

Asn Ile Pro Asp Phe Leu Ser Asn Phe Pro Asn Phe Gln His Asp Ile
                725                 730                 735

Asn Ile Ser Ser Ser Asn Glu Ile Ile Ser Ser Arg Gly Phe Ile Gln
            740                 745                 750

Thr Thr Asp Val Ser Ser Ser Ala Lys Lys Lys Ile Leu Leu Phe Gln
        755                 760                 765

Leu Arg Arg Ile Ala Glu Asp Cys Gln Ile Pro Leu Met Val Tyr Asn
    770                 775                 780

Gln Ala Phe Ile Tyr Phe Asp Gln Tyr Ala Ala Ile Leu Glu Asp Thr
785                 790                 795                 800

Val Arg Asn Val Leu Val Ala Ser Ala Ala Met Phe Ile Val Ser Leu
                805                 810                 815

Leu Leu Ile Pro Tyr Pro Leu Cys Ser Leu Trp Val Thr Phe Ala Ile
            820                 825                 830

Gly Ser Val Ile Val Gly Val Thr Gly Phe Met Ala Phe Trp Lys Val
        835                 840                 845

Asn Leu Asp Ser Ile Ser Met Ile Asn Leu Val Ile Cys Thr Gly Phe
    850                 855                 860

Ser Phe Asp Phe Ser Ala His Ile Ser Tyr Ala Phe Val Ser Ser Ser
865                 870                 875                 880

Gln Pro Ser Val Asn Gln Lys Ser Val Glu Ala Leu Tyr Leu Leu Gly
                885                 890                 895

Tyr Pro Val Leu Gln Ser Ala Ile Ser Thr Ile Ile Gly Val Cys Val
            900                 905                 910

Leu Ala Ala Ala Lys Ala Tyr Ile Phe Arg Thr Phe Phe Lys Ile Met

915                 920                 925

Phe Leu Val Met Ile Phe Gly Ala Ala His Gly Leu Ile Phe Ile Pro
    930                 935                 940

Val Phe Leu Thr Phe Phe Gly Arg Phe Ile
945                 950

<210> 4
<211> 3298
<212> DNA
<213> Homo sapiens

<400> 4
caggaaaccg tctggtggga tctccgcagc tgcttttcac ctgctgttcc tcctgcgctt 60
cctaagagga agaatcaatg ccgtgggtgg agcccaagcc caggccgggg ccggagcaga 120
agcccaagct caccaaaccg gactctgcca ccgggccgca gtggtaccag gaatctcagg 180
aatcggagtc ggaaggcaag cagccacccc cgggacccct ggcacccccg aaatcccccg 240
aaccctcagg acccctggcg tcggagcagg atgcacccct gccagagggg gacgatgcac 300
ccccccggcc gtcgatgctg gacgatgcac cccgcctgcc gctggagctg gacgatgcac 360
ccctgccgga ggaggaaacc cccgaaccca cggccatctg caggcaccgg caccgctgtc 420
acaccgactg cctagagggg ctgctgtccc gcaccttcca gtggctgggg tggcaggtgg 480
gcgcgcaccc ctggatcttc ctgctggcgc ccttgatgct gacagccgcg ctgggcaccg 540
gcttcctgta cctacccaag gacgaagagg aagacctaga ggagcattac acccctgtgg 600
ggagcccggc caaggcggag cggcgcttcg tgcagggcca tttcaccacc aacgactcct 660
accgcttctc cgcctccagg aggagcaccg aagccaattt cgtctcgctt ctggtggtct 720
cctacagcga ctcactgctg gacccagcta cctttgcaga agtcagcaaa ctggacggcg 780
cggtgcagga tctgcgcgtg gcgcgggaaa agggaagcca gatccagtac cagcaggtgt 840
gcgcgaggta cagggcgctc tgcgtgcccc ccaacccgat cctgtacgcc tggcaggtga 900
acaaaacgct caacctgagc agcatctcct tccccgccta caaccacggc aggcatcccc 960
tctacctgac cggcttcttc ggaggataca tcttgggggg cagcctagga atgggccagt 1020
tactcctgcg ggccaaagcc atgcggctgc tgtactacct gaagaccgag gaccctgagt 1080
acgacgtgca gagcaagcag tggctcaccc atttgctcga tcaatttacc aacattaaga 1140
acatcttggc cttgaaaaaa attgaggtag tccactttac atcgctttcc agacaactgg 1200
aatttgaggc aacttctgtg actgtgatcc ctgtgtttca cctggcatac attctcatca 1260
ttctgtttgc agtcacatca tgctttaggt ttgactgcat acgaaacaaa atgtgtgttg 1320
cggcctttgg agtgatttct gctttcttgg cagtggtgag cggctttggc ctgctgttgc 1380
acattggggt gccatttgtc atcatagttg ccaattcacc atttcttatt ctaggtgttg 1440
gggtcgatga catgtttatc atgatttctg cctggcataa gaccaacctt gcagatgaca 1500
tacgagagcg gatgtccaat gtctattcaa aagcggcagt gtctattaca atcaccacca 1560
tcactaacat cctggcctta tatacaggga ttatgagctc ttttaggtct gtacaatgtt 1620
tttgcatcta tacaggaatg accctgttat tttgctattt ttataacatc acgtgttttg 1680
gagcatttat ggccttggat ggtaaaagag aagtagtctg cctatgctgg ttgaaaaagg 1740
ctgacccaaa atggccctca tttaaaaagt tctgctgttt cccatttggt tctgtcccag 1800
atgaacatgg aactgatatc catccaataa gtttgttttt tagagactat tttggcccct 1860
ttctcacaag gagtgagtcc aagtattttg tagtctttat atatgttttg tacatcataa 1920
gcagtatata tgggtgtttc catgtgcagg aaggtttaga ccttcgaaat ctggcaagtg 1980
acgattccta catcacacca tattttaacg tagaggagaa ttatttttca gattatggtc 2040
ccagggttat ggttattgtt actaaaaaag ttgactactg ggataaagat gttaggcaaa 2100
aactggaaaa ctgtactaaa atttttgaaa aaaatgtcta tgtagataaa aatcttacag 2160
agttttggtt agatgcatat gtgcaatatt taaaaggtaa cagccaagat cctaatgaga 2220
agaatacttt tatgaacaat attcctgatt ttttaagcaa ttttccaaat tttcagcatg 2280
atattaatat ttcttcatca aatgaaatca tttcttcccg gggcttcatt cagacaacag 2340
atgtttcttc ctcagccaaa aagaaaatat tgttattcta attacgacgc atagctgaag 2400
actgtcaaat tcccctaatg gtgtataacc aggcatttat atattttgat cagtatgctg 2460
caatattaga agacactgtt agaaatgtat tggttgcatc agcagctatg ttcattgttt 2520
ccttattgtt aatcccttat ccattgtgtt ccttgtgggt gacttttgct attggttctg 2580
tgattgtagg ggtaacgggt ttcatggcat tctggaaagt caatcttgat tccatatcca 2640
tgattaatct tgtcatttgt atagggtttt ctttcgattt ttctgtacac atttcctatg 2700
catttgtttc tagttctcaa ccctcagtaa accaaaaatc agttgaggca ttgtatcttc 2760
taggctaccc agtgttacaa agtgcaattt caacaataat aggggtgtgt gttttagctg 2820

cagcgaaagc atacatcttc agaacatttt ttaagattat gtttcttgtt atgatatttg 2880
gggcagctca tggcctaatt tttattccag tattcttaac cttttttgga aggtttattt 2940
gaatatccac taacaagtca aagaccaatt ctagaattcc tgattgccct attccaatct 3000
gattttttta aatgcactat taagaatagt caacaaacta ctgggaggcc aaggtgggtg 3060
gatcacctga ggtcaagaat tcgagaccag cctggccaac atggtgaaac ctcgacacta 3120
ctgaaaatac aaaaattatc caggcatggt ggcgtgcacc tataatccca gctacctggg 3180
aggctgaggc aggagaatcg cttgaacctg ggagatggag gttgcagtga gctggagtgc 3240
accaggcact ccagcctggg tgacaagaat gagactccgt ctcagaaaaa aaaaaaaa 3298

<210> 5
<211> 2304
<212> DNA
<213> Homo sapiens

<400> 5
   atgccgtggg tggagcccaa gcccaggccg gggccggagc agaagcccaa gctcaccaaa 60
   ccggactctg ccaccgggcc gcagtggtac caggaatctc aggaatcgga gtcggaaggc 120
   aagcagccac ccccgggacc cctggcaccc ccgaaatccc ccgaaccctc aggacccctg 180
   gcgtcggagc aggatgcacc cctgccagag ggggacgatg cacccccccg gccgtcgatg 240
   ctggacgatg caccccgcct gccgctggag ctggacgatg cacccctgcc ggaggaggaa 300
   acccccgaac ccacggccat ctgcaggcac cggcaccgct gtcacaccga ctgcctagag 360
   gggctgctgt cccgcacctt ccagtggctg ggtggcaggt tgggcgcgca cccctggatc 420
   ttcctgctgg cgcccttgat gctgacagcc gcgctgggca ccggcttcct gtacctaccc 480
   aaggacgaag aggaagacct agaggagcat tacacccctg tggggagccc ggccaaggcg 540
   gagcggcgct tcgtgcaggg ccatttcacc accaacgact cctaccgctt ctccgcctcc 600
   aggaggagca ccgaagccaa tttcgtctcg cttctggtgg tctcctacag cgactcactg 660
   ctggacccag ctacctttgc agaagtcagc aaactggacg gcgcggtgca ggatctgcgc 720
   gtggcgcggg aaaagggaag ccagatccag taccagcagg tgtgcgcgag gtacagggcg 780
   ctctgcgtgc cccccaaccc gatcctgtac gcctggcagg tgaacaaaac gctcaacctg 840
   agcagcatct ccttccccgc ctacaaccac ggcaggcatc ccctctacct gaccggcttc 900
   ttcggaggat acatcttggg gggcagccta ggaatgggcc agttactcct gcgggccaaa 960
   gccatgcggc tgctgtacta cctgaagacc gaggaccctg agtacgacgt gcagagcaag 1020
   cagtggctca cccatttgct cgatcaattt accaacatta agaacatctt ggccttgaaa 1080
   aaaattgagg tagtccactt tacatcgctt tccagacaac tggaatttga ggcaacttct 1140
   gtgactgtga tccctgtgtt tcacctggca tacattctca tcattctgtt tgcagtcaca 1200
   tcatgcttta ggtttgactg catacgaaac aaaatgtgtg ttgcggcctt tggagtgatt 1260
   tctgctttct tggcagtggt gagcggcttt ggcctgctgt tgcacattgg ggtgccattt 1320
   gtcatcatag ttgccaattc accatttctt attctaggtg ttggggtcga tgacatgttt 1380
   atcatgattt ctgcctggca taagaccaac cttgcagatg acatacgaga gcggatgtcc 1440
   aatgtctatt caaaagcggc agtgtctatt acaatcacca ccatcactaa catcctggcc 1500
   ttatatacag ggattatgag ctcttttagg tctgtacaat gtttttgcat ctatacagga 1560
   atgaccctgt tattttgcta tttttataac atcacgtgtt ttggagcatt tatggccttg 1620
   gatggtaaaa gagaagtagt ctgcctatgc tggttgaaaa aggctgaccc aaaatggccc 1680
   tcatttaaaa agttctgctg tttcccattt ggttctgtcc cagatgaaca tggaactgat 1740
   atccatccaa taagtttgtt ttttagagac tattttggcc cctttctcac aaggagtgag 1800
   tccaagtatt ttgtagtctt tatatatgtt ttgtacatca taagcagtat atatgggtgt 1860
   ttccatgtgc aggaaggttt agaccttcga aatctggcaa gtgacgattc ctacatcaca 1920
   ccatatttta acgtagagga gaattatttt tcagattatg gtcccagggt tatggttatt 1980
   gttactaaaa aagttgacta ctgggataaa gatgttaggc aaaaactgga aaactgtact 2040
   aaaatttttg aaaaaaatgt ctatgtagat aaaaatctta cagagttttg gttagatgca 2100
   tatgtgcaat atttaaaagg taacagccaa gatcctaatg agaagaatac ttttatgaac 2160
   aatattcctg attttttaag caattttcca aattttcagc atgatattaa tatttcttca 2220
   tcaaatgaaa tcatttcttc ccggggcttc attcagacaa cagatgtttc ttcctcagcc 2280
   aaaaagaaaa tattgttatt ctaa 2304

<210> 6
<211> 767
<212> PRT
<213> Homo sapiens

<400> 6

Met Pro Trp Val Glu Pro Lys Pro Arg Pro Gly Pro Glu Gln Lys Pro
1               5                   10                  15

Lys Leu Thr Lys Pro Asp Ser Ala Thr Gly Pro Gln Trp Tyr Gln Glu
            20                  25                  30

Ser Gln Glu Ser Glu Ser Glu Gly Lys Gln Pro Pro Pro Gly Pro Leu
        35                  40                  45

Ala Pro Pro Lys Ser Pro Glu Pro Ser Gly Pro Leu Ala Ser Glu Gln
    50                  55                  60

Asp Ala Pro Leu Pro Glu Gly Asp Asp Ala Pro Pro Arg Pro Ser Met
65                  70                  75                  80

Leu Asp Asp Ala Pro Arg Leu Pro Leu Glu Leu Asp Asp Ala Pro Leu
                85                  90                  95

Pro Glu Glu Glu Thr Pro Glu Pro Thr Ala Ile Cys Arg His Arg His
            100                 105                 110

Arg Cys His Thr Asp Cys Leu Glu Gly Leu Leu Ser Arg Thr Phe Gln
        115                 120                 125

Trp Leu Gly Trp Gln Val Gly Ala His Pro Trp Ile Phe Leu Leu Ala
    130                 135                 140

Pro Leu Met Leu Thr Ala Ala Leu Gly Thr Gly Phe Leu Tyr Leu Pro
145                 150                 155                 160

Lys Asp Glu Glu Glu Asp Leu Glu Glu His Tyr Thr Pro Val Gly Ser
                165                 170                 175

Pro Ala Lys Ala Glu Arg Arg Phe Val Gln Gly His Phe Thr Thr Asn
            180                 185                 190

Asp Ser Tyr Arg Phe Ser Ala Ser Arg Arg Ser Thr Glu Ala Asn Phe
        195                 200                 205

Val Ser Leu Leu Val Val Ser Tyr Ser Asp Ser Leu Leu Asp Pro Ala
    210                 215                 220

Thr Phe Ala Glu Val Ser Lys Leu Asp Gly Ala Val Gln Asp Leu Arg
225                 230                 235                 240

Val Ala Arg Glu Lys Gly Ser Gln Ile Gln Tyr Gln Gln Val Cys Ala
                245                 250                 255

Arg Tyr Arg Ala Leu Cys Val Pro Pro Asn Pro Ile Leu Tyr Ala Trp
            260                 265                 270

Gln Val Asn Lys Thr Leu Asn Leu Ser Ser Ile Ser Phe Pro Ala Tyr
        275                 280                 285

Asn His Gly Arg His Pro Leu Tyr Leu Thr Gly Phe Phe Gly Gly Tyr
    290                 295                 300

Ile Leu Gly Gly Ser Leu Gly Met Gly Gln Leu Leu Leu Arg Ala Lys
305                 310                 315                 320

Ala Met Arg Leu Leu Tyr Tyr Leu Lys Thr Glu Asp Pro Glu Tyr Asp

325                 330                 335

Val Gln Ser Lys Gln Trp Leu Thr His Leu Leu Asp Gln Phe Thr Asn
            340                 345                 350

Ile Lys Asn Ile Leu Ala Leu Lys Lys Ile Glu Val Val His Phe Thr
        355                 360                 365

Ser Leu Ser Arg Gln Leu Glu Phe Glu Ala Thr Ser Val Thr Val Ile
    370                 375                 380

Pro Val Phe His Leu Ala Tyr Ile Leu Ile Ile Leu Phe Ala Val Thr
385                 390                 395                 400

Ser Cys Phe Arg Phe Asp Cys Ile Arg Asn Lys Met Cys Val Ala Ala
                405                 410                 415

Phe Gly Val Ile Ser Ala Phe Leu Ala Val Val Ser Gly Phe Gly Leu
            420                 425                 430

Leu Leu His Ile Gly Val Pro Phe Val Ile Ile Val Ala Asn Ser Pro
        435                 440                 445

Phe Leu Ile Leu Gly Val Gly Val Asp Asp Met Phe Ile Met Ile Ser
    450                 455                 460

Ala Trp His Lys Thr Asn Leu Ala Asp Asp Ile Arg Glu Arg Met Ser
465                 470                 475                 480

Asn Val Tyr Ser Lys Ala Ala Val Ser Ile Thr Ile Thr Thr Ile Thr
                485                 490                 495

Asn Ile Leu Ala Leu Tyr Thr Gly Ile Met Ser Ser Phe Arg Ser Val
            500                 505                 510

Gln Cys Phe Cys Ile Tyr Thr Gly Met Thr Leu Leu Phe Cys Tyr Phe
        515                 520                 525

Tyr Asn Ile Thr Cys Phe Gly Ala Phe Met Ala Leu Asp Gly Lys Arg
    530                 535                 540

Glu Val Val Cys Leu Cys Trp Leu Lys Lys Ala Asp Pro Lys Trp Pro
545                 550                 555                 560

Ser Phe Lys Lys Phe Cys Cys Phe Pro Phe Gly Ser Val Pro Asp Glu
                565                 570                 575

His Gly Thr Asp Ile His Pro Ile Ser Leu Phe Phe Arg Asp Tyr Phe
            580                 585                 590

Gly Pro Phe Leu Thr Arg Ser Glu Ser Lys Tyr Phe Val Val Phe Ile
        595                 600                 605

Tyr Val Leu Tyr Ile Ile Ser Ser Ile Tyr Gly Cys Phe His Val Gln
    610                 615                 620

Glu Gly Leu Asp Leu Arg Asn Leu Ala Ser Asp Asp Ser Tyr Ile Thr
625                 630                 635                 640

Pro Tyr Phe Asn Val Glu Glu Asn Tyr Phe Ser Asp Tyr Gly Pro Arg
                645                 650                 655

Val Met Val Ile Val Thr Lys Lys Val Asp Tyr Trp Asp Lys Asp Val
            660                 665                 670

Arg Gln Lys Leu Glu Asn Cys Thr Lys Ile Phe Glu Lys Asn Val Tyr
        675                 680                 685

Val Asp Lys Asn Leu Thr Glu Phe Trp Leu Asp Ala Tyr Val Gln Tyr
    690                 695                 700

Leu Lys Gly Asn Ser Gln Asp Pro Asn Glu Lys Asn Thr Phe Met Asn
705                 710                 715                 720

Asn Ile Pro Asp Phe Leu Ser Asn Phe Pro Asn Phe Gln His Asp Ile
                725                 730                 735

Asn Ile Ser Ser Ser Asn Glu Ile Ile Ser Ser Arg Gly Phe Ile Gln
            740                 745                 750

Thr Thr Asp Val Ser Ser Ser Ala Lys Lys Lys Ile Leu Leu Phe
        755                 760                 765

<210> 7
<211> 2021
<212> DNA
<213> Homo sapiens

<400> 7
caggaaaccg tctggtggga tctccgcagc tgcttttcac ctgctgttcc tcctgcgctt 60
cctaagagga agaatcaatg ccgtgggtgg agcccaagcc caggccgggg ccggagcaga 120
agcccaagct caccaaaccg gactctgcca ccgggccgca gtggtaccag gaatctcagg 180
aatcggagtc ggaaggcaag cagccacccc cgggacccct ggcacccccg aaatcccccg 240
aaccctcagg acccctggcg tcggagcagg atgcacccct gccagagggg gacgatgcac 300
ccccccggcc gtcgatgctg gacgatgcac cccgcctgcc gctggagctg gacgatgcac 360
ccctgccgga ggaggaaacc cccgaaccca cggccatctg caggcaccgg caccgctgtc 420
acaccgactg cctagagggg ctgctgtccc gcaccttcca gtggctgggg tggcaggtgg 480
gcgcgcaccc ctggatcttc ctgctggcgc ccttgatgct gacagccgcg ctgggcaccg 540
gcttcctgta cctacccaag gacgaagagg aagacctaga ggagcattac acccctgtgg 600
ggagcccggc caaggcggag cggcgcttcg tgcagggcca tttcaccacc aacgactcct 660
accgcttctc cgcctccagg aggagcaccg aagccaattt cgtctcgctt ctggtggtct 720
cctacagcga ctcactgctg gacccagcta cctttgcaga agtcagcaaa ctggacggcg 780
cggtgcagga tctgcgcgtg gcgcgggaaa agggaagcca gatccagtac cagcaggtgt 840
gcgcgaggta cagggcgctc tgcgtgcccc ccaacccgat cctgtacgcc tggcaggtga 900
acaaaacgct caacctgagc agcatctcct tccccgccta caaccacggc aggcatcccc 960
tctacctgac cggcttcttc ggaggataca tcttgggggg cagcctagga atgggccagt 1020
tactcctgcg ggccaaagcc atgcggctgc tgtactacct gaagaccgag gaccctgagt 1080
acgacgtgca gagcaagcag tggctcaccc atttgctcga tcaatttacc aacattaaga 1140
acatcttggc cttgaaaaaa attgaggtag tccactttac atcgctttcc agacaactgg 1200
aatttgaggc aacttctgtg actgtgatcc ctgtgtttca cctggcatac attctcatca 1260
ttctgtttgc agtcacatca tgctttaggt ttgactgcat acgaaacaaa atgtgtgttg 1320
cggcctttgg agtgatttct gctttcttgg cagtggtgag cggctttggc ctgctgttgc 1380
acattggggt gccatttgtc atcatagttg ccaattcacc atttcttatt ctaggtgttg 1440
gggtcgatga catgtttatc atgatttctg cctggcataa gaccaacctt gcaggtgaca 1500
tacgagagcg gatgtccaat gtctattcaa aagcggcagt gtctattaca atcaccacca 1560
tcactaacat cctggcctta tatacaggga ttatgagctc ttttaggtcc gtacaatgtt 1620
tttgcatcta tacaggaacg accctgttat tttgctattt ttataacatc acgtgttttg 1680
gagcatttat ggccttggat ggtaaaagag aagtagtctg cctatgctgg ttgaaaaagg 1740
ctgacccaaa atggccctca tttaaaaagt tctgctgttt cccatttggt tctgtcccag 1800
atgaacatgg aactgatatc catccaatga gtttgttttt tagagactat tttggcccct 1860
ttctcacaag gagtgagtcc aagtattttg tagtctttat atatgttttg tacatcataa 1920
gcagtatata tgggtgtttc catgtgcagg aaggtttaga ccttcgaaat ctggcaagtg 1980

acgattccta catcacacca tattttaacg tagaggagaa t 2021

<210> 8
<211> 77
<212> DNA
<213> Homo sapiens

<400> 8
caggaaaccg tctggtggga tctccgcagc tgcttttcac ctgctgttcc tcctgcgctt 60
cctaagagga agaatca 77

<210> 9
<211> 1944
<212> DNA
<213> Homo sapiens

<400> 9
atgccgtggg tggagcccaa gcccaggccg gggccggagc agaagcccaa gctcaccaaa 60
ccggactctg ccaccgggcc gcagtggtac caggaatctc aggaatcgga gtcggaaggc 120
aagcagccac ccccgggacc cctggcaccc ccgaaatccc ccgaaccctc aggacccctg 180
gcgtcggagc aggatgcacc cctgccagag ggggacgatg cacccccccg gccgtcgatg 240
ctggacgatg caccccgcct gccgctggag ctggacgatg cacccctgcc ggaggaggaa 300
acccccgaac ccacggccat ctgcaggcac cggcaccgct gtcacaccga ctgcctagag 360
gggctgctgt cccgcacctt ccagtggctg gggtggcagg tgggcgcgca cccctggatc 420
ttcctgctgg cgcccttgat gctgacagcc gcgctgggca ccggcttcct gtacctaccc 480
aaggacgaag aggaagacct agaggagcat tacacccctg tggggagccc ggccaaggcg 540
gagcggcgct tcgtgcaggg ccatttcacc accaacgact cctaccgctt ctccgcctcc 600
aggaggagca ccgaagccaa tttcgtctcg cttctggtgg tctcctacag cgactcactg 660
ctggacccag ctacctttgc agaagtcagc aaactggacg gcgcggtgca ggatctgcgc 720
gtggcgcggg aaaagggaag ccagatccag taccagcagg tgtgcgcgag gtacagggcg 780
ctctgcgtgc cccccaaccc gatcctgtac gcctggcagg tgaacaaaac gctcaacctg 840
agcagcatct ccttccccgc ctacaaccac ggcaggcatc ccctctacct gaccggcttc 900
ttcggaggat acatcttggg gggcagccta ggaatgggcc agttactcct gcgggccaaa 960
gccatgcggc tgctgtacta cctgaagacc gaggaccctg agtacgacgt gcagagcaag 1020
cagtggctca cccatttgct cgatcaattt accaacatta agaacatctt ggccttgaaa 1080
aaaattgagg tagtccactt tacatcgctt tccagacaac tggaatttga ggcaacttct 1140
gtgactgtga tccctgtgtt tcacctggca tacattctca tcattctgtt tgcagtcaca 1200
tcatgcttta ggtttgactg catacgaaac aaaatgtgtg ttgcggcctt tggagtgatt 1260
tctgctttct tggcagtggt gagcggcttt ggcctgctgt tgcacattgg ggtgccattt 1320
gtcatcatag ttgccaattc accatttctt attctaggtg ttggggtcga tgacatgttt 1380
atcatgattt ctgcctggca taagaccaac cttgcaggtg acatacgaga gcggatgtcc 1440
aatgtctatt caaaagcggc agtgtctatt acaatcacca ccatcactaa catcctggcc 1500
ttatatacag ggattatgag ctcttttagg tccgtacaat gtttttgcat ctatacagga 1560
acgaccctgt tattttgcta tttttataac atcacgtgtt ttggagcatt tatggccttg 1620
gatggtaaaa gagaagtagt ctgcctatgc tggttgaaaa aggctgaccc aaaatggccc 1680
tcatttaaaa agttctgctg tttcccattt ggttctgtcc cagatgaaca tggaactgat 1740
atccatccaa tgagtttgtt ttttagagac tattttggcc cctttctcac aaggagtgag 1800
tccaagtatt ttgtagtctt tatatatgtt ttgtacatca taagcagtat atatgggtgt 1860
ttccatgtgc aggaaggttt agaccttcga aatctggcaa gtgacgattc ctacatcaca 1920
ccatatttta acgtagagga gaat 1944

<210> 10
<211> 648
<212> PRT
<213> Homo sapiens

<400> 10
Met Pro Trp Val Glu Pro Lys Pro Arg Pro Gly Pro Glu Gln Lys Pro
1               5                   10                  15

Lys Leu Thr Lys Pro Asp Ser Ala Thr Gly Pro Gln Trp Tyr Gln Glu

20                  25                  30

Ser Gln Glu Ser Glu Ser Glu Gly Lys Gln Pro Pro Pro Gly Pro Leu
        35                  40                  45

Ala Pro Pro Lys Ser Pro Glu Pro Ser Gly Pro Leu Ala Ser Glu Gln
    50                  55                  60

Asp Ala Pro Leu Pro Glu Gly Asp Asp Ala Pro Pro Arg Pro Ser Met
65                  70                  75                  80

Leu Asp Asp Ala Pro Arg Leu Pro Leu Glu Leu Asp Asp Ala Pro Leu
                85                  90                  95

Pro Glu Glu Glu Thr Pro Glu Pro Thr Ala Ile Cys Arg His Arg His
            100                 105                 110

Arg Cys His Thr Asp Cys Leu Glu Gly Leu Leu Ser Arg Thr Phe Gln
        115                 120                 125

Trp Leu Gly Trp Gln Val Gly Ala His Pro Trp Ile Phe Leu Leu Ala
    130                 135                 140

Pro Leu Met Leu Thr Ala Ala Leu Gly Thr Gly Phe Leu Tyr Leu Pro
145                 150                 155                 160

Lys Asp Glu Glu Glu Asp Leu Glu Glu His Tyr Thr Pro Val Gly Ser
                165                 170                 175

Pro Ala Lys Ala Glu Arg Arg Phe Val Gln Gly His Phe Thr Thr Asn
            180                 185                 190

Asp Ser Tyr Arg Phe Ser Ala Ser Arg Arg Ser Thr Glu Ala Asn Phe
        195                 200                 205

Val Ser Leu Leu Val Val Ser Tyr Ser Asp Ser Leu Leu Asp Pro Ala
    210                 215                 220

Thr Phe Ala Glu Val Ser Lys Leu Asp Gly Ala Val Gln Asp Leu Arg
225                 230                 235                 240

Val Ala Arg Glu Lys Gly Ser Gln Ile Gln Tyr Gln Gln Val Cys Ala
                245                 250                 255

Arg Tyr Arg Ala Leu Cys Val Pro Pro Asn Pro Ile Leu Tyr Ala Trp
            260                 265                 270

Gln Val Asn Lys Thr Leu Asn Leu Ser Ser Ile Ser Phe Pro Ala Tyr
        275                 280                 285

Asn His Gly Arg His Pro Leu Tyr Leu Thr Gly Phe Phe Gly Gly Tyr
    290                 295                 300

Ile Leu Gly Gly Ser Leu Gly Met Gly Gln Leu Leu Leu Arg Ala Lys
305                 310                 315                 320

Ala Met Arg Leu Leu Tyr Tyr Leu Lys Thr Glu Asp Pro Glu Tyr Asp
                325                 330                 335

Val Gln Ser Lys Gln Trp Leu Thr His Leu Leu Asp Gln Phe Thr Asn
            340                 345                 350

Ile Lys Asn Ile Leu Ala Leu Lys Lys Ile Glu Val Val His Phe Thr
        355                 360                 365

Ser Leu Ser Arg Gln Leu Glu Phe Glu Ala Thr Ser Val Thr Val Ile
    370                 375                 380

Pro Val Phe His Leu Ala Tyr Ile Leu Ile Ile Leu Phe Ala Val Thr
385                 390                 395                 400

Ser Cys Phe Arg Phe Asp Cys Ile Arg Asn Lys Met Cys Val Ala Ala
                405                 410                 415

Phe Gly Val Ile Ser Ala Phe Leu Ala Val Val Ser Gly Phe Gly Leu
            420                 425                 430

Leu Leu His Ile Gly Val Pro Phe Val Ile Ile Val Ala Asn Ser Pro
        435                 440                 445

Phe Leu Ile Leu Gly Val Gly Val Asp Asp Met Phe Ile Met Ile Ser
    450                 455                 460

Ala Trp His Lys Thr Asn Leu Ala Gly Asp Ile Arg Glu Arg Met Ser
465                 470                 475                 480

Asn Val Tyr Ser Lys Ala Ala Val Ser Ile Thr Ile Thr Thr Ile Thr
                485                 490                 495

Asn Ile Leu Ala Leu Tyr Thr Gly Ile Met Ser Ser Phe Arg Ser Val
            500                 505                 510

Gln Cys Phe Cys Ile Tyr Thr Gly Thr Thr Leu Leu Phe Cys Tyr Phe
        515                 520                 525

Tyr Asn Ile Thr Cys Phe Gly Ala Phe Met Ala Leu Asp Gly Lys Arg
    530                 535                 540

Glu Val Val Cys Leu Cys Trp Leu Lys Lys Ala Asp Pro Lys Trp Pro
545                 550                 555                 560

Ser Phe Lys Lys Phe Cys Cys Phe Pro Phe Gly Ser Val Pro Asp Glu
                565                 570                 575

His Gly Thr Asp Ile His Pro Met Ser Leu Phe Phe Arg Asp Tyr Phe
            580                 585                 590

Gly Pro Phe Leu Thr Arg Ser Glu Ser Lys Tyr Phe Val Val Phe Ile
        595                 600                 605

Tyr Val Leu Tyr Ile Ile Ser Ser Ile Tyr Gly Cys Phe His Val Gln
    610                 615                 620

Glu Gly Leu Asp Leu Arg Asn Leu Ala Ser Asp Asp Ser Tyr Ile Thr
625                 630                 635                 640

Pro Tyr Phe Asn Val Glu Glu Asn
                645

<210> 11
<211> 405
<212> DNA

<213> Homo sapiens

<400> 11
tccatgatta atcttgtcat ttgtacaggg ttttctttcg atttttctgc acacatttcc 60
tatgcatttg tttctagttc tcaaccctca gtaaaccaaa aatcagttga ggcattgtat 120
cttctaggct acccagtgtt acaaagtgca atttcaacaa taataggggt gtgtgtttta 180
gctgcagcga aagcatacat cttcagaaca ttttttaaga ttatgtttct tgttatgata 240
tttggggcag ctcatggcct aatttttatt ccagtattct taaccttttt tggaaggttt 300
atttgaatat ccactaacaa gtcaaagacc aattctagaa ttcctgattg ccctattcca 360
atctgatttt tttaaatgca ctattaagaa tagtcaacaa actac 405

<210> 12
<211> 306
<212> DNA
<213> Homo sapiens

<400> 12
tccatgatta atcttgtcat ttgtacaggg ttttctttcg atttttctgc acacatttcc 60
tatgcatttg tttctagttc tcaaccctca gtaaaccaaa aatcagttga ggcattgtat 120
cttctaggct acccagtgtt acaaagtgca atttcaacaa taataggggt gtgtgtttta 180
gctgcagcga aagcatacat cttcagaaca ttttttaaga ttatgtttct tgttatgata 240
tttggggcag ctcatggcct aatttttatt ccagtattct taaccttttt tggaaggttt 300
atttga 306

<210> 13
<211> 99
<212> DNA
<213> Homo sapiens

<400> 13
atatccacta acaagtcaaa gaccaattct agaattcctg attgccctat tccaatctga 60
tttttttaaa tgcactatta agaatagtca acaaactac 99

<210> 14
<211> 101
<212> PRT
<213> Homo sapiens

<400> 14
Ser Met Ile Asn Leu Val Ile Cys Thr Gly Phe Ser Phe Asp Phe Ser
1               5                   10                  15

Ala His Ile Ser Tyr Ala Phe Val Ser Ser Ser Gln Pro Ser Val Asn
            20                  25                  30

Gln Lys Ser Val Glu Ala Leu Tyr Leu Leu Gly Tyr Pro Val Leu Gln
        35                  40                  45

Ser Ala Ile Ser Thr Ile Ile Gly Val Cys Val Leu Ala Ala Ala Lys
    50                  55                  60

Ala Tyr Ile Phe Arg Thr Phe Phe Lys Ile Met Phe Leu Val Met Ile
65                  70                  75                  80

Phe Gly Ala Ala His Gly Leu Ile Phe Ile Pro Val Phe Leu Thr Phe
                85                  90                  95

Phe Gly Arg Phe Ile
            100

<210> 15

<211> 1166
<212> DNA
<213> Homo sapiens

<400> 15
caggaaaccg tctggtggga tctccgcagc tgcttttcac ctgctgttcc tcctgcgctt 60
cctaagagga agaatcaatg ccgtgggtgg agcccaagcc caggccgggg ccggagcaga 120
agcccaagct caccaaaccg gactctgcca ccgggccgca gtggtaccag gaatctcagg 180
aatcggagtc ggaaggcaag cagccacccc cgggacccct ggcacccccg aaatcccccg 240
aaccctcagg acccctggcg tcggagcagg atgcacccct gccagagggg gacgatgcac 300
ccccccggcc gtcgatgctg gacgatgcac cccgcctgcc gctggagctg gacgatgcac 360
ccctgccgga ggaggaaacc cccgaaccca cggccatctg caggcaccgg caccgctgtc 420
acaccgactg cctagagggg ctgctgtccc gcaccttcca gtggctgggg tggcaggtgg 480
gcgcgcaccc ctggatcttc ctgctggcgc ccttgatgct gacagccgcg ctgggcaccg 540
gcttcctgta cctacccaag gacgaagagg aagacctaga ggagcattac acccctgtgg 600
ggagcccggc caaggcggag cggcgcttcg tgcagggcca tttcaccacc aacgactcct 660
accgcttctc cgcctccagg aggagcaccg aagccaattt cgtctcgctt ctggtggtct 720
cctacagcga ctcactgctg gacccagcta cctttgcaga agtcagcaaa ctggacggcg 780
cggtgcagga tctgcgcgtg gcgcgggaaa agggaagcca gatccagtac cagcaggtgt 840
gcgcgaggta cagggcgctc tgcgtgcccc ccaacccgat cctgtacgcc tggcaggtga 900
acaaaacgct caacctgagc agcatctcct tccccgccta caaccacggc aggcatcccc 960
tctacctgac cggcttcttc ggaggataca tcttgggggg cagcctagga atgggccagt 1020
tactcctgcg ggccaaagcc atgcggctgc tgtactacct gaagaccgag gaccctgagt 1080
acgacgtgca gagcaagcag tggctcaccc atttgctcga tcaatttacc aacattaaga 1140
acatcttggc cttgaaaaaa attgag 1166

<210> 16
<211> 122
<212> DNA
<213> Homo sapiens

<400> 16
gtagtccact ttacatcgct ttccagacaa ctggaatttg aggcaacttc tgtgactgtg 60
atccctgtgt ttcacctggc atacattctc atcattctgt ttgcagtcac atcatgcttt 120
ag 122

<210> 17
<211> 146
<212> DNA
<213> Homo sapiens

<400> 17
gtttgactgc atacgaaaca aaatgtgtgt tgcggccttt ggagtgattt ctgctttctt 60
ggcagtggtg agcggctttg gcctgctgtt gcacattggg gtgccatttg tcatcatagt 120
tgccaattca ccatttctta ttctag 146

<210> 18
<211> 1862
<212> DNA
<213> Homo sapiens

<400> 18
gtgttggggt cgatgacatg tttatcatga tttctgcctg gcataagacc aaccttgcag 60
gtgacatacg agagcggatg tccaatgtct attcaaaagc ggcagtgtct attacaatca 120
ccaccatcac taacatcctg gccttatata cagggattat gagctctttt aggtccgtac 180
aatgtttttg catctataca ggaacgaccc tgttattttg ctatttttat aacatcacgt 240
gttttggagc atttatggcc ttggatggta aaagagaagt agtctgccta tgctggttga 300
aaaaggctga cccaaaatgg ccctcattta aaaagttctg ctgtttccca tttggttctg 360
tcccagatga acatggaact gatatccatc caatgagttt gttttttaga gactattttg 420
gcccctttct cacaaggagt gagtccaagt attttgtagt ctttatatat gttttgtaca 480
tcataagcag tatatatggg tgtttccatg tgcaggaagg tttagacctt cgaaatctgg 540

caagtgacga ttcctacatc acaccatatt ttaacgtaga ggagaattat ttttcagatt 600
atggtcccag ggttatggtt attgttacta aaaaagttga ctactgggat aaagatgtta 660
ggcaaaaact ggaaaactgt actaaaattt ttgaaaaaaa tgtctatgta gataaaaatc 720
ttacagagtt ttggttagat gcatatgtgc aatatttaaa aggtaacagc caagatccta 780
atgagaagaa tacttttatg aacaatattc ctgatttttt aagcaatttt ccaaattttc 840
agcatgatat taatatttct tcatcaaatg aaatcatttc ttcccggggc ttcattcaga 900
caacagatgt ttcttcctca gccaaaaaga aaatattgtt attccaatta cgacgcatag 960
ctgaagactg tcaaattccc ctaatggtgt ataaccaggc atttatatat tttgatcagt 1020
atgctgcaat attagaagac actgttagaa atgtattggt tgcatcagca gctatgttca 1080
ttgtttcctt attgttaatc ccttatccat tgtgttcctt gtgggtgact tttgctatcg 1140
gttctgtgat tgtaggggta acgggtttca tggcattctg gaaagtcaat cttgattcca 1200
tatccatgat taatcttgtc atttgtacag ggtttctttt cgatttttct gcacacattt 1260
cctatgcatt tgtttctagt tctcaaccct cagtaaacca aaaatcagtt gaggcattgt 1320
atcttctagg ctacccagtg ttacaaagtg caatttcaac aataataggg gtgtgtgttt 1380
tagctgcagc gaaagcatac atcttcagaa cattttttaa gattatgttt cttgttatga 1440
tatttggggc agctcatggc ctaattttta ttccagtatt cttaaccttt tttggaaggt 1500
ttatttgaat atccactaac aagtcaaaga ccaattctag aattcctgat tgccctattc 1560
caatctgatt tttttaaatg cactattaag aatagtcaac aaactactgg gaggccaaag 1620
tgggtggatc acctgaggtc aagaattcga gaccagcctg gccaacatgg tgaaacctcg 1680
acactactga aaatacaaaa attatccagg catggtggcg tgcacctata atcccagcta 1740
cctgggaggc tgaggcagga gaatcgcttg aacctgggag atggaggttg cagtgagctg 1800
gagtgcacca ggcactccag cctgggtgac aagaatgaga ctccgtctca gaaaaaaaaa 1860
aa 1862

<210> 19
<211> 500
<212> DNA
<213> Homo sapiens

<400> 19
cctgccgctg gagctggacg atgcacccct gccggaggag gaaacccccg aacccacggc 60
catctgcagg caccggcacc gctgtcacac cgactgccta gaggggctgc tgtcccgcac 120
cttccagtgg ctggggtggc aggtgggcgc gcacccctgg atcttcctgc tggcgccctt 180
gatgctgaca gccgcgctgg gcaccggctt cctgtaccta cccaaggacg aagaggaaga 240
cctagaggag cattacaccc ctgtggggag cccggccaag gcggagcggc gcttcgtgca 300
gggccatttc accaccaacg actcctaccg cttctccgcc tccaggagga gcaccgaagc 360
caatttcgtc tcgcttctgg tggtctccta cagcgactca ctgctggacc cagctacctt 420
tgcagaagtc agcaaactgg acggcgcggt gcaggatctg cgcgtggcgc gggaaaaggg 480
aagccagatc cagtaccagc 500

<210> 20
<211> 500
<212> DNA
<213> Homo sapiens

<400> 20
tgtgtggaaa gaagcaaatc tacattattt tctctattct gaagtcataa tccatgtatt 60
tttatctttt gctgaatttt aacatacata ttgtttcatc actaagataa agtgtttcaa 120
tagtttttgt aactcacaca caaatattgt aattaaaagc tatttatctg agtatatttt 180
tgtggcaggt agtccacttt acatcgcttt ccagacaact ggaatttgag gcaacttctg 240
tgactgtgat ccctgtgttt cacctggcat acattctcat cattctgttt gcagtcacat 300
catgctttag gtaaaaagct ttgattagtt tttaaatctc ttactttatg aaaaatactt 360
tattgtggta aaatataata atatatatga cataacatat gatgttatag ccactcttaa 420
gtgtaaaatt cggtggcatt aattacatcc acattggcat gcaaccatca caatgatctg 480
ttttcaaata ttttgcatca 500

<210> 21
<211> 500
<212> DNA
<213> Homo sapiens

<400> 21
ttcctttttt ttttttcagt tgttctgaga tagtccctgg gaattaaagg tctccagcag 60
atatgtgtgg aaattactgt cttagagatt tatgactagg tgtccaagtc aagaatgtta 120
tccattgatg aaagctacca ccacaaattc catgattttt ccctccaaat ttacaggttt 180
gactgcatac gaaacaaaat gtgtgttgcg gcctttggag tgatttctgc tttcttggca 240
gtggtgagcg gctttggcct gctgttgcac attggggtgc catttgtcat catagttgcc 300
aattcaccat ttcttattct aggtgagtaa aacaaaacgg gaggctgcac accgtgtatg 360
tggtggggta ggggatatat ataaggaaaa gaattgtatt aaggtggctc tacagttcaa 420
aaatgaggtg ccaggaaaca gggaaatgaa attgaactgg atatctctag aggtggaaag 480
atcgagtatc ctaatggaaa 500

<210> 22
<211> 500
<212> DNA
<213> Homo sapiens

<400> 22
tacatcacac catattttaa cgtagaggag aattattttt cagattatgg tcccagggtt 60
atggttattg ttactaaaaa agttgactac tgggataaag atgttaggca aaaactggaa 120
aactgtacta aaatttttga aaaaaatgtc tatgtagata aaaatcttac agagttttgg 180
ttagatgcat atgtgcaata tttaaaaggt aacagccaag atcctaatga gaagaatact 240
tttatgaaca atattcctga ttttttaagc aattttccaa attttcagca tgatattaat 300
atttcttcat caaatgaaat catttcttcc cggggcttca ttcagacaac agatgtttct 360
tcctcagcca aaaagaaaat attgttattc caattacgac gcatagctga agactgtcaa 420
attcccctaa tggtgtataa ccaggcattt atatattttg atcagtatgc tgcaatatta 480
gaagacactg ttagaaatgt 500

<210> 23
<211> 1000
<212> DNA
<213> Homo sapiens

<400> 23
cattgtagga taggctgtgg caaattcata tattgcttat gagggtataa atcaatacaa 60
cttctttgga ttttaatttc tcaatagctc taaaaattta aaagaaccta tcctttggcc 120
tagtgaatcc actctaaaga attatctctc acagatatgg ttgctgtgta caggttaaaa 180
tatgatattt atttactgag cactgtttat aaaagcaaat gactaaacaa tgtatgggtc 240
catcagtaaa attctggcta aattatgttt catccataca ttggaaaagt atagagcagt 300
tcaaaaaaat aagataaatc catatgcact gacatgttca aatcttctgc cactgtagat 360
gaaaaatgtg atctacataa catgaatagt gaagtagtat ttatgaaaaa ggtatatttg 420
ctatatcttt gatatgcatg tatatgcata gaaagggttt gcaaggatgt agacccactt 480
tttaacactt cttttaagag gaatgggagt gaaagatttt gatttttttt tccccacttt 540
tcattctatg catttacttc tttttactag gagaatgtat gtacatgtta cacttgtgga 600
cagaaaataa aaataataac taagtcaagt gtgtcagatt cataaaagtc ttacttgaaa 660
ctgttactgg gtgaatgcca agaaaggtaa aatctgtcac tcaggacacg tggaatttct 720
gtgccatttg tgtgcatgga agaaacaccc acattctccc aataaattgc gagtccatag 780
gataggcacg catttgatgg ccttgaagaa gcgttccttg tatgtgcaga acccctgaga 840
aaccaggcac gccacaaaca catttcagat tagttgggcg acggggcaaa ccacgtgctc 900
ccaacgcagc ctccgcttcc ggagcgcggc tggagggcaa actcccctag aatttctcca 960
ggaaaccgtt gggtggggcc aggagagcgt tgggtggggc 1000

<210> 24
<211> 17
<212> DNA
<213> Homo sapiens

<400> 24
caggaaaccg tctggtg 17

<210> 25
<211> 17

<212> DNA
<213> Homo sapiens

<400> 25
aggaaaccgt ctggtgg                                                    17

<210> 26
<211> 17
<212> DNA
<213> Homo sapiens

<400> 26
ggaaaccgtc tggtggg                                                    17

<210> 27
<211> 17
<212> DNA
<213> Homo sapiens

<400> 27
gaaaccgtct ggtggga                                                    17

<210> 28
<211> 17
<212> DNA
<213> Homo sapiens

<400> 28
aaaccgtctg gtgggat                                                    17

<210> 29
<211> 17
<212> DNA
<213> Homo sapiens

<400> 29
aaccgtctgg tgggatc                                                    17

<210> 30
<211> 17
<212> DNA
<213> Homo sapiens

<400> 30
accgtctggt gggatct                                                    17

<210> 31
<211> 17
<212> DNA
<213> Homo sapiens

<400> 31
ccgtctggtg ggatctc                                                    17

<210> 32
<211> 17
<212> DNA
<213> Homo sapiens

<400> 32
cgtctggtgg gatctcc                                                    17

<210> 33
<211> 17
<212> DNA
<213> Homo sapiens

<400> 33
gtctggtggg atctccg                                                    17

<210> 34
<211> 17
<212> DNA
<213> Homo sapiens

<400> 34
tctggtggga tctccgc                                                    17

<210> 35
<211> 17
<212> DNA
<213> Homo sapiens

<400> 35
ctggtgggat ctccgca                                                    17

<210> 36
<211> 17
<212> DNA
<213> Homo sapiens

<400> 36
tggtgggatc tccgcag                                                    17

<210> 37
<211> 17
<212> DNA
<213> Homo sapiens

<400> 37
ggtgggatct ccgcagc                                                    17

<210> 38
<211> 17
<212> DNA
<213> Homo sapiens

<400> 38
gtgggatctc cgcagct                                                    17

<210> 39
<211> 17
<212> DNA
<213> Homo sapiens

<400> 39
tgggatctcc gcagctg                                                    17

<210> 40
<211> 17
<212> DNA
<213> Homo sapiens

<400> 40
gggatctccg cagctgc                                                    17

<210> 41
<211> 17
<212> DNA
<213> Homo sapiens

<400> 41
ggatctccgc agctgct                                                    17

<210> 42
<211> 17
<212> DNA
<213> Homo sapiens

<400> 42
gatctccgca gctgctt                                                    17

<210> 43
<211> 17
<212> DNA
<213> Homo sapiens

<400> 43
atctccgcag ctgcttt                                                    17

<210> 44
<211> 17
<212> DNA
<213> Homo sapiens

<400> 44
tctccgcagc tgctttt                                                    17

<210> 45
<211> 17
<212> DNA
<213> Homo sapiens

<400> 45
ctccgcagct gcttttc                                                    17

<210> 46
<211> 17
<212> DNA
<213> Homo sapiens

<400> 46
tccgcagctg cttttca                                                    17

<210> 47
<211> 17
<212> DNA
<213> Homo sapiens

<400> 47
ccgcagctgc ttttcac                                                    17

<210> 48

<211> 17
<212> DNA
<213> Homo sapiens

<400> 48
cgcagctgct tttcacc                                                    17

<210> 49
<211> 17
<212> DNA
<213> Homo sapiens

<400> 49
gcagctgctt ttcacct                                                    17

<210> 50
<211> 17
<212> DNA
<213> Homo sapiens

<400> 50
cagctgcttt tcacctg                                                    17

<210> 51
<211> 17
<212> DNA
<213> Homo sapiens

<400> 51
agctgctttt cacctgc                                                    17

<210> 52
<211> 17
<212> DNA
<213> Homo sapiens

<400> 52
gctgcttttc acctgct                                                    17

<210> 53
<211> 17
<212> DNA
<213> Homo sapiens

<400> 53
ctgcttttca cctgctg                                                    17

<210> 54
<211> 17
<212> DNA
<213> Homo sapiens

<400> 54
tgcttttcac ctgctgt                                                    17

<210> 55
<211> 17
<212> DNA
<213> Homo sapiens

<400> 55

gcttttcacc tgctgtt                                                    17

<210> 56
<211> 17
<212> DNA
<213> Homo sapiens

<400> 56
cttttcacct gctgttc                                                    17

<210> 57
<211> 17
<212> DNA
<213> Homo sapiens

<400> 57
ttttcacctg ctgttcc                                                    17

<210> 58
<211> 17
<212> DNA
<213> Homo sapiens

<400> 58
tttcacctgc tgttcct                                                    17

<210> 59
<211> 17
<212> DNA
<213> Homo sapiens

<400> 59
ttcacctgct gttcctc                                                    17

<210> 60
<211> 17
<212> DNA
<213> Homo sapiens

<400> 60
tcacctgctg ttcctcc                                                    17

<210> 61
<211> 17
<212> DNA
<213> Homo sapiens

<400> 61
cacctgctgt tcctcct                                                    17

<210> 62
<211> 17
<212> DNA
<213> Homo sapiens

<400> 62
acctgctgtt cctcctg                                                    17

<210> 63
<211> 17
<212> DNA

<213> Homo sapiens

<400> 63
cctgctgttc ctcctgc                                                    17

<210> 64
<211> 17
<212> DNA
<213> Homo sapiens

<400> 64
ctgctgttcc tcctgcg                                                    17

<210> 65
<211> 17
<212> DNA
<213> Homo sapiens

<400> 65
tgctgttcct cctgcgc                                                    17

<210> 66
<211> 17
<212> DNA
<213> Homo sapiens

<400> 66
gctgttcctc ctgcgct                                                    17

<210> 67
<211> 17
<212> DNA
<213> Homo sapiens

<400> 67
ctgttcctcc tgcgctt                                                    17

<210> 68
<211> 17
<212> DNA
<213> Homo sapiens

<400> 68
tgttcctcct gcgcttc                                                    17

<210> 69
<211> 17
<212> DNA
<213> Homo sapiens

<400> 69
gttcctcctg cgcttcc                                                    17

<210> 70
<211> 17
<212> DNA
<213> Homo sapiens

<400> 70
ttcctcctgc gcttcct                                                    17

<210> 71
<211> 17
<212> DNA
<213> Homo sapiens

<400> 71
tcctcctgcg cttccta                                                    17

<210> 72
<211> 17
<212> DNA
<213> Homo sapiens

<400> 72
cctcctgcgc ttcctaa                                                    17

<210> 73
<211> 17
<212> DNA
<213> Homo sapiens

<400> 73
ctcctgcgct tcctaag                                                    17

<210> 74
<211> 17
<212> DNA
<213> Homo sapiens

<400> 74
tcctgcgctt cctaaga                                                    17

<210> 75
<211> 17
<212> DNA
<213> Homo sapiens

<400> 75
cctgcgcttc ctaagag                                                    17

<210> 76
<211> 17
<212> DNA
<213> Homo sapiens

<400> 76
ctgcgcttcc taagagg                                                    17

<210> 77
<211> 17
<212> DNA
<213> Homo sapiens

<400> 77
tgcgcttcct aagagga                                                    17

<210> 78
<211> 17
<212> DNA
<213> Homo sapiens

<400> 78
gcgcttccta agaggaa                                                    17

<210> 79
<211> 17
<212> DNA
<213> Homo sapiens

<400> 79
cgcttcctaa gaggaag                                                    17

<210> 80
<211> 17
<212> DNA
<213> Homo sapiens

<400> 80
gcttcctaag aggaaga                                                    17

<210> 81
<211> 17
<212> DNA
<213> Homo sapiens

<400> 81
cttcctaaga ggaagaa                                                    17

<210> 82
<211> 17
<212> DNA
<213> Homo sapiens

<400> 82
ttcctaagag gaagaat                                                    17

<210> 83
<211> 17
<212> DNA
<213> Homo sapiens

<400> 83
tcctaagagg aagaatc                                                    17

<210> 84
<211> 17
<212> DNA
<213> Homo sapiens

<400> 84
cctaagagga agaatca                                                    17

<210> 85
<211> 17
<212> DNA
<213> Homo sapiens

<400> 85
ctaagaggaa gaatcaa                                                    17

<210> 86
<211> 17

<212> DNA
<213> Homo sapiens

<400> 86
taagaggaag aatcaat                                                    17

<210> 87
<211> 17
<212> DNA
<213> Homo sapiens

<400> 87
aagaggaaga atcaatg                                                    17

<210> 88
<211> 17
<212> DNA
<213> Homo sapiens

<400> 88
agaggaagaa tcaatgc                                                    17

<210> 89
<211> 17
<212> DNA
<213> Homo sapiens

<400> 89
gaggaagaat caatgcc                                                    17

<210> 90
<211> 17
<212> DNA
<213> Homo sapiens

<400> 90
aggaagaatc aatgccg                                                    17

<210> 91
<211> 17
<212> DNA
<213> Homo sapiens

<400> 91
ggaagaatca atgccgt                                                    17

<210> 92
<211> 17
<212> DNA
<213> Homo sapiens

<400> 92
gaagaatcaa tgccgtg                                                    17

<210> 93
<211> 17
<212> DNA
<213> Homo sapiens

<400> 93
aagaatcaat gccgtgg                                                    17

<210> 94
<211> 17
<212> DNA
<213> Homo sapiens

<400> 94
agaatcaatg ccgtggg                                                    17

<210> 95
<211> 17
<212> DNA
<213> Homo sapiens

<400> 95
gaatcaatgc cgtgggt                                                    17

<210> 96
<211> 17
<212> DNA
<213> Homo sapiens

<400> 96
aatcaatgcc gtgggtg                                                    17

<210> 97
<211> 17
<212> DNA
<213> Homo sapiens

<400> 97
atcaatgccg tgggtgg                                                    17

<210> 98
<211> 17
<212> DNA
<213> Homo sapiens

<400> 98
tcaatgccgt gggtgga                                                    17

<210> 99
<211> 17
<212> DNA
<213> Homo sapiens

<400> 99
caatgccgtg ggtggag                                                    17

<210> 100
<211> 17
<212> DNA
<213> Homo sapiens

<400> 100
aatgccgtgg gtggagc                                                    17

<210> 101
<211> 17
<212> DNA
<213> Homo sapiens

<400> 101
atgccgtggg tggagcc                                                    17

<210> 102
<211> 17
<212> DNA
<213> Homo sapiens

<400> 102
tgccgtgggt ggagccc                                                    17

<210> 103
<211> 17
<212> DNA
<213> Homo sapiens

<400> 103
gccgtgggtg gagccca                                                    17

<210> 104
<211> 17
<212> DNA
<213> Homo sapiens

<400> 104
ccgtgggtgg agcccaa                                                    17

<210> 105
<211> 17
<212> DNA
<213> Homo sapiens

<400> 105
cgtgggtgga gcccaag                                                    17

<210> 106
<211> 17
<212> DNA
<213> Homo sapiens

<400> 106
gtgggtggag cccaagc                                                    17

<210> 107
<211> 17
<212> DNA
<213> Homo sapiens

<400> 107
tgggtggagc ccaagcc                                                    17

<210> 108
<211> 17
<212> DNA
<213> Homo sapiens

<400> 108
gggtggagcc caagccc                                                    17

<210> 109

<211> 17
<212> DNA
<213> Homo sapiens

<400> 109
ggtggagccc aagccca                                                    17

<210> 110
<211> 17
<212> DNA
<213> Homo sapiens

<400> 110
gtggagccca agcccag                                                    17

<210> 111
<211> 17
<212> DNA
<213> Homo sapiens

<400> 111
tggagcccaa gcccagg                                                    17

<210> 112
<211> 17
<212> DNA
<213> Homo sapiens

<400> 112
ggagcccaag cccaggc                                                    17

<210> 113
<211> 17
<212> DNA
<213> Homo sapiens

<400> 113
gagcccaagc ccaggcc                                                    17

<210> 114
<211> 17
<212> DNA
<213> Homo sapiens

<400> 114
agcccaagcc caggccg                                                    17

<210> 115
<211> 17
<212> DNA
<213> Homo sapiens

<400> 115
gcccaagccc aggccgg                                                    17

<210> 116
<211> 17
<212> DNA
<213> Homo sapiens

<400> 116

cccaagccca ggccggg 17

<210> 117
<211> 17
<212> DNA
<213> Homo sapiens

<400> 117
ccaagcccag gccgggg 17

<210> 118
<211> 17
<212> DNA
<213> Homo sapiens

<400> 118
caagcccagg ccggggc 17

<210> 119
<211> 17
<212> DNA
<213> Homo sapiens

<400> 119
aagcccaggc cggggcc 17

<210> 120
<211> 17
<212> DNA
<213> Homo sapiens

<400> 120
agcccaggcc ggggccg 17

<210> 121
<211> 17
<212> DNA
<213> Homo sapiens

<400> 121
gcccaggccg gggccgg 17

<210> 122
<211> 17
<212> DNA
<213> Homo sapiens

<400> 122
cccaggccgg ggccgga 17

<210> 123
<211> 17
<212> DNA
<213> Homo sapiens

<400> 123
ccaggccggg gccggag 17

<210> 124
<211> 17
<212> DNA

<213> Homo sapiens

<400> 124
caggccgggg ccggagc                                                    17

<210> 125
<211> 17
<212> DNA
<213> Homo sapiens

<400> 125
aggccggggc cggagca                                                    17

<210> 126
<211> 17
<212> DNA
<213> Homo sapiens

<400> 126
ggccggggcc ggagcag                                                    17

<210> 127
<211> 17
<212> DNA
<213> Homo sapiens

<400> 127
gccggggccg gagcaga                                                    17

<210> 128
<211> 17
<212> DNA
<213> Homo sapiens

<400> 128
ccggggccgg agcagaa                                                    17

<210> 129
<211> 17
<212> DNA
<213> Homo sapiens

<400> 129
cggggccgga gcagaag                                                    17

<210> 130
<211> 17
<212> DNA
<213> Homo sapiens

<400> 130
ggggccggag cagaagc                                                    17

<210> 131
<211> 17
<212> DNA
<213> Homo sapiens

<400> 131
gggccggagc agaagcc                                                    17

<210> 132
<211> 17
<212> DNA
<213> Homo sapiens

<400> 132
ggccggagca gaagccc                                                    17

<210> 133
<211> 17
<212> DNA
<213> Homo sapiens

<400> 133
gccggagcag aagccca                                                    17

<210> 134
<211> 17
<212> DNA
<213> Homo sapiens

<400> 134
ccggagcaga agcccaa                                                    17

<210> 135
<211> 17
<212> DNA
<213> Homo sapiens

<400> 135
cggagcagaa gcccaag                                                    17

<210> 136
<211> 17
<212> DNA
<213> Homo sapiens

<400> 136
ggagcagaag cccaagc                                                    17

<210> 137
<211> 17
<212> DNA
<213> Homo sapiens

<400> 137
gagcagaagc ccaagct                                                    17

<210> 138
<211> 17
<212> DNA
<213> Homo sapiens

<400> 138
agcagaagcc caagctc                                                    17

<210> 139
<211> 17
<212> DNA
<213> Homo sapiens

<400> 139
gcagaagccc aagctca                                                    17

<210> 140
<211> 17
<212> DNA
<213> Homo sapiens

<400> 140
cagaagccca agctcac                                                    17

<210> 141
<211> 17
<212> DNA
<213> Homo sapiens

<400> 141
agaagcccaa gctcacc                                                    17

<210> 142
<211> 17
<212> DNA
<213> Homo sapiens

<400> 142
gaagcccaag ctcacca                                                    17

<210> 143
<211> 17
<212> DNA
<213> Homo sapiens

<400> 143
aagcccaagc tcaccaa                                                    17

<210> 144
<211> 17
<212> DNA
<213> Homo sapiens

<400> 144
agcccaagct caccaaa                                                    17

<210> 145
<211> 17
<212> DNA
<213> Homo sapiens

<400> 145
gcccaagctc accaaac                                                    17

<210> 146
<211> 17
<212> DNA
<213> Homo sapiens

<400> 146
cccaagctca ccaaacc                                                    17

<210> 147
<211> 17

<212> DNA
<213> Homo sapiens

<400> 147
ccaagctcac caaaccg                                                    17

<210> 148
<211> 17
<212> DNA
<213> Homo sapiens

<400> 148
caagctcacc aaaccgg                                                    17

<210> 149
<211> 17
<212> DNA
<213> Homo sapiens

<400> 149
aagctcacca aaccgga                                                    17

<210> 150
<211> 17
<212> DNA
<213> Homo sapiens

<400> 150
agctcaccaa accggac                                                    17

<210> 151
<211> 17
<212> DNA
<213> Homo sapiens

<400> 151
gctcaccaaa ccggact                                                    17

<210> 152
<211> 17
<212> DNA
<213> Homo sapiens

<400> 152
ctcaccaaac cggactc                                                    17

<210> 153
<211> 17
<212> DNA
<213> Homo sapiens

<400> 153
tcaccaaacc ggactct                                                    17

<210> 154
<211> 17
<212> DNA
<213> Homo sapiens

<400> 154
caccaaaccg gactctg                                                    17

<210> 155
<211> 17
<212> DNA
<213> Homo sapiens

<400> 155
accaaaccgg actctgc                                                    17

<210> 156
<211> 17
<212> DNA
<213> Homo sapiens

<400> 156
ccaaaccgga ctctgcc                                                    17

<210> 157
<211> 17
<212> DNA
<213> Homo sapiens

<400> 157
caaaccggac tctgcca                                                    17

<210> 158
<211> 17
<212> DNA
<213> Homo sapiens

<400> 158
aaaccggact ctgccac                                                    17

<210> 159
<211> 17
<212> DNA
<213> Homo sapiens

<400> 159
aaccggactc tgccacc                                                    17

<210> 160
<211> 17
<212> DNA
<213> Homo sapiens

<400> 160
accggactct gccaccg                                                    17

<210> 161
<211> 17
<212> DNA
<213> Homo sapiens

<400> 161
ccggactctg ccaccgg                                                    17

<210> 162
<211> 17
<212> DNA
<213> Homo sapiens

<400> 162
cggactctgc caccggg                                                    17

<210> 163
<211> 17
<212> DNA
<213> Homo sapiens

<400> 163
ggactctgcc accgggc                                                    17

<210> 164
<211> 17
<212> DNA
<213> Homo sapiens

<400> 164
gactctgcca ccgggcc                                                    17

<210> 165
<211> 17
<212> DNA
<213> Homo sapiens

<400> 165
actctgccac cgggccg                                                    17

<210> 166
<211> 17
<212> DNA
<213> Homo sapiens

<400> 166
ctctgccacc gggccgc                                                    17

<210> 167
<211> 17
<212> DNA
<213> Homo sapiens

<400> 167
tctgccaccg ggccgca                                                    17

<210> 168
<211> 17
<212> DNA
<213> Homo sapiens

<400> 168
ctgccaccgg gccgcag                                                    17

<210> 169
<211> 17
<212> DNA
<213> Homo sapiens

<400> 169
tgccaccggg ccgcagt                                                    17

<210> 170

<211> 17
<212> DNA
<213> Homo sapiens

<400> 170
gccaccgggc cgcagtg                                                    17

<210> 171
<211> 17
<212> DNA
<213> Homo sapiens

<400> 171
ccaccgggcc gcagtgg                                                    17

<210> 172
<211> 17
<212> DNA
<213> Homo sapiens

<400> 172
caccgggccg cagtggt                                                    17

<210> 173
<211> 17
<212> DNA
<213> Homo sapiens

<400> 173
accgggccgc agtggta                                                    17

<210> 174
<211> 17
<212> DNA
<213> Homo sapiens

<400> 174
ccgggccgca gtggtac                                                    17

<210> 175
<211> 17
<212> DNA
<213> Homo sapiens

<400> 175
cgggccgcag tggtacc                                                    17

<210> 176
<211> 17
<212> DNA
<213> Homo sapiens

<400> 176
gggccgcagt ggtacca                                                    17

<210> 177
<211> 17
<212> DNA
<213> Homo sapiens

<400> 177

ggccgcagtg gtaccag                                                    17

<210> 178
<211> 17
<212> DNA
<213> Homo sapiens

<400> 178
gccgcagtgg taccagg                                                    17

<210> 179
<211> 17
<212> DNA
<213> Homo sapiens

<400> 179
ccgcagtggt accagga                                                    17

<210> 180
<211> 17
<212> DNA
<213> Homo sapiens

<400> 180
cgcagtggta ccaggaa                                                    17

<210> 181
<211> 17
<212> DNA
<213> Homo sapiens

<400> 181
gcagtggtac caggaat                                                    17

<210> 182
<211> 17
<212> DNA
<213> Homo sapiens

<400> 182
cagtggtacc aggaatc                                                    17

<210> 183
<211> 17
<212> DNA
<213> Homo sapiens

<400> 183
agtggtacca ggaatct                                                    17

<210> 184
<211> 17
<212> DNA
<213> Homo sapiens

<400> 184
gtggtaccag gaatctc                                                    17

<210> 185
<211> 17
<212> DNA

<213> Homo sapiens

<400> 185
tggtaccagg aatctca                                                    17

<210> 186
<211> 17
<212> DNA
<213> Homo sapiens

<400> 186
ggtaccagga atctcag                                                    17

<210> 187
<211> 17
<212> DNA
<213> Homo sapiens

<400> 187
gtaccaggaa tctcagg                                                    17

<210> 188
<211> 17
<212> DNA
<213> Homo sapiens

<400> 188
taccaggaat ctcagga                                                    17

<210> 189
<211> 17
<212> DNA
<213> Homo sapiens

<400> 189
accaggaatc tcaggaa                                                    17

<210> 190
<211> 17
<212> DNA
<213> Homo sapiens

<400> 190
ccaggaatct caggaat                                                    17

<210> 191
<211> 17
<212> DNA
<213> Homo sapiens

<400> 191
caggaatctc aggaatc                                                    17

<210> 192
<211> 17
<212> DNA
<213> Homo sapiens

<400> 192
aggaatctca ggaatcg                                                    17

<210> 193
<211> 17
<212> DNA
<213> Homo sapiens

<400> 193
ggaatctcag gaatcgg                                                    17

<210> 194
<211> 17
<212> DNA
<213> Homo sapiens

<400> 194
gaatctcagg aatcgga                                                    17

<210> 195
<211> 17
<212> DNA
<213> Homo sapiens

<400> 195
aatctcagga atcggag                                                    17

<210> 196
<211> 17
<212> DNA
<213> Homo sapiens

<400> 196
atctcaggaa tcggagt                                                    17

<210> 197
<211> 17
<212> DNA
<213> Homo sapiens

<400> 197
tctcaggaat cggagtc                                                    17

<210> 198
<211> 17
<212> DNA
<213> Homo sapiens

<400> 198
ctcaggaatc ggagtcg                                                    17

<210> 199
<211> 17
<212> DNA
<213> Homo sapiens

<400> 199
tcaggaatcg gagtcgg                                                    17

<210> 200
<211> 17
<212> DNA
<213> Homo sapiens

<400> 200
caggaatcgg agtcgga                                                    17

<210> 201
<211> 17
<212> DNA
<213> Homo sapiens

<400> 201
aggaatcgga gtcggaa                                                    17

<210> 202
<211> 17
<212> DNA
<213> Homo sapiens

<400> 202
ggaatcggag tcggaag                                                    17

<210> 203
<211> 17
<212> DNA
<213> Homo sapiens

<400> 203
gaatcggagt cggaagg                                                    17

<210> 204
<211> 17
<212> DNA
<213> Homo sapiens

<400> 204
aatcggagtc ggaaggc                                                    17

<210> 205
<211> 17
<212> DNA
<213> Homo sapiens

<400> 205
atcggagtcg gaaggca                                                    17

<210> 206
<211> 17
<212> DNA
<213> Homo sapiens

<400> 206
tcggagtcgg aaggcaa                                                    17

<210> 207
<211> 17
<212> DNA
<213> Homo sapiens

<400> 207
cggagtcgga aggcaag                                                    17

<210> 208
<211> 17

<212> DNA
<213> Homo sapiens

<400> 208
ggagtcggaa ggcaagc                                                    17

<210> 209
<211> 17
<212> DNA
<213> Homo sapiens

<400> 209
gagtcggaag gcaagca                                                    17

<210> 210
<211> 17
<212> DNA
<213> Homo sapiens

<400> 210
agtcggaagg caagcag                                                    17

<210> 211
<211> 17
<212> DNA
<213> Homo sapiens

<400> 211
gtcggaaggc aagcagc                                                    17

<210> 212
<211> 17
<212> DNA
<213> Homo sapiens

<400> 212
tcggaaggca agcagcc                                                    17

<210> 213
<211> 17
<212> DNA
<213> Homo sapiens

<400> 213
cggaaggcaa gcagcca                                                    17

<210> 214
<211> 17
<212> DNA
<213> Homo sapiens

<400> 214
ggaaggcaag cagccac                                                    17

<210> 215
<211> 17
<212> DNA
<213> Homo sapiens

<400> 215
gaaggcaagc agccacc                                                    17

<210> 216
<211> 17
<212> DNA
<213> Homo sapiens

<400> 216
aaggcaagca gccaccc                                                    17

<210> 217
<211> 17
<212> DNA
<213> Homo sapiens

<400> 217
aggcaagcag ccacccc                                                    17

<210> 218
<211> 17
<212> DNA
<213> Homo sapiens

<400> 218
ggcaagcagc cacccccc                                                   17

<210> 219
<211> 17
<212> DNA
<213> Homo sapiens

<400> 219
gcaagcagcc acccccg                                                    17

<210> 220
<211> 17
<212> DNA
<213> Homo sapiens

<400> 220
caagcagcca cccccgg                                                    17

<210> 221
<211> 17
<212> DNA
<213> Homo sapiens

<400> 221
aagcagccac ccccggg                                                    17

<210> 222
<211> 17
<212> DNA
<213> Homo sapiens

<400> 222
agcagccacc cccggga                                                    17

<210> 223
<211> 17
<212> DNA
<213> Homo sapiens

<400> 223
gcagccaccc ccgggac                                                    17

<210> 224
<211> 17
<212> DNA
<213> Homo sapiens

<400> 224
cagccacccc cgggacc                                                    17

<210> 225
<211> 17
<212> DNA
<213> Homo sapiens

<400> 225
agccaccccc gggaccc                                                    17

<210> 226
<211> 17
<212> DNA
<213> Homo sapiens

<400> 226
gccacccccg ggacccc                                                    17

<210> 227
<211> 17
<212> DNA
<213> Homo sapiens

<400> 227
ccacccccgg gacccct                                                    17

<210> 228
<211> 17
<212> DNA
<213> Homo sapiens

<400> 228
cacccccggg acccctg                                                    17

<210> 229
<211> 17
<212> DNA
<213> Homo sapiens

<400> 229
acccccggga cccctgg                                                    17

<210> 230
<211> 17
<212> DNA
<213> Homo sapiens

<400> 230
cccccgggac ccctggc                                                    17

<210> 231

<211> 17
<212> DNA
<213> Homo sapiens

<400> 231
ccccgggacc cctggca                                                    17

<210> 232
<211> 17
<212> DNA
<213> Homo sapiens

<400> 232
cccgggaccc ctggcac                                                    17

<210> 233
<211> 17
<212> DNA
<213> Homo sapiens

<400> 233
ccgggacccc tggcacc                                                    17

<210> 234
<211> 17
<212> DNA
<213> Homo sapiens

<400> 234
cgggacccct ggcaccc                                                    17

<210> 235
<211> 17
<212> DNA
<213> Homo sapiens

<400> 235
gggacccctg gcacccc                                                    17

<210> 236
<211> 17
<212> DNA
<213> Homo sapiens

<400> 236
   ggacccctgg caccccc                                                   17

<210> 237
<211> 17
<212> DNA
<213> Homo sapiens

<400> 237
gacccctggc acccccg                                                    17

<210> 238
<211> 17
<212> DNA
<213> Homo sapiens

<400> 238

acccctggca cccccga                                                    17

<210> 239
<211> 17
<212> DNA
<213> Homo sapiens

<400> 239
cccctggcac ccccgaa                                                    17

<210> 240
<211> 17
<212> DNA
<213> Homo sapiens

<400> 240
ccctggcacc cccgaaa                                                    17

<210> 241
<211> 17
<212> DNA
<213> Homo sapiens

<400> 241
cctggcaccc ccgaaat                                                    17

<210> 242
<211> 17
<212> DNA
<213> Homo sapiens

<400> 242
ctggcacccc cgaaatc                                                    17

<210> 243
<211> 17
<212> DNA
<213> Homo sapiens

<400> 243
tggcaccccc gaaatcc                                                    17

<210> 244
<211> 17
<212> DNA
<213> Homo sapiens

<400> 244
ggcacccccg aaatccc                                                    17

<210> 245
<211> 17
<212> DNA
<213> Homo sapiens

<400> 245
gcacccccga aatcccc                                                    17

<210> 246
<211> 17
<212> DNA

<213> Homo sapiens

<400> 246
cacccccgaa atccccc                                                    17

<210> 247
<211> 17
<212> DNA
<213> Homo sapiens

<400> 247
acccccgaaa tcccccg                                                    17

<210> 248
<211> 17
<212> DNA
<213> Homo sapiens

<400> 248
cccccgaaat cccccga                                                    17

<210> 249
<211> 17
<212> DNA
<213> Homo sapiens

<400> 249
ccccgaaatc ccccgaa                                                    17

<210> 250
<211> 17
<212> DNA
<213> Homo sapiens

<400> 250
cccgaaatcc cccgaac                                                    17

<210> 251
<211> 17
<212> DNA
<213> Homo sapiens

<400> 251
ccgaaatccc ccgaacc                                                    17

<210> 252
<211> 17
<212> DNA
<213> Homo sapiens

<400> 252
cgaaatcccc cgaaccc                                                    17

<210> 253
<211> 17
<212> DNA
<213> Homo sapiens

<400> 253
gaaatccccc gaaccct                                                    17

<210> 254
<211> 17
<212> DNA
<213> Homo sapiens

<400> 254
aaatcccccg aaccctc                                                    17

<210> 255
<211> 17
<212> DNA
<213> Homo sapiens

<400> 255
aatcccccga accctca                                                    17

<210> 256
<211> 17
<212> DNA
<213> Homo sapiens

<400> 256
atcccccgaa ccctcag                                                    17

<210> 257
<211> 17
<212> DNA
<213> Homo sapiens

<400> 257
tcccccgaac cctcagg                                                    17

<210> 258
<211> 17
<212> DNA
<213> Homo sapiens

<400> 258
cccccgaacc ctcagga                                                    17

<210> 259
<211> 17
<212> DNA
<213> Homo sapiens

<400> 259
ccccgaaccc tcaggac                                                    17

<210> 260
<211> 17
<212> DNA
<213> Homo sapiens

<400> 260
cccgaaccct caggacc                                                    17

<210> 261
<211> 17
<212> DNA
<213> Homo sapiens

<400> 261
ccgaaccctc aggaccc                                                    17

<210> 262
<211> 17
<212> DNA
<213> Homo sapiens

<400> 262
cgaaccctca ggacccc                                                    17

<210> 263
<211> 17
<212> DNA
<213> Homo sapiens

<400> 263
gaaccctcag gacccct                                                    17

<210> 264
<211> 17
<212> DNA
<213> Homo sapiens

<400> 264
aaccctcagg acccctg                                                    17

<210> 265
<211> 17
<212> DNA
<213> Homo sapiens

<400> 265
accctcagga cccctgg                                                    17

<210> 266
<211> 17
<212> DNA
<213> Homo sapiens

<400> 266
ccctcaggac ccctggc                                                    17

<210> 267
<211> 17
<212> DNA
<213> Homo sapiens

<400> 267
cctcaggacc cctggcg                                                    17

<210> 268
<211> 17
<212> DNA
<213> Homo sapiens

<400> 268
ctcaggaccc ctggcgt                                                    17

<210> 269
<211> 17

<212> DNA
<213> Homo sapiens

<400> 269
tcaggacccc tggcgtc                                                    17

<210> 270
<211> 17
<212> DNA
<213> Homo sapiens

<400> 270
caggacccct ggcgtcg                                                    17

<210> 271
<211> 17
<212> DNA
<213> Homo sapiens

<400> 271
aggacccctg gcgtcgg                                                    17

<210> 272
<211> 17
<212> DNA
<213> Homo sapiens

<400> 272
ggacccctgg cgtcgga                                                    17

<210> 273
<211> 17
<212> DNA
<213> Homo sapiens

<400> 273
gacccctggc gtcggag                                                    17

<210> 274
<211> 17
<212> DNA
<213> Homo sapiens

<400> 274
acccctggcg tcggagc                                                    17

<210> 275
<211> 17
<212> DNA
<213> Homo sapiens

<400> 275
cccctggcgt cggagca                                                    17

<210> 276
<211> 17
<212> DNA
<213> Homo sapiens

<400> 276
ccctggcgtc ggagcag                                                    17

<210> 277
<211> 17
<212> DNA
<213> Homo sapiens

<400> 277
cctggcgtcg gagcagg                                                    17

<210> 278
<211> 17
<212> DNA
<213> Homo sapiens

<400> 278
ctggcgtcgg agcagga                                                    17

<210> 279
<211> 17
<212> DNA
<213> Homo sapiens

<400> 279
tggcgtcgga gcaggat                                                    17

<210> 280
<211> 17
<212> DNA
<213> Homo sapiens

<400> 280
ggcgtcggag caggatg                                                    17

<210> 281
<211> 17
<212> DNA
<213> Homo sapiens

<400> 281
gcgtcggagc aggatgc                                                    17

<210> 282
<211> 17
<212> DNA
<213> Homo sapiens

<400> 282
cgtcggagca ggatgca                                                    17

<210> 283
<211> 17
<212> DNA
<213> Homo sapiens

<400> 283
gtcggagcag gatgcac                                                    17

<210> 284
<211> 17
<212> DNA
<213> Homo sapiens

<400> 284
tcggagcagg atgcacc                                                    17

<210> 285
<211> 17
<212> DNA
<213> Homo sapiens

<400> 285
cggagcagga tgcaccc                                                    17

<210> 286
<211> 17
<212> DNA
<213> Homo sapiens

<400> 286
ggagcaggat gcacccc                                                    17

<210> 287
<211> 17
<212> DNA
<213> Homo sapiens

<400> 287
gagcaggatg cacccct                                                    17

<210> 288
<211> 17
<212> DNA
<213> Homo sapiens

<400> 288
agcaggatgc acccctg                                                    17

<210> 289
<211> 17
<212> DNA
<213> Homo sapiens

<400> 289
gcaggatgca cccctgc                                                    17

<210> 290
<211> 17
<212> DNA
<213> Homo sapiens

<400> 290
caggatgcac ccctgcc                                                    17

<210> 291
<211> 17
<212> DNA
<213> Homo sapiens

<400> 291
aggatgcacc cctgcca                                                    17

<210> 292

<211> 17
<212> DNA
<213> Homo sapiens

<400> 292
ggatgcaccc ctgccag                                                    17

<210> 293
<211> 17
<212> DNA
<213> Homo sapiens

<400> 293
gatgcacccc tgccaga                                                    17

<210> 294
<211> 17
<212> DNA
<213> Homo sapiens

<400> 294
atgcacccct gccagag                                                    17

<210> 295
<211> 17
<212> DNA
<213> Homo sapiens

<400> 295
tgcacccctg ccagagg                                                    17

<210> 296
<211> 17
<212> DNA
<213> Homo sapiens

<400> 296
gcacccctgc cagaggg                                                    17

<210> 297
<211> 17
<212> DNA
<213> Homo sapiens

<400> 297
cacccctgcc agagggg                                                    17

<210> 298
<211> 17
<212> DNA
<213> Homo sapiens

<400> 298
acccctgcca gaggggg                                                    17

<210> 299
<211> 17
<212> DNA
<213> Homo sapiens

<400> 299

cccctgccag aggggga                                                    17

<210> 300
<211> 17
<212> DNA
<213> Homo sapiens

<400> 300
ccctgccaga gggggac                                                    17

<210> 301
<211> 17
<212> DNA
<213> Homo sapiens

<400> 301
cctgccagag ggggacg                                                    17

<210> 302
<211> 17
<212> DNA
<213> Homo sapiens

<400> 302
ctgccagagg gggacga                                                    17

<210> 303
<211> 17
<212> DNA
<213> Homo sapiens

<400> 303
tgccagaggg ggacgat                                                    17

<210> 304
<211> 17
<212> DNA
<213> Homo sapiens

<400> 304
gccagagggg gacgatg                                                    17

<210> 305
<211> 17
<212> DNA
<213> Homo sapiens

<400> 305
ccagaggggg acgatgc                                                    17

<210> 306
<211> 17
<212> DNA
<213> Homo sapiens

<400> 306
cagaggggga cgatgca                                                    17

<210> 307
<211> 17
<212> DNA

<213> Homo sapiens

<400> 307
agagggggac gatgcac                                                    17

<210> 308
<211> 17
<212> DNA
<213> Homo sapiens

<400> 308
gagggggacg atgcacc                                                    17

<210> 309
<211> 17
<212> DNA
<213> Homo sapiens

<400> 309
agggggacga tgcaccc                                                    17

<210> 310
<211> 17
<212> DNA
<213> Homo sapiens

<400> 310
gggggacgat gcacccc                                                    17

<210> 311
<211> 17
<212> DNA
<213> Homo sapiens

<400> 311
   ggggacgatg caccccc                                                   17

<210> 312
<211> 17
<212> DNA
<213> Homo sapiens

<400> 312
   gggacgatgc acccccc                                                   17

<210> 313
<211> 17
<212> DNA
<213> Homo sapiens

<400> 313
ggacgatgca ccccccc                                                    17

<210> 314
<211> 17
<212> DNA
<213> Homo sapiens

<400> 314
gacgatgcac ccccccg                                                    17

<210> 315
<211> 17
<212> DNA
<213> Homo sapiens

<400> 315
acgatgcacc cccccgg                                                    17

<210> 316
<211> 17
<212> DNA
<213> Homo sapiens

<400> 316
cgatgcaccc ccccggc                                                    17

<210> 317
<211> 17
<212> DNA
<213> Homo sapiens

<400> 317
gatgcacccc cccggcc                                                    17

<210> 318
<211> 17
<212> DNA
<213> Homo sapiens

<400> 318
atgcaccccc ccggccg                                                    17

<210> 319
<211> 17
<212> DNA
<213> Homo sapiens

<400> 319
tgcacccccc cggccgt                                                    17

<210> 320
<211> 17
<212> DNA
<213> Homo sapiens

<400> 320
gcaccccccc ggccgtc                                                    17

<210> 321
<211> 17
<212> DNA
<213> Homo sapiens

<400> 321
cacccccccg gccgtcg                                                    17

<210> 322
<211> 17
<212> DNA
<213> Homo sapiens

<400> 322
   acccccccgg ccgtcga                                                    17

<210> 323
<211> 17
<212> DNA
<213> Homo sapiens

<400> 323
cccccccggc cgtcgat                                                     17

<210> 324
<211> 17
<212> DNA
<213> Homo sapiens

<400> 324
ccccccggcc gtcgatg                                                     17

<210> 325
<211> 17
<212> DNA
<213> Homo sapiens

<400> 325
cccccggccg tcgatgc                                                     17

<210> 326
<211> 17
<212> DNA
<213> Homo sapiens

<400> 326
ccccggccgt cgatgct                                                     17

<210> 327
<211> 17
<212> DNA
<213> Homo sapiens

<400> 327
cccggccgtc gatgctg                                                     17

<210> 328
<211> 17
<212> DNA
<213> Homo sapiens

<400> 328
ccggccgtcg atgctgg                                                     17

<210> 329
<211> 17
<212> DNA
<213> Homo sapiens

<400> 329
cggccgtcga tgctgga                                                     17

<210> 330
<211> 17

<212> DNA
<213> Homo sapiens

<400> 330
ggccgtcgat gctggac                                                    17

<210> 331
<211> 17
<212> DNA
<213> Homo sapiens

<400> 331
gccgtcgatg ctggacg                                                    17

<210> 332
<211> 17
<212> DNA
<213> Homo sapiens

<400> 332
ccgtcgatgc tggacga                                                    17

<210> 333
<211> 17
<212> DNA
<213> Homo sapiens

<400> 333
cgtcgatgct ggacgat                                                    17

<210> 334
<211> 17
<212> DNA
<213> Homo sapiens

<400> 334
gtcgatgctg gacgatg                                                    17

<210> 335
<211> 17
<212> DNA
<213> Homo sapiens

<400> 335
tcgatgctgg acgatgc                                                    17

<210> 336
<211> 17
<212> DNA
<213> Homo sapiens

<400> 336
cgatgctgga cgatgca                                                    17

<210> 337
<211> 17
<212> DNA
<213> Homo sapiens

<400> 337
gatgctggac gatgcac                                                    17

<210> 338
<211> 17
<212> DNA
<213> Homo sapiens

<400> 338
atgctggacg atgcacc                                                    17

<210> 339
<211> 17
<212> DNA
<213> Homo sapiens

<400> 339
tgctggacga tgcaccc                                                    17

<210> 340
<211> 17
<212> DNA
<213> Homo sapiens

<400> 340
gctggacgat gcacccc                                                    17

<210> 341
<211> 17
<212> DNA
<213> Homo sapiens

<400> 341
ctggacgatg caccccg                                                    17

<210> 342
<211> 17
<212> DNA
<213> Homo sapiens

<400> 342
tggacgatgc accccgc                                                    17

<210> 343
<211> 17
<212> DNA
<213> Homo sapiens

<400> 343
ggacgatgca ccccgcc                                                    17

<210> 344
<211> 17
<212> DNA
<213> Homo sapiens

<400> 344
gacgatgcac cccgcct                                                    17

<210> 345
<211> 17
<212> DNA
<213> Homo sapiens

<400> 345
acgatgcacc ccgcctg                                                    17

<210> 346
<211> 17
<212> DNA
<213> Homo sapiens

<400> 346
cgatgcaccc cgcctgc                                                    17

<210> 347
<211> 17
<212> DNA
<213> Homo sapiens

<400> 347
gatgcacccc gcctgcc                                                    17

<210> 348
<211> 17
<212> DNA
<213> Homo sapiens

<400> 348
atgcaccccg cctgccg                                                    17

<210> 349
<211> 17
<212> DNA
<213> Homo sapiens

<400> 349
tgcaccccgc ctgccgc                                                    17

<210> 350
<211> 17
<212> DNA
<213> Homo sapiens

<400> 350
gcaccccgcc tgccgct                                                    17

<210> 351
<211> 17
<212> DNA
<213> Homo sapiens

<400> 351
caccccgcct gccgctg                                                    17

<210> 352
<211> 17
<212> DNA
<213> Homo sapiens

<400> 352
accccgcctg ccgctgg                                                    17

<210> 353

<211> 17
<212> DNA
<213> Homo sapiens

<400> 353
ccccgcctgc cgctgga                                                    17

<210> 354
<211> 17
<212> DNA
<213> Homo sapiens

<400> 354
cccgcctgcc gctggag                                                    17

<210> 355
<211> 17
<212> DNA
<213> Homo sapiens

<400> 355
ccgcctgccg ctggagc                                                    17

<210> 356
<211> 17
<212> DNA
<213> Homo sapiens

<400> 356
cgcctgccgc tggagct                                                    17

<210> 357
<211> 17
<212> DNA
<213> Homo sapiens

<400> 357
gcctgccgct ggagctg                                                    17

<210> 358
<211> 17
<212> DNA
<213> Homo sapiens

<400> 358
cctgccgctg gagctgg                                                    17

<210> 359
<211> 17
<212> DNA
<213> Homo sapiens

<400> 359
ctgccgctgg agctgga                                                    17

<210> 360
<211> 17
<212> DNA
<213> Homo sapiens

<400> 360

tgccgctgga gctggac                                                    17

<210> 361
<211> 17
<212> DNA
<213> Homo sapiens

<400> 361
gccgctggag ctggacg                                                    17

<210> 362
<211> 17
<212> DNA
<213> Homo sapiens

<400> 362
ccgctggagc tggacga                                                    17

<210> 363
<211> 17
<212> DNA
<213> Homo sapiens

<400> 363
cgctggagct ggacgat                                                    17

<210> 364
<211> 17
<212> DNA
<213> Homo sapiens

<400> 364
gctggagctg gacgatg                                                    17

<210> 365
<211> 17
<212> DNA
<213> Homo sapiens

<400> 365
ctggagctgg acgatgc                                                    17

<210> 366
<211> 17
<212> DNA
<213> Homo sapiens

<400> 366
tggagctgga cgatgca                                                    17

<210> 367
<211> 17
<212> DNA
<213> Homo sapiens

<400> 367
ggagctggac gatgcac                                                    17

<210> 368
<211> 17
<212> DNA

<213> Homo sapiens

<400> 368
gagctggacg atgcacc                                                    17

<210> 369
<211> 17
<212> DNA
<213> Homo sapiens

<400> 369
agctggacga tgcaccc                                                    17

<210> 370
<211> 17
<212> DNA
<213> Homo sapiens

<400> 370
gctggacgat gcacccc                                                    17

<210> 371
<211> 17
<212> DNA
<213> Homo sapiens

<400> 371
ctggacgatg cacccct                                                    17

<210> 372
<211> 17
<212> DNA
<213> Homo sapiens

<400> 372
tggacgatgc acccctg                                                    17

<210> 373
<211> 17
<212> DNA
<213> Homo sapiens

<400> 373
ggacgatgca cccctgc                                                    17

<210> 374
<211> 17
<212> DNA
<213> Homo sapiens

<400> 374
gacgatgcac ccctgcc                                                    17

<210> 375
<211> 17
<212> DNA
<213> Homo sapiens

<400> 375
acgatgcacc cctgccg                                                    17

<210> 376
<211> 17
<212> DNA
<213> Homo sapiens

<400> 376
cgatgcaccc ctgccgg                                                    17

<210> 377
<211> 17
<212> DNA
<213> Homo sapiens

<400> 377
gatgcacccc tgccgga                                                    17

<210> 378
<211> 17
<212> DNA
<213> Homo sapiens

<400> 378
atgcacccct gccggag                                                    17

<210> 379
<211> 17
<212> DNA
<213> Homo sapiens

<400> 379
tgcacccctg ccggagg                                                    17

<210> 380
<211> 17
<212> DNA
<213> Homo sapiens

<400> 380
gcacccctgc cggagga                                                    17

<210> 381
<211> 17
<212> DNA
<213> Homo sapiens

<400> 381
cacccctgcc ggaggag                                                    17

<210> 382
<211> 17
<212> DNA
<213> Homo sapiens

<400> 382
acccctgccg gaggagg                                                    17

<210> 383
<211> 17
<212> DNA
<213> Homo sapiens

<400> 383
cccctgccgg aggagga                                                    17

<210> 384
<211> 17
<212> DNA
<213> Homo sapiens

<400> 384
ccctgccgga ggaggaa                                                    17

<210> 385
<211> 17
<212> DNA
<213> Homo sapiens

<400> 385
cctgccggag gaggaaa                                                    17

<210> 386
<211> 17
<212> DNA
<213> Homo sapiens

<400> 386
ctgccggagg aggaaac                                                    17

<210> 387
<211> 17
<212> DNA
<213> Homo sapiens

<400> 387
tgccggagga ggaaacc                                                    17

<210> 388
<211> 17
<212> DNA
<213> Homo sapiens

<400> 388
gccggaggag gaaaccc                                                    17

<210> 389
<211> 17
<212> DNA
<213> Homo sapiens

<400> 389
ccggaggagg aaacccc                                                    17

<210> 390
<211> 17
<212> DNA
<213> Homo sapiens

<400> 390
cggaggagga aaccccc                                                    17

<210> 391
<211> 17

<212> DNA
<213> Homo sapiens

<400> 391
ggaggaggaa acccccg                                                    17

<210> 392
<211> 17
<212> DNA
<213> Homo sapiens

<400> 392
gaggaggaaa cccccga                                                    17

<210> 393
<211> 17
<212> DNA
<213> Homo sapiens

<400> 393
aggaggaaac ccccgaa                                                    17

<210> 394
<211> 17
<212> DNA
<213> Homo sapiens

<400> 394
ggaggaaacc cccgaac                                                    17

<210> 395
<211> 17
<212> DNA
<213> Homo sapiens

<400> 395
gaggaaaccc ccgaacc                                                    17

<210> 396
<211> 17
<212> DNA
<213> Homo sapiens

<400> 396
aggaaacccc cgaaccc                                                    17

<210> 397
<211> 17
<212> DNA
<213> Homo sapiens

<400> 397
ggaaaccccc gaaccca                                                    17

<210> 398
<211> 17
<212> DNA
<213> Homo sapiens

<400> 398
gaaacccccg aacccac                                                    17

<210> 399
<211> 17
<212> DNA
<213> Homo sapiens

<400> 399
aaacccccga acccacg                                                    17

<210> 400
<211> 17
<212> DNA
<213> Homo sapiens

<400> 400
aacccccgaa cccacgg                                                    17

<210> 401
<211> 17
<212> DNA
<213> Homo sapiens

<400> 401
acccccgaac ccacggc                                                    17

<210> 402
<211> 17
<212> DNA
<213> Homo sapiens

<400> 402
cccccgaacc cacggcc                                                    17

<210> 403
<211> 17
<212> DNA
<213> Homo sapiens

<400> 403
ccccgaaccc acggcca                                                    17

<210> 404
<211> 17
<212> DNA
<213> Homo sapiens

<400> 404
cccgaaccca cggccat                                                    17

<210> 405
<211> 17
<212> DNA
<213> Homo sapiens

<400> 405
ccgaacccac ggccatc                                                    17

<210> 406
<211> 17
<212> DNA
<213> Homo sapiens

<400> 406
cgaacccacg gccatct                                                    17

<210> 407
<211> 17
<212> DNA
<213> Homo sapiens

<400> 407
gaacccacgg ccatctg                                                    17

<210> 408
<211> 17
<212> DNA
<213> Homo sapiens

<400> 408
aacccacggc catctgc                                                    17

<210> 409
<211> 17
<212> DNA
<213> Homo sapiens

<400> 409
acccacggcc atctgca                                                    17

<210> 410
<211> 17
<212> DNA
<213> Homo sapiens

<400> 410
cccacggcca tctgcag                                                    17

<210> 411
<211> 17
<212> DNA
<213> Homo sapiens

<400> 411
ccacggccat ctgcagg                                                    17

<210> 412
<211> 17
<212> DNA
<213> Homo sapiens

<400> 412
cacggccatc tgcaggc                                                    17

<210> 413
<211> 17
<212> DNA
<213> Homo sapiens

<400> 413
acggccatct gcaggca                                                    17

<210> 414

<211> 17
<212> DNA
<213> Homo sapiens

<400> 414
cggccatctg caggcac                                                    17

<210> 415
<211> 17
<212> DNA
<213> Homo sapiens

<400> 415
ggccatctgc aggcacc                                                    17

<210> 416
<211> 17
<212> DNA
<213> Homo sapiens

<400> 416
gccatctgca ggcaccg                                                    17

<210> 417
<211> 17
<212> DNA
<213> Homo sapiens

<400> 417
ccatctgcag gcaccgg                                                    17

<210> 418
<211> 17
<212> DNA
<213> Homo sapiens

<400> 418
catctgcagg caccggc                                                    17

<210> 419
<211> 17
<212> DNA
<213> Homo sapiens

<400> 419
atctgcaggc accggca                                                    17

<210> 420
<211> 17
<212> DNA
<213> Homo sapiens

<400> 420
tctgcaggca ccggcac                                                    17

<210> 421
<211> 17
<212> DNA
<213> Homo sapiens

<400> 421

ctgcaggcac cggcacc                                                    17

<210> 422
<211> 17
<212> DNA
<213> Homo sapiens

<400> 422
tgcaggcacc ggcaccg                                                    17

<210> 423
<211> 17
<212> DNA
<213> Homo sapiens

<400> 423
gcaggcaccg gcaccgc                                                    17

<210> 424
<211> 17
<212> DNA
<213> Homo sapiens

<400> 424
caggcaccgg caccgct                                                    17

<210> 425
<211> 17
<212> DNA
<213> Homo sapiens

<400> 425
aggcaccggc accgctg                                                    17

<210> 426
<211> 17
<212> DNA
<213> Homo sapiens

<400> 426
ggcaccggca ccgctgt                                                    17

<210> 427
<211> 17
<212> DNA
<213> Homo sapiens

<400> 427
gcaccggcac cgctgtc                                                    17

<210> 428
<211> 17
<212> DNA
<213> Homo sapiens

<400> 428
caccggcacc gctgtca                                                    17

<210> 429
<211> 17
<212> DNA

<213> Homo sapiens

<400> 429
accggcaccg ctgtcac                                                    17

<210> 430
<211> 17
<212> DNA
<213> Homo sapiens

<400> 430
ccggcaccgc tgtcaca                                                    17

<210> 431
<211> 17
<212> DNA
<213> Homo sapiens

<400> 431
cggcaccgct gtcacac                                                    17

<210> 432
<211> 17
<212> DNA
<213> Homo sapiens

<400> 432
ggcaccgctg tcacacc                                                    17

<210> 433
<211> 17
<212> DNA
<213> Homo sapiens

<400> 433
gcaccgctgt cacaccg                                                    17

<210> 434
<211> 17
<212> DNA
<213> Homo sapiens

<400> 434
caccgctgtc acaccga                                                    17

<210> 435
<211> 17
<212> DNA
<213> Homo sapiens

<400> 435
accgctgtca caccgac                                                    17

<210> 436
<211> 17
<212> DNA
<213> Homo sapiens

<400> 436
ccgctgtcac accgact                                                    17

<210> 437
<211> 17
<212> DNA
<213> Homo sapiens

<400> 437
cgctgtcaca ccgactg                                                    17

<210> 438
<211> 17
<212> DNA
<213> Homo sapiens

<400> 438
gctgtcacac cgactgc                                                    17

<210> 439
<211> 17
<212> DNA
<213> Homo sapiens

<400> 439
ctgtcacacc gactgcc                                                    17

<210> 440
<211> 17
<212> DNA
<213> Homo sapiens

<400> 440
tgtcacaccg actgcct                                                    17

<210> 441
<211> 17
<212> DNA
<213> Homo sapiens

<400> 441
gtcacaccga ctgccta                                                    17

<210> 442
<211> 17
<212> DNA
<213> Homo sapiens

<400> 442
tcacaccgac tgcctag                                                    17

<210> 443
<211> 17
<212> DNA
<213> Homo sapiens

<400> 443
cacaccgact gcctaga                                                    17

<210> 444
<211> 17
<212> DNA
<213> Homo sapiens

<400> 444
acaccgactg cctagag                                                    17

<210> 445
<211> 17
<212> DNA
<213> Homo sapiens

<400> 445
caccgactgc ctagagg                                                    17

<210> 446
<211> 17
<212> DNA
<213> Homo sapiens

<400> 446
accgactgcc tagaggg                                                    17

<210> 447
<211> 17
<212> DNA
<213> Homo sapiens

<400> 447
ccgactgcct agagggg                                                    17

<210> 448
<211> 17
<212> DNA
<213> Homo sapiens

<400> 448
cgactgccta gaggggc                                                    17

<210> 449
<211> 17
<212> DNA
<213> Homo sapiens

<400> 449
gactgcctag aggggct                                                    17

<210> 450
<211> 17
<212> DNA
<213> Homo sapiens

<400> 450
actgcctaga ggggctg                                                    17

<210> 451
<211> 17
<212> DNA
<213> Homo sapiens

<400> 451
ctgcctagag gggctgc                                                    17

<210> 452
<211> 17

<212> DNA
<213> Homo sapiens

<400> 452
tgcctagagg ggctgct                                                    17

<210> 453
<211> 17
<212> DNA
<213> Homo sapiens

<400> 453
gcctagaggg gctgctg                                                    17

<210> 454
<211> 17
<212> DNA
<213> Homo sapiens

<400> 454
cctagagggg ctgctgt                                                    17

<210> 455
<211> 17
<212> DNA
<213> Homo sapiens

<400> 455
ctagaggggc tgctgtc                                                    17

<210> 456
<211> 17
<212> DNA
<213> Homo sapiens

<400> 456
tagaggggct gctgtcc                                                    17

<210> 457
<211> 17
<212> DNA
<213> Homo sapiens

<400> 457
agaggggctg ctgtccc                                                    17

<210> 458
<211> 17
<212> DNA
<213> Homo sapiens

<400> 458
gaggggctgc tgtcccg                                                    17

<210> 459
<211> 17
<212> DNA
<213> Homo sapiens

<400> 459
aggggctgct gtcccgc                                                    17

<210> 460
<211> 17
<212> DNA
<213> Homo sapiens

<400> 460
ggggctgctg tcccgca                                                    17

<210> 461
<211> 17
<212> DNA
<213> Homo sapiens

<400> 461
gggctgctgt cccgcac                                                    17

<210> 462
<211> 17
<212> DNA
<213> Homo sapiens

<400> 462
ggctgctgtc ccgcacc                                                    17

<210> 463
<211> 17
<212> DNA
<213> Homo sapiens

<400> 463
gctgctgtcc cgcacct                                                    17

<210> 464
<211> 17
<212> DNA
<213> Homo sapiens

<400> 464
ctgctgtccc gcacctt                                                    17

<210> 465
<211> 17
<212> DNA
<213> Homo sapiens

<400> 465
tgctgtcccg caccttc                                                    17

<210> 466
<211> 17
<212> DNA
<213> Homo sapiens

<400> 466
gctgtcccgc accttcc                                                    17

<210> 467
<211> 17
<212> DNA
<213> Homo sapiens

<400> 467
ctgtcccgca ccttcca                                                    17

<210> 468
<211> 17
<212> DNA
<213> Homo sapiens

<400> 468
tgtcccgcac cttccag                                                    17

<210> 469
<211> 17
<212> DNA
<213> Homo sapiens

<400> 469
gtcccgcacc ttccagt                                                    17

<210> 470
<211> 17
<212> DNA
<213> Homo sapiens

<400> 470
tcccgcacct tccagtg                                                    17

<210> 471
<211> 17
<212> DNA
<213> Homo sapiens

<400> 471
cccgcacctt ccagtgg                                                    17

<210> 472
<211> 17
<212> DNA
<213> Homo sapiens

<400> 472
ccgcaccttc cagtggc                                                    17

<210> 473
<211> 17
<212> DNA
<213> Homo sapiens

<400> 473
cgcaccttcc agtggct                                                    17

<210> 474
<211> 17
<212> DNA
<213> Homo sapiens

<400> 474
gcaccttcca gtggctg                                                    17

<210> 475

<211> 17
<212> DNA
<213> Homo sapiens

<400> 475
caccttccag tggctgg                                                    17

<210> 476
<211> 17
<212> DNA
<213> Homo sapiens

<400> 476
accttccagt ggctggg                                                    17

<210> 477
<211> 17
<212> DNA
<213> Homo sapiens

<400> 477
ccttccagtg gctgggg                                                    17

<210> 478
<211> 17
<212> DNA
<213> Homo sapiens

<400> 478
cttccagtgg ctggggt                                                    17

<210> 479
<211> 17
<212> DNA
<213> Homo sapiens

<400> 479
ttccagtggc tggggtg                                                    17

<210> 480
<211> 17
<212> DNA
<213> Homo sapiens

<400> 480
tccagtggct ggggtgg                                                    17

<210> 481
<211> 17
<212> DNA
<213> Homo sapiens

<400> 481
ccagtggctg gggtggc                                                    17

<210> 482
<211> 17
<212> DNA
<213> Homo sapiens

<400> 482

cagtggctgg ggtggca                                                    17

<210> 483
<211> 17
<212> DNA
<213> Homo sapiens

<400> 483
agtggctggg gtggcag                                                    17

<210> 484
<211> 17
<212> DNA
<213> Homo sapiens

<400> 484
gtggctgggg tggcagg                                                    17

<210> 485
<211> 17
<212> DNA
<213> Homo sapiens

<400> 485
tggctggggt ggcaggt                                                    17

<210> 486
<211> 17
<212> DNA
<213> Homo sapiens

<400> 486
ggctggggtg gcaggtg                                                    17

<210> 487
<211> 17
<212> DNA
<213> Homo sapiens

<400> 487
gctggggtgg caggtgg                                                    17

<210> 488
<211> 17
<212> DNA
<213> Homo sapiens

<400> 488
ctggggtggc aggtggg                                                    17

<210> 489
<211> 17
<212> DNA
<213> Homo sapiens

<400> 489
tggggtggca ggtgggc                                                    17

<210> 490
<211> 17
<212> DNA

<213> Homo sapiens

<400> 490
ggggtggcag gtgggcg                                                      17

<210> 491
<211> 17
<212> DNA
<213> Homo sapiens

<400> 491
gggtggcagg tgggcgc                                                      17

<210> 492
<211> 17
<212> DNA
<213> Homo sapiens

<400> 492
ggtggcaggt gggcgcg                                                      17

<210> 493
<211> 17
<212> DNA
<213> Homo sapiens

<400> 493
gtggcaggtg ggcgcgc                                                      17

<210> 494
<211> 17
<212> DNA
<213> Homo sapiens

<400> 494
tggcaggtgg gcgcgca                                                      17

<210> 495
<211> 17
<212> DNA
<213> Homo sapiens

<400> 495
ggcaggtggg cgcgcac                                                      17

<210> 496
<211> 17
<212> DNA
<213> Homo sapiens

<400> 496
gcaggtgggc gcgcacc                                                      17

<210> 497
<211> 17
<212> DNA
<213> Homo sapiens

<400> 497
caggtgggcg cgcaccc                                                      17

<210> 498
<211> 17
<212> DNA
<213> Homo sapiens

<400> 498
aggtgggcgc gcacccc                                                    17

<210> 499
<211> 17
<212> DNA
<213> Homo sapiens

<400> 499
ggtgggcgcg cacccct                                                    17

<210> 500
<211> 17
<212> DNA
<213> Homo sapiens

<400> 500
gtgggcgcgc acccctg                                                    17

<210> 501
<211> 17
<212> DNA
<213> Homo sapiens

<400> 501
tgggcgcgca cccctgg                                                    17

<210> 502
<211> 17
<212> DNA
<213> Homo sapiens

<400> 502
gggcgcgcac ccctgga                                                    17

<210> 503
<211> 17
<212> DNA
<213> Homo sapiens

<400> 503
ggcgcgcacc cctggat                                                    17

<210> 504
<211> 17
<212> DNA
<213> Homo sapiens

<400> 504
gcgcgcaccc ctggatc                                                    17

<210> 505
<211> 17
<212> DNA
<213> Homo sapiens

<400> 505
cgcgcacccc tggatct                                                    17

<210> 506
<211> 17
<212> DNA
<213> Homo sapiens

<400> 506
gcgcacccct ggatctt                                                    17

<210> 507
<211> 17
<212> DNA
<213> Homo sapiens

<400> 507
cgcacccctg gatcttc                                                    17

<210> 508
<211> 17
<212> DNA
<213> Homo sapiens

<400> 508
gcacccctgg atcttcc                                                    17

<210> 509
<211> 17
<212> DNA
<213> Homo sapiens

<400> 509
cacccctgga tcttcct                                                    17

<210> 510
<211> 17
<212> DNA
<213> Homo sapiens

<400> 510
acccctggat cttcctg                                                    17

<210> 511
<211> 17
<212> DNA
<213> Homo sapiens

<400> 511
cccctggatc ttcctgc                                                    17

<210> 512
<211> 17
<212> DNA
<213> Homo sapiens

<400> 512
ccctggatct tcctgct                                                    17

<210> 513
<211> 17

<212> DNA
<213> Homo sapiens

<400> 513
cctggatctt cctgctg                                                    17

<210> 514
<211> 17
<212> DNA
<213> Homo sapiens

<400> 514
ctggatcttc ctgctgg                                                    17

<210> 515
<211> 17
<212> DNA
<213> Homo sapiens

<400> 515
tggatcttcc tgctggc                                                    17

<210> 516
<211> 17
<212> DNA
<213> Homo sapiens

<400> 516
ggatcttcct gctggcg                                                    17

<210> 517
<211> 17
<212> DNA
<213> Homo sapiens

<400> 517
gatcttcctg ctggcgc                                                    17

<210> 518
<211> 17
<212> DNA
<213> Homo sapiens

<400> 518
atcttcctgc tggcgcc                                                    17

<210> 519
<211> 17
<212> DNA
<213> Homo sapiens

<400> 519
tcttcctgct ggcgccc                                                    17

<210> 520
<211> 17
<212> DNA
<213> Homo sapiens

<400> 520
cttcctgctg gcgccct                                                    17

<210> 521
<211> 17
<212> DNA
<213> Homo sapiens

<400> 521
ttcctgctgg cgccctt                                                    17

<210> 522
<211> 17
<212> DNA
<213> Homo sapiens

<400> 522
tcctgctggc gcccttg                                                    17

<210> 523
<211> 17
<212> DNA
<213> Homo sapiens

<400> 523
cctgctggcg cccttga                                                    17

<210> 524
<211> 17
<212> DNA
<213> Homo sapiens

<400> 524
ctgctggcgc ccttgat                                                    17

<210> 525
<211> 17
<212> DNA
<213> Homo sapiens

<400> 525
tgctggcgcc cttgatg                                                    17

<210> 526
<211> 17
<212> DNA
<213> Homo sapiens

<400> 526
gctggcgccc ttgatgc                                                    17

<210> 527
<211> 17
<212> DNA
<213> Homo sapiens

<400> 527
ctggcgccct tgatgct                                                    17

<210> 528
<211> 17
<212> DNA
<213> Homo sapiens

<400> 528
tggcgccctt gatgctg                                                    17

<210> 529
<211> 17
<212> DNA
<213> Homo sapiens

<400> 529
ggcgcccttg atgctga                                                    17

<210> 530
<211> 17
<212> DNA
<213> Homo sapiens

<400> 530
gcgcccttga tgctgac                                                    17

<210> 531
<211> 17
<212> DNA
<213> Homo sapiens

<400> 531
cgcccttgat gctgaca                                                    17

<210> 532
<211> 17
<212> DNA
<213> Homo sapiens

<400> 532
gcccttgatg ctgacag                                                    17

<210> 533
<211> 17
<212> DNA
<213> Homo sapiens

<400> 533
cccttgatgc tgacagc                                                    17

<210> 534
<211> 17
<212> DNA
<213> Homo sapiens

<400> 534
ccttgatgct gacagcc                                                    17

<210> 535
<211> 17
<212> DNA
<213> Homo sapiens

<400> 535
cttgatgctg acagccg                                                    17

<210> 536

<211> 17
<212> DNA
<213> Homo sapiens

<400> 536
ttgatgctga cagccgc                                                    17

<210> 537
<211> 17
<212> DNA
<213> Homo sapiens

<400> 537
tgatgctgac agccgcg                                                    17

<210> 538
<211> 17
<212> DNA
<213> Homo sapiens

<400> 538
gatgctgaca gccgcgc                                                    17

<210> 539
<211> 17
<212> DNA
<213> Homo sapiens

<400> 539
atgctgacag ccgcgct                                                    17

<210> 540
<211> 17
<212> DNA
<213> Homo sapiens

<400> 540
tgctgacagc cgcgctg                                                    17

<210> 541
<211> 17
<212> DNA
<213> Homo sapiens

<400> 541
gctgacagcc gcgctgg                                                    17

<210> 542
<211> 17
<212> DNA
<213> Homo sapiens

<400> 542
ctgacagccg cgctggg                                                    17

<210> 543
<211> 17
<212> DNA
<213> Homo sapiens

<400> 543

tgacagccgc gctgggc                                                    17

<210> 544
<211> 17
<212> DNA
<213> Homo sapiens

<400> 544
gacagccgcg ctgggca                                                    17

<210> 545
<211> 17
<212> DNA
<213> Homo sapiens

<400> 545
acagccgcgc tgggcac                                                    17

<210> 546
<211> 17
<212> DNA
<213> Homo sapiens

<400> 546
cagccgcgct gggcacc                                                    17

<210> 547
<211> 17
<212> DNA
<213> Homo sapiens

<400> 547
agccgcgctg ggcaccg                                                    17

<210> 548
<211> 17
<212> DNA
<213> Homo sapiens

<400> 548
gccgcgctgg gcaccgg                                                    17

<210> 549
<211> 17
<212> DNA
<213> Homo sapiens

<400> 549
ccgcgctggg caccggc                                                    17

<210> 550
<211> 17
<212> DNA
<213> Homo sapiens

<400> 550
cgcgctgggc accggct                                                    17

<210> 551
<211> 17
<212> DNA

<213> Homo sapiens

<400> 551
gcgctgggca ccggctt                                                    17

<210> 552
<211> 17
<212> DNA
<213> Homo sapiens

<400> 552
cgctgggcac cggcttc                                                    17

<210> 553
<211> 17
<212> DNA
<213> Homo sapiens

<400> 553
gctgggcacc ggcttcc                                                    17

<210> 554
<211> 17
<212> DNA
<213> Homo sapiens

<400> 554
ctgggcaccg gcttcct                                                    17

<210> 555
<211> 17
<212> DNA
<213> Homo sapiens

<400> 555
tgggcaccgg cttcctg                                                    17

<210> 556
<211> 17
<212> DNA
<213> Homo sapiens

<400> 556
gggcaccggc ttcctgt                                                    17

<210> 557
<211> 17
<212> DNA
<213> Homo sapiens

<400> 557
ggcaccggct tcctgta                                                    17

<210> 558
<211> 17
<212> DNA
<213> Homo sapiens

<400> 558
gcaccggctt cctgtac                                                    17

<210> 559
<211> 17
<212> DNA
<213> Homo sapiens

<400> 559
caccggcttc ctgtacc                                                    17

<210> 560
<211> 17
<212> DNA
<213> Homo sapiens

<400> 560
accggcttcc tgtacct                                                    17

<210> 561
<211> 17
<212> DNA
<213> Homo sapiens

<400> 561
ccggcttcct gtaccta                                                    17

<210> 562
<211> 17
<212> DNA
<213> Homo sapiens

<400> 562
cggcttcctg tacctac                                                    17

<210> 563
<211> 17
<212> DNA
<213> Homo sapiens

<400> 563
ggcttcctgt acctacc                                                    17

<210> 564
<211> 17
<212> DNA
<213> Homo sapiens

<400> 564
gcttcctgta cctaccc                                                    17

<210> 565
<211> 17
<212> DNA
<213> Homo sapiens

<400> 565
cttcctgtac ctaccca                                                    17

<210> 566
<211> 17
<212> DNA
<213> Homo sapiens

<400> 566
ttcctgtacc tacccaa                                                    17

<210> 567
<211> 17
<212> DNA
<213> Homo sapiens

<400> 567
tcctgtacct acccaag                                                    17

<210> 568
<211> 17
<212> DNA
<213> Homo sapiens

<400> 568
cctgtaccta cccaagg                                                    17

<210> 569
<211> 17
<212> DNA
<213> Homo sapiens

<400> 569
ctgtacctac ccaagga                                                    17

<210> 570
<211> 17
<212> DNA
<213> Homo sapiens

<400> 570
tgtacctacc caaggac                                                    17

<210> 571
<211> 17
<212> DNA
<213> Homo sapiens

<400> 571
gtacctaccc aaggacg                                                    17

<210> 572
<211> 17
<212> DNA
<213> Homo sapiens

<400> 572
tacctaccca aggacga                                                    17

<210> 573
<211> 17
<212> DNA
<213> Homo sapiens

<400> 573
acctacccaa ggacgaa                                                    17

<210> 574
<211> 17

<212> DNA
<213> Homo sapiens

<400> 574
cctacccaag gacgaag                                                    17

<210> 575
<211> 17
<212> DNA
<213> Homo sapiens

<400> 575
ctacccaagg acgaaga                                                    17

<210> 576
<211> 17
<212> DNA
<213> Homo sapiens

<400> 576
tacccaagga cgaagag                                                    17

<210> 577
<211> 17
<212> DNA
<213> Homo sapiens

<400> 577
acccaaggac gaagagg                                                    17

<210> 578
<211> 17
<212> DNA
<213> Homo sapiens

<400> 578
cccaaggacg aagagga                                                    17

<210> 579
<211> 17
<212> DNA
<213> Homo sapiens

<400> 579
ccaaggacga agaggaa                                                    17

<210> 580
<211> 17
<212> DNA
<213> Homo sapiens

<400> 580
caaggacgaa gaggaag                                                    17

<210> 581
<211> 17
<212> DNA
<213> Homo sapiens

<400> 581
aaggacgaag aggaaga                                                    17

<210> 582
<211> 17
<212> DNA
<213> Homo sapiens

<400> 582
aggacgaaga ggaagac                                                    17

<210> 583
<211> 17
<212> DNA
<213> Homo sapiens

<400> 583
ggacgaagag gaagacc                                                    17

<210> 584
<211> 17
<212> DNA
<213> Homo sapiens

<400> 584
gacgaagagg aagacct                                                    17

<210> 585
<211> 17
<212> DNA
<213> Homo sapiens

<400> 585
acgaagagga agaccta                                                    17

<210> 586
<211> 17
<212> DNA
<213> Homo sapiens

<400> 586
cgaagaggaa gacctag                                                    17

<210> 587
<211> 17
<212> DNA
<213> Homo sapiens

<400> 587
gaagaggaag acctaga                                                    17

<210> 588
<211> 17
<212> DNA
<213> Homo sapiens

<400> 588
aagaggaaga cctagag                                                    17

<210> 589
<211> 17
<212> DNA
<213> Homo sapiens

<400> 589
agaggaagac ctagagg                                                      17

<210> 590
<211> 17
<212> DNA
<213> Homo sapiens

<400> 590
gaggaagacc tagagga                                                      17

<210> 591
<211> 17
<212> DNA
<213> Homo sapiens

<400> 591
aggaagacct agaggag                                                      17

<210> 592
<211> 17
<212> DNA
<213> Homo sapiens

<400> 592
ggaagaccta gaggagc                                                      17

<210> 593
<211> 17
<212> DNA
<213> Homo sapiens

<400> 593
gaagacctag aggagca                                                      17

<210> 594
<211> 17
<212> DNA
<213> Homo sapiens

<400> 594
aagacctaga ggagcat                                                      17

<210> 595
<211> 17
<212> DNA
<213> Homo sapiens

<400> 595
agacctagag gagcatt                                                      17

<210> 596
<211> 17
<212> DNA
<213> Homo sapiens

<400> 596
gacctagagg agcatta                                                      17

<210> 597

<211> 17
<212> DNA
<213> Homo sapiens

<400> 597
acctagagga gcattac                                                    17

<210> 598
<211> 17
<212> DNA
<213> Homo sapiens

<400> 598
cctagaggag cattaca                                                    17

<210> 599
<211> 17
<212> DNA
<213> Homo sapiens

<400> 599
ctagaggagc attacac                                                    17

<210> 600
<211> 17
<212> DNA
<213> Homo sapiens

<400> 600
tagaggagca ttacacc                                                    17

<210> 601
<211> 17
<212> DNA
<213> Homo sapiens

<400> 601
agaggagcat tacaccc                                                    17

<210> 602
<211> 17
<212> DNA
<213> Homo sapiens

<400> 602
gaggagcatt acacccc                                                    17

<210> 603
<211> 17
<212> DNA
<213> Homo sapiens

<400> 603
aggagcatta cacccct                                                    17

<210> 604
<211> 17
<212> DNA
<213> Homo sapiens

<400> 604

ggagcattac acccctg                                                    17

<210> 605
<211> 17
<212> DNA
<213> Homo sapiens

<400> 605
gagcattaca cccctgt                                                    17

<210> 606
<211> 17
<212> DNA
<213> Homo sapiens

<400> 606
agcattacac ccctgtg                                                    17

<210> 607
<211> 17
<212> DNA
<213> Homo sapiens

<400> 607
gcattacacc cctgtgg                                                    17

<210> 608
<211> 17
<212> DNA
<213> Homo sapiens

<400> 608
cattacaccc ctgtggg                                                    17

<210> 609
<211> 17
<212> DNA
<213> Homo sapiens

<400> 609
attacacccc tgtgggg                                                    17

<210> 610
<211> 17
<212> DNA
<213> Homo sapiens

<400> 610
ttacacccct gtgggga                                                    17

<210> 611
<211> 17
<212> DNA
<213> Homo sapiens

<400> 611
tacacccctg tggggag                                                    17

<210> 612
<211> 17
<212> DNA

<213> Homo sapiens

<400> 612
acacccctgt ggggagc                                                    17

<210> 613
<211> 17
<212> DNA
<213> Homo sapiens

<400> 613
cacccctgtg gggagcc                                                    17

<210> 614
<211> 17
<212> DNA
<213> Homo sapiens

<400> 614
acccctgtgg ggagccc                                                    17

<210> 615
<211> 17
<212> DNA
<213> Homo sapiens

<400> 615
cccctgtggg gagcccg                                                    17

<210> 616
<211> 17
<212> DNA
<213> Homo sapiens

<400> 616
ccctgtgggg agcccgg                                                    17

<210> 617
<211> 17
<212> DNA
<213> Homo sapiens

<400> 617
cctgtgggga gcccggc                                                    17

<210> 618
<211> 17
<212> DNA
<213> Homo sapiens

<400> 618
ctgtggggag cccggcc                                                    17

<210> 619
<211> 17
<212> DNA
<213> Homo sapiens

<400> 619
tgtggggagc ccggcca                                                    17

<210> 620
<211> 17
<212> DNA
<213> Homo sapiens

<400> 620
gtggggagcc cggccaa                                                    17

<210> 621
<211> 17
<212> DNA
<213> Homo sapiens

<400> 621
tggggagccc ggccaag                                                    17

<210> 622
<211> 17
<212> DNA
<213> Homo sapiens

<400> 622
ggggagcccg gccaagg                                                    17

<210> 623
<211> 17
<212> DNA
<213> Homo sapiens

<400> 623
gggagcccgg ccaaggc                                                    17

<210> 624
<211> 17
<212> DNA
<213> Homo sapiens

<400> 624
ggagcccggc caaggcg                                                    17

<210> 625
<211> 17
<212> DNA
<213> Homo sapiens

<400> 625
gagcccggcc aaggcgg                                                    17

<210> 626
<211> 17
<212> DNA
<213> Homo sapiens

<400> 626
agcccggcca aggcgga                                                    17

<210> 627
<211> 17
<212> DNA
<213> Homo sapiens

<400> 627
gcccggccaa ggcggag                                                    17

<210> 628
<211> 17
<212> DNA
<213> Homo sapiens

<400> 628
cccggccaag gcggagc                                                    17

<210> 629
<211> 17
<212> DNA
<213> Homo sapiens

<400> 629
ccggccaagg cggagcg                                                    17

<210> 630
<211> 17
<212> DNA
<213> Homo sapiens

<400> 630
cggccaaggc ggagcgg                                                    17

<210> 631
<211> 17
<212> DNA
<213> Homo sapiens

<400> 631
ggccaaggcg gagcggc                                                    17

<210> 632
<211> 17
<212> DNA
<213> Homo sapiens

<400> 632
gccaaggcgg agcggcg                                                    17

<210> 633
<211> 17
<212> DNA
<213> Homo sapiens

<400> 633
ccaaggcgga gcggcgc                                                    17

<210> 634
<211> 17
<212> DNA
<213> Homo sapiens

<400> 634
caaggcggag cggcgct                                                    17

<210> 635
<211> 17

<212> DNA
<213> Homo sapiens

<400> 635
aaggcggagc ggcgctt                                                    17

<210> 636
<211> 17
<212> DNA
<213> Homo sapiens

<400> 636
aggcggagcg gcgcttc                                                    17

<210> 637
<211> 17
<212> DNA
<213> Homo sapiens

<400> 637
ggcggagcgg cgcttcg                                                    17

<210> 638
<211> 17
<212> DNA
<213> Homo sapiens

<400> 638
gcggagcggc gcttcgt                                                    17

<210> 639
<211> 17
<212> DNA
<213> Homo sapiens

<400> 639
cggagcggcg cttcgtg                                                    17

<210> 640
<211> 17
<212> DNA
<213> Homo sapiens

<400> 640
ggagcggcgc ttcgtgc                                                    17

<210> 641
<211> 17
<212> DNA
<213> Homo sapiens

<400> 641
gagcggcgct tcgtgca                                                    17

<210> 642
<211> 17
<212> DNA
<213> Homo sapiens

<400> 642
agcggcgctt cgtgcag                                                    17

<210> 643
<211> 17
<212> DNA
<213> Homo sapiens

<400> 643
gcggcgcttc gtgcagg                                                    17

<210> 644
<211> 17
<212> DNA
<213> Homo sapiens

<400> 644
cggcgcttcg tgcaggg                                                    17

<210> 645
<211> 17
<212> DNA
<213> Homo sapiens

<400> 645
ggcgcttcgt gcagggc                                                    17

<210> 646
<211> 17
<212> DNA
<213> Homo sapiens

<400> 646
gcgcttcgtg cagggcc                                                    17

<210> 647
<211> 17
<212> DNA
<213> Homo sapiens

<400> 647
cgcttcgtgc agggcca                                                    17

<210> 648
<211> 17
<212> DNA
<213> Homo sapiens

<400> 648
gcttcgtgca gggccat                                                    17

<210> 649
<211> 17
<212> DNA
<213> Homo sapiens

<400> 649
cttcgtgcag ggccatt                                                    17

<210> 650
<211> 17
<212> DNA
<213> Homo sapiens

<400> 650
ttcgtgcagg gccattt                                                    17

<210> 651
<211> 17
<212> DNA
<213> Homo sapiens

<400> 651
tcgtgcaggg ccatttc                                                    17

<210> 652
<211> 17
<212> DNA
<213> Homo sapiens

<400> 652
cgtgcagggc catttca                                                    17

<210> 653
<211> 17
<212> DNA
<213> Homo sapiens

<400> 653
gtgcagggcc atttcac                                                    17

<210> 654
<211> 17
<212> DNA
<213> Homo sapiens

<400> 654
tgcagggcca tttcacc                                                    17

<210> 655
<211> 17
<212> DNA
<213> Homo sapiens

<400> 655
gcagggccat ttcacca                                                    17

<210> 656
<211> 17
<212> DNA
<213> Homo sapiens

<400> 656
cagggccatt tcaccac                                                    17

<210> 657
<211> 17
<212> DNA
<213> Homo sapiens

<400> 657
agggccattt caccacc                                                    17

<210> 658

<211> 17
<212> DNA
<213> Homo sapiens

<400> 658
gggccatttc accacca                                                    17

<210> 659
<211> 17
<212> DNA
<213> Homo sapiens

<400> 659
ggccatttca ccaccaa                                                    17

<210> 660
<211> 17
<212> DNA
<213> Homo sapiens

<400> 660
gccatttcac caccaac                                                    17

<210> 661
<211> 17
<212> DNA
<213> Homo sapiens

<400> 661
ccatttcacc accaacg                                                    17

<210> 662
<211> 17
<212> DNA
<213> Homo sapiens

<400> 662
catttcacca ccaacga                                                    17

<210> 663
<211> 17
<212> DNA
<213> Homo sapiens

<400> 663
atttcaccac caacgac                                                    17

<210> 664
<211> 17
<212> DNA
<213> Homo sapiens

<400> 664
tttcaccacc aacgact                                                    17

<210> 665
<211> 17
<212> DNA
<213> Homo sapiens

<400> 665

ttcaccacca acgactc                                                    17

<210> 666
<211> 17
<212> DNA
<213> Homo sapiens

<400> 666
tcaccaccaa cgactcc                                                    17

<210> 667
<211> 17
<212> DNA
<213> Homo sapiens

<400> 667
caccaccaac gactcct                                                    17

<210> 668
<211> 17
<212> DNA
<213> Homo sapiens

<400> 668
accaccaacg actccta                                                    17

<210> 669
<211> 17
<212> DNA
<213> Homo sapiens

<400> 669
ccaccaacga ctcctac                                                    17

<210> 670
<211> 17
<212> DNA
<213> Homo sapiens

<400> 670
caccaacgac tcctacc                                                    17

<210> 671
<211> 17
<212> DNA
<213> Homo sapiens

<400> 671
accaacgact cctaccg                                                    17

<210> 672
<211> 17
<212> DNA
<213> Homo sapiens

<400> 672
ccaacgactc ctaccgc                                                    17

<210> 673
<211> 17
<212> DNA

<213> Homo sapiens

<400> 673
caacgactcc taccgct                                                    17

<210> 674
<211> 17
<212> DNA
<213> Homo sapiens

<400> 674
aacgactcct accgctt                                                    17

<210> 675
<211> 17
<212> DNA
<213> Homo sapiens

<400> 675
acgactccta ccgcttc                                                    17

<210> 676
<211> 17
<212> DNA
<213> Homo sapiens

<400> 676
cgactcctac cgcttct                                                    17

<210> 677
<211> 17
<212> DNA
<213> Homo sapiens

<400> 677
gactcctacc gcttctc                                                    17

<210> 678
<211> 17
<212> DNA
<213> Homo sapiens

<400> 678
actcctaccg cttctcc                                                    17

<210> 679
<211> 17
<212> DNA
<213> Homo sapiens

<400> 679
ctcctaccgc ttctccg                                                    17

<210> 680
<211> 17
<212> DNA
<213> Homo sapiens

<400> 680
tcctaccgct tctccgc                                                    17

<210> 681
<211> 17
<212> DNA
<213> Homo sapiens

<400> 681
cctaccgctt ctccgcc                                                    17

<210> 682
<211> 17
<212> DNA
<213> Homo sapiens

<400> 682
ctaccgcttc tccgcct                                                    17

<210> 683
<211> 17
<212> DNA
<213> Homo sapiens

<400> 683
taccgcttct ccgcctc                                                    17

<210> 684
<211> 17
<212> DNA
<213> Homo sapiens

<400> 684
accgcttctc cgcctcc                                                    17

<210> 685
<211> 17
<212> DNA
<213> Homo sapiens

<400> 685
ccgcttctcc gcctcca                                                    17

<210> 686
<211> 17
<212> DNA
<213> Homo sapiens

<400> 686
cgcttctccg cctccag                                                    17

<210> 687
<211> 17
<212> DNA
<213> Homo sapiens

<400> 687
gcttctccgc ctccagg                                                    17

<210> 688
<211> 17
<212> DNA
<213> Homo sapiens

<400> 688
cttctccgcc tccagga                                                    17

<210> 689
<211> 17
<212> DNA
<213> Homo sapiens

<400> 689
ttctccgcct ccaggag                                                    17

<210> 690
<211> 17
<212> DNA
<213> Homo sapiens

<400> 690
tctccgcctc caggagg                                                    17

<210> 691
<211> 17
<212> DNA
<213> Homo sapiens

<400> 691
ctccgcctcc aggagga                                                    17

<210> 692
<211> 17
<212> DNA
<213> Homo sapiens

<400> 692
tccgcctcca ggaggag                                                    17

<210> 693
<211> 17
<212> DNA
<213> Homo sapiens

<400> 693
ccgcctccag gaggagc                                                    17

<210> 694
<211> 17
<212> DNA
<213> Homo sapiens

<400> 694
cgcctccagg aggagca                                                    17

<210> 695
<211> 17
<212> DNA
<213> Homo sapiens

<400> 695
gcctccagga ggagcac                                                    17

<210> 696
<211> 17

<212> DNA
<213> Homo sapiens

<400> 696
cctccaggag gagcacc                                                    17

<210> 697
<211> 17
<212> DNA
<213> Homo sapiens

<400> 697
ctccaggagg agcaccg                                                    17

<210> 698
<211> 17
<212> DNA
<213> Homo sapiens

<400> 698
tccaggagga gcaccga                                                    17

<210> 699
<211> 17
<212> DNA
<213> Homo sapiens

<400> 699
ccaggaggag caccgaa                                                    17

<210> 700
<211> 17
<212> DNA
<213> Homo sapiens

<400> 700
caggaggagc accgaag                                                    17

<210> 701
<211> 17
<212> DNA
<213> Homo sapiens

<400> 701
aggaggagca ccgaagc                                                    17

<210> 702
<211> 17
<212> DNA
<213> Homo sapiens

<400> 702
ggaggagcac cgaagcc                                                    17

<210> 703
<211> 17
<212> DNA
<213> Homo sapiens

<400> 703
gaggagcacc gaagcca                                                    17

<210> 704
<211> 17
<212> DNA
<213> Homo sapiens

<400> 704
aggagcaccg aagccaa                                                    17

<210> 705
<211> 17
<212> DNA
<213> Homo sapiens

<400> 705
ggagcaccga agccaat                                                    17

<210> 706
<211> 17
<212> DNA
<213> Homo sapiens

<400> 706
gagcaccgaa gccaatt                                                    17

<210> 707
<211> 17
<212> DNA
<213> Homo sapiens

<400> 707
agcaccgaag ccaattt                                                    17

<210> 708
<211> 17
<212> DNA
<213> Homo sapiens

<400> 708
gcaccgaagc caatttc                                                    17

<210> 709
<211> 17
<212> DNA
<213> Homo sapiens

<400> 709
caccgaagcc aatttcg                                                    17

<210> 710
<211> 17
<212> DNA
<213> Homo sapiens

<400> 710
accgaagcca atttcgt                                                    17

<210> 711
<211> 17
<212> DNA
<213> Homo sapiens

<400> 711
ccgaagccaa tttcgtc                                                    17

<210> 712
<211> 17
<212> DNA
<213> Homo sapiens

<400> 712
cgaagccaat ttcgtct                                                    17

<210> 713
<211> 17
<212> DNA
<213> Homo sapiens

<400> 713
gaagccaatt tcgtctc                                                    17

<210> 714
<211> 17
<212> DNA
<213> Homo sapiens

<400> 714
aagccaattt cgtctcg                                                    17

<210> 715
<211> 17
<212> DNA
<213> Homo sapiens

<400> 715
agccaatttc gtctcgc                                                    17

<210> 716
<211> 17
<212> DNA
<213> Homo sapiens

<400> 716
gccaatttcg tctcgct                                                    17

<210> 717
<211> 17
<212> DNA
<213> Homo sapiens

<400> 717
ccaatttcgt ctcgctt                                                    17

<210> 718
<211> 17
<212> DNA
<213> Homo sapiens

<400> 718
caatttcgtc tcgcttc                                                    17

<210> 719

<211> 17
<212> DNA
<213> Homo sapiens

<400> 719
aatttcgtct cgcttct                                                    17

<210> 720
<211> 17
<212> DNA
<213> Homo sapiens

<400> 720
atttcgtctc gcttctg                                                    17

<210> 721
<211> 17
<212> DNA
<213> Homo sapiens

<400> 721
tttcgtctcg cttctgg                                                    17

<210> 722
<211> 17
<212> DNA
<213> Homo sapiens

<400> 722
ttcgtctcgc ttctggt                                                    17

<210> 723
<211> 17
<212> DNA
<213> Homo sapiens

<400> 723
tcgtctcgct tctggtg                                                    17

<210> 724
<211> 17
<212> DNA
<213> Homo sapiens

<400> 724
cgtctcgctt ctggtgg                                                    17

<210> 725
<211> 17
<212> DNA
<213> Homo sapiens

<400> 725
gtctcgcttc tggtggt                                                    17

<210> 726
<211> 17
<212> DNA
<213> Homo sapiens

<400> 726

tctcgcttct ggtggtc 17

<210> 727
<211> 17
<212> DNA
<213> Homo sapiens

<400> 727
ctcgcttctg gtggtct 17

<210> 728
<211> 17
<212> DNA
<213> Homo sapiens

<400> 728
tcgcttctgg tggtctc 17

<210> 729
<211> 17
<212> DNA
<213> Homo sapiens

<400> 729
cgcttctggt ggtctcc 17

<210> 730
<211> 17
<212> DNA
<213> Homo sapiens

<400> 730
gcttctggtg gtctcct 17

<210> 731
<211> 17
<212> DNA
<213> Homo sapiens

<400> 731
cttctggtgg tctccta 17

<210> 732
<211> 17
<212> DNA
<213> Homo sapiens

<400> 732
ttctggtggt ctcctac 17

<210> 733
<211> 17
<212> DNA
<213> Homo sapiens

<400> 733
tctggtggtc tcctaca 17

<210> 734
<211> 17
<212> DNA

<213> Homo sapiens

<400> 734
ctggtggtct cctacag                                                    17

<210> 735
<211> 17
<212> DNA
<213> Homo sapiens

<400> 735
tggtggtctc ctacagc                                                    17

<210> 736
<211> 17
<212> DNA
<213> Homo sapiens

<400> 736
ggtggtctcc tacagcg                                                    17

<210> 737
<211> 17
<212> DNA
<213> Homo sapiens

<400> 737
gtggtctcct acagcga                                                    17

<210> 738
<211> 17
<212> DNA
<213> Homo sapiens

<400> 738
tggtctccta cagcgac                                                    17

<210> 739
<211> 17
<212> DNA
<213> Homo sapiens

<400> 739
ggtctcctac agcgact                                                    17

<210> 740
<211> 17
<212> DNA
<213> Homo sapiens

<400> 740
gtctcctaca gcgactc                                                    17

<210> 741
<211> 17
<212> DNA
<213> Homo sapiens

<400> 741
tctcctacag cgactca                                                    17

<210> 742
<211> 17
<212> DNA
<213> Homo sapiens

<400> 742
ctcctacagc gactcac                                                    17

<210> 743
<211> 17
<212> DNA
<213> Homo sapiens

<400> 743
tcctacagcg actcact                                                    17

<210> 744
<211> 17
<212> DNA
<213> Homo sapiens

<400> 744
cctacagcga ctcactg                                                    17

<210> 745
<211> 17
<212> DNA
<213> Homo sapiens

<400> 745
ctacagcgac tcactgc                                                    17

<210> 746
<211> 17
<212> DNA
<213> Homo sapiens

<400> 746
tacagcgact cactgct                                                    17

<210> 747
<211> 17
<212> DNA
<213> Homo sapiens

<400> 747
acagcgactc actgctg                                                    17

<210> 748
<211> 17
<212> DNA
<213> Homo sapiens

<400> 748
cagcgactca ctgctgg                                                    17

<210> 749
<211> 17
<212> DNA
<213> Homo sapiens

<400> 749
agcgactcac tgctgga                                                    17

<210> 750
<211> 17
<212> DNA
<213> Homo sapiens

<400> 750
gcgactcact gctggac                                                    17

<210> 751
<211> 17
<212> DNA
<213> Homo sapiens

<400> 751
cgactcactg ctggacc                                                    17

<210> 752
<211> 17
<212> DNA
<213> Homo sapiens

<400> 752
gactcactgc tggaccc                                                    17

<210> 753
<211> 17
<212> DNA
<213> Homo sapiens

<400> 753
actcactgct ggaccca                                                    17

<210> 754
<211> 17
<212> DNA
<213> Homo sapiens

<400> 754
ctcactgctg gacccag                                                    17

<210> 755
<211> 17
<212> DNA
<213> Homo sapiens

<400> 755
tcactgctgg acccagc                                                    17

<210> 756
<211> 17
<212> DNA
<213> Homo sapiens

<400> 756
cactgctgga cccagct                                                    17

<210> 757
<211> 17

<212> DNA
<213> Homo sapiens

<400> 757
actgctggac ccagcta                                                    17

<210> 758
<211> 17
<212> DNA
<213> Homo sapiens

<400> 758
ctgctggacc cagctac                                                    17

<210> 759
<211> 17
<212> DNA
<213> Homo sapiens

<400> 759
tgctggaccc agctacc                                                    17

<210> 760
<211> 17
<212> DNA
<213> Homo sapiens

<400> 760
gctggaccca gctacct                                                    17

<210> 761
<211> 17
<212> DNA
<213> Homo sapiens

<400> 761
ctggacccag ctacctt                                                    17

<210> 762
<211> 17
<212> DNA
<213> Homo sapiens

<400> 762
tggacccagc taccttt                                                    17

<210> 763
<211> 17
<212> DNA
<213> Homo sapiens

<400> 763
ggacccagct acctttg                                                    17

<210> 764
<211> 17
<212> DNA
<213> Homo sapiens

<400> 764
gacccagcta cctttgc                                                    17

<210> 765
<211> 17
<212> DNA
<213> Homo sapiens

<400> 765
acccagctac ctttgca                                                    17

<210> 766
<211> 17
<212> DNA
<213> Homo sapiens

<400> 766
cccagctacc tttgcag                                                    17

<210> 767
<211> 17
<212> DNA
<213> Homo sapiens

<400> 767
ccagctacct ttgcaga                                                    17

<210> 768
<211> 17
<212> DNA
<213> Homo sapiens

<400> 768
cagctacctt tgcagaa                                                    17

<210> 769
<211> 17
<212> DNA
<213> Homo sapiens

<400> 769
agctaccttt gcagaag                                                    17

<210> 770
<211> 17
<212> DNA
<213> Homo sapiens

<400> 770
gctacctttg cagaagt                                                    17

<210> 771
<211> 17
<212> DNA
<213> Homo sapiens

<400> 771
ctacctttgc agaagtc                                                    17

<210> 772
<211> 17
<212> DNA
<213> Homo sapiens

<400> 772
tacctttgca gaagtca                                                    17

<210> 773
<211> 17
<212> DNA
<213> Homo sapiens

<400> 773
acctttgcag aagtcag                                                    17

<210> 774
<211> 17
<212> DNA
<213> Homo sapiens

<400> 774
cctttgcaga agtcagc                                                    17

<210> 775
<211> 17
<212> DNA
<213> Homo sapiens

<400> 775
ctttgcagaa gtcagca                                                    17

<210> 776
<211> 17
<212> DNA
<213> Homo sapiens

<400> 776
tttgcagaag tcagcaa                                                    17

<210> 777
<211> 17
<212> DNA
<213> Homo sapiens

<400> 777
ttgcagaagt cagcaaa                                                    17

<210> 778
<211> 17
<212> DNA
<213> Homo sapiens

<400> 778
tgcagaagtc agcaaac                                                    17

<210> 779
<211> 17
<212> DNA
<213> Homo sapiens

<400> 779
gcagaagtca gcaaact                                                    17

<210> 780

<211> 17
<212> DNA
<213> Homo sapiens

<400> 780
cagaagtcag caaactg                                                    17

<210> 781
<211> 17
<212> DNA
<213> Homo sapiens

<400> 781
agaagtcagc aaactgg                                                    17

<210> 782
<211> 17
<212> DNA
<213> Homo sapiens

<400> 782
gaagtcagca aactgga                                                    17

<210> 783
<211> 17
<212> DNA
<213> Homo sapiens

<400> 783
aagtcagcaa actggac                                                    17

<210> 784
<211> 17
<212> DNA
<213> Homo sapiens

<400> 784
agtcagcaaa ctggacg                                                    17

<210> 785
<211> 17
<212> DNA
<213> Homo sapiens

<400> 785
gtcagcaaac tggacgg                                                    17

<210> 786
<211> 17
<212> DNA
<213> Homo sapiens

<400> 786
tcagcaaact ggacggc                                                    17

<210> 787
<211> 17
<212> DNA
<213> Homo sapiens

<400> 787

cagcaaactg gacggcg                                                    17

<210> 788
<211> 17
<212> DNA
<213> Homo sapiens

<400> 788
agcaaactgg acggcgc                                                    17

<210> 789
<211> 17
<212> DNA
<213> Homo sapiens

<400> 789
gcaaactgga cggcgcg                                                    17

<210> 790
<211> 17
<212> DNA
<213> Homo sapiens

<400> 790
caaactggac ggcgcgg                                                    17

<210> 791
<211> 17
<212> DNA
<213> Homo sapiens

<400> 791
aaactggacg gcgcggt                                                    17

<210> 792
<211> 17
<212> DNA
<213> Homo sapiens

<400> 792
aactggacgg cgcggtg                                                    17

<210> 793
<211> 17
<212> DNA
<213> Homo sapiens

<400> 793
actggacggc gcggtgc                                                    17

<210> 794
<211> 17
<212> DNA
<213> Homo sapiens

<400> 794
ctggacggcg cggtgca                                                    17

<210> 795
<211> 17
<212> DNA

<213> Homo sapiens

<400> 795
tggacggcgc ggtgcag                                                    17

<210> 796
<211> 17
<212> DNA
<213> Homo sapiens

<400> 796
ggacggcgcg gtgcagg                                                    17

<210> 797
<211> 17
<212> DNA
<213> Homo sapiens

<400> 797
gacggcgcgg tgcagga                                                    17

<210> 798
<211> 17
<212> DNA
<213> Homo sapiens

<400> 798
acggcgcggt gcaggat                                                    17

<210> 799
<211> 17
<212> DNA
<213> Homo sapiens

<400> 799
cggcgcggtg caggatc                                                    17

<210> 800
<211> 17
<212> DNA
<213> Homo sapiens

<400> 800
ggcgcggtgc aggatct                                                    17

<210> 801
<211> 17
<212> DNA
<213> Homo sapiens

<400> 801
gcgcggtgca ggatctg                                                    17

<210> 802
<211> 17
<212> DNA
<213> Homo sapiens

<400> 802
cgcggtgcag gatctgc                                                    17

<210> 803
<211> 17
<212> DNA
<213> Homo sapiens

<400> 803
gcggtgcagg atctgcg                                                    17

<210> 804
<211> 17
<212> DNA
<213> Homo sapiens

<400> 804
cggtgcagga tctgcgc                                                    17

<210> 805
<211> 17
<212> DNA
<213> Homo sapiens

<400> 805
ggtgcaggat ctgcgcg                                                    17

<210> 806
<211> 17
<212> DNA
<213> Homo sapiens

<400> 806
gtgcaggatc tgcgcgt                                                    17

<210> 807
<211> 17
<212> DNA
<213> Homo sapiens

<400> 807
tgcaggatct gcgcgtg                                                    17

<210> 808
<211> 17
<212> DNA
<213> Homo sapiens

<400> 808
gcaggatctg cgcgtgg                                                    17

<210> 809
<211> 17
<212> DNA
<213> Homo sapiens

<400> 809
caggatctgc gcgtggc                                                    17

<210> 810
<211> 17
<212> DNA
<213> Homo sapiens

<400> 810
aggatctgcg cgtggcg                                                    17

<210> 811
<211> 17
<212> DNA
<213> Homo sapiens

<400> 811
ggatctgcgc gtggcgc                                                    17

<210> 812
<211> 17
<212> DNA
<213> Homo sapiens

<400> 812
gatctgcgcg tggcgcg                                                    17

<210> 813
<211> 17
<212> DNA
<213> Homo sapiens

<400> 813
atctgcgcgt ggcgcgg                                                    17

<210> 814
<211> 17
<212> DNA
<213> Homo sapiens

<400> 814
tctgcgcgtg gcgcggg                                                    17

<210> 815
<211> 17
<212> DNA
<213> Homo sapiens

<400> 815
ctgcgcgtgg cgcggga                                                    17

<210> 816
<211> 17
<212> DNA
<213> Homo sapiens

<400> 816
tgcgcgtggc gcgggaa                                                    17

<210> 817
<211> 17
<212> DNA
<213> Homo sapiens

<400> 817
gcgcgtggcg cgggaaa                                                    17

<210> 818
<211> 17

<212> DNA
<213> Homo sapiens

<400> 818
cgcgtggcgc gggaaaa                                                    17

<210> 819
<211> 17
<212> DNA
<213> Homo sapiens

<400> 819
gcgtggcgcg ggaaaag                                                    17

<210> 820
<211> 17
<212> DNA
<213> Homo sapiens

<400> 820
cgtggcgcgg gaaaagg                                                    17

<210> 821
<211> 17
<212> DNA
<213> Homo sapiens

<400> 821
gtggcgcggg aaaaggg                                                    17

<210> 822
<211> 17
<212> DNA
<213> Homo sapiens

<400> 822
tggcgcggga aaaggga                                                    17

<210> 823
<211> 17
<212> DNA
<213> Homo sapiens

<400> 823
ggcgcgggaa aagggaa                                                    17

<210> 824
<211> 17
<212> DNA
<213> Homo sapiens

<400> 824
gcgcgggaaa agggaag                                                    17

<210> 825
<211> 17
<212> DNA
<213> Homo sapiens

<400> 825
cgcgggaaaa gggaagc                                                    17

<210> 826
<211> 17
<212> DNA
<213> Homo sapiens

<400> 826
gcgggaaaag ggaagcc                                                    17

<210> 827
<211> 17
<212> DNA
<213> Homo sapiens

<400> 827
cgggaaaagg gaagcca                                                    17

<210> 828
<211> 17
<212> DNA
<213> Homo sapiens

<400> 828
gggaaaaggg aagccag                                                    17

<210> 829
<211> 17
<212> DNA
<213> Homo sapiens

<400> 829
ggaaaaggga agccaga                                                    17

<210> 830
<211> 17
<212> DNA
<213> Homo sapiens

<400> 830
gaaaagggaa gccagat                                                    17

<210> 831
<211> 17
<212> DNA
<213> Homo sapiens

<400> 831
aaaagggaag ccagatc                                                    17

<210> 832
<211> 17
<212> DNA
<213> Homo sapiens

<400> 832
aaagggaagc cagatcc                                                    17

<210> 833
<211> 17
<212> DNA
<213> Homo sapiens

<400> 833
aagggaagcc agatcca                                                    17

<210> 834
<211> 17
<212> DNA
<213> Homo sapiens

<400> 834
agggaagcca gatccag                                                    17

<210> 835
<211> 17
<212> DNA
<213> Homo sapiens

<400> 835
gggaagccag atccagt                                                    17

<210> 836
<211> 17
<212> DNA
<213> Homo sapiens

<400> 836
ggaagccaga tccagta                                                    17

<210> 837
<211> 17
<212> DNA
<213> Homo sapiens

<400> 837
gaagccagat ccagtac                                                    17

<210> 838
<211> 17
<212> DNA
<213> Homo sapiens

<400> 838
aagccagatc cagtacc                                                    17

<210> 839
<211> 17
<212> DNA
<213> Homo sapiens

<400> 839
agccagatcc agtacca                                                    17

<210> 840
<211> 17
<212> DNA
<213> Homo sapiens

<400> 840
gccagatcca gtaccag                                                    17

<210> 841

<211> 17
<212> DNA
<213> Homo sapiens

<400> 841
ccagatccag taccagc                                                    17

<210> 842
<211> 17
<212> DNA
<213> Homo sapiens

<400> 842
cagatccagt accagca                                                    17

<210> 843
<211> 17
<212> DNA
<213> Homo sapiens

<400> 843
agatccagta ccagcag                                                    17

<210> 844
<211> 17
<212> DNA
<213> Homo sapiens

<400> 844
gatccagtac cagcagg                                                    17

<210> 845
<211> 17
<212> DNA
<213> Homo sapiens

<400> 845
atccagtacc agcaggt                                                    17

<210> 846
<211> 17
<212> DNA
<213> Homo sapiens

<400> 846
tccagtacca gcaggtg                                                    17

<210> 847
<211> 17
<212> DNA
<213> Homo sapiens

<400> 847
ccagtaccag caggtgt                                                    17

<210> 848
<211> 17
<212> DNA
<213> Homo sapiens

<400> 848

cagtaccagc aggtgtg                                                    17

<210> 849
<211> 17
<212> DNA
<213> Homo sapiens

<400> 849
agtaccagca ggtgtgc                                                    17

<210> 850
<211> 17
<212> DNA
<213> Homo sapiens

<400> 850
gtaccagcag gtgtgcg                                                    17

<210> 851
<211> 17
<212> DNA
<213> Homo sapiens

<400> 851
taccagcagg tgtgcgc                                                    17

<210> 852
<211> 17
<212> DNA
<213> Homo sapiens

<400> 852
accagcaggt gtgcgcg                                                    17

<210> 853
<211> 17
<212> DNA
<213> Homo sapiens

<400> 853
ccagcaggtg tgcgcga                                                    17

<210> 854
<211> 17
<212> DNA
<213> Homo sapiens

<400> 854
cagcaggtgt gcgcgag                                                    17

<210> 855
<211> 17
<212> DNA
<213> Homo sapiens

<400> 855
agcaggtgtg cgcgagg                                                    17

<210> 856
<211> 17
<212> DNA

<213> Homo sapiens

<400> 856
gcaggtgtgc gcgaggt                                                    17

<210> 857
<211> 17
<212> DNA
<213> Homo sapiens

<400> 857
caggtgtgcg cgaggta                                                    17

<210> 858
<211> 17
<212> DNA
<213> Homo sapiens

<400> 858
aggtgtgcgc gaggtac                                                    17

<210> 859
<211> 17
<212> DNA
<213> Homo sapiens

<400> 859
ggtgtgcgcg aggtaca                                                    17

<210> 860
<211> 17
<212> DNA
<213> Homo sapiens

<400> 860
gtgtgcgcga ggtacag                                                    17

<210> 861
<211> 17
<212> DNA
<213> Homo sapiens

<400> 861
tgtgcgcgag gtacagg                                                    17

<210> 862
<211> 17
<212> DNA
<213> Homo sapiens

<400> 862
gtgcgcgagg tacaggg                                                    17

<210> 863
<211> 17
<212> DNA
<213> Homo sapiens

<400> 863
tgcgcgaggt acagggc                                                    17

<210> 864
<211> 17
<212> DNA
<213> Homo sapiens

<400> 864
gcgcgaggta cagggcg                                                      17

<210> 865
<211> 17
<212> DNA
<213> Homo sapiens

<400> 865
cgcgaggtac agggcgc                                                      17

<210> 866
<211> 17
<212> DNA
<213> Homo sapiens

<400> 866
gcgaggtaca gggcgct                                                      17

<210> 867
<211> 17
<212> DNA
<213> Homo sapiens

<400> 867
cgaggtacag ggcgctc                                                      17

<210> 868
<211> 17
<212> DNA
<213> Homo sapiens

<400> 868
gaggtacagg gcgctct                                                      17

<210> 869
<211> 17
<212> DNA
<213> Homo sapiens

<400> 869
aggtacaggg cgctctg                                                      17

<210> 870
<211> 17
<212> DNA
<213> Homo sapiens

<400> 870
ggtacagggc gctctgc                                                      17

<210> 871
<211> 17
<212> DNA
<213> Homo sapiens

<400> 871
gtacagggcg ctctgcg                                                    17

<210> 872
<211> 17
<212> DNA
<213> Homo sapiens

<400> 872
tacagggcgc tctgcgt                                                    17

<210> 873
<211> 17
<212> DNA
<213> Homo sapiens

<400> 873
acagggcgct ctgcgtg                                                    17

<210> 874
<211> 17
<212> DNA
<213> Homo sapiens

<400> 874
cagggcgctc tgcgtgc                                                    17

<210> 875
<211> 17
<212> DNA
<213> Homo sapiens

<400> 875
agggcgctct gcgtgcc                                                    17

<210> 876
<211> 17
<212> DNA
<213> Homo sapiens

<400> 876
gggcgctctg cgtgccc                                                    17

<210> 877
<211> 17
<212> DNA
<213> Homo sapiens

<400> 877
ggcgctctgc gtgcccc                                                    17

<210> 878
<211> 17
<212> DNA
<213> Homo sapiens

<400> 878
gcgctctgcg tgccccc                                                    17

<210> 879
<211> 17

<212> DNA
<213> Homo sapiens

<400> 879
cgctctgcgt gcccccc                                                    17

<210> 880
<211> 17
<212> DNA
<213> Homo sapiens

<400> 880
gctctgcgtg cccccca                                                    17

<210> 881
<211> 17
<212> DNA
<213> Homo sapiens

<400> 881
ctctgcgtgc ccccaa                                                     17

<210> 882
<211> 17
<212> DNA
<213> Homo sapiens

<400> 882
tctgcgtgcc ccccaac                                                    17

<210> 883
<211> 17
<212> DNA
<213> Homo sapiens

<400> 883
ctgcgtgccc cccaacc                                                    17

<210> 884
<211> 17
<212> DNA
<213> Homo sapiens

<400> 884
tgcgtgcccc ccaaccc                                                    17

<210> 885
<211> 17
<212> DNA
<213> Homo sapiens

<400> 885
gcgtgccccc caacccg                                                    17

<210> 886
<211> 17
<212> DNA
<213> Homo sapiens

<400> 886
cgtgcccccc aacccga                                                    17

<210> 887
<211> 17
<212> DNA
<213> Homo sapiens

<400> 887
gtgcccccca acccgat                                                    17

<210> 888
<211> 17
<212> DNA
<213> Homo sapiens

<400> 888
tgccccccaa cccgatc                                                    17

<210> 889
<211> 17
<212> DNA
<213> Homo sapiens

<400> 889
gccccccaac ccgatcc                                                    17

<210> 890
<211> 17
<212> DNA
<213> Homo sapiens

<400> 890
ccccccaacc cgatcct                                                    17

<210> 891
<211> 17
<212> DNA
<213> Homo sapiens

<400> 891
cccccaaccc gatcctg                                                    17

<210> 892
<211> 17
<212> DNA
<213> Homo sapiens

<400> 892
ccccaacccg atcctgt                                                    17

<210> 893
<211> 17
<212> DNA
<213> Homo sapiens

<400> 893
cccaacccga tcctgta                                                    17

<210> 894
<211> 17
<212> DNA
<213> Homo sapiens

<400> 894
ccaacccgat cctgtac                                                    17

<210> 895
<211> 17
<212> DNA
<213> Homo sapiens

<400> 895
caacccgatc ctgtacg                                                    17

<210> 896
<211> 17
<212> DNA
<213> Homo sapiens

<400> 896
aacccgatcc tgtacgc                                                    17

<210> 897
<211> 17
<212> DNA
<213> Homo sapiens

<400> 897
acccgatcct gtacgcc                                                    17

<210> 898
<211> 17
<212> DNA
<213> Homo sapiens

<400> 898
cccgatcctg tacgcct                                                    17

<210> 899
<211> 17
<212> DNA
<213> Homo sapiens

<400> 899
ccgatcctgt acgcctg                                                    17

<210> 900
<211> 17
<212> DNA
<213> Homo sapiens

<400> 900
cgatcctgta cgcctgg                                                    17

<210> 901
<211> 17
<212> DNA
<213> Homo sapiens

<400> 901
gatcctgtac gcctggc                                                    17

<210> 902

<211> 17
<212> DNA
<213> Homo sapiens

<400> 902
atcctgtacg cctggca                                                    17

<210> 903
<211> 17
<212> DNA
<213> Homo sapiens

<400> 903
tcctgtacgc ctggcag                                                    17

<210> 904
<211> 17
<212> DNA
<213> Homo sapiens

<400> 904
cctgtacgcc tggcagg                                                    17

<210> 905
<211> 17
<212> DNA
<213> Homo sapiens

<400> 905
ctgtacgcct ggcaggt                                                    17

<210> 906
<211> 17
<212> DNA
<213> Homo sapiens

<400> 906
tgtacgcctg gcaggtg                                                    17

<210> 907
<211> 17
<212> DNA
<213> Homo sapiens

<400> 907
gtacgcctgg caggtga                                                    17

<210> 908
<211> 17
<212> DNA
<213> Homo sapiens

<400> 908
tacgcctggc aggtgaa                                                    17

<210> 909
<211> 17
<212> DNA
<213> Homo sapiens

<400> 909

acgcctggca ggtgaac 17

<210> 910
<211> 17
<212> DNA
<213> Homo sapiens

<400> 910
cgcctggcag gtgaaca 17

<210> 911
<211> 17
<212> DNA
<213> Homo sapiens

<400> 911
gcctggcagg tgaacaa 17

<210> 912
<211> 17
<212> DNA
<213> Homo sapiens

<400> 912
cctggcaggt gaacaaa 17

<210> 913
<211> 17
<212> DNA
<213> Homo sapiens

<400> 913
ctggcaggtg aacaaaa 17

<210> 914
<211> 17
<212> DNA
<213> Homo sapiens

<400> 914
tggcaggtga acaaaac 17

<210> 915
<211> 17
<212> DNA
<213> Homo sapiens

<400> 915
ggcaggtgaa caaaacg 17

<210> 916
<211> 17
<212> DNA
<213> Homo sapiens

<400> 916
gcaggtgaac aaaacgc 17

<210> 917
<211> 17
<212> DNA

<213> Homo sapiens

<400> 917
caggtgaaca aaacgct                                                    17

<210> 918
<211> 17
<212> DNA
<213> Homo sapiens

<400> 918
aggtgaacaa aacgctc                                                    17

<210> 919
<211> 17
<212> DNA
<213> Homo sapiens

<400> 919
ggtgaacaaa acgctca                                                    17

<210> 920
<211> 17
<212> DNA
<213> Homo sapiens

<400> 920
gtgaacaaaa cgctcaa                                                    17

<210> 921
<211> 17
<212> DNA
<213> Homo sapiens

<400> 921
tgaacaaaac gctcaac                                                    17

<210> 922
<211> 17
<212> DNA
<213> Homo sapiens

<400> 922
gaacaaaacg ctcaacc                                                    17

<210> 923
<211> 17
<212> DNA
<213> Homo sapiens

<400> 923
aacaaaacgc tcaacct                                                    17

<210> 924
<211> 17
<212> DNA
<213> Homo sapiens

<400> 924
acaaaacgct caacctg                                                    17

<210> 925
<211> 17
<212> DNA
<213> Homo sapiens

<400> 925
caaaacgctc aacctga                                                    17

<210> 926
<211> 17
<212> DNA
<213> Homo sapiens

<400> 926
aaaacgctca acctgag                                                    17

<210> 927
<211> 17
<212> DNA
<213> Homo sapiens

<400> 927
aaacgctcaa cctgagc                                                    17

<210> 928
<211> 17
<212> DNA
<213> Homo sapiens

<400> 928
aacgctcaac ctgagca                                                    17

<210> 929
<211> 17
<212> DNA
<213> Homo sapiens

<400> 929
acgctcaacc tgagcag                                                    17

<210> 930
<211> 17
<212> DNA
<213> Homo sapiens

<400> 930
cgctcaacct gagcagc                                                    17

<210> 931
<211> 17
<212> DNA
<213> Homo sapiens

<400> 931
gctcaacctg agcagca                                                    17

<210> 932
<211> 17
<212> DNA
<213> Homo sapiens

<400> 932
ctcaacctga gcagcat                                                    17

<210> 933
<211> 17
<212> DNA
<213> Homo sapiens

<400> 933
tcaacctgag cagcatc                                                    17

<210> 934
<211> 17
<212> DNA
<213> Homo sapiens

<400> 934
caacctgagc agcatct                                                    17

<210> 935
<211> 17
<212> DNA
<213> Homo sapiens

<400> 935
aacctgagca gcatctc                                                    17

<210> 936
<211> 17
<212> DNA
<213> Homo sapiens

<400> 936
acctgagcag catctcc                                                    17

<210> 937
<211> 17
<212> DNA
<213> Homo sapiens

<400> 937
cctgagcagc atctcct                                                    17

<210> 938
<211> 17
<212> DNA
<213> Homo sapiens

<400> 938
ctgagcagca tctcctt                                                    17

<210> 939
<211> 17
<212> DNA
<213> Homo sapiens

<400> 939
tgagcagcat ctccttc                                                    17

<210> 940
<211> 17

<212> DNA
<213> Homo sapiens

<400> 940
gagcagcatc tccttcc                                                    17

<210> 941
<211> 17
<212> DNA
<213> Homo sapiens

<400> 941
agcagcatct ccttccc                                                    17

<210> 942
<211> 17
<212> DNA
<213> Homo sapiens

<400> 942
gcagcatctc cttcccc                                                    17

<210> 943
<211> 17
<212> DNA
<213> Homo sapiens

<400> 943
cagcatctcc ttccccg                                                    17

<210> 944
<211> 17
<212> DNA
<213> Homo sapiens

<400> 944
agcatctcct tccccgc                                                    17

<210> 945
<211> 17
<212> DNA
<213> Homo sapiens

<400> 945
gcatctcctt ccccgcc                                                    17

<210> 946
<211> 17
<212> DNA
<213> Homo sapiens

<400> 946
catctccttc cccgcct                                                    17

<210> 947
<211> 17
<212> DNA
<213> Homo sapiens

<400> 947
atctccttcc ccgccta                                                    17

<210> 948
<211> 17
<212> DNA
<213> Homo sapiens

<400> 948
tctccttccc cgcctac                                                      17

<210> 949
<211> 17
<212> DNA
<213> Homo sapiens

<400> 949
ctccttcccc gcctaca                                                      17

<210> 950
<211> 17
<212> DNA
<213> Homo sapiens

<400> 950
tccttccccg cctacaa                                                      17

<210> 951
<211> 17
<212> DNA
<213> Homo sapiens

<400> 951
ccttccccgc ctacaac                                                      17

<210> 952
<211> 17
<212> DNA
<213> Homo sapiens

<400> 952
cttccccgcc tacaacc                                                      17

<210> 953
<211> 17
<212> DNA
<213> Homo sapiens

<400> 953
ttccccgcct acaacca                                                      17

<210> 954
<211> 17
<212> DNA
<213> Homo sapiens

<400> 954
tccccgccta caaccac                                                      17

<210> 955
<211> 17
<212> DNA
<213> Homo sapiens

<400> 955
ccccgcctac aaccacg                                                    17

<210> 956
<211> 17
<212> DNA
<213> Homo sapiens

<400> 956
cccgcctaca accacgg                                                    17

<210> 957
<211> 17
<212> DNA
<213> Homo sapiens

<400> 957
ccgcctacaa ccacggc                                                    17

<210> 958
<211> 17
<212> DNA
<213> Homo sapiens

<400> 958
cgcctacaac cacggca                                                    17

<210> 959
<211> 17
<212> DNA
<213> Homo sapiens

<400> 959
gcctacaacc acggcag                                                    17

<210> 960
<211> 17
<212> DNA
<213> Homo sapiens

<400> 960
cctacaacca cggcagg                                                    17

<210> 961
<211> 17
<212> DNA
<213> Homo sapiens

<400> 961
ctacaaccac ggcaggc                                                    17

<210> 962
<211> 17
<212> DNA
<213> Homo sapiens

<400> 962
tacaaccacg gcaggca                                                    17

<210> 963

<211> 17
<212> DNA
<213> Homo sapiens

<400> 963
acaaccacgg caggcat                                                    17

<210> 964
<211> 17
<212> DNA
<213> Homo sapiens

<400> 964
caaccacggc aggcatc                                                    17

<210> 965
<211> 17
<212> DNA
<213> Homo sapiens

<400> 965
aaccacggca ggcatcc                                                    17

<210> 966
<211> 17
<212> DNA
<213> Homo sapiens

<400> 966
accacggcag gcatccc                                                    17

<210> 967
<211> 17
<212> DNA
<213> Homo sapiens

<400> 967
ccacggcagg catcccc                                                    17

<210> 968
<211> 17
<212> DNA
<213> Homo sapiens

<400> 968
cacggcaggc atcccct                                                    17

<210> 969
<211> 17
<212> DNA
<213> Homo sapiens

<400> 969
acggcaggca tcccctc                                                    17

<210> 970
<211> 17
<212> DNA
<213> Homo sapiens

<400> 970

cggcaggcat cccctct 17

<210> 971
<211> 17
<212> DNA
<213> Homo sapiens

<400> 971
ggcaggcatc ccctcta 17

<210> 972
<211> 17
<212> DNA
<213> Homo sapiens

<400> 972
gcaggcatcc cctctac 17

<210> 973
<211> 17
<212> DNA
<213> Homo sapiens

<400> 973
caggcatccc ctctacc 17

<210> 974
<211> 17
<212> DNA
<213> Homo sapiens

<400> 974
aggcatcccc tctacct 17

<210> 975
<211> 17
<212> DNA
<213> Homo sapiens

<400> 975
ggcatcccct ctacctg 17

<210> 976
<211> 17
<212> DNA
<213> Homo sapiens

<400> 976
gcatcccctc tacctga 17

<210> 977
<211> 17
<212> DNA
<213> Homo sapiens

<400> 977
catcccctct acctgac 17

<210> 978
<211> 17
<212> DNA

<213> Homo sapiens

<400> 978
atcccctcta cctgacc                                                    17

<210> 979
<211> 17
<212> DNA
<213> Homo sapiens

<400> 979
tcccctctac ctgaccg                                                    17

<210> 980
<211> 17
<212> DNA
<213> Homo sapiens

<400> 980
cccctctacc tgaccgg                                                    17

<210> 981
<211> 17
<212> DNA
<213> Homo sapiens

<400> 981
ccctctacct gaccggc                                                    17

<210> 982
<211> 17
<212> DNA
<213> Homo sapiens

<400> 982
cctctacctg accggct                                                    17

<210> 983
<211> 17
<212> DNA
<213> Homo sapiens

<400> 983
ctctacctga ccggctt                                                    17

<210> 984
<211> 17
<212> DNA
<213> Homo sapiens

<400> 984
tctacctgac cggcttc                                                    17

<210> 985
<211> 17
<212> DNA
<213> Homo sapiens

<400> 985
ctacctgacc ggcttct                                                    17

<210> 986
<211> 17
<212> DNA
<213> Homo sapiens

<400> 986
tacctgaccg gcttctt                                                    17

<210> 987
<211> 17
<212> DNA
<213> Homo sapiens

<400> 987
acctgaccgg cttcttc                                                    17

<210> 988
<211> 17
<212> DNA
<213> Homo sapiens

<400> 988
cctgaccggc ttcttcg                                                    17

<210> 989
<211> 17
<212> DNA
<213> Homo sapiens

<400> 989
ctgaccggct tcttcgg                                                    17

<210> 990
<211> 17
<212> DNA
<213> Homo sapiens

<400> 990
tgaccggctt cttcgga                                                    17

<210> 991
<211> 17
<212> DNA
<213> Homo sapiens

<400> 991
gaccggcttc ttcggag                                                    17

<210> 992
<211> 17
<212> DNA
<213> Homo sapiens

<400> 992
accggcttct tcggagg                                                    17

<210> 993
<211> 17
<212> DNA
<213> Homo sapiens

<400> 993
ccggcttctt cggagga                                                    17

<210> 994
<211> 17
<212> DNA
<213> Homo sapiens

<400> 994
cggcttcttc ggaggat                                                    17

<210> 995
<211> 17
<212> DNA
<213> Homo sapiens

<400> 995
ggcttcttcg gaggata                                                    17

<210> 996
<211> 17
<212> DNA
<213> Homo sapiens

<400> 996
gcttcttcgg aggatac                                                    17

<210> 997
<211> 17
<212> DNA
<213> Homo sapiens

<400> 997
cttcttcgga ggataca                                                    17

<210> 998
<211> 17
<212> DNA
<213> Homo sapiens

<400> 998
ttcttcggag gatacat                                                    17

<210> 999
<211> 17
<212> DNA
<213> Homo sapiens

<400> 999
tcttcggagg atacatc                                                    17

<210> 1000
<211> 17
<212> DNA
<213> Homo sapiens

<400> 1000
cttcggagga tacatct                                                    17

<210> 1001
<211> 17

<212> DNA
<213> Homo sapiens

<400> 1001
ttcggaggat acatctt                                                    17

<210> 1002
<211> 17
<212> DNA
<213> Homo sapiens

<400> 1002
tcggaggata catcttg                                                    17

<210> 1003
<211> 17
<212> DNA
<213> Homo sapiens

<400> 1003
cggaggatac atcttgg                                                    17

<210> 1004
<211> 17
<212> DNA
<213> Homo sapiens

<400> 1004
ggaggataca tcttggg                                                    17

<210> 1005
<211> 17
<212> DNA
<213> Homo sapiens

<400> 1005
gaggatacat cttgggg                                                    17

<210> 1006
<211> 17
<212> DNA
<213> Homo sapiens

<400> 1006
aggatacatc ttggggg                                                    17

<210> 1007
<211> 17
<212> DNA
<213> Homo sapiens

<400> 1007
ggatacatct tgggggg                                                    17

<210> 1008
<211> 17
<212> DNA
<213> Homo sapiens

<400> 1008
gatacatctt ggggggc                                                    17

<210> 1009
<211> 17
<212> DNA
<213> Homo sapiens

<400> 1009
atacatcttg gggggca                                                    17

<210> 1010
<211> 17
<212> DNA
<213> Homo sapiens

<400> 1010
tacatcttgg ggggcag                                                    17

<210> 1011
<211> 17
<212> DNA
<213> Homo sapiens

<400> 1011
acatcttggg gggcagc                                                    17

<210> 1012
<211> 17
<212> DNA
<213> Homo sapiens

<400> 1012
catcttgggg ggcagcc                                                    17

<210> 1013
<211> 17
<212> DNA
<213> Homo sapiens

<400> 1013
atcttggggg gcagcct                                                    17

<210> 1014
<211> 17
<212> DNA
<213> Homo sapiens

<400> 1014
tcttgggggg cagccta                                                    17

<210> 1015
<211> 17
<212> DNA
<213> Homo sapiens

<400> 1015
cttggggggc agcctag                                                    17

<210> 1016
<211> 17
<212> DNA
<213> Homo sapiens

<400> 1016
ttgggggca gcctagg                                                    17

<210> 1017
<211> 17
<212> DNA
<213> Homo sapiens

<400> 1017
tgggggcag cctagga                                                    17

<210> 1018
<211> 17
<212> DNA
<213> Homo sapiens

<400> 1018
gggggcagc ctaggaa                                                    17

<210> 1019
<211> 17
<212> DNA
<213> Homo sapiens

<400> 1019
ggggcagcc taggaat                                                    17

<210> 1020
<211> 17
<212> DNA
<213> Homo sapiens

<400> 1020
gggcagcct aggaatg                                                    17

<210> 1021
<211> 17
<212> DNA
<213> Homo sapiens

<400> 1021
gggcagccta ggaatgg                                                   17

<210> 1022
<211> 17
<212> DNA
<213> Homo sapiens

<400> 1022
ggcagcctag gaatggg                                                   17

<210> 1023
<211> 17
<212> DNA
<213> Homo sapiens

<400> 1023
gcagcctagg aatgggc                                                   17

<210> 1024

<211> 17
<212> DNA
<213> Homo sapiens

<400> 1024
cagcctagga atgggcc                                                    17

<210> 1025
<211> 17
<212> DNA
<213> Homo sapiens

<400> 1025
agcctaggaa tgggcca                                                    17

<210> 1026
<211> 17
<212> DNA
<213> Homo sapiens

<400> 1026
gcctaggaat gggccag                                                    17

<210> 1027
<211> 17
<212> DNA
<213> Homo sapiens

<400> 1027
cctaggaatg ggccagt                                                    17

<210> 1028
<211> 17
<212> DNA
<213> Homo sapiens

<400> 1028
ctaggaatgg gccagtt                                                    17

<210> 1029
<211> 17
<212> DNA
<213> Homo sapiens

<400> 1029
taggaatggg ccagtta                                                    17

<210> 1030
<211> 17
<212> DNA
<213> Homo sapiens

<400> 1030
aggaatgggc cagttac                                                    17

<210> 1031
<211> 17
<212> DNA
<213> Homo sapiens

<400> 1031

ggaatgggcc agttact                                                    17

<210> 1032
<211> 17
<212> DNA
<213> Homo sapiens

<400> 1032
gaatgggcca gttactc                                                    17

<210> 1033
<211> 17
<212> DNA
<213> Homo sapiens

<400> 1033
aatgggccag ttactcc                                                    17

<210> 1034
<211> 17
<212> DNA
<213> Homo sapiens

<400> 1034
atgggccagt tactcct                                                    17

<210> 1035
<211> 17
<212> DNA
<213> Homo sapiens

<400> 1035
tgggccagtt actcctg                                                    17

<210> 1036
<211> 17
<212> DNA
<213> Homo sapiens

<400> 1036
gggccagtta ctcctgc                                                    17

<210> 1037
<211> 17
<212> DNA
<213> Homo sapiens

<400> 1037
ggccagttac tcctgcg                                                    17

<210> 1038
<211> 17
<212> DNA
<213> Homo sapiens

<400> 1038
gccagttact cctgcgg                                                    17

<210> 1039
<211> 17
<212> DNA

<213> Homo sapiens

<400> 1039
ccagttactc ctgcggg                                                    17

<210> 1040
<211> 17
<212> DNA
<213> Homo sapiens

<400> 1040
cagttactcc tgcgggc                                                    17

<210> 1041
<211> 17
<212> DNA
<213> Homo sapiens

<400> 1041
agttactcct gcgggcc                                                    17

<210> 1042
<211> 17
<212> DNA
<213> Homo sapiens

<400> 1042
gttactcctg cgggcca                                                    17

<210> 1043
<211> 17
<212> DNA
<213> Homo sapiens

<400> 1043
ttactcctgc gggccaa                                                    17

<210> 1044
<211> 17
<212> DNA
<213> Homo sapiens

<400> 1044
tactcctgcg ggccaaa                                                    17

<210> 1045
<211> 17
<212> DNA
<213> Homo sapiens

<400> 1045
actcctgcgg gccaaag                                                    17

<210> 1046
<211> 17
<212> DNA
<213> Homo sapiens

<400> 1046
ctcctgcggg ccaaagc                                                    17

<210> 1047
<211> 17
<212> DNA
<213> Homo sapiens

<400> 1047
tcctgcgggc caaagcc                                                    17

<210> 1048
<211> 17
<212> DNA
<213> Homo sapiens

<400> 1048
cctgcgggcc aaagcca                                                    17

<210> 1049
<211> 17
<212> DNA
<213> Homo sapiens

<400> 1049
ctgcgggcca aagccat                                                    17

<210> 1050
<211> 17
<212> DNA
<213> Homo sapiens

<400> 1050
tgcgggccaa agccatg                                                    17

<210> 1051
<211> 17
<212> DNA
<213> Homo sapiens

<400> 1051
gcgggccaaa gccatgc                                                    17

<210> 1052
<211> 17
<212> DNA
<213> Homo sapiens

<400> 1052
cgggccaaag ccatgcg                                                    17

<210> 1053
<211> 17
<212> DNA
<213> Homo sapiens

<400> 1053
gggccaaagc catgcgg                                                    17

<210> 1054
<211> 17
<212> DNA
<213> Homo sapiens

<400> 1054
ggccaaagcc atgcggc                                                    17

<210> 1055
<211> 17
<212> DNA
<213> Homo sapiens

<400> 1055
gccaaagcca tgcggct                                                    17

<210> 1056
<211> 17
<212> DNA
<213> Homo sapiens

<400> 1056
ccaaagccat gcggctg                                                    17

<210> 1057
<211> 17
<212> DNA
<213> Homo sapiens

<400> 1057
caaagccatg cggctgc                                                    17

<210> 1058
<211> 17
<212> DNA
<213> Homo sapiens

<400> 1058
aaagccatgc ggctgct                                                    17

<210> 1059
<211> 17
<212> DNA
<213> Homo sapiens

<400> 1059
aagccatgcg gctgctg                                                    17

<210> 1060
<211> 17
<212> DNA
<213> Homo sapiens

<400> 1060
agccatgcgg ctgctgt                                                    17

<210> 1061
<211> 17
<212> DNA
<213> Homo sapiens

<400> 1061
gccatgcggc tgctgta                                                    17

<210> 1062
<211> 17

<212> DNA
<213> Homo sapiens

<400> 1062
ccatgcggct gctgtac                                                    17

<210> 1063
<211> 17
<212> DNA
<213> Homo sapiens

<400> 1063
catgcggctg ctgtact                                                    17

<210> 1064
<211> 17
<212> DNA
<213> Homo sapiens

<400> 1064
atgcggctgc tgtacta                                                    17

<210> 1065
<211> 17
<212> DNA
<213> Homo sapiens

<400> 1065
tgcggctgct gtactac                                                    17

<210> 1066
<211> 17
<212> DNA
<213> Homo sapiens

<400> 1066
gcggctgctg tactacc                                                    17

<210> 1067
<211> 17
<212> DNA
<213> Homo sapiens

<400> 1067
cggctgctgt actacct                                                    17

<210> 1068
<211> 17
<212> DNA
<213> Homo sapiens

<400> 1068
ggctgctgta ctacctg                                                    17

<210> 1069
<211> 17
<212> DNA
<213> Homo sapiens

<400> 1069
gctgctgtac tacctga                                                    17

<210> 1070
<211> 17
<212> DNA
<213> Homo sapiens

<400> 1070
ctgctgtact acctgaa                                                    17

<210> 1071
<211> 17
<212> DNA
<213> Homo sapiens

<400> 1071
tgctgtacta cctgaag                                                    17

<210> 1072
<211> 17
<212> DNA
<213> Homo sapiens

<400> 1072
gctgtactac ctgaaga                                                    17

<210> 1073
<211> 17
<212> DNA
<213> Homo sapiens

<400> 1073
ctgtactacc tgaagac                                                    17

<210> 1074
<211> 17
<212> DNA
<213> Homo sapiens

<400> 1074
tgtactacct gaagacc                                                    17

<210> 1075
<211> 17
<212> DNA
<213> Homo sapiens

<400> 1075
gtactacctg aagaccg                                                    17

<210> 1076
<211> 17
<212> DNA
<213> Homo sapiens

<400> 1076
tactacctga agaccga                                                    17

<210> 1077
<211> 17
<212> DNA
<213> Homo sapiens

<400> 1077
actacctgaa gaccgag                                                    17

<210> 1078
<211> 17
<212> DNA
<213> Homo sapiens

<400> 1078
ctacctgaag accgagg                                                    17

<210> 1079
<211> 17
<212> DNA
<213> Homo sapiens

<400> 1079
tacctgaaga ccgagga                                                    17

<210> 1080
<211> 17
<212> DNA
<213> Homo sapiens

<400> 1080
acctgaagac cgaggac                                                    17

<210> 1081
<211> 17
<212> DNA
<213> Homo sapiens

<400> 1081
cctgaagacc gaggacc                                                    17

<210> 1082
<211> 17
<212> DNA
<213> Homo sapiens

<400> 1082
ctgaagaccg aggaccc                                                    17

<210> 1083
<211> 17
<212> DNA
<213> Homo sapiens

<400> 1083
tgaagaccga ggaccct                                                    17

<210> 1084
<211> 17
<212> DNA
<213> Homo sapiens

<400> 1084
gaagaccgag gaccctg                                                    17

<210> 1085

<211> 17
<212> DNA
<213> Homo sapiens

<400> 1085
aagaccgagg accctga                                                    17

<210> 1086
<211> 17
<212> DNA
<213> Homo sapiens

<400> 1086
agaccgagga ccctgag                                                    17

<210> 1087
<211> 17
<212> DNA
<213> Homo sapiens

<400> 1087
gaccgaggac cctgagt                                                    17

<210> 1088
<211> 17
<212> DNA
<213> Homo sapiens

<400> 1088
accgaggacc ctgagta                                                    17

<210> 1089
<211> 17
<212> DNA
<213> Homo sapiens

<400> 1089
ccgaggaccc tgagtac                                                    17

<210> 1090
<211> 17
<212> DNA
<213> Homo sapiens

<400> 1090
cgaggaccct gagtacg                                                    17

<210> 1091
<211> 17
<212> DNA
<213> Homo sapiens

<400> 1091
gaggaccctg agtacga                                                    17

<210> 1092
<211> 17
<212> DNA
<213> Homo sapiens

<400> 1092

aggaccctga gtacgac                                                    17

<210> 1093
<211> 17
<212> DNA
<213> Homo sapiens

<400> 1093
ggaccctgag tacgacg                                                    17

<210> 1094
<211> 17
<212> DNA
<213> Homo sapiens

<400> 1094
gaccctgagt acgacgt                                                    17

<210> 1095
<211> 17
<212> DNA
<213> Homo sapiens

<400> 1095
accctgagta cgacgtg                                                    17

<210> 1096
<211> 17
<212> DNA
<213> Homo sapiens

<400> 1096
ccctgagtac gacgtgc                                                    17

<210> 1097
<211> 17
<212> DNA
<213> Homo sapiens

<400> 1097
cctgagtacg acgtgca                                                    17

<210> 1098
<211> 17
<212> DNA
<213> Homo sapiens

<400> 1098
ctgagtacga cgtgcag                                                    17

<210> 1099
<211> 17
<212> DNA
<213> Homo sapiens

<400> 1099
tgagtacgac gtgcaga                                                    17

<210> 1100
<211> 17
<212> DNA

<213> Homo sapiens

<400> 1100
gagtacgacg tgcagag                                                    17

<210> 1101
<211> 17
<212> DNA
<213> Homo sapiens

<400> 1101
agtacgacgt gcagagc                                                    17

<210> 1102
<211> 17
<212> DNA
<213> Homo sapiens

<400> 1102
gtacgacgtg cagagca                                                    17

<210> 1103
<211> 17
<212> DNA
<213> Homo sapiens

<400> 1103
tacgacgtgc agagcaa                                                    17

<210> 1104
<211> 17
<212> DNA
<213> Homo sapiens

<400> 1104
acgacgtgca gagcaag                                                    17

<210> 1105
<211> 17
<212> DNA
<213> Homo sapiens

<400> 1105
cgacgtgcag agcaagc                                                    17

<210> 1106
<211> 17
<212> DNA
<213> Homo sapiens

<400> 1106
gacgtgcaga gcaagca                                                    17

<210> 1107
<211> 17
<212> DNA
<213> Homo sapiens

<400> 1107
acgtgcagag caagcag                                                    17

<210> 1108
<211> 17
<212> DNA
<213> Homo sapiens

<400> 1108
cgtgcagagc aagcagt                                                      17

<210> 1109
<211> 17
<212> DNA
<213> Homo sapiens

<400> 1109
gtgcagagca agcagtg                                                      17

<210> 1110
<211> 17
<212> DNA
<213> Homo sapiens

<400> 1110
tgcagagcaa gcagtgg                                                      17

<210> 1111
<211> 17
<212> DNA
<213> Homo sapiens

<400> 1111
gcagagcaag cagtggc                                                      17

<210> 1112
<211> 17
<212> DNA
<213> Homo sapiens

<400> 1112
cagagcaagc agtggct                                                      17

<210> 1113
<211> 17
<212> DNA
<213> Homo sapiens

<400> 1113
agagcaagca gtggctc                                                      17

<210> 1114
<211> 17
<212> DNA
<213> Homo sapiens

<400> 1114
gagcaagcag tggctca                                                      17

<210> 1115
<211> 17
<212> DNA
<213> Homo sapiens

<400> 1115
agcaagcagt ggctcac                                                    17

<210> 1116
<211> 17
<212> DNA
<213> Homo sapiens

<400> 1116
gcaagcagtg gctcacc                                                    17

<210> 1117
<211> 17
<212> DNA
<213> Homo sapiens

<400> 1117
caagcagtgg ctcaccc                                                    17

<210> 1118
<211> 17
<212> DNA
<213> Homo sapiens

<400> 1118
aagcagtggc tcaccca                                                    17

<210> 1119
<211> 17
<212> DNA
<213> Homo sapiens

<400> 1119
agcagtggct cacccat                                                    17

<210> 1120
<211> 17
<212> DNA
<213> Homo sapiens

<400> 1120
gcagtggctc acccatt                                                    17

<210> 1121
<211> 17
<212> DNA
<213> Homo sapiens

<400> 1121
cagtggctca cccattt                                                    17

<210> 1122
<211> 17
<212> DNA
<213> Homo sapiens

<400> 1122
agtggctcac ccatttg                                                    17

<210> 1123
<211> 17

<212> DNA
<213> Homo sapiens

<400> 1123
gtggctcacc catttgc                                                    17

<210> 1124
<211> 17
<212> DNA
<213> Homo sapiens

<400> 1124
tggctcaccc atttgct                                                    17

<210> 1125
<211> 17
<212> DNA
<213> Homo sapiens

<400> 1125
ggctcaccca tttgctc                                                    17

<210> 1126
<211> 17
<212> DNA
<213> Homo sapiens

<400> 1126
gctcacccat ttgctcg                                                    17

<210> 1127
<211> 17
<212> DNA
<213> Homo sapiens

<400> 1127
ctcacccatt tgctcga                                                    17

<210> 1128
<211> 17
<212> DNA
<213> Homo sapiens

<400> 1128
tcacccattt gctcgat                                                    17

<210> 1129
<211> 17
<212> DNA
<213> Homo sapiens

<400> 1129
cacccatttg ctcgatc                                                    17

<210> 1130
<211> 17
<212> DNA
<213> Homo sapiens

<400> 1130
acccatttgc tcgatca                                                    17

<210> 1131
<211> 17
<212> DNA
<213> Homo sapiens

<400> 1131
cccatttgct cgatcaa                                                    17

<210> 1132
<211> 17
<212> DNA
<213> Homo sapiens

<400> 1132
ccatttgctc gatcaat                                                    17

<210> 1133
<211> 17
<212> DNA
<213> Homo sapiens

<400> 1133
catttgctcg atcaatt                                                    17

<210> 1134
<211> 17
<212> DNA
<213> Homo sapiens

<400> 1134
atttgctcga tcaattt                                                    17

<210> 1135
<211> 17
<212> DNA
<213> Homo sapiens

<400> 1135
tttgctcgat caattta                                                    17

<210> 1136
<211> 17
<212> DNA
<213> Homo sapiens

<400> 1136
ttgctcgatc aatttac                                                    17

<210> 1137
<211> 17
<212> DNA
<213> Homo sapiens

<400> 1137
tgctcgatca atttacc                                                    17

<210> 1138
<211> 17
<212> DNA
<213> Homo sapiens

<400> 1138
gctcgatcaa tttacca                                                    17

<210> 1139
<211> 17
<212> DNA
<213> Homo sapiens

<400> 1139
ctcgatcaat ttaccaa                                                    17

<210> 1140
<211> 17
<212> DNA
<213> Homo sapiens

<400> 1140
tcgatcaatt taccaac                                                    17

<210> 1141
<211> 17
<212> DNA
<213> Homo sapiens

<400> 1141
cgatcaattt accaaca                                                    17

<210> 1142
<211> 17
<212> DNA
<213> Homo sapiens

<400> 1142
gatcaattta ccaacat                                                    17

<210> 1143
<211> 17
<212> DNA
<213> Homo sapiens

<400> 1143
atcaatttac caacatt                                                    17

<210> 1144
<211> 17
<212> DNA
<213> Homo sapiens

<400> 1144
tcaatttacc aacatta                                                    17

<210> 1145
<211> 17
<212> DNA
<213> Homo sapiens

<400> 1145
caatttacca acattaa                                                    17

<210> 1146

<211> 17
<212> DNA
<213> Homo sapiens

<400> 1146
aatttaccaa cattaag                                                    17

<210> 1147
<211> 17
<212> DNA
<213> Homo sapiens

<400> 1147
atttaccaac attaaga                                                    17

<210> 1148
<211> 17
<212> DNA
<213> Homo sapiens

<400> 1148
tttaccaaca ttaagaa                                                    17

<210> 1149
<211> 17
<212> DNA
<213> Homo sapiens

<400> 1149
ttaccaacat taagaac                                                    17

<210> 1150
<211> 17
<212> DNA
<213> Homo sapiens

<400> 1150
taccaacatt aagaaca                                                    17

<210> 1151
<211> 17
<212> DNA
<213> Homo sapiens

<400> 1151
accaacatta agaacat                                                    17

<210> 1152
<211> 17
<212> DNA
<213> Homo sapiens

<400> 1152
ccaacattaa gaacatc                                                    17

<210> 1153
<211> 17
<212> DNA
<213> Homo sapiens

<400> 1153

caacattaag aacatct                                                    17

<210> 1154
<211> 17
<212> DNA
<213> Homo sapiens

<400> 1154
aacattaaga acatctt                                                    17

<210> 1155
<211> 17
<212> DNA
<213> Homo sapiens

<400> 1155
acattaagaa catcttg                                                    17

<210> 1156
<211> 17
<212> DNA
<213> Homo sapiens

<400> 1156
cattaagaac atcttgg                                                    17

<210> 1157
<211> 17
<212> DNA
<213> Homo sapiens

<400> 1157
attaagaaca tcttggc                                                    17

<210> 1158
<211> 17
<212> DNA
<213> Homo sapiens

<400> 1158
ttaagaacat cttggcc                                                    17

<210> 1159
<211> 17
<212> DNA
<213> Homo sapiens

<400> 1159
taagaacatc ttggcct                                                    17

<210> 1160
<211> 17
<212> DNA
<213> Homo sapiens

<400> 1160
aagaacatct tggcctt                                                    17

<210> 1161
<211> 17
<212> DNA

<213> Homo sapiens

<400> 1161
agaacatctt ggccttg                                                    17

<210> 1162
<211> 17
<212> DNA
<213> Homo sapiens

<400> 1162
gaacatcttg gccttga                                                    17

<210> 1163
<211> 17
<212> DNA
<213> Homo sapiens

<400> 1163
aacatcttgg ccttgaa                                                    17

<210> 1164
<211> 17
<212> DNA
<213> Homo sapiens

<400> 1164
acatcttggc cttgaaa                                                    17

<210> 1165
<211> 17
<212> DNA
<213> Homo sapiens

<400> 1165
catcttggcc ttgaaaa                                                    17

<210> 1166
<211> 17
<212> DNA
<213> Homo sapiens

<400> 1166
atcttggcct tgaaaaa                                                    17

<210> 1167
<211> 17
<212> DNA
<213> Homo sapiens

<400> 1167
tcttggcctt gaaaaaa                                                    17

<210> 1168
<211> 17
<212> DNA
<213> Homo sapiens

<400> 1168
cttggccttg aaaaaaa                                                    17

<210> 1169
<211> 17
<212> DNA
<213> Homo sapiens

<400> 1169
ttggccttga aaaaaat                                                    17

<210> 1170
<211> 17
<212> DNA
<213> Homo sapiens

<400> 1170
tggccttgaa aaaaatt                                                    17

<210> 1171
<211> 17
<212> DNA
<213> Homo sapiens

<400> 1171
ggccttgaaa aaaattg                                                    17

<210> 1172
<211> 17
<212> DNA
<213> Homo sapiens

<400> 1172
gccttgaaaa aaattga                                                    17

<210> 1173
<211> 17
<212> DNA
<213> Homo sapiens

<400> 1173
ccttgaaaaa aattgag                                                    17

<210> 1174
<211> 17
<212> DNA
<213> Homo sapiens

<400> 1174
cttgaaaaaa attgagg                                                    17

<210> 1175
<211> 17
<212> DNA
<213> Homo sapiens

<400> 1175
ttgaaaaaaa ttgaggt                                                    17

<210> 1176
<211> 17
<212> DNA
<213> Homo sapiens

<400> 1176
tgaaaaaaat tgaggta                                                    17

<210> 1177
<211> 17
<212> DNA
<213> Homo sapiens

<400> 1177
gaaaaaaatt gaggtag                                                    17

<210> 1178
<211> 17
<212> DNA
<213> Homo sapiens

<400> 1178
aaaaaaattg aggtagt                                                    17

<210> 1179
<211> 17
<212> DNA
<213> Homo sapiens

<400> 1179
aaaaaattga ggtagtc                                                    17

<210> 1180
<211> 17
<212> DNA
<213> Homo sapiens

<400> 1180
aaaaattgag gtagtcc                                                    17

<210> 1181
<211> 17
<212> DNA
<213> Homo sapiens

<400> 1181
aaaattgagg tagtcca                                                    17

<210> 1182
<211> 17
<212> DNA
<213> Homo sapiens

<400> 1182
aaattgaggt agtccac                                                    17

<210> 1183
<211> 17
<212> DNA
<213> Homo sapiens

<400> 1183
aattgaggta gtccact                                                    17

<210> 1184
<211> 17

<212> DNA
<213> Homo sapiens

<400> 1184
attgaggtag tccactt                                                    17

<210> 1185
<211> 17
<212> DNA
<213> Homo sapiens

<400> 1185
ttgaggtagt ccacttt                                                    17

<210> 1186
<211> 17
<212> DNA
<213> Homo sapiens

<400> 1186
tgaggtagtc cacttta                                                    17

<210> 1187
<211> 17
<212> DNA
<213> Homo sapiens

<400> 1187
gaggtagtcc actttac                                                    17

<210> 1188
<211> 17
<212> DNA
<213> Homo sapiens

<400> 1188
aggtagtcca ctttaca                                                    17

<210> 1189
<211> 17
<212> DNA
<213> Homo sapiens

<400> 1189
ggtagtccac tttacat                                                    17

<210> 1190
<211> 17
<212> DNA
<213> Homo sapiens

<400> 1190
gtagtccact ttacatc                                                    17

<210> 1191
<211> 17
<212> DNA
<213> Homo sapiens

<400> 1191
tagtccactt tacatcg                                                    17

<210> 1192
<211> 17
<212> DNA
<213> Homo sapiens

<400> 1192
agtccacttt acatcgc                                                    17

<210> 1193
<211> 17
<212> DNA
<213> Homo sapiens

<400> 1193
gtccacttta catcgct                                                    17

<210> 1194
<211> 17
<212> DNA
<213> Homo sapiens

<400> 1194
tccactttac atcgctt                                                    17

<210> 1195
<211> 17
<212> DNA
<213> Homo sapiens

<400> 1195
ccactttaca tcgcttt                                                    17

<210> 1196
<211> 17
<212> DNA
<213> Homo sapiens

<400> 1196
cactttacat cgctttc                                                    17

<210> 1197
<211> 17
<212> DNA
<213> Homo sapiens

<400> 1197
actttacatc gctttcc                                                    17

<210> 1198
<211> 17
<212> DNA
<213> Homo sapiens

<400> 1198
ctttacatcg ctttcca                                                    17

<210> 1199
<211> 17
<212> DNA
<213> Homo sapiens

<400> 1199
tttacatcgc tttccag                                                    17

<210> 1200
<211> 17
<212> DNA
<213> Homo sapiens

<400> 1200
ttacatcgct ttccaga                                                    17

<210> 1201
<211> 17
<212> DNA
<213> Homo sapiens

<400> 1201
tacatcgctt tccagac                                                    17

<210> 1202
<211> 17
<212> DNA
<213> Homo sapiens

<400> 1202
acatcgcttt ccagaca                                                    17

<210> 1203
<211> 17
<212> DNA
<213> Homo sapiens

<400> 1203
catcgctttc cagacaa                                                    17

<210> 1204
<211> 17
<212> DNA
<213> Homo sapiens

<400> 1204
atcgctttcc agacaac                                                    17

<210> 1205
<211> 17
<212> DNA
<213> Homo sapiens

<400> 1205
tcgctttcca gacaact                                                    17

<210> 1206
<211> 17
<212> DNA
<213> Homo sapiens

<400> 1206
cgctttccag acaactg                                                    17

<210> 1207

<211> 17
<212> DNA
<213> Homo sapiens

<400> 1207
gctttccaga caactgg                                                    17

<210> 1208
<211> 17
<212> DNA
<213> Homo sapiens

<400> 1208
ctttccagac aactgga                                                    17

<210> 1209
<211> 17
<212> DNA
<213> Homo sapiens

<400> 1209
tttccagaca actggaa                                                    17

<210> 1210
<211> 17
<212> DNA
<213> Homo sapiens

<400> 1210
ttccagacaa ctggaat                                                    17

<210> 1211
<211> 17
<212> DNA
<213> Homo sapiens

<400> 1211
tccagacaac tggaatt                                                    17

<210> 1212
<211> 17
<212> DNA
<213> Homo sapiens

<400> 1212
ccagacaact ggaattt                                                    17

<210> 1213
<211> 17
<212> DNA
<213> Homo sapiens

<400> 1213
cagacaactg gaatttg                                                    17

<210> 1214
<211> 17
<212> DNA
<213> Homo sapiens

<400> 1214

agacaactgg aatttga                                                    17

<210> 1215
<211> 17
<212> DNA
<213> Homo sapiens

<400> 1215
gacaactgga atttgag                                                    17

<210> 1216
<211> 17
<212> DNA
<213> Homo sapiens

<400> 1216
acaactggaa tttgagg                                                    17

<210> 1217
<211> 17
<212> DNA
<213> Homo sapiens

<400> 1217
caactggaat ttgaggc                                                    17

<210> 1218
<211> 17
<212> DNA
<213> Homo sapiens

<400> 1218
aactggaatt tgaggca                                                    17

<210> 1219
<211> 17
<212> DNA
<213> Homo sapiens

<400> 1219
actggaattt gaggcaa                                                    17

<210> 1220
<211> 17
<212> DNA
<213> Homo sapiens

<400> 1220
ctggaatttg aggcaac                                                    17

<210> 1221
<211> 17
<212> DNA
<213> Homo sapiens

<400> 1221
tggaatttga ggcaact                                                    17

<210> 1222
<211> 17
<212> DNA

<213> Homo sapiens

<400> 1222
ggaatttgag gcaactt                                                    17

<210> 1223
<211> 17
<212> DNA
<213> Homo sapiens

<400> 1223
gaatttgagg caacttc                                                    17

<210> 1224
<211> 17
<212> DNA
<213> Homo sapiens

<400> 1224
aatttgaggc aacttct                                                    17

<210> 1225
<211> 17
<212> DNA
<213> Homo sapiens

<400> 1225
atttgaggca acttctg                                                    17

<210> 1226
<211> 17
<212> DNA
<213> Homo sapiens

<400> 1226
tttgaggcaa cttctgt                                                    17

<210> 1227
<211> 17
<212> DNA
<213> Homo sapiens

<400> 1227
ttgaggcaac ttctgtg                                                    17

<210> 1228
<211> 17
<212> DNA
<213> Homo sapiens

<400> 1228
tgaggcaact tctgtga                                                    17

<210> 1229
<211> 17
<212> DNA
<213> Homo sapiens

<400> 1229
gaggcaactt ctgtgac                                                    17

<210> 1230
<211> 17
<212> DNA
<213> Homo sapiens

<400> 1230
aggcaacttc tgtgact                                                    17

<210> 1231
<211> 17
<212> DNA
<213> Homo sapiens

<400> 1231
ggcaacttct gtgactg                                                    17

<210> 1232
<211> 17
<212> DNA
<213> Homo sapiens

<400> 1232
gcaacttctg tgactgt                                                    17

<210> 1233
<211> 17
<212> DNA
<213> Homo sapiens

<400> 1233
caacttctgt gactgtg                                                    17

<210> 1234
<211> 17
<212> DNA
<213> Homo sapiens

<400> 1234
aacttctgtg actgtga                                                    17

<210> 1235
<211> 17
<212> DNA
<213> Homo sapiens

<400> 1235
acttctgtga ctgtgat                                                    17

<210> 1236
<211> 17
<212> DNA
<213> Homo sapiens

<400> 1236
cttctgtgac tgtgatc                                                    17

<210> 1237
<211> 17
<212> DNA
<213> Homo sapiens

<400> 1237
ttctgtgact gtgatcc                                                    17

<210> 1238
<211> 17
<212> DNA
<213> Homo sapiens

<400> 1238
tctgtgactg tgatccc                                                    17

<210> 1239
<211> 17
<212> DNA
<213> Homo sapiens

<400> 1239
ctgtgactgt gatccct                                                    17

<210> 1240
<211> 17
<212> DNA
<213> Homo sapiens

<400> 1240
tgtgactgtg atccctg                                                    17

<210> 1241
<211> 17
<212> DNA
<213> Homo sapiens

<400> 1241
gtgactgtga tccctgt                                                    17

<210> 1242
<211> 17
<212> DNA
<213> Homo sapiens

<400> 1242
tgactgtgat ccctgtg                                                    17

<210> 1243
<211> 17
<212> DNA
<213> Homo sapiens

<400> 1243
gactgtgatc cctgtgt                                                    17

<210> 1244
<211> 17
<212> DNA
<213> Homo sapiens

<400> 1244
actgtgatcc ctgtgtt                                                    17

<210> 1245
<211> 17

<212> DNA
<213> Homo sapiens

<400> 1245
ctgtgatccc tgtgttt                                                    17

<210> 1246
<211> 17
<212> DNA
<213> Homo sapiens

<400> 1246
tgtgatccct gtgtttc                                                    17

<210> 1247
<211> 17
<212> DNA
<213> Homo sapiens

<400> 1247
gtgatccctg tgtttca                                                    17

<210> 1248
<211> 17
<212> DNA
<213> Homo sapiens

<400> 1248
tgatccctgt gtttcac                                                    17

<210> 1249
<211> 17
<212> DNA
<213> Homo sapiens

<400> 1249
gatccctgtg tttcacc                                                    17

<210> 1250
<211> 17
<212> DNA
<213> Homo sapiens

<400> 1250
atccctgtgt ttcacct                                                    17

<210> 1251
<211> 17
<212> DNA
<213> Homo sapiens

<400> 1251
tccctgtgtt tcacctg                                                    17

<210> 1252
<211> 17
<212> DNA
<213> Homo sapiens

<400> 1252
ccctgtgttt cacctgg                                                    17

<210> 1253
<211> 17
<212> DNA
<213> Homo sapiens

<400> 1253
cctgtgtttc acctggc                                                    17

<210> 1254
<211> 17
<212> DNA
<213> Homo sapiens

<400> 1254
ctgtgtttca cctggca                                                    17

<210> 1255
<211> 17
<212> DNA
<213> Homo sapiens

<400> 1255
tgtgtttcac ctggcat                                                    17

<210> 1256
<211> 17
<212> DNA
<213> Homo sapiens

<400> 1256
gtgtttcacc tggcata                                                    17

<210> 1257
<211> 17
<212> DNA
<213> Homo sapiens

<400> 1257
tgtttcacct ggcatac                                                    17

<210> 1258
<211> 17
<212> DNA
<213> Homo sapiens

<400> 1258
gtttcacctg gcataca                                                    17

<210> 1259
<211> 17
<212> DNA
<213> Homo sapiens

<400> 1259
tttcacctgg catacat                                                    17

<210> 1260
<211> 17
<212> DNA
<213> Homo sapiens

<400> 1260
ttcacctggc atacatt                                                    17

<210> 1261
<211> 17
<212> DNA
<213> Homo sapiens

<400> 1261
tcacctggca tacattc                                                    17

<210> 1262
<211> 17
<212> DNA
<213> Homo sapiens

<400> 1262
cacctggcat acattct                                                    17

<210> 1263
<211> 17
<212> DNA
<213> Homo sapiens

<400> 1263
acctggcata cattctc                                                    17

<210> 1264
<211> 17
<212> DNA
<213> Homo sapiens

<400> 1264
cctggcatac attctca                                                    17

<210> 1265
<211> 17
<212> DNA
<213> Homo sapiens

<400> 1265
ctggcataca ttctcat                                                    17

<210> 1266
<211> 17
<212> DNA
<213> Homo sapiens

<400> 1266
tggcatacat tctcatc                                                    17

<210> 1267
<211> 17
<212> DNA
<213> Homo sapiens

<400> 1267
ggcatacatt ctcatca                                                    17

<210> 1268

<211> 17
<212> DNA
<213> Homo sapiens

<400> 1268
gcatacattc tcatcat                                                    17

<210> 1269
<211> 17
<212> DNA
<213> Homo sapiens

<400> 1269
catacattct catcatt                                                    17

<210> 1270
<211> 17
<212> DNA
<213> Homo sapiens

<400> 1270
atacattctc atcattc                                                    17

<210> 1271
<211> 17
<212> DNA
<213> Homo sapiens

<400> 1271
tacattctca tcattct                                                    17

<210> 1272
<211> 17
<212> DNA
<213> Homo sapiens

<400> 1272
acattctcat cattctg                                                    17

<210> 1273
<211> 17
<212> DNA
<213> Homo sapiens

<400> 1273
cattctcatc attctgt                                                    17

<210> 1274
<211> 17
<212> DNA
<213> Homo sapiens

<400> 1274
attctcatca ttctgtt                                                    17

<210> 1275
<211> 17
<212> DNA
<213> Homo sapiens

<400> 1275

ttctcatcat tctgttt                                                    17

<210> 1276
<211> 17
<212> DNA
<213> Homo sapiens

<400> 1276
tctcatcatt ctgtttg                                                    17

<210> 1277
<211> 17
<212> DNA
<213> Homo sapiens

<400> 1277
ctcatcattc tgtttgc                                                    17

<210> 1278
<211> 17
<212> DNA
<213> Homo sapiens

<400> 1278
tcatcattct gtttgca                                                    17

<210> 1279
<211> 17
<212> DNA
<213> Homo sapiens

<400> 1279
catcattctg tttgcag                                                    17

<210> 1280
<211> 17
<212> DNA
<213> Homo sapiens

<400> 1280
atcattctgt ttgcagt                                                    17

<210> 1281
<211> 17
<212> DNA
<213> Homo sapiens

<400> 1281
tcattctgtt tgcagtc                                                    17

<210> 1282
<211> 17
<212> DNA
<213> Homo sapiens

<400> 1282
cattctgttt gcagtca                                                    17

<210> 1283
<211> 17
<212> DNA

<213> Homo sapiens

<400> 1283
attctgtttg cagtcac                                                    17

<210> 1284
<211> 17
<212> DNA
<213> Homo sapiens

<400> 1284
ttctgtttgc agtcaca                                                    17

<210> 1285
<211> 17
<212> DNA
<213> Homo sapiens

<400> 1285
tctgtttgca gtcacat                                                    17

<210> 1286
<211> 17
<212> DNA
<213> Homo sapiens

<400> 1286
ctgtttgcag tcacatc                                                    17

<210> 1287
<211> 17
<212> DNA
<213> Homo sapiens

<400> 1287
tgtttgcagt cacatca                                                    17

<210> 1288
<211> 17
<212> DNA
<213> Homo sapiens

<400> 1288
gtttgcagtc acatcat                                                    17

<210> 1289
<211> 17
<212> DNA
<213> Homo sapiens

<400> 1289
tttgcagtca catcatg                                                    17

<210> 1290
<211> 17
<212> DNA
<213> Homo sapiens

<400> 1290
ttgcagtcac atcatgc                                                    17

<210> 1291
<211> 17
<212> DNA
<213> Homo sapiens

<400> 1291
tgcagtcaca tcatgct                                                    17

<210> 1292
<211> 17
<212> DNA
<213> Homo sapiens

<400> 1292
gcagtcacat catgctt                                                    17

<210> 1293
<211> 17
<212> DNA
<213> Homo sapiens

<400> 1293
cagtcacatc atgcttt                                                    17

<210> 1294
<211> 17
<212> DNA
<213> Homo sapiens

<400> 1294
agtcacatca tgcttta                                                    17

<210> 1295
<211> 17
<212> DNA
<213> Homo sapiens

<400> 1295
gtcacatcat gctttag                                                    17

<210> 1296
<211> 17
<212> DNA
<213> Homo sapiens

<400> 1296
tcacatcatg ctttagg                                                    17

<210> 1297
<211> 17
<212> DNA
<213> Homo sapiens

<400> 1297
cacatcatgc tttaggt                                                    17

<210> 1298
<211> 17
<212> DNA
<213> Homo sapiens

<400> 1298
acatcatgct ttaggtt                                                    17

<210> 1299
<211> 17
<212> DNA
<213> Homo sapiens

<400> 1299
catcatgctt taggttt                                                    17

<210> 1300
<211> 17
<212> DNA
<213> Homo sapiens

<400> 1300
atcatgcttt aggtttg                                                    17

<210> 1301
<211> 17
<212> DNA
<213> Homo sapiens

<400> 1301
tcatgcttta ggtttga                                                    17

<210> 1302
<211> 17
<212> DNA
<213> Homo sapiens

<400> 1302
catgctttag gtttgac                                                    17

<210> 1303
<211> 17
<212> DNA
<213> Homo sapiens

<400> 1303
atgctttagg tttgact                                                    17

<210> 1304
<211> 17
<212> DNA
<213> Homo sapiens

<400> 1304
tgctttaggt ttgactg                                                    17

<210> 1305
<211> 17
<212> DNA
<213> Homo sapiens

<400> 1305
gctttaggtt tgactgc                                                    17

<210> 1306
<211> 17

<212> DNA
<213> Homo sapiens

<400> 1306
ctttaggttt gactgca                                                    17

<210> 1307
<211> 17
<212> DNA
<213> Homo sapiens

<400> 1307
tttaggtttg actgcat                                                    17

<210> 1308
<211> 17
<212> DNA
<213> Homo sapiens

<400> 1308
ttaggtttga ctgcata                                                    17

<210> 1309
<211> 17
<212> DNA
<213> Homo sapiens

<400> 1309
taggtttgac tgcatac                                                    17

<210> 1310
<211> 17
<212> DNA
<213> Homo sapiens

<400> 1310
aggtttgact gcatacg                                                    17

<210> 1311
<211> 17
<212> DNA
<213> Homo sapiens

<400> 1311
ggtttgactg catacga                                                    17

<210> 1312
<211> 17
<212> DNA
<213> Homo sapiens

<400> 1312
gtttgactgc atacgaa                                                    17

<210> 1313
<211> 17
<212> DNA
<213> Homo sapiens

<400> 1313
tttgactgca tacgaaa                                                    17

<210> 1314
<211> 17
<212> DNA
<213> Homo sapiens

<400> 1314
ttgactgcat acgaaac                                                    17

<210> 1315
<211> 17
<212> DNA
<213> Homo sapiens

<400> 1315
tgactgcata cgaaaca                                                    17

<210> 1316
<211> 17
<212> DNA
<213> Homo sapiens

<400> 1316
gactgcatac gaaacaa                                                    17

<210> 1317
<211> 17
<212> DNA
<213> Homo sapiens

<400> 1317
actgcatacg aaacaaa                                                    17

<210> 1318
<211> 17
<212> DNA
<213> Homo sapiens

<400> 1318
ctgcatacga aacaaaa                                                    17

<210> 1319
<211> 17
<212> DNA
<213> Homo sapiens

<400> 1319
tgcatacgaa acaaaat                                                    17

<210> 1320
<211> 17
<212> DNA
<213> Homo sapiens

<400> 1320
gcatacgaaa caaaatg                                                    17

<210> 1321
<211> 17
<212> DNA
<213> Homo sapiens

<400> 1321
catacgaaac aaaatgt                                                    17

<210> 1322
<211> 17
<212> DNA
<213> Homo sapiens

<400> 1322
atacgaaaca aaatgtg                                                    17

<210> 1323
<211> 17
<212> DNA
<213> Homo sapiens

<400> 1323
tacgaaacaa aatgtgt                                                    17

<210> 1324
<211> 17
<212> DNA
<213> Homo sapiens

<400> 1324
acgaaacaaa atgtgtg                                                    17

<210> 1325
<211> 17
<212> DNA
<213> Homo sapiens

<400> 1325
cgaaacaaaa tgtgtgt                                                    17

<210> 1326
<211> 17
<212> DNA
<213> Homo sapiens

<400> 1326
gaaacaaaat gtgtgtt                                                    17

<210> 1327
<211> 17
<212> DNA
<213> Homo sapiens

<400> 1327
aaacaaaatg tgtgttg                                                    17

<210> 1328
<211> 17
<212> DNA
<213> Homo sapiens

<400> 1328
aacaaaatgt gtgttgc                                                    17

<210> 1329

<211> 17
<212> DNA
<213> Homo sapiens

<400> 1329
acaaaatgtg tgttgcg                                                    17

<210> 1330
<211> 17
<212> DNA
<213> Homo sapiens

<400> 1330
caaaatgtgt gttgcgg                                                    17

<210> 1331
<211> 17
<212> DNA
<213> Homo sapiens

<400> 1331
aaaatgtgtg ttgcggc                                                    17

<210> 1332
<211> 17
<212> DNA
<213> Homo sapiens

<400> 1332
aaatgtgtgt tgcggcc                                                    17

<210> 1333
<211> 17
<212> DNA
<213> Homo sapiens

<400> 1333
aatgtgtgtt gcggcct                                                    17

<210> 1334
<211> 17
<212> DNA
<213> Homo sapiens

<400> 1334
atgtgtgttg cggcctt                                                    17

<210> 1335
<211> 17
<212> DNA
<213> Homo sapiens

<400> 1335
tgtgtgttgc ggccttt                                                    17

<210> 1336
<211> 17
<212> DNA
<213> Homo sapiens

<400> 1336

gtgtgttgcg gcctttg                                                    17

<210> 1337
<211> 17
<212> DNA
<213> Homo sapiens

<400> 1337
tgtgttgcgg cctttgg                                                    17

<210> 1338
<211> 17
<212> DNA
<213> Homo sapiens

<400> 1338
gtgttgcggc ctttgga                                                    17

<210> 1339
<211> 17
<212> DNA
<213> Homo sapiens

<400> 1339
tgttgcggcc tttggag                                                    17

<210> 1340
<211> 17
<212> DNA
<213> Homo sapiens

<400> 1340
gttgcggcct ttggagt                                                    17

<210> 1341
<211> 17
<212> DNA
<213> Homo sapiens

<400> 1341
ttgcggcctt tggagtg                                                    17

<210> 1342
<211> 17
<212> DNA
<213> Homo sapiens

<400> 1342
tgcggccttt ggagtga                                                    17

<210> 1343
<211> 17
<212> DNA
<213> Homo sapiens

<400> 1343
gcggcctttg gagtgat                                                    17

<210> 1344
<211> 17
<212> DNA

<213> Homo sapiens

<400> 1344
cggcctttgg agtgatt                                                    17

<210> 1345
<211> 17
<212> DNA
<213> Homo sapiens

<400> 1345
ggcctttgga gtgattt                                                    17

<210> 1346
<211> 17
<212> DNA
<213> Homo sapiens

<400> 1346
gcctttggag tgatttc                                                    17

<210> 1347
<211> 17
<212> DNA
<213> Homo sapiens

<400> 1347
cctttggagt gatttct                                                    17

<210> 1348
<211> 17
<212> DNA
<213> Homo sapiens

<400> 1348
ctttggagtg atttctg                                                    17

<210> 1349
<211> 17
<212> DNA
<213> Homo sapiens

<400> 1349
tttggagtga tttctgc                                                    17

<210> 1350
<211> 17
<212> DNA
<213> Homo sapiens

<400> 1350
ttggagtgat ttctgct                                                    17

<210> 1351
<211> 17
<212> DNA
<213> Homo sapiens

<400> 1351
tggagtgatt tctgctt                                                    17

<210> 1352
<211> 17
<212> DNA
<213> Homo sapiens

<400> 1352
ggagtgattt ctgcttt                                                    17

<210> 1353
<211> 17
<212> DNA
<213> Homo sapiens

<400> 1353
gagtgatttc tgctttc                                                    17

<210> 1354
<211> 17
<212> DNA
<213> Homo sapiens

<400> 1354
agtgatttct gctttct                                                    17

<210> 1355
<211> 17
<212> DNA
<213> Homo sapiens

<400> 1355
gtgatttctg ctttctt                                                    17

<210> 1356
<211> 17
<212> DNA
<213> Homo sapiens

<400> 1356
tgatttctgc tttcttg                                                    17

<210> 1357
<211> 17
<212> DNA
<213> Homo sapiens

<400> 1357
gatttctgct ttcttgg                                                    17

<210> 1358
<211> 17
<212> DNA
<213> Homo sapiens

<400> 1358
atttctgctt tcttggc                                                    17

<210> 1359
<211> 17
<212> DNA
<213> Homo sapiens

<400> 1359
tttctgcttt cttggca                                                    17

<210> 1360
<211> 17
<212> DNA
<213> Homo sapiens

<400> 1360
ttctgctttc ttggcag                                                    17

<210> 1361
<211> 17
<212> DNA
<213> Homo sapiens

<400> 1361
tctgctttct tggcagt                                                    17

<210> 1362
<211> 17
<212> DNA
<213> Homo sapiens

<400> 1362
ctgctttctt ggcagtg                                                    17

<210> 1363
<211> 17
<212> DNA
<213> Homo sapiens

<400> 1363
tgctttcttg gcagtgg                                                    17

<210> 1364
<211> 17
<212> DNA
<213> Homo sapiens

<400> 1364
gctttcttgg cagtggt                                                    17

<210> 1365
<211> 17
<212> DNA
<213> Homo sapiens

<400> 1365
ctttcttggc agtggtg                                                    17

<210> 1366
<211> 17
<212> DNA
<213> Homo sapiens

<400> 1366
tttcttggca gtggtga                                                    17

<210> 1367
<211> 17

<212> DNA
<213> Homo sapiens

<400> 1367
ttcttggcag tggtgag                                                    17

<210> 1368
<211> 17
<212> DNA
<213> Homo sapiens

<400> 1368
tcttggcagt ggtgagc                                                    17

<210> 1369
<211> 17
<212> DNA
<213> Homo sapiens

<400> 1369
cttggcagtg gtgagcg                                                    17

<210> 1370
<211> 17
<212> DNA
<213> Homo sapiens

<400> 1370
ttggcagtgg tgagcgg                                                    17

<210> 1371
<211> 17
<212> DNA
<213> Homo sapiens

<400> 1371
tggcagtggt gagcggc                                                    17

<210> 1372
<211> 17
<212> DNA
<213> Homo sapiens

<400> 1372
ggcagtggtg agcggct                                                    17

<210> 1373
<211> 17
<212> DNA
<213> Homo sapiens

<400> 1373
gcagtggtga gcggctt                                                    17

<210> 1374
<211> 17
<212> DNA
<213> Homo sapiens

<400> 1374
cagtggtgag cggcttt                                                    17

<210> 1375
<211> 17
<212> DNA
<213> Homo sapiens

<400> 1375
agtggtgagc ggctttg                                                    17

<210> 1376
<211> 17
<212> DNA
<213> Homo sapiens

<400> 1376
gtggtgagcg gctttgg                                                    17

<210> 1377
<211> 17
<212> DNA
<213> Homo sapiens

<400> 1377
tggtgagcgg ctttggc                                                    17

<210> 1378
<211> 17
<212> DNA
<213> Homo sapiens

<400> 1378
ggtgagcggc tttggcc                                                    17

<210> 1379
<211> 17
<212> DNA
<213> Homo sapiens

<400> 1379
gtgagcggct ttggcct                                                    17

<210> 1380
<211> 17
<212> DNA
<213> Homo sapiens

<400> 1380
tgagcggctt tggcctg                                                    17

<210> 1381
<211> 17
<212> DNA
<213> Homo sapiens

<400> 1381
gagcggcttt ggcctgc                                                    17

<210> 1382
<211> 17
<212> DNA
<213> Homo sapiens

<400> 1382
agcggctttg gcctgct                                                    17

<210> 1383
<211> 17
<212> DNA
<213> Homo sapiens

<400> 1383
gcggctttgg cctgctg                                                    17

<210> 1384
<211> 17
<212> DNA
<213> Homo sapiens

<400> 1384
cggctttggc ctgctgt                                                    17

<210> 1385
<211> 17
<212> DNA
<213> Homo sapiens

<400> 1385
ggctttggcc tgctgtt                                                    17

<210> 1386
<211> 17
<212> DNA
<213> Homo sapiens

<400> 1386
gctttggcct gctgttg                                                    17

<210> 1387
<211> 17
<212> DNA
<213> Homo sapiens

<400> 1387
ctttggcctg ctgttgc                                                    17

<210> 1388
<211> 17
<212> DNA
<213> Homo sapiens

<400> 1388
tttggcctgc tgttgca                                                    17

<210> 1389
<211> 17
<212> DNA
<213> Homo sapiens

<400> 1389
ttggcctgct gttgcac                                                    17

<210> 1390

<211> 17
<212> DNA
<213> Homo sapiens

<400> 1390
tggcctgctg ttgcaca                                                    17

<210> 1391
<211> 17
<212> DNA
<213> Homo sapiens

<400> 1391
ggcctgctgt tgcacat                                                    17

<210> 1392
<211> 17
<212> DNA
<213> Homo sapiens

<400> 1392
gcctgctgtt gcacatt                                                    17

<210> 1393
<211> 17
<212> DNA
<213> Homo sapiens

<400> 1393
cctgctgttg cacattg                                                    17

<210> 1394
<211> 17
<212> DNA
<213> Homo sapiens

<400> 1394
ctgctgttgc acattgg                                                    17

<210> 1395
<211> 17
<212> DNA
<213> Homo sapiens

<400> 1395
tgctgttgca cattggg                                                    17

<210> 1396
<211> 17
<212> DNA
<213> Homo sapiens

<400> 1396
gctgttgcac attgggg                                                    17

<210> 1397
<211> 17
<212> DNA
<213> Homo sapiens

<400> 1397

ctgttgcaca ttggggt                                                    17

<210> 1398
<211> 17
<212> DNA
<213> Homo sapiens

<400> 1398
tgttgcacat tggggtg                                                    17

<210> 1399
<211> 17
<212> DNA
<213> Homo sapiens

<400> 1399
gttgcacatt ggggtgc                                                    17

<210> 1400
<211> 17
<212> DNA
<213> Homo sapiens

<400> 1400
ttgcacattg gggtgcc                                                    17

<210> 1401
<211> 17
<212> DNA
<213> Homo sapiens

<400> 1401
tgcacattgg ggtgcca                                                    17

<210> 1402
<211> 17
<212> DNA
<213> Homo sapiens

<400> 1402
gcacattggg gtgccat                                                    17

<210> 1403
<211> 17
<212> DNA
<213> Homo sapiens

<400> 1403
cacattgggg tgccatt                                                    17

<210> 1404
<211> 17
<212> DNA
<213> Homo sapiens

<400> 1404
acattggggt gccattt                                                    17

<210> 1405
<211> 17
<212> DNA

<213> Homo sapiens

<400> 1405
cattggggtg ccatttg                                                    17

<210> 1406
<211> 17
<212> DNA
<213> Homo sapiens

<400> 1406
attggggtgc catttgt                                                    17

<210> 1407
<211> 17
<212> DNA
<213> Homo sapiens

<400> 1407
ttggggtgcc atttgtc                                                    17

<210> 1408
<211> 17
<212> DNA
<213> Homo sapiens

<400> 1408
tggggtgcca tttgtca                                                    17

<210> 1409
<211> 17
<212> DNA
<213> Homo sapiens

<400> 1409
ggggtgccat ttgtcat                                                    17

<210> 1410
<211> 17
<212> DNA
<213> Homo sapiens

<400> 1410
gggtgccatt tgtcatc                                                    17

<210> 1411
<211> 17
<212> DNA
<213> Homo sapiens

<400> 1411
ggtgccattt gtcatca                                                    17

<210> 1412
<211> 17
<212> DNA
<213> Homo sapiens

<400> 1412
gtgccatttg tcatcat                                                    17

<210> 1413
<211> 17
<212> DNA
<213> Homo sapiens

<400> 1413
tgccatttgt catcata                                                    17

<210> 1414
<211> 17
<212> DNA
<213> Homo sapiens

<400> 1414
gccatttgtc atcatag                                                    17

<210> 1415
<211> 17
<212> DNA
<213> Homo sapiens

<400> 1415
ccatttgtca tcatagt                                                    17

<210> 1416
<211> 17
<212> DNA
<213> Homo sapiens

<400> 1416
catttgtcat catagtt                                                    17

<210> 1417
<211> 17
<212> DNA
<213> Homo sapiens

<400> 1417
atttgtcatc atagttg                                                    17

<210> 1418
<211> 17
<212> DNA
<213> Homo sapiens

<400> 1418
tttgtcatca tagttgc                                                    17

<210> 1419
<211> 17
<212> DNA
<213> Homo sapiens

<400> 1419
ttgtcatcat agttgcc                                                    17

<210> 1420
<211> 17
<212> DNA
<213> Homo sapiens

<400> 1420
tgtcatcata gttgcca                                                    17

<210> 1421
<211> 17
<212> DNA
<213> Homo sapiens

<400> 1421
gtcatcatag ttgccaa                                                    17

<210> 1422
<211> 17
<212> DNA
<213> Homo sapiens

<400> 1422
tcatcatagt tgccaat                                                    17

<210> 1423
<211> 17
<212> DNA
<213> Homo sapiens

<400> 1423
catcatagtt gccaatt                                                    17

<210> 1424
<211> 17
<212> DNA
<213> Homo sapiens

<400> 1424
atcatagttg ccaattc                                                    17

<210> 1425
<211> 17
<212> DNA
<213> Homo sapiens

<400> 1425
tcatagttgc caattca                                                    17

<210> 1426
<211> 17
<212> DNA
<213> Homo sapiens

<400> 1426
catagttgcc aattcac                                                    17

<210> 1427
<211> 17
<212> DNA
<213> Homo sapiens

<400> 1427
atagttgcca attcacc                                                    17

<210> 1428
<211> 17

<212> DNA
<213> Homo sapiens

<400> 1428
tagttgccaa ttcacca                                                    17

<210> 1429
<211> 17
<212> DNA
<213> Homo sapiens

<400> 1429
agttgccaat tcaccat                                                    17

<210> 1430
<211> 17
<212> DNA
<213> Homo sapiens

<400> 1430
gttgccaatt caccatt                                                    17

<210> 1431
<211> 17
<212> DNA
<213> Homo sapiens

<400> 1431
ttgccaattc accattt                                                    17

<210> 1432
<211> 17
<212> DNA
<213> Homo sapiens

<400> 1432
tgccaattca ccatttc                                                    17

<210> 1433
<211> 17
<212> DNA
<213> Homo sapiens

<400> 1433
gccaattcac catttct                                                    17

<210> 1434
<211> 17
<212> DNA
<213> Homo sapiens

<400> 1434
ccaattcacc atttctt                                                    17

<210> 1435
<211> 17
<212> DNA
<213> Homo sapiens

<400> 1435
caattcacca tttctta                                                    17

<210> 1436
<211> 17
<212> DNA
<213> Homo sapiens

<400> 1436
aattcaccat ttcttat                                                    17

<210> 1437
<211> 17
<212> DNA
<213> Homo sapiens

<400> 1437
attcaccatt tcttatt                                                    17

<210> 1438
<211> 17
<212> DNA
<213> Homo sapiens

<400> 1438
ttcaccattt cttattc                                                    17

<210> 1439
<211> 17
<212> DNA
<213> Homo sapiens

<400> 1439
tcaccatttc ttattct                                                    17

<210> 1440
<211> 17
<212> DNA
<213> Homo sapiens

<400> 1440
caccatttct tattcta                                                    17

<210> 1441
<211> 17
<212> DNA
<213> Homo sapiens

<400> 1441
accatttctt attctag                                                    17

<210> 1442
<211> 17
<212> DNA
<213> Homo sapiens

<400> 1442
ccatttctta ttctagg                                                    17

<210> 1443
<211> 17
<212> DNA
<213> Homo sapiens

<400> 1443
catttcttat tctaggt                                                    17

<210> 1444
<211> 17
<212> DNA
<213> Homo sapiens

<400> 1444
atttcttatt ctaggtg                                                    17

<210> 1445
<211> 17
<212> DNA
<213> Homo sapiens

<400> 1445
tttcttattc taggtgt                                                    17

<210> 1446
<211> 17
<212> DNA
<213> Homo sapiens

<400> 1446
ttcttattct aggtgtt                                                    17

<210> 1447
<211> 17
<212> DNA
<213> Homo sapiens

<400> 1447
tcttattcta ggtgttg                                                    17

<210> 1448
<211> 17
<212> DNA
<213> Homo sapiens

<400> 1448
cttattctag gtgttgg                                                    17

<210> 1449
<211> 17
<212> DNA
<213> Homo sapiens

<400> 1449
ttattctagg tgttggg                                                    17

<210> 1450
<211> 17
<212> DNA
<213> Homo sapiens

<400> 1450
tattctaggt gttgggg                                                    17

<210> 1451

<211> 17
<212> DNA
<213> Homo sapiens

<400> 1451
attctaggtg ttggggt                                                    17

<210> 1452
<211> 17
<212> DNA
<213> Homo sapiens

<400> 1452
ttctaggtgt tggggtc                                                    17

<210> 1453
<211> 17
<212> DNA
<213> Homo sapiens

<400> 1453
tctaggtgtt ggggtcg                                                    17

<210> 1454
<211> 17
<212> DNA
<213> Homo sapiens

<400> 1454
ctaggtgttg gggtcga                                                    17

<210> 1455
<211> 17
<212> DNA
<213> Homo sapiens

<400> 1455
taggtgttgg ggtcgat                                                    17

<210> 1456
<211> 17
<212> DNA
<213> Homo sapiens

<400> 1456
aggtgttggg gtcgatg                                                    17

<210> 1457
<211> 17
<212> DNA
<213> Homo sapiens

<400> 1457
ggtgttgggg tcgatga                                                    17

<210> 1458
<211> 17
<212> DNA
<213> Homo sapiens

<400> 1458

gtgttggggt cgatgac                                                    17

<210> 1459
<211> 17
<212> DNA
<213> Homo sapiens

<400> 1459
tgttggggtc gatgaca                                                    17

<210> 1460
<211> 17
<212> DNA
<213> Homo sapiens

<400> 1460
gttggggtcg atgacat                                                    17

<210> 1461
<211> 17
<212> DNA
<213> Homo sapiens

<400> 1461
ttggggtcga tgacatg                                                    17

<210> 1462
<211> 17
<212> DNA
<213> Homo sapiens

<400> 1462
tggggtcgat gacatgt                                                    17

<210> 1463
<211> 17
<212> DNA
<213> Homo sapiens

<400> 1463
ggggtcgatg acatgtt                                                    17

<210> 1464
<211> 17
<212> DNA
<213> Homo sapiens

<400> 1464
gggtcgatga catgttt                                                    17

<210> 1465
<211> 17
<212> DNA
<213> Homo sapiens

<400> 1465
ggtcgatgac atgttta                                                    17

<210> 1466
<211> 17
<212> DNA

<213> Homo sapiens

<400> 1466
gtcgatgaca tgtttat                                                    17

<210> 1467
<211> 17
<212> DNA
<213> Homo sapiens

<400> 1467
tcgatgacat gtttatc                                                    17

<210> 1468
<211> 17
<212> DNA
<213> Homo sapiens

<400> 1468
cgatgacatg tttatca                                                    17

<210> 1469
<211> 17
<212> DNA
<213> Homo sapiens

<400> 1469
gatgacatgt ttatcat                                                    17

<210> 1470
<211> 17
<212> DNA
<213> Homo sapiens

<400> 1470
atgacatgtt tatcatg                                                    17

<210> 1471
<211> 17
<212> DNA
<213> Homo sapiens

<400> 1471
tgacatgttt atcatga                                                    17

<210> 1472
<211> 17
<212> DNA
<213> Homo sapiens

<400> 1472
gacatgttta tcatgat                                                    17

<210> 1473
<211> 17
<212> DNA
<213> Homo sapiens

<400> 1473
acatgtttat catgatt                                                    17

<210> 1474
<211> 17
<212> DNA
<213> Homo sapiens

<400> 1474
catgtttatc atgattt                                                    17

<210> 1475
<211> 17
<212> DNA
<213> Homo sapiens

<400> 1475
atgtttatca tgatttc                                                    17

<210> 1476
<211> 17
<212> DNA
<213> Homo sapiens

<400> 1476
tgtttatcat gatttct                                                    17

<210> 1477
<211> 17
<212> DNA
<213> Homo sapiens

<400> 1477
gtttatcatg atttctg                                                    17

<210> 1478
<211> 17
<212> DNA
<213> Homo sapiens

<400> 1478
tttatcatga tttctgc                                                    17

<210> 1479
<211> 17
<212> DNA
<213> Homo sapiens

<400> 1479
ttatcatgat ttctgcc                                                    17

<210> 1480
<211> 17
<212> DNA
<213> Homo sapiens

<400> 1480
tatcatgatt tctgcct                                                    17

<210> 1481
<211> 17
<212> DNA
<213> Homo sapiens

<400> 1481
atcatgattt ctgcctg                                                    17

<210> 1482
<211> 17
<212> DNA
<213> Homo sapiens

<400> 1482
tcatgatttc tgcctgg                                                    17

<210> 1483
<211> 17
<212> DNA
<213> Homo sapiens

<400> 1483
catgatttct gcctggc                                                    17

<210> 1484
<211> 17
<212> DNA
<213> Homo sapiens

<400> 1484
atgatttctg cctggca                                                    17

<210> 1485
<211> 17
<212> DNA
<213> Homo sapiens

<400> 1485
tgatttctgc ctggcat                                                    17

<210> 1486
<211> 17
<212> DNA
<213> Homo sapiens

<400> 1486
gatttctgcc tggcata                                                    17

<210> 1487
<211> 17
<212> DNA
<213> Homo sapiens

<400> 1487
atttctgcct ggcataa                                                    17

<210> 1488
<211> 17
<212> DNA
<213> Homo sapiens

<400> 1488
tttctgcctg gcataag                                                    17

<210> 1489
<211> 17

<212> DNA
<213> Homo sapiens

<400> 1489
ttctgcctgg cataaga                                                    17

<210> 1490
<211> 17
<212> DNA
<213> Homo sapiens

<400> 1490
tctgcctggc ataagac                                                    17

<210> 1491
<211> 17
<212> DNA
<213> Homo sapiens

<400> 1491
ctgcctggca taagacc                                                    17

<210> 1492
<211> 17
<212> DNA
<213> Homo sapiens

<400> 1492
tgcctggcat aagacca                                                    17

<210> 1493
<211> 17
<212> DNA
<213> Homo sapiens

<400> 1493
gcctggcata agaccaa                                                    17

<210> 1494
<211> 17
<212> DNA
<213> Homo sapiens

<400> 1494
cctggcataa gaccaac                                                    17

<210> 1495
<211> 17
<212> DNA
<213> Homo sapiens

<400> 1495
ctggcataag accaacc                                                    17

<210> 1496
<211> 17
<212> DNA
<213> Homo sapiens

<400> 1496
tggcataaga ccaacct                                                    17

<210> 1497
<211> 17
<212> DNA
<213> Homo sapiens

<400> 1497
ggcataagac caacctt                                                    17

<210> 1498
<211> 17
<212> DNA
<213> Homo sapiens

<400> 1498
gcataagacc aaccttg                                                    17

<210> 1499
<211> 17
<212> DNA
<213> Homo sapiens

<400> 1499
cataagacca accttgc                                                    17

<210> 1500
<211> 17
<212> DNA
<213> Homo sapiens

<400> 1500
ataagaccaa ccttgca                                                    17

<210> 1501
<211> 17
<212> DNA
<213> Homo sapiens

<400> 1501
taagaccaac cttgcag                                                    17

<210> 1502
<211> 17
<212> DNA
<213> Homo sapiens

<400> 1502
aagaccaacc ttgcagg                                                    17

<210> 1503
<211> 17
<212> DNA
<213> Homo sapiens

<400> 1503
agaccaacct tgcaggt                                                    17

<210> 1504
<211> 17
<212> DNA
<213> Homo sapiens

<400> 1504
gaccaacctt gcaggtg                                                    17

<210> 1505
<211> 17
<212> DNA
<213> Homo sapiens

<400> 1505
accaaccttg caggtga                                                    17

<210> 1506
<211> 17
<212> DNA
<213> Homo sapiens

<400> 1506
ccaaccttgc aggtgac                                                    17

<210> 1507
<211> 17
<212> DNA
<213> Homo sapiens

<400> 1507
caaccttgca ggtgaca                                                    17

<210> 1508
<211> 17
<212> DNA
<213> Homo sapiens

<400> 1508
aaccttgcag gtgacat                                                    17

<210> 1509
<211> 17
<212> DNA
<213> Homo sapiens

<400> 1509
accttgcagg tgacata                                                    17

<210> 1510
<211> 17
<212> DNA
<213> Homo sapiens

<400> 1510
ccttgcaggt gacatac                                                    17

<210> 1511
<211> 17
<212> DNA
<213> Homo sapiens

<400> 1511
cttgcaggtg acatacg                                                    17

<210> 1512

<211> 17
<212> DNA
<213> Homo sapiens

<400> 1512
ttgcaggtga catacga                                                      17

<210> 1513
<211> 17
<212> DNA
<213> Homo sapiens

<400> 1513
tgcaggtgac atacgag                                                      17

<210> 1514
<211> 17
<212> DNA
<213> Homo sapiens

<400> 1514
gcaggtgaca tacgaga                                                      17

<210> 1515
<211> 17
<212> DNA
<213> Homo sapiens

<400> 1515
caggtgacat acgagag                                                      17

<210> 1516
<211> 17
<212> DNA
<213> Homo sapiens

<400> 1516
aggtgacata cgagagc                                                      17

<210> 1517
<211> 17
<212> DNA
<213> Homo sapiens

<400> 1517
ggtgacatac gagagcg                                                      17

<210> 1518
<211> 17
<212> DNA
<213> Homo sapiens

<400> 1518
gtgacatacg agagcgg                                                      17

<210> 1519
<211> 17
<212> DNA
<213> Homo sapiens

<400> 1519

tgacatacga gagcgga                                                    17

<210> 1520
<211> 17
<212> DNA
<213> Homo sapiens

<400> 1520
gacatacgag agcggat                                                    17

<210> 1521
<211> 17
<212> DNA
<213> Homo sapiens

<400> 1521
acatacgaga gcggatg                                                    17

<210> 1522
<211> 17
<212> DNA
<213> Homo sapiens

<400> 1522
catacgagag cggatgt                                                    17

<210> 1523
<211> 17
<212> DNA
<213> Homo sapiens

<400> 1523
atacgagagc ggatgtc                                                    17

<210> 1524
<211> 17
<212> DNA
<213> Homo sapiens

<400> 1524
tacgagagcg gatgtcc                                                    17

<210> 1525
<211> 17
<212> DNA
<213> Homo sapiens

<400> 1525
acgagagcgg atgtcca                                                    17

<210> 1526
<211> 17
<212> DNA
<213> Homo sapiens

<400> 1526
cgagagcgga tgtccaa                                                    17

<210> 1527
<211> 17
<212> DNA

<213> Homo sapiens

<400> 1527
gagagcggat gtccaat                                                    17

<210> 1528
<211> 17
<212> DNA
<213> Homo sapiens

<400> 1528
agagcggatg tccaatg                                                    17

<210> 1529
<211> 17
<212> DNA
<213> Homo sapiens

<400> 1529
gagcggatgt ccaatgt                                                    17

<210> 1530
<211> 17
<212> DNA
<213> Homo sapiens

<400> 1530
agcggatgtc caatgtc                                                    17

<210> 1531
<211> 17
<212> DNA
<213> Homo sapiens

<400> 1531
gcggatgtcc aatgtct                                                    17

<210> 1532
<211> 17
<212> DNA
<213> Homo sapiens

<400> 1532
cggatgtcca atgtcta                                                    17

<210> 1533
<211> 17
<212> DNA
<213> Homo sapiens

<400> 1533
ggatgtccaa tgtctat                                                    17

<210> 1534
<211> 17
<212> DNA
<213> Homo sapiens

<400> 1534
gatgtccaat gtctatt                                                    17

<210> 1535
<211> 17
<212> DNA
<213> Homo sapiens

<400> 1535
atgtccaatg tctattc                                                    17

<210> 1536
<211> 17
<212> DNA
<213> Homo sapiens

<400> 1536
tgtccaatgt ctattca                                                    17

<210> 1537
<211> 17
<212> DNA
<213> Homo sapiens

<400> 1537
gtccaatgtc tattcaa                                                    17

<210> 1538
<211> 17
<212> DNA
<213> Homo sapiens

<400> 1538
tccaatgtct attcaaa                                                    17

<210> 1539
<211> 17
<212> DNA
<213> Homo sapiens

<400> 1539
ccaatgtcta ttcaaaa                                                    17

<210> 1540
<211> 17
<212> DNA
<213> Homo sapiens

<400> 1540
caatgtctat tcaaaag                                                    17

<210> 1541
<211> 17
<212> DNA
<213> Homo sapiens

<400> 1541
aatgtctatt caaaagc                                                    17

<210> 1542
<211> 17
<212> DNA
<213> Homo sapiens

<400> 1542
atgtctattc aaaagcg                                                    17

<210> 1543
<211> 17
<212> DNA
<213> Homo sapiens

<400> 1543
tgtctattca aaagcgg                                                    17

<210> 1544
<211> 17
<212> DNA
<213> Homo sapiens

<400> 1544
gtctattcaa aagcggc                                                    17

<210> 1545
<211> 17
<212> DNA
<213> Homo sapiens

<400> 1545
tctattcaaa agcggca                                                    17

<210> 1546
<211> 17
<212> DNA
<213> Homo sapiens

<400> 1546
ctattcaaaa gcggcag                                                    17

<210> 1547
<211> 17
<212> DNA
<213> Homo sapiens

<400> 1547
tattcaaaag cggcagt                                                    17

<210> 1548
<211> 17
<212> DNA
<213> Homo sapiens

<400> 1548
attcaaaagc ggcagtg                                                    17

<210> 1549
<211> 17
<212> DNA
<213> Homo sapiens

<400> 1549
ttcaaaagcg gcagtgt                                                    17

<210> 1550
<211> 17

<212> DNA
<213> Homo sapiens

<400> 1550
tcaaaagcgg cagtgtc                                                    17

<210> 1551
<211> 17
<212> DNA
<213> Homo sapiens

<400> 1551
caaaagcggc agtgtct                                                    17

<210> 1552
<211> 17
<212> DNA
<213> Homo sapiens

<400> 1552
aaaagcggca gtgtcta                                                    17

<210> 1553
<211> 17
<212> DNA
<213> Homo sapiens

<400> 1553
aaagcggcag tgtctat                                                    17

<210> 1554
<211> 17
<212> DNA
<213> Homo sapiens

<400> 1554
aagcggcagt gtctatt                                                    17

<210> 1555
<211> 17
<212> DNA
<213> Homo sapiens

<400> 1555
agcggcagtg tctatta                                                    17

<210> 1556
<211> 17
<212> DNA
<213> Homo sapiens

<400> 1556
gcggcagtgt ctattac                                                    17

<210> 1557
<211> 17
<212> DNA
<213> Homo sapiens

<400> 1557
cggcagtgtc tattaca                                                    17

<210> 1558
<211> 17
<212> DNA
<213> Homo sapiens

<400> 1558
ggcagtgtct attacaa                                                    17

<210> 1559
<211> 17
<212> DNA
<213> Homo sapiens

<400> 1559
gcagtgtcta ttacaat                                                    17

<210> 1560
<211> 17
<212> DNA
<213> Homo sapiens

<400> 1560
cagtgtctat tacaatc                                                    17

<210> 1561
<211> 17
<212> DNA
<213> Homo sapiens

<400> 1561
agtgtctatt acaatca                                                    17

<210> 1562
<211> 17
<212> DNA
<213> Homo sapiens

<400> 1562
gtgtctatta caatcac                                                    17

<210> 1563
<211> 17
<212> DNA
<213> Homo sapiens

<400> 1563
tgtctattac aatcacc                                                    17

<210> 1564
<211> 17
<212> DNA
<213> Homo sapiens

<400> 1564
gtctattaca atcacca                                                    17

<210> 1565
<211> 17
<212> DNA
<213> Homo sapiens

<400> 1565
tctattacaa tcaccac                                                        17

<210> 1566
<211> 17
<212> DNA
<213> Homo sapiens

<400> 1566
ctattacaat caccacc                                                        17

<210> 1567
<211> 17
<212> DNA
<213> Homo sapiens

<400> 1567
tattacaatc accacca                                                        17

<210> 1568
<211> 17
<212> DNA
<213> Homo sapiens

<400> 1568
attacaatca ccaccat                                                        17

<210> 1569
<211> 17
<212> DNA
<213> Homo sapiens

<400> 1569
ttacaatcac caccatc                                                        17

<210> 1570
<211> 17
<212> DNA
<213> Homo sapiens

<400> 1570
tacaatcacc accatca                                                        17

<210> 1571
<211> 17
<212> DNA
<213> Homo sapiens

<400> 1571
acaatcacca ccatcac                                                        17

<210> 1572
<211> 17
<212> DNA
<213> Homo sapiens

<400> 1572
caatcaccac catcact                                                        17

<210> 1573

<211> 17
<212> DNA
<213> Homo sapiens

<400> 1573
aatcaccacc atcacta                                                    17

<210> 1574
<211> 17
<212> DNA
<213> Homo sapiens

<400> 1574
atcaccacca tcactaa                                                    17

<210> 1575
<211> 17
<212> DNA
<213> Homo sapiens

<400> 1575
tcaccaccat cactaac                                                    17

<210> 1576
<211> 17
<212> DNA
<213> Homo sapiens

<400> 1576
caccaccatc actaaca                                                    17

<210> 1577
<211> 17
<212> DNA
<213> Homo sapiens

<400> 1577
accaccatca ctaacat                                                    17

<210> 1578
<211> 17
<212> DNA
<213> Homo sapiens

<400> 1578
ccaccatcac taacatc                                                    17

<210> 1579
<211> 17
<212> DNA
<213> Homo sapiens

<400> 1579
caccatcact aacatcc                                                    17

<210> 1580
<211> 17
<212> DNA
<213> Homo sapiens

<400> 1580

accatcacta acatcct                                                    17

<210> 1581
<211> 17
<212> DNA
<213> Homo sapiens

<400> 1581
ccatcactaa catcctg                                                    17

<210> 1582
<211> 17
<212> DNA
<213> Homo sapiens

<400> 1582
catcactaac atcctgg                                                    17

<210> 1583
<211> 17
<212> DNA
<213> Homo sapiens

<400> 1583
atcactaaca tcctggc                                                    17

<210> 1584
<211> 17
<212> DNA
<213> Homo sapiens

<400> 1584
tcactaacat cctggcc                                                    17

<210> 1585
<211> 17
<212> DNA
<213> Homo sapiens

<400> 1585
cactaacatc ctggcct                                                    17

<210> 1586
<211> 17
<212> DNA
<213> Homo sapiens

<400> 1586
actaacatcc tggcctt                                                    17

<210> 1587
<211> 17
<212> DNA
<213> Homo sapiens

<400> 1587
ctaacatcct ggcctta                                                    17

<210> 1588
<211> 17
<212> DNA

<213> Homo sapiens

<400> 1588
taacatcctg gccttat                                                    17

<210> 1589
<211> 17
<212> DNA
<213> Homo sapiens

<400> 1589
aacatcctgg ccttata                                                    17

<210> 1590
<211> 17
<212> DNA
<213> Homo sapiens

<400> 1590
acatcctggc cttatat                                                    17

<210> 1591
<211> 17
<212> DNA
<213> Homo sapiens

<400> 1591
catcctggcc ttatata                                                    17

<210> 1592
<211> 17
<212> DNA
<213> Homo sapiens

<400> 1592
atcctggcct tatatac                                                    17

<210> 1593
<211> 17
<212> DNA
<213> Homo sapiens

<400> 1593
tcctggcctt atataca                                                    17

<210> 1594
<211> 17
<212> DNA
<213> Homo sapiens

<400> 1594
cctggcctta tatacag                                                    17

<210> 1595
<211> 17
<212> DNA
<213> Homo sapiens

<400> 1595
ctggccttat atacagg                                                    17

<210> 1596
<211> 17
<212> DNA
<213> Homo sapiens

<400> 1596
tggccttata tacaggg                                                    17

<210> 1597
<211> 17
<212> DNA
<213> Homo sapiens

<400> 1597
ggccttatat acaggga                                                    17

<210> 1598
<211> 17
<212> DNA
<213> Homo sapiens

<400> 1598
gccttatata cagggat                                                    17

<210> 1599
<211> 17
<212> DNA
<213> Homo sapiens

<400> 1599
ccttatatac agggatt                                                    17

<210> 1600
<211> 17
<212> DNA
<213> Homo sapiens

<400> 1600
cttatataca gggatta                                                    17

<210> 1601
<211> 17
<212> DNA
<213> Homo sapiens

<400> 1601
ttatatacag ggattat                                                    17

<210> 1602
<211> 17
<212> DNA
<213> Homo sapiens

<400> 1602
tatatacagg gattatg                                                    17

<210> 1603
<211> 17
<212> DNA
<213> Homo sapiens

<400> 1603
atatacaggg attatga                                                    17

<210> 1604
<211> 17
<212> DNA
<213> Homo sapiens

<400> 1604
tatacaggga ttatgag                                                    17

<210> 1605
<211> 17
<212> DNA
<213> Homo sapiens

<400> 1605
atacagggat tatgagc                                                    17

<210> 1606
<211> 17
<212> DNA
<213> Homo sapiens

<400> 1606
tacagggatt atgagct                                                    17

<210> 1607
<211> 17
<212> DNA
<213> Homo sapiens

<400> 1607
acagggatta tgagctc                                                    17

<210> 1608
<211> 17
<212> DNA
<213> Homo sapiens

<400> 1608
cagggattat gagctct                                                    17

<210> 1609
<211> 17
<212> DNA
<213> Homo sapiens

<400> 1609
agggattatg agctctt                                                    17

<210> 1610
<211> 17
<212> DNA
<213> Homo sapiens

<400> 1610
gggattatga gctcttt                                                    17

<210> 1611
<211> 17

<212> DNA
<213> Homo sapiens

<400> 1611
ggattatgag ctctttt                                                    17

<210> 1612
<211> 17
<212> DNA
<213> Homo sapiens

<400> 1612
gattatgagc tctttta                                                    17

<210> 1613
<211> 17
<212> DNA
<213> Homo sapiens

<400> 1613
attatgagct cttttag                                                    17

<210> 1614
<211> 17
<212> DNA
<213> Homo sapiens

<400> 1614
ttatgagctc ttttagg                                                    17

<210> 1615
<211> 17
<212> DNA
<213> Homo sapiens

<400> 1615
tatgagctct tttaggt                                                    17

<210> 1616
<211> 17
<212> DNA
<213> Homo sapiens

<400> 1616
atgagctctt ttaggtc                                                    17

<210> 1617
<211> 17
<212> DNA
<213> Homo sapiens

<400> 1617
tgagctcttt taggtcc                                                    17

<210> 1618
<211> 17
<212> DNA
<213> Homo sapiens

<400> 1618
gagctctttt aggtccg                                                    17

<210> 1619
<211> 17
<212> DNA
<213> Homo sapiens

<400> 1619
agctctttta ggtccgt                                                    17

<210> 1620
<211> 17
<212> DNA
<213> Homo sapiens

<400> 1620
gctcttttag gtccgta                                                    17

<210> 1621
<211> 17
<212> DNA
<213> Homo sapiens

<400> 1621
ctcttttagg tccgtac                                                    17

<210> 1622
<211> 17
<212> DNA
<213> Homo sapiens

<400> 1622
tcttttaggt ccgtaca                                                    17

<210> 1623
<211> 17
<212> DNA
<213> Homo sapiens

<400> 1623
cttttaggtc cgtacaa                                                    17

<210> 1624
<211> 17
<212> DNA
<213> Homo sapiens

<400> 1624
ttttaggtcc gtacaat                                                    17

<210> 1625
<211> 17
<212> DNA
<213> Homo sapiens

<400> 1625
tttaggtccg tacaatg                                                    17

<210> 1626
<211> 17
<212> DNA
<213> Homo sapiens

<400> 1626
ttaggtccgt acaatgt                                                    17

<210> 1627
<211> 17
<212> DNA
<213> Homo sapiens

<400> 1627
taggtccgta caatgtt                                                    17

<210> 1628
<211> 17
<212> DNA
<213> Homo sapiens

<400> 1628
aggtccgtac aatgttt                                                    17

<210> 1629
<211> 17
<212> DNA
<213> Homo sapiens

<400> 1629
ggtccgtaca atgtttt                                                    17

<210> 1630
<211> 17
<212> DNA
<213> Homo sapiens

<400> 1630
gtccgtacaa tgttttt                                                    17

<210> 1631
<211> 17
<212> DNA
<213> Homo sapiens

<400> 1631
tccgtacaat gtttttg                                                    17

<210> 1632
<211> 17
<212> DNA
<213> Homo sapiens

<400> 1632
ccgtacaatg tttttgc                                                    17

<210> 1633
<211> 17
<212> DNA
<213> Homo sapiens

<400> 1633
cgtacaatgt ttttgca                                                    17

<210> 1634

<211> 17
<212> DNA
<213> Homo sapiens

<400> 1634
gtacaatgtt tttgcat                                                    17

<210> 1635
<211> 17
<212> DNA
<213> Homo sapiens

<400> 1635
tacaatgttt ttgcatc                                                    17

<210> 1636
<211> 17
<212> DNA
<213> Homo sapiens

<400> 1636
acaatgtttt tgcatct                                                    17

<210> 1637
<211> 17
<212> DNA
<213> Homo sapiens

<400> 1637
caatgttttt gcatcta                                                    17

<210> 1638
<211> 17
<212> DNA
<213> Homo sapiens

<400> 1638
aatgtttttg catctat                                                    17

<210> 1639
<211> 17
<212> DNA
<213> Homo sapiens

<400> 1639
atgtttttgc atctata                                                    17

<210> 1640
<211> 17
<212> DNA
<213> Homo sapiens

<400> 1640
tgtttttgca tctatac                                                    17

<210> 1641
<211> 17
<212> DNA
<213> Homo sapiens

<400> 1641

gtttttgcat ctataca                                                    17

<210> 1642
<211> 17
<212> DNA
<213> Homo sapiens

<400> 1642
tttttgcatc tatacag                                                    17

<210> 1643
<211> 17
<212> DNA
<213> Homo sapiens

<400> 1643
ttttgcatct atacagg                                                    17

<210> 1644
<211> 17
<212> DNA
<213> Homo sapiens

<400> 1644
tttgcatcta tacagga                                                    17

<210> 1645
<211> 17
<212> DNA
<213> Homo sapiens

<400> 1645
ttgcatctat acaggaa                                                    17

<210> 1646
<211> 17
<212> DNA
<213> Homo sapiens

<400> 1646
tgcatctata caggaac                                                    17

<210> 1647
<211> 17
<212> DNA
<213> Homo sapiens

<400> 1647
gcatctatac aggaacg                                                    17

<210> 1648
<211> 17
<212> DNA
<213> Homo sapiens

<400> 1648
catctataca ggaacga                                                    17

<210> 1649
<211> 17
<212> DNA

<213> Homo sapiens

<400> 1649
atctatacag gaacgac                                                    17

<210> 1650
<211> 17
<212> DNA
<213> Homo sapiens

<400> 1650
tctatacagg aacgacc                                                    17

<210> 1651
<211> 17
<212> DNA
<213> Homo sapiens

<400> 1651
ctatacagga acgaccc                                                    17

<210> 1652
<211> 17
<212> DNA
<213> Homo sapiens

<400> 1652
tatacaggaa cgaccct                                                    17

<210> 1653
<211> 17
<212> DNA
<213> Homo sapiens

<400> 1653
atacaggaac gaccctg                                                    17

<210> 1654
<211> 17
<212> DNA
<213> Homo sapiens

<400> 1654
tacaggaacg accctgt                                                    17

<210> 1655
<211> 17
<212> DNA
<213> Homo sapiens

<400> 1655
acaggaacga ccctgtt                                                    17

<210> 1656
<211> 17
<212> DNA
<213> Homo sapiens

<400> 1656
caggaacgac cctgtta                                                    17

<210> 1657
<211> 17
<212> DNA
<213> Homo sapiens

<400> 1657
aggaacgacc ctgttat                                                    17

<210> 1658
<211> 17
<212> DNA
<213> Homo sapiens

<400> 1658
ggaacgaccc tgttatt                                                    17

<210> 1659
<211> 17
<212> DNA
<213> Homo sapiens

<400> 1659
gaacgaccct gttattt                                                    17

<210> 1660
<211> 17
<212> DNA
<213> Homo sapiens

<400> 1660
aacgaccctg ttatttt                                                    17

<210> 1661
<211> 17
<212> DNA
<213> Homo sapiens

<400> 1661
acgaccctgt tattttg                                                    17

<210> 1662
<211> 17
<212> DNA
<213> Homo sapiens

<400> 1662
cgaccctgtt attttgc                                                    17

<210> 1663
<211> 17
<212> DNA
<213> Homo sapiens

<400> 1663
gaccctgtta ttttgct                                                    17

<210> 1664
<211> 17
<212> DNA
<213> Homo sapiens

<400> 1664
accctgttat tttgcta                                                    17

<210> 1665
<211> 17
<212> DNA
<213> Homo sapiens

<400> 1665
ccctgttatt ttgctat                                                    17

<210> 1666
<211> 17
<212> DNA
<213> Homo sapiens

<400> 1666
cctgttattt tgctatt                                                    17

<210> 1667
<211> 17
<212> DNA
<213> Homo sapiens

<400> 1667
ctgttatttt gctattt                                                    17

<210> 1668
<211> 17
<212> DNA
<213> Homo sapiens

<400> 1668
tgttattttg ctatttt                                                    17

<210> 1669
<211> 17
<212> DNA
<213> Homo sapiens

<400> 1669
gttattttgc tattttt                                                    17

<210> 1670
<211> 17
<212> DNA
<213> Homo sapiens

<400> 1670
ttattttgct attttta                                                    17

<210> 1671
<211> 17
<212> DNA
<213> Homo sapiens

<400> 1671
tattttgcta tttttat                                                    17

<210> 1672
<211> 17

<212> DNA
<213> Homo sapiens

<400> 1672
attttgctat ttttata                                                    17

<210> 1673
<211> 17
<212> DNA
<213> Homo sapiens

<400> 1673
ttttgctatt tttataa                                                    17

<210> 1674
<211> 17
<212> DNA
<213> Homo sapiens

<400> 1674
tttgctattt ttataac                                                    17

<210> 1675
<211> 17
<212> DNA
<213> Homo sapiens

<400> 1675
ttgctatttt tataaca                                                    17

<210> 1676
<211> 17
<212> DNA
<213> Homo sapiens

<400> 1676
tgctattttt ataacat                                                    17

<210> 1677
<211> 17
<212> DNA
<213> Homo sapiens

<400> 1677
gctattttta taacatc                                                    17

<210> 1678
<211> 17
<212> DNA
<213> Homo sapiens

<400> 1678
ctatttttat aacatca                                                    17

<210> 1679
<211> 17
<212> DNA
<213> Homo sapiens

<400> 1679
tatttttata acatcac                                                    17

<210> 1680
<211> 17
<212> DNA
<213> Homo sapiens

<400> 1680
atttttataa catcacg                                                    17

<210> 1681
<211> 17
<212> DNA
<213> Homo sapiens

<400> 1681
tttttataac atcacgt                                                    17

<210> 1682
<211> 17
<212> DNA
<213> Homo sapiens

<400> 1682
ttttataaca tcacgtg                                                    17

<210> 1683
<211> 17
<212> DNA
<213> Homo sapiens

<400> 1683
tttataacat cacgtgt                                                    17

<210> 1684
<211> 17
<212> DNA
<213> Homo sapiens

<400> 1684
ttataacatc acgtgtt                                                    17

<210> 1685
<211> 17
<212> DNA
<213> Homo sapiens

<400> 1685
tataacatca cgtgttt                                                    17

<210> 1686
<211> 17
<212> DNA
<213> Homo sapiens

<400> 1686
ataacatcac gtgtttt                                                    17

<210> 1687
<211> 17
<212> DNA
<213> Homo sapiens

<400> 1687
taacatcacg tgttttg                                                    17

<210> 1688
<211> 17
<212> DNA
<213> Homo sapiens

<400> 1688
aacatcacgt gttttgg                                                    17

<210> 1689
<211> 17
<212> DNA
<213> Homo sapiens

<400> 1689
acatcacgtg ttttgga                                                    17

<210> 1690
<211> 17
<212> DNA
<213> Homo sapiens

<400> 1690
catcacgtgt tttggag                                                    17

<210> 1691
<211> 17
<212> DNA
<213> Homo sapiens

<400> 1691
atcacgtgtt ttggagc                                                    17

<210> 1692
<211> 17
<212> DNA
<213> Homo sapiens

<400> 1692
tcacgtgttt tggagca                                                    17

<210> 1693
<211> 17
<212> DNA
<213> Homo sapiens

<400> 1693
cacgtgtttt ggagcat                                                    17

<210> 1694
<211> 17
<212> DNA
<213> Homo sapiens

<400> 1694
acgtgttttg gagcatt                                                    17

<210> 1695

<211> 17
<212> DNA
<213> Homo sapiens

<400> 1695
cgtgttttgg agcattt                                                    17

<210> 1696
<211> 17
<212> DNA
<213> Homo sapiens

<400> 1696
gtgttttgga gcattta                                                    17

<210> 1697
<211> 17
<212> DNA
<213> Homo sapiens

<400> 1697
tgttttggag catttat                                                    17

<210> 1698
<211> 17
<212> DNA
<213> Homo sapiens

<400> 1698
gttttggagc atttatg                                                    17

<210> 1699
<211> 17
<212> DNA
<213> Homo sapiens

<400> 1699
ttttggagca tttatgg                                                    17

<210> 1700
<211> 17
<212> DNA
<213> Homo sapiens

<400> 1700
tttggagcat ttatggc                                                    17

<210> 1701
<211> 17
<212> DNA
<213> Homo sapiens

<400> 1701
ttggagcatt tatggcc                                                    17

<210> 1702
<211> 17
<212> DNA
<213> Homo sapiens

<400> 1702

tggagcattt atggcct                                                    17

<210> 1703
<211> 17
<212> DNA
<213> Homo sapiens

<400> 1703
ggagcattta tggcctt                                                    17

<210> 1704
<211> 17
<212> DNA
<213> Homo sapiens

<400> 1704
gagcatttat ggccttg                                                    17

<210> 1705
<211> 17
<212> DNA
<213> Homo sapiens

<400> 1705
agcatttatg gccttgg                                                    17

<210> 1706
<211> 17
<212> DNA
<213> Homo sapiens

<400> 1706
gcatttatgg ccttgga                                                    17

<210> 1707
<211> 17
<212> DNA
<213> Homo sapiens

<400> 1707
catttatggc cttggat                                                    17

<210> 1708
<211> 17
<212> DNA
<213> Homo sapiens

<400> 1708
atttatggcc ttggatg                                                    17

<210> 1709
<211> 17
<212> DNA
<213> Homo sapiens

<400> 1709
tttatggcct tggatgg                                                    17

<210> 1710
<211> 17
<212> DNA

<213> Homo sapiens

<400> 1710
ttatggcctt ggatggt                                                    17

<210> 1711
<211> 17
<212> DNA
<213> Homo sapiens

<400> 1711
tatggccttg gatggta                                                    17

<210> 1712
<211> 17
<212> DNA
<213> Homo sapiens

<400> 1712
atggccttgg atggtaa                                                    17

<210> 1713
<211> 17
<212> DNA
<213> Homo sapiens

<400> 1713
tggccttgga tggtaaa                                                    17

<210> 1714
<211> 17
<212> DNA
<213> Homo sapiens

<400> 1714
ggccttggat ggtaaaa                                                    17

<210> 1715
<211> 17
<212> DNA
<213> Homo sapiens

<400> 1715
gccttggatg gtaaaag                                                    17

<210> 1716
<211> 17
<212> DNA
<213> Homo sapiens

<400> 1716
ccttggatgg taaaaga                                                    17

<210> 1717
<211> 17
<212> DNA
<213> Homo sapiens

<400> 1717
cttggatggt aaaagag                                                    17

<210> 1718
<211> 17
<212> DNA
<213> Homo sapiens

<400> 1718
ttggatggta aaagaga                                                    17

<210> 1719
<211> 17
<212> DNA
<213> Homo sapiens

<400> 1719
tggatggtaa aagagaa                                                    17

<210> 1720
<211> 17
<212> DNA
<213> Homo sapiens

<400> 1720
ggatggtaaa agagaag                                                    17

<210> 1721
<211> 17
<212> DNA
<213> Homo sapiens

<400> 1721
gatggtaaaa gagaagt                                                    17

<210> 1722
<211> 17
<212> DNA
<213> Homo sapiens

<400> 1722
atggtaaaag agaagta                                                    17

<210> 1723
<211> 17
<212> DNA
<213> Homo sapiens

<400> 1723
tggtaaaaga gaagtag                                                    17

<210> 1724
<211> 17
<212> DNA
<213> Homo sapiens

<400> 1724
ggtaaaagag aagtagt                                                    17

<210> 1725
<211> 17
<212> DNA
<213> Homo sapiens

<400> 1725
gtaaaagaga agtagtc                                                    17

<210> 1726
<211> 17
<212> DNA
<213> Homo sapiens

<400> 1726
taaaagagaa gtagtct                                                    17

<210> 1727
<211> 17
<212> DNA
<213> Homo sapiens

<400> 1727
aaaagagaag tagtctg                                                    17

<210> 1728
<211> 17
<212> DNA
<213> Homo sapiens

<400> 1728
aaagagaagt agtctgc                                                    17

<210> 1729
<211> 17
<212> DNA
<213> Homo sapiens

<400> 1729
aagagaagta gtctgcc                                                    17

<210> 1730
<211> 17
<212> DNA
<213> Homo sapiens

<400> 1730
agagaagtag tctgcct                                                    17

<210> 1731
<211> 17
<212> DNA
<213> Homo sapiens

<400> 1731
gagaagtagt ctgccta                                                    17

<210> 1732
<211> 17
<212> DNA
<213> Homo sapiens

<400> 1732
agaagtagtc tgcctat                                                    17

<210> 1733
<211> 17

<212> DNA
<213> Homo sapiens

<400> 1733
gaagtagtct gcctatg                                                    17

<210> 1734
<211> 17
<212> DNA
<213> Homo sapiens

<400> 1734
aagtagtctg cctatgc                                                    17

<210> 1735
<211> 17
<212> DNA
<213> Homo sapiens

<400> 1735
agtagtctgc ctatgct                                                    17

<210> 1736
<211> 17
<212> DNA
<213> Homo sapiens

<400> 1736
gtagtctgcc tatgctg                                                    17

<210> 1737
<211> 17
<212> DNA
<213> Homo sapiens

<400> 1737
tagtctgcct atgctgg                                                    17

<210> 1738
<211> 17
<212> DNA
<213> Homo sapiens

<400> 1738
agtctgccta tgctggt                                                    17

<210> 1739
<211> 17
<212> DNA
<213> Homo sapiens

<400> 1739
gtctgcctat gctggtt                                                    17

<210> 1740
<211> 17
<212> DNA
<213> Homo sapiens

<400> 1740
tctgcctatg ctggttg                                                    17

<210> 1741
<211> 17
<212> DNA
<213> Homo sapiens

<400> 1741
ctgcctatgc tggttga                                                    17

<210> 1742
<211> 17
<212> DNA
<213> Homo sapiens

<400> 1742
tgcctatgct ggttgaa                                                    17

<210> 1743
<211> 17
<212> DNA
<213> Homo sapiens

<400> 1743
gcctatgctg gttgaaa                                                    17

<210> 1744
<211> 17
<212> DNA
<213> Homo sapiens

<400> 1744
cctatgctgg ttgaaaa                                                    17

<210> 1745
<211> 17
<212> DNA
<213> Homo sapiens

<400> 1745
ctatgctggt tgaaaaa                                                    17

<210> 1746
<211> 17
<212> DNA
<213> Homo sapiens

<400> 1746
tatgctggtt gaaaaag                                                    17

<210> 1747
<211> 17
<212> DNA
<213> Homo sapiens

<400> 1747
atgctggttg aaaaagg                                                    17

<210> 1748
<211> 17
<212> DNA
<213> Homo sapiens

<400> 1748
tgctggttga aaaaggc                                                    17

<210> 1749
<211> 17
<212> DNA
<213> Homo sapiens

<400> 1749
gctggttgaa aaaggct                                                    17

<210> 1750
<211> 17
<212> DNA
<213> Homo sapiens

<400> 1750
ctggttgaaa aaggctg                                                    17

<210> 1751
<211> 17
<212> DNA
<213> Homo sapiens

<400> 1751
tggttgaaaa aggctga                                                    17

<210> 1752
<211> 17
<212> DNA
<213> Homo sapiens

<400> 1752
ggttgaaaaa ggctgac                                                    17

<210> 1753
<211> 17
<212> DNA
<213> Homo sapiens

<400> 1753
gttgaaaaag gctgacc                                                    17

<210> 1754
<211> 17
<212> DNA
<213> Homo sapiens

<400> 1754
ttgaaaaagg ctgaccc                                                    17

<210> 1755
<211> 17
<212> DNA
<213> Homo sapiens

<400> 1755
tgaaaaaggc tgaccca                                                    17

<210> 1756

<211> 17
<212> DNA
<213> Homo sapiens

<400> 1756
gaaaaaggct gacccaa                                                    17

<210> 1757
<211> 17
<212> DNA
<213> Homo sapiens

<400> 1757
aaaaaggctg acccaaa                                                    17

<210> 1758
<211> 17
<212> DNA
<213> Homo sapiens

<400> 1758
aaaaggctga cccaaaa                                                    17

<210> 1759
<211> 17
<212> DNA
<213> Homo sapiens

<400> 1759
aaaggctgac ccaaaat                                                    17

<210> 1760
<211> 17
<212> DNA
<213> Homo sapiens

<400> 1760
aaggctgacc caaaatg                                                    17

<210> 1761
<211> 17
<212> DNA
<213> Homo sapiens

<400> 1761
aggctgaccc aaaatgg                                                    17

<210> 1762
<211> 17
<212> DNA
<213> Homo sapiens

<400> 1762
ggctgaccca aaatggc                                                    17

<210> 1763
<211> 17
<212> DNA
<213> Homo sapiens

<400> 1763

gctgacccaa aatggcc                                                    17

<210> 1764
<211> 17
<212> DNA
<213> Homo sapiens

<400> 1764
ctgacccaaa atggccc                                                    17

<210> 1765
<211> 17
<212> DNA
<213> Homo sapiens

<400> 1765
tgacccaaaa tggccct                                                    17

<210> 1766
<211> 17
<212> DNA
<213> Homo sapiens

<400> 1766
gacccaaaat ggccctc                                                    17

<210> 1767
<211> 17
<212> DNA
<213> Homo sapiens

<400> 1767
acccaaaatg gccctca                                                    17

<210> 1768
<211> 17
<212> DNA
<213> Homo sapiens

<400> 1768
cccaaaatgg ccctcat                                                    17

<210> 1769
<211> 17
<212> DNA
<213> Homo sapiens

<400> 1769
ccaaaatggc cctcatt                                                    17

<210> 1770
<211> 17
<212> DNA
<213> Homo sapiens

<400> 1770
caaaatggcc ctcattt                                                    17

<210> 1771
<211> 17
<212> DNA

<213> Homo sapiens

<400> 1771
aaaatggccc tcattta                                                    17

<210> 1772
<211> 17
<212> DNA
<213> Homo sapiens

<400> 1772
aaatggccct catttaa                                                    17

<210> 1773
<211> 17
<212> DNA
<213> Homo sapiens

<400> 1773
aatggccctc atttaaa                                                    17

<210> 1774
<211> 17
<212> DNA
<213> Homo sapiens

<400> 1774
atggccctca tttaaaa                                                    17

<210> 1775
<211> 17
<212> DNA
<213> Homo sapiens

<400> 1775
tggccctcat ttaaaaa                                                    17

<210> 1776
<211> 17
<212> DNA
<213> Homo sapiens

<400> 1776
ggccctcatt taaaaag                                                    17

<210> 1777
<211> 17
<212> DNA
<213> Homo sapiens

<400> 1777
gccctcattt aaaaagt                                                    17

<210> 1778
<211> 17
<212> DNA
<213> Homo sapiens

<400> 1778
ccctcattta aaaagtt                                                    17

<210> 1779
<211> 17
<212> DNA
<213> Homo sapiens

<400> 1779
cctcatttaa aaagttc                                                    17

<210> 1780
<211> 17
<212> DNA
<213> Homo sapiens

<400> 1780
ctcatttaaa aagttct                                                    17

<210> 1781
<211> 17
<212> DNA
<213> Homo sapiens

<400> 1781
tcatttaaaa agttctg                                                    17

<210> 1782
<211> 17
<212> DNA
<213> Homo sapiens

<400> 1782
catttaaaaa gttctgc                                                    17

<210> 1783
<211> 17
<212> DNA
<213> Homo sapiens

<400> 1783
atttaaaaag ttctgct                                                    17

<210> 1784
<211> 17
<212> DNA
<213> Homo sapiens

<400> 1784
tttaaaaagt tctgctg                                                    17

<210> 1785
<211> 17
<212> DNA
<213> Homo sapiens

<400> 1785
ttaaaaagtt ctgctgt                                                    17

<210> 1786
<211> 17
<212> DNA
<213> Homo sapiens

<400> 1786
taaaaagttc tgctgtt                                                    17

<210> 1787
<211> 17
<212> DNA
<213> Homo sapiens

<400> 1787
aaaaagttct gctgttt                                                    17

<210> 1788
<211> 17
<212> DNA
<213> Homo sapiens

<400> 1788
aaaagttctg ctgtttc                                                    17

<210> 1789
<211> 17
<212> DNA
<213> Homo sapiens

<400> 1789
aaagttctgc tgtttcc                                                    17

<210> 1790
<211> 17
<212> DNA
<213> Homo sapiens

<400> 1790
aagttctgct gtttccc                                                    17

<210> 1791
<211> 17
<212> DNA
<213> Homo sapiens

<400> 1791
agttctgctg tttccca                                                    17

<210> 1792
<211> 17
<212> DNA
<213> Homo sapiens

<400> 1792
gttctgctgt ttcccat                                                    17

<210> 1793
<211> 17
<212> DNA
<213> Homo sapiens

<400> 1793
ttctgctgtt tcccatt                                                    17

<210> 1794
<211> 17

<212> DNA
<213> Homo sapiens

<400> 1794
tctgctgttt cccattt                                                    17

<210> 1795
<211> 17
<212> DNA
<213> Homo sapiens

<400> 1795
ctgctgtttc ccatttg                                                    17

<210> 1796
<211> 17
<212> DNA
<213> Homo sapiens

<400> 1796
tgctgtttcc catttgg                                                    17

<210> 1797
<211> 17
<212> DNA
<213> Homo sapiens

<400> 1797
gctgtttccc atttggt                                                    17

<210> 1798
<211> 17
<212> DNA
<213> Homo sapiens

<400> 1798
ctgtttccca tttggtt                                                    17

<210> 1799
<211> 17
<212> DNA
<213> Homo sapiens

<400> 1799
tgtttcccat ttggttc                                                    17

<210> 1800
<211> 17
<212> DNA
<213> Homo sapiens

<400> 1800
gtttcccatt tggttct                                                    17

<210> 1801
<211> 17
<212> DNA
<213> Homo sapiens

<400> 1801
tttcccattt ggttctg                                                    17

<210> 1802
<211> 17
<212> DNA
<213> Homo sapiens

<400> 1802
ttcccatttg gttctgt                                                    17

<210> 1803
<211> 17
<212> DNA
<213> Homo sapiens

<400> 1803
tcccatttgg ttctgtc                                                    17

<210> 1804
<211> 17
<212> DNA
<213> Homo sapiens

<400> 1804
cccatttggt tctgtcc                                                    17

<210> 1805
<211> 17
<212> DNA
<213> Homo sapiens

<400> 1805
ccatttggtt ctgtccc                                                    17

<210> 1806
<211> 17
<212> DNA
<213> Homo sapiens

<400> 1806
catttggttc tgtccca                                                    17

<210> 1807
<211> 17
<212> DNA
<213> Homo sapiens

<400> 1807
atttggttct gtcccag                                                    17

<210> 1808
<211> 17
<212> DNA
<213> Homo sapiens

<400> 1808
tttggttctg tcccaga                                                    17

<210> 1809
<211> 17
<212> DNA
<213> Homo sapiens

<400> 1809
ttggttctgt cccagat                                                    17

<210> 1810
<211> 17
<212> DNA
<213> Homo sapiens

<400> 1810
tggttctgtc ccagatg                                                    17

<210> 1811
<211> 17
<212> DNA
<213> Homo sapiens

<400> 1811
ggttctgtcc cagatga                                                    17

<210> 1812
<211> 17
<212> DNA
<213> Homo sapiens

<400> 1812
gttctgtccc agatgaa                                                    17

<210> 1813
<211> 17
<212> DNA
<213> Homo sapiens

<400> 1813
ttctgtccca gatgaac                                                    17

<210> 1814
<211> 17
<212> DNA
<213> Homo sapiens

<400> 1814
tctgtcccag atgaaca                                                    17

<210> 1815
<211> 17
<212> DNA
<213> Homo sapiens

<400> 1815
ctgtcccaga tgaacat                                                    17

<210> 1816
<211> 17
<212> DNA
<213> Homo sapiens

<400> 1816
tgtcccagat gaacatg                                                    17

<210> 1817

<211> 17
<212> DNA
<213> Homo sapiens

<400> 1817
gtcccagatg aacatgg                                                    17

<210> 1818
<211> 17
<212> DNA
<213> Homo sapiens

<400> 1818
tcccagatga acatgga                                                    17

<210> 1819
<211> 17
<212> DNA
<213> Homo sapiens

<400> 1819
cccagatgaa catggaa                                                    17

<210> 1820
<211> 17
<212> DNA
<213> Homo sapiens

<400> 1820
ccagatgaac atggaac                                                    17

<210> 1821
<211> 17
<212> DNA
<213> Homo sapiens

<400> 1821
cagatgaaca tggaact                                                    17

<210> 1822
<211> 17
<212> DNA
<213> Homo sapiens

<400> 1822
agatgaacat ggaactg                                                    17

<210> 1823
<211> 17
<212> DNA
<213> Homo sapiens

<400> 1823
gatgaacatg gaactga                                                    17

<210> 1824
<211> 17
<212> DNA
<213> Homo sapiens

<400> 1824

atgaacatgg aactgat 17

<210> 1825
<211> 17
<212> DNA
<213> Homo sapiens

<400> 1825
tgaacatgga actgata 17

<210> 1826
<211> 17
<212> DNA
<213> Homo sapiens

<400> 1826
gaacatggaa ctgatat 17

<210> 1827
<211> 17
<212> DNA
<213> Homo sapiens

<400> 1827
aacatggaac tgatatc 17

<210> 1828
<211> 17
<212> DNA
<213> Homo sapiens

<400> 1828
acatggaact gatatcc 17

<210> 1829
<211> 17
<212> DNA
<213> Homo sapiens

<400> 1829
catggaactg atatcca 17

<210> 1830
<211> 17
<212> DNA
<213> Homo sapiens

<400> 1830
atggaactga tatccat 17

<210> 1831
<211> 17
<212> DNA
<213> Homo sapiens

<400> 1831
tggaactgat atccatc 17

<210> 1832
<211> 17
<212> DNA

<213> Homo sapiens

<400> 1832
ggaactgata tccatcc                                                    17

<210> 1833
<211> 17
<212> DNA
<213> Homo sapiens

<400> 1833
gaactgatat ccatcca                                                    17

<210> 1834
<211> 17
<212> DNA
<213> Homo sapiens

<400> 1834
aactgatatc catccaa                                                    17

<210> 1835
<211> 17
<212> DNA
<213> Homo sapiens

<400> 1835
actgatatcc atccaat                                                    17

<210> 1836
<211> 17
<212> DNA
<213> Homo sapiens

<400> 1836
ctgatatcca tccaatg                                                    17

<210> 1837
<211> 17
<212> DNA
<213> Homo sapiens

<400> 1837
tgatatccat ccaatga                                                    17

<210> 1838
<211> 17
<212> DNA
<213> Homo sapiens

<400> 1838
gatatccatc caatgag                                                    17

<210> 1839
<211> 17
<212> DNA
<213> Homo sapiens

<400> 1839
atatccatcc aatgagt                                                    17

<210> 1840
<211> 17
<212> DNA
<213> Homo sapiens

<400> 1840
tatccatcca atgagtt                                                    17

<210> 1841
<211> 17
<212> DNA
<213> Homo sapiens

<400> 1841
atccatccaa tgagttt                                                    17

<210> 1842
<211> 17
<212> DNA
<213> Homo sapiens

<400> 1842
tccatccaat gagtttg                                                    17

<210> 1843
<211> 17
<212> DNA
<213> Homo sapiens

<400> 1843
ccatccaatg agtttgt                                                    17

<210> 1844
<211> 17
<212> DNA
<213> Homo sapiens

<400> 1844
catccaatga gtttgtt                                                    17

<210> 1845
<211> 17
<212> DNA
<213> Homo sapiens

<400> 1845
atccaatgag tttgttt                                                    17

<210> 1846
<211> 17
<212> DNA
<213> Homo sapiens

<400> 1846
tccaatgagt ttgtttt                                                    17

<210> 1847
<211> 17
<212> DNA
<213> Homo sapiens

<400> 1847
ccaatgagtt tgttttt                                                    17

<210> 1848
<211> 17
<212> DNA
<213> Homo sapiens

<400> 1848
caatgagttt gtttttt                                                    17

<210> 1849
<211> 17
<212> DNA
<213> Homo sapiens

<400> 1849
aatgagtttg tttttta                                                    17

<210> 1850
<211> 17
<212> DNA
<213> Homo sapiens

<400> 1850
atgagtttgt ttttttag                                                   17

<210> 1851
<211> 17
<212> DNA
<213> Homo sapiens

<400> 1851
tgagtttgtt ttttaga                                                    17

<210> 1852
<211> 17
<212> DNA
<213> Homo sapiens

<400> 1852
gagtttgttt tttagag                                                    17

<210> 1853
<211> 17
<212> DNA
<213> Homo sapiens

<400> 1853
agtttgtttt ttagaga                                                    17

<210> 1854
<211> 17
<212> DNA
<213> Homo sapiens

<400> 1854
gtttgttttt tagagac                                                    17

<210> 1855
<211> 17

<212> DNA
<213> Homo sapiens

<400> 1855
tttgtttttt agagact                                                      17

<210> 1856
<211> 17
<212> DNA
<213> Homo sapiens

<400> 1856
ttgtttttta gagacta                                                      17

<210> 1857
<211> 17
<212> DNA
<213> Homo sapiens

<400> 1857
tgttttttag agactat                                                      17

<210> 1858
<211> 17
<212> DNA
<213> Homo sapiens

<400> 1858
gttttttaga gactatt                                                      17

<210> 1859
<211> 17
<212> DNA
<213> Homo sapiens

<400> 1859
ttttttagag actattt                                                      17

<210> 1860
<211> 17
<212> DNA
<213> Homo sapiens

<400> 1860
tttttagaga ctatttt                                                      17

<210> 1861
<211> 17
<212> DNA
<213> Homo sapiens

<400> 1861
ttttagagac tattttg                                                      17

<210> 1862
<211> 17
<212> DNA
<213> Homo sapiens

<400> 1862
tttagagact attttgg                                                      17

<210> 1863
<211> 17
<212> DNA
<213> Homo sapiens

<400> 1863
ttagagacta ttttggc                                                    17

<210> 1864
<211> 17
<212> DNA
<213> Homo sapiens

<400> 1864
tagagactat tttggcc                                                    17

<210> 1865
<211> 17
<212> DNA
<213> Homo sapiens

<400> 1865
agagactatt ttggccc                                                    17

<210> 1866
<211> 17
<212> DNA
<213> Homo sapiens

<400> 1866
gagactattt tggcccc                                                    17

<210> 1867
<211> 17
<212> DNA
<213> Homo sapiens

<400> 1867
agactatttt ggcccct                                                    17

<210> 1868
<211> 17
<212> DNA
<213> Homo sapiens

<400> 1868
   gactattttg gcccctt                                                    17

<210> 1869
<211> 17
<212> DNA
<213> Homo sapiens

<400> 1869
   actattttgg ccccttt                                                    17

<210> 1870
<211> 17
<212> DNA
<213> Homo sapiens

<400> 1870
ctattttggc ccctttc                                                    17

<210> 1871
<211> 17
<212> DNA
<213> Homo sapiens

<400> 1871
tattttggcc cctttct                                                    17

<210> 1872
<211> 17
<212> DNA
<213> Homo sapiens

<400> 1872
attttggccc ctttctc                                                    17

<210> 1873
<211> 17
<212> DNA
<213> Homo sapiens

<400> 1873
ttttggcccc tttctca                                                    17

<210> 1874
<211> 17
<212> DNA
<213> Homo sapiens

<400> 1874
tttggcccct ttctcac                                                    17

<210> 1875
<211> 17
<212> DNA
<213> Homo sapiens

<400> 1875
ttggcccctt tctcaca                                                    17

<210> 1876
<211> 17
<212> DNA
<213> Homo sapiens

<400> 1876
tggccccttt ctcacaa                                                    17

<210> 1877
<211> 17
<212> DNA
<213> Homo sapiens

<400> 1877
ggcccctttc tcacaag                                                    17

<210> 1878

<211> 17
<212> DNA
<213> Homo sapiens

<400> 1878
gccccttct cacaagg                                                    17

<210> 1879
<211> 17
<212> DNA
<213> Homo sapiens

<400> 1879
cccctttctc acaagga                                                   17

<210> 1880
<211> 17
<212> DNA
<213> Homo sapiens

<400> 1880
ccctttctca caaggag                                                   17

<210> 1881
<211> 17
<212> DNA
<213> Homo sapiens

<400> 1881
cctttctcac aaggagt                                                   17

<210> 1882
<211> 17
<212> DNA
<213> Homo sapiens

<400> 1882
ctttctcaca aggagtg                                                   17

<210> 1883
<211> 17
<212> DNA
<213> Homo sapiens

<400> 1883
tttctcacaa ggagtga                                                   17

<210> 1884
<211> 17
<212> DNA
<213> Homo sapiens

<400> 1884
ttctcacaag gagtgag                                                   17

<210> 1885
<211> 17
<212> DNA
<213> Homo sapiens

<400> 1885

tctcacaagg agtgagt 17

<210> 1886
<211> 17
<212> DNA
<213> Homo sapiens

<400> 1886
ctcacaagga gtgagtc 17

<210> 1887
<211> 17
<212> DNA
<213> Homo sapiens

<400> 1887
tcacaaggag tgagtcc 17

<210> 1888
<211> 17
<212> DNA
<213> Homo sapiens

<400> 1888
cacaaggagt gagtcca 17

<210> 1889
<211> 17
<212> DNA
<213> Homo sapiens

<400> 1889
acaaggagtg agtccaa 17

<210> 1890
<211> 17
<212> DNA
<213> Homo sapiens

<400> 1890
caaggagtga gtccaag 17

<210> 1891
<211> 17
<212> DNA
<213> Homo sapiens

<400> 1891
aaggagtgag tccaagt 17

<210> 1892
<211> 17
<212> DNA
<213> Homo sapiens

<400> 1892
aggagtgagt ccaagta 17

<210> 1893
<211> 17
<212> DNA

<213> Homo sapiens

<400> 1893
ggagtgagtc caagtat                                                    17

<210> 1894
<211> 17
<212> DNA
<213> Homo sapiens

<400> 1894
gagtgagtcc aagtatt                                                    17

<210> 1895
<211> 17
<212> DNA
<213> Homo sapiens

<400> 1895
agtgagtcca agtattt                                                    17

<210> 1896
<211> 17
<212> DNA
<213> Homo sapiens

<400> 1896
gtgagtccaa gtatttt                                                    17

<210> 1897
<211> 17
<212> DNA
<213> Homo sapiens

<400> 1897
tgagtccaag tattttg                                                    17

<210> 1898
<211> 17
<212> DNA
<213> Homo sapiens

<400> 1898
gagtccaagt attttgt                                                    17

<210> 1899
<211> 17
<212> DNA
<213> Homo sapiens

<400> 1899
agtccaagta ttttgta                                                    17

<210> 1900
<211> 17
<212> DNA
<213> Homo sapiens

<400> 1900
gtccaagtat tttgtag                                                    17

<210> 1901
<211> 17
<212> DNA
<213> Homo sapiens

<400> 1901
tccaagtatt ttgtagt                                                    17

<210> 1902
<211> 17
<212> DNA
<213> Homo sapiens

<400> 1902
ccaagtattt tgtagtc                                                    17

<210> 1903
<211> 17
<212> DNA
<213> Homo sapiens

<400> 1903
caagtatttt gtagtct                                                    17

<210> 1904
<211> 17
<212> DNA
<213> Homo sapiens

<400> 1904
aagtattttg tagtctt                                                    17

<210> 1905
<211> 17
<212> DNA
<213> Homo sapiens

<400> 1905
agtattttgt agtcttt                                                    17

<210> 1906
<211> 17
<212> DNA
<213> Homo sapiens

<400> 1906
gtattttgta gtcttta                                                    17

<210> 1907
<211> 17
<212> DNA
<213> Homo sapiens

<400> 1907
tattttgtag tctttat                                                    17

<210> 1908
<211> 17
<212> DNA
<213> Homo sapiens

<400> 1908
   attttgtagt ctttata                                                    17

<210> 1909
<211> 17
<212> DNA
<213> Homo sapiens

<400> 1909
ttttgtagtc tttatat                                                   17

<210> 1910
<211> 17
<212> DNA
<213> Homo sapiens

<400> 1910
tttgtagtct ttatata                                                   17

<210> 1911
<211> 17
<212> DNA
<213> Homo sapiens

<400> 1911
ttgtagtctt tatatat                                                   17

<210> 1912
<211> 17
<212> DNA
<213> Homo sapiens

<400> 1912
tgtagtcttt atatatg                                                   17

<210> 1913
<211> 17
<212> DNA
<213> Homo sapiens

<400> 1913
gtagtcttta tatatgt                                                   17

<210> 1914
<211> 17
<212> DNA
<213> Homo sapiens

<400> 1914
tagtctttat atatgtt                                                   17

<210> 1915
<211> 17
<212> DNA
<213> Homo sapiens

<400> 1915
agtctttata tatgttt                                                   17

<210> 1916
<211> 17

<212> DNA
<213> Homo sapiens

<400> 1916
gtctttatat atgtttt                                                    17

<210> 1917
<211> 17
<212> DNA
<213> Homo sapiens

<400> 1917
tctttatata tgttttg                                                    17

<210> 1918
<211> 17
<212> DNA
<213> Homo sapiens

<400> 1918
ctttatatat gttttgt                                                    17

<210> 1919
<211> 17
<212> DNA
<213> Homo sapiens

<400> 1919
tttatatatg ttttgta                                                    17

<210> 1920
<211> 17
<212> DNA
<213> Homo sapiens

<400> 1920
ttatatatgt tttgtac                                                    17

<210> 1921
<211> 17
<212> DNA
<213> Homo sapiens

<400> 1921
tatatatgtt ttgtaca                                                    17

<210> 1922
<211> 17
<212> DNA
<213> Homo sapiens

<400> 1922
atatatgttt tgtacat                                                    17

<210> 1923
<211> 17
<212> DNA
<213> Homo sapiens

<400> 1923
tatatgtttt gtacatc                                                    17

<210> 1924
<211> 17
<212> DNA
<213> Homo sapiens

<400> 1924
atatgttttg tacatca                                                    17

<210> 1925
<211> 17
<212> DNA
<213> Homo sapiens

<400> 1925
tatgttttgt acatcat                                                    17

<210> 1926
<211> 17
<212> DNA
<213> Homo sapiens

<400> 1926
atgttttgta catcata                                                    17

<210> 1927
<211> 17
<212> DNA
<213> Homo sapiens

<400> 1927
tgttttgtac atcataa                                                    17

<210> 1928
<211> 17
<212> DNA
<213> Homo sapiens

<400> 1928
gttttgtaca tcataag                                                    17

<210> 1929
<211> 17
<212> DNA
<213> Homo sapiens

<400> 1929
ttttgtacat cataagc                                                    17

<210> 1930
<211> 17
<212> DNA
<213> Homo sapiens

<400> 1930
tttgtacatc ataagca                                                    17

<210> 1931
<211> 17
<212> DNA
<213> Homo sapiens

<400> 1931
ttgtacatca taagcag                                                    17

<210> 1932
<211> 17
<212> DNA
<213> Homo sapiens

<400> 1932
tgtacatcat aagcagt                                                    17

<210> 1933
<211> 17
<212> DNA
<213> Homo sapiens

<400> 1933
gtacatcata agcagta                                                    17

<210> 1934
<211> 17
<212> DNA
<213> Homo sapiens

<400> 1934
tacatcataa gcagtat                                                    17

<210> 1935
<211> 17
<212> DNA
<213> Homo sapiens

<400> 1935
acatcataag cagtata                                                    17

<210> 1936
<211> 17
<212> DNA
<213> Homo sapiens

<400> 1936
catcataagc agtatat                                                    17

<210> 1937
<211> 17
<212> DNA
<213> Homo sapiens

<400> 1937
atcataagca gtatata                                                    17

<210> 1938
<211> 17
<212> DNA
<213> Homo sapiens

<400> 1938
tcataagcag tatatat                                                    17

<210> 1939

<211> 17
<212> DNA
<213> Homo sapiens

<400> 1939
cataagcagt atatatg                                                    17

<210> 1940
<211> 17
<212> DNA
<213> Homo sapiens

<400> 1940
ataagcagta tatatgg                                                    17

<210> 1941
<211> 17
<212> DNA
<213> Homo sapiens

<400> 1941
taagcagtat atatggg                                                    17

<210> 1942
<211> 17
<212> DNA
<213> Homo sapiens

<400> 1942
aagcagtata tatgggt                                                    17

<210> 1943
<211> 17
<212> DNA
<213> Homo sapiens

<400> 1943
agcagtatat atgggtg                                                    17

<210> 1944
<211> 17
<212> DNA
<213> Homo sapiens

<400> 1944
gcagtatata tgggtgt                                                    17

<210> 1945
<211> 17
<212> DNA
<213> Homo sapiens

<400> 1945
cagtatatat gggtgtt                                                    17

<210> 1946
<211> 17
<212> DNA
<213> Homo sapiens

<400> 1946

agtatatatg ggtgttt                                                    17

<210> 1947
<211> 17
<212> DNA
<213> Homo sapiens

<400> 1947
gtatatatgg gtgtttc                                                    17

<210> 1948
<211> 17
<212> DNA
<213> Homo sapiens

<400> 1948
tatatatggg tgtttcc                                                    17

<210> 1949
<211> 17
<212> DNA
<213> Homo sapiens

<400> 1949
atatatgggt gtttcca                                                    17

<210> 1950
<211> 17
<212> DNA
<213> Homo sapiens

<400> 1950
tatatgggtg tttccat                                                    17

<210> 1951
<211> 17
<212> DNA
<213> Homo sapiens

<400> 1951
atatgggtgt ttccatg                                                    17

<210> 1952
<211> 17
<212> DNA
<213> Homo sapiens

<400> 1952
tatgggtgtt tccatgt                                                    17

<210> 1953
<211> 17
<212> DNA
<213> Homo sapiens

<400> 1953
atgggtgttt ccatgtg                                                    17

<210> 1954
<211> 17
<212> DNA

<213> Homo sapiens

<400> 1954
tgggtgtttc catgtgc                                                    17

<210> 1955
<211> 17
<212> DNA
<213> Homo sapiens

<400> 1955
gggtgtttcc atgtgca                                                    17

<210> 1956
<211> 17
<212> DNA
<213> Homo sapiens

<400> 1956
ggtgtttcca tgtgcag                                                    17

<210> 1957
<211> 17
<212> DNA
<213> Homo sapiens

<400> 1957
gtgtttccat gtgcagg                                                    17

<210> 1958
<211> 17
<212> DNA
<213> Homo sapiens

<400> 1958
tgtttccatg tgcagga                                                    17

<210> 1959
<211> 17
<212> DNA
<213> Homo sapiens

<400> 1959
gtttccatgt gcaggaa                                                    17

<210> 1960
<211> 17
<212> DNA
<213> Homo sapiens

<400> 1960
tttccatgtg caggaag                                                    17

<210> 1961
<211> 17
<212> DNA
<213> Homo sapiens

<400> 1961
ttccatgtgc aggaagg                                                    17

<210> 1962
<211> 17
<212> DNA
<213> Homo sapiens

<400> 1962
tccatgtgca ggaaggt                                                    17

<210> 1963
<211> 17
<212> DNA
<213> Homo sapiens

<400> 1963
ccatgtgcag gaaggtt                                                    17

<210> 1964
<211> 17
<212> DNA
<213> Homo sapiens

<400> 1964
catgtgcagg aaggttt                                                    17

<210> 1965
<211> 17
<212> DNA
<213> Homo sapiens

<400> 1965
atgtgcagga aggttta                                                    17

<210> 1966
<211> 17
<212> DNA
<213> Homo sapiens

<400> 1966
tgtgcaggaa ggtttag                                                    17

<210> 1967
<211> 17
<212> DNA
<213> Homo sapiens

<400> 1967
gtgcaggaag gtttaga                                                    17

<210> 1968
<211> 17
<212> DNA
<213> Homo sapiens

<400> 1968
tgcaggaagg tttagac                                                    17

<210> 1969
<211> 17
<212> DNA
<213> Homo sapiens

<400> 1969
gcaggaaggt ttagacc                                                    17

<210> 1970
<211> 17
<212> DNA
<213> Homo sapiens

<400> 1970
caggaaggtt tagacct                                                    17

<210> 1971
<211> 17
<212> DNA
<213> Homo sapiens

<400> 1971
aggaaggttt agacctt                                                    17

<210> 1972
<211> 17
<212> DNA
<213> Homo sapiens

<400> 1972
ggaaggttta gaccttc                                                    17

<210> 1973
<211> 17
<212> DNA
<213> Homo sapiens

<400> 1973
gaaggtttag accttcg                                                    17

<210> 1974
<211> 17
<212> DNA
<213> Homo sapiens

<400> 1974
aaggtttaga ccttcga                                                    17

<210> 1975
<211> 17
<212> DNA
<213> Homo sapiens

<400> 1975
aggtttagac cttcgaa                                                    17

<210> 1976
<211> 17
<212> DNA
<213> Homo sapiens

<400> 1976
ggtttagacc ttcgaaa                                                    17

<210> 1977
<211> 17

<212> DNA
<213> Homo sapiens

<400> 1977
gtttagacct tcgaaat                                                    17

<210> 1978
<211> 17
<212> DNA
<213> Homo sapiens

<400> 1978
tttagacctt cgaaatc                                                    17

<210> 1979
<211> 17
<212> DNA
<213> Homo sapiens

<400> 1979
ttagaccttc gaaatct                                                    17

<210> 1980
<211> 17
<212> DNA
<213> Homo sapiens

<400> 1980
tagaccttcg aaatctg                                                    17

<210> 1981
<211> 17
<212> DNA
<213> Homo sapiens

<400> 1981
agaccttcga aatctgg                                                    17

<210> 1982
<211> 17
<212> DNA
<213> Homo sapiens

<400> 1982
gaccttcgaa atctggc                                                    17

<210> 1983
<211> 17
<212> DNA
<213> Homo sapiens

<400> 1983
accttcgaaa tctggca                                                    17

<210> 1984
<211> 17
<212> DNA
<213> Homo sapiens

<400> 1984
ccttcgaaat ctggcaa                                                    17

<210> 1985
<211> 17
<212> DNA
<213> Homo sapiens

<400> 1985
cttcgaaatc tggcaag                                                    17

<210> 1986
<211> 17
<212> DNA
<213> Homo sapiens

<400> 1986
ttcgaaatct ggcaagt                                                    17

<210> 1987
<211> 17
<212> DNA
<213> Homo sapiens

<400> 1987
tcgaaatctg gcaagtg                                                    17

<210> 1988
<211> 17
<212> DNA
<213> Homo sapiens

<400> 1988
cgaaatctgg caagtga                                                    17

<210> 1989
<211> 17
<212> DNA
<213> Homo sapiens

<400> 1989
gaaatctggc aagtgac                                                    17

<210> 1990
<211> 17
<212> DNA
<213> Homo sapiens

<400> 1990
aaatctggca agtgacg                                                    17

<210> 1991
<211> 17
<212> DNA
<213> Homo sapiens

<400> 1991
aatctggcaa gtgacga                                                    17

<210> 1992
<211> 17
<212> DNA
<213> Homo sapiens

<400> 1992
atctggcaag tgacgat                                                    17

<210> 1993
<211> 17
<212> DNA
<213> Homo sapiens

<400> 1993
tctggcaagt gacgatt                                                    17

<210> 1994
<211> 17
<212> DNA
<213> Homo sapiens

<400> 1994
ctggcaagtg acgattc                                                    17

<210> 1995
<211> 17
<212> DNA
<213> Homo sapiens

<400> 1995
tggcaagtga cgattcc                                                    17

<210> 1996
<211> 17
<212> DNA
<213> Homo sapiens

<400> 1996
ggcaagtgac gattcct                                                    17

<210> 1997
<211> 17
<212> DNA
<213> Homo sapiens

<400> 1997
gcaagtgacg attccta                                                    17

<210> 1998
<211> 17
<212> DNA
<213> Homo sapiens

<400> 1998
caagtgacga ttcctac                                                    17

<210> 1999
<211> 17
<212> DNA
<213> Homo sapiens

<400> 1999
aagtgacgat tcctaca                                                    17

<210> 2000

<211> 17
<212> DNA
<213> Homo sapiens

<400> 2000
agtgacgatt cctacat                                                    17

<210> 2001
<211> 17
<212> DNA
<213> Homo sapiens

<400> 2001
gtgacgattc ctacatc                                                    17

<210> 2002
<211> 17
<212> DNA
<213> Homo sapiens

<400> 2002
tgacgattcc tacatca                                                    17

<210> 2003
<211> 17
<212> DNA
<213> Homo sapiens

<400> 2003
gacgattcct acatcac                                                    17

<210> 2004
<211> 17
<212> DNA
<213> Homo sapiens

<400> 2004
acgattccta catcaca                                                    17

<210> 2005
<211> 17
<212> DNA
<213> Homo sapiens

<400> 2005
cgattcctac atcacac                                                    17

<210> 2006
<211> 17
<212> DNA
<213> Homo sapiens

<400> 2006
gattcctaca tcacacc                                                    17

<210> 2007
<211> 17
<212> DNA
<213> Homo sapiens

<400> 2007

attcctacat cacacca                                                    17

<210> 2008
<211> 17
<212> DNA
<213> Homo sapiens

<400> 2008
ttcctacatc acaccat                                                    17

<210> 2009
<211> 17
<212> DNA
<213> Homo sapiens

<400> 2009
tcctacatca caccata                                                    17

<210> 2010
<211> 17
<212> DNA
<213> Homo sapiens

<400> 2010
cctacatcac accatat                                                    17

<210> 2011
<211> 17
<212> DNA
<213> Homo sapiens

<400> 2011
ctacatcaca ccatatt                                                    17

<210> 2012
<211> 17
<212> DNA
<213> Homo sapiens

<400> 2012
tacatcacac catattt                                                    17

<210> 2013
<211> 17
<212> DNA
<213> Homo sapiens

<400> 2013
acatcacacc atatttt                                                    17

<210> 2014
<211> 17
<212> DNA
<213> Homo sapiens

<400> 2014
catcacacca tatttta                                                    17

<210> 2015
<211> 17
<212> DNA

<213> Homo sapiens

<400> 2015
atcacaccat attttaa                                                    17

<210> 2016
<211> 17
<212> DNA
<213> Homo sapiens

<400> 2016
tcacaccata ttttaac                                                    17

<210> 2017
<211> 17
<212> DNA
<213> Homo sapiens

<400> 2017
cacaccatat tttaacg                                                    17

<210> 2018
<211> 17
<212> DNA
<213> Homo sapiens

<400> 2018
acaccatatt ttaacgt                                                    17

<210> 2019
<211> 17
<212> DNA
<213> Homo sapiens

<400> 2019
caccatattt taacgta                                                    17

<210> 2020
<211> 17
<212> DNA
<213> Homo sapiens

<400> 2020
accatatttt aacgtag                                                    17

<210> 2021
<211> 17
<212> DNA
<213> Homo sapiens

<400> 2021
ccatatttta acgtaga                                                    17

<210> 2022
<211> 17
<212> DNA
<213> Homo sapiens

<400> 2022
catattttaa cgtagag                                                    17

<210> 2023
<211> 17
<212> DNA
<213> Homo sapiens

<400> 2023
atattttaac gtagagg                                                    17

<210> 2024
<211> 17
<212> DNA
<213> Homo sapiens

<400> 2024
tattttaacg tagagga                                                    17

<210> 2025
<211> 17
<212> DNA
<213> Homo sapiens

<400> 2025
attttaacgt agaggag                                                    17

<210> 2026
<211> 17
<212> DNA
<213> Homo sapiens

<400> 2026
ttttaacgta gaggaga                                                    17

<210> 2027
<211> 17
<212> DNA
<213> Homo sapiens

<400> 2027
tttaacgtag aggagaa                                                    17

<210> 2028
<211> 17
<212> DNA
<213> Homo sapiens

<400> 2028
ttaacgtaga ggagaat                                                    17

<210> 2029
<211> 25
<212> DNA
<213> Homo sapiens

<400> 2029
caggaaaccg tctggtggga tctcc                                           25

<210> 2030
<211> 25
<212> DNA
<213> Homo sapiens

<400> 2030
aggaaaccgt ctggtgggat ctccg                                          25

<210> 2031
<211> 25
<212> DNA
<213> Homo sapiens

<400> 2031
ggaaaccgtc tggtgggatc tccgc                                          25

<210> 2032
<211> 25
<212> DNA
<213> Homo sapiens

<400> 2032
gaaaccgtct ggtgggatct ccgca                                          25

<210> 2033
<211> 25
<212> DNA
<213> Homo sapiens

<400> 2033
aaaccgtctg gtgggatctc cgcag                                          25

<210> 2034
<211> 25
<212> DNA
<213> Homo sapiens

<400> 2034
aaccgtctgg tgggatctcc gcagc                                          25

<210> 2035
<211> 25
<212> DNA
<213> Homo sapiens

<400> 2035
accgtctggt gggatctccg cagct                                          25

<210> 2036
<211> 25
<212> DNA
<213> Homo sapiens

<400> 2036
ccgtctggtg ggatctccgc agctg                                          25

<210> 2037
<211> 25
<212> DNA
<213> Homo sapiens

<400> 2037
cgtctggtgg gatctccgca gctgc                                          25

<210> 2038
<211> 25

<212> DNA
<213> Homo sapiens

<400> 2038
gtctggtggg atctccgcag ctgct                                          25

<210> 2039
<211> 25
<212> DNA
<213> Homo sapiens

<400> 2039
tctggtggga tctccgcagc tgctt                                          25

<210> 2040
<211> 25
<212> DNA
<213> Homo sapiens

<400> 2040
ctggtgggat ctccgcagct gcttt                                          25

<210> 2041
<211> 25
<212> DNA
<213> Homo sapiens

<400> 2041
tggtgggatc tccgcagctg ctttt                                          25

<210> 2042
<211> 25
<212> DNA
<213> Homo sapiens

<400> 2042
ggtgggatct ccgcagctgc ttttc                                          25

<210> 2043
<211> 25
<212> DNA
<213> Homo sapiens

<400> 2043
gtgggatctc cgcagctgct tttca                                          25

<210> 2044
<211> 25
<212> DNA
<213> Homo sapiens

<400> 2044
tgggatctcc gcagctgctt ttcac                                          25

<210> 2045
<211> 25
<212> DNA
<213> Homo sapiens

<400> 2045
gggatctccg cagctgcttt tcacc                                          25

<210> 2046
<211> 25
<212> DNA
<213> Homo sapiens

<400> 2046
ggatctccgc agctgctttt cacct                                          25

<210> 2047
<211> 25
<212> DNA
<213> Homo sapiens

<400> 2047
gatctccgca gctgcttttc acctg                                          25

<210> 2048
<211> 25
<212> DNA
<213> Homo sapiens

<400> 2048
atctccgcag ctgcttttca cctgc                                          25

<210> 2049
<211> 25
<212> DNA
<213> Homo sapiens

<400> 2049
tctccgcagc tgcttttcac ctgct                                          25

<210> 2050
<211> 25
<212> DNA
<213> Homo sapiens

<400> 2050
ctccgcagct gcttttcacc tgctg                                          25

<210> 2051
<211> 25
<212> DNA
<213> Homo sapiens

<400> 2051
tccgcagctg cttttcacct gctgt                                          25

<210> 2052
<211> 25
<212> DNA
<213> Homo sapiens

<400> 2052
ccgcagctgc ttttcacctg ctgtt                                          25

<210> 2053
<211> 25
<212> DNA
<213> Homo sapiens

<400> 2053
cgcagctgct tttcacctgc tgttc                                          25

<210> 2054
<211> 25
<212> DNA
<213> Homo sapiens

<400> 2054
gcagctgctt ttcacctgct gttcc                                          25

<210> 2055
<211> 25
<212> DNA
<213> Homo sapiens

<400> 2055
cagctgcttt tcacctgctg ttcct                                          25

<210> 2056
<211> 25
<212> DNA
<213> Homo sapiens

<400> 2056
agctgctttt cacctgctgt tcctc                                          25

<210> 2057
<211> 25
<212> DNA
<213> Homo sapiens

<400> 2057
gctgcttttc acctgctgtt cctcc                                          25

<210> 2058
<211> 25
<212> DNA
<213> Homo sapiens

<400> 2058
ctgcttttca cctgctgttc ctcct                                          25

<210> 2059
<211> 25
<212> DNA
<213> Homo sapiens

<400> 2059
tgcttttcac ctgctgttcc tcctg                                          25

<210> 2060
<211> 25
<212> DNA
<213> Homo sapiens

<400> 2060
gcttttcacc tgctgttcct cctgc                                          25

<210> 2061

<211> 25
<212> DNA
<213> Homo sapiens

<400> 2061
cttttcacct gctgttcctc ctgcg                                            25

<210> 2062
<211> 25
<212> DNA
<213> Homo sapiens

<400> 2062
ttttcacctg ctgttcctcc tgcgc                                            25

<210> 2063
<211> 25
<212> DNA
<213> Homo sapiens

<400> 2063
tttcacctgc tgttcctcct gcgct                                            25

<210> 2064
<211> 25
<212> DNA
<213> Homo sapiens

<400> 2064
ttcacctgct gttcctcctg cgctt                                            25

<210> 2065
<211> 25
<212> DNA
<213> Homo sapiens

<400> 2065
tcacctgctg ttcctcctgc gcttc                                            25

<210> 2066
<211> 25
<212> DNA
<213> Homo sapiens

<400> 2066
cacctgctgt tcctcctgcg cttcc                                            25

<210> 2067
<211> 25
<212> DNA
<213> Homo sapiens

<400> 2067
acctgctgtt cctcctgcgc ttcct                                            25

<210> 2068
<211> 25
<212> DNA
<213> Homo sapiens

<400> 2068

cctgctgttc ctcctgcgct tccta 25

<210> 2069
<211> 25
<212> DNA
<213> Homo sapiens

<400> 2069
ctgctgttcc tcctgcgctt cctaa 25

<210> 2070
<211> 25
<212> DNA
<213> Homo sapiens

<400> 2070
tgctgttcct cctgcgcttc ctaag 25

<210> 2071
<211> 25
<212> DNA
<213> Homo sapiens

<400> 2071
gctgttcctc ctgcgcttcc taaga 25

<210> 2072
<211> 25
<212> DNA
<213> Homo sapiens

<400> 2072
ctgttcctcc tgcgcttcct aagag 25

<210> 2073
<211> 25
<212> DNA
<213> Homo sapiens

<400> 2073
tgttcctcct gcgcttccta agagg 25

<210> 2074
<211> 25
<212> DNA
<213> Homo sapiens

<400> 2074
gttcctcctg cgcttcctaa gagga 25

<210> 2075
<211> 25
<212> DNA
<213> Homo sapiens

<400> 2075
ttcctcctgc gcttcctaag aggaa 25

<210> 2076
<211> 25
<212> DNA

<213> Homo sapiens

<400> 2076
tcctcctgcg cttcctaaga ggaag                                          25

<210> 2077
<211> 25
<212> DNA
<213> Homo sapiens

<400> 2077
cctcctgcgc ttcctaagag gaaga                                          25

<210> 2078
<211> 25
<212> DNA
<213> Homo sapiens

<400> 2078
ctcctgcgct tcctaagagg aagaa                                          25

<210> 2079
<211> 25
<212> DNA
<213> Homo sapiens

<400> 2079
tcctgcgctt cctaagagga agaat                                          25

<210> 2080
<211> 25
<212> DNA
<213> Homo sapiens

<400> 2080
cctgcgcttc ctaagaggaa gaatc                                          25

<210> 2081
<211> 25
<212> DNA
<213> Homo sapiens

<400> 2081
ctgcgcttcc taagaggaag aatca                                          25

<210> 2082
<211> 25
<212> DNA
<213> Homo sapiens

<400> 2082
tgcgcttcct aagaggaaga atcaa                                          25

<210> 2083
<211> 25
<212> DNA
<213> Homo sapiens

<400> 2083
gcgcttccta agaggaagaa tcaat                                          25

<210> 2084
<211> 25
<212> DNA
<213> Homo sapiens

<400> 2084
cgcttcctaa gaggaagaat caatg                                          25

<210> 2085
<211> 25
<212> DNA
<213> Homo sapiens

<400> 2085
gcttcctaag aggaagaatc aatgc                                          25

<210> 2086
<211> 25
<212> DNA
<213> Homo sapiens

<400> 2086
cttcctaaga ggaagaatca atgcc                                          25

<210> 2087
<211> 25
<212> DNA
<213> Homo sapiens

<400> 2087
ttcctaagag gaagaatcaa tgccg                                          25

<210> 2088
<211> 25
<212> DNA
<213> Homo sapiens

<400> 2088
tcctaagagg aagaatcaat gccgt                                          25

<210> 2089
<211> 25
<212> DNA
<213> Homo sapiens

<400> 2089
cctaagagga agaatcaatg ccgtg                                          25

<210> 2090
<211> 25
<212> DNA
<213> Homo sapiens

<400> 2090
ctaagaggaa gaatcaatgc cgtgg                                          25

<210> 2091
<211> 25
<212> DNA
<213> Homo sapiens

<400> 2091
taagaggaag aatcaatgcc gtggg                                          25

<210> 2092
<211> 25
<212> DNA
<213> Homo sapiens

<400> 2092
aagaggaaga atcaatgccg tgggt                                          25

<210> 2093
<211> 25
<212> DNA
<213> Homo sapiens

<400> 2093
agaggaagaa tcaatgccgt gggtg                                          25

<210> 2094
<211> 25
<212> DNA
<213> Homo sapiens

<400> 2094
gaggaagaat caatgccgtg ggtgg                                          25

<210> 2095
<211> 25
<212> DNA
<213> Homo sapiens

<400> 2095
aggaagaatc aatgccgtgg gtgga                                          25

<210> 2096
<211> 25
<212> DNA
<213> Homo sapiens

<400> 2096
ggaagaatca atgccgtggg tggag                                          25

<210> 2097
<211> 25
<212> DNA
<213> Homo sapiens

<400> 2097
gaagaatcaa tgccgtgggt ggagc                                          25

<210> 2098
<211> 25
<212> DNA
<213> Homo sapiens

<400> 2098
aagaatcaat gccgtgggtg gagcc                                          25

<210> 2099
<211> 25

<212> DNA
<213> Homo sapiens

<400> 2099
agaatcaatg ccgtgggtgg agccc                                          25

<210> 2100
<211> 25
<212> DNA
<213> Homo sapiens

<400> 2100
gaatcaatgc cgtgggtgga gccca                                          25

<210> 2101
<211> 25
<212> DNA
<213> Homo sapiens

<400> 2101
aatcaatgcc gtgggtggag cccaa                                          25

<210> 2102
<211> 25
<212> DNA
<213> Homo sapiens

<400> 2102
atcaatgccg tgggtggagc ccaag                                          25

<210> 2103
<211> 25
<212> DNA
<213> Homo sapiens

<400> 2103
tcaatgccgt gggtggagcc caagc                                          25

<210> 2104
<211> 25
<212> DNA
<213> Homo sapiens

<400> 2104
caatgccgtg ggtggagccc aagcc                                          25

<210> 2105
<211> 25
<212> DNA
<213> Homo sapiens

<400> 2105
aatgccgtgg gtggagccca agccc                                          25

<210> 2106
<211> 25
<212> DNA
<213> Homo sapiens

<400> 2106
atgccgtggg tggagcccaa gccca                                          25

<210> 2107
<211> 25
<212> DNA
<213> Homo sapiens

<400> 2107
tgccgtgggt ggagcccaag cccag                                          25

<210> 2108
<211> 25
<212> DNA
<213> Homo sapiens

<400> 2108
gccgtgggtg gagcccaagc ccagg                                          25

<210> 2109
<211> 25
<212> DNA
<213> Homo sapiens

<400> 2109
ccgtgggtgg agcccaagcc caggc                                          25

<210> 2110
<211> 25
<212> DNA
<213> Homo sapiens

<400> 2110
cgtgggtgga gcccaagccc aggcc                                          25

<210> 2111
<211> 25
<212> DNA
<213> Homo sapiens

<400> 2111
gtgggtggag cccaagccca ggccg                                          25

<210> 2112
<211> 25
<212> DNA
<213> Homo sapiens

<400> 2112
tgggtggagc ccaagcccag gccgg                                          25

<210> 2113
<211> 25
<212> DNA
<213> Homo sapiens

<400> 2113
gggtggagcc caagcccagg ccggg                                          25

<210> 2114
<211> 25
<212> DNA
<213> Homo sapiens

<400> 2114
ggtggagccc aagcccaggc cgggg                                          25

<210> 2115
<211> 25
<212> DNA
<213> Homo sapiens

<400> 2115
gtggagccca agcccaggcc ggggc                                          25

<210> 2116
<211> 25
<212> DNA
<213> Homo sapiens

<400> 2116
tggagcccaa gcccaggccg gggcc                                          25

<210> 2117
<211> 25
<212> DNA
<213> Homo sapiens

<400> 2117
ggagcccaag cccaggccgg ggccg                                          25

<210> 2118
<211> 25
<212> DNA
<213> Homo sapiens

<400> 2118
gagcccaagc ccaggccggg gccgg                                          25

<210> 2119
<211> 25
<212> DNA
<213> Homo sapiens

<400> 2119
agcccaagcc caggccgggg ccgga                                          25

<210> 2120
<211> 25
<212> DNA
<213> Homo sapiens

<400> 2120
gcccaagccc aggccggggc cggag                                          25

<210> 2121
<211> 25
<212> DNA
<213> Homo sapiens

<400> 2121
cccaagccca ggccggggcc ggagc                                          25

<210> 2122

<211> 25
<212> DNA
<213> Homo sapiens

<400> 2122
ccaagcccag gccggggccg gagca                                            25

<210> 2123
<211> 25
<212> DNA
<213> Homo sapiens

<400> 2123
caagcccagg ccggggccgg agcag                                            25

<210> 2124
<211> 25
<212> DNA
<213> Homo sapiens

<400> 2124
aagcccaggc cggggccgga gcaga                                            25

<210> 2125
<211> 25
<212> DNA
<213> Homo sapiens

<400> 2125
agcccaggcc ggggccggag cagaa                                            25

<210> 2126
<211> 25
<212> DNA
<213> Homo sapiens

<400> 2126
gcccaggccg gggccggagc agaag                                            25

<210> 2127
<211> 25
<212> DNA
<213> Homo sapiens

<400> 2127
cccaggccgg ggccggagca gaagc                                            25

<210> 2128
<211> 25
<212> DNA
<213> Homo sapiens

<400> 2128
ccaggccggg gccggagcag aagcc                                            25

<210> 2129
<211> 25
<212> DNA
<213> Homo sapiens

<400> 2129

caggccgggg ccggagcaga agccc 25

<210> 2130
<211> 25
<212> DNA
<213> Homo sapiens

<400> 2130
aggccggggc cggagcagaa gccca 25

<210> 2131
<211> 25
<212> DNA
<213> Homo sapiens

<400> 2131
ggccggggcc ggagcagaag cccaa 25

<210> 2132
<211> 25
<212> DNA
<213> Homo sapiens

<400> 2132
gccggggccg gagcagaagc ccaag 25

<210> 2133
<211> 25
<212> DNA
<213> Homo sapiens

<400> 2133
ccggggccgg agcagaagcc caagc 25

<210> 2134
<211> 25
<212> DNA
<213> Homo sapiens

<400> 2134
cggggccgga gcagaagccc aagct 25

<210> 2135
<211> 25
<212> DNA
<213> Homo sapiens

<400> 2135
ggggccggag cagaagccca agctc 25

<210> 2136
<211> 25
<212> DNA
<213> Homo sapiens

<400> 2136
gggccggagc agaagcccaa gctca 25

<210> 2137
<211> 25
<212> DNA

<213> Homo sapiens

<400> 2137
ggccggagca gaagcccaag ctcac                                          25

<210> 2138
<211> 25
<212> DNA
<213> Homo sapiens

<400> 2138
gccggagcag aagcccaagc tcacc                                          25

<210> 2139
<211> 25
<212> DNA
<213> Homo sapiens

<400> 2139
ccggagcaga agcccaagct cacca                                          25

<210> 2140
<211> 25
<212> DNA
<213> Homo sapiens

<400> 2140
cggagcagaa gcccaagctc accaa                                          25

<210> 2141
<211> 25
<212> DNA
<213> Homo sapiens

<400> 2141
ggagcagaag cccaagctca ccaaa                                          25

<210> 2142
<211> 25
<212> DNA
<213> Homo sapiens

<400> 2142
gagcagaagc ccaagctcac caaac                                          25

<210> 2143
<211> 25
<212> DNA
<213> Homo sapiens

<400> 2143
agcagaagcc caagctcacc aaacc                                          25

<210> 2144
<211> 25
<212> DNA
<213> Homo sapiens

<400> 2144
gcagaagccc aagctcacca aaccg                                          25

<210> 2145
<211> 25
<212> DNA
<213> Homo sapiens

<400> 2145
cagaagccca agctcaccaa accgg                                          25

<210> 2146
<211> 25
<212> DNA
<213> Homo sapiens

<400> 2146
agaagcccaa gctcaccaaa ccgga                                          25

<210> 2147
<211> 25
<212> DNA
<213> Homo sapiens

<400> 2147
gaagcccaag ctcaccaaac cggac                                          25

<210> 2148
<211> 25
<212> DNA
<213> Homo sapiens

<400> 2148
aagcccaagc tcaccaaacc ggact                                          25

<210> 2149
<211> 25
<212> DNA
<213> Homo sapiens

<400> 2149
agcccaagct caccaaaccg gactc                                          25

<210> 2150
<211> 25
<212> DNA
<213> Homo sapiens

<400> 2150
gcccaagctc accaaaccgg actct                                          25

<210> 2151
<211> 25
<212> DNA
<213> Homo sapiens

<400> 2151
cccaagctca ccaaaccgga ctctg                                          25

<210> 2152
<211> 25
<212> DNA
<213> Homo sapiens

<400> 2152
ccaagctcac caaaccggac tctgc                                          25

<210> 2153
<211> 25
<212> DNA
<213> Homo sapiens

<400> 2153
caagctcacc aaaccggact ctgcc                                          25

<210> 2154
<211> 25
<212> DNA
<213> Homo sapiens

<400> 2154
aagctcacca aaccggactc tgcca                                          25

<210> 2155
<211> 25
<212> DNA
<213> Homo sapiens

<400> 2155
agctcaccaa accggactct gccac                                          25

<210> 2156
<211> 25
<212> DNA
<213> Homo sapiens

<400> 2156
gctcaccaaa ccggactctg ccacc                                          25

<210> 2157
<211> 25
<212> DNA
<213> Homo sapiens

<400> 2157
ctcaccaaac cggactctgc caccg                                          25

<210> 2158
<211> 25
<212> DNA
<213> Homo sapiens

<400> 2158
tcaccaaacc ggactctgcc accgg                                          25

<210> 2159
<211> 25
<212> DNA
<213> Homo sapiens

<400> 2159
caccaaaccg gactctgcca ccggg                                          25

<210> 2160
<211> 25

<212> DNA
<213> Homo sapiens

<400> 2160
accaaaccgg actctgccac cgggc                                          25

<210> 2161
<211> 25
<212> DNA
<213> Homo sapiens

<400> 2161
ccaaaccgga ctctgccacc gggcc                                          25

<210> 2162
<211> 25
<212> DNA
<213> Homo sapiens

<400> 2162
caaaccggac tctgccaccg ggccg                                          25

<210> 2163
<211> 25
<212> DNA
<213> Homo sapiens

<400> 2163
aaaccggact ctgccaccgg gccgc                                          25

<210> 2164
<211> 25
<212> DNA
<213> Homo sapiens

<400> 2164
aaccggactc tgccaccggg ccgca                                          25

<210> 2165
<211> 25
<212> DNA
<213> Homo sapiens

<400> 2165
accggactct gccaccgggc cgcag                                          25

<210> 2166
<211> 25
<212> DNA
<213> Homo sapiens

<400> 2166
ccggactctg ccaccgggcc gcagt                                          25

<210> 2167
<211> 25
<212> DNA
<213> Homo sapiens

<400> 2167
cggactctgc caccgggccg cagtg                                          25

<210> 2168
<211> 25
<212> DNA
<213> Homo sapiens

<400> 2168
ggactctgcc accgggccgc agtgg                                          25

<210> 2169
<211> 25
<212> DNA
<213> Homo sapiens

<400> 2169
gactctgcca ccgggccgca gtggt                                          25

<210> 2170
<211> 25
<212> DNA
<213> Homo sapiens

<400> 2170
actctgccac cgggccgcag tggta                                          25

<210> 2171
<211> 25
<212> DNA
<213> Homo sapiens

<400> 2171
ctctgccacc gggccgcagt ggtac                                          25

<210> 2172
<211> 25
<212> DNA
<213> Homo sapiens

<400> 2172
tctgccaccg ggccgcagtg gtacc                                          25

<210> 2173
<211> 25
<212> DNA
<213> Homo sapiens

<400> 2173
ctgccaccgg gccgcagtgg tacca                                          25

<210> 2174
<211> 25
<212> DNA
<213> Homo sapiens

<400> 2174
tgccaccggg ccgcagtggt accag                                          25

<210> 2175
<211> 25
<212> DNA
<213> Homo sapiens

<400> 2175
gccaccgggc cgcagtggta ccagg                                          25

<210> 2176
<211> 25
<212> DNA
<213> Homo sapiens

<400> 2176
ccaccgggcc gcagtggtac cagga                                          25

<210> 2177
<211> 25
<212> DNA
<213> Homo sapiens

<400> 2177
caccgggccg cagtggtacc aggaa                                          25

<210> 2178
<211> 25
<212> DNA
<213> Homo sapiens

<400> 2178
accgggccgc agtggtacca ggaat                                          25

<210> 2179
<211> 25
<212> DNA
<213> Homo sapiens

<400> 2179
ccgggccgca gtggtaccag gaatc                                          25

<210> 2180
<211> 25
<212> DNA
<213> Homo sapiens

<400> 2180
cgggccgcag tggtaccagg aatct                                          25

<210> 2181
<211> 25
<212> DNA
<213> Homo sapiens

<400> 2181
gggccgcagt ggtaccagga atctc                                          25

<210> 2182
<211> 25
<212> DNA
<213> Homo sapiens

<400> 2182
ggccgcagtg gtaccaggaa tctca                                          25

<210> 2183

<211> 25
<212> DNA
<213> Homo sapiens

<400> 2183
gccgcagtgg taccaggaat ctcag                                          25

<210> 2184
<211> 25
<212> DNA
<213> Homo sapiens

<400> 2184
ccgcagtggt accaggaatc tcagg                                          25

<210> 2185
<211> 25
<212> DNA
<213> Homo sapiens

<400> 2185
cgcagtggta ccaggaatct cagga                                          25

<210> 2186
<211> 25
<212> DNA
<213> Homo sapiens

<400> 2186
gcagtggtac caggaatctc aggaa                                          25

<210> 2187
<211> 25
<212> DNA
<213> Homo sapiens

<400> 2187
cagtggtacc aggaatctca ggaat                                          25

<210> 2188
<211> 25
<212> DNA
<213> Homo sapiens

<400> 2188
agtggtacca ggaatctcag gaatc                                          25

<210> 2189
<211> 25
<212> DNA
<213> Homo sapiens

<400> 2189
gtggtaccag gaatctcagg aatcg                                          25

<210> 2190
<211> 25
<212> DNA
<213> Homo sapiens

<400> 2190

tggtaccagg aatctcagga atcgg 25

<210> 2191
<211> 25
<212> DNA
<213> Homo sapiens

<400> 2191
ggtaccagga atctcaggaa tcgga 25

<210> 2192
<211> 25
<212> DNA
<213> Homo sapiens

<400> 2192
gtaccaggaa tctcaggaat cggag 25

<210> 2193
<211> 25
<212> DNA
<213> Homo sapiens

<400> 2193
taccaggaat ctcaggaatc ggagt 25

<210> 2194
<211> 25
<212> DNA
<213> Homo sapiens

<400> 2194
accaggaatc tcaggaatcg gagtc 25

<210> 2195
<211> 25
<212> DNA
<213> Homo sapiens

<400> 2195
ccaggaatct caggaatcgg agtcg 25

<210> 2196
<211> 25
<212> DNA
<213> Homo sapiens

<400> 2196
caggaatctc aggaatcgga gtcgg 25

<210> 2197
<211> 25
<212> DNA
<213> Homo sapiens

<400> 2197
aggaatctca ggaatcggag tcgga 25

<210> 2198
<211> 25
<212> DNA

<213> Homo sapiens

<400> 2198
ggaatctcag gaatcggagt cggaa                                          25

<210> 2199
<211> 25
<212> DNA
<213> Homo sapiens

<400> 2199
gaatctcagg aatcggagtc ggaag                                          25

<210> 2200
<211> 25
<212> DNA
<213> Homo sapiens

<400> 2200
aatctcagga atcggagtcg gaagg                                          25

<210> 2201
<211> 25
<212> DNA
<213> Homo sapiens

<400> 2201
atctcaggaa tcggagtcgg aaggc                                          25

<210> 2202
<211> 25
<212> DNA
<213> Homo sapiens

<400> 2202
tctcaggaat cggagtcgga aggca                                          25

<210> 2203
<211> 25
<212> DNA
<213> Homo sapiens

<400> 2203
ctcaggaatc ggagtcggaa ggcaa                                          25

<210> 2204
<211> 25
<212> DNA
<213> Homo sapiens

<400> 2204
tcaggaatcg gagtcggaag gcaag                                          25

<210> 2205
<211> 25
<212> DNA
<213> Homo sapiens

<400> 2205
caggaatcgg agtcggaagg caagc                                          25

<210> 2206
<211> 25
<212> DNA
<213> Homo sapiens

<400> 2206
aggaatcgga gtcggaaggc aagca                                          25

<210> 2207
<211> 25
<212> DNA
<213> Homo sapiens

<400> 2207
ggaatcggag tcggaaggca agcag                                          25

<210> 2208
<211> 25
<212> DNA
<213> Homo sapiens

<400> 2208
gaatcggagt cggaaggcaa gcagc                                          25

<210> 2209
<211> 25
<212> DNA
<213> Homo sapiens

<400> 2209
aatcggagtc ggaaggcaag cagcc                                          25

<210> 2210
<211> 25
<212> DNA
<213> Homo sapiens

<400> 2210
atcggagtcg gaaggcaagc agcca                                          25

<210> 2211
<211> 25
<212> DNA
<213> Homo sapiens

<400> 2211
tcggagtcgg aaggcaagca gccac                                          25

<210> 2212
<211> 25
<212> DNA
<213> Homo sapiens

<400> 2212
cggagtcgga aggcaagcag ccacc                                          25

<210> 2213
<211> 25
<212> DNA
<213> Homo sapiens

<400> 2213
ggagtcggaa ggcaagcagc caccc                                          25

<210> 2214
<211> 25
<212> DNA
<213> Homo sapiens

<400> 2214
gagtcggaag gcaagcagcc acccc                                          25

<210> 2215
<211> 25
<212> DNA
<213> Homo sapiens

<400> 2215
agtcggaagg caagcagcca ccccc                                          25

<210> 2216
<211> 25
<212> DNA
<213> Homo sapiens

<400> 2216
gtcggaaggc aagcagccac ccccg                                          25

<210> 2217
<211> 25
<212> DNA
<213> Homo sapiens

<400> 2217
tcggaaggca agcagccacc cccgg                                          25

<210> 2218
<211> 25
<212> DNA
<213> Homo sapiens

<400> 2218
cggaaggcaa gcagccaccc ccggg                                          25

<210> 2219
<211> 25
<212> DNA
<213> Homo sapiens

<400> 2219
ggaaggcaag cagccacccc cggga                                          25

<210> 2220
<211> 25
<212> DNA
<213> Homo sapiens

<400> 2220
gaaggcaagc agccaccccc gggac                                          25

<210> 2221
<211> 25

<212> DNA
<213> Homo sapiens

<400> 2221
aaggcaagca gccacccccg ggacc                                          25

<210> 2222
<211> 25
<212> DNA
<213> Homo sapiens

<400> 2222
aggcaagcag ccacccccgg gaccc                                          25

<210> 2223
<211> 25
<212> DNA
<213> Homo sapiens

<400> 2223
ggcaagcagc cacccccggg acccc                                          25

<210> 2224
<211> 25
<212> DNA
<213> Homo sapiens

<400> 2224
gcaagcagcc acccccggga cccct                                          25

<210> 2225
<211> 25
<212> DNA
<213> Homo sapiens

<400> 2225
caagcagcca cccccgggac ccctg                                          25

<210> 2226
<211> 25
<212> DNA
<213> Homo sapiens

<400> 2226
aagcagccac ccccgggacc cctgg                                          25

<210> 2227
<211> 25
<212> DNA
<213> Homo sapiens

<400> 2227
agcagccacc cccgggaccc ctggc                                          25

<210> 2228
<211> 25
<212> DNA
<213> Homo sapiens

<400> 2228
gcagccaccc ccgggacccc tggca                                          25

<210> 2229
<211> 25
<212> DNA
<213> Homo sapiens

<400> 2229
cagccacccc cgggacccct ggcac                                          25

<210> 2230
<211> 25
<212> DNA
<213> Homo sapiens

<400> 2230
agccaccccc gggacccctg gcacc                                          25

<210> 2231
<211> 25
<212> DNA
<213> Homo sapiens

<400> 2231
gccacccccg ggacccctgg caccc                                          25

<210> 2232
<211> 25
<212> DNA
<213> Homo sapiens

<400> 2232
ccacccccgg gacccctggc acccc                                          25

<210> 2233
<211> 25
<212> DNA
<213> Homo sapiens

<400> 2233
cacccccggg acccctggca ccccc                                          25

<210> 2234
<211> 25
<212> DNA
<213> Homo sapiens

<400> 2234
acccccggga cccctggcac ccccg                                          25

<210> 2235
<211> 25
<212> DNA
<213> Homo sapiens

<400> 2235
cccccgggac ccctggcacc cccga                                          25

<210> 2236
<211> 25
<212> DNA
<213> Homo sapiens

<400> 2236
ccccgggacc cctggcaccc ccgaa                                          25

<210> 2237
<211> 25
<212> DNA
<213> Homo sapiens

<400> 2237
cccgggaccc ctggcacccc cgaaa                                          25

<210> 2238
<211> 25
<212> DNA
<213> Homo sapiens

<400> 2238
ccgggacccc tggcaccccc gaaat                                          25

<210> 2239
<211> 25
<212> DNA
<213> Homo sapiens

<400> 2239
cgggacccct ggcacccccg aaatc                                          25

<210> 2240
<211> 25
<212> DNA
<213> Homo sapiens

<400> 2240
gggacccctg gcacccccga aatcc                                          25

<210> 2241
<211> 25
<212> DNA
<213> Homo sapiens

<400> 2241
ggacccctgg cacccccgaa atccc                                          25

<210> 2242
<211> 25
<212> DNA
<213> Homo sapiens

<400> 2242
gacccctggc acccccgaaa tcccc                                          25

<210> 2243
<211> 25
<212> DNA
<213> Homo sapiens

<400> 2243
acccctggca cccccgaaat ccccc                                          25

<210> 2244

<211> 25
<212> DNA
<213> Homo sapiens

<400> 2244
cccctggcac ccccgaaatc ccccg                                          25

<210> 2245
<211> 25
<212> DNA
<213> Homo sapiens

<400> 2245
ccctggcacc cccgaaatcc cccga                                          25

<210> 2246
<211> 25
<212> DNA
<213> Homo sapiens

<400> 2246
cctggcaccc ccgaaatccc ccgaa                                          25

<210> 2247
<211> 25
<212> DNA
<213> Homo sapiens

<400> 2247
ctggcacccc cgaaatcccc cgaac                                          25

<210> 2248
<211> 25
<212> DNA
<213> Homo sapiens

<400> 2248
tggcaccccc gaaatccccc gaacc                                          25

<210> 2249
<211> 25
<212> DNA
<213> Homo sapiens

<400> 2249
ggcacccccg aaatcccccg aaccc                                          25

<210> 2250
<211> 25
<212> DNA
<213> Homo sapiens

<400> 2250
gcacccccga aatcccccga accct                                          25

<210> 2251
<211> 25
<212> DNA
<213> Homo sapiens

<400> 2251

cacccccgaa atcccccgaa ccctc 25

<210> 2252
<211> 25
<212> DNA
<213> Homo sapiens

<400> 2252
acccccgaaa tcccccgaac cctca 25

<210> 2253
<211> 25
<212> DNA
<213> Homo sapiens

<400> 2253
cccccgaaat cccccgaacc ctcag 25

<210> 2254
<211> 25
<212> DNA
<213> Homo sapiens

<400> 2254
ccccgaaatc ccccgaaccc tcagg 25

<210> 2255
<211> 25
<212> DNA
<213> Homo sapiens

<400> 2255
cccgaaatcc cccgaaccct cagga 25

<210> 2256
<211> 25
<212> DNA
<213> Homo sapiens

<400> 2256
ccgaaatccc ccgaaccctc aggac 25

<210> 2257
<211> 25
<212> DNA
<213> Homo sapiens

<400> 2257
cgaaatcccc cgaaccctca ggacc 25

<210> 2258
<211> 25
<212> DNA
<213> Homo sapiens

<400> 2258
gaaatccccc gaaccctcag gaccc 25

<210> 2259
<211> 25
<212> DNA

<213> Homo sapiens

<400> 2259
aaatcccccg aaccctcagg acccc                                          25

<210> 2260
<211> 25
<212> DNA
<213> Homo sapiens

<400> 2260
aatcccccga accctcagga cccct                                          25

<210> 2261
<211> 25
<212> DNA
<213> Homo sapiens

<400> 2261
atcccccgaa ccctcaggac ccctg                                          25

<210> 2262
<211> 25
<212> DNA
<213> Homo sapiens

<400> 2262
tcccccgaac cctcaggacc cctgg                                          25

<210> 2263
<211> 25
<212> DNA
<213> Homo sapiens

<400> 2263
cccccgaacc ctcaggaccc ctggc                                          25

<210> 2264
<211> 25
<212> DNA
<213> Homo sapiens

<400> 2264
ccccgaaccc tcaggacccc tggcg                                          25

<210> 2265
<211> 25
<212> DNA
<213> Homo sapiens

<400> 2265
cccgaaccct caggacccct ggcgt                                          25

<210> 2266
<211> 25
<212> DNA
<213> Homo sapiens

<400> 2266
ccgaaccctc aggacccctg gcgtc                                          25

<210> 2267
<211> 25
<212> DNA
<213> Homo sapiens

<400> 2267
cgaaccctca ggacccctgg cgtcg                                          25

<210> 2268
<211> 25
<212> DNA
<213> Homo sapiens

<400> 2268
gaaccctcag gacccctggc gtcgg                                          25

<210> 2269
<211> 25
<212> DNA
<213> Homo sapiens

<400> 2269
aaccctcagg acccctggcg tcgga                                          25

<210> 2270
<211> 25
<212> DNA
<213> Homo sapiens

<400> 2270
accctcagga cccctggcgt cggag                                          25

<210> 2271
<211> 25
<212> DNA
<213> Homo sapiens

<400> 2271
ccctcaggac ccctggcgtc ggagc                                          25

<210> 2272
<211> 25
<212> DNA
<213> Homo sapiens

<400> 2272
cctcaggacc cctggcgtcg gagca                                          25

<210> 2273
<211> 25
<212> DNA
<213> Homo sapiens

<400> 2273
ctcaggaccc ctggcgtcgg agcag                                          25

<210> 2274
<211> 25
<212> DNA
<213> Homo sapiens

<400> 2274
tcaggacccc tggcgtcgga gcagg                                          25

<210> 2275
<211> 25
<212> DNA
<213> Homo sapiens

<400> 2275
caggacccct ggcgtcggag cagga                                          25

<210> 2276
<211> 25
<212> DNA
<213> Homo sapiens

<400> 2276
aggacccctg gcgtcggagc aggat                                          25

<210> 2277
<211> 25
<212> DNA
<213> Homo sapiens

<400> 2277
ggacccctgg cgtcggagca ggatg                                          25

<210> 2278
<211> 25
<212> DNA
<213> Homo sapiens

<400> 2278
gacccctggc gtcggagcag gatgc                                          25

<210> 2279
<211> 25
<212> DNA
<213> Homo sapiens

<400> 2279
acccctggcg tcggagcagg atgca                                          25

<210> 2280
<211> 25
<212> DNA
<213> Homo sapiens

<400> 2280
cccctggcgt cggagcagga tgcac                                          25

<210> 2281
<211> 25
<212> DNA
<213> Homo sapiens

<400> 2281
ccctggcgtc ggagcaggat gcacc                                          25

<210> 2282
<211> 25

<212> DNA
<213> Homo sapiens

<400> 2282
cctggcgtcg gagcaggatg caccc                                          25

<210> 2283
<211> 25
<212> DNA
<213> Homo sapiens

<400> 2283
ctggcgtcgg agcaggatgc acccc                                          25

<210> 2284
<211> 25
<212> DNA
<213> Homo sapiens

<400> 2284
tggcgtcgga gcaggatgca cccct                                          25

<210> 2285
<211> 25
<212> DNA
<213> Homo sapiens

<400> 2285
ggcgtcggag caggatgcac ccctg                                          25

<210> 2286
<211> 25
<212> DNA
<213> Homo sapiens

<400> 2286
gcgtcggagc aggatgcacc cctgc                                          25

<210> 2287
<211> 25
<212> DNA
<213> Homo sapiens

<400> 2287
cgtcggagca ggatgcaccc ctgcc                                          25

<210> 2288
<211> 25
<212> DNA
<213> Homo sapiens

<400> 2288
gtcggagcag gatgcacccc tgcca                                          25

<210> 2289
<211> 25
<212> DNA
<213> Homo sapiens

<400> 2289
tcggagcagg atgcacccct gccag                                          25

<210> 2290
<211> 25
<212> DNA
<213> Homo sapiens

<400> 2290
cggagcagga tgcacccctg ccaga                                          25

<210> 2291
<211> 25
<212> DNA
<213> Homo sapiens

<400> 2291
ggagcaggat gcacccctgc cagag                                          25

<210> 2292
<211> 25
<212> DNA
<213> Homo sapiens

<400> 2292
gagcaggatg cacccctgcc agagg                                          25

<210> 2293
<211> 25
<212> DNA
<213> Homo sapiens

<400> 2293
agcaggatgc acccctgcca gaggg                                          25

<210> 2294
<211> 25
<212> DNA
<213> Homo sapiens

<400> 2294
gcaggatgca cccctgccag agggg                                          25

<210> 2295
<211> 25
<212> DNA
<213> Homo sapiens

<400> 2295
caggatgcac ccctgccaga ggggg                                          25

<210> 2296
<211> 25
<212> DNA
<213> Homo sapiens

<400> 2296
aggatgcacc cctgccagag gggga                                          25

<210> 2297
<211> 25
<212> DNA
<213> Homo sapiens

<400> 2297
ggatgcaccc ctgccagagg gggac                                          25

<210> 2298
<211> 25
<212> DNA
<213> Homo sapiens

<400> 2298
gatgcacccc tgccagaggg ggacg                                          25

<210> 2299
<211> 25
<212> DNA
<213> Homo sapiens

<400> 2299
atgcacccct gccagagggg gacga                                          25

<210> 2300
<211> 25
<212> DNA
<213> Homo sapiens

<400> 2300
tgcacccctg ccagaggggg acgat                                          25

<210> 2301
<211> 25
<212> DNA
<213> Homo sapiens

<400> 2301
gcacccctgc cagaggggga cgatg                                          25

<210> 2302
<211> 25
<212> DNA
<213> Homo sapiens

<400> 2302
cacccctgcc agagggggac gatgc                                          25

<210> 2303
<211> 25
<212> DNA
<213> Homo sapiens

<400> 2303
acccctgcca gagggggacg atgca                                          25

<210> 2304
<211> 25
<212> DNA
<213> Homo sapiens

<400> 2304
cccctgccag agggggacga tgcac                                          25

<210> 2305

<211> 25
<212> DNA
<213> Homo sapiens

<400> 2305
ccctgccaga gggggacgat gcacc                                          25

<210> 2306
<211> 25
<212> DNA
<213> Homo sapiens

<400> 2306
cctgccagag ggggacgatg caccc                                          25

<210> 2307
<211> 25
<212> DNA
<213> Homo sapiens

<400> 2307
ctgccagagg gggacgatgc acccc                                          25

<210> 2308
<211> 25
<212> DNA
<213> Homo sapiens

<400> 2308
tgccagaggg ggacgatgca ccccc                                          25

<210> 2309
<211> 25
<212> DNA
<213> Homo sapiens

<400> 2309
gccagagggg gacgatgcac ccccc                                          25

<210> 2310
<211> 25
<212> DNA
<213> Homo sapiens

<400> 2310
ccagaggggg acgatgcacc ccccc                                          25

<210> 2311
<211> 25
<212> DNA
<213> Homo sapiens

<400> 2311
cagaggggga cgatgcaccc ccccg                                          25

<210> 2312
<211> 25
<212> DNA
<213> Homo sapiens

<400> 2312
   agagggggac gatgcacccc cccgg                                                25

<210> 2313
<211> 25
<212> DNA
<213> Homo sapiens

<400> 2313
gaggggacg atgcaccccc ccggc                                                25

<210> 2314
<211> 25
<212> DNA
<213> Homo sapiens

<400> 2314
   agggggacga tgcacccccc cggcc                                                25

<210> 2315
<211> 25
<212> DNA
<213> Homo sapiens

<400> 2315
gggggacgat gcaccccccc ggccg                                                25

<210> 2316
<211> 25
<212> DNA
<213> Homo sapiens

<400> 2316
   ggggacgatg cacccccccg gccgt                                                25

<210> 2317
<211> 25
<212> DNA
<213> Homo sapiens

<400> 2317
   gggacgatgc acccccccgg ccgtc                                                25

<210> 2318
<211> 25
<212> DNA
<213> Homo sapiens

<400> 2318
ggacgatgca cccccccggc cgtcg                                                25

<210> 2319
<211> 25
<212> DNA
<213> Homo sapiens

<400> 2319
   gacgatgcac ccccccggcc gtcga                                                25

<210> 2320
<211> 25
<212> DNA

<213> Homo sapiens

<400> 2320
acgatgcacc cccccggccg tcgat                                          25

<210> 2321
<211> 25
<212> DNA
<213> Homo sapiens

<400> 2321
cgatgcaccc ccccggccgt cgatg                                          25

<210> 2322
<211> 25
<212> DNA
<213> Homo sapiens

<400> 2322
gatgcacccc cccggccgtc gatgc                                          25

<210> 2323
<211> 25
<212> DNA
<213> Homo sapiens

<400> 2323
atgcaccccc ccggccgtcg atgct                                          25

<210> 2324
<211> 25
<212> DNA
<213> Homo sapiens

<400> 2324
tgcacccccc cggccgtcga tgctg                                          25

<210> 2325
<211> 25
<212> DNA
<213> Homo sapiens

<400> 2325
gcaccccccc ggccgtcgat gctgg                                          25

<210> 2326
<211> 25
<212> DNA
<213> Homo sapiens

<400> 2326
cacccccccg gccgtcgatg ctgga                                          25

<210> 2327
<211> 25
<212> DNA
<213> Homo sapiens

<400> 2327
acccccccgg ccgtcgatgc tggac                                          25

<210> 2328
<211> 25
<212> DNA
<213> Homo sapiens

<400> 2328
cccccccggc cgtcgatgct ggacg                                            25

<210> 2329
<211> 25
<212> DNA
<213> Homo sapiens

<400> 2329
ccccccggcc gtcgatgctg gacga                                            25

<210> 2330
<211> 25
<212> DNA
<213> Homo sapiens

<400> 2330
cccccggccg tcgatgctgg acgat                                            25

<210> 2331
<211> 25
<212> DNA
<213> Homo sapiens

<400> 2331
ccccggccgt cgatgctgga cgatg                                            25

<210> 2332
<211> 25
<212> DNA
<213> Homo sapiens

<400> 2332
cccggccgtc gatgctggac gatgc                                            25

<210> 2333
<211> 25
<212> DNA
<213> Homo sapiens

<400> 2333
ccggccgtcg atgctggacg atgca                                            25

<210> 2334
<211> 25
<212> DNA
<213> Homo sapiens

<400> 2334
cggccgtcga tgctggacga tgcac                                            25

<210> 2335
<211> 25
<212> DNA
<213> Homo sapiens

<400> 2335
ggccgtcgat gctggacgat gcacc                                          25

<210> 2336
<211> 25
<212> DNA
<213> Homo sapiens

<400> 2336
gccgtcgatg ctggacgatg caccc                                          25

<210> 2337
<211> 25
<212> DNA
<213> Homo sapiens

<400> 2337
ccgtcgatgc tggacgatgc acccc                                          25

<210> 2338
<211> 25
<212> DNA
<213> Homo sapiens

<400> 2338
cgtcgatgct ggacgatgca ccccg                                          25

<210> 2339
<211> 25
<212> DNA
<213> Homo sapiens

<400> 2339
gtcgatgctg gacgatgcac cccgc                                          25

<210> 2340
<211> 25
<212> DNA
<213> Homo sapiens

<400> 2340
tcgatgctgg acgatgcacc ccgcc                                          25

<210> 2341
<211> 25
<212> DNA
<213> Homo sapiens

<400> 2341
cgatgctgga cgatgcaccc cgcct                                          25

<210> 2342
<211> 25
<212> DNA
<213> Homo sapiens

<400> 2342
gatgctggac gatgcacccc gcctg                                          25

<210> 2343
<211> 25

<212> DNA
<213> Homo sapiens

<400> 2343
atgctggacg atgcaccccg cctgc                                          25

<210> 2344
<211> 25
<212> DNA
<213> Homo sapiens

<400> 2344
tgctggacga tgcaccccgc ctgcc                                          25

<210> 2345
<211> 25
<212> DNA
<213> Homo sapiens

<400> 2345
gctggacgat gcaccccgcc tgccg                                          25

<210> 2346
<211> 25
<212> DNA
<213> Homo sapiens

<400> 2346
ctggacgatg caccccgcct gccgc                                          25

<210> 2347
<211> 25
<212> DNA
<213> Homo sapiens

<400> 2347
tggacgatgc accccgcctg ccgct                                          25

<210> 2348
<211> 25
<212> DNA
<213> Homo sapiens

<400> 2348
ggacgatgca ccccgcctgc cgctg                                          25

<210> 2349
<211> 25
<212> DNA
<213> Homo sapiens

<400> 2349
gacgatgcac cccgcctgcc gctgg                                          25

<210> 2350
<211> 25
<212> DNA
<213> Homo sapiens

<400> 2350
acgatgcacc ccgcctgccg ctgga                                          25

<210> 2351
<211> 25
<212> DNA
<213> Homo sapiens

<400> 2351
cgatgcaccc cgcctgccgc tggag                                          25

<210> 2352
<211> 25
<212> DNA
<213> Homo sapiens

<400> 2352
gatgcacccc gcctgccgct ggagc                                          25

<210> 2353
<211> 25
<212> DNA
<213> Homo sapiens

<400> 2353
atgcaccccg cctgccgctg gagct                                          25

<210> 2354
<211> 25
<212> DNA
<213> Homo sapiens

<400> 2354
tgcaccccgc ctgccgctgg agctg                                          25

<210> 2355
<211> 25
<212> DNA
<213> Homo sapiens

<400> 2355
gcaccccgcc tgccgctgga gctgg                                          25

<210> 2356
<211> 25
<212> DNA
<213> Homo sapiens

<400> 2356
caccccgcct gccgctggag ctgga                                          25

<210> 2357
<211> 25
<212> DNA
<213> Homo sapiens

<400> 2357
accccgcctg ccgctggagc tggac                                          25

<210> 2358
<211> 25
<212> DNA
<213> Homo sapiens

<400> 2358
ccccgcctgc cgctggagct ggacg                                          25

<210> 2359
<211> 25
<212> DNA
<213> Homo sapiens

<400> 2359
cccgcctgcc gctggagctg gacga                                          25

<210> 2360
<211> 25
<212> DNA
<213> Homo sapiens

<400> 2360
ccgcctgccg ctggagctgg acgat                                          25

<210> 2361
<211> 25
<212> DNA
<213> Homo sapiens

<400> 2361
cgcctgccgc tggagctgga cgatg                                          25

<210> 2362
<211> 25
<212> DNA
<213> Homo sapiens

<400> 2362
gcctgccgct ggagctggac gatgc                                          25

<210> 2363
<211> 25
<212> DNA
<213> Homo sapiens

<400> 2363
cctgccgctg gagctggacg atgca                                          25

<210> 2364
<211> 25
<212> DNA
<213> Homo sapiens

<400> 2364
ctgccgctgg agctggacga tgcac                                          25

<210> 2365
<211> 25
<212> DNA
<213> Homo sapiens

<400> 2365
tgccgctgga gctggacgat gcacc                                          25

<210> 2366

<211> 25
<212> DNA
<213> Homo sapiens

<400> 2366
gccgctggag ctggacgatg caccc                                          25

<210> 2367
<211> 25
<212> DNA
<213> Homo sapiens

<400> 2367
ccgctggagc tggacgatgc acccc                                          25

<210> 2368
<211> 25
<212> DNA
<213> Homo sapiens

<400> 2368
cgctggagct ggacgatgca cccct                                          25

<210> 2369
<211> 25
<212> DNA
<213> Homo sapiens

<400> 2369
gctggagctg gacgatgcac ccctg                                          25

<210> 2370
<211> 25
<212> DNA
<213> Homo sapiens

<400> 2370
ctggagctgg acgatgcacc cctgc                                          25

<210> 2371
<211> 25
<212> DNA
<213> Homo sapiens

<400> 2371
tggagctgga cgatgcaccc ctgcc                                          25

<210> 2372
<211> 25
<212> DNA
<213> Homo sapiens

<400> 2372
ggagctggac gatgcacccc tgccg                                          25

<210> 2373
<211> 25
<212> DNA
<213> Homo sapiens

<400> 2373

gagctggacg atgcacccct gccgg                                          25

<210> 2374
<211> 25
<212> DNA
<213> Homo sapiens

<400> 2374
agctggacga tgcacccctg ccgga                                          25

<210> 2375
<211> 25
<212> DNA
<213> Homo sapiens

<400> 2375
gctggacgat gcacccctgc cggag                                          25

<210> 2376
<211> 25
<212> DNA
<213> Homo sapiens

<400> 2376
ctggacgatg cacccctgcc ggagg                                          25

<210> 2377
<211> 25
<212> DNA
<213> Homo sapiens

<400> 2377
tggacgatgc acccctgccg gagga                                          25

<210> 2378
<211> 25
<212> DNA
<213> Homo sapiens

<400> 2378
ggacgatgca cccctgccgg aggag                                          25

<210> 2379
<211> 25
<212> DNA
<213> Homo sapiens

<400> 2379
gacgatgcac ccctgccgga ggagg                                          25

<210> 2380
<211> 25
<212> DNA
<213> Homo sapiens

<400> 2380
acgatgcacc cctgccggag gagga                                          25

<210> 2381
<211> 25
<212> DNA

<213> Homo sapiens

<400> 2381
cgatgcaccc ctgccggagg aggaa                                          25

<210> 2382
<211> 25
<212> DNA
<213> Homo sapiens

<400> 2382
gatgcacccc tgccggagga ggaaa                                          25

<210> 2383
<211> 25
<212> DNA
<213> Homo sapiens

<400> 2383
atgcacccct gccggaggag gaaac                                          25

<210> 2384
<211> 25
<212> DNA
<213> Homo sapiens

<400> 2384
tgcacccctg ccggaggagg aaacc                                          25

<210> 2385
<211> 25
<212> DNA
<213> Homo sapiens

<400> 2385
gcacccctgc cggaggagga aaccc                                          25

<210> 2386
<211> 25
<212> DNA
<213> Homo sapiens

<400> 2386
cacccctgcc ggaggaggaa acccc                                          25

<210> 2387
<211> 25
<212> DNA
<213> Homo sapiens

<400> 2387
acccctgccg gaggaggaaa ccccc                                          25

<210> 2388
<211> 25
<212> DNA
<213> Homo sapiens

<400> 2388
cccctgccgg aggaggaaac ccccg                                          25

<210> 2389
<211> 25
<212> DNA
<213> Homo sapiens

<400> 2389
ccctgccgga ggaggaaacc cccga                                          25

<210> 2390
<211> 25
<212> DNA
<213> Homo sapiens

<400> 2390
cctgccggag gaggaaaccc ccgaa                                          25

<210> 2391
<211> 25
<212> DNA
<213> Homo sapiens

<400> 2391
ctgccggagg aggaaacccc cgaac                                          25

<210> 2392
<211> 25
<212> DNA
<213> Homo sapiens

<400> 2392
tgccggagga ggaaaccccc gaacc                                          25

<210> 2393
<211> 25
<212> DNA
<213> Homo sapiens

<400> 2393
gccggaggag gaaacccccg aaccc                                          25

<210> 2394
<211> 25
<212> DNA
<213> Homo sapiens

<400> 2394
ccggaggagg aaacccccga accca                                          25

<210> 2395
<211> 25
<212> DNA
<213> Homo sapiens

<400> 2395
cggaggagga aacccccgaa cccac                                          25

<210> 2396
<211> 25
<212> DNA
<213> Homo sapiens

<400> 2396
ggaggaggaa acccccgaac ccacg                                          25

<210> 2397
<211> 25
<212> DNA
<213> Homo sapiens

<400> 2397
gaggaggaaa cccccgaacc cacgg                                          25

<210> 2398
<211> 25
<212> DNA
<213> Homo sapiens

<400> 2398
aggaggaaac ccccgaaccc acggc                                          25

<210> 2399
<211> 25
<212> DNA
<213> Homo sapiens

<400> 2399
ggaggaaacc cccgaaccca cggcc                                          25

<210> 2400
<211> 25
<212> DNA
<213> Homo sapiens

<400> 2400
gaggaaaccc ccgaacccac ggcca                                          25

<210> 2401
<211> 25
<212> DNA
<213> Homo sapiens

<400> 2401
aggaaacccc cgaacccacg gccat                                          25

<210> 2402
<211> 25
<212> DNA
<213> Homo sapiens

<400> 2402
ggaaaccccc gaacccacgg ccatc                                          25

<210> 2403
<211> 25
<212> DNA
<213> Homo sapiens

<400> 2403
gaaacccccg aacccacggc catct                                          25

<210> 2404
<211> 25

<212> DNA
<213> Homo sapiens

<400> 2404
aaacccccga acccacggcc atctg                                          25

<210> 2405
<211> 25
<212> DNA
<213> Homo sapiens

<400> 2405
aacccccgaa cccacggcca tctgc                                          25

<210> 2406
<211> 25
<212> DNA
<213> Homo sapiens

<400> 2406
acccccgaac ccacggccat ctgca                                          25

<210> 2407
<211> 25
<212> DNA
<213> Homo sapiens

<400> 2407
cccccgaacc cacggccatc tgcag                                          25

<210> 2408
<211> 25
<212> DNA
<213> Homo sapiens

<400> 2408
ccccgaaccc acggccatct gcagg                                          25

<210> 2409
<211> 25
<212> DNA
<213> Homo sapiens

<400> 2409
cccgaaccca cggccatctg caggc                                          25

<210> 2410
<211> 25
<212> DNA
<213> Homo sapiens

<400> 2410
ccgaacccac ggccatctgc aggca                                          25

<210> 2411
<211> 25
<212> DNA
<213> Homo sapiens

<400> 2411
cgaacccacg gccatctgca ggcac                                          25

<210> 2412
<211> 25
<212> DNA
<213> Homo sapiens

<400> 2412
gaacccacgg ccatctgcag gcacc                                          25

<210> 2413
<211> 25
<212> DNA
<213> Homo sapiens

<400> 2413
aacccacggc catctgcagg caccg                                          25

<210> 2414
<211> 25
<212> DNA
<213> Homo sapiens

<400> 2414
acccacggcc atctgcaggc accgg                                          25

<210> 2415
<211> 25
<212> DNA
<213> Homo sapiens

<400> 2415
cccacggcca tctgcaggca ccggc                                          25

<210> 2416
<211> 25
<212> DNA
<213> Homo sapiens

<400> 2416
ccacggccat ctgcaggcac cggca                                          25

<210> 2417
<211> 25
<212> DNA
<213> Homo sapiens

<400> 2417
cacggccatc tgcaggcacc ggcac                                          25

<210> 2418
<211> 25
<212> DNA
<213> Homo sapiens

<400> 2418
acggccatct gcaggcaccg gcacc                                          25

<210> 2419
<211> 25
<212> DNA
<213> Homo sapiens

<400> 2419
cggccatctg caggcaccgg caccg                                          25

<210> 2420
<211> 25
<212> DNA
<213> Homo sapiens

<400> 2420
ggccatctgc aggcaccggc accgc                                          25

<210> 2421
<211> 25
<212> DNA
<213> Homo sapiens

<400> 2421
gccatctgca ggcaccggca ccgct                                          25

<210> 2422
<211> 25
<212> DNA
<213> Homo sapiens

<400> 2422
ccatctgcag gcaccggcac cgctg                                          25

<210> 2423
<211> 25
<212> DNA
<213> Homo sapiens

<400> 2423
catctgcagg caccggcacc gctgt                                          25

<210> 2424
<211> 25
<212> DNA
<213> Homo sapiens

<400> 2424
atctgcaggc accggcaccg ctgtc                                          25

<210> 2425
<211> 25
<212> DNA
<213> Homo sapiens

<400> 2425
tctgcaggca ccggcaccgc tgtca                                          25

<210> 2426
<211> 25
<212> DNA
<213> Homo sapiens

<400> 2426
ctgcaggcac cggcaccgct gtcac                                          25

<210> 2427

<211> 25
<212> DNA
<213> Homo sapiens

<400> 2427
tgcaggcacc ggcaccgctg tcaca                                          25

<210> 2428
<211> 25
<212> DNA
<213> Homo sapiens

<400> 2428
gcaggcaccg gcaccgctgt cacac                                          25

<210> 2429
<211> 25
<212> DNA
<213> Homo sapiens

<400> 2429
caggcaccgg caccgctgtc acacc                                          25

<210> 2430
<211> 25
<212> DNA
<213> Homo sapiens

<400> 2430
aggcaccggc accgctgtca caccg                                          25

<210> 2431
<211> 25
<212> DNA
<213> Homo sapiens

<400> 2431
ggcaccggca ccgctgtcac accga                                          25

<210> 2432
<211> 25
<212> DNA
<213> Homo sapiens

<400> 2432
gcaccggcac cgctgtcaca ccgac                                          25

<210> 2433
<211> 25
<212> DNA
<213> Homo sapiens

<400> 2433
caccggcacc gctgtcacac cgact                                          25

<210> 2434
<211> 25
<212> DNA
<213> Homo sapiens

<400> 2434

accggcaccg ctgtcacacc gactg 25

<210> 2435
<211> 25
<212> DNA
<213> Homo sapiens

<400> 2435
ccggcaccgc tgtcacaccg actgc 25

<210> 2436
<211> 25
<212> DNA
<213> Homo sapiens

<400> 2436
cggcaccgct gtcacaccga ctgcc 25

<210> 2437
<211> 25
<212> DNA
<213> Homo sapiens

<400> 2437
ggcaccgctg tcacaccgac tgcct 25

<210> 2438
<211> 25
<212> DNA
<213> Homo sapiens

<400> 2438
gcaccgctgt cacaccgact gccta 25

<210> 2439
<211> 25
<212> DNA
<213> Homo sapiens

<400> 2439
caccgctgtc acaccgactg cctag 25

<210> 2440
<211> 25
<212> DNA
<213> Homo sapiens

<400> 2440
accgctgtca caccgactgc ctaga 25

<210> 2441
<211> 25
<212> DNA
<213> Homo sapiens

<400> 2441
ccgctgtcac accgactgcc tagag 25

<210> 2442
<211> 25
<212> DNA

<213> Homo sapiens

<400> 2442
cgctgtcaca ccgactgcct agagg                                          25

<210> 2443
<211> 25
<212> DNA
<213> Homo sapiens

<400> 2443
gctgtcacac cgactgccta gaggg                                          25

<210> 2444
<211> 25
<212> DNA
<213> Homo sapiens

<400> 2444
ctgtcacacc gactgcctag agggg                                          25

<210> 2445
<211> 25
<212> DNA
<213> Homo sapiens

<400> 2445
tgtcacaccg actgcctaga ggggc                                          25

<210> 2446
<211> 25
<212> DNA
<213> Homo sapiens

<400> 2446
gtcacaccga ctgcctagag gggct                                          25

<210> 2447
<211> 25
<212> DNA
<213> Homo sapiens

<400> 2447
tcacaccgac tgcctagagg ggctg                                          25

<210> 2448
<211> 25
<212> DNA
<213> Homo sapiens

<400> 2448
cacaccgact gcctagaggg gctgc                                          25

<210> 2449
<211> 25
<212> DNA
<213> Homo sapiens

<400> 2449
acaccgactg cctagagggg ctgct                                          25

<210> 2450
<211> 25
<212> DNA
<213> Homo sapiens

<400> 2450
caccgactgc ctagaggggc tgctg                                            25

<210> 2451
<211> 25
<212> DNA
<213> Homo sapiens

<400> 2451
accgactgcc tagaggggct gctgt                                            25

<210> 2452
<211> 25
<212> DNA
<213> Homo sapiens

<400> 2452
ccgactgcct agaggggctg ctgtc                                            25

<210> 2453
<211> 25
<212> DNA
<213> Homo sapiens

<400> 2453
cgactgccta gaggggctgc tgtcc                                            25

<210> 2454
<211> 25
<212> DNA
<213> Homo sapiens

<400> 2454
gactgcctag aggggctgct gtccc                                            25

<210> 2455
<211> 25
<212> DNA
<213> Homo sapiens

<400> 2455
actgcctaga ggggctgctg tcccg                                            25

<210> 2456
<211> 25
<212> DNA
<213> Homo sapiens

<400> 2456
ctgcctagag gggctgctgt cccgc                                            25

<210> 2457
<211> 25
<212> DNA
<213> Homo sapiens

<400> 2457
tgcctagagg ggctgctgtc ccgca                                          25

<210> 2458
<211> 25
<212> DNA
<213> Homo sapiens

<400> 2458
gcctagaggg gctgctgtcc cgcac                                          25

<210> 2459
<211> 25
<212> DNA
<213> Homo sapiens

<400> 2459
cctagagggg ctgctgtccc gcacc                                          25

<210> 2460
<211> 25
<212> DNA
<213> Homo sapiens

<400> 2460
ctagaggggc tgctgtcccg cacct                                          25

<210> 2461
<211> 25
<212> DNA
<213> Homo sapiens

<400> 2461
tagaggggct gctgtcccgc acctt                                          25

<210> 2462
<211> 25
<212> DNA
<213> Homo sapiens

<400> 2462
agaggggctg ctgtcccgca ccttc                                          25

<210> 2463
<211> 25
<212> DNA
<213> Homo sapiens

<400> 2463
gaggggctgc tgtcccgcac cttcc                                          25

<210> 2464
<211> 25
<212> DNA
<213> Homo sapiens

<400> 2464
aggggctgct gtcccgcacc ttcca                                          25

<210> 2465
<211> 25

<212> DNA
<213> Homo sapiens

<400> 2465
ggggctgctg tcccgcacct tccag                                            25

<210> 2466
<211> 25
<212> DNA
<213> Homo sapiens

<400> 2466
gggctgctgt cccgcacctt ccagt                                            25

<210> 2467
<211> 25
<212> DNA
<213> Homo sapiens

<400> 2467
ggctgctgtc ccgcaccttc cagtg                                            25

<210> 2468
<211> 25
<212> DNA
<213> Homo sapiens

<400> 2468
gctgctgtcc cgcaccttcc agtgg                                            25

<210> 2469
<211> 25
<212> DNA
<213> Homo sapiens

<400> 2469
ctgctgtccc gcaccttcca gtggc                                            25

<210> 2470
<211> 25
<212> DNA
<213> Homo sapiens

<400> 2470
tgctgtcccg caccttccag tggct                                            25

<210> 2471
<211> 25
<212> DNA
<213> Homo sapiens

<400> 2471
gctgtcccgc accttccagt ggctg                                            25

<210> 2472
<211> 25
<212> DNA
<213> Homo sapiens

<400> 2472
ctgtcccgca ccttccagtg gctgg                                            25

<210> 2473
<211> 25
<212> DNA
<213> Homo sapiens

<400> 2473
tgtcccgcac cttccagtgg ctggg                                          25

<210> 2474
<211> 25
<212> DNA
<213> Homo sapiens

<400> 2474
gtcccgcacc ttccagtggc tgggg                                          25

<210> 2475
<211> 25
<212> DNA
<213> Homo sapiens

<400> 2475
tcccgcacct tccagtggct ggggt                                          25

<210> 2476
<211> 25
<212> DNA
<213> Homo sapiens

<400> 2476
cccgcacctt ccagtggctg gggtg                                          25

<210> 2477
<211> 25
<212> DNA
<213> Homo sapiens

<400> 2477
ccgcaccttc cagtggctgg ggtgg                                          25

<210> 2478
<211> 25
<212> DNA
<213> Homo sapiens

<400> 2478
cgcaccttcc agtggctggg gtggc                                          25

<210> 2479
<211> 25
<212> DNA
<213> Homo sapiens

<400> 2479
gcaccttcca gtggctgggg tggca                                          25

<210> 2480
<211> 25
<212> DNA
<213> Homo sapiens

<400> 2480
caccttccag tggctggggt ggcag                                          25

<210> 2481
<211> 25
<212> DNA
<213> Homo sapiens

<400> 2481
accttccagt ggctggggtg gcagg                                          25

<210> 2482
<211> 25
<212> DNA
<213> Homo sapiens

<400> 2482
ccttccagtg gctggggtgg caggt                                          25

<210> 2483
<211> 25
<212> DNA
<213> Homo sapiens

<400> 2483
cttccagtgg ctggggtggc aggtg                                          25

<210> 2484
<211> 25
<212> DNA
<213> Homo sapiens

<400> 2484
ttccagtggc tggggtggca ggtgg                                          25

<210> 2485
<211> 25
<212> DNA
<213> Homo sapiens

<400> 2485
tccagtggct ggggtggcag gtggg                                          25

<210> 2486
<211> 25
<212> DNA
<213> Homo sapiens

<400> 2486
ccagtggctg gggtggcagg tgggc                                          25

<210> 2487
<211> 25
<212> DNA
<213> Homo sapiens

<400> 2487
cagtggctgg ggtggcaggt gggcg                                          25

<210> 2488

<211> 25
<212> DNA
<213> Homo sapiens

<400> 2488
agtggctggg gtggcaggtg ggcgc                                                25

<210> 2489
<211> 25
<212> DNA
<213> Homo sapiens

<400> 2489
gtggctgggg tggcaggtgg gcgcg                                                25

<210> 2490
<211> 25
<212> DNA
<213> Homo sapiens

<400> 2490
tggctggggt ggcaggtggg cgcgc                                                25

<210> 2491
<211> 25
<212> DNA
<213> Homo sapiens

<400> 2491
ggctggggtg gcaggtgggc gcgca                                                25

<210> 2492
<211> 25
<212> DNA
<213> Homo sapiens

<400> 2492
gctggggtgg caggtgggcg cgcac                                                25

<210> 2493
<211> 25
<212> DNA
<213> Homo sapiens

<400> 2493
ctggggtggc aggtgggcgc gcacc                                                25

<210> 2494
<211> 25
<212> DNA
<213> Homo sapiens

<400> 2494
tggggtggca ggtgggcgcg caccc                                                25

<210> 2495
<211> 25
<212> DNA
<213> Homo sapiens

<400> 2495

ggggtggcag gtgggcgcgc acccc 25

<210> 2496
<211> 25
<212> DNA
<213> Homo sapiens

<400> 2496
gggtggcagg tgggcgcgca cccct 25

<210> 2497
<211> 25
<212> DNA
<213> Homo sapiens

<400> 2497
ggtggcaggt gggcgcgcac ccctg 25

<210> 2498
<211> 25
<212> DNA
<213> Homo sapiens

<400> 2498
gtggcaggtg ggcgcgcacc cctgg 25

<210> 2499
<211> 25
<212> DNA
<213> Homo sapiens

<400> 2499
tggcaggtgg gcgcgcaccc ctgga 25

<210> 2500
<211> 25
<212> DNA
<213> Homo sapiens

<400> 2500
ggcaggtggg cgcgcacccc tggat 25

<210> 2501
<211> 25
<212> DNA
<213> Homo sapiens

<400> 2501
gcaggtgggc gcgcacccct ggatc 25

<210> 2502
<211> 25
<212> DNA
<213> Homo sapiens

<400> 2502
caggtgggcg cgcacccctg gatct 25

<210> 2503
<211> 25
<212> DNA

<213> Homo sapiens

<400> 2503
aggtgggcgc gcacccctgg atctt                                          25

<210> 2504
<211> 25
<212> DNA
<213> Homo sapiens

<400> 2504
ggtgggcgcg cacccctgga tcttc                                          25

<210> 2505
<211> 25
<212> DNA
<213> Homo sapiens

<400> 2505
gtgggcgcgc acccctggat cttcc                                          25

<210> 2506
<211> 25
<212> DNA
<213> Homo sapiens

<400> 2506
tgggcgcgca cccctggatc ttcct                                          25

<210> 2507
<211> 25
<212> DNA
<213> Homo sapiens

<400> 2507
gggcgcgcac ccctggatct tcctg                                          25

<210> 2508
<211> 25
<212> DNA
<213> Homo sapiens

<400> 2508
ggcgcgcacc cctggatctt cctgc                                          25

<210> 2509
<211> 25
<212> DNA
<213> Homo sapiens

<400> 2509
gcgcgcaccc ctggatcttc ctgct                                          25

<210> 2510
<211> 25
<212> DNA
<213> Homo sapiens

<400> 2510
cgcgcacccc tggatcttcc tgctg                                          25

<210> 2511
<211> 25
<212> DNA
<213> Homo sapiens

<400> 2511
gcgcacccct ggatcttcct gctgg                                          25

<210> 2512
<211> 25
<212> DNA
<213> Homo sapiens

<400> 2512
cgcacccctg gatcttcctg ctggc                                          25

<210> 2513
<211> 25
<212> DNA
<213> Homo sapiens

<400> 2513
gcacccctgg atcttcctgc tggcg                                          25

<210> 2514
<211> 25
<212> DNA
<213> Homo sapiens

<400> 2514
cacccctgga tcttcctgct ggcgc                                          25

<210> 2515
<211> 25
<212> DNA
<213> Homo sapiens

<400> 2515
acccctggat cttcctgctg gcgcc                                          25

<210> 2516
<211> 25
<212> DNA
<213> Homo sapiens

<400> 2516
cccctggatc ttcctgctgg cgccc                                          25

<210> 2517
<211> 25
<212> DNA
<213> Homo sapiens

<400> 2517
ccctggatct tcctgctggc gccct                                          25

<210> 2518
<211> 25
<212> DNA
<213> Homo sapiens

<400> 2518
cctggatctt cctgctggcg ccctt                                          25

<210> 2519
<211> 25
<212> DNA
<213> Homo sapiens

<400> 2519
ctggatcttc ctgctggcgc ccttg                                          25

<210> 2520
<211> 25
<212> DNA
<213> Homo sapiens

<400> 2520
tggatcttcc tgctggcgcc cttga                                          25

<210> 2521
<211> 25
<212> DNA
<213> Homo sapiens

<400> 2521
ggatcttcct gctggcgccc ttgat                                          25

<210> 2522
<211> 25
<212> DNA
<213> Homo sapiens

<400> 2522
gatcttcctg ctggcgccct tgatg                                          25

<210> 2523
<211> 25
<212> DNA
<213> Homo sapiens

<400> 2523
atcttcctgc tggcgccctt gatgc                                          25

<210> 2524
<211> 25
<212> DNA
<213> Homo sapiens

<400> 2524
tcttcctgct ggcgcccttg atgct                                          25

<210> 2525
<211> 25
<212> DNA
<213> Homo sapiens

<400> 2525
cttcctgctg gcgcccttga tgctg                                          25

<210> 2526
<211> 25

<212> DNA
<213> Homo sapiens

<400> 2526
ttcctgctgg cgcccttgat gctga                                          25

<210> 2527
<211> 25
<212> DNA
<213> Homo sapiens

<400> 2527
tcctgctggc gcccttgatg ctgac                                          25

<210> 2528
<211> 25
<212> DNA
<213> Homo sapiens

<400> 2528
cctgctggcg cccttgatgc tgaca                                          25

<210> 2529
<211> 25
<212> DNA
<213> Homo sapiens

<400> 2529
ctgctggcgc ccttgatgct gacag                                          25

<210> 2530
<211> 25
<212> DNA
<213> Homo sapiens

<400> 2530
tgctggcgcc cttgatgctg acagc                                          25

<210> 2531
<211> 25
<212> DNA
<213> Homo sapiens

<400> 2531
gctggcgccc ttgatgctga cagcc                                          25

<210> 2532
<211> 25
<212> DNA
<213> Homo sapiens

<400> 2532
ctggcgccct tgatgctgac agccg                                          25

<210> 2533
<211> 25
<212> DNA
<213> Homo sapiens

<400> 2533
tggcgccctt gatgctgaca gccgc                                          25

<210> 2534
<211> 25
<212> DNA
<213> Homo sapiens

<400> 2534
ggcgcccttg atgctgacag ccgcg                                            25

<210> 2535
<211> 25
<212> DNA
<213> Homo sapiens

<400> 2535
gcgcccttga tgctgacagc cgcgc                                            25

<210> 2536
<211> 25
<212> DNA
<213> Homo sapiens

<400> 2536
cgcccttgat gctgacagcc gcgct                                            25

<210> 2537
<211> 25
<212> DNA
<213> Homo sapiens

<400> 2537
gcccttgatg ctgacagccg cgctg                                            25

<210> 2538
<211> 25
<212> DNA
<213> Homo sapiens

<400> 2538
cccttgatgc tgacagccgc gctgg                                            25

<210> 2539
<211> 25
<212> DNA
<213> Homo sapiens

<400> 2539
ccttgatgct gacagccgcg ctggg                                            25

<210> 2540
<211> 25
<212> DNA
<213> Homo sapiens

<400> 2540
cttgatgctg acagccgcgc tgggc                                            25

<210> 2541
<211> 25
<212> DNA
<213> Homo sapiens

<400> 2541
ttgatgctga cagccgcgct gggca                                          25

<210> 2542
<211> 25
<212> DNA
<213> Homo sapiens

<400> 2542
tgatgctgac agccgcgctg ggcac                                          25

<210> 2543
<211> 25
<212> DNA
<213> Homo sapiens

<400> 2543
gatgctgaca gccgcgctgg gcacc                                          25

<210> 2544
<211> 25
<212> DNA
<213> Homo sapiens

<400> 2544
atgctgacag ccgcgctggg caccg                                          25

<210> 2545
<211> 25
<212> DNA
<213> Homo sapiens

<400> 2545
tgctgacagc cgcgctgggc accgg                                          25

<210> 2546
<211> 25
<212> DNA
<213> Homo sapiens

<400> 2546
gctgacagcc gcgctgggca ccggc                                          25

<210> 2547
<211> 25
<212> DNA
<213> Homo sapiens

<400> 2547
ctgacagccg cgctgggcac cggct                                          25

<210> 2548
<211> 25
<212> DNA
<213> Homo sapiens

<400> 2548
tgacagccgc gctgggcacc ggctt                                          25

<210> 2549

<211> 25
<212> DNA
<213> Homo sapiens

<400> 2549
gacagccgcg ctgggcaccg gcttc                                          25

<210> 2550
<211> 25
<212> DNA
<213> Homo sapiens

<400> 2550
acagccgcgc tgggcaccgg cttcc                                          25

<210> 2551
<211> 25
<212> DNA
<213> Homo sapiens

<400> 2551
cagccgcgct gggcaccggc ttcct                                          25

<210> 2552
<211> 25
<212> DNA
<213> Homo sapiens

<400> 2552
agccgcgctg ggcaccggct tcctg                                          25

<210> 2553
<211> 25
<212> DNA
<213> Homo sapiens

<400> 2553
gccgcgctgg gcaccggctt cctgt                                          25

<210> 2554
<211> 25
<212> DNA
<213> Homo sapiens

<400> 2554
ccgcgctggg caccggcttc ctgta                                          25

<210> 2555
<211> 25
<212> DNA
<213> Homo sapiens

<400> 2555
cgcgctgggc accggcttcc tgtac                                          25

<210> 2556
<211> 25
<212> DNA
<213> Homo sapiens

<400> 2556

gcgctgggca ccggcttcct gtacc                                          25

<210> 2557
<211> 25
<212> DNA
<213> Homo sapiens

<400> 2557
cgctgggcac cggcttcctg tacct                                          25

<210> 2558
<211> 25
<212> DNA
<213> Homo sapiens

<400> 2558
gctgggcacc ggcttcctgt accta                                          25

<210> 2559
<211> 25
<212> DNA
<213> Homo sapiens

<400> 2559
ctgggcaccg gcttcctgta cctac                                          25

<210> 2560
<211> 25
<212> DNA
<213> Homo sapiens

<400> 2560
tgggcaccgg cttcctgtac ctacc                                          25

<210> 2561
<211> 25
<212> DNA
<213> Homo sapiens

<400> 2561
gggcaccggc ttcctgtacc taccc                                          25

<210> 2562
<211> 25
<212> DNA
<213> Homo sapiens

<400> 2562
ggcaccggct tcctgtacct accca                                          25

<210> 2563
<211> 25
<212> DNA
<213> Homo sapiens

<400> 2563
gcaccggctt cctgtaccta cccaa                                          25

<210> 2564
<211> 25
<212> DNA

<213> Homo sapiens

<400> 2564
caccggcttc ctgtacctac ccaag                                          25

<210> 2565
<211> 25
<212> DNA
<213> Homo sapiens

<400> 2565
accggcttcc tgtacctacc caagg                                          25

<210> 2566
<211> 25
<212> DNA
<213> Homo sapiens

<400> 2566
ccggcttcct gtacctaccc aagga                                          25

<210> 2567
<211> 25
<212> DNA
<213> Homo sapiens

<400> 2567
cggcttcctg tacctaccca aggac                                          25

<210> 2568
<211> 25
<212> DNA
<213> Homo sapiens

<400> 2568
ggcttcctgt acctacccaa ggacg                                          25

<210> 2569
<211> 25
<212> DNA
<213> Homo sapiens

<400> 2569
gcttcctgta cctacccaag gacga                                          25

<210> 2570
<211> 25
<212> DNA
<213> Homo sapiens

<400> 2570
cttcctgtac ctacccaagg acgaa                                          25

<210> 2571
<211> 25
<212> DNA
<213> Homo sapiens

<400> 2571
ttcctgtacc tacccaagga cgaag                                          25

<210> 2572
<211> 25
<212> DNA
<213> Homo sapiens

<400> 2572
tcctgtacct acccaaggac gaaga                                          25

<210> 2573
<211> 25
<212> DNA
<213> Homo sapiens

<400> 2573
cctgtaccta cccaaggacg aagag                                          25

<210> 2574
<211> 25
<212> DNA
<213> Homo sapiens

<400> 2574
ctgtacctac ccaaggacga agagg                                          25

<210> 2575
<211> 25
<212> DNA
<213> Homo sapiens

<400> 2575
tgtacctacc caaggacgaa gagga                                          25

<210> 2576
<211> 25
<212> DNA
<213> Homo sapiens

<400> 2576
gtacctaccc aaggacgaag aggaa                                          25

<210> 2577
<211> 25
<212> DNA
<213> Homo sapiens

<400> 2577
tacctaccca aggacgaaga ggaag                                          25

<210> 2578
<211> 25
<212> DNA
<213> Homo sapiens

<400> 2578
acctacccaa ggacgaagag gaaga                                          25

<210> 2579
<211> 25
<212> DNA
<213> Homo sapiens

<400> 2579
cctacccaag gacgaagagg aagac                                          25

<210> 2580
<211> 25
<212> DNA
<213> Homo sapiens

<400> 2580
ctacccaagg acgaagagga agacc                                          25

<210> 2581
<211> 25
<212> DNA
<213> Homo sapiens

<400> 2581
tacccaagga cgaagaggaa gacct                                          25

<210> 2582
<211> 25
<212> DNA
<213> Homo sapiens

<400> 2582
acccaaggac gaagaggaag accta                                          25

<210> 2583
<211> 25
<212> DNA
<213> Homo sapiens

<400> 2583
cccaaggacg aagaggaaga cctag                                          25

<210> 2584
<211> 25
<212> DNA
<213> Homo sapiens

<400> 2584
ccaaggacga agaggaagac ctaga                                          25

<210> 2585
<211> 25
<212> DNA
<213> Homo sapiens

<400> 2585
caaggacgaa gaggaagacc tagag                                          25

<210> 2586
<211> 25
<212> DNA
<213> Homo sapiens

<400> 2586
aaggacgaag aggaagacct agagg                                          25

<210> 2587
<211> 25

<212> DNA
<213> Homo sapiens

<400> 2587
aggacgaaga ggaagaccta gagga                                          25

<210> 2588
<211> 25
<212> DNA
<213> Homo sapiens

<400> 2588
ggacgaagag gaagacctag aggag                                          25

<210> 2589
<211> 25
<212> DNA
<213> Homo sapiens

<400> 2589
gacgaagagg aagacctaga ggagc                                          25

<210> 2590
<211> 25
<212> DNA
<213> Homo sapiens

<400> 2590
acgaagagga agacctagag gagca                                          25

<210> 2591
<211> 25
<212> DNA
<213> Homo sapiens

<400> 2591
cgaagaggaa gacctagagg agcat                                          25

<210> 2592
<211> 25
<212> DNA
<213> Homo sapiens

<400> 2592
gaagaggaag acctagagga gcatt                                          25

<210> 2593
<211> 25
<212> DNA
<213> Homo sapiens

<400> 2593
aagaggaaga cctagaggag catta                                          25

<210> 2594
<211> 25
<212> DNA
<213> Homo sapiens

<400> 2594
agaggaagac ctagaggagc attac                                          25

<210> 2595
<211> 25
<212> DNA
<213> Homo sapiens

<400> 2595
gaggaagacc tagaggagca ttaca                                          25

<210> 2596
<211> 25
<212> DNA
<213> Homo sapiens

<400> 2596
aggaagacct agaggagcat tacac                                          25

<210> 2597
<211> 25
<212> DNA
<213> Homo sapiens

<400> 2597
ggaagaccta gaggagcatt acacc                                          25

<210> 2598
<211> 25
<212> DNA
<213> Homo sapiens

<400> 2598
gaagacctag aggagcatta caccc                                          25

<210> 2599
<211> 25
<212> DNA
<213> Homo sapiens

<400> 2599
aagacctaga ggagcattac acccc                                          25

<210> 2600
<211> 25
<212> DNA
<213> Homo sapiens

<400> 2600
agacctagag gagcattaca cccct                                          25

<210> 2601
<211> 25
<212> DNA
<213> Homo sapiens

<400> 2601
gacctagagg agcattacac ccctg                                          25

<210> 2602
<211> 25
<212> DNA
<213> Homo sapiens

<400> 2602
acctagagga gcattacacc cctgt                                          25

<210> 2603
<211> 25
<212> DNA
<213> Homo sapiens

<400> 2603
cctagaggag cattacaccc ctgtg                                          25

<210> 2604
<211> 25
<212> DNA
<213> Homo sapiens

<400> 2604
ctagaggagc attacacccc tgtgg                                          25

<210> 2605
<211> 25
<212> DNA
<213> Homo sapiens

<400> 2605
tagaggagca ttacacccct gtggg                                          25

<210> 2606
<211> 25
<212> DNA
<213> Homo sapiens

<400> 2606
agaggagcat tacacccctg tgggg                                          25

<210> 2607
<211> 25
<212> DNA
<213> Homo sapiens

<400> 2607
gaggagcatt acacccctgt gggga                                          25

<210> 2608
<211> 25
<212> DNA
<213> Homo sapiens

<400> 2608
aggagcatta cacccctgtg gggag                                          25

<210> 2609
<211> 25
<212> DNA
<213> Homo sapiens

<400> 2609
ggagcattac acccctgtgg ggagc                                          25

<210> 2610

<211> 25
<212> DNA
<213> Homo sapiens

<400> 2610
gagcattaca cccctgtggg gagcc                                          25

<210> 2611
<211> 25
<212> DNA
<213> Homo sapiens

<400> 2611
agcattacac ccctgtgggg agccc                                          25

<210> 2612
<211> 25
<212> DNA
<213> Homo sapiens

<400> 2612
gcattacacc cctgtgggga gcccg                                          25

<210> 2613
<211> 25
<212> DNA
<213> Homo sapiens

<400> 2613
cattacaccc ctgtggggag cccgg                                          25

<210> 2614
<211> 25
<212> DNA
<213> Homo sapiens

<400> 2614
attacacccc tgtggggagc ccggc                                          25

<210> 2615
<211> 25
<212> DNA
<213> Homo sapiens

<400> 2615
ttacacccct gtggggagcc cggcc                                          25

<210> 2616
<211> 25
<212> DNA
<213> Homo sapiens

<400> 2616
tacacccctg tggggagccc ggcca                                          25

<210> 2617
<211> 25
<212> DNA
<213> Homo sapiens

<400> 2617

acacccctgt ggggagcccg gccaa 25

<210> 2618
<211> 25
<212> DNA
<213> Homo sapiens

<400> 2618
cacccctgtg gggagcccgg ccaag 25

<210> 2619
<211> 25
<212> DNA
<213> Homo sapiens

<400> 2619
acccctgtgg ggagcccggc caagg 25

<210> 2620
<211> 25
<212> DNA
<213> Homo sapiens

<400> 2620
cccctgtggg gagcccggcc aaggc 25

<210> 2621
<211> 25
<212> DNA
<213> Homo sapiens

<400> 2621
ccctgtgggg agcccggcca aggcg 25

<210> 2622
<211> 25
<212> DNA
<213> Homo sapiens

<400> 2622
cctgtgggga gcccggccaa ggcgg 25

<210> 2623
<211> 25
<212> DNA
<213> Homo sapiens

<400> 2623
ctgtggggag cccggccaag gcgga 25

<210> 2624
<211> 25
<212> DNA
<213> Homo sapiens

<400> 2624
tgtggggagc ccggccaagg cggag 25

<210> 2625
<211> 25
<212> DNA

<213> Homo sapiens

<400> 2625
gtggggagcc cggccaaggc ggagc                                          25

<210> 2626
<211> 25
<212> DNA
<213> Homo sapiens

<400> 2626
tggggagccc ggccaaggcg gagcg                                          25

<210> 2627
<211> 25
<212> DNA
<213> Homo sapiens

<400> 2627
ggggagcccg gccaaggcgg agcgg                                          25

<210> 2628
<211> 25
<212> DNA
<213> Homo sapiens

<400> 2628
gggagcccgg ccaaggcgga gcggc                                          25

<210> 2629
<211> 25
<212> DNA
<213> Homo sapiens

<400> 2629
ggagcccggc caaggcggag cggcg                                          25

<210> 2630
<211> 25
<212> DNA
<213> Homo sapiens

<400> 2630
gagcccggcc aaggcggagc ggcgc                                          25

<210> 2631
<211> 25
<212> DNA
<213> Homo sapiens

<400> 2631
agcccggcca aggcggagcg gcgct                                          25

<210> 2632
<211> 25
<212> DNA
<213> Homo sapiens

<400> 2632
gcccggccaa ggcggagcgg cgctt                                          25

<210> 2633
<211> 25
<212> DNA
<213> Homo sapiens

<400> 2633
cccggccaag gcggagcggc gcttc                                          25

<210> 2634
<211> 25
<212> DNA
<213> Homo sapiens

<400> 2634
ccggccaagg cggagcggcg cttcg                                          25

<210> 2635
<211> 25
<212> DNA
<213> Homo sapiens

<400> 2635
cggccaaggc ggagcggcgc ttcgt                                          25

<210> 2636
<211> 25
<212> DNA
<213> Homo sapiens

<400> 2636
ggccaaggcg gagcggcgct tcgtg                                          25

<210> 2637
<211> 25
<212> DNA
<213> Homo sapiens

<400> 2637
gccaaggcgg agcggcgctt cgtgc                                          25

<210> 2638
<211> 25
<212> DNA
<213> Homo sapiens

<400> 2638
ccaaggcgga gcggcgcttc gtgca                                          25

<210> 2639
<211> 25
<212> DNA
<213> Homo sapiens

<400> 2639
caaggcggag cggcgcttcg tgcag                                          25

<210> 2640
<211> 25
<212> DNA
<213> Homo sapiens

<400> 2640
aaggcggagc ggcgcttcgt gcagg                                          25

<210> 2641
<211> 25
<212> DNA
<213> Homo sapiens

<400> 2641
aggcggagcg gcgcttcgtg caggg                                          25

<210> 2642
<211> 25
<212> DNA
<213> Homo sapiens

<400> 2642
ggcggagcgg cgcttcgtgc agggc                                          25

<210> 2643
<211> 25
<212> DNA
<213> Homo sapiens

<400> 2643
gcggagcggc gcttcgtgca gggcc                                          25

<210> 2644
<211> 25
<212> DNA
<213> Homo sapiens

<400> 2644
cggagcggcg cttcgtgcag ggcca                                          25

<210> 2645
<211> 25
<212> DNA
<213> Homo sapiens

<400> 2645
ggagcggcgc ttcgtgcagg gccat                                          25

<210> 2646
<211> 25
<212> DNA
<213> Homo sapiens

<400> 2646
gagcggcgct tcgtgcaggg ccatt                                          25

<210> 2647
<211> 25
<212> DNA
<213> Homo sapiens

<400> 2647
agcggcgctt cgtgcagggc cattt                                          25

<210> 2648
<211> 25

<212> DNA
<213> Homo sapiens

<400> 2648
gcggcgcttc gtgcagggcc atttc                                          25

<210> 2649
<211> 25
<212> DNA
<213> Homo sapiens

<400> 2649
cggcgcttcg tgcagggcca tttca                                          25

<210> 2650
<211> 25
<212> DNA
<213> Homo sapiens

<400> 2650
ggcgcttcgt gcagggccat ttcac                                          25

<210> 2651
<211> 25
<212> DNA
<213> Homo sapiens

<400> 2651
gcgcttcgtg cagggccatt tcacc                                          25

<210> 2652
<211> 25
<212> DNA
<213> Homo sapiens

<400> 2652
cgcttcgtgc agggccattt cacca                                          25

<210> 2653
<211> 25
<212> DNA
<213> Homo sapiens

<400> 2653
gcttcgtgca gggccatttc accac                                          25

<210> 2654
<211> 25
<212> DNA
<213> Homo sapiens

<400> 2654
cttcgtgcag ggccatttca ccacc                                          25

<210> 2655
<211> 25
<212> DNA
<213> Homo sapiens

<400> 2655
ttcgtgcagg gccatttcac cacca                                          25

<210> 2656
<211> 25
<212> DNA
<213> Homo sapiens

<400> 2656
tcgtgcaggg ccatttcacc accaa                                          25

<210> 2657
<211> 25
<212> DNA
<213> Homo sapiens

<400> 2657
cgtgcagggc catttcacca ccaac                                          25

<210> 2658
<211> 25
<212> DNA
<213> Homo sapiens

<400> 2658
gtgcagggcc atttcaccac caacg                                          25

<210> 2659
<211> 25
<212> DNA
<213> Homo sapiens

<400> 2659
tgcagggcca tttcaccacc aacga                                          25

<210> 2660
<211> 25
<212> DNA
<213> Homo sapiens

<400> 2660
gcagggccat ttcaccacca acgac                                          25

<210> 2661
<211> 25
<212> DNA
<213> Homo sapiens

<400> 2661
cagggccatt tcaccaccaa cgact                                          25

<210> 2662
<211> 25
<212> DNA
<213> Homo sapiens

<400> 2662
agggccattt caccaccaac gactc                                          25

<210> 2663
<211> 25
<212> DNA
<213> Homo sapiens

<400> 2663
gggccatttc accaccaacg actcc                                          25

<210> 2664
<211> 25
<212> DNA
<213> Homo sapiens

<400> 2664
ggccatttca ccaccaacga ctcct                                          25

<210> 2665
<211> 25
<212> DNA
<213> Homo sapiens

<400> 2665
gccatttcac caccaacgac tccta                                          25

<210> 2666
<211> 25
<212> DNA
<213> Homo sapiens

<400> 2666
ccatttcacc accaacgact cctac                                          25

<210> 2667
<211> 25
<212> DNA
<213> Homo sapiens

<400> 2667
catttcacca ccaacgactc ctacc                                          25

<210> 2668
<211> 25
<212> DNA
<213> Homo sapiens

<400> 2668
atttcaccac caacgactcc taccg                                          25

<210> 2669
<211> 25
<212> DNA
<213> Homo sapiens

<400> 2669
tttcaccacc aacgactcct accgc                                          25

<210> 2670
<211> 25
<212> DNA
<213> Homo sapiens

<400> 2670
ttcaccacca acgactccta ccgct                                          25

<210> 2671

<211> 25
<212> DNA
<213> Homo sapiens

<400> 2671
tcaccaccaa cgactcctac cgctt                                            25

<210> 2672
<211> 25
<212> DNA
<213> Homo sapiens

<400> 2672
caccaccaac gactcctacc gcttc                                            25

<210> 2673
<211> 25
<212> DNA
<213> Homo sapiens

<400> 2673
accaccaacg actcctaccg cttct                                            25

<210> 2674
<211> 25
<212> DNA
<213> Homo sapiens

<400> 2674
ccaccaacga ctcctaccgc ttctc                                            25

<210> 2675
<211> 25
<212> DNA
<213> Homo sapiens

<400> 2675
caccaacgac tcctaccgct tctcc                                            25

<210> 2676
<211> 25
<212> DNA
<213> Homo sapiens

<400> 2676
accaacgact cctaccgctt ctccg                                            25

<210> 2677
<211> 25
<212> DNA
<213> Homo sapiens

<400> 2677
ccaacgactc ctaccgcttc tccgc                                            25

<210> 2678
<211> 25
<212> DNA
<213> Homo sapiens

<400> 2678

caacgactcc taccgcttct ccgcc                                          25

<210> 2679
<211> 25
<212> DNA
<213> Homo sapiens

<400> 2679
aacgactcct accgcttctc cgcct                                          25

<210> 2680
<211> 25
<212> DNA
<213> Homo sapiens

<400> 2680
acgactccta ccgcttctcc gcctc                                          25

<210> 2681
<211> 25
<212> DNA
<213> Homo sapiens

<400> 2681
cgactcctac cgcttctccg cctcc                                          25

<210> 2682
<211> 25
<212> DNA
<213> Homo sapiens

<400> 2682
gactcctacc gcttctccgc ctcca                                          25

<210> 2683
<211> 25
<212> DNA
<213> Homo sapiens

<400> 2683
actcctaccg cttctccgcc tccag                                          25

<210> 2684
<211> 25
<212> DNA
<213> Homo sapiens

<400> 2684
ctcctaccgc ttctccgcct ccagg                                          25

<210> 2685
<211> 25
<212> DNA
<213> Homo sapiens

<400> 2685
tcctaccgct tctccgcctc cagga                                          25

<210> 2686
<211> 25
<212> DNA

<213> Homo sapiens

<400> 2686
cctaccgctt ctccgcctcc aggag                                          25

<210> 2687
<211> 25
<212> DNA
<213> Homo sapiens

<400> 2687
ctaccgcttc tccgcctcca ggagg                                          25

<210> 2688
<211> 25
<212> DNA
<213> Homo sapiens

<400> 2688
taccgcttct ccgcctccag gagga                                          25

<210> 2689
<211> 25
<212> DNA
<213> Homo sapiens

<400> 2689
accgcttctc cgcctccagg aggag                                          25

<210> 2690
<211> 25
<212> DNA
<213> Homo sapiens

<400> 2690
ccgcttctcc gcctccagga ggagc                                          25

<210> 2691
<211> 25
<212> DNA
<213> Homo sapiens

<400> 2691
cgcttctccg cctccaggag gagca                                          25

<210> 2692
<211> 25
<212> DNA
<213> Homo sapiens

<400> 2692
gcttctccgc ctccaggagg agcac                                          25

<210> 2693
<211> 25
<212> DNA
<213> Homo sapiens

<400> 2693
cttctccgcc tccaggagga gcacc                                          25

<210> 2694
<211> 25
<212> DNA
<213> Homo sapiens

<400> 2694
ttctccgcct ccaggaggag caccg                                          25

<210> 2695
<211> 25
<212> DNA
<213> Homo sapiens

<400> 2695
tctccgcctc caggaggagc accga                                          25

<210> 2696
<211> 25
<212> DNA
<213> Homo sapiens

<400> 2696
ctccgcctcc aggaggagca ccgaa                                          25

<210> 2697
<211> 25
<212> DNA
<213> Homo sapiens

<400> 2697
tccgcctcca ggaggagcac cgaag                                          25

<210> 2698
<211> 25
<212> DNA
<213> Homo sapiens

<400> 2698
ccgcctccag gaggagcacc gaagc                                          25

<210> 2699
<211> 25
<212> DNA
<213> Homo sapiens

<400> 2699
cgcctccagg aggagcaccg aagcc                                          25

<210> 2700
<211> 25
<212> DNA
<213> Homo sapiens

<400> 2700
gcctccagga ggagcaccga agcca                                          25

<210> 2701
<211> 25
<212> DNA
<213> Homo sapiens

<400> 2701
cctccaggag gagcaccgaa gccaa                                          25

<210> 2702
<211> 25
<212> DNA
<213> Homo sapiens

<400> 2702
ctccaggagg agcaccgaag ccaat                                          25

<210> 2703
<211> 25
<212> DNA
<213> Homo sapiens

<400> 2703
tccaggagga gcaccgaagc caatt                                          25

<210> 2704
<211> 25
<212> DNA
<213> Homo sapiens

<400> 2704
ccaggaggag caccgaagcc aattt                                          25

<210> 2705
<211> 25
<212> DNA
<213> Homo sapiens

<400> 2705
caggaggagc accgaagcca atttc                                          25

<210> 2706
<211> 25
<212> DNA
<213> Homo sapiens

<400> 2706
aggaggagca ccgaagccaa tttcg                                          25

<210> 2707
<211> 25
<212> DNA
<213> Homo sapiens

<400> 2707
ggaggagcac cgaagccaat ttcgt                                          25

<210> 2708
<211> 25
<212> DNA
<213> Homo sapiens

<400> 2708
gaggagcacc gaagccaatt tcgtc                                          25

<210> 2709
<211> 25

<212> DNA
<213> Homo sapiens

<400> 2709
aggagcaccg aagccaattt cgtct                                            25

<210> 2710
<211> 25
<212> DNA
<213> Homo sapiens

<400> 2710
ggagcaccga agccaatttc gtctc                                            25

<210> 2711
<211> 25
<212> DNA
<213> Homo sapiens

<400> 2711
gagcaccgaa gccaatttcg tctcg                                            25

<210> 2712
<211> 25
<212> DNA
<213> Homo sapiens

<400> 2712
agcaccgaag ccaatttcgt ctcgc                                            25

<210> 2713
<211> 25
<212> DNA
<213> Homo sapiens

<400> 2713
gcaccgaagc caatttcgtc tcgct                                            25

<210> 2714
<211> 25
<212> DNA
<213> Homo sapiens

<400> 2714
caccgaagcc aatttcgtct cgctt                                            25

<210> 2715
<211> 25
<212> DNA
<213> Homo sapiens

<400> 2715
accgaagcca atttcgtctc gcttc                                            25

<210> 2716
<211> 25
<212> DNA
<213> Homo sapiens

<400> 2716
ccgaagccaa tttcgtctcg cttct                                            25

<210> 2717
<211> 25
<212> DNA
<213> Homo sapiens

<400> 2717
cgaagccaat ttcgtctcgc ttctg                                          25

<210> 2718
<211> 25
<212> DNA
<213> Homo sapiens

<400> 2718
gaagccaatt tcgtctcgct tctgg                                          25

<210> 2719
<211> 25
<212> DNA
<213> Homo sapiens

<400> 2719
aagccaattt cgtctcgctt ctggt                                          25

<210> 2720
<211> 25
<212> DNA
<213> Homo sapiens

<400> 2720
agccaatttc gtctcgcttc tggtg                                          25

<210> 2721
<211> 25
<212> DNA
<213> Homo sapiens

<400> 2721
gccaatttcg tctcgcttct ggtgg                                          25

<210> 2722
<211> 25
<212> DNA
<213> Homo sapiens

<400> 2722
ccaatttcgt ctcgcttctg gtggt                                          25

<210> 2723
<211> 25
<212> DNA
<213> Homo sapiens

<400> 2723
caatttcgtc tcgcttctgg tggtc                                          25

<210> 2724
<211> 25
<212> DNA
<213> Homo sapiens

<400> 2724
aatttcgtct cgcttctggt ggtct                                          25

<210> 2725
<211> 25
<212> DNA
<213> Homo sapiens

<400> 2725
atttcgtctc gcttctggtg gtctc                                          25

<210> 2726
<211> 25
<212> DNA
<213> Homo sapiens

<400> 2726
tttcgtctcg cttctggtgg tctcc                                          25

<210> 2727
<211> 25
<212> DNA
<213> Homo sapiens

<400> 2727
ttcgtctcgc ttctggtggt ctcct                                          25

<210> 2728
<211> 25
<212> DNA
<213> Homo sapiens

<400> 2728
tcgtctcgct tctggtggtc tccta                                          25

<210> 2729
<211> 25
<212> DNA
<213> Homo sapiens

<400> 2729
cgtctcgctt ctggtggtct cctac                                          25

<210> 2730
<211> 25
<212> DNA
<213> Homo sapiens

<400> 2730
gtctcgcttc tggtggtctc ctaca                                          25

<210> 2731
<211> 25
<212> DNA
<213> Homo sapiens

<400> 2731
tctcgcttct ggtggtctcc tacag                                          25

<210> 2732

<211> 25
<212> DNA
<213> Homo sapiens

<400> 2732
ctcgcttctg gtggtctcct acagc                                          25

<210> 2733
<211> 25
<212> DNA
<213> Homo sapiens

<400> 2733
tcgcttctgg tggtctccta cagcg                                          25

<210> 2734
<211> 25
<212> DNA
<213> Homo sapiens

<400> 2734
cgcttctggt ggtctcctac agcga                                          25

<210> 2735
<211> 25
<212> DNA
<213> Homo sapiens

<400> 2735
gcttctggtg gtctcctaca gcgac                                          25

<210> 2736
<211> 25
<212> DNA
<213> Homo sapiens

<400> 2736
cttctggtgg tctcctacag cgact                                          25

<210> 2737
<211> 25
<212> DNA
<213> Homo sapiens

<400> 2737
ttctggtggt ctcctacagc gactc                                          25

<210> 2738
<211> 25
<212> DNA
<213> Homo sapiens

<400> 2738
tctggtggtc tcctacagcg actca                                          25

<210> 2739
<211> 25
<212> DNA
<213> Homo sapiens

<400> 2739

ctggtggtct cctacagcga ctcac                                          25

<210> 2740
<211> 25
<212> DNA
<213> Homo sapiens

<400> 2740
tggtggtctc ctacagcgac tcact                                          25

<210> 2741
<211> 25
<212> DNA
<213> Homo sapiens

<400> 2741
ggtggtctcc tacagcgact cactg                                          25

<210> 2742
<211> 25
<212> DNA
<213> Homo sapiens

<400> 2742
gtggtctcct acagcgactc actgc                                          25

<210> 2743
<211> 25
<212> DNA
<213> Homo sapiens

<400> 2743
tggtctccta cagcgactca ctgct                                          25

<210> 2744
<211> 25
<212> DNA
<213> Homo sapiens

<400> 2744
ggtctcctac agcgactcac tgctg                                          25

<210> 2745
<211> 25
<212> DNA
<213> Homo sapiens

<400> 2745
gtctcctaca gcgactcact gctgg                                          25

<210> 2746
<211> 25
<212> DNA
<213> Homo sapiens

<400> 2746
tctcctacag cgactcactg ctgga                                          25

<210> 2747
<211> 25
<212> DNA

<213> Homo sapiens

<400> 2747
ctcctacagc gactcactgc tggac                                          25

<210> 2748
<211> 25
<212> DNA
<213> Homo sapiens

<400> 2748
tcctacagcg actcactgct ggacc                                          25

<210> 2749
<211> 25
<212> DNA
<213> Homo sapiens

<400> 2749
cctacagcga ctcactgctg gaccc                                          25

<210> 2750
<211> 25
<212> DNA
<213> Homo sapiens

<400> 2750
ctacagcgac tcactgctgg accca                                          25

<210> 2751
<211> 25
<212> DNA
<213> Homo sapiens

<400> 2751
tacagcgact cactgctgga cccag                                          25

<210> 2752
<211> 25
<212> DNA
<213> Homo sapiens

<400> 2752
acagcgactc actgctggac ccagc                                          25

<210> 2753
<211> 25
<212> DNA
<213> Homo sapiens

<400> 2753
cagcgactca ctgctggacc cagct                                          25

<210> 2754
<211> 25
<212> DNA
<213> Homo sapiens

<400> 2754
agcgactcac tgctggaccc agcta                                          25

<210> 2755
<211> 25
<212> DNA
<213> Homo sapiens

<400> 2755
gcgactcact gctggaccca gctac                                          25

<210> 2756
<211> 25
<212> DNA
<213> Homo sapiens

<400> 2756
cgactcactg ctggacccag ctacc                                          25

<210> 2757
<211> 25
<212> DNA
<213> Homo sapiens

<400> 2757
gactcactgc tggacccagc tacct                                          25

<210> 2758
<211> 25
<212> DNA
<213> Homo sapiens

<400> 2758
actcactgct ggacccagct acctt                                          25

<210> 2759
<211> 25
<212> DNA
<213> Homo sapiens

<400> 2759
ctcactgctg gacccagcta ccttt                                          25

<210> 2760
<211> 25
<212> DNA
<213> Homo sapiens

<400> 2760
tcactgctgg acccagctac ctttg                                          25

<210> 2761
<211> 25
<212> DNA
<213> Homo sapiens

<400> 2761
cactgctgga cccagctacc tttgc                                          25

<210> 2762
<211> 25
<212> DNA
<213> Homo sapiens

<400> 2762
actgctggac ccagctacct ttgca                                          25

<210> 2763
<211> 25
<212> DNA
<213> Homo sapiens

<400> 2763
ctgctggacc cagctacctt tgcag                                          25

<210> 2764
<211> 25
<212> DNA
<213> Homo sapiens

<400> 2764
tgctggaccc agctaccttt gcaga                                          25

<210> 2765
<211> 25
<212> DNA
<213> Homo sapiens

<400> 2765
gctggaccca gctacctttg cagaa                                          25

<210> 2766
<211> 25
<212> DNA
<213> Homo sapiens

<400> 2766
ctggacccag ctacctttgc agaag                                          25

<210> 2767
<211> 25
<212> DNA
<213> Homo sapiens

<400> 2767
tggacccagc tacctttgca gaagt                                          25

<210> 2768
<211> 25
<212> DNA
<213> Homo sapiens

<400> 2768
ggacccagct acctttgcag aagtc                                          25

<210> 2769
<211> 25
<212> DNA
<213> Homo sapiens

<400> 2769
gacccagcta cctttgcaga agtca                                          25

<210> 2770
<211> 25

<212> DNA
<213> Homo sapiens

<400> 2770
acccagctac ctttgcagaa gtcag                                          25

<210> 2771
<211> 25
<212> DNA
<213> Homo sapiens

<400> 2771
cccagctacc tttgcagaag tcagc                                          25

<210> 2772
<211> 25
<212> DNA
<213> Homo sapiens

<400> 2772
ccagctacct ttgcagaagt cagca                                          25

<210> 2773
<211> 25
<212> DNA
<213> Homo sapiens

<400> 2773
cagctacctt tgcagaagtc agcaa                                          25

<210> 2774
<211> 25
<212> DNA
<213> Homo sapiens

<400> 2774
agctaccttt gcagaagtca gcaaa                                          25

<210> 2775
<211> 25
<212> DNA
<213> Homo sapiens

<400> 2775
gctacctttg cagaagtcag caaac                                          25

<210> 2776
<211> 25
<212> DNA
<213> Homo sapiens

<400> 2776
ctacctttgc agaagtcagc aaact                                          25

<210> 2777
<211> 25
<212> DNA
<213> Homo sapiens

<400> 2777
tacctttgca gaagtcagca aactg                                          25

<210> 2778
<211> 25
<212> DNA
<213> Homo sapiens

<400> 2778
acctttgcag aagtcagcaa actgg                                             25

<210> 2779
<211> 25
<212> DNA
<213> Homo sapiens

<400> 2779
cctttgcaga agtcagcaaa ctgga                                             25

<210> 2780
<211> 25
<212> DNA
<213> Homo sapiens

<400> 2780
ctttgcagaa gtcagcaaac tggac                                             25

<210> 2781
<211> 25
<212> DNA
<213> Homo sapiens

<400> 2781
tttgcagaag tcagcaaact ggacg                                             25

<210> 2782
<211> 25
<212> DNA
<213> Homo sapiens

<400> 2782
ttgcagaagt cagcaaactg gacgg                                             25

<210> 2783
<211> 25
<212> DNA
<213> Homo sapiens

<400> 2783
tgcagaagtc agcaaactgg acggc                                             25

<210> 2784
<211> 25
<212> DNA
<213> Homo sapiens

<400> 2784
gcagaagtca gcaaactgga cggcg                                             25

<210> 2785
<211> 25
<212> DNA
<213> Homo sapiens

<400> 2785
cagaagtcag caaactggac ggcgc                                          25

<210> 2786
<211> 25
<212> DNA
<213> Homo sapiens

<400> 2786
agaagtcagc aaactggacg gcgcg                                          25

<210> 2787
<211> 25
<212> DNA
<213> Homo sapiens

<400> 2787
gaagtcagca aactggacgg cgcgg                                          25

<210> 2788
<211> 25
<212> DNA
<213> Homo sapiens

<400> 2788
aagtcagcaa actggacggc gcggt                                          25

<210> 2789
<211> 25
<212> DNA
<213> Homo sapiens

<400> 2789
agtcagcaaa ctggacggcg cggtg                                          25

<210> 2790
<211> 25
<212> DNA
<213> Homo sapiens

<400> 2790
gtcagcaaac tggacggcgc ggtgc                                          25

<210> 2791
<211> 25
<212> DNA
<213> Homo sapiens

<400> 2791
tcagcaaact ggacggcgcg gtgca                                          25

<210> 2792
<211> 25
<212> DNA
<213> Homo sapiens

<400> 2792
cagcaaactg gacggcgcgg tgcag                                          25

<210> 2793

<211> 25
<212> DNA
<213> Homo sapiens

<400> 2793
agcaaactgg acggcgcggt gcagg                                          25

<210> 2794
<211> 25
<212> DNA
<213> Homo sapiens

<400> 2794
gcaaactgga cggcgcggtg cagga                                          25

<210> 2795
<211> 25
<212> DNA
<213> Homo sapiens

<400> 2795
caaactggac ggcgcggtgc aggat                                          25

<210> 2796
<211> 25
<212> DNA
<213> Homo sapiens

<400> 2796
aaactggacg gcgcggtgca ggatc                                          25

<210> 2797
<211> 25
<212> DNA
<213> Homo sapiens

<400> 2797
aactggacgg cgcggtgcag gatct                                          25

<210> 2798
<211> 25
<212> DNA
<213> Homo sapiens

<400> 2798
actggacggc gcggtgcagg atctg                                          25

<210> 2799
<211> 25
<212> DNA
<213> Homo sapiens

<400> 2799
ctggacggcg cggtgcagga tctgc                                          25

<210> 2800
<211> 25
<212> DNA
<213> Homo sapiens

<400> 2800

tggacggcgc ggtgcaggat ctgcg                                          25

<210> 2801
<211> 25
<212> DNA
<213> Homo sapiens

<400> 2801
ggacggcgcg gtgcaggatc tgcgc                                          25

<210> 2802
<211> 25
<212> DNA
<213> Homo sapiens

<400> 2802
gacggcgcgg tgcaggatct gcgcg                                          25

<210> 2803
<211> 25
<212> DNA
<213> Homo sapiens

<400> 2803
acggcgcggt gcaggatctg cgcgt                                          25

<210> 2804
<211> 25
<212> DNA
<213> Homo sapiens

<400> 2804
cggcgcggtg caggatctgc gcgtg                                          25

<210> 2805
<211> 25
<212> DNA
<213> Homo sapiens

<400> 2805
ggcgcggtgc aggatctgcg cgtgg                                          25

<210> 2806
<211> 25
<212> DNA
<213> Homo sapiens

<400> 2806
gcgcggtgca ggatctgcgc gtggc                                          25

<210> 2807
<211> 25
<212> DNA
<213> Homo sapiens

<400> 2807
cgcggtgcag gatctgcgcg tggcg                                          25

<210> 2808
<211> 25
<212> DNA

<213> Homo sapiens

<400> 2808
gcggtgcagg atctgcgcgt ggcgc                                          25

<210> 2809
<211> 25
<212> DNA
<213> Homo sapiens

<400> 2809
cggtgcagga tctgcgcgtg gcgcg                                          25

<210> 2810
<211> 25
<212> DNA
<213> Homo sapiens

<400> 2810
ggtgcaggat ctgcgcgtgg cgcgg                                          25

<210> 2811
<211> 25
<212> DNA
<213> Homo sapiens

<400> 2811
gtgcaggatc tgcgcgtggc gcggg                                          25

<210> 2812
<211> 25
<212> DNA
<213> Homo sapiens

<400> 2812
tgcaggatct gcgcgtggcg cggga                                          25

<210> 2813
<211> 25
<212> DNA
<213> Homo sapiens

<400> 2813
gcaggatctg cgcgtggcgc gggaa                                          25

<210> 2814
<211> 25
<212> DNA
<213> Homo sapiens

<400> 2814
caggatctgc gcgtggcgcg ggaaa                                          25

<210> 2815
<211> 25
<212> DNA
<213> Homo sapiens

<400> 2815
aggatctgcg cgtggcgcgg gaaaa                                          25

<210> 2816
<211> 25
<212> DNA
<213> Homo sapiens

<400> 2816
ggatctgcgc gtggcgcggg aaaag                                              25

<210> 2817
<211> 25
<212> DNA
<213> Homo sapiens

<400> 2817
gatctgcgcg tggcgcggga aaagg                                              25

<210> 2818
<211> 25
<212> DNA
<213> Homo sapiens

<400> 2818
atctgcgcgt ggcgcgggaa aaggg                                              25

<210> 2819
<211> 25
<212> DNA
<213> Homo sapiens

<400> 2819
tctgcgcgtg gcgcgggaaa aggga                                              25

<210> 2820
<211> 25
<212> DNA
<213> Homo sapiens

<400> 2820
ctgcgcgtgg cgcgggaaaa gggaa                                              25

<210> 2821
<211> 25
<212> DNA
<213> Homo sapiens

<400> 2821
tgcgcgtggc gcgggaaaag ggaag                                              25

<210> 2822
<211> 25
<212> DNA
<213> Homo sapiens

<400> 2822
gcgcgtggcg cgggaaaagg gaagc                                              25

<210> 2823
<211> 25
<212> DNA
<213> Homo sapiens

<400> 2823
cgcgtggcgc gggaaaaggg aagcc                                          25

<210> 2824
<211> 25
<212> DNA
<213> Homo sapiens

<400> 2824
gcgtggcgcg ggaaaaggga agcca                                          25

<210> 2825
<211> 25
<212> DNA
<213> Homo sapiens

<400> 2825
cgtggcgcgg gaaaagggaa gccag                                          25

<210> 2826
<211> 25
<212> DNA
<213> Homo sapiens

<400> 2826
gtggcgcggg aaaagggaag ccaga                                          25

<210> 2827
<211> 25
<212> DNA
<213> Homo sapiens

<400> 2827
tggcgcggga aaagggaagc cagat                                          25

<210> 2828
<211> 25
<212> DNA
<213> Homo sapiens

<400> 2828
ggcgcgggaa aagggaagcc agatc                                          25

<210> 2829
<211> 25
<212> DNA
<213> Homo sapiens

<400> 2829
gcgcgggaaa agggaagcca gatcc                                          25

<210> 2830
<211> 25
<212> DNA
<213> Homo sapiens

<400> 2830
cgcgggaaaa gggaagccag atcca                                          25

<210> 2831
<211> 25

<212> DNA
<213> Homo sapiens

<400> 2831
gcgggaaaag ggaagccaga tccag                                          25

<210> 2832
<211> 25
<212> DNA
<213> Homo sapiens

<400> 2832
cgggaaaagg gaagccagat ccagt                                          25

<210> 2833
<211> 25
<212> DNA
<213> Homo sapiens

<400> 2833
gggaaaaggg aagccagatc cagta                                          25

<210> 2834
<211> 25
<212> DNA
<213> Homo sapiens

<400> 2834
ggaaaaggga agccagatcc agtac                                          25

<210> 2835
<211> 25
<212> DNA
<213> Homo sapiens

<400> 2835
gaaaagggaa gccagatcca gtacc                                          25

<210> 2836
<211> 25
<212> DNA
<213> Homo sapiens

<400> 2836
aaaagggaag ccagatccag tacca                                          25

<210> 2837
<211> 25
<212> DNA
<213> Homo sapiens

<400> 2837
aaagggaagc cagatccagt accag                                          25

<210> 2838
<211> 25
<212> DNA
<213> Homo sapiens

<400> 2838
aagggaagcc agatccagta ccagc                                          25

<210> 2839
<211> 25
<212> DNA
<213> Homo sapiens

<400> 2839
agggaagcca gatccagtac cagca                                          25

<210> 2840
<211> 25
<212> DNA
<213> Homo sapiens

<400> 2840
gggaagccag atccagtacc agcag                                          25

<210> 2841
<211> 25
<212> DNA
<213> Homo sapiens

<400> 2841
ggaagccaga tccagtacca gcagg                                          25

<210> 2842
<211> 25
<212> DNA
<213> Homo sapiens

<400> 2842
gaagccagat ccagtaccag caggt                                          25

<210> 2843
<211> 25
<212> DNA
<213> Homo sapiens

<400> 2843
aagccagatc cagtaccagc aggtg                                          25

<210> 2844
<211> 25
<212> DNA
<213> Homo sapiens

<400> 2844
agccagatcc agtaccagca ggtgt                                          25

<210> 2845
<211> 25
<212> DNA
<213> Homo sapiens

<400> 2845
gccagatcca gtaccagcag gtgtg                                          25

<210> 2846
<211> 25
<212> DNA
<213> Homo sapiens

<400> 2846
ccagatccag taccagcagg tgtgc                                          25

<210> 2847
<211> 25
<212> DNA
<213> Homo sapiens

<400> 2847
cagatccagt accagcaggt gtgcg                                          25

<210> 2848
<211> 25
<212> DNA
<213> Homo sapiens

<400> 2848
agatccagta ccagcaggtg tgcgc                                          25

<210> 2849
<211> 25
<212> DNA
<213> Homo sapiens

<400> 2849
gatccagtac cagcaggtgt gcgcg                                          25

<210> 2850
<211> 25
<212> DNA
<213> Homo sapiens

<400> 2850
atccagtacc agcaggtgtg cgcga                                          25

<210> 2851
<211> 25
<212> DNA
<213> Homo sapiens

<400> 2851
tccagtacca gcaggtgtgc gcgag                                          25

<210> 2852
<211> 25
<212> DNA
<213> Homo sapiens

<400> 2852
ccagtaccag caggtgtgcg cgagg                                          25

<210> 2853
<211> 25
<212> DNA
<213> Homo sapiens

<400> 2853
cagtaccagc aggtgtgcgc gaggt                                          25

<210> 2854

<211> 25
<212> DNA
<213> Homo sapiens

<400> 2854
agtaccagca ggtgtgcgcg aggta                                            25

<210> 2855
<211> 25
<212> DNA
<213> Homo sapiens

<400> 2855
gtaccagcag gtgtgcgcga ggtac                                            25

<210> 2856
<211> 25
<212> DNA
<213> Homo sapiens

<400> 2856
taccagcagg tgtgcgcgag gtaca                                            25

<210> 2857
<211> 25
<212> DNA
<213> Homo sapiens

<400> 2857
accagcaggt gtgcgcgagg tacag                                            25

<210> 2858
<211> 25
<212> DNA
<213> Homo sapiens

<400> 2858
ccagcaggtg tgcgcgaggt acagg                                            25

<210> 2859
<211> 25
<212> DNA
<213> Homo sapiens

<400> 2859
cagcaggtgt gcgcgaggta caggg                                            25

<210> 2860
<211> 25
<212> DNA
<213> Homo sapiens

<400> 2860
agcaggtgtg cgcgaggtac agggc                                            25

<210> 2861
<211> 25
<212> DNA
<213> Homo sapiens

<400> 2861

gcaggtgtgc gcgaggtaca gggcg                                          25

<210> 2862
<211> 25
<212> DNA
<213> Homo sapiens

<400> 2862
caggtgtgcg cgaggtacag ggcgc                                          25

<210> 2863
<211> 25
<212> DNA
<213> Homo sapiens

<400> 2863
aggtgtgcgc gaggtacagg gcgct                                          25

<210> 2864
<211> 25
<212> DNA
<213> Homo sapiens

<400> 2864
ggtgtgcgcg aggtacaggg cgctc                                          25

<210> 2865
<211> 25
<212> DNA
<213> Homo sapiens

<400> 2865
gtgtgcgcga ggtacagggc gctct                                          25

<210> 2866
<211> 25
<212> DNA
<213> Homo sapiens

<400> 2866
tgtgcgcgag gtacagggcg ctctg                                          25

<210> 2867
<211> 25
<212> DNA
<213> Homo sapiens

<400> 2867
gtgcgcgagg tacagggcgc tctgc                                          25

<210> 2868
<211> 25
<212> DNA
<213> Homo sapiens

<400> 2868
tgcgcgaggt acagggcgct ctgcg                                          25

<210> 2869
<211> 25
<212> DNA

<213> Homo sapiens

<400> 2869
gcgcgaggta cagggcgctc tgcgt                                            25

<210> 2870
<211> 25
<212> DNA
<213> Homo sapiens

<400> 2870
cgcgaggtac agggcgctct gcgtg                                            25

<210> 2871
<211> 25
<212> DNA
<213> Homo sapiens

<400> 2871
gcgaggtaca gggcgctctg cgtgc                                            25

<210> 2872
<211> 25
<212> DNA
<213> Homo sapiens

<400> 2872
cgaggtacag ggcgctctgc gtgcc                                            25

<210> 2873
<211> 25
<212> DNA
<213> Homo sapiens

<400> 2873
gaggtacagg gcgctctgcg tgccc                                            25

<210> 2874
<211> 25
<212> DNA
<213> Homo sapiens

<400> 2874
aggtacaggg cgctctgcgt gcccc                                            25

<210> 2875
<211> 25
<212> DNA
<213> Homo sapiens

<400> 2875
ggtacagggc gctctgcgtg ccccc                                            25

<210> 2876
<211> 25
<212> DNA
<213> Homo sapiens

<400> 2876
gtacagggcg ctctgcgtgc ccccc                                            25

<210> 2877
<211> 25
<212> DNA
<213> Homo sapiens

<400> 2877
tacagggcgc tctgcgtgcc cccca                                          25

<210> 2878
<211> 25
<212> DNA
<213> Homo sapiens

<400> 2878
acagggcgct ctgcgtgccc cccaa                                          25

<210> 2879
<211> 25
<212> DNA
<213> Homo sapiens

<400> 2879
cagggcgctc tgcgtgcccc ccaac                                          25

<210> 2880
<211> 25
<212> DNA
<213> Homo sapiens

<400> 2880
agggcgctct gcgtgccccc caacc                                          25

<210> 2881
<211> 25
<212> DNA
<213> Homo sapiens

<400> 2881
gggcgctctg cgtgcccccc aaccc                                          25

<210> 2882
<211> 25
<212> DNA
<213> Homo sapiens

<400> 2882
ggcgctctgc gtgcccccca acccg                                          25

<210> 2883
<211> 25
<212> DNA
<213> Homo sapiens

<400> 2883
gcgctctgcg tgccccccaa cccga                                          25

<210> 2884
<211> 25
<212> DNA
<213> Homo sapiens

<400> 2884
cgctctgcgt gccccccaac ccgat                                          25

<210> 2885
<211> 25
<212> DNA
<213> Homo sapiens

<400> 2885
gctctgcgtg ccccccaacc cgatc                                          25

<210> 2886
<211> 25
<212> DNA
<213> Homo sapiens

<400> 2886
ctctgcgtgc cccccaaccc gatcc                                          25

<210> 2887
<211> 25
<212> DNA
<213> Homo sapiens

<400> 2887
tctgcgtgcc ccccaacccg atcct                                          25

<210> 2888
<211> 25
<212> DNA
<213> Homo sapiens

<400> 2888
ctgcgtgccc cccaacccga tcctg                                          25

<210> 2889
<211> 25
<212> DNA
<213> Homo sapiens

<400> 2889
tgcgtgcccc ccaacccgat cctgt                                          25

<210> 2890
<211> 25
<212> DNA
<213> Homo sapiens

<400> 2890
gcgtgccccc caacccgatc ctgta                                          25

<210> 2891
<211> 25
<212> DNA
<213> Homo sapiens

<400> 2891
cgtgcccccc aacccgatcc tgtac                                          25

<210> 2892
<211> 25

<212> DNA
<213> Homo sapiens

<400> 2892
gtgcccccca acccgatcct gtacg                                          25

<210> 2893
<211> 25
<212> DNA
<213> Homo sapiens

<400> 2893
tgccccccaa cccgatcctg tacgc                                          25

<210> 2894
<211> 25
<212> DNA
<213> Homo sapiens

<400> 2894
gccccccaac ccgatcctgt acgcc                                          25

<210> 2895
<211> 25
<212> DNA
<213> Homo sapiens

<400> 2895
ccccccaacc cgatcctgta cgcct                                          25

<210> 2896
<211> 25
<212> DNA
<213> Homo sapiens

<400> 2896
cccccaaccc gatcctgtac gcctg                                          25

<210> 2897
<211> 25
<212> DNA
<213> Homo sapiens

<400> 2897
ccccaacccg atcctgtacg cctgg                                          25

<210> 2898
<211> 25
<212> DNA
<213> Homo sapiens

<400> 2898
cccaacccga tcctgtacgc ctggc                                          25

<210> 2899
<211> 25
<212> DNA
<213> Homo sapiens

<400> 2899
ccaacccgat cctgtacgcc tggca                                          25

<210> 2900
<211> 25
<212> DNA
<213> Homo sapiens

<400> 2900
caacccgatc ctgtacgcct ggcag                                          25

<210> 2901
<211> 25
<212> DNA
<213> Homo sapiens

<400> 2901
aacccgatcc tgtacgcctg gcagg                                          25

<210> 2902
<211> 25
<212> DNA
<213> Homo sapiens

<400> 2902
acccgatcct gtacgcctgg caggt                                          25

<210> 2903
<211> 25
<212> DNA
<213> Homo sapiens

<400> 2903
cccgatcctg tacgcctggc aggtg                                          25

<210> 2904
<211> 25
<212> DNA
<213> Homo sapiens

<400> 2904
ccgatcctgt acgcctggca ggtga                                          25

<210> 2905
<211> 25
<212> DNA
<213> Homo sapiens

<400> 2905
cgatcctgta cgcctggcag gtgaa                                          25

<210> 2906
<211> 25
<212> DNA
<213> Homo sapiens

<400> 2906
gatcctgtac gcctggcagg tgaac                                          25

<210> 2907
<211> 25
<212> DNA
<213> Homo sapiens

<400> 2907
atcctgtacg cctggcaggt gaaca                                          25

<210> 2908
<211> 25
<212> DNA
<213> Homo sapiens

<400> 2908
tcctgtacgc ctggcaggtg aacaa                                          25

<210> 2909
<211> 25
<212> DNA
<213> Homo sapiens

<400> 2909
cctgtacgcc tggcaggtga acaaa                                          25

<210> 2910
<211> 25
<212> DNA
<213> Homo sapiens

<400> 2910
ctgtacgcct ggcaggtgaa caaaa                                          25

<210> 2911
<211> 25
<212> DNA
<213> Homo sapiens

<400> 2911
tgtacgcctg gcaggtgaac aaaac                                          25

<210> 2912
<211> 25
<212> DNA
<213> Homo sapiens

<400> 2912
gtacgcctgg caggtgaaca aaacg                                          25

<210> 2913
<211> 25
<212> DNA
<213> Homo sapiens

<400> 2913
tacgcctggc aggtgaacaa aacgc                                          25

<210> 2914
<211> 25
<212> DNA
<213> Homo sapiens

<400> 2914
acgcctggca ggtgaacaaa acgct                                          25

<210> 2915

<211> 25
<212> DNA
<213> Homo sapiens

<400> 2915
cgcctggcag gtgaacaaaa cgctc                                          25

<210> 2916
<211> 25
<212> DNA
<213> Homo sapiens

<400> 2916
gcctggcagg tgaacaaaac gctca                                          25

<210> 2917
<211> 25
<212> DNA
<213> Homo sapiens

<400> 2917
cctggcaggt gaacaaaacg ctcaa                                          25

<210> 2918
<211> 25
<212> DNA
<213> Homo sapiens

<400> 2918
ctggcaggtg aacaaaacgc tcaac                                          25

<210> 2919
<211> 25
<212> DNA
<213> Homo sapiens

<400> 2919
tggcaggtga acaaaacgct caacc                                          25

<210> 2920
<211> 25
<212> DNA
<213> Homo sapiens

<400> 2920
ggcaggtgaa caaaacgctc aacct                                          25

<210> 2921
<211> 25
<212> DNA
<213> Homo sapiens

<400> 2921
gcaggtgaac aaaacgctca acctg                                          25

<210> 2922
<211> 25
<212> DNA
<213> Homo sapiens

<400> 2922

caggtgaaca aaacgctcaa cctga                                          25

<210> 2923
<211> 25
<212> DNA
<213> Homo sapiens

<400> 2923
aggtgaacaa aacgctcaac ctgag                                          25

<210> 2924
<211> 25
<212> DNA
<213> Homo sapiens

<400> 2924
ggtgaacaaa acgctcaacc tgagc                                          25

<210> 2925
<211> 25
<212> DNA
<213> Homo sapiens

<400> 2925
gtgaacaaaa cgctcaacct gagca                                          25

<210> 2926
<211> 25
<212> DNA
<213> Homo sapiens

<400> 2926
tgaacaaaac gctcaacctg agcag                                          25

<210> 2927
<211> 25
<212> DNA
<213> Homo sapiens

<400> 2927
gaacaaaacg ctcaacctga gcagc                                          25

<210> 2928
<211> 25
<212> DNA
<213> Homo sapiens

<400> 2928
aacaaaacgc tcaacctgag cagca                                          25

<210> 2929
<211> 25
<212> DNA
<213> Homo sapiens

<400> 2929
acaaaacgct caacctgagc agcat                                          25

<210> 2930
<211> 25
<212> DNA

<213> Homo sapiens

<400> 2930
caaaacgctc aacctgagca gcatc                                          25

<210> 2931
<211> 25
<212> DNA
<213> Homo sapiens

<400> 2931
aaaacgctca acctgagcag catct                                          25

<210> 2932
<211> 25
<212> DNA
<213> Homo sapiens

<400> 2932
aaacgctcaa cctgagcagc atctc                                          25

<210> 2933
<211> 25
<212> DNA
<213> Homo sapiens

<400> 2933
aacgctcaac ctgagcagca tctcc                                          25

<210> 2934
<211> 25
<212> DNA
<213> Homo sapiens

<400> 2934
acgctcaacc tgagcagcat ctcct                                          25

<210> 2935
<211> 25
<212> DNA
<213> Homo sapiens

<400> 2935
cgctcaacct gagcagcatc tcctt                                          25

<210> 2936
<211> 25
<212> DNA
<213> Homo sapiens

<400> 2936
gctcaacctg agcagcatct ccttc                                          25

<210> 2937
<211> 25
<212> DNA
<213> Homo sapiens

<400> 2937
ctcaacctga gcagcatctc cttcc                                          25

<210> 2938
<211> 25
<212> DNA
<213> Homo sapiens

<400> 2938
tcaacctgag cagcatctcc ttccc                                          25

<210> 2939
<211> 25
<212> DNA
<213> Homo sapiens

<400> 2939
caacctgagc agcatctcct tcccc                                          25

<210> 2940
<211> 25
<212> DNA
<213> Homo sapiens

<400> 2940
aacctgagca gcatctcctt ccccg                                          25

<210> 2941
<211> 25
<212> DNA
<213> Homo sapiens

<400> 2941
acctgagcag catctccttc cccgc                                          25

<210> 2942
<211> 25
<212> DNA
<213> Homo sapiens

<400> 2942
cctgagcagc atctccttcc ccgcc                                          25

<210> 2943
<211> 25
<212> DNA
<213> Homo sapiens

<400> 2943
ctgagcagca tctccttccc cgcct                                          25

<210> 2944
<211> 25
<212> DNA
<213> Homo sapiens

<400> 2944
tgagcagcat ctccttcccc gccta                                          25

<210> 2945
<211> 25
<212> DNA
<213> Homo sapiens

<400> 2945
gagcagcatc tccttccccg cctac                                          25

<210> 2946
<211> 25
<212> DNA
<213> Homo sapiens

<400> 2946
agcagcatct ccttccccgc ctaca                                          25

<210> 2947
<211> 25
<212> DNA
<213> Homo sapiens

<400> 2947
gcagcatctc cttccccgcc tacaa                                          25

<210> 2948
<211> 25
<212> DNA
<213> Homo sapiens

<400> 2948
cagcatctcc ttccccgcct acaac                                          25

<210> 2949
<211> 25
<212> DNA
<213> Homo sapiens

<400> 2949
agcatctcct tccccgccta caacc                                          25

<210> 2950
<211> 25
<212> DNA
<213> Homo sapiens

<400> 2950
gcatctcctt ccccgcctac aacca                                          25

<210> 2951
<211> 25
<212> DNA
<213> Homo sapiens

<400> 2951
catctccttc cccgcctaca accac                                          25

<210> 2952
<211> 25
<212> DNA
<213> Homo sapiens

<400> 2952
atctccttcc ccgcctacaa ccacg                                          25

<210> 2953
<211> 25

<212> DNA
<213> Homo sapiens

<400> 2953
tctccttccc cgcctacaac cacgg                                            25

<210> 2954
<211> 25
<212> DNA
<213> Homo sapiens

<400> 2954
ctccttcccc gcctacaacc acggc                                            25

<210> 2955
<211> 25
<212> DNA
<213> Homo sapiens

<400> 2955
tccttccccg cctacaacca cggca                                            25

<210> 2956
<211> 25
<212> DNA
<213> Homo sapiens

<400> 2956
ccttccccgc ctacaaccac ggcag                                            25

<210> 2957
<211> 25
<212> DNA
<213> Homo sapiens

<400> 2957
cttccccgcc tacaaccacg gcagg                                            25

<210> 2958
<211> 25
<212> DNA
<213> Homo sapiens

<400> 2958
ttccccgcct acaaccacgg caggc                                            25

<210> 2959
<211> 25
<212> DNA
<213> Homo sapiens

<400> 2959
tccccgccta caaccacggc aggca                                            25

<210> 2960
<211> 25
<212> DNA
<213> Homo sapiens

<400> 2960
ccccgcctac aaccacggca ggcat                                            25

<210> 2961
<211> 25
<212> DNA
<213> Homo sapiens

<400> 2961
cccgcctaca accacggcag gcatc                                          25

<210> 2962
<211> 25
<212> DNA
<213> Homo sapiens

<400> 2962
ccgcctacaa ccacggcagg catcc                                          25

<210> 2963
<211> 25
<212> DNA
<213> Homo sapiens

<400> 2963
cgcctacaac cacggcaggc atccc                                          25

<210> 2964
<211> 25
<212> DNA
<213> Homo sapiens

<400> 2964
gcctacaacc acggcaggca tcccc                                          25

<210> 2965
<211> 25
<212> DNA
<213> Homo sapiens

<400> 2965
cctacaacca cggcaggcat cccct                                          25

<210> 2966
<211> 25
<212> DNA
<213> Homo sapiens

<400> 2966
ctacaaccac ggcaggcatc ccctc                                          25

<210> 2967
<211> 25
<212> DNA
<213> Homo sapiens

<400> 2967
tacaaccacg gcaggcatcc cctct                                          25

<210> 2968
<211> 25
<212> DNA
<213> Homo sapiens

<400> 2968
acaaccacgg caggcatccc ctcta                                          25

<210> 2969
<211> 25
<212> DNA
<213> Homo sapiens

<400> 2969
caaccacggc aggcatcccc tctac                                          25

<210> 2970
<211> 25
<212> DNA
<213> Homo sapiens

<400> 2970
aaccacggca ggcatcccct ctacc                                          25

<210> 2971
<211> 25
<212> DNA
<213> Homo sapiens

<400> 2971
accacggcag gcatcccctc tacct                                          25

<210> 2972
<211> 25
<212> DNA
<213> Homo sapiens

<400> 2972
ccacggcagg catcccctct acctg                                          25

<210> 2973
<211> 25
<212> DNA
<213> Homo sapiens

<400> 2973
cacggcaggc atcccctcta cctga                                          25

<210> 2974
<211> 25
<212> DNA
<213> Homo sapiens

<400> 2974
acggcaggca tcccctctac ctgac                                          25

<210> 2975
<211> 25
<212> DNA
<213> Homo sapiens

<400> 2975
cggcaggcat cccctctacc tgacc                                          25

<210> 2976

<211> 25
<212> DNA
<213> Homo sapiens

<400> 2976
ggcaggcatc ccctctacct gaccg                                            25

<210> 2977
<211> 25
<212> DNA
<213> Homo sapiens

<400> 2977
gcaggcatcc cctctacctg accgg                                            25

<210> 2978
<211> 25
<212> DNA
<213> Homo sapiens

<400> 2978
caggcatccc ctctacctga ccggc                                            25

<210> 2979
<211> 25
<212> DNA
<213> Homo sapiens

<400> 2979
aggcatcccc tctacctgac cggct                                            25

<210> 2980
<211> 25
<212> DNA
<213> Homo sapiens

<400> 2980
ggcatcccct ctacctgacc ggctt                                            25

<210> 2981
<211> 25
<212> DNA
<213> Homo sapiens

<400> 2981
gcatcccctc tacctgaccg gcttc                                            25

<210> 2982
<211> 25
<212> DNA
<213> Homo sapiens

<400> 2982
catcccctct acctgaccgg cttct                                            25

<210> 2983
<211> 25
<212> DNA
<213> Homo sapiens

<400> 2983

atcccctcta cctgaccggc ttctt                                          25

<210> 2984
<211> 25
<212> DNA
<213> Homo sapiens

<400> 2984
tcccctctac ctgaccggct tcttc                                          25

<210> 2985
<211> 25
<212> DNA
<213> Homo sapiens

<400> 2985
cccctctacc tgaccggctt cttcg                                          25

<210> 2986
<211> 25
<212> DNA
<213> Homo sapiens

<400> 2986
ccctctacct gaccggcttc ttcgg                                          25

<210> 2987
<211> 25
<212> DNA
<213> Homo sapiens

<400> 2987
cctctacctg accggcttct tcgga                                          25

<210> 2988
<211> 25
<212> DNA
<213> Homo sapiens

<400> 2988
ctctacctga ccggcttctt cggag                                          25

<210> 2989
<211> 25
<212> DNA
<213> Homo sapiens

<400> 2989
tctacctgac cggcttcttc ggagg                                          25

<210> 2990
<211> 25
<212> DNA
<213> Homo sapiens

<400> 2990
ctacctgacc ggcttcttcg gagga                                          25

<210> 2991
<211> 25
<212> DNA

<213> Homo sapiens

<400> 2991
tacctgaccg gcttcttcgg aggat                                          25

<210> 2992
<211> 25
<212> DNA
<213> Homo sapiens

<400> 2992
acctgaccgg cttcttcgga ggata                                          25

<210> 2993
<211> 25
<212> DNA
<213> Homo sapiens

<400> 2993
cctgaccggc ttcttcggag gatac                                          25

<210> 2994
<211> 25
<212> DNA
<213> Homo sapiens

<400> 2994
ctgaccggct tcttcggagg ataca                                          25

<210> 2995
<211> 25
<212> DNA
<213> Homo sapiens

<400> 2995
tgaccggctt cttcggagga tacat                                          25

<210> 2996
<211> 25
<212> DNA
<213> Homo sapiens

<400> 2996
gaccggcttc ttcggaggat acatc                                          25

<210> 2997
<211> 25
<212> DNA
<213> Homo sapiens

<400> 2997
accggcttct tcggaggata catct                                          25

<210> 2998
<211> 25
<212> DNA
<213> Homo sapiens

<400> 2998
ccggcttctt cggaggatac atctt                                          25

<210> 2999
<211> 25
<212> DNA
<213> Homo sapiens

<400> 2999
cggcttcttc ggaggataca tcttg                                             25

<210> 3000
<211> 25
<212> DNA
<213> Homo sapiens

<400> 3000
ggcttcttcg gaggatacat cttgg                                             25

<210> 3001
<211> 25
<212> DNA
<213> Homo sapiens

<400> 3001
gcttcttcgg aggatacatc ttggg                                             25

<210> 3002
<211> 25
<212> DNA
<213> Homo sapiens

<400> 3002
cttcttcgga ggatacatct tgggg                                             25

<210> 3003
<211> 25
<212> DNA
<213> Homo sapiens

<400> 3003
ttcttcggag gatacatctt ggggg                                             25

<210> 3004
<211> 25
<212> DNA
<213> Homo sapiens

<400> 3004
tcttcggagg atacatcttg ggggg                                             25

<210> 3005
<211> 25
<212> DNA
<213> Homo sapiens

<400> 3005
cttcggagga tacatcttgg ggggc                                             25

<210> 3006
<211> 25
<212> DNA
<213> Homo sapiens

<400> 3006
ttcggaggat acatcttggg gggca                                          25

<210> 3007
<211> 25
<212> DNA
<213> Homo sapiens

<400> 3007
tcggaggata catcttgggg ggcag                                          25

<210> 3008
<211> 25
<212> DNA
<213> Homo sapiens

<400> 3008
cggaggatac atcttggggg gcagc                                          25

<210> 3009
<211> 25
<212> DNA
<213> Homo sapiens

<400> 3009
ggaggataca tcttgggggg cagcc                                          25

<210> 3010
<211> 25
<212> DNA
<213> Homo sapiens

<400> 3010
   gaggatacat cttggggggc agcct                                           25

<210> 3011
<211> 25
<212> DNA
<213> Homo sapiens

<400> 3011
   aggatacatc ttggggggca gccta                                           25

<210> 3012
<211> 25
<212> DNA
<213> Homo sapiens

<400> 3012
   ggatacatct tggggggcag cctag                                           25

<210> 3013
<211> 25
<212> DNA
<213> Homo sapiens

<400> 3013
   gatacatctt ggggggcagc ctagg                                           25

<210> 3014
<211> 25

<212> DNA
<213> Homo sapiens

<400> 3014
atacatcttg gggggcagcc tagga                                              25

<210> 3015
<211> 25
<212> DNA
<213> Homo sapiens

<400> 3015
tacatcttgg ggggcagcct aggaa                                              25

<210> 3016
<211> 25
<212> DNA
<213> Homo sapiens

<400> 3016
acatcttggg gggcagccta ggaat                                              25

<210> 3017
<211> 25
<212> DNA
<213> Homo sapiens

<400> 3017
catcttgggg ggcagcctag gaatg                                              25

<210> 3018
<211> 25
<212> DNA
<213> Homo sapiens

<400> 3018
atcttggggg gcagcctagg aatgg                                              25

<210> 3019
<211> 25
<212> DNA
<213> Homo sapiens

<400> 3019
tcttgggggg cagcctagga atggg                                              25

<210> 3020
<211> 25
<212> DNA
<213> Homo sapiens

<400> 3020
cttggggggc agcctaggaa tgggc                                              25

<210> 3021
<211> 25
<212> DNA
<213> Homo sapiens

<400> 3021
ttggggggca gcctaggaat gggcc                                              25

<210> 3022
<211> 25
<212> DNA
<213> Homo sapiens

<400> 3022
   tggggggcag cctaggaatg ggcca                                              25

<210> 3023
<211> 25
<212> DNA
<213> Homo sapiens

<400> 3023
   ggggggcagc ctaggaatgg gccag                                              25

<210> 3024
<211> 25
<212> DNA
<213> Homo sapiens

<400> 3024
gggggcagcc taggaatggg ccagt                                             25

<210> 3025
<211> 25
<212> DNA
<213> Homo sapiens

<400> 3025
ggggcagcct aggaatgggc cagtt                                             25

<210> 3026
<211> 25
<212> DNA
<213> Homo sapiens

<400> 3026
gggcagccta ggaatgggcc agtta                                             25

<210> 3027
<211> 25
<212> DNA
<213> Homo sapiens

<400> 3027
ggcagcctag gaatgggcca gttac                                             25

<210> 3028
<211> 25
<212> DNA
<213> Homo sapiens

<400> 3028
gcagcctagg aatgggccag ttact                                             25

<210> 3029
<211> 25
<212> DNA
<213> Homo sapiens

<400> 3029
cagcctagga atgggccagt tactc                                          25

<210> 3030
<211> 25
<212> DNA
<213> Homo sapiens

<400> 3030
agcctaggaa tgggccagtt actcc                                          25

<210> 3031
<211> 25
<212> DNA
<213> Homo sapiens

<400> 3031
gcctaggaat gggccagtta ctcct                                          25

<210> 3032
<211> 25
<212> DNA
<213> Homo sapiens

<400> 3032
cctaggaatg ggccagttac tcctg                                          25

<210> 3033
<211> 25
<212> DNA
<213> Homo sapiens

<400> 3033
ctaggaatgg gccagttact cctgc                                          25

<210> 3034
<211> 25
<212> DNA
<213> Homo sapiens

<400> 3034
taggaatggg ccagttactc ctgcg                                          25

<210> 3035
<211> 25
<212> DNA
<213> Homo sapiens

<400> 3035
aggaatgggc cagttactcc tgcgg                                          25

<210> 3036
<211> 25
<212> DNA
<213> Homo sapiens

<400> 3036
ggaatgggcc agttactcct gcggg                                          25

<210> 3037

<211> 25
<212> DNA
<213> Homo sapiens

<400> 3037
gaatgggcca gttactcctg cgggc                                          25

<210> 3038
<211> 25
<212> DNA
<213> Homo sapiens

<400> 3038
aatgggccag ttactcctgc gggcc                                          25

<210> 3039
<211> 25
<212> DNA
<213> Homo sapiens

<400> 3039
atgggccagt tactcctgcg ggcca                                          25

<210> 3040
<211> 25
<212> DNA
<213> Homo sapiens

<400> 3040
tgggccagtt actcctgcgg gccaa                                          25

<210> 3041
<211> 25
<212> DNA
<213> Homo sapiens

<400> 3041
gggccagtta ctcctgcggg ccaaa                                          25

<210> 3042
<211> 25
<212> DNA
<213> Homo sapiens

<400> 3042
ggccagttac tcctgcgggc caaag                                          25

<210> 3043
<211> 25
<212> DNA
<213> Homo sapiens

<400> 3043
gccagttact cctgcgggcc aaagc                                          25

<210> 3044
<211> 25
<212> DNA
<213> Homo sapiens

<400> 3044

ccagttactc ctgcgggcca aagcc 25

<210> 3045
<211> 25
<212> DNA
<213> Homo sapiens

<400> 3045
cagttactcc tgcgggccaa agcca 25

<210> 3046
<211> 25
<212> DNA
<213> Homo sapiens

<400> 3046
agttactcct gcgggccaaa gccat 25

<210> 3047
<211> 25
<212> DNA
<213> Homo sapiens

<400> 3047
gttactcctg cgggccaaag ccatg 25

<210> 3048
<211> 25
<212> DNA
<213> Homo sapiens

<400> 3048
ttactcctgc gggccaaagc catgc 25

<210> 3049
<211> 25
<212> DNA
<213> Homo sapiens

<400> 3049
tactcctgcg ggccaaagcc atgcg 25

<210> 3050
<211> 25
<212> DNA
<213> Homo sapiens

<400> 3050
actcctgcgg gccaaagcca tgcgg 25

<210> 3051
<211> 25
<212> DNA
<213> Homo sapiens

<400> 3051
ctcctgcggg ccaaagccat gcggc 25

<210> 3052
<211> 25
<212> DNA

<213> Homo sapiens

<400> 3052
tcctgcgggc caaagccatg cggct                                          25

<210> 3053
<211> 25
<212> DNA
<213> Homo sapiens

<400> 3053
cctgcgggcc aaagccatgc ggctg                                          25

<210> 3054
<211> 25
<212> DNA
<213> Homo sapiens

<400> 3054
ctgcgggcca aagccatgcg gctgc                                          25

<210> 3055
<211> 25
<212> DNA
<213> Homo sapiens

<400> 3055
tgcgggccaa agccatgcgg ctgct                                          25

<210> 3056
<211> 25
<212> DNA
<213> Homo sapiens

<400> 3056
gcgggccaaa gccatgcggc tgctg                                          25

<210> 3057
<211> 25
<212> DNA
<213> Homo sapiens

<400> 3057
cgggccaaag ccatgcggct gctgt                                          25

<210> 3058
<211> 25
<212> DNA
<213> Homo sapiens

<400> 3058
gggccaaagc catgcggctg ctgta                                          25

<210> 3059
<211> 25
<212> DNA
<213> Homo sapiens

<400> 3059
ggccaaagcc atgcggctgc tgtac                                          25

<210> 3060
<211> 25
<212> DNA
<213> Homo sapiens

<400> 3060
gccaaagcca tgcggctgct gtact                                          25

<210> 3061
<211> 25
<212> DNA
<213> Homo sapiens

<400> 3061
ccaaagccat gcggctgctg tacta                                          25

<210> 3062
<211> 25
<212> DNA
<213> Homo sapiens

<400> 3062
caaagccatg cggctgctgt actac                                          25

<210> 3063
<211> 25
<212> DNA
<213> Homo sapiens

<400> 3063
aaagccatgc ggctgctgta ctacc                                          25

<210> 3064
<211> 25
<212> DNA
<213> Homo sapiens

<400> 3064
aagccatgcg gctgctgtac tacct                                          25

<210> 3065
<211> 25
<212> DNA
<213> Homo sapiens

<400> 3065
agccatgcgg ctgctgtact acctg                                          25

<210> 3066
<211> 25
<212> DNA
<213> Homo sapiens

<400> 3066
gccatgcggc tgctgtacta cctga                                          25

<210> 3067
<211> 25
<212> DNA
<213> Homo sapiens

<400> 3067
ccatgcggct gctgtactac ctgaa                                          25

<210> 3068
<211> 25
<212> DNA
<213> Homo sapiens

<400> 3068
catgcggctg ctgtactacc tgaag                                          25

<210> 3069
<211> 25
<212> DNA
<213> Homo sapiens

<400> 3069
atgcggctgc tgtactacct gaaga                                          25

<210> 3070
<211> 25
<212> DNA
<213> Homo sapiens

<400> 3070
tgcggctgct gtactacctg aagac                                          25

<210> 3071
<211> 25
<212> DNA
<213> Homo sapiens

<400> 3071
gcggctgctg tactacctga agacc                                          25

<210> 3072
<211> 25
<212> DNA
<213> Homo sapiens

<400> 3072
cggctgctgt actacctgaa gaccg                                          25

<210> 3073
<211> 25
<212> DNA
<213> Homo sapiens

<400> 3073
ggctgctgta ctacctgaag accga                                          25

<210> 3074
<211> 25
<212> DNA
<213> Homo sapiens

<400> 3074
gctgctgtac tacctgaaga ccgag                                          25

<210> 3075
<211> 25

<212> DNA
<213> Homo sapiens

<400> 3075
ctgctgtact acctgaagac cgagg                                          25

<210> 3076
<211> 25
<212> DNA
<213> Homo sapiens

<400> 3076
tgctgtacta cctgaagacc gagga                                          25

<210> 3077
<211> 25
<212> DNA
<213> Homo sapiens

<400> 3077
gctgtactac ctgaagaccg aggac                                          25

<210> 3078
<211> 25
<212> DNA
<213> Homo sapiens

<400> 3078
ctgtactacc tgaagaccga ggacc                                          25

<210> 3079
<211> 25
<212> DNA
<213> Homo sapiens

<400> 3079
tgtactacct gaagaccgag gaccc                                          25

<210> 3080
<211> 25
<212> DNA
<213> Homo sapiens

<400> 3080
gtactacctg aagaccgagg accct                                          25

<210> 3081
<211> 25
<212> DNA
<213> Homo sapiens

<400> 3081
tactacctga agaccgagga ccctg                                          25

<210> 3082
<211> 25
<212> DNA
<213> Homo sapiens

<400> 3082
actacctgaa gaccgaggac cctga                                          25

<210> 3083
<211> 25
<212> DNA
<213> Homo sapiens

<400> 3083
ctacctgaag accgaggacc ctgag                                            25

<210> 3084
<211> 25
<212> DNA
<213> Homo sapiens

<400> 3084
tacctgaaga ccgaggaccc tgagt                                            25

<210> 3085
<211> 25
<212> DNA
<213> Homo sapiens

<400> 3085
acctgaagac cgaggaccct gagta                                            25

<210> 3086
<211> 25
<212> DNA
<213> Homo sapiens

<400> 3086
cctgaagacc gaggaccctg agtac                                            25

<210> 3087
<211> 25
<212> DNA
<213> Homo sapiens

<400> 3087
ctgaagaccg aggaccctga gtacg                                            25

<210> 3088
<211> 25
<212> DNA
<213> Homo sapiens

<400> 3088
tgaagaccga ggaccctgag tacga                                            25

<210> 3089
<211> 25
<212> DNA
<213> Homo sapiens

<400> 3089
gaagaccgag gaccctgagt acgac                                            25

<210> 3090
<211> 25
<212> DNA
<213> Homo sapiens

<400> 3090
aagaccgagg accctgagta cgacg                                          25

<210> 3091
<211> 25
<212> DNA
<213> Homo sapiens

<400> 3091
agaccgagga ccctgagtac gacgt                                          25

<210> 3092
<211> 25
<212> DNA
<213> Homo sapiens

<400> 3092
gaccgaggac cctgagtacg acgtg                                          25

<210> 3093
<211> 25
<212> DNA
<213> Homo sapiens

<400> 3093
accgaggacc ctgagtacga cgtgc                                          25

<210> 3094
<211> 25
<212> DNA
<213> Homo sapiens

<400> 3094
ccgaggaccc tgagtacgac gtgca                                          25

<210> 3095
<211> 25
<212> DNA
<213> Homo sapiens

<400> 3095
cgaggaccct gagtacgacg tgcag                                          25

<210> 3096
<211> 25
<212> DNA
<213> Homo sapiens

<400> 3096
gaggaccctg agtacgacgt gcaga                                          25

<210> 3097
<211> 25
<212> DNA
<213> Homo sapiens

<400> 3097
aggaccctga gtacgacgtg cagag                                          25

<210> 3098

<211> 25
<212> DNA
<213> Homo sapiens

<400> 3098
ggaccctgag tacgacgtgc agagc                                          25

<210> 3099
<211> 25
<212> DNA
<213> Homo sapiens

<400> 3099
gaccctgagt acgacgtgca gagca                                          25

<210> 3100
<211> 25
<212> DNA
<213> Homo sapiens

<400> 3100
accctgagta cgacgtgcag agcaa                                          25

<210> 3101
<211> 25
<212> DNA
<213> Homo sapiens

<400> 3101
ccctgagtac gacgtgcaga gcaag                                          25

<210> 3102
<211> 25
<212> DNA
<213> Homo sapiens

<400> 3102
cctgagtacg acgtgcagag caagc                                          25

<210> 3103
<211> 25
<212> DNA
<213> Homo sapiens

<400> 3103
ctgagtacga cgtgcagagc aagca                                          25

<210> 3104
<211> 25
<212> DNA
<213> Homo sapiens

<400> 3104
tgagtacgac gtgcagagca agcag                                          25

<210> 3105
<211> 25
<212> DNA
<213> Homo sapiens

<400> 3105

gagtacgacg tgcagagcaa gcagt                                          25

<210> 3106
<211> 25
<212> DNA
<213> Homo sapiens

<400> 3106
agtacgacgt gcagagcaag cagtg                                          25

<210> 3107
<211> 25
<212> DNA
<213> Homo sapiens

<400> 3107
gtacgacgtg cagagcaagc agtgg                                          25

<210> 3108
<211> 25
<212> DNA
<213> Homo sapiens

<400> 3108
tacgacgtgc agagcaagca gtggc                                          25

<210> 3109
<211> 25
<212> DNA
<213> Homo sapiens

<400> 3109
acgacgtgca gagcaagcag tggct                                          25

<210> 3110
<211> 25
<212> DNA
<213> Homo sapiens

<400> 3110
cgacgtgcag agcaagcagt ggctc                                          25

<210> 3111
<211> 25
<212> DNA
<213> Homo sapiens

<400> 3111
gacgtgcaga gcaagcagtg gctca                                          25

<210> 3112
<211> 25
<212> DNA
<213> Homo sapiens

<400> 3112
acgtgcagag caagcagtgg ctcac                                          25

<210> 3113
<211> 25
<212> DNA

<213> Homo sapiens

<400> 3113
cgtgcagagc aagcagtggc tcacc                                          25

<210> 3114
<211> 25
<212> DNA
<213> Homo sapiens

<400> 3114
gtgcagagca agcagtggct caccc                                          25

<210> 3115
<211> 25
<212> DNA
<213> Homo sapiens

<400> 3115
tgcagagcaa gcagtggctc accca                                          25

<210> 3116
<211> 25
<212> DNA
<213> Homo sapiens

<400> 3116
gcagagcaag cagtggctca cccat                                          25

<210> 3117
<211> 25
<212> DNA
<213> Homo sapiens

<400> 3117
cagagcaagc agtggctcac ccatt                                          25

<210> 3118
<211> 25
<212> DNA
<213> Homo sapiens

<400> 3118
agagcaagca gtggctcacc cattt                                          25

<210> 3119
<211> 25
<212> DNA
<213> Homo sapiens

<400> 3119
gagcaagcag tggctcaccc atttg                                          25

<210> 3120
<211> 25
<212> DNA
<213> Homo sapiens

<400> 3120
agcaagcagt ggctcaccca tttgc                                          25

<210> 3121
<211> 25
<212> DNA
<213> Homo sapiens

<400> 3121
gcaagcagtg gctcacccat ttgct                                          25

<210> 3122
<211> 25
<212> DNA
<213> Homo sapiens

<400> 3122
caagcagtgg ctcacccatt tgctc                                          25

<210> 3123
<211> 25
<212> DNA
<213> Homo sapiens

<400> 3123
aagcagtggc tcacccattt gctcg                                          25

<210> 3124
<211> 25
<212> DNA
<213> Homo sapiens

<400> 3124
agcagtggct cacccatttg ctcga                                          25

<210> 3125
<211> 25
<212> DNA
<213> Homo sapiens

<400> 3125
gcagtggctc acccatttgc tcgat                                          25

<210> 3126
<211> 25
<212> DNA
<213> Homo sapiens

<400> 3126
cagtggctca cccatttgct cgatc                                          25

<210> 3127
<211> 25
<212> DNA
<213> Homo sapiens

<400> 3127
agtggctcac ccatttgctc gatca                                          25

<210> 3128
<211> 25
<212> DNA
<213> Homo sapiens

<400> 3128
gtggctcacc catttgctcg atcaa                                          25

<210> 3129
<211> 25
<212> DNA
<213> Homo sapiens

<400> 3129
tggctcaccc atttgctcga tcaat                                          25

<210> 3130
<211> 25
<212> DNA
<213> Homo sapiens

<400> 3130
ggctcaccca tttgctcgat caatt                                          25

<210> 3131
<211> 25
<212> DNA
<213> Homo sapiens

<400> 3131
gctcacccat ttgctcgatc aattt                                          25

<210> 3132
<211> 25
<212> DNA
<213> Homo sapiens

<400> 3132
ctcacccatt tgctcgatca attta                                          25

<210> 3133
<211> 25
<212> DNA
<213> Homo sapiens

<400> 3133
tcacccattt gctcgatcaa tttac                                          25

<210> 3134
<211> 25
<212> DNA
<213> Homo sapiens

<400> 3134
cacccatttg ctcgatcaat ttacc                                          25

<210> 3135
<211> 25
<212> DNA
<213> Homo sapiens

<400> 3135
acccatttgc tcgatcaatt tacca                                          25

<210> 3136
<211> 25

<212> DNA
<213> Homo sapiens

<400> 3136
cccatttgct cgatcaattt accaa                                          25

<210> 3137
<211> 25
<212> DNA
<213> Homo sapiens

<400> 3137
ccatttgctc gatcaattta ccaac                                          25

<210> 3138
<211> 25
<212> DNA
<213> Homo sapiens

<400> 3138
catttgctcg atcaatttac caaca                                          25

<210> 3139
<211> 25
<212> DNA
<213> Homo sapiens

<400> 3139
atttgctcga tcaatttacc aacat                                          25

<210> 3140
<211> 25
<212> DNA
<213> Homo sapiens

<400> 3140
tttgctcgat caatttacca acatt                                          25

<210> 3141
<211> 25
<212> DNA
<213> Homo sapiens

<400> 3141
ttgctcgatc aatttaccaa catta                                          25

<210> 3142
<211> 25
<212> DNA
<213> Homo sapiens

<400> 3142
tgctcgatca atttaccaac attaa                                          25

<210> 3143
<211> 25
<212> DNA
<213> Homo sapiens

<400> 3143
gctcgatcaa tttaccaaca ttaag                                          25

<210> 3144
<211> 25
<212> DNA
<213> Homo sapiens

<400> 3144
ctcgatcaat ttaccaacat taaga                                          25

<210> 3145
<211> 25
<212> DNA
<213> Homo sapiens

<400> 3145
tcgatcaatt taccaacatt aagaa                                          25

<210> 3146
<211> 25
<212> DNA
<213> Homo sapiens

<400> 3146
cgatcaattt accaacatta agaac                                          25

<210> 3147
<211> 25
<212> DNA
<213> Homo sapiens

<400> 3147
gatcaattta ccaacattaa gaaca                                          25

<210> 3148
<211> 25
<212> DNA
<213> Homo sapiens

<400> 3148
atcaatttac caacattaag aacat                                          25

<210> 3149
<211> 25
<212> DNA
<213> Homo sapiens

<400> 3149
tcaatttacc aacattaaga acatc                                          25

<210> 3150
<211> 25
<212> DNA
<213> Homo sapiens

<400> 3150
caatttacca acattaagaa catct                                          25

<210> 3151
<211> 25
<212> DNA
<213> Homo sapiens

<400> 3151
aatttaccaa cattaagaac atctt                                          25

<210> 3152
<211> 25
<212> DNA
<213> Homo sapiens

<400> 3152
atttaccaac attaagaaca tcttg                                          25

<210> 3153
<211> 25
<212> DNA
<213> Homo sapiens

<400> 3153
tttaccaaca ttaagaacat cttgg                                          25

<210> 3154
<211> 25
<212> DNA
<213> Homo sapiens

<400> 3154
ttaccaacat taagaacatc ttggc                                          25

<210> 3155
<211> 25
<212> DNA
<213> Homo sapiens

<400> 3155
taccaacatt aagaacatct tggcc                                          25

<210> 3156
<211> 25
<212> DNA
<213> Homo sapiens

<400> 3156
accaacatta agaacatctt ggcct                                          25

<210> 3157
<211> 25
<212> DNA
<213> Homo sapiens

<400> 3157
ccaacattaa gaacatcttg gcctt                                          25

<210> 3158
<211> 25
<212> DNA
<213> Homo sapiens

<400> 3158
caacattaag aacatcttgg ccttg                                          25

<210> 3159

<211> 25
<212> DNA
<213> Homo sapiens

<400> 3159
aacattaaga acatcttggc cttga                                          25

<210> 3160
<211> 25
<212> DNA
<213> Homo sapiens

<400> 3160
acattaagaa catcttggcc ttgaa                                          25

<210> 3161
<211> 25
<212> DNA
<213> Homo sapiens

<400> 3161
cattaagaac atcttggcct tgaaa                                          25

<210> 3162
<211> 25
<212> DNA
<213> Homo sapiens

<400> 3162
attaagaaca tcttggcctt gaaaa                                          25

<210> 3163
<211> 25
<212> DNA
<213> Homo sapiens

<400> 3163
ttaagaacat cttggccttg aaaaa                                          25

<210> 3164
<211> 25
<212> DNA
<213> Homo sapiens

<400> 3164
taagaacatc ttggccttga aaaaa                                          25

<210> 3165
<211> 25
<212> DNA
<213> Homo sapiens

<400> 3165
aagaacatct tggccttgaa aaaaa                                          25

<210> 3166
<211> 25
<212> DNA
<213> Homo sapiens

<400> 3166

agaacatctt ggccttgaaa aaaat                                          25

<210> 3167
<211> 25
<212> DNA
<213> Homo sapiens

<400> 3167
gaacatcttg gccttgaaaa aaatt                                          25

<210> 3168
<211> 25
<212> DNA
<213> Homo sapiens

<400> 3168
aacatcttgg ccttgaaaaa aattg                                          25

<210> 3169
<211> 25
<212> DNA
<213> Homo sapiens

<400> 3169
acatcttggc cttgaaaaaa attga                                          25

<210> 3170
<211> 25
<212> DNA
<213> Homo sapiens

<400> 3170
catcttggcc ttgaaaaaaa ttgag                                          25

<210> 3171
<211> 25
<212> DNA
<213> Homo sapiens

<400> 3171
atcttggcct tgaaaaaaat tgagg                                          25

<210> 3172
<211> 25
<212> DNA
<213> Homo sapiens

<400> 3172
tcttggcctt gaaaaaaatt gaggt                                          25

<210> 3173
<211> 25
<212> DNA
<213> Homo sapiens

<400> 3173
cttggccttg aaaaaaattg aggta                                          25

<210> 3174
<211> 25
<212> DNA

<213> Homo sapiens

<400> 3174
ttggccttga aaaaaattga ggtag                                          25

<210> 3175
<211> 25
<212> DNA
<213> Homo sapiens

<400> 3175
tggccttgaa aaaaattgag gtagt                                          25

<210> 3176
<211> 25
<212> DNA
<213> Homo sapiens

<400> 3176
ggccttgaaa aaaattgagg tagtc                                          25

<210> 3177
<211> 25
<212> DNA
<213> Homo sapiens

<400> 3177
gccttgaaaa aaattgaggt agtcc                                          25

<210> 3178
<211> 25
<212> DNA
<213> Homo sapiens

<400> 3178
ccttgaaaaa aattgaggta gtcca                                          25

<210> 3179
<211> 25
<212> DNA
<213> Homo sapiens

<400> 3179
cttgaaaaaa attgaggtag tccac                                          25

<210> 3180
<211> 25
<212> DNA
<213> Homo sapiens

<400> 3180
ttgaaaaaaa ttgaggtagt ccact                                          25

<210> 3181
<211> 25
<212> DNA
<213> Homo sapiens

<400> 3181
tgaaaaaaat tgaggtagtc cactt                                          25

<210> 3182
<211> 25
<212> DNA
<213> Homo sapiens

<400> 3182
gaaaaaaatt gaggtagtcc acttt                                          25

<210> 3183
<211> 25
<212> DNA
<213> Homo sapiens

<400> 3183
aaaaaaattg aggtagtcca cttta                                          25

<210> 3184
<211> 25
<212> DNA
<213> Homo sapiens

<400> 3184
aaaaaattga ggtagtccac tttac                                          25

<210> 3185
<211> 25
<212> DNA
<213> Homo sapiens

<400> 3185
aaaaattgag gtagtccact ttaca                                          25

<210> 3186
<211> 25
<212> DNA
<213> Homo sapiens

<400> 3186
aaaattgagg tagtccactt tacat                                          25

<210> 3187
<211> 25
<212> DNA
<213> Homo sapiens

<400> 3187
aaattgaggt agtccacttt acatc                                          25

<210> 3188
<211> 25
<212> DNA
<213> Homo sapiens

<400> 3188
aattgaggta gtccacttta catcg                                          25

<210> 3189
<211> 25
<212> DNA
<213> Homo sapiens

<400> 3189
attgaggtag tccactttac atcgc                                          25

<210> 3190
<211> 25
<212> DNA
<213> Homo sapiens

<400> 3190
ttgaggtagt ccactttaca tcgct                                          25

<210> 3191
<211> 25
<212> DNA
<213> Homo sapiens

<400> 3191
tgaggtagtc cactttacat cgctt                                          25

<210> 3192
<211> 25
<212> DNA
<213> Homo sapiens

<400> 3192
gaggtagtcc actttacatc gcttt                                          25

<210> 3193
<211> 25
<212> DNA
<213> Homo sapiens

<400> 3193
aggtagtcca ctttacatcg ctttc                                          25

<210> 3194
<211> 25
<212> DNA
<213> Homo sapiens

<400> 3194
ggtagtccac tttacatcgc tttcc                                          25

<210> 3195
<211> 25
<212> DNA
<213> Homo sapiens

<400> 3195
gtagtccact ttacatcgct ttcca                                          25

<210> 3196
<211> 25
<212> DNA
<213> Homo sapiens

<400> 3196
tagtccactt tacatcgctt tccag                                          25

<210> 3197
<211> 25

<212> DNA
<213> Homo sapiens

<400> 3197
agtccacttt acatcgcttt ccaga                                              25

<210> 3198
<211> 25
<212> DNA
<213> Homo sapiens

<400> 3198
gtccacttta catcgctttc cagac                                              25

<210> 3199
<211> 25
<212> DNA
<213> Homo sapiens

<400> 3199
tccactttac atcgctttcc agaca                                              25

<210> 3200
<211> 25
<212> DNA
<213> Homo sapiens

<400> 3200
ccactttaca tcgctttcca gacaa                                              25

<210> 3201
<211> 25
<212> DNA
<213> Homo sapiens

<400> 3201
cactttacat cgctttccag acaac                                              25

<210> 3202
<211> 25
<212> DNA
<213> Homo sapiens

<400> 3202
actttacatc gctttccaga caact                                              25

<210> 3203
<211> 25
<212> DNA
<213> Homo sapiens

<400> 3203
ctttacatcg ctttccagac aactg                                              25

<210> 3204
<211> 25
<212> DNA
<213> Homo sapiens

<400> 3204
tttacatcgc tttccagaca actgg                                              25

<210> 3205
<211> 25
<212> DNA
<213> Homo sapiens

<400> 3205
ttacatcgct ttccagacaa ctgga                                          25

<210> 3206
<211> 25
<212> DNA
<213> Homo sapiens

<400> 3206
tacatcgctt tccagacaac tggaa                                          25

<210> 3207
<211> 25
<212> DNA
<213> Homo sapiens

<400> 3207
acatcgcttt ccagacaact ggaat                                          25

<210> 3208
<211> 25
<212> DNA
<213> Homo sapiens

<400> 3208
catcgctttc cagacaactg gaatt                                          25

<210> 3209
<211> 25
<212> DNA
<213> Homo sapiens

<400> 3209
atcgctttcc agacaactgg aattt                                          25

<210> 3210
<211> 25
<212> DNA
<213> Homo sapiens

<400> 3210
tcgctttcca gacaactgga atttg                                          25

<210> 3211
<211> 25
<212> DNA
<213> Homo sapiens

<400> 3211
cgctttccag acaactggaa tttga                                          25

<210> 3212
<211> 25
<212> DNA
<213> Homo sapiens

<400> 3212
gctttccaga caactggaat ttgag                                          25

<210> 3213
<211> 25
<212> DNA
<213> Homo sapiens

<400> 3213
ctttccagac aactggaatt tgagg                                          25

<210> 3214
<211> 25
<212> DNA
<213> Homo sapiens

<400> 3214
tttccagaca actggaattt gaggc                                          25

<210> 3215
<211> 25
<212> DNA
<213> Homo sapiens

<400> 3215
ttccagacaa ctggaatttg aggca                                          25

<210> 3216
<211> 25
<212> DNA
<213> Homo sapiens

<400> 3216
tccagacaac tggaatttga ggcaa                                          25

<210> 3217
<211> 25
<212> DNA
<213> Homo sapiens

<400> 3217
ccagacaact ggaatttgag gcaac                                          25

<210> 3218
<211> 25
<212> DNA
<213> Homo sapiens

<400> 3218
cagacaactg gaatttgagg caact                                          25

<210> 3219
<211> 25
<212> DNA
<213> Homo sapiens

<400> 3219
agacaactgg aatttgaggc aactt                                          25

<210> 3220

<211> 25
<212> DNA
<213> Homo sapiens

<400> 3220
gacaactgga atttgaggca acttc                                          25

<210> 3221
<211> 25
<212> DNA
<213> Homo sapiens

<400> 3221
acaactggaa tttgaggcaa cttct                                          25

<210> 3222
<211> 25
<212> DNA
<213> Homo sapiens

<400> 3222
caactggaat ttgaggcaac ttctg                                          25

<210> 3223
<211> 25
<212> DNA
<213> Homo sapiens

<400> 3223
aactggaatt tgaggcaact tctgt                                          25

<210> 3224
<211> 25
<212> DNA
<213> Homo sapiens

<400> 3224
actggaattt gaggcaactt ctgtg                                          25

<210> 3225
<211> 25
<212> DNA
<213> Homo sapiens

<400> 3225
ctggaatttg aggcaacttc tgtga                                          25

<210> 3226
<211> 25
<212> DNA
<213> Homo sapiens

<400> 3226
tggaatttga ggcaacttct gtgac                                          25

<210> 3227
<211> 25
<212> DNA
<213> Homo sapiens

<400> 3227

ggaatttgag gcaacttctg tgact 25

<210> 3228
<211> 25
<212> DNA
<213> Homo sapiens

<400> 3228
gaatttgagg caacttctgt gactg 25

<210> 3229
<211> 25
<212> DNA
<213> Homo sapiens

<400> 3229
aatttgaggc aacttctgtg actgt 25

<210> 3230
<211> 25
<212> DNA
<213> Homo sapiens

<400> 3230
atttgaggca acttctgtga ctgtg 25

<210> 3231
<211> 25
<212> DNA
<213> Homo sapiens

<400> 3231
tttgaggcaa cttctgtgac tgtga 25

<210> 3232
<211> 25
<212> DNA
<213> Homo sapiens

<400> 3232
ttgaggcaac ttctgtgact gtgat 25

<210> 3233
<211> 25
<212> DNA
<213> Homo sapiens

<400> 3233
tgaggcaact tctgtgactg tgatc 25

<210> 3234
<211> 25
<212> DNA
<213> Homo sapiens

<400> 3234
gaggcaactt ctgtgactgt gatcc 25

<210> 3235
<211> 25
<212> DNA

<213> Homo sapiens

<400> 3235
aggcaacttc tgtgactgtg atccc                                          25

<210> 3236
<211> 25
<212> DNA
<213> Homo sapiens

<400> 3236
ggcaacttct gtgactgtga tccct                                          25

<210> 3237
<211> 25
<212> DNA
<213> Homo sapiens

<400> 3237
gcaacttctg tgactgtgat ccctg                                          25

<210> 3238
<211> 25
<212> DNA
<213> Homo sapiens

<400> 3238
caacttctgt gactgtgatc cctgt                                          25

<210> 3239
<211> 25
<212> DNA
<213> Homo sapiens

<400> 3239
aacttctgtg actgtgatcc ctgtg                                          25

<210> 3240
<211> 25
<212> DNA
<213> Homo sapiens

<400> 3240
acttctgtga ctgtgatccc tgtgt                                          25

<210> 3241
<211> 25
<212> DNA
<213> Homo sapiens

<400> 3241
cttctgtgac tgtgatccct gtgtt                                          25

<210> 3242
<211> 25
<212> DNA
<213> Homo sapiens

<400> 3242
ttctgtgact gtgatccctg tgttt                                          25

<210> 3243
<211> 25
<212> DNA
<213> Homo sapiens

<400> 3243
tctgtgactg tgatccctgt gtttc                                            25

<210> 3244
<211> 25
<212> DNA
<213> Homo sapiens

<400> 3244
ctgtgactgt gatccctgtg tttca                                            25

<210> 3245
<211> 25
<212> DNA
<213> Homo sapiens

<400> 3245
tgtgactgtg atccctgtgt ttcac                                            25

<210> 3246
<211> 25
<212> DNA
<213> Homo sapiens

<400> 3246
gtgactgtga tccctgtgtt tcacc                                            25

<210> 3247
<211> 25
<212> DNA
<213> Homo sapiens

<400> 3247
tgactgtgat ccctgtgttt cacct                                            25

<210> 3248
<211> 25
<212> DNA
<213> Homo sapiens

<400> 3248
gactgtgatc cctgtgtttc acctg                                            25

<210> 3249
<211> 25
<212> DNA
<213> Homo sapiens

<400> 3249
actgtgatcc ctgtgtttca cctgg                                            25

<210> 3250
<211> 25
<212> DNA
<213> Homo sapiens

<400> 3250
ctgtgatccc tgtgtttcac ctggc                                          25

<210> 3251
<211> 25
<212> DNA
<213> Homo sapiens

<400> 3251
tgtgatccct gtgtttcacc tggca                                          25

<210> 3252
<211> 25
<212> DNA
<213> Homo sapiens

<400> 3252
gtgatccctg tgtttcacct ggcat                                          25

<210> 3253
<211> 25
<212> DNA
<213> Homo sapiens

<400> 3253
tgatccctgt gtttcacctg gcata                                          25

<210> 3254
<211> 25
<212> DNA
<213> Homo sapiens

<400> 3254
gatccctgtg tttcacctgg catac                                          25

<210> 3255
<211> 25
<212> DNA
<213> Homo sapiens

<400> 3255
atccctgtgt ttcacctggc ataca                                          25

<210> 3256
<211> 25
<212> DNA
<213> Homo sapiens

<400> 3256
tccctgtgtt tcacctggca tacat                                          25

<210> 3257
<211> 25
<212> DNA
<213> Homo sapiens

<400> 3257
ccctgtgttt cacctggcat acatt                                          25

<210> 3258
<211> 25

<212> DNA
<213> Homo sapiens

<400> 3258
cctgtgtttc acctggcata cattc                                          25

<210> 3259
<211> 25
<212> DNA
<213> Homo sapiens

<400> 3259
ctgtgtttca cctggcatac attct                                          25

<210> 3260
<211> 25
<212> DNA
<213> Homo sapiens

<400> 3260
tgtgtttcac ctggcataca ttctc                                          25

<210> 3261
<211> 25
<212> DNA
<213> Homo sapiens

<400> 3261
gtgtttcacc tggcatacat tctca                                          25

<210> 3262
<211> 25
<212> DNA
<213> Homo sapiens

<400> 3262
tgtttcacct ggcatacatt ctcat                                          25

<210> 3263
<211> 25
<212> DNA
<213> Homo sapiens

<400> 3263
gtttcacctg gcatacattc tcatc                                          25

<210> 3264
<211> 25
<212> DNA
<213> Homo sapiens

<400> 3264
tttcacctgg catacattct catca                                          25

<210> 3265
<211> 25
<212> DNA
<213> Homo sapiens

<400> 3265
ttcacctggc atacattctc atcat                                          25

<210> 3266
<211> 25
<212> DNA
<213> Homo sapiens

<400> 3266
tcacctggca tacattctca tcatt                                          25

<210> 3267
<211> 25
<212> DNA
<213> Homo sapiens

<400> 3267
cacctggcat acattctcat cattc                                          25

<210> 3268
<211> 25
<212> DNA
<213> Homo sapiens

<400> 3268
acctggcata cattctcatc attct                                          25

<210> 3269
<211> 25
<212> DNA
<213> Homo sapiens

<400> 3269
cctggcatac attctcatca ttctg                                          25

<210> 3270
<211> 25
<212> DNA
<213> Homo sapiens

<400> 3270
ctggcataca ttctcatcat tctgt                                          25

<210> 3271
<211> 25
<212> DNA
<213> Homo sapiens

<400> 3271
tggcatacat tctcatcatt ctgtt                                          25

<210> 3272
<211> 25
<212> DNA
<213> Homo sapiens

<400> 3272
ggcatacatt ctcatcattc tgttt                                          25

<210> 3273
<211> 25
<212> DNA
<213> Homo sapiens

<400> 3273
gcatacattc tcatcattct gtttg                                          25

<210> 3274
<211> 25
<212> DNA
<213> Homo sapiens

<400> 3274
catacattct catcattctg tttgc                                          25

<210> 3275
<211> 25
<212> DNA
<213> Homo sapiens

<400> 3275
atacattctc atcattctgt ttgca                                          25

<210> 3276
<211> 25
<212> DNA
<213> Homo sapiens

<400> 3276
tacattctca tcattctgtt tgcag                                          25

<210> 3277
<211> 25
<212> DNA
<213> Homo sapiens

<400> 3277
acattctcat cattctgttt gcagt                                          25

<210> 3278
<211> 25
<212> DNA
<213> Homo sapiens

<400> 3278
cattctcatc attctgtttg cagtc                                          25

<210> 3279
<211> 25
<212> DNA
<213> Homo sapiens

<400> 3279
attctcatca ttctgtttgc agtca                                          25

<210> 3280
<211> 25
<212> DNA
<213> Homo sapiens

<400> 3280
ttctcatcat tctgtttgca gtcac                                          25

<210> 3281

<211> 25
<212> DNA
<213> Homo sapiens

<400> 3281
tctcatcatt ctgtttgcag tcaca                                          25

<210> 3282
<211> 25
<212> DNA
<213> Homo sapiens

<400> 3282
ctcatcattc tgtttgcagt cacat                                          25

<210> 3283
<211> 25
<212> DNA
<213> Homo sapiens

<400> 3283
tcatcattct gtttgcagtc acatc                                          25

<210> 3284
<211> 25
<212> DNA
<213> Homo sapiens

<400> 3284
catcattctg tttgcagtca catca                                          25

<210> 3285
<211> 25
<212> DNA
<213> Homo sapiens

<400> 3285
atcattctgt ttgcagtcac atcat                                          25

<210> 3286
<211> 25
<212> DNA
<213> Homo sapiens

<400> 3286
tcattctgtt tgcagtcaca tcatg                                          25

<210> 3287
<211> 25
<212> DNA
<213> Homo sapiens

<400> 3287
cattctgttt gcagtcacat catgc                                          25

<210> 3288
<211> 25
<212> DNA
<213> Homo sapiens

<400> 3288

attctgtttg cagtcacatc atgct 25

<210> 3289
<211> 25
<212> DNA
<213> Homo sapiens

<400> 3289
ttctgtttgc agtcacatca tgctt 25

<210> 3290
<211> 25
<212> DNA
<213> Homo sapiens

<400> 3290
tctgtttgca gtcacatcat gcttt 25

<210> 3291
<211> 25
<212> DNA
<213> Homo sapiens

<400> 3291
ctgtttgcag tcacatcatg cttta 25

<210> 3292
<211> 25
<212> DNA
<213> Homo sapiens

<400> 3292
tgtttgcagt cacatcatgc tttag 25

<210> 3293
<211> 25
<212> DNA
<213> Homo sapiens

<400> 3293
gtttgcagtc acatcatgct ttagg 25

<210> 3294
<211> 25
<212> DNA
<213> Homo sapiens

<400> 3294
tttgcagtca catcatgctt taggt 25

<210> 3295
<211> 25
<212> DNA
<213> Homo sapiens

<400> 3295
ttgcagtcac atcatgcttt aggtt 25

<210> 3296
<211> 25
<212> DNA

<213> Homo sapiens

<400> 3296
tgcagtcaca tcatgcttta ggttt                                            25

<210> 3297
<211> 25
<212> DNA
<213> Homo sapiens

<400> 3297
gcagtcacat catgctttag gtttg                                            25

<210> 3298
<211> 25
<212> DNA
<213> Homo sapiens

<400> 3298
cagtcacatc atgctttagg tttga                                            25

<210> 3299
<211> 25
<212> DNA
<213> Homo sapiens

<400> 3299
agtcacatca tgctttaggt ttgac                                            25

<210> 3300
<211> 25
<212> DNA
<213> Homo sapiens

<400> 3300
gtcacatcat gctttaggtt tgact                                            25

<210> 3301
<211> 25
<212> DNA
<213> Homo sapiens

<400> 3301
tcacatcatg ctttaggttt gactg                                            25

<210> 3302
<211> 25
<212> DNA
<213> Homo sapiens

<400> 3302
cacatcatgc tttaggtttg actgc                                            25

<210> 3303
<211> 25
<212> DNA
<213> Homo sapiens

<400> 3303
acatcatgct ttaggtttga ctgca                                            25

<210> 3304
<211> 25
<212> DNA
<213> Homo sapiens

<400> 3304
catcatgctt taggtttgac tgcat                                             25

<210> 3305
<211> 25
<212> DNA
<213> Homo sapiens

<400> 3305
atcatgcttt aggtttgact gcata                                             25

<210> 3306
<211> 25
<212> DNA
<213> Homo sapiens

<400> 3306
tcatgcttta ggtttgactg catac                                             25

<210> 3307
<211> 25
<212> DNA
<213> Homo sapiens

<400> 3307
catgctttag gtttgactgc atacg                                             25

<210> 3308
<211> 25
<212> DNA
<213> Homo sapiens

<400> 3308
atgctttagg tttgactgca tacga                                             25

<210> 3309
<211> 25
<212> DNA
<213> Homo sapiens

<400> 3309
tgctttaggt ttgactgcat acgaa                                             25

<210> 3310
<211> 25
<212> DNA
<213> Homo sapiens

<400> 3310
gctttaggtt tgactgcata cgaaa                                             25

<210> 3311
<211> 25
<212> DNA
<213> Homo sapiens

<400> 3311
ctttaggttt gactgcatac gaaac                                          25

<210> 3312
<211> 25
<212> DNA
<213> Homo sapiens

<400> 3312
tttaggtttg actgcatacg aaaca                                          25

<210> 3313
<211> 25
<212> DNA
<213> Homo sapiens

<400> 3313
ttaggtttga ctgcatacga aacaa                                          25

<210> 3314
<211> 25
<212> DNA
<213> Homo sapiens

<400> 3314
taggtttgac tgcatacgaa acaaa                                          25

<210> 3315
<211> 25
<212> DNA
<213> Homo sapiens

<400> 3315
aggtttgact gcatacgaaa caaaa                                          25

<210> 3316
<211> 25
<212> DNA
<213> Homo sapiens

<400> 3316
ggtttgactg catacgaaac aaaat                                          25

<210> 3317
<211> 25
<212> DNA
<213> Homo sapiens

<400> 3317
gtttgactgc atacgaaaca aaatg                                          25

<210> 3318
<211> 25
<212> DNA
<213> Homo sapiens

<400> 3318
tttgactgca tacgaaacaa aatgt                                          25

<210> 3319
<211> 25

<212> DNA
<213> Homo sapiens

<400> 3319
ttgactgcat acgaaacaaa atgtg                                          25

<210> 3320
<211> 25
<212> DNA
<213> Homo sapiens

<400> 3320
tgactgcata cgaaacaaaa tgtgt                                          25

<210> 3321
<211> 25
<212> DNA
<213> Homo sapiens

<400> 3321
gactgcatac gaaacaaaat gtgtg                                          25

<210> 3322
<211> 25
<212> DNA
<213> Homo sapiens

<400> 3322
actgcatacg aaacaaaatg tgtgt                                          25

<210> 3323
<211> 25
<212> DNA
<213> Homo sapiens

<400> 3323
ctgcatacga aacaaaatgt gtgtt                                          25

<210> 3324
<211> 25
<212> DNA
<213> Homo sapiens

<400> 3324
tgcatacgaa acaaaatgtg tgttg                                          25

<210> 3325
<211> 25
<212> DNA
<213> Homo sapiens

<400> 3325
gcatacgaaa caaaatgtgt gttgc                                          25

<210> 3326
<211> 25
<212> DNA
<213> Homo sapiens

<400> 3326
catacgaaac aaaatgtgtg ttgcg                                          25

<210> 3327
<211> 25
<212> DNA
<213> Homo sapiens

<400> 3327
atacgaaaca aaatgtgtgt tgcgg                                          25

<210> 3328
<211> 25
<212> DNA
<213> Homo sapiens

<400> 3328
tacgaaacaa aatgtgtgtt gcggc                                          25

<210> 3329
<211> 25
<212> DNA
<213> Homo sapiens

<400> 3329
acgaaacaaa atgtgtgttg cggcc                                          25

<210> 3330
<211> 25
<212> DNA
<213> Homo sapiens

<400> 3330
cgaaacaaaa tgtgtgttgc ggcct                                          25

<210> 3331
<211> 25
<212> DNA
<213> Homo sapiens

<400> 3331
gaaacaaaat gtgtgttgcg gcctt                                          25

<210> 3332
<211> 25
<212> DNA
<213> Homo sapiens

<400> 3332
aaacaaaatg tgtgttgcgg ccttt                                          25

<210> 3333
<211> 25
<212> DNA
<213> Homo sapiens

<400> 3333
aacaaaatgt gtgttgcggc ctttg                                          25

<210> 3334
<211> 25
<212> DNA
<213> Homo sapiens

<400> 3334
acaaaatgtg tgttgcggcc tttgg                                                25

<210> 3335
<211> 25
<212> DNA
<213> Homo sapiens

<400> 3335
caaaatgtgt gttgcggcct ttgga                                                25

<210> 3336
<211> 25
<212> DNA
<213> Homo sapiens

<400> 3336
aaaatgtgtg ttgcggcctt tggag                                                25

<210> 3337
<211> 25
<212> DNA
<213> Homo sapiens

<400> 3337
aaatgtgtgt tgcggccttt ggagt                                                25

<210> 3338
<211> 25
<212> DNA
<213> Homo sapiens

<400> 3338
aatgtgtgtt gcggcctttg gagtg                                                25

<210> 3339
<211> 25
<212> DNA
<213> Homo sapiens

<400> 3339
atgtgtgttg cggcctttgg agtga                                                25

<210> 3340
<211> 25
<212> DNA
<213> Homo sapiens

<400> 3340
tgtgtgttgc ggcctttgga gtgat                                                25

<210> 3341
<211> 25
<212> DNA
<213> Homo sapiens

<400> 3341
gtgtgttgcg gcctttggag tgatt                                                25

<210> 3342

<211> 25
<212> DNA
<213> Homo sapiens

<400> 3342
tgtgttgcgg cctttggagt gattt                                          25

<210> 3343
<211> 25
<212> DNA
<213> Homo sapiens

<400> 3343
gtgttgcggc ctttggagtg atttc                                          25

<210> 3344
<211> 25
<212> DNA
<213> Homo sapiens

<400> 3344
tgttgcggcc tttggagtga tttct                                          25

<210> 3345
<211> 25
<212> DNA
<213> Homo sapiens

<400> 3345
gttgcggcct ttggagtgat ttctg                                          25

<210> 3346
<211> 25
<212> DNA
<213> Homo sapiens

<400> 3346
ttgcggcctt tggagtgatt tctgc                                          25

<210> 3347
<211> 25
<212> DNA
<213> Homo sapiens

<400> 3347
tgcggccttt ggagtgattt ctgct                                          25

<210> 3348
<211> 25
<212> DNA
<213> Homo sapiens

<400> 3348
gcggcctttg gagtgatttc tgctt                                          25

<210> 3349
<211> 25
<212> DNA
<213> Homo sapiens

<400> 3349

cggcctttgg agtgatttct gcttt                                                25

<210> 3350
<211> 25
<212> DNA
<213> Homo sapiens

<400> 3350
ggcctttgga gtgatttctg ctttc                                                25

<210> 3351
<211> 25
<212> DNA
<213> Homo sapiens

<400> 3351
gcctttggag tgatttctgc tttct                                                25

<210> 3352
<211> 25
<212> DNA
<213> Homo sapiens

<400> 3352
cctttggagt gatttctgct ttctt                                                25

<210> 3353
<211> 25
<212> DNA
<213> Homo sapiens

<400> 3353
ctttggagtg atttctgctt tcttg                                                25

<210> 3354
<211> 25
<212> DNA
<213> Homo sapiens

<400> 3354
tttggagtga tttctgcttt cttgg                                                25

<210> 3355
<211> 25
<212> DNA
<213> Homo sapiens

<400> 3355
ttggagtgat ttctgctttc ttggc                                                25

<210> 3356
<211> 25
<212> DNA
<213> Homo sapiens

<400> 3356
tggagtgatt tctgctttct tggca                                                25

<210> 3357
<211> 25
<212> DNA

<213> Homo sapiens

<400> 3357
ggagtgattt ctgctttctt ggcag                                            25

<210> 3358
<211> 25
<212> DNA
<213> Homo sapiens

<400> 3358
gagtgatttc tgctttcttg gcagt                                            25

<210> 3359
<211> 25
<212> DNA
<213> Homo sapiens

<400> 3359
agtgatttct gctttcttgg cagtg                                            25

<210> 3360
<211> 25
<212> DNA
<213> Homo sapiens

<400> 3360
gtgatttctg ctttcttggc agtgg                                            25

<210> 3361
<211> 25
<212> DNA
<213> Homo sapiens

<400> 3361
tgatttctgc tttcttggca gtggt                                            25

<210> 3362
<211> 25
<212> DNA
<213> Homo sapiens

<400> 3362
gatttctgct ttcttggcag tggtg                                            25

<210> 3363
<211> 25
<212> DNA
<213> Homo sapiens

<400> 3363
atttctgctt tcttggcagt ggtga                                            25

<210> 3364
<211> 25
<212> DNA
<213> Homo sapiens

<400> 3364
tttctgcttt cttggcagtg gtgag                                            25

<210> 3365
<211> 25
<212> DNA
<213> Homo sapiens

<400> 3365
ttctgctttc ttggcagtgg tgagc                                             25

<210> 3366
<211> 25
<212> DNA
<213> Homo sapiens

<400> 3366
tctgctttct tggcagtggt gagcg                                             25

<210> 3367
<211> 25
<212> DNA
<213> Homo sapiens

<400> 3367
ctgctttctt ggcagtggtg agcgg                                             25

<210> 3368
<211> 25
<212> DNA
<213> Homo sapiens

<400> 3368
tgctttcttg gcagtggtga gcggc                                             25

<210> 3369
<211> 25
<212> DNA
<213> Homo sapiens

<400> 3369
gctttcttgg cagtggtgag cggct                                             25

<210> 3370
<211> 25
<212> DNA
<213> Homo sapiens

<400> 3370
ctttcttggc agtggtgagc ggctt                                             25

<210> 3371
<211> 25
<212> DNA
<213> Homo sapiens

<400> 3371
tttcttggca gtggtgagcg gcttt                                             25

<210> 3372
<211> 25
<212> DNA
<213> Homo sapiens

<400> 3372
ttcttggcag tggtgagcgg ctttg                                          25

<210> 3373
<211> 25
<212> DNA
<213> Homo sapiens

<400> 3373
tcttggcagt ggtgagcggc tttgg                                          25

<210> 3374
<211> 25
<212> DNA
<213> Homo sapiens

<400> 3374
cttggcagtg gtgagcggct ttggc                                          25

<210> 3375
<211> 25
<212> DNA
<213> Homo sapiens

<400> 3375
ttggcagtgg tgagcggctt tggcc                                          25

<210> 3376
<211> 25
<212> DNA
<213> Homo sapiens

<400> 3376
tggcagtggt gagcggcttt ggcct                                          25

<210> 3377
<211> 25
<212> DNA
<213> Homo sapiens

<400> 3377
ggcagtggtg agcggctttg gcctg                                          25

<210> 3378
<211> 25
<212> DNA
<213> Homo sapiens

<400> 3378
gcagtggtga gcggctttgg cctgc                                          25

<210> 3379
<211> 25
<212> DNA
<213> Homo sapiens

<400> 3379
cagtggtgag cggctttggc ctgct                                          25

<210> 3380
<211> 25

<212> DNA
<213> Homo sapiens

<400> 3380
agtggtgagc ggctttggcc tgctg                                          25

<210> 3381
<211> 25
<212> DNA
<213> Homo sapiens

<400> 3381
gtggtgagcg gctttggcct gctgt                                          25

<210> 3382
<211> 25
<212> DNA
<213> Homo sapiens

<400> 3382
tggtgagcgg ctttggcctg ctgtt                                          25

<210> 3383
<211> 25
<212> DNA
<213> Homo sapiens

<400> 3383
ggtgagcggc tttggcctgc tgttg                                          25

<210> 3384
<211> 25
<212> DNA
<213> Homo sapiens

<400> 3384
gtgagcggct ttggcctgct gttgc                                          25

<210> 3385
<211> 25
<212> DNA
<213> Homo sapiens

<400> 3385
tgagcggctt tggcctgctg ttgca                                          25

<210> 3386
<211> 25
<212> DNA
<213> Homo sapiens

<400> 3386
gagcggcttt ggcctgctgt tgcac                                          25

<210> 3387
<211> 25
<212> DNA
<213> Homo sapiens

<400> 3387
agcggctttg gcctgctgtt gcaca                                          25

<210> 3388
<211> 25
<212> DNA
<213> Homo sapiens

<400> 3388
gcggctttgg cctgctgttg cacat                                            25

<210> 3389
<211> 25
<212> DNA
<213> Homo sapiens

<400> 3389
cggctttggc ctgctgttgc acatt                                            25

<210> 3390
<211> 25
<212> DNA
<213> Homo sapiens

<400> 3390
ggctttggcc tgctgttgca cattg                                            25

<210> 3391
<211> 25
<212> DNA
<213> Homo sapiens

<400> 3391
gctttggcct gctgttgcac attgg                                            25

<210> 3392
<211> 25
<212> DNA
<213> Homo sapiens

<400> 3392
ctttggcctg ctgttgcaca ttggg                                            25

<210> 3393
<211> 25
<212> DNA
<213> Homo sapiens

<400> 3393
tttggcctgc tgttgcacat tgggg                                            25

<210> 3394
<211> 25
<212> DNA
<213> Homo sapiens

<400> 3394
ttggcctgct gttgcacatt ggggt                                            25

<210> 3395
<211> 25
<212> DNA
<213> Homo sapiens

<400> 3395
tggcctgctg ttgcacattg gggtg                                          25

<210> 3396
<211> 25
<212> DNA
<213> Homo sapiens

<400> 3396
ggcctgctgt tgcacattgg ggtgc                                          25

<210> 3397
<211> 25
<212> DNA
<213> Homo sapiens

<400> 3397
gcctgctgtt gcacattggg gtgcc                                          25

<210> 3398
<211> 25
<212> DNA
<213> Homo sapiens

<400> 3398
cctgctgttg cacattgggg tgcca                                          25

<210> 3399
<211> 25
<212> DNA
<213> Homo sapiens

<400> 3399
ctgctgttgc acattggggt gccat                                          25

<210> 3400
<211> 25
<212> DNA
<213> Homo sapiens

<400> 3400
tgctgttgca cattggggtg ccatt                                          25

<210> 3401
<211> 25
<212> DNA
<213> Homo sapiens

<400> 3401
gctgttgcac attggggtgc cattt                                          25

<210> 3402
<211> 25
<212> DNA
<213> Homo sapiens

<400> 3402
ctgttgcaca ttggggtgcc atttg                                          25

<210> 3403

<211> 25
<212> DNA
<213> Homo sapiens

<400> 3403
tgttgcacat tggggtgcca tttgt                                          25

<210> 3404
<211> 25
<212> DNA
<213> Homo sapiens

<400> 3404
gttgcacatt ggggtgccat ttgtc                                          25

<210> 3405
<211> 25
<212> DNA
<213> Homo sapiens

<400> 3405
ttgcacattg gggtgccatt tgtca                                          25

<210> 3406
<211> 25
<212> DNA
<213> Homo sapiens

<400> 3406
tgcacattgg ggtgccattt gtcat                                          25

<210> 3407
<211> 25
<212> DNA
<213> Homo sapiens

<400> 3407
gcacattggg gtgccatttg tcatc                                          25

<210> 3408
<211> 25
<212> DNA
<213> Homo sapiens

<400> 3408
cacattgggg tgccatttgt catca                                          25

<210> 3409
<211> 25
<212> DNA
<213> Homo sapiens

<400> 3409
acattggggt gccatttgtc atcat                                          25

<210> 3410
<211> 25
<212> DNA
<213> Homo sapiens

<400> 3410

cattggggtg ccatttgtca tcata 25

<210> 3411
<211> 25
<212> DNA
<213> Homo sapiens

<400> 3411
attggggtgc catttgtcat catag 25

<210> 3412
<211> 25
<212> DNA
<213> Homo sapiens

<400> 3412
ttggggtgcc atttgtcatc atagt 25

<210> 3413
<211> 25
<212> DNA
<213> Homo sapiens

<400> 3413
tggggtgcca tttgtcatca tagtt 25

<210> 3414
<211> 25
<212> DNA
<213> Homo sapiens

<400> 3414
ggggtgccat ttgtcatcat agttg 25

<210> 3415
<211> 25
<212> DNA
<213> Homo sapiens

<400> 3415
gggtgccatt tgtcatcata gttgc 25

<210> 3416
<211> 25
<212> DNA
<213> Homo sapiens

<400> 3416
ggtgccattt gtcatcatag ttgcc 25

<210> 3417
<211> 25
<212> DNA
<213> Homo sapiens

<400> 3417
gtgccatttg tcatcatagt tgcca 25

<210> 3418
<211> 25
<212> DNA

<213> Homo sapiens

<400> 3418
tgccatttgt catcatagtt gccaa                                            25

<210> 3419
<211> 25
<212> DNA
<213> Homo sapiens

<400> 3419
gccatttgtc atcatagttg ccaat                                            25

<210> 3420
<211> 25
<212> DNA
<213> Homo sapiens

<400> 3420
ccatttgtca tcatagttgc caatt                                            25

<210> 3421
<211> 25
<212> DNA
<213> Homo sapiens

<400> 3421
catttgtcat catagttgcc aattc                                            25

<210> 3422
<211> 25
<212> DNA
<213> Homo sapiens

<400> 3422
atttgtcatc atagttgcca attca                                            25

<210> 3423
<211> 25
<212> DNA
<213> Homo sapiens

<400> 3423
tttgtcatca tagttgccaa ttcac                                            25

<210> 3424
<211> 25
<212> DNA
<213> Homo sapiens

<400> 3424
ttgtcatcat agttgccaat tcacc                                            25

<210> 3425
<211> 25
<212> DNA
<213> Homo sapiens

<400> 3425
tgtcatcata gttgccaatt cacca                                            25

<210> 3426
<211> 25
<212> DNA
<213> Homo sapiens

<400> 3426
gtcatcatag ttgccaattc accat                                            25

<210> 3427
<211> 25
<212> DNA
<213> Homo sapiens

<400> 3427
tcatcatagt tgccaattca ccatt                                            25

<210> 3428
<211> 25
<212> DNA
<213> Homo sapiens

<400> 3428
catcatagtt gccaattcac cattt                                            25

<210> 3429
<211> 25
<212> DNA
<213> Homo sapiens

<400> 3429
atcatagttg ccaattcacc atttc                                            25

<210> 3430
<211> 25
<212> DNA
<213> Homo sapiens

<400> 3430
tcatagttgc caattcacca tttct                                            25

<210> 3431
<211> 25
<212> DNA
<213> Homo sapiens

<400> 3431
catagttgcc aattcaccat ttctt                                            25

<210> 3432
<211> 25
<212> DNA
<213> Homo sapiens

<400> 3432
atagttgcca attcaccatt tctta                                            25

<210> 3433
<211> 25
<212> DNA
<213> Homo sapiens

<400> 3433
tagttgccaa ttcaccattt cttat                                          25

<210> 3434
<211> 25
<212> DNA
<213> Homo sapiens

<400> 3434
agttgccaat tcaccatttc ttatt                                          25

<210> 3435
<211> 25
<212> DNA
<213> Homo sapiens

<400> 3435
gttgccaatt caccatttct tattc                                          25

<210> 3436
<211> 25
<212> DNA
<213> Homo sapiens

<400> 3436
ttgccaattc accatttctt attct                                          25

<210> 3437
<211> 25
<212> DNA
<213> Homo sapiens

<400> 3437
tgccaattca ccatttctta ttcta                                          25

<210> 3438
<211> 25
<212> DNA
<213> Homo sapiens

<400> 3438
gccaattcac catttcttat tctag                                          25

<210> 3439
<211> 25
<212> DNA
<213> Homo sapiens

<400> 3439
ccaattcacc atttcttatt ctagg                                          25

<210> 3440
<211> 25
<212> DNA
<213> Homo sapiens

<400> 3440
caattcacca tttcttattc taggt                                          25

<210> 3441
<211> 25

<212> DNA
<213> Homo sapiens

<400> 3441
aattcaccat ttcttattct aggtg                                          25

<210> 3442
<211> 25
<212> DNA
<213> Homo sapiens

<400> 3442
attcaccatt tcttattcta ggtgt                                          25

<210> 3443
<211> 25
<212> DNA
<213> Homo sapiens

<400> 3443
ttcaccattt cttattctag gtgtt                                          25

<210> 3444
<211> 25
<212> DNA
<213> Homo sapiens

<400> 3444
tcaccatttc ttattctagg tgttg                                          25

<210> 3445
<211> 25
<212> DNA
<213> Homo sapiens

<400> 3445
caccatttct tattctaggt gttgg                                          25

<210> 3446
<211> 25
<212> DNA
<213> Homo sapiens

<400> 3446
accatttctt attctaggtg ttggg                                          25

<210> 3447
<211> 25
<212> DNA
<213> Homo sapiens

<400> 3447
ccatttctta ttctaggtgt tgggg                                          25

<210> 3448
<211> 25
<212> DNA
<213> Homo sapiens

<400> 3448
catttcttat tctaggtgtt ggggt                                          25

<210> 3449
<211> 25
<212> DNA
<213> Homo sapiens

<400> 3449
atttcttatt ctaggtgttg gggtc                                          25

<210> 3450
<211> 25
<212> DNA
<213> Homo sapiens

<400> 3450
tttcttattc taggtgttgg ggtcg                                          25

<210> 3451
<211> 25
<212> DNA
<213> Homo sapiens

<400> 3451
ttcttattct aggtgttggg gtcga                                          25

<210> 3452
<211> 25
<212> DNA
<213> Homo sapiens

<400> 3452
tcttattcta ggtgttgggg tcgat                                          25

<210> 3453
<211> 25
<212> DNA
<213> Homo sapiens

<400> 3453
cttattctag gtgttggggt cgatg                                          25

<210> 3454
<211> 25
<212> DNA
<213> Homo sapiens

<400> 3454
ttattctagg tgttggggtc gatga                                          25

<210> 3455
<211> 25
<212> DNA
<213> Homo sapiens

<400> 3455
tattctaggt gttggggtcg atgac                                          25

<210> 3456
<211> 25
<212> DNA
<213> Homo sapiens

<400> 3456
attctaggtg ttggggtcga tgaca                                          25

<210> 3457
<211> 25
<212> DNA
<213> Homo sapiens

<400> 3457
ttctaggtgt tggggtcgat gacat                                          25

<210> 3458
<211> 25
<212> DNA
<213> Homo sapiens

<400> 3458
tctaggtgtt ggggtcgatg acatg                                          25

<210> 3459
<211> 25
<212> DNA
<213> Homo sapiens

<400> 3459
ctaggtgttg gggtcgatga catgt                                          25

<210> 3460
<211> 25
<212> DNA
<213> Homo sapiens

<400> 3460
taggtgttgg ggtcgatgac atgtt                                          25

<210> 3461
<211> 25
<212> DNA
<213> Homo sapiens

<400> 3461
aggtgttggg gtcgatgaca tgttt                                          25

<210> 3462
<211> 25
<212> DNA
<213> Homo sapiens

<400> 3462
ggtgttgggg tcgatgacat gttta                                          25

<210> 3463
<211> 25
<212> DNA
<213> Homo sapiens

<400> 3463
gtgttggggt cgatgacatg tttat                                          25

<210> 3464

<211> 25
<212> DNA
<213> Homo sapiens

<400> 3464
tgttggggtc gatgacatgt ttatc                                          25

<210> 3465
<211> 25
<212> DNA
<213> Homo sapiens

<400> 3465
gttggggtcg atgacatgtt tatca                                          25

<210> 3466
<211> 25
<212> DNA
<213> Homo sapiens

<400> 3466
ttggggtcga tgacatgttt atcat                                          25

<210> 3467
<211> 25
<212> DNA
<213> Homo sapiens

<400> 3467
tggggtcgat gacatgttta tcatg                                          25

<210> 3468
<211> 25
<212> DNA
<213> Homo sapiens

<400> 3468
ggggtcgatg acatgtttat catga                                          25

<210> 3469
<211> 25
<212> DNA
<213> Homo sapiens

<400> 3469
gggtcgatga catgtttatc atgat                                          25

<210> 3470
<211> 25
<212> DNA
<213> Homo sapiens

<400> 3470
ggtcgatgac atgtttatca tgatt                                          25

<210> 3471
<211> 25
<212> DNA
<213> Homo sapiens

<400> 3471

gtcgatgaca tgtttatcat gattt 25

<210> 3472
<211> 25
<212> DNA
<213> Homo sapiens

<400> 3472
tcgatgacat gtttatcatg atttc 25

<210> 3473
<211> 25
<212> DNA
<213> Homo sapiens

<400> 3473
cgatgacatg tttatcatga tttct 25

<210> 3474
<211> 25
<212> DNA
<213> Homo sapiens

<400> 3474
gatgacatgt ttatcatgat ttctg 25

<210> 3475
<211> 25
<212> DNA
<213> Homo sapiens

<400> 3475
atgacatgtt tatcatgatt tctgc 25

<210> 3476
<211> 25
<212> DNA
<213> Homo sapiens

<400> 3476
tgacatgttt atcatgattt ctgcc 25

<210> 3477
<211> 25
<212> DNA
<213> Homo sapiens

<400> 3477
gacatgttta tcatgatttc tgcct 25

<210> 3478
<211> 25
<212> DNA
<213> Homo sapiens

<400> 3478
acatgtttat catgatttct gcctg 25

<210> 3479
<211> 25
<212> DNA

<213> Homo sapiens

<400> 3479
catgtttatc atgatttctg cctgg                                          25

<210> 3480
<211> 25
<212> DNA
<213> Homo sapiens

<400> 3480
atgtttatca tgatttctgc ctggc                                          25

<210> 3481
<211> 25
<212> DNA
<213> Homo sapiens

<400> 3481
tgtttatcat gatttctgcc tggca                                          25

<210> 3482
<211> 25
<212> DNA
<213> Homo sapiens

<400> 3482
gtttatcatg atttctgcct ggcat                                          25

<210> 3483
<211> 25
<212> DNA
<213> Homo sapiens

<400> 3483
tttatcatga tttctgcctg gcata                                          25

<210> 3484
<211> 25
<212> DNA
<213> Homo sapiens

<400> 3484
ttatcatgat ttctgcctgg cataa                                          25

<210> 3485
<211> 25
<212> DNA
<213> Homo sapiens

<400> 3485
tatcatgatt tctgcctggc ataag                                          25

<210> 3486
<211> 25
<212> DNA
<213> Homo sapiens

<400> 3486
atcatgattt ctgcctggca taaga                                          25

<210> 3487
<211> 25
<212> DNA
<213> Homo sapiens

<400> 3487
tcatgatttc tgcctggcat aagac                                          25

<210> 3488
<211> 25
<212> DNA
<213> Homo sapiens

<400> 3488
catgatttct gcctggcata agacc                                          25

<210> 3489
<211> 25
<212> DNA
<213> Homo sapiens

<400> 3489
atgatttctg cctggcataa gacca                                          25

<210> 3490
<211> 25
<212> DNA
<213> Homo sapiens

<400> 3490
tgatttctgc ctggcataag accaa                                          25

<210> 3491
<211> 25
<212> DNA
<213> Homo sapiens

<400> 3491
gatttctgcc tggcataaga ccaac                                          25

<210> 3492
<211> 25
<212> DNA
<213> Homo sapiens

<400> 3492
atttctgcct ggcataagac caacc                                          25

<210> 3493
<211> 25
<212> DNA
<213> Homo sapiens

<400> 3493
tttctgcctg gcataagacc aacct                                          25

<210> 3494
<211> 25
<212> DNA
<213> Homo sapiens

<400> 3494
ttctgcctgg cataagacca acctt                                          25

<210> 3495
<211> 25
<212> DNA
<213> Homo sapiens

<400> 3495
tctgcctggc ataagaccaa ccttg                                          25

<210> 3496
<211> 25
<212> DNA
<213> Homo sapiens

<400> 3496
ctgcctggca taagaccaac cttgc                                          25

<210> 3497
<211> 25
<212> DNA
<213> Homo sapiens

<400> 3497
tgcctggcat aagaccaacc ttgca                                          25

<210> 3498
<211> 25
<212> DNA
<213> Homo sapiens

<400> 3498
gcctggcata agaccaacct tgcag                                          25

<210> 3499
<211> 25
<212> DNA
<213> Homo sapiens

<400> 3499
cctggcataa gaccaacctt gcagg                                          25

<210> 3500
<211> 25
<212> DNA
<213> Homo sapiens

<400> 3500
ctggcataag accaaccttg caggt                                          25

<210> 3501
<211> 25
<212> DNA
<213> Homo sapiens

<400> 3501
tggcataaga ccaaccttgc aggtg                                          25

<210> 3502
<211> 25

<212> DNA
<213> Homo sapiens

<400> 3502
ggcataagac caaccttgca ggtga                                          25

<210> 3503
<211> 25
<212> DNA
<213> Homo sapiens

<400> 3503
gcataagacc aaccttgcag gtgac                                          25

<210> 3504
<211> 25
<212> DNA
<213> Homo sapiens

<400> 3504
cataagacca accttgcagg tgaca                                          25

<210> 3505
<211> 25
<212> DNA
<213> Homo sapiens

<400> 3505
ataagaccaa ccttgcaggt gacat                                          25

<210> 3506
<211> 25
<212> DNA
<213> Homo sapiens

<400> 3506
taagaccaac cttgcaggtg acata                                          25

<210> 3507
<211> 25
<212> DNA
<213> Homo sapiens

<400> 3507
aagaccaacc ttgcaggtga catac                                          25

<210> 3508
<211> 25
<212> DNA
<213> Homo sapiens

<400> 3508
agaccaacct tgcaggtgac atacg                                          25

<210> 3509
<211> 25
<212> DNA
<213> Homo sapiens

<400> 3509
gaccaacctt gcaggtgaca tacga                                          25

<210> 3510
<211> 25
<212> DNA
<213> Homo sapiens

<400> 3510
accaaccttg caggtgacat acgag                                          25

<210> 3511
<211> 25
<212> DNA
<213> Homo sapiens

<400> 3511
ccaaccttgc aggtgacata cgaga                                          25

<210> 3512
<211> 25
<212> DNA
<213> Homo sapiens

<400> 3512
caaccttgca ggtgacatac gagag                                          25

<210> 3513
<211> 25
<212> DNA
<213> Homo sapiens

<400> 3513
aaccttgcag gtgacatacg agagc                                          25

<210> 3514
<211> 25
<212> DNA
<213> Homo sapiens

<400> 3514
accttgcagg tgacatacga gagcg                                          25

<210> 3515
<211> 25
<212> DNA
<213> Homo sapiens

<400> 3515
ccttgcaggt gacatacgag agcgg                                          25

<210> 3516
<211> 25
<212> DNA
<213> Homo sapiens

<400> 3516
cttgcaggtg acatacgaga gcgga                                          25

<210> 3517
<211> 25
<212> DNA
<213> Homo sapiens

<400> 3517
ttgcaggtga catacgagag cggat                                          25

<210> 3518
<211> 25
<212> DNA
<213> Homo sapiens

<400> 3518
tgcaggtgac atacgagagc ggatg                                          25

<210> 3519
<211> 25
<212> DNA
<213> Homo sapiens

<400> 3519
gcaggtgaca tacgagagcg gatgt                                          25

<210> 3520
<211> 25
<212> DNA
<213> Homo sapiens

<400> 3520
caggtgacat acgagagcgg atgtc                                          25

<210> 3521
<211> 25
<212> DNA
<213> Homo sapiens

<400> 3521
aggtgacata cgagagcgga tgtcc                                          25

<210> 3522
<211> 25
<212> DNA
<213> Homo sapiens

<400> 3522
ggtgacatac gagagcggat gtcca                                          25

<210> 3523
<211> 25
<212> DNA
<213> Homo sapiens

<400> 3523
gtgacatacg agagcggatg tccaa                                          25

<210> 3524
<211> 25
<212> DNA
<213> Homo sapiens

<400> 3524
tgacatacga gagcggatgt ccaat                                          25

<210> 3525

<211> 25
<212> DNA
<213> Homo sapiens

<400> 3525
gacatacgag agcggatgtc caatg                                          25

<210> 3526
<211> 25
<212> DNA
<213> Homo sapiens

<400> 3526
acatacgaga gcggatgtcc aatgt                                          25

<210> 3527
<211> 25
<212> DNA
<213> Homo sapiens

<400> 3527
catacgagag cggatgtcca atgtc                                          25

<210> 3528
<211> 25
<212> DNA
<213> Homo sapiens

<400> 3528
atacgagagc ggatgtccaa tgtct                                          25

<210> 3529
<211> 25
<212> DNA
<213> Homo sapiens

<400> 3529
tacgagagcg gatgtccaat gtcta                                          25

<210> 3530
<211> 25
<212> DNA
<213> Homo sapiens

<400> 3530
acgagagcgg atgtccaatg tctat                                          25

<210> 3531
<211> 25
<212> DNA
<213> Homo sapiens

<400> 3531
cgagagcgga tgtccaatgt ctatt                                          25

<210> 3532
<211> 25
<212> DNA
<213> Homo sapiens

<400> 3532

gagagcggat gtccaatgtc tattc 25

<210> 3533
<211> 25
<212> DNA
<213> Homo sapiens

<400> 3533
agagcggatg tccaatgtct attca 25

<210> 3534
<211> 25
<212> DNA
<213> Homo sapiens

<400> 3534
gagcggatgt ccaatgtcta ttcaa 25

<210> 3535
<211> 25
<212> DNA
<213> Homo sapiens

<400> 3535
agcggatgtc caatgtctat tcaaa 25

<210> 3536
<211> 25
<212> DNA
<213> Homo sapiens

<400> 3536
gcggatgtcc aatgtctatt caaaa 25

<210> 3537
<211> 25
<212> DNA
<213> Homo sapiens

<400> 3537
cggatgtcca atgtctattc aaaag 25

<210> 3538
<211> 25
<212> DNA
<213> Homo sapiens

<400> 3538
ggatgtccaa tgtctattca aaagc 25

<210> 3539
<211> 25
<212> DNA
<213> Homo sapiens

<400> 3539
gatgtccaat gtctattcaa aagcg 25

<210> 3540
<211> 25
<212> DNA

<213> Homo sapiens

<400> 3540
atgtccaatg tctattcaaa agcgg                                             25

<210> 3541
<211> 25
<212> DNA
<213> Homo sapiens

<400> 3541
tgtccaatgt ctattcaaaa gcggc                                             25

<210> 3542
<211> 25
<212> DNA
<213> Homo sapiens

<400> 3542
gtccaatgtc tattcaaaag cggca                                             25

<210> 3543
<211> 25
<212> DNA
<213> Homo sapiens

<400> 3543
tccaatgtct attcaaaagc ggcag                                             25

<210> 3544
<211> 25
<212> DNA
<213> Homo sapiens

<400> 3544
ccaatgtcta ttcaaaagcg gcagt                                             25

<210> 3545
<211> 25
<212> DNA
<213> Homo sapiens

<400> 3545
caatgtctat tcaaaagcgg cagtg                                             25

<210> 3546
<211> 25
<212> DNA
<213> Homo sapiens

<400> 3546
aatgtctatt caaaagcggc agtgt                                             25

<210> 3547
<211> 25
<212> DNA
<213> Homo sapiens

<400> 3547
atgtctattc aaaagcggca gtgtc                                             25

<210> 3548
<211> 25
<212> DNA
<213> Homo sapiens

<400> 3548
tgtctattca aaagcggcag tgtct                                          25

<210> 3549
<211> 25
<212> DNA
<213> Homo sapiens

<400> 3549
gtctattcaa aagcggcagt gtcta                                          25

<210> 3550
<211> 25
<212> DNA
<213> Homo sapiens

<400> 3550
tctattcaaa agcggcagtg tctat                                          25

<210> 3551
<211> 25
<212> DNA
<213> Homo sapiens

<400> 3551
ctattcaaaa gcggcagtgt ctatt                                          25

<210> 3552
<211> 25
<212> DNA
<213> Homo sapiens

<400> 3552
tattcaaaag cggcagtgtc tatta                                          25

<210> 3553
<211> 25
<212> DNA
<213> Homo sapiens

<400> 3553
attcaaaagc ggcagtgtct attac                                          25

<210> 3554
<211> 25
<212> DNA
<213> Homo sapiens

<400> 3554
ttcaaaagcg gcagtgtcta ttaca                                          25

<210> 3555
<211> 25
<212> DNA
<213> Homo sapiens

<400> 3555
tcaaaagcgg cagtgtctat tacaa                                          25

<210> 3556
<211> 25
<212> DNA
<213> Homo sapiens

<400> 3556
caaaagcggc agtgtctatt acaat                                          25

<210> 3557
<211> 25
<212> DNA
<213> Homo sapiens

<400> 3557
aaaagcggca gtgtctatta caatc                                          25

<210> 3558
<211> 25
<212> DNA
<213> Homo sapiens

<400> 3558
aaagcggcag tgtctattac aatca                                          25

<210> 3559
<211> 25
<212> DNA
<213> Homo sapiens

<400> 3559
aagcggcagt gtctattaca atcac                                          25

<210> 3560
<211> 25
<212> DNA
<213> Homo sapiens

<400> 3560
agcggcagtg tctattacaa tcacc                                          25

<210> 3561
<211> 25
<212> DNA
<213> Homo sapiens

<400> 3561
gcggcagtgt ctattacaat cacca                                          25

<210> 3562
<211> 25
<212> DNA
<213> Homo sapiens

<400> 3562
cggcagtgtc tattacaatc accac                                          25

<210> 3563
<211> 25

<212> DNA
<213> Homo sapiens

<400> 3563
ggcagtgtct attacaatca ccacc                                          25

<210> 3564
<211> 25
<212> DNA
<213> Homo sapiens

<400> 3564
gcagtgtcta ttacaatcac cacca                                          25

<210> 3565
<211> 25
<212> DNA
<213> Homo sapiens

<400> 3565
cagtgtctat tacaatcacc accat                                          25

<210> 3566
<211> 25
<212> DNA
<213> Homo sapiens

<400> 3566
agtgtctatt acaatcacca ccatc                                          25

<210> 3567
<211> 25
<212> DNA
<213> Homo sapiens

<400> 3567
gtgtctatta caatcaccac catca                                          25

<210> 3568
<211> 25
<212> DNA
<213> Homo sapiens

<400> 3568
tgtctattac aatcaccacc atcac                                          25

<210> 3569
<211> 25
<212> DNA
<213> Homo sapiens

<400> 3569
gtctattaca atcaccacca tcact                                          25

<210> 3570
<211> 25
<212> DNA
<213> Homo sapiens

<400> 3570
tctattacaa tcaccaccat cacta                                          25

<210> 3571
<211> 25
<212> DNA
<213> Homo sapiens

<400> 3571
ctattacaat caccaccatc actaa                                          25

<210> 3572
<211> 25
<212> DNA
<213> Homo sapiens

<400> 3572
tattacaatc accaccatca ctaac                                          25

<210> 3573
<211> 25
<212> DNA
<213> Homo sapiens

<400> 3573
attacaatca ccaccatcac taaca                                          25

<210> 3574
<211> 25
<212> DNA
<213> Homo sapiens

<400> 3574
ttacaatcac caccatcact aacat                                          25

<210> 3575
<211> 25
<212> DNA
<213> Homo sapiens

<400> 3575
tacaatcacc accatcacta acatc                                          25

<210> 3576
<211> 25
<212> DNA
<213> Homo sapiens

<400> 3576
acaatcacca ccatcactaa catcc                                          25

<210> 3577
<211> 25
<212> DNA
<213> Homo sapiens

<400> 3577
caatcaccac catcactaac atcct                                          25

<210> 3578
<211> 25
<212> DNA
<213> Homo sapiens

<400> 3578
aatcaccacc atcactaaca tcctg                                          25

<210> 3579
<211> 25
<212> DNA
<213> Homo sapiens

<400> 3579
atcaccacca tcactaacat cctgg                                          25

<210> 3580
<211> 25
<212> DNA
<213> Homo sapiens

<400> 3580
tcaccaccat cactaacatc ctggc                                          25

<210> 3581
<211> 25
<212> DNA
<213> Homo sapiens

<400> 3581
caccaccatc actaacatcc tggcc                                          25

<210> 3582
<211> 25
<212> DNA
<213> Homo sapiens

<400> 3582
accaccatca ctaacatcct ggcct                                          25

<210> 3583
<211> 25
<212> DNA
<213> Homo sapiens

<400> 3583
ccaccatcac taacatcctg gcctt                                          25

<210> 3584
<211> 25
<212> DNA
<213> Homo sapiens

<400> 3584
caccatcact aacatcctgg cctta                                          25

<210> 3585
<211> 25
<212> DNA
<213> Homo sapiens

<400> 3585
accatcacta acatcctggc cttat                                          25

<210> 3586

<211> 25
<212> DNA
<213> Homo sapiens

<400> 3586
ccatcactaa catcctggcc ttata                                          25

<210> 3587
<211> 25
<212> DNA
<213> Homo sapiens

<400> 3587
catcactaac atcctggcct tatat                                          25

<210> 3588
<211> 25
<212> DNA
<213> Homo sapiens

<400> 3588
atcactaaca tcctggcctt atata                                          25

<210> 3589
<211> 25
<212> DNA
<213> Homo sapiens

<400> 3589
tcactaacat cctggcctta tatac                                          25

<210> 3590
<211> 25
<212> DNA
<213> Homo sapiens

<400> 3590
cactaacatc ctggccttat ataca                                          25

<210> 3591
<211> 25
<212> DNA
<213> Homo sapiens

<400> 3591
actaacatcc tggccttata tacag                                          25

<210> 3592
<211> 25
<212> DNA
<213> Homo sapiens

<400> 3592
ctaacatcct ggccttatat acagg                                          25

<210> 3593
<211> 25
<212> DNA
<213> Homo sapiens

<400> 3593

taacatcctg gccttatata caggg                                          25

<210> 3594
<211> 25
<212> DNA
<213> Homo sapiens

<400> 3594
aacatcctgg ccttatatac aggga                                          25

<210> 3595
<211> 25
<212> DNA
<213> Homo sapiens

<400> 3595
acatcctggc cttatataca gggat                                          25

<210> 3596
<211> 25
<212> DNA
<213> Homo sapiens

<400> 3596
catcctggcc ttatatacag ggatt                                          25

<210> 3597
<211> 25
<212> DNA
<213> Homo sapiens

<400> 3597
atcctggcct tatatacagg gatta                                          25

<210> 3598
<211> 25
<212> DNA
<213> Homo sapiens

<400> 3598
tcctggcctt atatacaggg attat                                          25

<210> 3599
<211> 25
<212> DNA
<213> Homo sapiens

<400> 3599
cctggcctta tatacaggga ttatg                                          25

<210> 3600
<211> 25
<212> DNA
<213> Homo sapiens

<400> 3600
ctggccttat atacagggat tatga                                          25

<210> 3601
<211> 25
<212> DNA

<213> Homo sapiens

<400> 3601
tggccttata tacagggatt atgag                                          25

<210> 3602
<211> 25
<212> DNA
<213> Homo sapiens

<400> 3602
ggccttatat acagggatta tgagc                                          25

<210> 3603
<211> 25
<212> DNA
<213> Homo sapiens

<400> 3603
gccttatata cagggattat gagct                                          25

<210> 3604
<211> 25
<212> DNA
<213> Homo sapiens

<400> 3604
ccttatatac agggattatg agctc                                          25

<210> 3605
<211> 25
<212> DNA
<213> Homo sapiens

<400> 3605
cttatataca gggattatga gctct                                          25

<210> 3606
<211> 25
<212> DNA
<213> Homo sapiens

<400> 3606
ttatatacag ggattatgag ctctt                                          25

<210> 3607
<211> 25
<212> DNA
<213> Homo sapiens

<400> 3607
tatatacagg gattatgagc tcttt                                          25

<210> 3608
<211> 25
<212> DNA
<213> Homo sapiens

<400> 3608
atatacaggg attatgagct ctttt                                          25

<210> 3609
<211> 25
<212> DNA
<213> Homo sapiens

<400> 3609
tatacaggga ttatgagctc tttta                                            25

<210> 3610
<211> 25
<212> DNA
<213> Homo sapiens

<400> 3610
atacagggat tatgagctct tttag                                            25

<210> 3611
<211> 25
<212> DNA
<213> Homo sapiens

<400> 3611
tacagggatt atgagctctt ttagg                                            25

<210> 3612
<211> 25
<212> DNA
<213> Homo sapiens

<400> 3612
acagggatta tgagctcttt taggt                                            25

<210> 3613
<211> 25
<212> DNA
<213> Homo sapiens

<400> 3613
cagggattat gagctctttt aggtc                                            25

<210> 3614
<211> 25
<212> DNA
<213> Homo sapiens

<400> 3614
agggattatg agctctttta ggtcc                                            25

<210> 3615
<211> 25
<212> DNA
<213> Homo sapiens

<400> 3615
gggattatga gctcttttag gtccg                                            25

<210> 3616
<211> 25
<212> DNA
<213> Homo sapiens

<400> 3616
ggattatgag ctcttttagg tccgt                                          25

<210> 3617
<211> 25
<212> DNA
<213> Homo sapiens

<400> 3617
gattatgagc tcttttaggt ccgta                                          25

<210> 3618
<211> 25
<212> DNA
<213> Homo sapiens

<400> 3618
attatgagct cttttaggtc cgtac                                          25

<210> 3619
<211> 25
<212> DNA
<213> Homo sapiens

<400> 3619
ttatgagctc ttttaggtcc gtaca                                          25

<210> 3620
<211> 25
<212> DNA
<213> Homo sapiens

<400> 3620
tatgagctct tttaggtccg tacaa                                          25

<210> 3621
<211> 25
<212> DNA
<213> Homo sapiens

<400> 3621
atgagctctt ttaggtccgt acaat                                          25

<210> 3622
<211> 25
<212> DNA
<213> Homo sapiens

<400> 3622
tgagctcttt taggtccgta caatg                                          25

<210> 3623
<211> 25
<212> DNA
<213> Homo sapiens

<400> 3623
gagctctttt aggtccgtac aatgt                                          25

<210> 3624
<211> 25

<212> DNA
<213> Homo sapiens

<400> 3624
agctctttta ggtccgtaca atgtt                                          25

<210> 3625
<211> 25
<212> DNA
<213> Homo sapiens

<400> 3625
gctcttttag gtccgtacaa tgttt                                          25

<210> 3626
<211> 25
<212> DNA
<213> Homo sapiens

<400> 3626
ctcttttagg tccgtacaat gtttt                                          25

<210> 3627
<211> 25
<212> DNA
<213> Homo sapiens

<400> 3627
tcttttaggt ccgtacaatg ttttt                                          25

<210> 3628
<211> 25
<212> DNA
<213> Homo sapiens

<400> 3628
cttttaggtc cgtacaatgt ttttg                                          25

<210> 3629
<211> 25
<212> DNA
<213> Homo sapiens

<400> 3629
ttttaggtcc gtacaatgtt tttgc                                          25

<210> 3630
<211> 25
<212> DNA
<213> Homo sapiens

<400> 3630
tttaggtccg tacaatgttt ttgca                                          25

<210> 3631
<211> 25
<212> DNA
<213> Homo sapiens

<400> 3631
ttaggtccgt acaatgtttt tgcat                                          25

<210> 3632
<211> 25
<212> DNA
<213> Homo sapiens

<400> 3632
taggtccgta caatgttttt gcatc                                          25

<210> 3633
<211> 25
<212> DNA
<213> Homo sapiens

<400> 3633
aggtccgtac aatgtttttg catct                                          25

<210> 3634
<211> 25
<212> DNA
<213> Homo sapiens

<400> 3634
ggtccgtaca atgtttttgc atcta                                          25

<210> 3635
<211> 25
<212> DNA
<213> Homo sapiens

<400> 3635
gtccgtacaa tgtttttgca tctat                                          25

<210> 3636
<211> 25
<212> DNA
<213> Homo sapiens

<400> 3636
tccgtacaat gtttttgcat ctata                                          25

<210> 3637
<211> 25
<212> DNA
<213> Homo sapiens

<400> 3637
ccgtacaatg tttttgcatc tatac                                          25

<210> 3638
<211> 25
<212> DNA
<213> Homo sapiens

<400> 3638
cgtacaatgt ttttgcatct ataca                                          25

<210> 3639
<211> 25
<212> DNA
<213> Homo sapiens

<400> 3639
gtacaatgtt tttgcatcta tacag                                          25

<210> 3640
<211> 25
<212> DNA
<213> Homo sapiens

<400> 3640
tacaatgttt ttgcatctat acagg                                          25

<210> 3641
<211> 25
<212> DNA
<213> Homo sapiens

<400> 3641
acaatgtttt tgcatctata cagga                                          25

<210> 3642
<211> 25
<212> DNA
<213> Homo sapiens

<400> 3642
caatgttttt gcatctatac aggaa                                          25

<210> 3643
<211> 25
<212> DNA
<213> Homo sapiens

<400> 3643
aatgtttttg catctataca ggaac                                          25

<210> 3644
<211> 25
<212> DNA
<213> Homo sapiens

<400> 3644
atgtttttgc atctatacag gaacg                                          25

<210> 3645
<211> 25
<212> DNA
<213> Homo sapiens

<400> 3645
tgtttttgca tctatacagg aacga                                          25

<210> 3646
<211> 25
<212> DNA
<213> Homo sapiens

<400> 3646
gtttttgcat ctatacagga acgac                                          25

<210> 3647

<211> 25
<212> DNA
<213> Homo sapiens

<400> 3647
tttttgcatc tatacaggaa cgacc                                          25

<210> 3648
<211> 25
<212> DNA
<213> Homo sapiens

<400> 3648
ttttgcatct atacaggaac gaccc                                          25

<210> 3649
<211> 25
<212> DNA
<213> Homo sapiens

<400> 3649
tttgcatcta tacaggaacg accct                                          25

<210> 3650
<211> 25
<212> DNA
<213> Homo sapiens

<400> 3650
ttgcatctat acaggaacga ccctg                                          25

<210> 3651
<211> 25
<212> DNA
<213> Homo sapiens

<400> 3651
tgcatctata caggaacgac cctgt                                          25

<210> 3652
<211> 25
<212> DNA
<213> Homo sapiens

<400> 3652
gcatctatac aggaacgacc ctgtt                                          25

<210> 3653
<211> 25
<212> DNA
<213> Homo sapiens

<400> 3653
catctataca ggaacgaccc tgtta                                          25

<210> 3654
<211> 25
<212> DNA
<213> Homo sapiens

<400> 3654

atctatacag gaacgaccct gttat                                          25

<210> 3655
<211> 25
<212> DNA
<213> Homo sapiens

<400> 3655
tctatacagg aacgaccctg ttatt                                          25

<210> 3656
<211> 25
<212> DNA
<213> Homo sapiens

<400> 3656
ctatacagga acgaccctgt tattt                                          25

<210> 3657
<211> 25
<212> DNA
<213> Homo sapiens

<400> 3657
tatacaggaa cgaccctgtt atttt                                          25

<210> 3658
<211> 25
<212> DNA
<213> Homo sapiens

<400> 3658
atacaggaac gaccctgtta ttttg                                          25

<210> 3659
<211> 25
<212> DNA
<213> Homo sapiens

<400> 3659
tacaggaacg accctgttat tttgc                                          25

<210> 3660
<211> 25
<212> DNA
<213> Homo sapiens

<400> 3660
acaggaacga ccctgttatt ttgct                                          25

<210> 3661
<211> 25
<212> DNA
<213> Homo sapiens

<400> 3661
caggaacgac cctgttattt tgcta                                          25

<210> 3662
<211> 25
<212> DNA

<213> Homo sapiens

<400> 3662
aggaacgacc ctgttatttt gctat                                          25

<210> 3663
<211> 25
<212> DNA
<213> Homo sapiens

<400> 3663
ggaacgaccc tgttattttg ctatt                                          25

<210> 3664
<211> 25
<212> DNA
<213> Homo sapiens

<400> 3664
gaacgaccct gttattttgc tattt                                          25

<210> 3665
<211> 25
<212> DNA
<213> Homo sapiens

<400> 3665
aacgaccctg ttattttgct atttt                                          25

<210> 3666
<211> 25
<212> DNA
<213> Homo sapiens

<400> 3666
acgaccctgt tattttgcta ttttt                                          25

<210> 3667
<211> 25
<212> DNA
<213> Homo sapiens

<400> 3667
cgaccctgtt attttgctat tttta                                          25

<210> 3668
<211> 25
<212> DNA
<213> Homo sapiens

<400> 3668
gaccctgtta ttttgctatt tttat                                          25

<210> 3669
<211> 25
<212> DNA
<213> Homo sapiens

<400> 3669
accctgttat tttgctattt ttata                                          25

<210> 3670
<211> 25
<212> DNA
<213> Homo sapiens

<400> 3670
ccctgttatt ttgctatttt tataa                                             25

<210> 3671
<211> 25
<212> DNA
<213> Homo sapiens

<400> 3671
cctgttattt tgctattttt ataac                                             25

<210> 3672
<211> 25
<212> DNA
<213> Homo sapiens

<400> 3672
ctgttatttt gctattttta taaca                                             25

<210> 3673
<211> 25
<212> DNA
<213> Homo sapiens

<400> 3673
tgttattttg ctatttttat aacat                                             25

<210> 3674
<211> 25
<212> DNA
<213> Homo sapiens

<400> 3674
gttattttgc tatttttata acatc                                             25

<210> 3675
<211> 25
<212> DNA
<213> Homo sapiens

<400> 3675
ttattttgct atttttataa catca                                             25

<210> 3676
<211> 25
<212> DNA
<213> Homo sapiens

<400> 3676
tattttgcta tttttataac atcac                                             25

<210> 3677
<211> 25
<212> DNA
<213> Homo sapiens

<400> 3677
attttgctat ttttataaca tcacg                                          25

<210> 3678
<211> 25
<212> DNA
<213> Homo sapiens

<400> 3678
ttttgctatt tttataacat cacgt                                          25

<210> 3679
<211> 25
<212> DNA
<213> Homo sapiens

<400> 3679
tttgctattt ttataacatc acgtg                                          25

<210> 3680
<211> 25
<212> DNA
<213> Homo sapiens

<400> 3680
ttgctatttt tataacatca cgtgt                                          25

<210> 3681
<211> 25
<212> DNA
<213> Homo sapiens

<400> 3681
tgctattttt ataacatcac gtgtt                                          25

<210> 3682
<211> 25
<212> DNA
<213> Homo sapiens

<400> 3682
gctattttta taacatcacg tgttt                                          25

<210> 3683
<211> 25
<212> DNA
<213> Homo sapiens

<400> 3683
ctatttttat aacatcacgt gtttt                                          25

<210> 3684
<211> 25
<212> DNA
<213> Homo sapiens

<400> 3684
tatttttata acatcacgtg ttttg                                          25

<210> 3685
<211> 25

<212> DNA
<213> Homo sapiens

<400> 3685
atttttataa catcacgtgt tttgg                                            25

<210> 3686
<211> 25
<212> DNA
<213> Homo sapiens

<400> 3686
tttttataac atcacgtgtt ttgga                                            25

<210> 3687
<211> 25
<212> DNA
<213> Homo sapiens

<400> 3687
ttttataaca tcacgtgttt tggag                                            25

<210> 3688
<211> 25
<212> DNA
<213> Homo sapiens

<400> 3688
tttataacat cacgtgtttt ggagc                                            25

<210> 3689
<211> 25
<212> DNA
<213> Homo sapiens

<400> 3689
ttataacatc acgtgttttg gagca                                            25

<210> 3690
<211> 25
<212> DNA
<213> Homo sapiens

<400> 3690
tataacatca cgtgttttgg agcat                                            25

<210> 3691
<211> 25
<212> DNA
<213> Homo sapiens

<400> 3691
ataacatcac gtgttttgga gcatt                                            25

<210> 3692
<211> 25
<212> DNA
<213> Homo sapiens

<400> 3692
taacatcacg tgttttggag cattt                                            25

<210> 3693
<211> 25
<212> DNA
<213> Homo sapiens

<400> 3693
aacatcacgt gttttggagc attta                                          25

<210> 3694
<211> 25
<212> DNA
<213> Homo sapiens

<400> 3694
acatcacgtg ttttggagca tttat                                          25

<210> 3695
<211> 25
<212> DNA
<213> Homo sapiens

<400> 3695
catcacgtgt tttggagcat ttatg                                          25

<210> 3696
<211> 25
<212> DNA
<213> Homo sapiens

<400> 3696
atcacgtgtt ttggagcatt tatgg                                          25

<210> 3697
<211> 25
<212> DNA
<213> Homo sapiens

<400> 3697
tcacgtgttt tggagcattt atggc                                          25

<210> 3698
<211> 25
<212> DNA
<213> Homo sapiens

<400> 3698
cacgtgtttt ggagcattta tggcc                                          25

<210> 3699
<211> 25
<212> DNA
<213> Homo sapiens

<400> 3699
acgtgttttg gagcatttat ggcct                                          25

<210> 3700
<211> 25
<212> DNA
<213> Homo sapiens

<400> 3700
cgtgttttgg agcatttatg gcctt                                          25

<210> 3701
<211> 25
<212> DNA
<213> Homo sapiens

<400> 3701
gtgttttgga gcatttatgg ccttg                                          25

<210> 3702
<211> 25
<212> DNA
<213> Homo sapiens

<400> 3702
tgttttggag catttatggc cttgg                                          25

<210> 3703
<211> 25
<212> DNA
<213> Homo sapiens

<400> 3703
gttttggagc atttatggcc ttgga                                          25

<210> 3704
<211> 25
<212> DNA
<213> Homo sapiens

<400> 3704
ttttggagca tttatggcct tggat                                          25

<210> 3705
<211> 25
<212> DNA
<213> Homo sapiens

<400> 3705
tttggagcat ttatggcctt ggatg                                          25

<210> 3706
<211> 25
<212> DNA
<213> Homo sapiens

<400> 3706
ttggagcatt tatggccttg gatgg                                          25

<210> 3707
<211> 25
<212> DNA
<213> Homo sapiens

<400> 3707
tggagcattt atggccttgg atggt                                          25

<210> 3708

<211> 25
<212> DNA
<213> Homo sapiens

<400> 3708
ggagcattta tggccttgga tggta                                            25

<210> 3709
<211> 25
<212> DNA
<213> Homo sapiens

<400> 3709
gagcatttat ggccttggat ggtaa                                            25

<210> 3710
<211> 25
<212> DNA
<213> Homo sapiens

<400> 3710
agcatttatg gccttggatg gtaaa                                            25

<210> 3711
<211> 25
<212> DNA
<213> Homo sapiens

<400> 3711
gcatttatgg ccttggatgg taaaa                                            25

<210> 3712
<211> 25
<212> DNA
<213> Homo sapiens

<400> 3712
catttatggc cttggatggt aaaag                                            25

<210> 3713
<211> 25
<212> DNA
<213> Homo sapiens

<400> 3713
atttatggcc ttggatggta aaaga                                            25

<210> 3714
<211> 25
<212> DNA
<213> Homo sapiens

<400> 3714
tttatggcct tggatggtaa aagag                                            25

<210> 3715
<211> 25
<212> DNA
<213> Homo sapiens

<400> 3715

ttatggcctt ggatggtaaa agaga 25

<210> 3716
<211> 25
<212> DNA
<213> Homo sapiens

<400> 3716
tatggccttg gatggtaaaa gagaa 25

<210> 3717
<211> 25
<212> DNA
<213> Homo sapiens

<400> 3717
atggccttgg atggtaaaag agaag 25

<210> 3718
<211> 25
<212> DNA
<213> Homo sapiens

<400> 3718
tggccttgga tggtaaaaga gaagt 25

<210> 3719
<211> 25
<212> DNA
<213> Homo sapiens

<400> 3719
ggccttggat ggtaaaagag aagta 25

<210> 3720
<211> 25
<212> DNA
<213> Homo sapiens

<400> 3720
gccttggatg gtaaaagaga agtag 25

<210> 3721
<211> 25
<212> DNA
<213> Homo sapiens

<400> 3721
ccttggatgg taaaagagaa gtagt 25

<210> 3722
<211> 25
<212> DNA
<213> Homo sapiens

<400> 3722
cttggatggt aaaagagaag tagtc 25

<210> 3723
<211> 25
<212> DNA

<213> Homo sapiens

<400> 3723
ttggatggta aaagagaagt agtct                                            25

<210> 3724
<211> 25
<212> DNA
<213> Homo sapiens

<400> 3724
tggatggtaa aagagaagta gtctg                                            25

<210> 3725
<211> 25
<212> DNA
<213> Homo sapiens

<400> 3725
ggatggtaaa agagaagtag tctgc                                            25

<210> 3726
<211> 25
<212> DNA
<213> Homo sapiens

<400> 3726
gatggtaaaa gagaagtagt ctgcc                                            25

<210> 3727
<211> 25
<212> DNA
<213> Homo sapiens

<400> 3727
atggtaaaag agaagtagtc tgcct                                            25

<210> 3728
<211> 25
<212> DNA
<213> Homo sapiens

<400> 3728
tggtaaaaga gaagtagtct gccta                                            25

<210> 3729
<211> 25
<212> DNA
<213> Homo sapiens

<400> 3729
ggtaaaagag aagtagtctg cctat                                            25

<210> 3730
<211> 25
<212> DNA
<213> Homo sapiens

<400> 3730
gtaaaagaga agtagtctgc ctatg                                            25

<210> 3731
<211> 25
<212> DNA
<213> Homo sapiens

<400> 3731
taaaagagaa gtagtctgcc tatgc                                              25

<210> 3732
<211> 25
<212> DNA
<213> Homo sapiens

<400> 3732
aaaagagaag tagtctgcct atgct                                              25

<210> 3733
<211> 25
<212> DNA
<213> Homo sapiens

<400> 3733
aaagagaagt agtctgccta tgctg                                              25

<210> 3734
<211> 25
<212> DNA
<213> Homo sapiens

<400> 3734
aagagaagta gtctgcctat gctgg                                              25

<210> 3735
<211> 25
<212> DNA
<213> Homo sapiens

<400> 3735
agagaagtag tctgcctatg ctggt                                              25

<210> 3736
<211> 25
<212> DNA
<213> Homo sapiens

<400> 3736
gagaagtagt ctgcctatgc tggtt                                              25

<210> 3737
<211> 25
<212> DNA
<213> Homo sapiens

<400> 3737
agaagtagtc tgcctatgct ggttg                                              25

<210> 3738
<211> 25
<212> DNA
<213> Homo sapiens

<400> 3738
gaagtagtct gcctatgctg gttga                                          25

<210> 3739
<211> 25
<212> DNA
<213> Homo sapiens

<400> 3739
aagtagtctg cctatgctgg ttgaa                                          25

<210> 3740
<211> 25
<212> DNA
<213> Homo sapiens

<400> 3740
agtagtctgc ctatgctggt tgaaa                                          25

<210> 3741
<211> 25
<212> DNA
<213> Homo sapiens

<400> 3741
gtagtctgcc tatgctggtt gaaaa                                          25

<210> 3742
<211> 25
<212> DNA
<213> Homo sapiens

<400> 3742
tagtctgcct atgctggttg aaaaa                                          25

<210> 3743
<211> 25
<212> DNA
<213> Homo sapiens

<400> 3743
agtctgccta tgctggttga aaaag                                          25

<210> 3744
<211> 25
<212> DNA
<213> Homo sapiens

<400> 3744
gtctgcctat gctggttgaa aaagg                                          25

<210> 3745
<211> 25
<212> DNA
<213> Homo sapiens

<400> 3745
tctgcctatg ctggttgaaa aaggc                                          25

<210> 3746
<211> 25

<212> DNA
<213> Homo sapiens

<400> 3746
ctgcctatgc tggttgaaaa aggct                                          25

<210> 3747
<211> 25
<212> DNA
<213> Homo sapiens

<400> 3747
tgcctatgct ggttgaaaaa ggctg                                          25

<210> 3748
<211> 25
<212> DNA
<213> Homo sapiens

<400> 3748
gcctatgctg gttgaaaaag gctga                                          25

<210> 3749
<211> 25
<212> DNA
<213> Homo sapiens

<400> 3749
cctatgctgg ttgaaaaagg ctgac                                          25

<210> 3750
<211> 25
<212> DNA
<213> Homo sapiens

<400> 3750
ctatgctggt tgaaaaaggc tgacc                                          25

<210> 3751
<211> 25
<212> DNA
<213> Homo sapiens

<400> 3751
tatgctggtt gaaaaaggct gaccc                                          25

<210> 3752
<211> 25
<212> DNA
<213> Homo sapiens

<400> 3752
atgctggttg aaaaaggctg accca                                          25

<210> 3753
<211> 25
<212> DNA
<213> Homo sapiens

<400> 3753
tgctggttga aaaaggctga cccaa                                          25

<210> 3754
<211> 25
<212> DNA
<213> Homo sapiens

<400> 3754
gctggttgaa aaaggctgac ccaaa                                          25

<210> 3755
<211> 25
<212> DNA
<213> Homo sapiens

<400> 3755
ctggttgaaa aaggctgacc caaaa                                          25

<210> 3756
<211> 25
<212> DNA
<213> Homo sapiens

<400> 3756
tggttgaaaa aggctgaccc aaaat                                          25

<210> 3757
<211> 25
<212> DNA
<213> Homo sapiens

<400> 3757
ggttgaaaaa ggctgaccca aaatg                                          25

<210> 3758
<211> 25
<212> DNA
<213> Homo sapiens

<400> 3758
gttgaaaaag gctgacccaa aatgg                                          25

<210> 3759
<211> 25
<212> DNA
<213> Homo sapiens

<400> 3759
ttgaaaaagg ctgacccaaa atggc                                          25

<210> 3760
<211> 25
<212> DNA
<213> Homo sapiens

<400> 3760
tgaaaaaggc tgacccaaaa tggcc                                          25

<210> 3761
<211> 25
<212> DNA
<213> Homo sapiens

<400> 3761
gaaaaaggct gacccaaaat ggccc                                          25

<210> 3762
<211> 25
<212> DNA
<213> Homo sapiens

<400> 3762
aaaaaggctg acccaaaatg gccct                                          25

<210> 3763
<211> 25
<212> DNA
<213> Homo sapiens

<400> 3763
aaaaggctga cccaaaatgg ccctc                                          25

<210> 3764
<211> 25
<212> DNA
<213> Homo sapiens

<400> 3764
aaaggctgac ccaaaatggc cctca                                          25

<210> 3765
<211> 25
<212> DNA
<213> Homo sapiens

<400> 3765
aaggctgacc caaaatggcc ctcat                                          25

<210> 3766
<211> 25
<212> DNA
<213> Homo sapiens

<400> 3766
aggctgaccc aaaatggccc tcatt                                          25

<210> 3767
<211> 25
<212> DNA
<213> Homo sapiens

<400> 3767
ggctgaccca aaatggccct cattt                                          25

<210> 3768
<211> 25
<212> DNA
<213> Homo sapiens

<400> 3768
gctgacccaa aatggccctc attta                                          25

<210> 3769

<211> 25
<212> DNA
<213> Homo sapiens

<400> 3769
ctgacccaaa atggccctca tttaa                                          25

<210> 3770
<211> 25
<212> DNA
<213> Homo sapiens

<400> 3770
tgacccaaaa tggccctcat ttaaa                                          25

<210> 3771
<211> 25
<212> DNA
<213> Homo sapiens

<400> 3771
gacccaaaat ggccctcatt taaaa                                          25

<210> 3772
<211> 25
<212> DNA
<213> Homo sapiens

<400> 3772
acccaaaatg gccctcattt aaaaa                                          25

<210> 3773
<211> 25
<212> DNA
<213> Homo sapiens

<400> 3773
cccaaaatgg ccctcattta aaaag                                          25

<210> 3774
<211> 25
<212> DNA
<213> Homo sapiens

<400> 3774
ccaaaatggc cctcatttaa aaagt                                          25

<210> 3775
<211> 25
<212> DNA
<213> Homo sapiens

<400> 3775
caaaatggcc ctcatttaaa aagtt                                          25

<210> 3776
<211> 25
<212> DNA
<213> Homo sapiens

<400> 3776

aaaatggccc tcatttaaaa agttc                                          25

<210> 3777
<211> 25
<212> DNA
<213> Homo sapiens

<400> 3777
aaatggccct catttaaaaa gttct                                          25

<210> 3778
<211> 25
<212> DNA
<213> Homo sapiens

<400> 3778
aatggccctc atttaaaaag ttctg                                          25

<210> 3779
<211> 25
<212> DNA
<213> Homo sapiens

<400> 3779
atggccctca tttaaaaagt tctgc                                          25

<210> 3780
<211> 25
<212> DNA
<213> Homo sapiens

<400> 3780
tggccctcat ttaaaaagtt ctgct                                          25

<210> 3781
<211> 25
<212> DNA
<213> Homo sapiens

<400> 3781
ggccctcatt taaaaagttc tgctg                                          25

<210> 3782
<211> 25
<212> DNA
<213> Homo sapiens

<400> 3782
gccctcattt aaaaagttct gctgt                                          25

<210> 3783
<211> 25
<212> DNA
<213> Homo sapiens

<400> 3783
ccctcattta aaaagttctg ctgtt                                          25

<210> 3784
<211> 25
<212> DNA

<213> Homo sapiens

<400> 3784
cctcatttaa aaagttctgc tgttt                                          25

<210> 3785
<211> 25
<212> DNA
<213> Homo sapiens

<400> 3785
ctcatttaaa aagttctgct gtttc                                          25

<210> 3786
<211> 25
<212> DNA
<213> Homo sapiens

<400> 3786
tcatttaaaa agttctgctg tttcc                                          25

<210> 3787
<211> 25
<212> DNA
<213> Homo sapiens

<400> 3787
catttaaaaa gttctgctgt ttccc                                          25

<210> 3788
<211> 25
<212> DNA
<213> Homo sapiens

<400> 3788
atttaaaaag ttctgctgtt tccca                                          25

<210> 3789
<211> 25
<212> DNA
<213> Homo sapiens

<400> 3789
tttaaaaagt tctgctgttt cccat                                          25

<210> 3790
<211> 25
<212> DNA
<213> Homo sapiens

<400> 3790
ttaaaaagtt ctgctgtttc ccatt                                          25

<210> 3791
<211> 25
<212> DNA
<213> Homo sapiens

<400> 3791
taaaaagttc tgctgtttcc cattt                                          25

<210> 3792
<211> 25
<212> DNA
<213> Homo sapiens

<400> 3792
aaaaagttct gctgtttccc atttg                                          25

<210> 3793
<211> 25
<212> DNA
<213> Homo sapiens

<400> 3793
aaaagttctg ctgtttccca tttgg                                          25

<210> 3794
<211> 25
<212> DNA
<213> Homo sapiens

<400> 3794
aaagttctgc tgtttcccat ttggt                                          25

<210> 3795
<211> 25
<212> DNA
<213> Homo sapiens

<400> 3795
aagttctgct gtttcccatt tggtt                                          25

<210> 3796
<211> 25
<212> DNA
<213> Homo sapiens

<400> 3796
agttctgctg tttcccattt ggttc                                          25

<210> 3797
<211> 25
<212> DNA
<213> Homo sapiens

<400> 3797
gttctgctgt ttcccatttg gttct                                          25

<210> 3798
<211> 25
<212> DNA
<213> Homo sapiens

<400> 3798
ttctgctgtt tcccatttgg ttctg                                          25

<210> 3799
<211> 25
<212> DNA
<213> Homo sapiens

<400> 3799
tctgctgttt cccatttggt tctgt                                          25

<210> 3800
<211> 25
<212> DNA
<213> Homo sapiens

<400> 3800
ctgctgtttc ccatttggtt ctgtc                                          25

<210> 3801
<211> 25
<212> DNA
<213> Homo sapiens

<400> 3801
tgctgtttcc catttggttc tgtcc                                          25

<210> 3802
<211> 25
<212> DNA
<213> Homo sapiens

<400> 3802
gctgtttccc atttggttct gtccc                                          25

<210> 3803
<211> 25
<212> DNA
<213> Homo sapiens

<400> 3803
ctgtttccca tttggttctg tccca                                          25

<210> 3804
<211> 25
<212> DNA
<213> Homo sapiens

<400> 3804
tgtttcccat ttggttctgt cccag                                          25

<210> 3805
<211> 25
<212> DNA
<213> Homo sapiens

<400> 3805
gtttcccatt tggttctgtc ccaga                                          25

<210> 3806
<211> 25
<212> DNA
<213> Homo sapiens

<400> 3806
tttcccattt ggttctgtcc cagat                                          25

<210> 3807
<211> 25

<212> DNA
<213> Homo sapiens

<400> 3807
ttcccatttg gttctgtccc agatg                                            25

<210> 3808
<211> 25
<212> DNA
<213> Homo sapiens

<400> 3808
tcccatttgg ttctgtccca gatga                                            25

<210> 3809
<211> 25
<212> DNA
<213> Homo sapiens

<400> 3809
cccatttggt tctgtcccag atgaa                                            25

<210> 3810
<211> 25
<212> DNA
<213> Homo sapiens

<400> 3810
ccatttggtt ctgtcccaga tgaac                                            25

<210> 3811
<211> 25
<212> DNA
<213> Homo sapiens

<400> 3811
catttggttc tgtcccagat gaaca                                            25

<210> 3812
<211> 25
<212> DNA
<213> Homo sapiens

<400> 3812
atttggttct gtcccagatg aacat                                            25

<210> 3813
<211> 25
<212> DNA
<213> Homo sapiens

<400> 3813
tttggttctg tcccagatga acatg                                            25

<210> 3814
<211> 25
<212> DNA
<213> Homo sapiens

<400> 3814
ttggttctgt cccagatgaa catgg                                            25

<210> 3815
<211> 25
<212> DNA
<213> Homo sapiens

<400> 3815
tggttctgtc ccagatgaac atgga                                          25

<210> 3816
<211> 25
<212> DNA
<213> Homo sapiens

<400> 3816
ggttctgtcc cagatgaaca tggaa                                          25

<210> 3817
<211> 25
<212> DNA
<213> Homo sapiens

<400> 3817
gttctgtccc agatgaacat ggaac                                          25

<210> 3818
<211> 25
<212> DNA
<213> Homo sapiens

<400> 3818
ttctgtccca gatgaacatg gaact                                          25

<210> 3819
<211> 25
<212> DNA
<213> Homo sapiens

<400> 3819
tctgtcccag atgaacatgg aactg                                          25

<210> 3820
<211> 25
<212> DNA
<213> Homo sapiens

<400> 3820
ctgtcccaga tgaacatgga actga                                          25

<210> 3821
<211> 25
<212> DNA
<213> Homo sapiens

<400> 3821
tgtcccagat gaacatggaa ctgat                                          25

<210> 3822
<211> 25
<212> DNA
<213> Homo sapiens

<400> 3822
gtcccagatg aacatggaac tgata                                          25

<210> 3823
<211> 25
<212> DNA
<213> Homo sapiens

<400> 3823
tcccagatga acatggaact gatat                                          25

<210> 3824
<211> 25
<212> DNA
<213> Homo sapiens

<400> 3824
cccagatgaa catggaactg atatc                                          25

<210> 3825
<211> 25
<212> DNA
<213> Homo sapiens

<400> 3825
ccagatgaac atggaactga tatcc                                          25

<210> 3826
<211> 25
<212> DNA
<213> Homo sapiens

<400> 3826
cagatgaaca tggaactgat atcca                                          25

<210> 3827
<211> 25
<212> DNA
<213> Homo sapiens

<400> 3827
agatgaacat ggaactgata tccat                                          25

<210> 3828
<211> 25
<212> DNA
<213> Homo sapiens

<400> 3828
gatgaacatg gaactgatat ccatc                                          25

<210> 3829
<211> 25
<212> DNA
<213> Homo sapiens

<400> 3829
atgaacatgg aactgatatc catcc                                          25

<210> 3830

<211> 25
<212> DNA
<213> Homo sapiens

<400> 3830
tgaacatgga actgatatcc atcca                                          25

<210> 3831
<211> 25
<212> DNA
<213> Homo sapiens

<400> 3831
gaacatggaa ctgatatcca tccaa                                          25

<210> 3832
<211> 25
<212> DNA
<213> Homo sapiens

<400> 3832
aacatggaac tgatatccat ccaat                                          25

<210> 3833
<211> 25
<212> DNA
<213> Homo sapiens

<400> 3833
acatggaact gatatccatc caatg                                          25

<210> 3834
<211> 25
<212> DNA
<213> Homo sapiens

<400> 3834
catggaactg atatccatcc aatga                                          25

<210> 3835
<211> 25
<212> DNA
<213> Homo sapiens

<400> 3835
atggaactga tatccatcca atgag                                          25

<210> 3836
<211> 25
<212> DNA
<213> Homo sapiens

<400> 3836
tggaactgat atccatccaa tgagt                                          25

<210> 3837
<211> 25
<212> DNA
<213> Homo sapiens

<400> 3837

ggaactgata tccatccaat gagtt 25

<210> 3838
<211> 25
<212> DNA
<213> Homo sapiens

<400> 3838
gaactgatat ccatccaatg agttt 25

<210> 3839
<211> 25
<212> DNA
<213> Homo sapiens

<400> 3839
aactgatatc catccaatga gtttg 25

<210> 3840
<211> 25
<212> DNA
<213> Homo sapiens

<400> 3840
actgatatcc atccaatgag tttgt 25

<210> 3841
<211> 25
<212> DNA
<213> Homo sapiens

<400> 3841
ctgatatcca tccaatgagt ttgtt 25

<210> 3842
<211> 25
<212> DNA
<213> Homo sapiens

<400> 3842
tgatatccat ccaatgagtt tgttt 25

<210> 3843
<211> 25
<212> DNA
<213> Homo sapiens

<400> 3843
gatatccatc caatgagttt gtttt 25

<210> 3844
<211> 25
<212> DNA
<213> Homo sapiens

<400> 3844
atatccatcc aatgagtttg ttttt 25

<210> 3845
<211> 25
<212> DNA

<213> Homo sapiens

<400> 3845
tatccatcca atgagtttgt ttttt                                          25

<210> 3846
<211> 25
<212> DNA
<213> Homo sapiens

<400> 3846
atccatccaa tgagtttgtt tttta                                          25

<210> 3847
<211> 25
<212> DNA
<213> Homo sapiens

<400> 3847
tccatccaat gagtttgttt tttag                                          25

<210> 3848
<211> 25
<212> DNA
<213> Homo sapiens

<400> 3848
ccatccaatg agtttgtttt ttaga                                          25

<210> 3849
<211> 25
<212> DNA
<213> Homo sapiens

<400> 3849
catccaatga gtttgttttt tagag                                          25

<210> 3850
<211> 25
<212> DNA
<213> Homo sapiens

<400> 3850
atccaatgag tttgtttttt agaga                                          25

<210> 3851
<211> 25
<212> DNA
<213> Homo sapiens

<400> 3851
tccaatgagt ttgtttttta gagac                                          25

<210> 3852
<211> 25
<212> DNA
<213> Homo sapiens

<400> 3852
ccaatgagtt tgttttttag agact                                          25

<210> 3853
<211> 25
<212> DNA
<213> Homo sapiens

<400> 3853
   caatgagttt gttttttaga gacta                                          25

<210> 3854
<211> 25
<212> DNA
<213> Homo sapiens

<400> 3854
aatgagtttg ttttttagag actat                                           25

<210> 3855
<211> 25
<212> DNA
<213> Homo sapiens

<400> 3855
atgagtttgt tttttagaga ctatt                                           25

<210> 3856
<211> 25
<212> DNA
<213> Homo sapiens

<400> 3856
tgagtttgtt ttttagagac tattt                                           25

<210> 3857
<211> 25
<212> DNA
<213> Homo sapiens

<400> 3857
gagtttgttt tttagagact atttt                                           25

<210> 3858
<211> 25
<212> DNA
<213> Homo sapiens

<400> 3858
agtttgtttt ttagagacta ttttg                                           25

<210> 3859
<211> 25
<212> DNA
<213> Homo sapiens

<400> 3859
gtttgttttt tagagactat tttgg                                           25

<210> 3860
<211> 25
<212> DNA
<213> Homo sapiens

<400> 3860
tttgtttttt agagactatt ttggc                                          25

<210> 3861
<211> 25
<212> DNA
<213> Homo sapiens

<400> 3861
ttgtttttta gagactattt tggcc                                          25

<210> 3862
<211> 25
<212> DNA
<213> Homo sapiens

<400> 3862
tgttttttag agactatttt ggccc                                          25

<210> 3863
<211> 25
<212> DNA
<213> Homo sapiens

<400> 3863
gttttttaga gactattttg gcccc                                          25

<210> 3864
<211> 25
<212> DNA
<213> Homo sapiens

<400> 3864
ttttttagag actattttgg cccct                                          25

<210> 3865
<211> 25
<212> DNA
<213> Homo sapiens

<400> 3865
tttttagaga ctattttggc ccctt                                          25

<210> 3866
<211> 25
<212> DNA
<213> Homo sapiens

<400> 3866
ttttagagac tattttggcc ccttt                                          25

<210> 3867
<211> 25
<212> DNA
<213> Homo sapiens

<400> 3867
tttagagact attttggccc ctttc                                          25

<210> 3868
<211> 25

<212> DNA
<213> Homo sapiens

<400> 3868
ttagagacta ttttggcccc tttct                                          25

<210> 3869
<211> 25
<212> DNA
<213> Homo sapiens

<400> 3869
tagagactat tttggcccct ttctc                                          25

<210> 3870
<211> 25
<212> DNA
<213> Homo sapiens

<400> 3870
agagactatt ttggcccctt tctca                                          25

<210> 3871
<211> 25
<212> DNA
<213> Homo sapiens

<400> 3871
gagactattt tggccccttt ctcac                                          25

<210> 3872
<211> 25
<212> DNA
<213> Homo sapiens

<400> 3872
agactatttt ggcccctttc tcaca                                          25

<210> 3873
<211> 25
<212> DNA
<213> Homo sapiens

<400> 3873
gactattttg gcccctttct cacaa                                          25

<210> 3874
<211> 25
<212> DNA
<213> Homo sapiens

<400> 3874
actattttgg cccctttctc acaag                                          25

<210> 3875
<211> 25
<212> DNA
<213> Homo sapiens

<400> 3875
ctattttggc ccctttctca caagg                                          25

<210> 3876
<211> 25
<212> DNA
<213> Homo sapiens

<400> 3876
tattttggcc cctttctcac aagga                                            25

<210> 3877
<211> 25
<212> DNA
<213> Homo sapiens

<400> 3877
attttggccc ctttctcaca aggag                                            25

<210> 3878
<211> 25
<212> DNA
<213> Homo sapiens

<400> 3878
ttttggcccc tttctcacaa ggagt                                            25

<210> 3879
<211> 25
<212> DNA
<213> Homo sapiens

<400> 3879
tttggcccct ttctcacaag gagtg                                            25

<210> 3880
<211> 25
<212> DNA
<213> Homo sapiens

<400> 3880
ttggcccctt tctcacaagg agtga                                            25

<210> 3881
<211> 25
<212> DNA
<213> Homo sapiens

<400> 3881
tggccccttt ctcacaagga gtgag                                            25

<210> 3882
<211> 25
<212> DNA
<213> Homo sapiens

<400> 3882
ggcccctttc tcacaaggag tgagt                                            25

<210> 3883
<211> 25
<212> DNA
<213> Homo sapiens

<400> 3883
   gcccctttct cacaaggagt gagtc                                          25

<210> 3884
<211> 25
<212> DNA
<213> Homo sapiens

<400> 3884
cccctttctc acaaggagtg agtcc                                           25

<210> 3885
<211> 25
<212> DNA
<213> Homo sapiens

<400> 3885
ccctttctca caaggagtga gtcca                                           25

<210> 3886
<211> 25
<212> DNA
<213> Homo sapiens

<400> 3886
cctttctcac aaggagtgag tccaa                                           25

<210> 3887
<211> 25
<212> DNA
<213> Homo sapiens

<400> 3887
ctttctcaca aggagtgagt ccaag                                           25

<210> 3888
<211> 25
<212> DNA
<213> Homo sapiens

<400> 3888
tttctcacaa ggagtgagtc caagt                                           25

<210> 3889
<211> 25
<212> DNA
<213> Homo sapiens

<400> 3889
ttctcacaag gagtgagtcc aagta                                           25

<210> 3890
<211> 25
<212> DNA
<213> Homo sapiens

<400> 3890
tctcacaagg agtgagtcca agtat                                           25

<210> 3891

<211> 25
<212> DNA
<213> Homo sapiens

<400> 3891
ctcacaagga gtgagtccaa gtatt                                          25

<210> 3892
<211> 25
<212> DNA
<213> Homo sapiens

<400> 3892
tcacaaggag tgagtccaag tattt                                          25

<210> 3893
<211> 25
<212> DNA
<213> Homo sapiens

<400> 3893
cacaaggagt gagtccaagt atttt                                          25

<210> 3894
<211> 25
<212> DNA
<213> Homo sapiens

<400> 3894
acaaggagtg agtccaagta ttttg                                          25

<210> 3895
<211> 25
<212> DNA
<213> Homo sapiens

<400> 3895
caaggagtga gtccaagtat tttgt                                          25

<210> 3896
<211> 25
<212> DNA
<213> Homo sapiens

<400> 3896
aaggagtgag tccaagtatt ttgta                                          25

<210> 3897
<211> 25
<212> DNA
<213> Homo sapiens

<400> 3897
aggagtgagt ccaagtattt tgtag                                          25

<210> 3898
<211> 25
<212> DNA
<213> Homo sapiens

<400> 3898

ggagtgagtc caagtatttt gtagt 25

<210> 3899
<211> 25
<212> DNA
<213> Homo sapiens

<400> 3899
gagtgagtcc aagtattttg tagtc 25

<210> 3900
<211> 25
<212> DNA
<213> Homo sapiens

<400> 3900
agtgagtcca agtattttgt agtct 25

<210> 3901
<211> 25
<212> DNA
<213> Homo sapiens

<400> 3901
gtgagtccaa gtattttgta gtctt 25

<210> 3902
<211> 25
<212> DNA
<213> Homo sapiens

<400> 3902
tgagtccaag tattttgtag tcttt 25

<210> 3903
<211> 25
<212> DNA
<213> Homo sapiens

<400> 3903
gagtccaagt attttgtagt cttta 25

<210> 3904
<211> 25
<212> DNA
<213> Homo sapiens

<400> 3904
agtccaagta ttttgtagtc tttat 25

<210> 3905
<211> 25
<212> DNA
<213> Homo sapiens

<400> 3905
gtccaagtat tttgtagtct ttata 25

<210> 3906
<211> 25
<212> DNA

<213> Homo sapiens

<400> 3906
tccaagtatt ttgtagtctt tatat                                          25

<210> 3907
<211> 25
<212> DNA
<213> Homo sapiens

<400> 3907
ccaagtattt tgtagtcttt atata                                          25

<210> 3908
<211> 25
<212> DNA
<213> Homo sapiens

<400> 3908
caagtatttt gtagtcttta tatat                                          25

<210> 3909
<211> 25
<212> DNA
<213> Homo sapiens

<400> 3909
aagtattttg tagtctttat atatg                                          25

<210> 3910
<211> 25
<212> DNA
<213> Homo sapiens

<400> 3910
agtattttgt agtctttata tatgt                                          25

<210> 3911
<211> 25
<212> DNA
<213> Homo sapiens

<400> 3911
gtattttgta gtctttatat atgtt                                          25

<210> 3912
<211> 25
<212> DNA
<213> Homo sapiens

<400> 3912
tattttgtag tctttatata tgttt                                          25

<210> 3913
<211> 25
<212> DNA
<213> Homo sapiens

<400> 3913
attttgtagt ctttatatat gtttt                                          25

<210> 3914
<211> 25
<212> DNA
<213> Homo sapiens

<400> 3914
ttttgtagtc tttatatatg ttttg                                          25

<210> 3915
<211> 25
<212> DNA
<213> Homo sapiens

<400> 3915
tttgtagtct ttatatatgt tttgt                                          25

<210> 3916
<211> 25
<212> DNA
<213> Homo sapiens

<400> 3916
ttgtagtctt tatatatgtt ttgta                                          25

<210> 3917
<211> 25
<212> DNA
<213> Homo sapiens

<400> 3917
tgtagtcttt atatatgttt tgtac                                          25

<210> 3918
<211> 25
<212> DNA
<213> Homo sapiens

<400> 3918
gtagtcttta tatatgtttt gtaca                                          25

<210> 3919
<211> 25
<212> DNA
<213> Homo sapiens

<400> 3919
tagtctttat atatgttttg tacat                                          25

<210> 3920
<211> 25
<212> DNA
<213> Homo sapiens

<400> 3920
agtctttata tatgttttgt acatc                                          25

<210> 3921
<211> 25
<212> DNA
<213> Homo sapiens

<400> 3921
gtctttatat atgttttgta catca                                          25

<210> 3922
<211> 25
<212> DNA
<213> Homo sapiens

<400> 3922
tctttatata tgttttgtac atcat                                          25

<210> 3923
<211> 25
<212> DNA
<213> Homo sapiens

<400> 3923
ctttatatat gttttgtaca tcata                                          25

<210> 3924
<211> 25
<212> DNA
<213> Homo sapiens

<400> 3924
tttatatatg ttttgtacat cataa                                          25

<210> 3925
<211> 25
<212> DNA
<213> Homo sapiens

<400> 3925
ttatatatgt tttgtacatc ataag                                          25

<210> 3926
<211> 25
<212> DNA
<213> Homo sapiens

<400> 3926
tatatatgtt ttgtacatca taagc                                          25

<210> 3927
<211> 25
<212> DNA
<213> Homo sapiens

<400> 3927
atatatgttt tgtacatcat aagca                                          25

<210> 3928
<211> 25
<212> DNA
<213> Homo sapiens

<400> 3928
tatatgtttt gtacatcata agcag                                          25

<210> 3929
<211> 25

<212> DNA
<213> Homo sapiens

<400> 3929
atatgttttg tacatcataa gcagt                                            25

<210> 3930
<211> 25
<212> DNA
<213> Homo sapiens

<400> 3930
tatgttttgt acatcataag cagta                                            25

<210> 3931
<211> 25
<212> DNA
<213> Homo sapiens

<400> 3931
atgttttgta catcataagc agtat                                            25

<210> 3932
<211> 25
<212> DNA
<213> Homo sapiens

<400> 3932
tgttttgtac atcataagca gtata                                            25

<210> 3933
<211> 25
<212> DNA
<213> Homo sapiens

<400> 3933
gttttgtaca tcataagcag tatat                                            25

<210> 3934
<211> 25
<212> DNA
<213> Homo sapiens

<400> 3934
ttttgtacat cataagcagt atata                                            25

<210> 3935
<211> 25
<212> DNA
<213> Homo sapiens

<400> 3935
tttgtacatc ataagcagta tatat                                            25

<210> 3936
<211> 25
<212> DNA
<213> Homo sapiens

<400> 3936
ttgtacatca taagcagtat atatg                                            25

<210> 3937
<211> 25
<212> DNA
<213> Homo sapiens

<400> 3937
tgtacatcat aagcagtata tatgg                                          25

<210> 3938
<211> 25
<212> DNA
<213> Homo sapiens

<400> 3938
gtacatcata agcagtatat atggg                                          25

<210> 3939
<211> 25
<212> DNA
<213> Homo sapiens

<400> 3939
tacatcataa gcagtatata tgggt                                          25

<210> 3940
<211> 25
<212> DNA
<213> Homo sapiens

<400> 3940
acatcataag cagtatatat gggtg                                          25

<210> 3941
<211> 25
<212> DNA
<213> Homo sapiens

<400> 3941
catcataagc agtatatatg ggtgt                                          25

<210> 3942
<211> 25
<212> DNA
<213> Homo sapiens

<400> 3942
atcataagca gtatatatgg gtgtt                                          25

<210> 3943
<211> 25
<212> DNA
<213> Homo sapiens

<400> 3943
tcataagcag tatatatggg tgttt                                          25

<210> 3944
<211> 25
<212> DNA
<213> Homo sapiens

<400> 3944
cataagcagt atatatgggt gtttc                                          25

<210> 3945
<211> 25
<212> DNA
<213> Homo sapiens

<400> 3945
ataagcagta tatatgggtg tttcc                                          25

<210> 3946
<211> 25
<212> DNA
<213> Homo sapiens

<400> 3946
taagcagtat atatgggtgt ttcca                                          25

<210> 3947
<211> 25
<212> DNA
<213> Homo sapiens

<400> 3947
aagcagtata tatgggtgtt tccat                                          25

<210> 3948
<211> 25
<212> DNA
<213> Homo sapiens

<400> 3948
agcagtatat atgggtgttt ccatg                                          25

<210> 3949
<211> 25
<212> DNA
<213> Homo sapiens

<400> 3949
gcagtatata tgggtgtttc catgt                                          25

<210> 3950
<211> 25
<212> DNA
<213> Homo sapiens

<400> 3950
cagtatatat gggtgtttcc atgtg                                          25

<210> 3951
<211> 25
<212> DNA
<213> Homo sapiens

<400> 3951
agtatatatg ggtgtttcca tgtgc                                          25

<210> 3952

<211> 25
<212> DNA
<213> Homo sapiens

<400> 3952
gtatatatgg gtgtttccat gtgca                                          25

<210> 3953
<211> 25
<212> DNA
<213> Homo sapiens

<400> 3953
tatatatggg tgtttccatg tgcag                                          25

<210> 3954
<211> 25
<212> DNA
<213> Homo sapiens

<400> 3954
atatatgggt gtttccatgt gcagg                                          25

<210> 3955
<211> 25
<212> DNA
<213> Homo sapiens

<400> 3955
tatatgggtg tttccatgtg cagga                                          25

<210> 3956
<211> 25
<212> DNA
<213> Homo sapiens

<400> 3956
atatgggtgt ttccatgtgc aggaa                                          25

<210> 3957
<211> 25
<212> DNA
<213> Homo sapiens

<400> 3957
tatgggtgtt tccatgtgca ggaag                                          25

<210> 3958
<211> 25
<212> DNA
<213> Homo sapiens

<400> 3958
atgggtgttt ccatgtgcag gaagg                                          25

<210> 3959
<211> 25
<212> DNA
<213> Homo sapiens

<400> 3959

tgggtgtttc catgtgcagg aaggt                                          25

<210> 3960
<211> 25
<212> DNA
<213> Homo sapiens

<400> 3960
gggtgtttcc atgtgcagga aggtt                                          25

<210> 3961
<211> 25
<212> DNA
<213> Homo sapiens

<400> 3961
ggtgtttcca tgtgcaggaa ggttt                                          25

<210> 3962
<211> 25
<212> DNA
<213> Homo sapiens

<400> 3962
gtgtttccat gtgcaggaag gttta                                          25

<210> 3963
<211> 25
<212> DNA
<213> Homo sapiens

<400> 3963
tgtttccatg tgcaggaagg tttag                                          25

<210> 3964
<211> 25
<212> DNA
<213> Homo sapiens

<400> 3964
gtttccatgt gcaggaaggt ttaga                                          25

<210> 3965
<211> 25
<212> DNA
<213> Homo sapiens

<400> 3965
tttccatgtg caggaaggtt tagac                                          25

<210> 3966
<211> 25
<212> DNA
<213> Homo sapiens

<400> 3966
ttccatgtgc aggaaggttt agacc                                          25

<210> 3967
<211> 25
<212> DNA

<213> Homo sapiens

<400> 3967
tccatgtgca ggaaggttta gacct                                          25

<210> 3968
<211> 25
<212> DNA
<213> Homo sapiens

<400> 3968
ccatgtgcag gaaggtttag acctt                                          25

<210> 3969
<211> 25
<212> DNA
<213> Homo sapiens

<400> 3969
catgtgcagg aaggtttaga ccttc                                          25

<210> 3970
<211> 25
<212> DNA
<213> Homo sapiens

<400> 3970
atgtgcagga aggtttagac cttcg                                          25

<210> 3971
<211> 25
<212> DNA
<213> Homo sapiens

<400> 3971
tgtgcaggaa ggtttagacc ttcga                                          25

<210> 3972
<211> 25
<212> DNA
<213> Homo sapiens

<400> 3972
gtgcaggaag gtttagacct tcgaa                                          25

<210> 3973
<211> 25
<212> DNA
<213> Homo sapiens

<400> 3973
tgcaggaagg tttagacctt cgaaa                                          25

<210> 3974
<211> 25
<212> DNA
<213> Homo sapiens

<400> 3974
gcaggaaggt ttagaccttc gaaat                                          25

<210> 3975
<211> 25
<212> DNA
<213> Homo sapiens

<400> 3975
caggaaggtt tagaccttcg aaatc                                          25

<210> 3976
<211> 25
<212> DNA
<213> Homo sapiens

<400> 3976
aggaaggttt agaccttcga aatct                                          25

<210> 3977
<211> 25
<212> DNA
<213> Homo sapiens

<400> 3977
ggaaggttta gaccttcgaa atctg                                          25

<210> 3978
<211> 25
<212> DNA
<213> Homo sapiens

<400> 3978
gaaggtttag accttcgaaa tctgg                                          25

<210> 3979
<211> 25
<212> DNA
<213> Homo sapiens

<400> 3979
aaggtttaga ccttcgaaat ctggc                                          25

<210> 3980
<211> 25
<212> DNA
<213> Homo sapiens

<400> 3980
aggtttagac cttcgaaatc tggca                                          25

<210> 3981
<211> 25
<212> DNA
<213> Homo sapiens

<400> 3981
ggtttagacc ttcgaaatct ggcaa                                          25

<210> 3982
<211> 25
<212> DNA
<213> Homo sapiens

<400> 3982
gtttagacct tcgaaatctg gcaag                                          25

<210> 3983
<211> 25
<212> DNA
<213> Homo sapiens

<400> 3983
tttagacctt cgaaatctgg caagt                                          25

<210> 3984
<211> 25
<212> DNA
<213> Homo sapiens

<400> 3984
ttagaccttc gaaatctggc aagtg                                          25

<210> 3985
<211> 25
<212> DNA
<213> Homo sapiens

<400> 3985
tagaccttcg aaatctggca agtga                                          25

<210> 3986
<211> 25
<212> DNA
<213> Homo sapiens

<400> 3986
agaccttcga aatctggcaa gtgac                                          25

<210> 3987
<211> 25
<212> DNA
<213> Homo sapiens

<400> 3987
gaccttcgaa atctggcaag tgacg                                          25

<210> 3988
<211> 25
<212> DNA
<213> Homo sapiens

<400> 3988
accttcgaaa tctggcaagt gacga                                          25

<210> 3989
<211> 25
<212> DNA
<213> Homo sapiens

<400> 3989
ccttcgaaat ctggcaagtg acgat                                          25

<210> 3990
<211> 25

<212> DNA
<213> Homo sapiens

<400> 3990
cttcgaaatc tggcaagtga cgatt                                          25

<210> 3991
<211> 25
<212> DNA
<213> Homo sapiens

<400> 3991
ttcgaaatct ggcaagtgac gattc                                          25

<210> 3992
<211> 25
<212> DNA
<213> Homo sapiens

<400> 3992
tcgaaatctg gcaagtgacg attcc                                          25

<210> 3993
<211> 25
<212> DNA
<213> Homo sapiens

<400> 3993
cgaaatctgg caagtgacga ttcct                                          25

<210> 3994
<211> 25
<212> DNA
<213> Homo sapiens

<400> 3994
gaaatctggc aagtgacgat tccta                                          25

<210> 3995
<211> 25
<212> DNA
<213> Homo sapiens

<400> 3995
aaatctggca agtgacgatt cctac                                          25

<210> 3996
<211> 25
<212> DNA
<213> Homo sapiens

<400> 3996
aatctggcaa gtgacgattc ctaca                                          25

<210> 3997
<211> 25
<212> DNA
<213> Homo sapiens

<400> 3997
atctggcaag tgacgattcc tacat                                          25

<210> 3998
<211> 25
<212> DNA
<213> Homo sapiens

<400> 3998
tctggcaagt gacgattcct acatc                                          25

<210> 3999
<211> 25
<212> DNA
<213> Homo sapiens

<400> 3999
ctggcaagtg acgattccta catca                                          25

<210> 4000
<211> 25
<212> DNA
<213> Homo sapiens

<400> 4000
tggcaagtga cgattcctac atcac                                          25

<210> 4001
<211> 25
<212> DNA
<213> Homo sapiens

<400> 4001
ggcaagtgac gattcctaca tcaca                                          25

<210> 4002
<211> 25
<212> DNA
<213> Homo sapiens

<400> 4002
gcaagtgacg attcctacat cacac                                          25

<210> 4003
<211> 25
<212> DNA
<213> Homo sapiens

<400> 4003
caagtgacga ttcctacatc acacc                                          25

<210> 4004
<211> 25
<212> DNA
<213> Homo sapiens

<400> 4004
aagtgacgat tcctacatca cacca                                          25

<210> 4005
<211> 25
<212> DNA
<213> Homo sapiens

<400> 4005
agtgacgatt cctacatcac accat                                          25

<210> 4006
<211> 25
<212> DNA
<213> Homo sapiens

<400> 4006
gtgacgattc ctacatcaca ccata                                          25

<210> 4007
<211> 25
<212> DNA
<213> Homo sapiens

<400> 4007
tgacgattcc tacatcacac catat                                          25

<210> 4008
<211> 25
<212> DNA
<213> Homo sapiens

<400> 4008
gacgattcct acatcacacc atatt                                          25

<210> 4009
<211> 25
<212> DNA
<213> Homo sapiens

<400> 4009
acgattccta catcacacca tattt                                          25

<210> 4010
<211> 25
<212> DNA
<213> Homo sapiens

<400> 4010
cgattcctac atcacaccat atttt                                          25

<210> 4011
<211> 25
<212> DNA
<213> Homo sapiens

<400> 4011
gattcctaca tcacaccata tttta                                          25

<210> 4012
<211> 25
<212> DNA
<213> Homo sapiens

<400> 4012
attcctacat cacaccatat tttaa                                          25

<210> 4013

<211> 25
<212> DNA
<213> Homo sapiens

<400> 4013
ttcctacatc acaccatatt ttaac                                          25

<210> 4014
<211> 25
<212> DNA
<213> Homo sapiens

<400> 4014
tcctacatca caccatattt taacg                                          25

<210> 4015
<211> 25
<212> DNA
<213> Homo sapiens

<400> 4015
cctacatcac accatatttt aacgt                                          25

<210> 4016
<211> 25
<212> DNA
<213> Homo sapiens

<400> 4016
ctacatcaca ccatatttta acgta                                          25

<210> 4017
<211> 25
<212> DNA
<213> Homo sapiens

<400> 4017
tacatcacac catattttaa cgtag                                          25

<210> 4018
<211> 25
<212> DNA
<213> Homo sapiens

<400> 4018
acatcacacc atattttaac gtaga                                          25

<210> 4019
<211> 25
<212> DNA
<213> Homo sapiens

<400> 4019
catcacacca tattttaacg tagag                                          25

<210> 4020
<211> 25
<212> DNA
<213> Homo sapiens

<400> 4020

atcacaccat attttaacgt agagg 25

<210> 4021
<211> 25
<212> DNA
<213> Homo sapiens

<400> 4021
tcacaccata ttttaacgta gagga 25

<210> 4022
<211> 25
<212> DNA
<213> Homo sapiens

<400> 4022
cacaccatat tttaacgtag aggag 25

<210> 4023
<211> 25
<212> DNA
<213> Homo sapiens

<400> 4023
acaccatatt ttaacgtaga ggaga 25

<210> 4024
<211> 25
<212> DNA
<213> Homo sapiens

<400> 4024
caccatattt taacgtagag gagaa 25

<210> 4025
<211> 25
<212> DNA
<213> Homo sapiens

<400> 4025
accatatttt aacgtagagg agaat 25

<210> 4026
<211> 17
<212> DNA
<213> Homo sapiens

<400> 4026
tccatgatta atcttgt 17

<210> 4027
<211> 17
<212> DNA
<213> Homo sapiens

<400> 4027
ccatgattaa tcttgtc 17

<210> 4028
<211> 17
<212> DNA

<213> Homo sapiens

<400> 4028
catgattaat cttgtca                                                    17

<210> 4029
<211> 17
<212> DNA
<213> Homo sapiens

<400> 4029
atgattaatc ttgtcat                                                    17

<210> 4030
<211> 17
<212> DNA
<213> Homo sapiens

<400> 4030
tgattaatct tgtcatt                                                    17

<210> 4031
<211> 17
<212> DNA
<213> Homo sapiens

<400> 4031
gattaatctt gtcattt                                                    17

<210> 4032
<211> 17
<212> DNA
<213> Homo sapiens

<400> 4032
attaatcttg tcatttg                                                    17

<210> 4033
<211> 17
<212> DNA
<213> Homo sapiens

<400> 4033
ttaatcttgt catttgt                                                    17

<210> 4034
<211> 17
<212> DNA
<213> Homo sapiens

<400> 4034
taatcttgtc atttgta                                                    17

<210> 4035
<211> 17
<212> DNA
<213> Homo sapiens

<400> 4035
aatcttgtca tttgtac                                                    17

<210> 4036
<211> 17
<212> DNA
<213> Homo sapiens

<400> 4036
atcttgtcat ttgtaca                                                    17

<210> 4037
<211> 17
<212> DNA
<213> Homo sapiens

<400> 4037
tcttgtcatt tgtacag                                                    17

<210> 4038
<211> 17
<212> DNA
<213> Homo sapiens

<400> 4038
cttgtcattt gtacagg                                                    17

<210> 4039
<211> 17
<212> DNA
<213> Homo sapiens

<400> 4039
ttgtcatttg tacaggg                                                    17

<210> 4040
<211> 17
<212> DNA
<213> Homo sapiens

<400> 4040
tgtcatttgt acagggt                                                    17

<210> 4041
<211> 17
<212> DNA
<213> Homo sapiens

<400> 4041
gtcatttgta cagggtt                                                    17

<210> 4042
<211> 17
<212> DNA
<213> Homo sapiens

<400> 4042
tcatttgtac agggttt                                                    17

<210> 4043
<211> 17
<212> DNA
<213> Homo sapiens

<400> 4043
catttgtaca gggtttt                                                    17

<210> 4044
<211> 17
<212> DNA
<213> Homo sapiens

<400> 4044
atttgtacag ggttttc                                                    17

<210> 4045
<211> 17
<212> DNA
<213> Homo sapiens

<400> 4045
tttgtacagg gttttct                                                    17

<210> 4046
<211> 17
<212> DNA
<213> Homo sapiens

<400> 4046
ttgtacaggg ttttctt                                                    17

<210> 4047
<211> 17
<212> DNA
<213> Homo sapiens

<400> 4047
tgtacagggt tttcttt                                                    17

<210> 4048
<211> 17
<212> DNA
<213> Homo sapiens

<400> 4048
gtacagggtt ttctttc                                                    17

<210> 4049
<211> 17
<212> DNA
<213> Homo sapiens

<400> 4049
tacagggttt tctttcg                                                    17

<210> 4050
<211> 17
<212> DNA
<213> Homo sapiens

<400> 4050
acagggtttt ctttcga                                                    17

<210> 4051
<211> 17

<212> DNA
<213> Homo sapiens

<400> 4051
cagggttttc tttcgat                                                    17

<210> 4052
<211> 17
<212> DNA
<213> Homo sapiens

<400> 4052
agggttttct ttcgatt                                                    17

<210> 4053
<211> 17
<212> DNA
<213> Homo sapiens

<400> 4053
gggttttctt tcgattt                                                    17

<210> 4054
<211> 17
<212> DNA
<213> Homo sapiens

<400> 4054
ggttttcttt cgatttt                                                    17

<210> 4055
<211> 17
<212> DNA
<213> Homo sapiens

<400> 4055
gttttctttc gattttt                                                    17

<210> 4056
<211> 17
<212> DNA
<213> Homo sapiens

<400> 4056
ttttctttcg atttttc                                                    17

<210> 4057
<211> 17
<212> DNA
<213> Homo sapiens

<400> 4057
tttctttcga tttttct                                                    17

<210> 4058
<211> 17
<212> DNA
<213> Homo sapiens

<400> 4058
ttctttcgat ttttctg                                                    17

<210> 4059
<211> 17
<212> DNA
<213> Homo sapiens

<400> 4059
tctttcgatt tttctgc                                                    17

<210> 4060
<211> 17
<212> DNA
<213> Homo sapiens

<400> 4060
ctttcgattt ttctgca                                                    17

<210> 4061
<211> 17
<212> DNA
<213> Homo sapiens

<400> 4061
tttcgatttt tctgcac                                                    17

<210> 4062
<211> 17
<212> DNA
<213> Homo sapiens

<400> 4062
ttcgattttt ctgcaca                                                    17

<210> 4063
<211> 17
<212> DNA
<213> Homo sapiens

<400> 4063
tcgatttttc tgcacac                                                    17

<210> 4064
<211> 17
<212> DNA
<213> Homo sapiens

<400> 4064
cgatttttct gcacaca                                                    17

<210> 4065
<211> 17
<212> DNA
<213> Homo sapiens

<400> 4065
gatttttctg cacacat                                                    17

<210> 4066
<211> 17
<212> DNA
<213> Homo sapiens

<400> 4066
atttttctgc acacatt                                                    17

<210> 4067
<211> 17
<212> DNA
<213> Homo sapiens

<400> 4067
tttttctgca cacattt                                                    17

<210> 4068
<211> 17
<212> DNA
<213> Homo sapiens

<400> 4068
ttttctgcac acatttc                                                    17

<210> 4069
<211> 17
<212> DNA
<213> Homo sapiens

<400> 4069
tttctgcaca catttcc                                                    17

<210> 4070
<211> 17
<212> DNA
<213> Homo sapiens

<400> 4070
ttctgcacac atttcct                                                    17

<210> 4071
<211> 17
<212> DNA
<213> Homo sapiens

<400> 4071
tctgcacaca tttccta                                                    17

<210> 4072
<211> 17
<212> DNA
<213> Homo sapiens

<400> 4072
ctgcacacat ttcctat                                                    17

<210> 4073
<211> 17
<212> DNA
<213> Homo sapiens

<400> 4073
tgcacacatt tcctatg                                                    17

<210> 4074

<211> 17
<212> DNA
<213> Homo sapiens

<400> 4074
gcacacattt cctatgc                                                    17

<210> 4075
<211> 17
<212> DNA
<213> Homo sapiens

<400> 4075
cacacatttc ctatgca                                                    17

<210> 4076
<211> 17
<212> DNA
<213> Homo sapiens

<400> 4076
acacatttcc tatgcat                                                    17

<210> 4077
<211> 17
<212> DNA
<213> Homo sapiens

<400> 4077
cacatttcct atgcatt                                                    17

<210> 4078
<211> 17
<212> DNA
<213> Homo sapiens

<400> 4078
acatttccta tgcattt                                                    17

<210> 4079
<211> 17
<212> DNA
<213> Homo sapiens

<400> 4079
catttcctat gcatttg                                                    17

<210> 4080
<211> 17
<212> DNA
<213> Homo sapiens

<400> 4080
atttcctatg catttgt                                                    17

<210> 4081
<211> 17
<212> DNA
<213> Homo sapiens

<400> 4081

tttcctatgc atttgtt                                                    17

<210> 4082
<211> 17
<212> DNA
<213> Homo sapiens

<400> 4082
ttcctatgca tttgttt                                                    17

<210> 4083
<211> 17
<212> DNA
<213> Homo sapiens

<400> 4083
tcctatgcat ttgtttc                                                    17

<210> 4084
<211> 17
<212> DNA
<213> Homo sapiens

<400> 4084
cctatgcatt tgtttct                                                    17

<210> 4085
<211> 17
<212> DNA
<213> Homo sapiens

<400> 4085
ctatgcattt gtttcta                                                    17

<210> 4086
<211> 17
<212> DNA
<213> Homo sapiens

<400> 4086
tatgcatttg tttctag                                                    17

<210> 4087
<211> 17
<212> DNA
<213> Homo sapiens

<400> 4087
atgcatttgt ttctagt                                                    17

<210> 4088
<211> 17
<212> DNA
<213> Homo sapiens

<400> 4088
tgcatttgtt tctagtt                                                    17

<210> 4089
<211> 17
<212> DNA

<213> Homo sapiens

<400> 4089
gcatttgttt ctagttc                                                    17

<210> 4090
<211> 17
<212> DNA
<213> Homo sapiens

<400> 4090
catttgtttc tagttct                                                    17

<210> 4091
<211> 17
<212> DNA
<213> Homo sapiens

<400> 4091
atttgtttct agttctc                                                    17

<210> 4092
<211> 17
<212> DNA
<213> Homo sapiens

<400> 4092
tttgtttcta gttctca                                                    17

<210> 4093
<211> 17
<212> DNA
<213> Homo sapiens

<400> 4093
ttgtttctag ttctcaa                                                    17

<210> 4094
<211> 17
<212> DNA
<213> Homo sapiens

<400> 4094
tgtttctagt tctcaac                                                    17

<210> 4095
<211> 17
<212> DNA
<213> Homo sapiens

<400> 4095
gtttctagtt ctcaacc                                                    17

<210> 4096
<211> 17
<212> DNA
<213> Homo sapiens

<400> 4096
tttctagttc tcaaccc                                                    17

<210> 4097
<211> 17
<212> DNA
<213> Homo sapiens

<400> 4097
ttctagttct caaccct                                                    17

<210> 4098
<211> 17
<212> DNA
<213> Homo sapiens

<400> 4098
tctagttctc aaccctc                                                    17

<210> 4099
<211> 17
<212> DNA
<213> Homo sapiens

<400> 4099
ctagttctca accctca                                                    17

<210> 4100
<211> 17
<212> DNA
<213> Homo sapiens

<400> 4100
tagttctcaa ccctcag                                                    17

<210> 4101
<211> 17
<212> DNA
<213> Homo sapiens

<400> 4101
agttctcaac cctcagt                                                    17

<210> 4102
<211> 17
<212> DNA
<213> Homo sapiens

<400> 4102
gttctcaacc ctcagta                                                    17

<210> 4103
<211> 17
<212> DNA
<213> Homo sapiens

<400> 4103
ttctcaaccc tcagtaa                                                    17

<210> 4104
<211> 17
<212> DNA
<213> Homo sapiens

<400> 4104
tctcaaccct cagtaaa                                                    17

<210> 4105
<211> 17
<212> DNA
<213> Homo sapiens

<400> 4105
ctcaaccctc agtaaac                                                    17

<210> 4106
<211> 17
<212> DNA
<213> Homo sapiens

<400> 4106
tcaaccctca gtaaacc                                                    17

<210> 4107
<211> 17
<212> DNA
<213> Homo sapiens

<400> 4107
caaccctcag taaacca                                                    17

<210> 4108
<211> 17
<212> DNA
<213> Homo sapiens

<400> 4108
aaccctcagt aaaccaa                                                    17

<210> 4109
<211> 17
<212> DNA
<213> Homo sapiens

<400> 4109
accctcagta aaccaaa                                                    17

<210> 4110
<211> 17
<212> DNA
<213> Homo sapiens

<400> 4110
ccctcagtaa accaaaa                                                    17

<210> 4111
<211> 17
<212> DNA
<213> Homo sapiens

<400> 4111
cctcagtaaa ccaaaaa                                                    17

<210> 4112
<211> 17

<212> DNA
<213> Homo sapiens

<400> 4112
ctcagtaaac caaaaat                                                    17

<210> 4113
<211> 17
<212> DNA
<213> Homo sapiens

<400> 4113
tcagtaaacc aaaaatc                                                    17

<210> 4114
<211> 17
<212> DNA
<213> Homo sapiens

<400> 4114
cagtaaacca aaaatca                                                    17

<210> 4115
<211> 17
<212> DNA
<213> Homo sapiens

<400> 4115
agtaaaccaa aaatcag                                                    17

<210> 4116
<211> 17
<212> DNA
<213> Homo sapiens

<400> 4116
gtaaaccaaa aatcagt                                                    17

<210> 4117
<211> 17
<212> DNA
<213> Homo sapiens

<400> 4117
taaaccaaaa atcagtt                                                    17

<210> 4118
<211> 17
<212> DNA
<213> Homo sapiens

<400> 4118
aaaccaaaaa tcagttg                                                    17

<210> 4119
<211> 17
<212> DNA
<213> Homo sapiens

<400> 4119
aaccaaaaat cagttga                                                    17

<210> 4120
<211> 17
<212> DNA
<213> Homo sapiens

<400> 4120
accaaaaatc agttgag                                                    17

<210> 4121
<211> 17
<212> DNA
<213> Homo sapiens

<400> 4121
ccaaaaatca gttgagg                                                    17

<210> 4122
<211> 17
<212> DNA
<213> Homo sapiens

<400> 4122
caaaaatcag ttgaggc                                                    17

<210> 4123
<211> 17
<212> DNA
<213> Homo sapiens

<400> 4123
aaaaatcagt tgaggca                                                    17

<210> 4124
<211> 17
<212> DNA
<213> Homo sapiens

<400> 4124
aaaatcagtt gaggcat                                                    17

<210> 4125
<211> 17
<212> DNA
<213> Homo sapiens

<400> 4125
aaatcagttg aggcatt                                                    17

<210> 4126
<211> 17
<212> DNA
<213> Homo sapiens

<400> 4126
aatcagttga ggcattg                                                    17

<210> 4127
<211> 17
<212> DNA
<213> Homo sapiens

<400> 4127
atcagttgag gcattgt                                                    17

<210> 4128
<211> 17
<212> DNA
<213> Homo sapiens

<400> 4128
tcagttgagg cattgta                                                    17

<210> 4129
<211> 17
<212> DNA
<213> Homo sapiens

<400> 4129
cagttgaggc attgtat                                                    17

<210> 4130
<211> 17
<212> DNA
<213> Homo sapiens

<400> 4130
agttgaggca ttgtatc                                                    17

<210> 4131
<211> 17
<212> DNA
<213> Homo sapiens

<400> 4131
gttgaggcat tgtatct                                                    17

<210> 4132
<211> 17
<212> DNA
<213> Homo sapiens

<400> 4132
ttgaggcatt gtatctt                                                    17

<210> 4133
<211> 17
<212> DNA
<213> Homo sapiens

<400> 4133
tgaggcattg tatcttc                                                    17

<210> 4134
<211> 17
<212> DNA
<213> Homo sapiens

<400> 4134
gaggcattgt atcttct                                                    17

<210> 4135

<211> 17
<212> DNA
<213> Homo sapiens

<400> 4135
aggcattgta tcttcta                                                    17

<210> 4136
<211> 17
<212> DNA
<213> Homo sapiens

<400> 4136
ggcattgtat cttctag                                                    17

<210> 4137
<211> 17
<212> DNA
<213> Homo sapiens

<400> 4137
gcattgtatc ttctagg                                                    17

<210> 4138
<211> 17
<212> DNA
<213> Homo sapiens

<400> 4138
cattgtatct tctaggc                                                    17

<210> 4139
<211> 17
<212> DNA
<213> Homo sapiens

<400> 4139
attgtatctt ctaggct                                                    17

<210> 4140
<211> 17
<212> DNA
<213> Homo sapiens

<400> 4140
ttgtatcttc taggcta                                                    17

<210> 4141
<211> 17
<212> DNA
<213> Homo sapiens

<400> 4141
tgtatcttct aggctac                                                    17

<210> 4142
<211> 17
<212> DNA
<213> Homo sapiens

<400> 4142

gtatcttcta ggctacc 17

<210> 4143
<211> 17
<212> DNA
<213> Homo sapiens

<400> 4143
tatcttctag gctaccc 17

<210> 4144
<211> 17
<212> DNA
<213> Homo sapiens

<400> 4144
atcttctagg ctaccca 17

<210> 4145
<211> 17
<212> DNA
<213> Homo sapiens

<400> 4145
tcttctaggc tacccag 17

<210> 4146
<211> 17
<212> DNA
<213> Homo sapiens

<400> 4146
cttctaggct acccagt 17

<210> 4147
<211> 17
<212> DNA
<213> Homo sapiens

<400> 4147
ttctaggcta cccagtg 17

<210> 4148
<211> 17
<212> DNA
<213> Homo sapiens

<400> 4148
tctaggctac ccagtgt 17

<210> 4149
<211> 17
<212> DNA
<213> Homo sapiens

<400> 4149
ctaggctacc cagtgtt 17

<210> 4150
<211> 17
<212> DNA

<213> Homo sapiens

<400> 4150
taggctaccc agtgtta                                                    17

<210> 4151
<211> 17
<212> DNA
<213> Homo sapiens

<400> 4151
aggctaccca gtgttac                                                    17

<210> 4152
<211> 17
<212> DNA
<213> Homo sapiens

<400> 4152
ggctacccag tgttaca                                                    17

<210> 4153
<211> 17
<212> DNA
<213> Homo sapiens

<400> 4153
gctacccagt gttacaa                                                    17

<210> 4154
<211> 17
<212> DNA
<213> Homo sapiens

<400> 4154
ctacccagtg ttacaaa                                                    17

<210> 4155
<211> 17
<212> DNA
<213> Homo sapiens

<400> 4155
tacccagtgt tacaaag                                                    17

<210> 4156
<211> 17
<212> DNA
<213> Homo sapiens

<400> 4156
acccagtgtt acaaagt                                                    17

<210> 4157
<211> 17
<212> DNA
<213> Homo sapiens

<400> 4157
cccagtgtta caaagtg                                                    17

<210> 4158
<211> 17
<212> DNA
<213> Homo sapiens

<400> 4158
ccagtgttac aaagtgc                                                    17

<210> 4159
<211> 17
<212> DNA
<213> Homo sapiens

<400> 4159
cagtgttaca aagtgca                                                    17

<210> 4160
<211> 17
<212> DNA
<213> Homo sapiens

<400> 4160
agtgttacaa agtgcaa                                                    17

<210> 4161
<211> 17
<212> DNA
<213> Homo sapiens

<400> 4161
gtgttacaaa gtgcaat                                                    17

<210> 4162
<211> 17
<212> DNA
<213> Homo sapiens

<400> 4162
tgttacaaag tgcaatt                                                    17

<210> 4163
<211> 17
<212> DNA
<213> Homo sapiens

<400> 4163
gttacaaagt gcaattt                                                    17

<210> 4164
<211> 17
<212> DNA
<213> Homo sapiens

<400> 4164
ttacaaagtg caatttc                                                    17

<210> 4165
<211> 17
<212> DNA
<213> Homo sapiens

<400> 4165
tacaaagtgc aatttca                                                    17

<210> 4166
<211> 17
<212> DNA
<213> Homo sapiens

<400> 4166
acaaagtgca atttcaa                                                    17

<210> 4167
<211> 17
<212> DNA
<213> Homo sapiens

<400> 4167
caaagtgcaa tttcaac                                                    17

<210> 4168
<211> 17
<212> DNA
<213> Homo sapiens

<400> 4168
aaagtgcaat ttcaaca                                                    17

<210> 4169
<211> 17
<212> DNA
<213> Homo sapiens

<400> 4169
aagtgcaatt tcaacaa                                                    17

<210> 4170
<211> 17
<212> DNA
<213> Homo sapiens

<400> 4170
agtgcaattt caacaat                                                    17

<210> 4171
<211> 17
<212> DNA
<213> Homo sapiens

<400> 4171
gtgcaatttc aacaata                                                    17

<210> 4172
<211> 17
<212> DNA
<213> Homo sapiens

<400> 4172
tgcaatttca acaataa                                                    17

<210> 4173
<211> 17

<212> DNA
<213> Homo sapiens

<400> 4173
gcaatttcaa caataat                                                    17

<210> 4174
<211> 17
<212> DNA
<213> Homo sapiens

<400> 4174
caatttcaac aataata                                                    17

<210> 4175
<211> 17
<212> DNA
<213> Homo sapiens

<400> 4175
aatttcaaca ataatag                                                    17

<210> 4176
<211> 17
<212> DNA
<213> Homo sapiens

<400> 4176
atttcaacaa taatagg                                                    17

<210> 4177
<211> 17
<212> DNA
<213> Homo sapiens

<400> 4177
tttcaacaat aataggg                                                    17

<210> 4178
<211> 17
<212> DNA
<213> Homo sapiens

<400> 4178
ttcaacaata atagggg                                                    17

<210> 4179
<211> 17
<212> DNA
<213> Homo sapiens

<400> 4179
tcaacaataa taggggt                                                    17

<210> 4180
<211> 17
<212> DNA
<213> Homo sapiens

<400> 4180
caacaataat aggggtg                                                    17

<210> 4181
<211> 17
<212> DNA
<213> Homo sapiens

<400> 4181
aacaataata ggggtgt                                                      17

<210> 4182
<211> 17
<212> DNA
<213> Homo sapiens

<400> 4182
acaataatag gggtgtg                                                      17

<210> 4183
<211> 17
<212> DNA
<213> Homo sapiens

<400> 4183
caataatagg ggtgtgt                                                      17

<210> 4184
<211> 17
<212> DNA
<213> Homo sapiens

<400> 4184
aataataggg gtgtgtg                                                      17

<210> 4185
<211> 17
<212> DNA
<213> Homo sapiens

<400> 4185
ataatagggg tgtgtgt                                                      17

<210> 4186
<211> 17
<212> DNA
<213> Homo sapiens

<400> 4186
taataggggt gtgtgtt                                                      17

<210> 4187
<211> 17
<212> DNA
<213> Homo sapiens

<400> 4187
aataggggtg tgtgttt                                                      17

<210> 4188
<211> 17
<212> DNA
<213> Homo sapiens

<400> 4188
atagggtgt gtgtttt                                                    17

<210> 4189
<211> 17
<212> DNA
<213> Homo sapiens

<400> 4189
taggggtgtg tgtttta                                                   17

<210> 4190
<211> 17
<212> DNA
<213> Homo sapiens

<400> 4190
aggggtgtgt gttttag                                                   17

<210> 4191
<211> 17
<212> DNA
<213> Homo sapiens

<400> 4191
ggggtgtgtg ttttagc                                                   17

<210> 4192
<211> 17
<212> DNA
<213> Homo sapiens

<400> 4192
gggtgtgtgt tttagct                                                   17

<210> 4193
<211> 17
<212> DNA
<213> Homo sapiens

<400> 4193
ggtgtgtgtt ttagctg                                                   17

<210> 4194
<211> 17
<212> DNA
<213> Homo sapiens

<400> 4194
gtgtgtgttt tagctgc                                                   17

<210> 4195
<211> 17
<212> DNA
<213> Homo sapiens

<400> 4195
tgtgtgtttt agctgca                                                   17

<210> 4196

<211> 17
<212> DNA
<213> Homo sapiens

<400> 4196
gtgtgtttta gctgcag                                                    17

<210> 4197
<211> 17
<212> DNA
<213> Homo sapiens

<400> 4197
tgtgttttag ctgcagc                                                    17

<210> 4198
<211> 17
<212> DNA
<213> Homo sapiens

<400> 4198
gtgttttagc tgcagcg                                                    17

<210> 4199
<211> 17
<212> DNA
<213> Homo sapiens

<400> 4199
tgttttagct gcagcga                                                    17

<210> 4200
<211> 17
<212> DNA
<213> Homo sapiens

<400> 4200
gttttagctg cagcgaa                                                    17

<210> 4201
<211> 17
<212> DNA
<213> Homo sapiens

<400> 4201
ttttagctgc agcgaaa                                                    17

<210> 4202
<211> 17
<212> DNA
<213> Homo sapiens

<400> 4202
tttagctgca gcgaaag                                                    17

<210> 4203
<211> 17
<212> DNA
<213> Homo sapiens

<400> 4203

ttagctgcag cgaaagc                                                    17

<210> 4204
<211> 17
<212> DNA
<213> Homo sapiens

<400> 4204
tagctgcagc gaaagca                                                    17

<210> 4205
<211> 17
<212> DNA
<213> Homo sapiens

<400> 4205
agctgcagcg aaagcat                                                    17

<210> 4206
<211> 17
<212> DNA
<213> Homo sapiens

<400> 4206
gctgcagcga aagcata                                                    17

<210> 4207
<211> 17
<212> DNA
<213> Homo sapiens

<400> 4207
ctgcagcgaa agcatac                                                    17

<210> 4208
<211> 17
<212> DNA
<213> Homo sapiens

<400> 4208
tgcagcgaaa gcataca                                                    17

<210> 4209
<211> 17
<212> DNA
<213> Homo sapiens

<400> 4209
gcagcgaaag catacat                                                    17

<210> 4210
<211> 17
<212> DNA
<213> Homo sapiens

<400> 4210
cagcgaaagc atacatc                                                    17

<210> 4211
<211> 17
<212> DNA

<213> Homo sapiens

<400> 4211
agcgaaagca tacatct                                                    17

<210> 4212
<211> 17
<212> DNA
<213> Homo sapiens

<400> 4212
gcgaaagcat acatctt                                                    17

<210> 4213
<211> 17
<212> DNA
<213> Homo sapiens

<400> 4213
cgaaagcata catcttc                                                    17

<210> 4214
<211> 17
<212> DNA
<213> Homo sapiens

<400> 4214
gaaagcatac atcttca                                                    17

<210> 4215
<211> 17
<212> DNA
<213> Homo sapiens

<400> 4215
aaagcataca tcttcag                                                    17

<210> 4216
<211> 17
<212> DNA
<213> Homo sapiens

<400> 4216
aagcatacat cttcaga                                                    17

<210> 4217
<211> 17
<212> DNA
<213> Homo sapiens

<400> 4217
agcatacatc ttcagaa                                                    17

<210> 4218
<211> 17
<212> DNA
<213> Homo sapiens

<400> 4218
gcatacatct tcagaac                                                    17

<210> 4219
<211> 17
<212> DNA
<213> Homo sapiens

<400> 4219
catacatctt cagaaca                                                    17

<210> 4220
<211> 17
<212> DNA
<213> Homo sapiens

<400> 4220
atacatcttc agaacat                                                    17

<210> 4221
<211> 17
<212> DNA
<213> Homo sapiens

<400> 4221
tacatcttca gaacatt                                                    17

<210> 4222
<211> 17
<212> DNA
<213> Homo sapiens

<400> 4222
acatcttcag aacattt                                                    17

<210> 4223
<211> 17
<212> DNA
<213> Homo sapiens

<400> 4223
catcttcaga acatttt                                                    17

<210> 4224
<211> 17
<212> DNA
<213> Homo sapiens

<400> 4224
atcttcagaa cattttt                                                    17

<210> 4225
<211> 17
<212> DNA
<213> Homo sapiens

<400> 4225
   tcttcagaac atttttt                                                    17

<210> 4226
<211> 17
<212> DNA
<213> Homo sapiens

<400> 4226
cttcagaaca tttttta                                                    17

<210> 4227
<211> 17
<212> DNA
<213> Homo sapiens

<400> 4227
ttcagaacat ttttaa                                                     17

<210> 4228
<211> 17
<212> DNA
<213> Homo sapiens

<400> 4228
tcagaacatt ttttaag                                                    17

<210> 4229
<211> 17
<212> DNA
<213> Homo sapiens

<400> 4229
cagaacattt tttaaga                                                    17

<210> 4230
<211> 17
<212> DNA
<213> Homo sapiens

<400> 4230
agaacatttt ttaagat                                                    17

<210> 4231
<211> 17
<212> DNA
<213> Homo sapiens

<400> 4231
gaacattttt taagatt                                                    17

<210> 4232
<211> 17
<212> DNA
<213> Homo sapiens

<400> 4232
aacatttttt aagatta                                                    17

<210> 4233
<211> 17
<212> DNA
<213> Homo sapiens

<400> 4233
acatttttta agattat                                                    17

<210> 4234
<211> 17

<212> DNA
<213> Homo sapiens

<400> 4234
catttttaa gattatg                                                    17

<210> 4235
<211> 17
<212> DNA
<213> Homo sapiens

<400> 4235
   attttttaag attatgt                                                    17

<210> 4236
<211> 17
<212> DNA
<213> Homo sapiens

<400> 4236
tttttaaga ttatgtt                                                    17

<210> 4237
<211> 17
<212> DNA
<213> Homo sapiens

<400> 4237
   tttttaagat tatgttt                                                    17

<210> 4238
<211> 17
<212> DNA
<213> Homo sapiens

<400> 4238
   ttttaagatt atgtttc                                                    17

<210> 4239
<211> 17
<212> DNA
<213> Homo sapiens

<400> 4239
tttaagatta tgtttct                                                   17

<210> 4240
<211> 17
<212> DNA
<213> Homo sapiens

<400> 4240
ttaagattat gtttctt                                                   17

<210> 4241
<211> 17
<212> DNA
<213> Homo sapiens

<400> 4241
taagattatg tttcttg                                                   17

<210> 4242
<211> 17
<212> DNA
<213> Homo sapiens

<400> 4242
aagattatgt ttcttgt                                                    17

<210> 4243
<211> 17
<212> DNA
<213> Homo sapiens

<400> 4243
agattatgtt tcttgtt                                                    17

<210> 4244
<211> 17
<212> DNA
<213> Homo sapiens

<400> 4244
gattatgttt cttgtta                                                    17

<210> 4245
<211> 17
<212> DNA
<213> Homo sapiens

<400> 4245
attatgtttc ttgttat                                                    17

<210> 4246
<211> 17
<212> DNA
<213> Homo sapiens

<400> 4246
ttatgtttct tgttatg                                                    17

<210> 4247
<211> 17
<212> DNA
<213> Homo sapiens

<400> 4247
tatgtttctt gttatga                                                    17

<210> 4248
<211> 17
<212> DNA
<213> Homo sapiens

<400> 4248
atgtttcttg ttatgat                                                    17

<210> 4249
<211> 17
<212> DNA
<213> Homo sapiens

<400> 4249
tgtttcttgt tatgata                                                    17

<210> 4250
<211> 17
<212> DNA
<213> Homo sapiens

<400> 4250
gtttcttgtt atgatat                                                    17

<210> 4251
<211> 17
<212> DNA
<213> Homo sapiens

<400> 4251
tttcttgtta tgatatt                                                    17

<210> 4252
<211> 17
<212> DNA
<213> Homo sapiens

<400> 4252
ttcttgttat gatattt                                                    17

<210> 4253
<211> 17
<212> DNA
<213> Homo sapiens

<400> 4253
tcttgttatg atatttg                                                    17

<210> 4254
<211> 17
<212> DNA
<213> Homo sapiens

<400> 4254
cttgttatga tatttgg                                                    17

<210> 4255
<211> 17
<212> DNA
<213> Homo sapiens

<400> 4255
ttgttatgat atttggg                                                    17

<210> 4256
<211> 17
<212> DNA
<213> Homo sapiens

<400> 4256
tgttatgata tttgggg                                                    17

<210> 4257

<211> 17
<212> DNA
<213> Homo sapiens

<400> 4257
gttatgatat ttggggc                                                    17

<210> 4258
<211> 17
<212> DNA
<213> Homo sapiens

<400> 4258
ttatgatatt tggggca                                                    17

<210> 4259
<211> 17
<212> DNA
<213> Homo sapiens

<400> 4259
tatgatattt ggggcag                                                    17

<210> 4260
<211> 17
<212> DNA
<213> Homo sapiens

<400> 4260
atgatatttg gggcagc                                                    17

<210> 4261
<211> 17
<212> DNA
<213> Homo sapiens

<400> 4261
tgatatttgg ggcagct                                                    17

<210> 4262
<211> 17
<212> DNA
<213> Homo sapiens

<400> 4262
gatatttggg gcagctc                                                    17

<210> 4263
<211> 17
<212> DNA
<213> Homo sapiens

<400> 4263
atatttgggg cagctca                                                    17

<210> 4264
<211> 17
<212> DNA
<213> Homo sapiens

<400> 4264

tatttggggc agctcat                                                    17

<210> 4265
<211> 17
<212> DNA
<213> Homo sapiens

<400> 4265
atttggggca gctcatg                                                    17

<210> 4266
<211> 17
<212> DNA
<213> Homo sapiens

<400> 4266
tttggggcag ctcatgg                                                    17

<210> 4267
<211> 17
<212> DNA
<213> Homo sapiens

<400> 4267
ttggggcagc tcatggc                                                    17

<210> 4268
<211> 17
<212> DNA
<213> Homo sapiens

<400> 4268
tggggcagct catggcc                                                    17

<210> 4269
<211> 17
<212> DNA
<213> Homo sapiens

<400> 4269
ggggcagctc atggcct                                                    17

<210> 4270
<211> 17
<212> DNA
<213> Homo sapiens

<400> 4270
gggcagctca tggccta                                                    17

<210> 4271
<211> 17
<212> DNA
<213> Homo sapiens

<400> 4271
ggcagctcat ggcctaa                                                    17

<210> 4272
<211> 17
<212> DNA

<213> Homo sapiens

<400> 4272
gcagctcatg gcctaat                                                    17

<210> 4273
<211> 17
<212> DNA
<213> Homo sapiens

<400> 4273
cagctcatgg cctaatt                                                    17

<210> 4274
<211> 17
<212> DNA
<213> Homo sapiens

<400> 4274
agctcatggc ctaattt                                                    17

<210> 4275
<211> 17
<212> DNA
<213> Homo sapiens

<400> 4275
gctcatggcc taatttt                                                    17

<210> 4276
<211> 17
<212> DNA
<213> Homo sapiens

<400> 4276
ctcatggcct aattttt                                                    17

<210> 4277
<211> 17
<212> DNA
<213> Homo sapiens

<400> 4277
tcatggccta attttta                                                    17

<210> 4278
<211> 17
<212> DNA
<213> Homo sapiens

<400> 4278
catggcctaa tttttat                                                    17

<210> 4279
<211> 17
<212> DNA
<213> Homo sapiens

<400> 4279
atggcctaat ttttatt                                                    17

<210> 4280
<211> 17
<212> DNA
<213> Homo sapiens

<400> 4280
tggcctaatt tttattc                                                    17

<210> 4281
<211> 17
<212> DNA
<213> Homo sapiens

<400> 4281
ggcctaattt ttattcc                                                    17

<210> 4282
<211> 17
<212> DNA
<213> Homo sapiens

<400> 4282
gcctaatttt tattcca                                                    17

<210> 4283
<211> 17
<212> DNA
<213> Homo sapiens

<400> 4283
cctaattttt attccag                                                    17

<210> 4284
<211> 17
<212> DNA
<213> Homo sapiens

<400> 4284
ctaattttta ttccagt                                                    17

<210> 4285
<211> 17
<212> DNA
<213> Homo sapiens

<400> 4285
taatttttat tccagta                                                    17

<210> 4286
<211> 17
<212> DNA
<213> Homo sapiens

<400> 4286
aatttttatt ccagtat                                                    17

<210> 4287
<211> 17
<212> DNA
<213> Homo sapiens

<400> 4287
atttttattc cagtatt                                                    17

<210> 4288
<211> 17
<212> DNA
<213> Homo sapiens

<400> 4288
tttttattcc agtattc                                                    17

<210> 4289
<211> 17
<212> DNA
<213> Homo sapiens

<400> 4289
ttttattcca gtattct                                                    17

<210> 4290
<211> 17
<212> DNA
<213> Homo sapiens

<400> 4290
tttattccag tattctt                                                    17

<210> 4291
<211> 17
<212> DNA
<213> Homo sapiens

<400> 4291
ttattccagt attctta                                                    17

<210> 4292
<211> 17
<212> DNA
<213> Homo sapiens

<400> 4292
tattccagta ttcttaa                                                    17

<210> 4293
<211> 17
<212> DNA
<213> Homo sapiens

<400> 4293
attccagtat tcttaac                                                    17

<210> 4294
<211> 17
<212> DNA
<213> Homo sapiens

<400> 4294
ttccagtatt cttaacc                                                    17

<210> 4295
<211> 17

<212> DNA
<213> Homo sapiens

<400> 4295
tccagtattc ttaacct                                                    17

<210> 4296
<211> 17
<212> DNA
<213> Homo sapiens

<400> 4296
ccagtattct taacctt                                                    17

<210> 4297
<211> 17
<212> DNA
<213> Homo sapiens

<400> 4297
cagtattctt aaccttt                                                    17

<210> 4298
<211> 17
<212> DNA
<213> Homo sapiens

<400> 4298
agtattctta acctttt                                                    17

<210> 4299
<211> 17
<212> DNA
<213> Homo sapiens

<400> 4299
gtattcttaa ccttttt                                                    17

<210> 4300
<211> 17
<212> DNA
<213> Homo sapiens

<400> 4300
tattcttaac ctttttt                                                    17

<210> 4301
<211> 17
<212> DNA
<213> Homo sapiens

<400> 4301
attcttaacc ttttttg                                                    17

<210> 4302
<211> 17
<212> DNA
<213> Homo sapiens

<400> 4302
ttcttaacct tttttgg                                                    17

<210> 4303
<211> 17
<212> DNA
<213> Homo sapiens

<400> 4303
tcttaacctt ttttgga                                                    17

<210> 4304
<211> 17
<212> DNA
<213> Homo sapiens

<400> 4304
cttaaccttt tttggaa                                                    17

<210> 4305
<211> 17
<212> DNA
<213> Homo sapiens

<400> 4305
ttaacctttt ttggaag                                                    17

<210> 4306
<211> 17
<212> DNA
<213> Homo sapiens

<400> 4306
taaccttttt tggaagg                                                    17

<210> 4307
<211> 17
<212> DNA
<213> Homo sapiens

<400> 4307
aacctttttt ggaaggt                                                    17

<210> 4308
<211> 17
<212> DNA
<213> Homo sapiens

<400> 4308
accttttttg gaaggtt                                                    17

<210> 4309
<211> 17
<212> DNA
<213> Homo sapiens

<400> 4309
ccttttttgg aaggttt                                                    17

<210> 4310
<211> 17
<212> DNA
<213> Homo sapiens

<400> 4310
   cttttttgga aggttta                                                    17

<210> 4311
<211> 17
<212> DNA
<213> Homo sapiens

<400> 4311
ttttttggaa ggtttat                                                     17

<210> 4312
<211> 17
<212> DNA
<213> Homo sapiens

<400> 4312
tttttggaag gtttatt                                                     17

<210> 4313
<211> 17
<212> DNA
<213> Homo sapiens

<400> 4313
ttttggaagg tttattt                                                     17

<210> 4314
<211> 17
<212> DNA
<213> Homo sapiens

<400> 4314
tttggaaggt ttatttg                                                     17

<210> 4315
<211> 17
<212> DNA
<213> Homo sapiens

<400> 4315
ttggaaggtt tatttga                                                     17

<210> 4316
<211> 17
<212> DNA
<213> Homo sapiens

<400> 4316
tggaaggttt atttgaa                                                     17

<210> 4317
<211> 17
<212> DNA
<213> Homo sapiens

<400> 4317
ggaaggttta tttgaat                                                     17

<210> 4318

<211> 17
<212> DNA
<213> Homo sapiens

<400> 4318
gaaggtttat ttgaata                                                    17

<210> 4319
<211> 17
<212> DNA
<213> Homo sapiens

<400> 4319
aaggtttatt tgaatat                                                    17

<210> 4320
<211> 17
<212> DNA
<213> Homo sapiens

<400> 4320
aggtttattt gaatatc                                                    17

<210> 4321
<211> 17
<212> DNA
<213> Homo sapiens

<400> 4321
ggtttatttg aatatcc                                                    17

<210> 4322
<211> 17
<212> DNA
<213> Homo sapiens

<400> 4322
gtttatttga atatcca                                                    17

<210> 4323
<211> 17
<212> DNA
<213> Homo sapiens

<400> 4323
tttatttgaa tatccac                                                    17

<210> 4324
<211> 17
<212> DNA
<213> Homo sapiens

<400> 4324
ttatttgaat atccact                                                    17

<210> 4325
<211> 17
<212> DNA
<213> Homo sapiens

<400> 4325

tatttgaata tccacta                                                    17

<210> 4326
<211> 17
<212> DNA
<213> Homo sapiens

<400> 4326
atttgaatat ccactaa                                                    17

<210> 4327
<211> 17
<212> DNA
<213> Homo sapiens

<400> 4327
tttgaatatc cactaac                                                    17

<210> 4328
<211> 17
<212> DNA
<213> Homo sapiens

<400> 4328
ttgaatatcc actaaca                                                    17

<210> 4329
<211> 17
<212> DNA
<213> Homo sapiens

<400> 4329
tgaatatcca ctaacaa                                                    17

<210> 4330
<211> 17
<212> DNA
<213> Homo sapiens

<400> 4330
gaatatccac taacaag                                                    17

<210> 4331
<211> 17
<212> DNA
<213> Homo sapiens

<400> 4331
aatatccact aacaagt                                                    17

<210> 4332
<211> 17
<212> DNA
<213> Homo sapiens

<400> 4332
atatccacta acaagtc                                                    17

<210> 4333
<211> 17
<212> DNA

<213> Homo sapiens

<400> 4333
tatccactaa caagtca                                                    17

<210> 4334
<211> 17
<212> DNA
<213> Homo sapiens

<400> 4334
atccactaac aagtcaa                                                    17

<210> 4335
<211> 17
<212> DNA
<213> Homo sapiens

<400> 4335
tccactaaca agtcaaa                                                    17

<210> 4336
<211> 17
<212> DNA
<213> Homo sapiens

<400> 4336
ccactaacaa gtcaaag                                                    17

<210> 4337
<211> 17
<212> DNA
<213> Homo sapiens

<400> 4337
cactaacaag tcaaaga                                                    17

<210> 4338
<211> 17
<212> DNA
<213> Homo sapiens

<400> 4338
actaacaagt caaagac                                                    17

<210> 4339
<211> 17
<212> DNA
<213> Homo sapiens

<400> 4339
ctaacaagtc aaagacc                                                    17

<210> 4340
<211> 17
<212> DNA
<213> Homo sapiens

<400> 4340
taacaagtca aagacca                                                    17

<210> 4341
<211> 17
<212> DNA
<213> Homo sapiens

<400> 4341
aacaagtcaa agaccaa                                                    17

<210> 4342
<211> 17
<212> DNA
<213> Homo sapiens

<400> 4342
acaagtcaaa gaccaat                                                    17

<210> 4343
<211> 17
<212> DNA
<213> Homo sapiens

<400> 4343
caagtcaaag accaatt                                                    17

<210> 4344
<211> 17
<212> DNA
<213> Homo sapiens

<400> 4344
aagtcaaaga ccaattc                                                    17

<210> 4345
<211> 17
<212> DNA
<213> Homo sapiens

<400> 4345
agtcaaagac caattct                                                    17

<210> 4346
<211> 17
<212> DNA
<213> Homo sapiens

<400> 4346
gtcaaagacc aattcta                                                    17

<210> 4347
<211> 17
<212> DNA
<213> Homo sapiens

<400> 4347
tcaaagacca attctag                                                    17

<210> 4348
<211> 17
<212> DNA
<213> Homo sapiens

<400> 4348
caaagaccaa ttctaga                                                    17

<210> 4349
<211> 17
<212> DNA
<213> Homo sapiens

<400> 4349
aaagaccaat tctagaa                                                    17

<210> 4350
<211> 17
<212> DNA
<213> Homo sapiens

<400> 4350
aagaccaatt ctagaat                                                    17

<210> 4351
<211> 17
<212> DNA
<213> Homo sapiens

<400> 4351
agaccaattc tagaatt                                                    17

<210> 4352
<211> 17
<212> DNA
<213> Homo sapiens

<400> 4352
gaccaattct agaattc                                                    17

<210> 4353
<211> 17
<212> DNA
<213> Homo sapiens

<400> 4353
accaattcta gaattcc                                                    17

<210> 4354
<211> 17
<212> DNA
<213> Homo sapiens

<400> 4354
ccaattctag aattcct                                                    17

<210> 4355
<211> 17
<212> DNA
<213> Homo sapiens

<400> 4355
caattctaga attcctg                                                    17

<210> 4356
<211> 17

<212> DNA
<213> Homo sapiens

<400> 4356
aattctagaa ttcctga                                                    17

<210> 4357
<211> 17
<212> DNA
<213> Homo sapiens

<400> 4357
attctagaat tcctgat                                                    17

<210> 4358
<211> 17
<212> DNA
<213> Homo sapiens

<400> 4358
ttctagaatt cctgatt                                                    17

<210> 4359
<211> 17
<212> DNA
<213> Homo sapiens

<400> 4359
tctagaattc ctgattg                                                    17

<210> 4360
<211> 17
<212> DNA
<213> Homo sapiens

<400> 4360
ctagaattcc tgattgc                                                    17

<210> 4361
<211> 17
<212> DNA
<213> Homo sapiens

<400> 4361
tagaattcct gattgcc                                                    17

<210> 4362
<211> 17
<212> DNA
<213> Homo sapiens

<400> 4362
agaattcctg attgccc                                                    17

<210> 4363
<211> 17
<212> DNA
<213> Homo sapiens

<400> 4363
gaattcctga ttgccct                                                    17

<210> 4364
<211> 17
<212> DNA
<213> Homo sapiens

<400> 4364
aattcctgat tgcccta                                                    17

<210> 4365
<211> 17
<212> DNA
<213> Homo sapiens

<400> 4365
attcctgatt gccctat                                                    17

<210> 4366
<211> 17
<212> DNA
<213> Homo sapiens

<400> 4366
ttcctgattg ccctatt                                                    17

<210> 4367
<211> 17
<212> DNA
<213> Homo sapiens

<400> 4367
tcctgattgc cctattc                                                    17

<210> 4368
<211> 17
<212> DNA
<213> Homo sapiens

<400> 4368
cctgattgcc ctattcc                                                    17

<210> 4369
<211> 17
<212> DNA
<213> Homo sapiens

<400> 4369
ctgattgccc tattcca                                                    17

<210> 4370
<211> 17
<212> DNA
<213> Homo sapiens

<400> 4370
tgattgccct attccaa                                                    17

<210> 4371
<211> 17
<212> DNA
<213> Homo sapiens

<400> 4371
gattgcccta ttccaat                                                    17

<210> 4372
<211> 17
<212> DNA
<213> Homo sapiens

<400> 4372
attgccctat tccaatc                                                    17

<210> 4373
<211> 17
<212> DNA
<213> Homo sapiens

<400> 4373
ttgccctatt ccaatct                                                    17

<210> 4374
<211> 17
<212> DNA
<213> Homo sapiens

<400> 4374
tgccctattc caatctg                                                    17

<210> 4375
<211> 17
<212> DNA
<213> Homo sapiens

<400> 4375
gccctattcc aatctga                                                    17

<210> 4376
<211> 17
<212> DNA
<213> Homo sapiens

<400> 4376
ccctattcca atctgat                                                    17

<210> 4377
<211> 17
<212> DNA
<213> Homo sapiens

<400> 4377
cctattccaa tctgatt                                                    17

<210> 4378
<211> 17
<212> DNA
<213> Homo sapiens

<400> 4378
ctattccaat ctgattt                                                    17

<210> 4379

<211> 17
<212> DNA
<213> Homo sapiens

<400> 4379
tattccaatc tgatttt                                                    17

<210> 4380
<211> 17
<212> DNA
<213> Homo sapiens

<400> 4380
attccaatct gattttt                                                    17

<210> 4381
<211> 17
<212> DNA
<213> Homo sapiens

<400> 4381
ttccaatctg attttttt                                                   17

<210> 4382
<211> 17
<212> DNA
<213> Homo sapiens

<400> 4382
tccaatctga ttttttt                                                    17

<210> 4383
<211> 17
<212> DNA
<213> Homo sapiens

<400> 4383
ccaatctgat tttttta                                                    17

<210> 4384
<211> 17
<212> DNA
<213> Homo sapiens

<400> 4384
   caatctgatt tttttaa                                                   17

<210> 4385
<211> 17
<212> DNA
<213> Homo sapiens

<400> 4385
aatctgattt ttttaaa                                                    17

<210> 4386
<211> 17
<212> DNA
<213> Homo sapiens

<400> 4386

atctgatttt tttaaat                                                    17

<210> 4387
<211> 17
<212> DNA
<213> Homo sapiens

<400> 4387
tctgattttt ttaaatg                                                    17

<210> 4388
<211> 17
<212> DNA
<213> Homo sapiens

<400> 4388
ctgatttttt taaatgc                                                    17

<210> 4389
<211> 17
<212> DNA
<213> Homo sapiens

<400> 4389
tgattttttt aaatgca                                                    17

<210> 4390
<211> 17
<212> DNA
<213> Homo sapiens

<400> 4390
gattttttta aatgcac                                                    17

<210> 4391
<211> 17
<212> DNA
<213> Homo sapiens

<400> 4391
atttttttaa atgcact                                                    17

<210> 4392
<211> 17
<212> DNA
<213> Homo sapiens

<400> 4392
tttttttaaa tgcacta                                                    17

<210> 4393
<211> 17
<212> DNA
<213> Homo sapiens

<400> 4393
ttttttaaat gcactat                                                    17

<210> 4394
<211> 17
<212> DNA

<213> Homo sapiens

<400> 4394
tttttaaatg cactatt                                                    17

<210> 4395
<211> 17
<212> DNA
<213> Homo sapiens

<400> 4395
ttttaaatgc actatta                                                    17

<210> 4396
<211> 17
<212> DNA
<213> Homo sapiens

<400> 4396
tttaaatgca ctattaa                                                    17

<210> 4397
<211> 17
<212> DNA
<213> Homo sapiens

<400> 4397
ttaaatgcac tattaag                                                    17

<210> 4398
<211> 17
<212> DNA
<213> Homo sapiens

<400> 4398
taaatgcact attaaga                                                    17

<210> 4399
<211> 17
<212> DNA
<213> Homo sapiens

<400> 4399
aaatgcacta ttaagaa                                                    17

<210> 4400
<211> 17
<212> DNA
<213> Homo sapiens

<400> 4400
aatgcactat taagaat                                                    17

<210> 4401
<211> 17
<212> DNA
<213> Homo sapiens

<400> 4401
atgcactatt aagaata                                                    17

<210> 4402
<211> 17
<212> DNA
<213> Homo sapiens

<400> 4402
tgcactatta agaatag                                                    17

<210> 4403
<211> 17
<212> DNA
<213> Homo sapiens

<400> 4403
gcactattaa gaatagt                                                    17

<210> 4404
<211> 17
<212> DNA
<213> Homo sapiens

<400> 4404
cactattaag aatagtc                                                    17

<210> 4405
<211> 17
<212> DNA
<213> Homo sapiens

<400> 4405
actattaaga atagtca                                                    17

<210> 4406
<211> 17
<212> DNA
<213> Homo sapiens

<400> 4406
ctattaagaa tagtcaa                                                    17

<210> 4407
<211> 17
<212> DNA
<213> Homo sapiens

<400> 4407
tattaagaat agtcaac                                                    17

<210> 4408
<211> 17
<212> DNA
<213> Homo sapiens

<400> 4408
attaagaata gtcaaca                                                    17

<210> 4409
<211> 17
<212> DNA
<213> Homo sapiens

<400> 4409
ttaagaatag tcaacaa                                                    17

<210> 4410
<211> 17
<212> DNA
<213> Homo sapiens

<400> 4410
taagaatagt caacaaa                                                    17

<210> 4411
<211> 17
<212> DNA
<213> Homo sapiens

<400> 4411
aagaatagtc aacaaac                                                    17

<210> 4412
<211> 17
<212> DNA
<213> Homo sapiens

<400> 4412
agaatagtca acaaact                                                    17

<210> 4413
<211> 17
<212> DNA
<213> Homo sapiens

<400> 4413
gaatagtcaa caaacta                                                    17

<210> 4414
<211> 17
<212> DNA
<213> Homo sapiens

<400> 4414
aatagtcaac aaactac                                                    17

<210> 4415
<211> 25
<212> DNA
<213> Homo sapiens

<400> 4415
tccatgatta atcttgtcat ttgta                                           25

<210> 4416
<211> 25
<212> DNA
<213> Homo sapiens

<400> 4416
ccatgattaa tcttgtcatt tgtac                                           25

<210> 4417
<211> 25

<212> DNA
<213> Homo sapiens

<400> 4417
catgattaat cttgtcattt gtaca                                            25

<210> 4418
<211> 25
<212> DNA
<213> Homo sapiens

<400> 4418
atgattaatc ttgtcatttg tacag                                            25

<210> 4419
<211> 25
<212> DNA
<213> Homo sapiens

<400> 4419
tgattaatct tgtcatttgt acagg                                            25

<210> 4420
<211> 25
<212> DNA
<213> Homo sapiens

<400> 4420
gattaatctt gtcatttgta caggg                                            25

<210> 4421
<211> 25
<212> DNA
<213> Homo sapiens

<400> 4421
attaatcttg tcatttgtac agggt                                            25

<210> 4422
<211> 25
<212> DNA
<213> Homo sapiens

<400> 4422
ttaatcttgt catttgtaca gggtt                                            25

<210> 4423
<211> 25
<212> DNA
<213> Homo sapiens

<400> 4423
taatcttgtc atttgtacag ggttt                                            25

<210> 4424
<211> 25
<212> DNA
<213> Homo sapiens

<400> 4424
aatcttgtca tttgtacagg gtttt                                            25

<210> 4425
<211> 25
<212> DNA
<213> Homo sapiens

<400> 4425
atcttgtcat ttgtacaggg ttttc                                          25

<210> 4426
<211> 25
<212> DNA
<213> Homo sapiens

<400> 4426
tcttgtcatt tgtacagggt tttct                                          25

<210> 4427
<211> 25
<212> DNA
<213> Homo sapiens

<400> 4427
cttgtcattt gtacagggtt ttctt                                          25

<210> 4428
<211> 25
<212> DNA
<213> Homo sapiens

<400> 4428
ttgtcatttg tacagggttt tcttt                                          25

<210> 4429
<211> 25
<212> DNA
<213> Homo sapiens

<400> 4429
tgtcatttgt acagggtttt ctttc                                          25

<210> 4430
<211> 25
<212> DNA
<213> Homo sapiens

<400> 4430
gtcatttgta cagggttttc tttcg                                          25

<210> 4431
<211> 25
<212> DNA
<213> Homo sapiens

<400> 4431
tcatttgtac agggttttct ttcga                                          25

<210> 4432
<211> 25
<212> DNA
<213> Homo sapiens

<400> 4432
catttgtaca gggttttctt tcgat                                          25

<210> 4433
<211> 25
<212> DNA
<213> Homo sapiens

<400> 4433
atttgtacag ggttttcttt cgatt                                          25

<210> 4434
<211> 25
<212> DNA
<213> Homo sapiens

<400> 4434
tttgtacagg gttttctttc gattt                                          25

<210> 4435
<211> 25
<212> DNA
<213> Homo sapiens

<400> 4435
ttgtacaggg ttttctttcg atttt                                          25

<210> 4436
<211> 25
<212> DNA
<213> Homo sapiens

<400> 4436
tgtacagggt tttctttcga ttttt                                          25

<210> 4437
<211> 25
<212> DNA
<213> Homo sapiens

<400> 4437
gtacagggtt ttctttcgat ttttc                                          25

<210> 4438
<211> 25
<212> DNA
<213> Homo sapiens

<400> 4438
tacagggttt tctttcgatt tttct                                          25

<210> 4439
<211> 25
<212> DNA
<213> Homo sapiens

<400> 4439
acagggtttt ctttcgattt ttctg                                          25

<210> 4440

<211> 25
<212> DNA
<213> Homo sapiens

<400> 4440
cagggttttc tttcgatttt tctgc                                          25

<210> 4441
<211> 25
<212> DNA
<213> Homo sapiens

<400> 4441
agggttttct ttcgattttt ctgca                                          25

<210> 4442
<211> 25
<212> DNA
<213> Homo sapiens

<400> 4442
gggttttctt tcgatttttc tgcac                                          25

<210> 4443
<211> 25
<212> DNA
<213> Homo sapiens

<400> 4443
ggttttcttt cgatttttct gcaca                                          25

<210> 4444
<211> 25
<212> DNA
<213> Homo sapiens

<400> 4444
gttttctttc gatttttctg cacac                                          25

<210> 4445
<211> 25
<212> DNA
<213> Homo sapiens

<400> 4445
ttttctttcg atttttctgc acaca                                          25

<210> 4446
<211> 25
<212> DNA
<213> Homo sapiens

<400> 4446
tttctttcga tttttctgca cacat                                          25

<210> 4447
<211> 25
<212> DNA
<213> Homo sapiens

<400> 4447

ttctttcgat ttttctgcac acatt 25

<210> 4448
<211> 25
<212> DNA
<213> Homo sapiens

<400> 4448
tctttcgatt tttctgcaca cattt 25

<210> 4449
<211> 25
<212> DNA
<213> Homo sapiens

<400> 4449
ctttcgattt ttctgcacac atttc 25

<210> 4450
<211> 25
<212> DNA
<213> Homo sapiens

<400> 4450
tttcgatttt tctgcacaca tttcc 25

<210> 4451
<211> 25
<212> DNA
<213> Homo sapiens

<400> 4451
ttcgattttt ctgcacacat ttcct 25

<210> 4452
<211> 25
<212> DNA
<213> Homo sapiens

<400> 4452
tcgatttttc tgcacacatt tccta 25

<210> 4453
<211> 25
<212> DNA
<213> Homo sapiens

<400> 4453
cgatttttct gcacacattt cctat 25

<210> 4454
<211> 25
<212> DNA
<213> Homo sapiens

<400> 4454
gatttttctg cacacatttc ctatg 25

<210> 4455
<211> 25
<212> DNA

<213> Homo sapiens

<400> 4455
atttttctgc acacatttcc tatgc                                          25

<210> 4456
<211> 25
<212> DNA
<213> Homo sapiens

<400> 4456
tttttctgca cacatttcct atgca                                          25

<210> 4457
<211> 25
<212> DNA
<213> Homo sapiens

<400> 4457
ttttctgcac acatttccta tgcat                                          25

<210> 4458
<211> 25
<212> DNA
<213> Homo sapiens

<400> 4458
tttctgcaca catttcctat gcatt                                          25

<210> 4459
<211> 25
<212> DNA
<213> Homo sapiens

<400> 4459
ttctgcacac atttcctatg cattt                                          25

<210> 4460
<211> 25
<212> DNA
<213> Homo sapiens

<400> 4460
tctgcacaca tttcctatgc atttg                                          25

<210> 4461
<211> 25
<212> DNA
<213> Homo sapiens

<400> 4461
ctgcacacat ttcctatgca tttgt                                          25

<210> 4462
<211> 25
<212> DNA
<213> Homo sapiens

<400> 4462
tgcacacatt tcctatgcat ttgtt                                          25

<210> 4463
<211> 25
<212> DNA
<213> Homo sapiens

<400> 4463
gcacacattt cctatgcatt tgttt                                          25

<210> 4464
<211> 25
<212> DNA
<213> Homo sapiens

<400> 4464
cacacatttc ctatgcattt gtttc                                          25

<210> 4465
<211> 25
<212> DNA
<213> Homo sapiens

<400> 4465
acacatttcc tatgcatttg tttct                                          25

<210> 4466
<211> 25
<212> DNA
<213> Homo sapiens

<400> 4466
cacatttcct atgcatttgt ttcta                                          25

<210> 4467
<211> 25
<212> DNA
<213> Homo sapiens

<400> 4467
acatttccta tgcatttgtt tctag                                          25

<210> 4468
<211> 25
<212> DNA
<213> Homo sapiens

<400> 4468
catttcctat gcatttgttt ctagt                                          25

<210> 4469
<211> 25
<212> DNA
<213> Homo sapiens

<400> 4469
atttcctatg catttgtttc tagtt                                          25

<210> 4470
<211> 25
<212> DNA
<213> Homo sapiens

<400> 4470
tttcctatgc atttgtttct agttc                                          25

<210> 4471
<211> 25
<212> DNA
<213> Homo sapiens

<400> 4471
ttcctatgca tttgtttcta gttct                                          25

<210> 4472
<211> 25
<212> DNA
<213> Homo sapiens

<400> 4472
tcctatgcat ttgtttctag ttctc                                          25

<210> 4473
<211> 25
<212> DNA
<213> Homo sapiens

<400> 4473
cctatgcatt tgtttctagt tctca                                          25

<210> 4474
<211> 25
<212> DNA
<213> Homo sapiens

<400> 4474
ctatgcattt gtttctagtt ctcaa                                          25

<210> 4475
<211> 25
<212> DNA
<213> Homo sapiens

<400> 4475
tatgcatttg tttctagttc tcaac                                          25

<210> 4476
<211> 25
<212> DNA
<213> Homo sapiens

<400> 4476
atgcatttgt ttctagttct caacc                                          25

<210> 4477
<211> 25
<212> DNA
<213> Homo sapiens

<400> 4477
tgcatttgtt tctagttctc aaccc                                          25

<210> 4478
<211> 25

<212> DNA
<213> Homo sapiens

<400> 4478
gcatttgttt ctagttctca accct                                          25

<210> 4479
<211> 25
<212> DNA
<213> Homo sapiens

<400> 4479
catttgtttc tagttctcaa ccctc                                          25

<210> 4480
<211> 25
<212> DNA
<213> Homo sapiens

<400> 4480
atttgtttct agttctcaac cctca                                          25

<210> 4481
<211> 25
<212> DNA
<213> Homo sapiens

<400> 4481
tttgtttcta gttctcaacc ctcag                                          25

<210> 4482
<211> 25
<212> DNA
<213> Homo sapiens

<400> 4482
ttgtttctag ttctcaaccc tcagt                                          25

<210> 4483
<211> 25
<212> DNA
<213> Homo sapiens

<400> 4483
tgtttctagt tctcaaccct cagta                                          25

<210> 4484
<211> 25
<212> DNA
<213> Homo sapiens

<400> 4484
gtttctagtt ctcaaccctc agtaa                                          25

<210> 4485
<211> 25
<212> DNA
<213> Homo sapiens

<400> 4485
tttctagttc tcaaccctca gtaaa                                          25

<210> 4486
<211> 25
<212> DNA
<213> Homo sapiens

<400> 4486
ttctagttct caaccctcag taaac                                          25

<210> 4487
<211> 25
<212> DNA
<213> Homo sapiens

<400> 4487
tctagttctc aaccctcagt aaacc                                          25

<210> 4488
<211> 25
<212> DNA
<213> Homo sapiens

<400> 4488
ctagttctca accctcagta aacca                                          25

<210> 4489
<211> 25
<212> DNA
<213> Homo sapiens

<400> 4489
tagttctcaa ccctcagtaa accaa                                          25

<210> 4490
<211> 25
<212> DNA
<213> Homo sapiens

<400> 4490
agttctcaac cctcagtaaa ccaaa                                          25

<210> 4491
<211> 25
<212> DNA
<213> Homo sapiens

<400> 4491
gttctcaacc ctcagtaaac caaaa                                          25

<210> 4492
<211> 25
<212> DNA
<213> Homo sapiens

<400> 4492
ttctcaaccc tcagtaaacc aaaaa                                          25

<210> 4493
<211> 25
<212> DNA
<213> Homo sapiens

<400> 4493
tctcaaccct cagtaaacca aaaat                                          25

<210> 4494
<211> 25
<212> DNA
<213> Homo sapiens

<400> 4494
ctcaaccctc agtaaaccaa aaatc                                          25

<210> 4495
<211> 25
<212> DNA
<213> Homo sapiens

<400> 4495
tcaaccctca gtaaaccaaa aatca                                          25

<210> 4496
<211> 25
<212> DNA
<213> Homo sapiens

<400> 4496
caaccctcag taaaccaaaa atcag                                          25

<210> 4497
<211> 25
<212> DNA
<213> Homo sapiens

<400> 4497
aaccctcagt aaaccaaaaa tcagt                                          25

<210> 4498
<211> 25
<212> DNA
<213> Homo sapiens

<400> 4498
accctcagta aaccaaaaat cagtt                                          25

<210> 4499
<211> 25
<212> DNA
<213> Homo sapiens

<400> 4499
ccctcagtaa accaaaaatc agttg                                          25

<210> 4500
<211> 25
<212> DNA
<213> Homo sapiens

<400> 4500
cctcagtaaa ccaaaaatca gttga                                          25

<210> 4501

<211> 25
<212> DNA
<213> Homo sapiens

<400> 4501
ctcagtaaac caaaaatcag ttgag                                          25

<210> 4502
<211> 25
<212> DNA
<213> Homo sapiens

<400> 4502
tcagtaaacc aaaaatcagt tgagg                                          25

<210> 4503
<211> 25
<212> DNA
<213> Homo sapiens

<400> 4503
cagtaaacca aaaatcagtt gaggc                                          25

<210> 4504
<211> 25
<212> DNA
<213> Homo sapiens

<400> 4504
agtaaaccaa aaatcagttg aggca                                          25

<210> 4505
<211> 25
<212> DNA
<213> Homo sapiens

<400> 4505
gtaaaccaaa aatcagttga ggcat                                          25

<210> 4506
<211> 25
<212> DNA
<213> Homo sapiens

<400> 4506
taaaccaaaa atcagttgag gcatt                                          25

<210> 4507
<211> 25
<212> DNA
<213> Homo sapiens

<400> 4507
aaaccaaaaa tcagttgagg cattg                                          25

<210> 4508
<211> 25
<212> DNA
<213> Homo sapiens

<400> 4508

aaccaaaaat cagttgaggc attgt 25

<210> 4509
<211> 25
<212> DNA
<213> Homo sapiens

<400> 4509
accaaaaatc agttgaggca ttgta 25

<210> 4510
<211> 25
<212> DNA
<213> Homo sapiens

<400> 4510
ccaaaaatca gttgaggcat tgtat 25

<210> 4511
<211> 25
<212> DNA
<213> Homo sapiens

<400> 4511
caaaaatcag ttgaggcatt gtatc 25

<210> 4512
<211> 25
<212> DNA
<213> Homo sapiens

<400> 4512
aaaaatcagt tgaggcattg tatct 25

<210> 4513
<211> 25
<212> DNA
<213> Homo sapiens

<400> 4513
aaaatcagtt gaggcattgt atctt 25

<210> 4514
<211> 25
<212> DNA
<213> Homo sapiens

<400> 4514
aaatcagttg aggcattgta tcttc 25

<210> 4515
<211> 25
<212> DNA
<213> Homo sapiens

<400> 4515
aatcagttga ggcattgtat cttct 25

<210> 4516
<211> 25
<212> DNA

<213> Homo sapiens

<400> 4516
atcagttgag gcattgtatc ttcta                                          25

<210> 4517
<211> 25
<212> DNA
<213> Homo sapiens

<400> 4517
tcagttgagg cattgtatct tctag                                          25

<210> 4518
<211> 25
<212> DNA
<213> Homo sapiens

<400> 4518
cagttgaggc attgtatctt ctagg                                          25

<210> 4519
<211> 25
<212> DNA
<213> Homo sapiens

<400> 4519
agttgaggca ttgtatcttc taggc                                          25

<210> 4520
<211> 25
<212> DNA
<213> Homo sapiens

<400> 4520
gttgaggcat tgtatcttct aggct                                          25

<210> 4521
<211> 25
<212> DNA
<213> Homo sapiens

<400> 4521
ttgaggcatt gtatcttcta ggcta                                          25

<210> 4522
<211> 25
<212> DNA
<213> Homo sapiens

<400> 4522
tgaggcattg tatcttctag gctac                                          25

<210> 4523
<211> 25
<212> DNA
<213> Homo sapiens

<400> 4523
gaggcattgt atcttctagg ctacc                                          25

<210> 4524
<211> 25
<212> DNA
<213> Homo sapiens

<400> 4524
aggcattgta tcttctaggc taccc                                          25

<210> 4525
<211> 25
<212> DNA
<213> Homo sapiens

<400> 4525
ggcattgtat cttctaggct accca                                          25

<210> 4526
<211> 25
<212> DNA
<213> Homo sapiens

<400> 4526
gcattgtatc ttctaggcta cccag                                          25

<210> 4527
<211> 25
<212> DNA
<213> Homo sapiens

<400> 4527
cattgtatct tctaggctac ccagt                                          25

<210> 4528
<211> 25
<212> DNA
<213> Homo sapiens

<400> 4528
attgtatctt ctaggctacc cagtg                                          25

<210> 4529
<211> 25
<212> DNA
<213> Homo sapiens

<400> 4529
ttgtatcttc taggctaccc agtgt                                          25

<210> 4530
<211> 25
<212> DNA
<213> Homo sapiens

<400> 4530
tgtatcttct aggctaccca gtgtt                                          25

<210> 4531
<211> 25
<212> DNA
<213> Homo sapiens

<400> 4531
gtatcttcta ggctacccag tgtta                                          25

<210> 4532
<211> 25
<212> DNA
<213> Homo sapiens

<400> 4532
tatcttctag gctacccagt gttac                                          25

<210> 4533
<211> 25
<212> DNA
<213> Homo sapiens

<400> 4533
atcttctagg ctacccagtg ttaca                                          25

<210> 4534
<211> 25
<212> DNA
<213> Homo sapiens

<400> 4534
tcttctaggc tacccagtgt tacaa                                          25

<210> 4535
<211> 25
<212> DNA
<213> Homo sapiens

<400> 4535
cttctaggct acccagtgtt acaaa                                          25

<210> 4536
<211> 25
<212> DNA
<213> Homo sapiens

<400> 4536
ttctaggcta cccagtgtta caaag                                          25

<210> 4537
<211> 25
<212> DNA
<213> Homo sapiens

<400> 4537
tctaggctac ccagtgttac aaagt                                          25

<210> 4538
<211> 25
<212> DNA
<213> Homo sapiens

<400> 4538
ctaggctacc cagtgttaca aagtg                                          25

<210> 4539
<211> 25

<212> DNA
<213> Homo sapiens

<400> 4539
taggctaccc agtgttacaa agtgc                                              25

<210> 4540
<211> 25
<212> DNA
<213> Homo sapiens

<400> 4540
aggctaccca gtgttacaaa gtgca                                              25

<210> 4541
<211> 25
<212> DNA
<213> Homo sapiens

<400> 4541
ggctacccag tgttacaaag tgcaa                                              25

<210> 4542
<211> 25
<212> DNA
<213> Homo sapiens

<400> 4542
gctacccagt gttacaaagt gcaat                                              25

<210> 4543
<211> 25
<212> DNA
<213> Homo sapiens

<400> 4543
ctacccagtg ttacaaagtg caatt                                              25

<210> 4544
<211> 25
<212> DNA
<213> Homo sapiens

<400> 4544
tacccagtgt tacaaagtgc aattt                                              25

<210> 4545
<211> 25
<212> DNA
<213> Homo sapiens

<400> 4545
acccagtgtt acaaagtgca atttc                                              25

<210> 4546
<211> 25
<212> DNA
<213> Homo sapiens

<400> 4546
cccagtgtta caaagtgcaa tttca                                              25

<210> 4547
<211> 25
<212> DNA
<213> Homo sapiens

<400> 4547
ccagtgttac aaagtgcaat ttcaa                                          25

<210> 4548
<211> 25
<212> DNA
<213> Homo sapiens

<400> 4548
cagtgttaca aagtgcaatt tcaac                                          25

<210> 4549
<211> 25
<212> DNA
<213> Homo sapiens

<400> 4549
agtgttacaa agtgcaattt caaca                                          25

<210> 4550
<211> 25
<212> DNA
<213> Homo sapiens

<400> 4550
gtgttacaaa gtgcaatttc aacaa                                          25

<210> 4551
<211> 25
<212> DNA
<213> Homo sapiens

<400> 4551
tgttacaaag tgcaatttca acaat                                          25

<210> 4552
<211> 25
<212> DNA
<213> Homo sapiens

<400> 4552
gttacaaagt gcaatttcaa caata                                          25

<210> 4553
<211> 25
<212> DNA
<213> Homo sapiens

<400> 4553
ttacaaagtg caatttcaac aataa                                          25

<210> 4554
<211> 25
<212> DNA
<213> Homo sapiens

<400> 4554
tacaaagtgc aatttcaaca ataat                                          25

<210> 4555
<211> 25
<212> DNA
<213> Homo sapiens

<400> 4555
acaaagtgca atttcaacaa taata                                          25

<210> 4556
<211> 25
<212> DNA
<213> Homo sapiens

<400> 4556
caaagtgcaa tttcaacaat aatag                                          25

<210> 4557
<211> 25
<212> DNA
<213> Homo sapiens

<400> 4557
aaagtgcaat ttcaacaata atagg                                          25

<210> 4558
<211> 25
<212> DNA
<213> Homo sapiens

<400> 4558
aagtgcaatt tcaacaataa taggg                                          25

<210> 4559
<211> 25
<212> DNA
<213> Homo sapiens

<400> 4559
agtgcaattt caacaataat agggg                                          25

<210> 4560
<211> 25
<212> DNA
<213> Homo sapiens

<400> 4560
gtgcaatttc aacaataata ggggt                                          25

<210> 4561
<211> 25
<212> DNA
<213> Homo sapiens

<400> 4561
tgcaatttca acaataatag gggtg                                          25

<210> 4562

<211> 25
<212> DNA
<213> Homo sapiens

<400> 4562
gcaatttcaa caataatagg ggtgt                                            25

<210> 4563
<211> 25
<212> DNA
<213> Homo sapiens

<400> 4563
caatttcaac aataataggg gtgtg                                            25

<210> 4564
<211> 25
<212> DNA
<213> Homo sapiens

<400> 4564
aatttcaaca ataatagggg tgtgt                                            25

<210> 4565
<211> 25
<212> DNA
<213> Homo sapiens

<400> 4565
atttcaacaa taataggggt gtgtg                                            25

<210> 4566
<211> 25
<212> DNA
<213> Homo sapiens

<400> 4566
tttcaacaat aataggggtg tgtgt                                            25

<210> 4567
<211> 25
<212> DNA
<213> Homo sapiens

<400> 4567
ttcaacaata ataggggtgt gtgtt                                            25

<210> 4568
<211> 25
<212> DNA
<213> Homo sapiens

<400> 4568
tcaacaataa taggggtgtg tgttt                                            25

<210> 4569
<211> 25
<212> DNA
<213> Homo sapiens

<400> 4569

caacaataat aggggtgtgt gtttt                                          25

<210> 4570
<211> 25
<212> DNA
<213> Homo sapiens

<400> 4570
aacaataata ggggtgtgtg tttta                                          25

<210> 4571
<211> 25
<212> DNA
<213> Homo sapiens

<400> 4571
acaataatag gggtgtgtgt tttag                                          25

<210> 4572
<211> 25
<212> DNA
<213> Homo sapiens

<400> 4572
caataatagg ggtgtgtgtt ttagc                                          25

<210> 4573
<211> 25
<212> DNA
<213> Homo sapiens

<400> 4573
aataataggg gtgtgtgttt tagct                                          25

<210> 4574
<211> 25
<212> DNA
<213> Homo sapiens

<400> 4574
ataatagggg tgtgtgtttt agctg                                          25

<210> 4575
<211> 25
<212> DNA
<213> Homo sapiens

<400> 4575
taataggggt gtgtgtttta gctgc                                          25

<210> 4576
<211> 25
<212> DNA
<213> Homo sapiens

<400> 4576
aataggggtg tgtgttttag ctgca                                          25

<210> 4577
<211> 25
<212> DNA

<213> Homo sapiens

<400> 4577
ataggggtgt gtgttttagc tgcag                                          25

<210> 4578
<211> 25
<212> DNA
<213> Homo sapiens

<400> 4578
taggggtgtg tgttttagct gcagc                                          25

<210> 4579
<211> 25
<212> DNA
<213> Homo sapiens

<400> 4579
aggggtgtgt gttttagctg cagcg                                          25

<210> 4580
<211> 25
<212> DNA
<213> Homo sapiens

<400> 4580
ggggtgtgtg ttttagctgc agcga                                          25

<210> 4581
<211> 25
<212> DNA
<213> Homo sapiens

<400> 4581
gggtgtgtgt tttagctgca gcgaa                                          25

<210> 4582
<211> 25
<212> DNA
<213> Homo sapiens

<400> 4582
ggtgtgtgtt ttagctgcag cgaaa                                          25

<210> 4583
<211> 25
<212> DNA
<213> Homo sapiens

<400> 4583
gtgtgtgttt tagctgcagc gaaag                                          25

<210> 4584
<211> 25
<212> DNA
<213> Homo sapiens

<400> 4584
tgtgtgtttt agctgcagcg aaagc                                          25

<210> 4585
<211> 25
<212> DNA
<213> Homo sapiens

<400> 4585
gtgtgtttta gctgcagcga aagca                                            25

<210> 4586
<211> 25
<212> DNA
<213> Homo sapiens

<400> 4586
tgtgttttag ctgcagcgaa agcat                                            25

<210> 4587
<211> 25
<212> DNA
<213> Homo sapiens

<400> 4587
gtgttttagc tgcagcgaaa gcata                                            25

<210> 4588
<211> 25
<212> DNA
<213> Homo sapiens

<400> 4588
tgttttagct gcagcgaaag catac                                            25

<210> 4589
<211> 25
<212> DNA
<213> Homo sapiens

<400> 4589
gttttagctg cagcgaaagc ataca                                            25

<210> 4590
<211> 25
<212> DNA
<213> Homo sapiens

<400> 4590
ttttagctgc agcgaaagca tacat                                            25

<210> 4591
<211> 25
<212> DNA
<213> Homo sapiens

<400> 4591
tttagctgca gcgaaagcat acatc                                            25

<210> 4592
<211> 25
<212> DNA
<213> Homo sapiens

<400> 4592
ttagctgcag cgaaagcata catct                                          25

<210> 4593
<211> 25
<212> DNA
<213> Homo sapiens

<400> 4593
tagctgcagc gaaagcatac atctt                                          25

<210> 4594
<211> 25
<212> DNA
<213> Homo sapiens

<400> 4594
agctgcagcg aaagcataca tcttc                                          25

<210> 4595
<211> 25
<212> DNA
<213> Homo sapiens

<400> 4595
gctgcagcga aagcatacat cttca                                          25

<210> 4596
<211> 25
<212> DNA
<213> Homo sapiens

<400> 4596
ctgcagcgaa agcatacatc ttcag                                          25

<210> 4597
<211> 25
<212> DNA
<213> Homo sapiens

<400> 4597
tgcagcgaaa gcatacatct tcaga                                          25

<210> 4598
<211> 25
<212> DNA
<213> Homo sapiens

<400> 4598
gcagcgaaag catacatctt cagaa                                          25

<210> 4599
<211> 25
<212> DNA
<213> Homo sapiens

<400> 4599
cagcgaaagc atacatcttc agaac                                          25

<210> 4600
<211> 25

<212> DNA
<213> Homo sapiens

<400> 4600
agcgaaagca tacatcttca gaaca                                          25

<210> 4601
<211> 25
<212> DNA
<213> Homo sapiens

<400> 4601
gcgaaagcat acatcttcag aacat                                          25

<210> 4602
<211> 25
<212> DNA
<213> Homo sapiens

<400> 4602
cgaaagcata catcttcaga acatt                                          25

<210> 4603
<211> 25
<212> DNA
<213> Homo sapiens

<400> 4603
gaaagcatac atcttcagaa cattt                                          25

<210> 4604
<211> 25
<212> DNA
<213> Homo sapiens

<400> 4604
aaagcataca tcttcagaac atttt                                          25

<210> 4605
<211> 25
<212> DNA
<213> Homo sapiens

<400> 4605
aagcatacat cttcagaaca ttttt                                          25

<210> 4606
<211> 25
<212> DNA
<213> Homo sapiens

<400> 4606
agcatacatc ttcagaacat ttttt                                          25

<210> 4607
<211> 25
<212> DNA
<213> Homo sapiens

<400> 4607
gcatacatct tcagaacatt tttta                                          25

<210> 4608
<211> 25
<212> DNA
<213> Homo sapiens

<400> 4608
catacatctt cagaacattt tttaa                                          25

<210> 4609
<211> 25
<212> DNA
<213> Homo sapiens

<400> 4609
atacatcttc agaacatttt ttaag                                          25

<210> 4610
<211> 25
<212> DNA
<213> Homo sapiens

<400> 4610
tacatcttca gaacattttt taaga                                          25

<210> 4611
<211> 25
<212> DNA
<213> Homo sapiens

<400> 4611
acatcttcag aacatttttt aagat                                          25

<210> 4612
<211> 25
<212> DNA
<213> Homo sapiens

<400> 4612
catcttcaga acatttttta agatt                                          25

<210> 4613
<211> 25
<212> DNA
<213> Homo sapiens

<400> 4613
atcttcagaa cattttttaa gatta                                          25

<210> 4614
<211> 25
<212> DNA
<213> Homo sapiens

<400> 4614
tcttcagaac attttttaag attat                                          25

<210> 4615
<211> 25
<212> DNA
<213> Homo sapiens

<400> 4615
   cttcagaaca ttttttaaga ttatg                                          25

<210> 4616
<211> 25
<212> DNA
<213> Homo sapiens

<400> 4616
   ttcagaacat tttttaagat tatgt                                          25

<210> 4617
<211> 25
<212> DNA
<213> Homo sapiens

<400> 4617
tcagaacatt ttttaagatt atgtt                                          25

<210> 4618
<211> 25
<212> DNA
<213> Homo sapiens

<400> 4618
cagaacattt tttaagatta tgttt                                          25

<210> 4619
<211> 25
<212> DNA
<213> Homo sapiens

<400> 4619
agaacatttt ttaagattat gtttc                                          25

<210> 4620
<211> 25
<212> DNA
<213> Homo sapiens

<400> 4620
gaacattttt taagattatg tttct                                          25

<210> 4621
<211> 25
<212> DNA
<213> Homo sapiens

<400> 4621
aacatttttt aagattatgt ttctt                                          25

<210> 4622
<211> 25
<212> DNA
<213> Homo sapiens

<400> 4622
acatttttta agattatgtt tcttg                                          25

<210> 4623

<211> 25
<212> DNA
<213> Homo sapiens

<400> 4623
catttttaa gattatgttt cttgt                                              25

<210> 4624
<211> 25
<212> DNA
<213> Homo sapiens

<400> 4624
   attttttaag attatgtttc ttgtt                                              25

<210> 4625
<211> 25
<212> DNA
<213> Homo sapiens

<400> 4625
tttttaaga ttatgtttct tgtta                                              25

<210> 4626
<211> 25
<212> DNA
<213> Homo sapiens

<400> 4626
   tttttaagat tatgtttctt gttat                                              25

<210> 4627
<211> 25
<212> DNA
<213> Homo sapiens

<400> 4627
   ttttaagatt atgtttcttg ttatg                                              25

<210> 4628
<211> 25
<212> DNA
<213> Homo sapiens

<400> 4628
tttaagatta tgtttcttgt tatga                                              25

<210> 4629
<211> 25
<212> DNA
<213> Homo sapiens

<400> 4629
ttaagattat gtttcttgtt atgat                                              25

<210> 4630
<211> 25
<212> DNA
<213> Homo sapiens

<400> 4630

taagattatg tttcttgtta tgata                                          25

<210> 4631
<211> 25
<212> DNA
<213> Homo sapiens

<400> 4631
aagattatgt ttcttgttat gatat                                          25

<210> 4632
<211> 25
<212> DNA
<213> Homo sapiens

<400> 4632
agattatgtt tcttgttatg atatt                                          25

<210> 4633
<211> 25
<212> DNA
<213> Homo sapiens

<400> 4633
gattatgttt cttgttatga tattt                                          25

<210> 4634
<211> 25
<212> DNA
<213> Homo sapiens

<400> 4634
attatgtttc ttgttatgat atttg                                          25

<210> 4635
<211> 25
<212> DNA
<213> Homo sapiens

<400> 4635
ttatgtttct tgttatgata tttgg                                          25

<210> 4636
<211> 25
<212> DNA
<213> Homo sapiens

<400> 4636
tatgtttctt gttatgatat ttggg                                          25

<210> 4637
<211> 25
<212> DNA
<213> Homo sapiens

<400> 4637
atgtttcttg ttatgatatt tgggg                                          25

<210> 4638
<211> 25
<212> DNA

<213> Homo sapiens

<400> 4638
tgtttcttgt tatgatattt ggggc                                          25

<210> 4639
<211> 25
<212> DNA
<213> Homo sapiens

<400> 4639
gtttcttgtt atgatatttg gggca                                          25

<210> 4640
<211> 25
<212> DNA
<213> Homo sapiens

<400> 4640
tttcttgtta tgatatttgg ggcag                                          25

<210> 4641
<211> 25
<212> DNA
<213> Homo sapiens

<400> 4641
ttcttgttat gatatttggg gcagc                                          25

<210> 4642
<211> 25
<212> DNA
<213> Homo sapiens

<400> 4642
tcttgttatg atatttgggg cagct                                          25

<210> 4643
<211> 25
<212> DNA
<213> Homo sapiens

<400> 4643
cttgttatga tatttggggc agctc                                          25

<210> 4644
<211> 25
<212> DNA
<213> Homo sapiens

<400> 4644
ttgttatgat atttggggca gctca                                          25

<210> 4645
<211> 25
<212> DNA
<213> Homo sapiens

<400> 4645
tgttatgata tttggggcag ctcat                                          25

<210> 4646
<211> 25
<212> DNA
<213> Homo sapiens

<400> 4646
gttatgatat ttggggcagc tcatg                                          25

<210> 4647
<211> 25
<212> DNA
<213> Homo sapiens

<400> 4647
ttatgatatt tggggcagct catgg                                          25

<210> 4648
<211> 25
<212> DNA
<213> Homo sapiens

<400> 4648
tatgatattt ggggcagctc atggc                                          25

<210> 4649
<211> 25
<212> DNA
<213> Homo sapiens

<400> 4649
atgatatttg gggcagctca tggcc                                          25

<210> 4650
<211> 25
<212> DNA
<213> Homo sapiens

<400> 4650
tgatatttgg ggcagctcat ggcct                                          25

<210> 4651
<211> 25
<212> DNA
<213> Homo sapiens

<400> 4651
gatatttggg gcagctcatg gccta                                          25

<210> 4652
<211> 25
<212> DNA
<213> Homo sapiens

<400> 4652
atatttgggg cagctcatgg cctaa                                          25

<210> 4653
<211> 25
<212> DNA
<213> Homo sapiens

<400> 4653
tatttggggc agctcatggc ctaat                                          25

<210> 4654
<211> 25
<212> DNA
<213> Homo sapiens

<400> 4654
atttggggca gctcatggcc taatt                                          25

<210> 4655
<211> 25
<212> DNA
<213> Homo sapiens

<400> 4655
tttggggcag ctcatggcct aattt                                          25

<210> 4656
<211> 25
<212> DNA
<213> Homo sapiens

<400> 4656
ttggggcagc tcatggccta atttt                                          25

<210> 4657
<211> 25
<212> DNA
<213> Homo sapiens

<400> 4657
tggggcagct catggcctaa ttttt                                          25

<210> 4658
<211> 25
<212> DNA
<213> Homo sapiens

<400> 4658
ggggcagctc atggcctaat tttta                                          25

<210> 4659
<211> 25
<212> DNA
<213> Homo sapiens

<400> 4659
gggcagctca tggcctaatt tttat                                          25

<210> 4660
<211> 25
<212> DNA
<213> Homo sapiens

<400> 4660
ggcagctcat ggcctaattt ttatt                                          25

<210> 4661
<211> 25

<212> DNA
<213> Homo sapiens

<400> 4661
gcagctcatg gcctaatttt tattc                                          25

<210> 4662
<211> 25
<212> DNA
<213> Homo sapiens

<400> 4662
cagctcatgg cctaattttt attcc                                          25

<210> 4663
<211> 25
<212> DNA
<213> Homo sapiens

<400> 4663
agctcatggc ctaattttta ttcca                                          25

<210> 4664
<211> 25
<212> DNA
<213> Homo sapiens

<400> 4664
gctcatggcc taatttttat tccag                                          25

<210> 4665
<211> 25
<212> DNA
<213> Homo sapiens

<400> 4665
ctcatggcct aatttttatt ccagt                                          25

<210> 4666
<211> 25
<212> DNA
<213> Homo sapiens

<400> 4666
tcatggccta atttttattc cagta                                          25

<210> 4667
<211> 25
<212> DNA
<213> Homo sapiens

<400> 4667
catggcctaa tttttattcc agtat                                          25

<210> 4668
<211> 25
<212> DNA
<213> Homo sapiens

<400> 4668
atggcctaat ttttattcca gtatt                                          25

<210> 4669
<211> 25
<212> DNA
<213> Homo sapiens

<400> 4669
tggcctaatt tttattccag tattc                                              25

<210> 4670
<211> 25
<212> DNA
<213> Homo sapiens

<400> 4670
ggcctaattt ttattccagt attct                                              25

<210> 4671
<211> 25
<212> DNA
<213> Homo sapiens

<400> 4671
gcctaatttt tattccagta ttctt                                              25

<210> 4672
<211> 25
<212> DNA
<213> Homo sapiens

<400> 4672
cctaattttt attccagtat tctta                                              25

<210> 4673
<211> 25
<212> DNA
<213> Homo sapiens

<400> 4673
ctaattttta ttccagtatt cttaa                                              25

<210> 4674
<211> 25
<212> DNA
<213> Homo sapiens

<400> 4674
taatttttat tccagtattc ttaac                                              25

<210> 4675
<211> 25
<212> DNA
<213> Homo sapiens

<400> 4675
aatttttatt ccagtattct taacc                                              25

<210> 4676
<211> 25
<212> DNA
<213> Homo sapiens

<400> 4676
attttattc cagtattctt aacct                                          25

<210> 4677
<211> 25
<212> DNA
<213> Homo sapiens

<400> 4677
tttttattcc agtattctta acctt                                         25

<210> 4678
<211> 25
<212> DNA
<213> Homo sapiens

<400> 4678
ttttattcca gtattcttaa ccttt                                         25

<210> 4679
<211> 25
<212> DNA
<213> Homo sapiens

<400> 4679
tttattccag tattcttaac ctttt                                         25

<210> 4680
<211> 25
<212> DNA
<213> Homo sapiens

<400> 4680
ttattccagt attcttaacc ttttt                                         25

<210> 4681
<211> 25
<212> DNA
<213> Homo sapiens

<400> 4681
tattccagta ttcttaacct ttttt                                         25

<210> 4682
<211> 25
<212> DNA
<213> Homo sapiens

<400> 4682
attccagtat tcttaacctt ttttg                                         25

<210> 4683
<211> 25
<212> DNA
<213> Homo sapiens

<400> 4683
ttccagtatt cttaaccttt tttgg                                         25

<210> 4684

<211> 25
<212> DNA
<213> Homo sapiens

<400> 4684
tccagtattc ttaacctttt ttgga                                          25

<210> 4685
<211> 25
<212> DNA
<213> Homo sapiens

<400> 4685
ccagtattct taaccttttt tggaa                                          25

<210> 4686
<211> 25
<212> DNA
<213> Homo sapiens

<400> 4686
cagtattctt aacctttttt ggaag                                          25

<210> 4687
<211> 25
<212> DNA
<213> Homo sapiens

<400> 4687
agtattctta accttttttg gaagg                                          25

<210> 4688
<211> 25
<212> DNA
<213> Homo sapiens

<400> 4688
gtattcttaa ccttttttgg aaggt                                          25

<210> 4689
<211> 25
<212> DNA
<213> Homo sapiens

<400> 4689
tattcttaac cttttttgga aggtt                                          25

<210> 4690
<211> 25
<212> DNA
<213> Homo sapiens

<400> 4690
attcttaacc ttttttggaa ggttt                                          25

<210> 4691
<211> 25
<212> DNA
<213> Homo sapiens

<400> 4691

ttcttaacct tttttggaag gttta                                          25

<210> 4692
<211> 25
<212> DNA
<213> Homo sapiens

<400> 4692
tcttaacctt ttttggaagg tttat                                          25

<210> 4693
<211> 25
<212> DNA
<213> Homo sapiens

<400> 4693
cttaaccttt tttggaaggt ttatt                                          25

<210> 4694
<211> 25
<212> DNA
<213> Homo sapiens

<400> 4694
ttaacctttt ttggaaggtt tattt                                          25

<210> 4695
<211> 25
<212> DNA
<213> Homo sapiens

<400> 4695
taaccttttt tggaaggttt atttg                                          25

<210> 4696
<211> 25
<212> DNA
<213> Homo sapiens

<400> 4696
aacctttttt ggaaggttta tttga                                          25

<210> 4697
<211> 25
<212> DNA
<213> Homo sapiens

<400> 4697
accttttttg gaaggtttat ttgaa                                          25

<210> 4698
<211> 25
<212> DNA
<213> Homo sapiens

<400> 4698
ccttttttgg aaggtttatt tgaat                                          25

<210> 4699
<211> 25
<212> DNA

<213> Homo sapiens

<400> 4699
   cttttttgga aggtttattt gaata                                          25

<210> 4700
<211> 25
<212> DNA
<213> Homo sapiens

<400> 4700
   ttttttggaa ggtttatttg aatat                                            25

<210> 4701
<211> 25
<212> DNA
<213> Homo sapiens

<400> 4701
tttttggaag gtttatttga atatc                                           25

<210> 4702
<211> 25
<212> DNA
<213> Homo sapiens

<400> 4702
ttttggaagg tttatttgaa tatcc                                           25

<210> 4703
<211> 25
<212> DNA
<213> Homo sapiens

<400> 4703
tttggaaggt ttatttgaat atcca                                           25

<210> 4704
<211> 25
<212> DNA
<213> Homo sapiens

<400> 4704
ttggaaggtt tatttgaata tccac                                           25

<210> 4705
<211> 25
<212> DNA
<213> Homo sapiens

<400> 4705
tggaaggttt atttgaatat ccact                                           25

<210> 4706
<211> 25
<212> DNA
<213> Homo sapiens

<400> 4706
ggaaggttta tttgaatatc cacta                                           25

<210> 4707
<211> 25
<212> DNA
<213> Homo sapiens

<400> 4707
gaaggtttat ttgaatatcc actaa                                          25

<210> 4708
<211> 25
<212> DNA
<213> Homo sapiens

<400> 4708
aaggtttatt tgaatatcca ctaac                                          25

<210> 4709
<211> 25
<212> DNA
<213> Homo sapiens

<400> 4709
aggtttattt gaatatccac taaca                                          25

<210> 4710
<211> 25
<212> DNA
<213> Homo sapiens

<400> 4710
ggtttatttg aatatccact aacaa                                          25

<210> 4711
<211> 25
<212> DNA
<213> Homo sapiens

<400> 4711
gtttatttga atatccacta acaag                                          25

<210> 4712
<211> 25
<212> DNA
<213> Homo sapiens

<400> 4712
tttatttgaa tatccactaa caagt                                          25

<210> 4713
<211> 25
<212> DNA
<213> Homo sapiens

<400> 4713
ttatttgaat atccactaac aagtc                                          25

<210> 4714
<211> 25
<212> DNA
<213> Homo sapiens

<400> 4714
tatttgaata tccactaaca agtca                                          25

<210> 4715
<211> 25
<212> DNA
<213> Homo sapiens

<400> 4715
atttgaatat ccactaacaa gtcaa                                          25

<210> 4716
<211> 25
<212> DNA
<213> Homo sapiens

<400> 4716
tttgaatatc cactaacaag tcaaa                                          25

<210> 4717
<211> 25
<212> DNA
<213> Homo sapiens

<400> 4717
ttgaatatcc actaacaagt caaag                                          25

<210> 4718
<211> 25
<212> DNA
<213> Homo sapiens

<400> 4718
tgaatatcca ctaacaagtc aaaga                                          25

<210> 4719
<211> 25
<212> DNA
<213> Homo sapiens

<400> 4719
gaatatccac taacaagtca aagac                                          25

<210> 4720
<211> 25
<212> DNA
<213> Homo sapiens

<400> 4720
aatatccact aacaagtcaa agacc                                          25

<210> 4721
<211> 25
<212> DNA
<213> Homo sapiens

<400> 4721
atatccacta acaagtcaaa gacca                                          25

<210> 4722
<211> 25

<212> DNA
<213> Homo sapiens

<400> 4722
tatccactaa caagtcaaag accaa                                          25

<210> 4723
<211> 25
<212> DNA
<213> Homo sapiens

<400> 4723
atccactaac aagtcaaaga ccaat                                          25

<210> 4724
<211> 25
<212> DNA
<213> Homo sapiens

<400> 4724
tccactaaca agtcaaagac caatt                                          25

<210> 4725
<211> 25
<212> DNA
<213> Homo sapiens

<400> 4725
ccactaacaa gtcaaagacc aattc                                          25

<210> 4726
<211> 25
<212> DNA
<213> Homo sapiens

<400> 4726
cactaacaag tcaaagacca attct                                          25

<210> 4727
<211> 25
<212> DNA
<213> Homo sapiens

<400> 4727
actaacaagt caaagaccaa ttcta                                          25

<210> 4728
<211> 25
<212> DNA
<213> Homo sapiens

<400> 4728
ctaacaagtc aaagaccaat tctag                                          25

<210> 4729
<211> 25
<212> DNA
<213> Homo sapiens

<400> 4729
taacaagtca aagaccaatt ctaga                                          25

<210> 4730
<211> 25
<212> DNA
<213> Homo sapiens

<400> 4730
aacaagtcaa agaccaattc tagaa                                          25

<210> 4731
<211> 25
<212> DNA
<213> Homo sapiens

<400> 4731
acaagtcaaa gaccaattct agaat                                          25

<210> 4732
<211> 25
<212> DNA
<213> Homo sapiens

<400> 4732
caagtcaaag accaattcta gaatt                                          25

<210> 4733
<211> 25
<212> DNA
<213> Homo sapiens

<400> 4733
aagtcaaaga ccaattctag aattc                                          25

<210> 4734
<211> 25
<212> DNA
<213> Homo sapiens

<400> 4734
agtcaaagac caattctaga attcc                                          25

<210> 4735
<211> 25
<212> DNA
<213> Homo sapiens

<400> 4735
gtcaaagacc aattctagaa ttcct                                          25

<210> 4736
<211> 25
<212> DNA
<213> Homo sapiens

<400> 4736
tcaaagacca attctagaat tcctg                                          25

<210> 4737
<211> 25
<212> DNA
<213> Homo sapiens

<400> 4737
caaagaccaa ttctagaatt cctga                                          25

<210> 4738
<211> 25
<212> DNA
<213> Homo sapiens

<400> 4738
aaagaccaat tctagaattc ctgat                                          25

<210> 4739
<211> 25
<212> DNA
<213> Homo sapiens

<400> 4739
aagaccaatt ctagaattcc tgatt                                          25

<210> 4740
<211> 25
<212> DNA
<213> Homo sapiens

<400> 4740
agaccaattc tagaattcct gattg                                          25

<210> 4741
<211> 25
<212> DNA
<213> Homo sapiens

<400> 4741
gaccaattct agaattcctg attgc                                          25

<210> 4742
<211> 25
<212> DNA
<213> Homo sapiens

<400> 4742
accaattcta gaattcctga ttgcc                                          25

<210> 4743
<211> 25
<212> DNA
<213> Homo sapiens

<400> 4743
ccaattctag aattcctgat tgccc                                          25

<210> 4744
<211> 25
<212> DNA
<213> Homo sapiens

<400> 4744
caattctaga attcctgatt gccct                                          25

<210> 4745

<211> 25
<212> DNA
<213> Homo sapiens

<400> 4745
aattctagaa ttcctgattg cccta                                          25

<210> 4746
<211> 25
<212> DNA
<213> Homo sapiens

<400> 4746
attctagaat tcctgattgc cctat                                          25

<210> 4747
<211> 25
<212> DNA
<213> Homo sapiens

<400> 4747
ttctagaatt cctgattgcc ctatt                                          25

<210> 4748
<211> 25
<212> DNA
<213> Homo sapiens

<400> 4748
tctagaattc ctgattgccc tattc                                          25

<210> 4749
<211> 25
<212> DNA
<213> Homo sapiens

<400> 4749
ctagaattcc tgattgccct attcc                                          25

<210> 4750
<211> 25
<212> DNA
<213> Homo sapiens

<400> 4750
tagaattcct gattgcccta ttcca                                          25

<210> 4751
<211> 25
<212> DNA
<213> Homo sapiens

<400> 4751
agaattcctg attgccctat tccaa                                          25

<210> 4752
<211> 25
<212> DNA
<213> Homo sapiens

<400> 4752

gaattcctga ttgccctatt ccaat                                          25

<210> 4753
<211> 25
<212> DNA
<213> Homo sapiens

<400> 4753
aattcctgat tgccctattc caatc                                          25

<210> 4754
<211> 25
<212> DNA
<213> Homo sapiens

<400> 4754
attcctgatt gccctattcc aatct                                          25

<210> 4755
<211> 25
<212> DNA
<213> Homo sapiens

<400> 4755
ttcctgattg ccctattcca atctg                                          25

<210> 4756
<211> 25
<212> DNA
<213> Homo sapiens

<400> 4756
tcctgattgc cctattccaa tctga                                          25

<210> 4757
<211> 25
<212> DNA
<213> Homo sapiens

<400> 4757
cctgattgcc ctattccaat ctgat                                          25

<210> 4758
<211> 25
<212> DNA
<213> Homo sapiens

<400> 4758
ctgattgccc tattccaatc tgatt                                          25

<210> 4759
<211> 25
<212> DNA
<213> Homo sapiens

<400> 4759
tgattgccct attccaatct gattt                                          25

<210> 4760
<211> 25
<212> DNA

<213> Homo sapiens

<400> 4760
gattgcccta ttccaatctg atttt                                          25

<210> 4761
<211> 25
<212> DNA
<213> Homo sapiens

<400> 4761
attgccctat tccaatctga ttttt                                          25

<210> 4762
<211> 25
<212> DNA
<213> Homo sapiens

<400> 4762
ttgccctatt ccaatctgat ttttt                                          25

<210> 4763
<211> 25
<212> DNA
<213> Homo sapiens

<400> 4763
tgccctattc caatctgatt ttttt                                          25

<210> 4764
<211> 25
<212> DNA
<213> Homo sapiens

<400> 4764
gccctattcc aatctgattt tttta                                          25

<210> 4765
<211> 25
<212> DNA
<213> Homo sapiens

<400> 4765
ccctattcca atctgatttt tttaa                                          25

<210> 4766
<211> 25
<212> DNA
<213> Homo sapiens

<400> 4766
cctattccaa tctgattttt ttaaa                                          25

<210> 4767
<211> 25
<212> DNA
<213> Homo sapiens

<400> 4767
ctattccaat ctgatttttt taaat                                          25

<210> 4768
<211> 25
<212> DNA
<213> Homo sapiens

<400> 4768
tattccaatc tgattttttt aaatg                                              25

<210> 4769
<211> 25
<212> DNA
<213> Homo sapiens

<400> 4769
attccaatct gattttttta aatgc                                              25

<210> 4770
<211> 25
<212> DNA
<213> Homo sapiens

<400> 4770
ttccaatctg atttttttaa atgca                                              25

<210> 4771
<211> 25
<212> DNA
<213> Homo sapiens

<400> 4771
tccaatctga tttttttaaa tgcac                                              25

<210> 4772
<211> 25
<212> DNA
<213> Homo sapiens

<400> 4772
ccaatctgat ttttttaaat gcact                                              25

<210> 4773
<211> 25
<212> DNA
<213> Homo sapiens

<400> 4773
caatctgatt tttttaaatg cacta                                              25

<210> 4774
<211> 25
<212> DNA
<213> Homo sapiens

<400> 4774
aatctgattt ttttaaatgc actat                                              25

<210> 4775
<211> 25
<212> DNA
<213> Homo sapiens

<400> 4775
atctgatttt tttaaatgca ctatt                                          25

<210> 4776
<211> 25
<212> DNA
<213> Homo sapiens

<400> 4776
tctgattttt ttaaatgcac tatta                                          25

<210> 4777
<211> 25
<212> DNA
<213> Homo sapiens

<400> 4777
ctgatttttt taaatgcact attaa                                          25

<210> 4778
<211> 25
<212> DNA
<213> Homo sapiens

<400> 4778
tgattttttt aaatgcacta ttaag                                          25

<210> 4779
<211> 25
<212> DNA
<213> Homo sapiens

<400> 4779
gattttttta aatgcactat taaga                                          25

<210> 4780
<211> 25
<212> DNA
<213> Homo sapiens

<400> 4780
atttttttaa atgcactatt aagaa                                          25

<210> 4781
<211> 25
<212> DNA
<213> Homo sapiens

<400> 4781
tttttttaaa tgcactatta agaat                                          25

<210> 4782
<211> 25
<212> DNA
<213> Homo sapiens

<400> 4782
ttttttaaat gcactattaa gaata                                          25

<210> 4783
<211> 25

<212> DNA
<213> Homo sapiens

<400> 4783
tttttaaatg cactattaag aatag                                          25

<210> 4784
<211> 25
<212> DNA
<213> Homo sapiens

<400> 4784
ttttaaatgc actattaaga atagt                                          25

<210> 4785
<211> 25
<212> DNA
<213> Homo sapiens

<400> 4785
tttaaatgca ctattaagaa tagtc                                          25

<210> 4786
<211> 25
<212> DNA
<213> Homo sapiens

<400> 4786
ttaaatgcac tattaagaat agtca                                          25

<210> 4787
<211> 25
<212> DNA
<213> Homo sapiens

<400> 4787
taaatgcact attaagaata gtcaa                                          25

<210> 4788
<211> 25
<212> DNA
<213> Homo sapiens

<400> 4788
aaatgcacta ttaagaatag tcaac                                          25

<210> 4789
<211> 25
<212> DNA
<213> Homo sapiens

<400> 4789
aatgcactat taagaatagt caaca                                          25

<210> 4790
<211> 25
<212> DNA
<213> Homo sapiens

<400> 4790
atgcactatt aagaatagtc aacaa                                          25

<210> 4791
<211> 25
<212> DNA
<213> Homo sapiens

<400> 4791
tgcactatta agaatagtca acaaa                                          25

<210> 4792
<211> 25
<212> DNA
<213> Homo sapiens

<400> 4792
gcactattaa gaatagtcaa caaac                                          25

<210> 4793
<211> 25
<212> DNA
<213> Homo sapiens

<400> 4793
cactattaag aatagtcaac aaact                                          25

<210> 4794
<211> 25
<212> DNA
<213> Homo sapiens

<400> 4794
actattaaga atagtcaaca aacta                                          25

<210> 4795
<211> 25
<212> DNA
<213> Homo sapiens

<400> 4795
ctattaagaa tagtcaacaa actac                                          25

<210> 4796
<211> 2021
<212> DNA
<213> Homo sapiens

<400> 4796
caggaaaccg tctggtggga tctccgcagc tgcttttcac ctgctgttcc tcctgcgctt 60
cctaagagga agaatcaatg ccgtgggtgg agcccaagcc caggccgggg ccggagcaga 120
agcccaagct caccaaaccg gactctgcca ccgggccgca gtggtaccag gaatctcagg 180
aatcggagtc ggaaggcaag cagccacccc cgggacccct ggcacccccg aaatcccccg 240
aaccctcagg acccctggcg tcggagcagg atgcacccct gccagagggg gacgatgcac 300
ccccccggcc gtcgatgctg gacgatgcac cccgcctgcc gctggagctg gacgatgcac 360
ccctgccgga ggaggaaacc cccgaaccca cggccatctg caggcaccgg caccgctgtc 420
acaccgactg cctagagggg ctgctgtccc gcaccttcca gtggctgggg tggcaggtgg 480
gcgcgcaccc ctggatcttc ctgctggcgc ccttgatgct gacagccgcg ctgggcaccg 540
gcttcctgta cctacccaag gacgaagagg aagacctaga ggagcattac acccctgtgg 600
ggagcccggc caaggcggag cggcgcttcg tgcagggcca tttcaccacc aacgactcct 660
accgcttctc cgcctccagg aggagcaccg aagccaattt cgtctcgctt ctggtggtct 720
cctacagcga ctcactgctg gacccagcta cctttgcaga agtcagcaaa ctggacggcg 780
cggtgcagga tctgcgcgtg gcgcgggaaa agggaagcca gatccagtac cagcaggtgt 840

gcgcgaggta cagggcgctc tgcgtgcccc ccaacccgat cctgtacgcc tggcaggtga 900
acaaaacgct caacctgagc agcatctcct tccccgccta caaccacggc aggcatcccc 960
tctacctgac cggcttcttc ggaggataca tcttgggggg cagcctagga atgggccagt 1020
tactcctgcg ggccaaagcc atgcggctgc tgtactacct gaagaccgag gaccctgagt 1080
acgacgtgca gagcaagcag tggctcaccc atttgctcga tcaatttacc aacattaaga 1140
acatcttggc cttgaaaaaa attgaggtag tccactttac atcgctttcc agacaactgg 1200
aatttgaggc aacttctgtg actgtgatcc ctgtgtttca cctggcatac attctcatca 1260
ttctgtttgc agtcacatca tgctttaggt ttgactgcat acgaaacaaa atgtgtgttg 1320
cggcctttgg agtgatttct gctttcttgg cagtggtgag cggctttggc ctgctgttgc 1380
acattggggt gccatttgtc atcatagttg ccaattcacc atttcttatt ctaggtgttg 1440
gggtcgatga catgtttatc atgatttctg cctggcataa gaccaacctt gcagatgaca 1500
tacgagagcg gatgtccaat gtctattcaa aagcggcagt gtctattaca atcaccacca 1560
tcactaacat cctggcctta tatacaggga ttatgagctc ttttaggtct gtacaatgtt 1620
tttgcatcta tacaggaatg accctgttat tttgctattt ttataacatc acgtgttttg 1680
gagcatttat ggccttggat ggtaaaagag aagtagtctg cctatgctgg ttgaaaaagg 1740
ctgacccaaa atggccctca tttaaaaagt tctgctgttt cccatttggt tctgtcccag 1800
atgaacatgg aactgatatc catccaataa gtttgttttt tagagactat tttggcccct 1860
ttctcacaag gagtgagtcc aagtattttg tagtctttat atatgttttg tacatcataa 1920
gcagtatata tgggtgtttc catgtgcagg aaggtttaga ccttcgaaat ctggcaagtg 1980
acgattccta catcacacca tattttaacg tagaggagaa t 2021

<210> 4797
<211> 77
<212> DNA
<213> Homo sapiens

<400> 4797
caggaaaccg tctggtggga tctccgcagc tgcttttcac ctgctgttcc tcctgcgctt 60
cctaagagga agaatca 77

<210> 4798
<211> 1944
<212> DNA
<213> Homo sapiens

<400> 4798
atgccgtggg tggagcccaa gcccaggccg gggccggagc agaagcccaa gctcaccaaa 60
ccggactctg ccaccgggcc gcagtggtac caggaatctc aggaatcgga gtcggaaggc 120
aagcagccac ccccgggacc cctggcaccc ccgaaatccc ccgaaccctc aggacccctg 180
gcgtcggagc aggatgcacc cctgccagag ggggacgatg cacccccccg gccgtcgatg 240
ctggacgatg caccccgcct gccgctggag ctggacgatg cacccctgcc ggaggaggaa 300
acccccgaac ccacggccat ctgcaggcac cggcaccgct gtcacaccga ctgcctagag 360
gggctgctgt cccgcacctt ccagtggctg gggtggcagg tgggcgcgca cccctggatc 420
ttcctgctgg cgcccttgat gctgacagcc gcgctgggca ccggcttcct gtacctaccc 480
aaggacgaag aggaagacct agaggagcat tacacccctg tggggagccc ggccaaggcg 540
gagcggcgct tcgtgcaggg ccatttcacc accaacgact cctaccgctt ctccgcctcc 600
aggaggagca ccgaagccaa tttcgtctcg cttctggtgg tctcctacag cgactcactg 660
ctggacccag ctacctttgc agaagtcagc aaactggacg gcgcggtgca ggatctgcgc 720
gtggcgcggg aaaagggaag ccagatccag taccagcagg tgtgcgcgag gtacagggcg 780
ctctgcgtgc cccccaaccc gatcctgtac gcctggcagg tgaacaaaac gctcaacctg 840
agcagcatct ccttccccgc ctacaaccac ggcaggcatc ccctctacct gaccggcttc 900
ttcggaggat acatcttggg gggcagccta ggaatgggcc agttactcct gcgggccaaa 960
gccatgcggc tgctgtacta cctgaagacc gaggaccctg agtacgacgt gcagagcaag 1020
cagtggctca cccatttgct cgatcaattt accaacatta agaacatctt ggccttgaaa 1080
aaaattgagg tagtccactt tacatcgctt tccagacaac tggaatttga ggcaacttct 1140
gtgactgtga tccctgtgtt tcacctggca tacattctca tcattctgtt tgcagtcaca 1200
tcatgcttta ggtttgactg catacgaaac aaaatgtgtg ttgcggcctt tggagtgatt 1260
tctgctttct tggcagtggt gagcggcttt ggcctgctgt tgcacattgg ggtgccattt 1320
gtcatcatag ttgccaattc accatttctt attctaggtg ttggggtcga tgacatgttt 1380
atcatgattt ctgcctggca taagaccaac cttgcagatg acatacgaga gcggatgtcc 1440
aatgtctatt caaaagcggc agtgtctatt acaatcacca ccatcactaa catcctggcc 1500

ttatatacag ggattatgag ctcttttagg tctgtacaat gtttttgcat ctatacagga 1560
atgaccctgt tattttgcta tttttataac atcacgtgtt ttggagcatt tatggccttg 1620
gatggtaaaa gagaagtagt ctgcctatgc tggttgaaaa aggctgaccc aaaatggccc 1680
tcatttaaaa agttctgctg tttcccattt ggttctgtcc cagatgaaca tggaactgat 1740
atccatccaa taagtttgtt ttttagagac tattttggcc cctttctcac aaggagtgag 1800
tccaagtatt ttgtagtctt tatatatgtt ttgtacatca taagcagtat atatgggtgt 1860
ttccatgtgc aggaaggttt agaccttcga aatctggcaa gtgacgattc ctacatcaca 1920
ccatatttta acgtagagga gaat 1944

<210> 4799
<211> 648
<212> DNA
<213> Homo sapiens

<220>
<221> unsure
<222> 192

<220>
<221> unsure
<222> 207

<220>
<221> unsure
<222> 266

<220>
<221> unsure
<222> 275

<220>
<221> unsure
<222> 279

<220>
<221> unsure
<222> 289

<220>
<221> unsure
<222> 352

<220>
<221> unsure
<222> 355

<220>
<221> unsure
<222> 410

<220>
<221> unsure
<222> 446

<220>
<221> unsure
<222> 470

<220>
<221> unsure
<222> 481

<220>
<221> unsure
<222> 497

<220>
<221> unsure
<222> 530

<220>
<221> unsure
<222> 631

<220>
<221> unsure
<222> 644

<220>
<221> unsure
<222> 648

<400> 4799
mpwvepkprp gpeqkpkltk pdsatgpqwy qesqeseseg kqpppgplap pkspepsgpl 60
aseqdaplpe gddapprpsm lddaprlple lddaplpeee tpeptaicrh rhrchtdcle 120
gllsrtfqwl gwqvgahpwi fllaplmlta algtgflylp kdeeedleeh ytpvgspaka 180
errfvqghft tndsyrfsas rrsteanfvs llvvsysdsl ldpatfaevs kldgavqdlr 240
varekgsqiq yqqvcaryra lcvppnpily awqvnktlnl ssisfpaynh grhplyltgf 300
fggyilggsl gmgqlllrak amrllyylkt edpeydvqsk qwlthlldqf tniknilalk 360
kievvhftsl srqlefeats vtvipvfhla yiliilfavt scfrfdcirn kmcvaafgvi 420
saflavvsgf glllhigvpf viivanspfl ilgvgvddmf imisawhktn laddirerms 480
nvyskaavsi tittitnila lytgimssfr svqcfciytg mtllfcyfyn itcfgafmal 540
dgkrevvclc wlkkadpkwp sfkkfccfpf gsvpdehgtd ihpislffrd yfgpfltrse 600
skyfvvfiyv lyiissiygc fhvqegldlr nlasddsyit pyfnveen 648

<210> 4800
<211> 405
<212> DNA
<213> Homo sapiens

<400> 4800
tccatgatta atcttgtcat ttgtataggg ttttctttcg atttttctgt acacatttcc 60
tatgcatttg tttctagttc tcaaccctca gtaaaccaaa aatcagttga ggcattgtat 120
cttctaggct acccagtgtt acaaagtgca atttcaacaa taataggggt gtgtgtttta 180
gctgcagcga aagcatacat cttcagaaca ttttttaaga ttatgtttct tgttatgata 240
tttggggcag ctcatggcct aatttttatt ccagtattct taaccttttt tggaaggttt 300
atttgaatat ccactaacaa gtcaaagacc aattctagaa ttcctgattg ccctattcca 360
atctgatttt tttaaatgca ctattaagaa tagtcaacaa actac 405

<210> 4801
<211> 24
<212> DNA
<213> Homo sapiens

<400> 4801
caggaaaccg tctggtggga tctc 24

<210> 4802
<211> 27
<212> DNA
<213> Homo sapiens

<400> 4802
ctgagacgga gtctcattct tgtcacc                                          27

<210> 4803
<211> 22
<212> DNA
<213> Homo sapiens

<400> 4803
acatacgaga gcggatgtcc aa                                               22

<210> 4804
<211> 22
<212> DNA
<213> Homo sapiens

<400> 4804
gggacagaac caaatgggaa ac                                               22

Page 647 of 647

PB0177 - sequence listing

## Claims

1. An isolated nucleic acid that encodes a Patched like transmembrane protein, functioning in male germ cell development, and as a potential tumor suppressor, comprising:
(a) a nucleotide sequence selected from the group consisting of:
(i) SEQ ID NO: 1, SEQ ID NO: 4;
(ii) the complement of the sequences set forth in (i);
(iii) the nucleotide sequence of SEQ ID NO: 2, SEQ ID NO: 5;
(iv) a degenerate variant of the sequences set forth in (iii); and
(v) the complement of the sequences set forth in (iii) and (iv); or
(b) a nucleotide sequence selected from the group consisting of:
(i) a nucleotide sequence that encodes a polypeptide having the sequence of SEQ_ID_NO: 3, SEQ ID NO: 6;
(ii) a nucleotide sequence that encodes a polypeptide having the sequence of SEQ_ID_NO: 3, SEQ ID NO: 6, with conservative amino acid substitutions; and
(iii) the complement of the sequences set forth in (i) and (ii),
wherein said isolated nucleic acid comprising a nucleotide sequence selected from group (b) is no more than about 100 kb in length.

2. The isolated nucleic acid of claim 1 wherein said nucleic acid, or the complement of said nucleic acid, encodes a polypeptide having a role in male germ cell development, or is a potential tumor suppressor.

3. The isolated nucleic acid of either of claims 1 or 2, wherein said nucleic acid, or the complement of said nucleic acid, is expressed in testis, as well as in adrenal, adult and fetal liver, bone marrow, brain, kidney, lung, placenta, prostate, skeletal muscle and/or colon.

4. A nucleic acid probe, comprising:
(a) the nucleic acid of claim 1; or
(b) at least 17 contiguous nucleotides of SEQ ID NO: 7, SEQ ID NO: 11, SEQ ID NO: 4796, SEQ ID NO: 4800,
wherein said probe according to (b) is no longer than about 100 kb in length.

5. The probe of claim 4, wherein said probe is detectably labeled.

6. The probe of either of claims 4 or 5, attached to a substrate.

7. A microarray, wherein at least one probe of said array is a probe according to claim 4.

8. The isolated nucleic acid molecule of any of claims 1 - 3, wherein said nucleic acid molecule is operably linked to one or more expression control elements.

9. A replicable vector comprising a nucleic acid molecule of any of claims 1 - 3 or 8.

10. A host cell transformed to contain the nucleic acid molecule of any one of claims 1 - 3 or 8 or 9, or the progeny thereof.

11. A method for producing a polypeptide, the method comprising: culturing the host cell of claim 10 under conditions in which the protein encoded by said nucleic acid molecule is expressed.

12. An isolated polypeptide produced by the method of claim 11.

13. An isolated polypeptide, comprising:
(a) an amino acid sequence from SEQ ID NO: 3, SEQ ID NO: 6;
(b) an amino acid sequence having at least 65% amino acid sequence identity to that of (a);
(c) an amino acid sequence according to (a) in which at least 95% of deviations from the sequence of (a) are conservative substitutions; or
(d) a fragment of at least 8 contiguous amino acids of any of (a) - (c).

14. A fusion protein, said fusion protein comprising a polypeptide of claim 13 fused to a heterologous amino acid sequence.

15. An isolated antibody, or antigen-binding fragment or derivative thereof, the binding of which can be competitively inhibited by a polypeptide of claim 13.

16. A transgenic non-human animal modified to contain the nucleic acid molecule of any one of claims 1 - 3 or 8 or 9.

17. A transgenic non-human animal unable to express the endogenous orthologue of the nucleic acid molecule of any one of claims 1 - 3 or 8 or 9.

18. A method of identifying agents that modulate the expression of HTPL, the method comprising:
contacting a cell or tissue sample believed to express HTPL with a chemical or biological agent, and then
comparing the amount of HTPL expression in said cell or tissue sample with that of a control,
changes in the amount relative to control identifying an agent that modulates expression of HTPL.

19. A method of identifying agonists and antagonists of HTPL, the method comprising:
contacting a cell or tissue sample believed to express HTPL with a chemical or biological agent, and then
comparing the activity of HTPL with that of a control,
increased activity relative to a control identifying an agonist, decreased activity relative to a control identifying an antagonist.

20. A purified agonist of the polypeptide of claim 13.

21. A purified antagonist of the polypeptide of claim 13.

22. A method of identifying a specific binding partner for a polypeptide according to claim 13, the method comprising:
contacting said polypeptide to a potential binding partner; and
determining if the potential binding partner binds to said polypeptide.

23. The method of claim 22, wherein said contacting is performed *in vivo.*

24. A purified binding partner of the polypeptide of claim 13.

25. A method for detecting a target nucleic acid in a sample, said target being a nucleic acid according to any of claims 1 - 3 or 8 or 9, the method comprising:
a) hybridizing the sample with a probe comprising at least 17 contiguous nucleotides of a sequence complementary to said target nucleic acid in said sample under high stringency hybridization conditions, and
b) detecting the presence or absence, and optionally the amount, of said binding.

26. A method of diagnosing a disease caused by mutation in HTPL, comprising:
detecting said mutation in a sample of nucleic acids that derives from a subject suspected to have said disease.

27. A method of diagnosing or monitoring a disease caused by altered expression of HTPL, comprising:
determining the level of expression of HTPL in a sample of nucleic acids or proteins that derives from a subject suspected to have said disease,
alterations from a normal level of expression providing diagnostic and/or monitoring information.

28. A diagnostic composition comprising the nucleic acid of any of claims 1 - 3, said nucleic acid being detectably labeled.

29. The diagnostic composition of claim 28, wherein said composition is further suitable for *in vivo* administration.

30. A diagnostic composition comprising the polypeptide of claim 13, said polypeptide being detectably labeled.

31. The diagnostic composition of claim 30, wherein said composition is further suitable for *in vivo* administration.

32. A diagnostic composition comprising the antibody, or antigen-binding fragment or derivative thereof, of claim 15.

33. The diagnostic composition of claim 32, wherein said antibody or antigen-binding fragment or derivative thereof is detectably labeled.

34. The diagnostic composition of claim 33, wherein said composition is further suitable for *in vivo* administration.

35. A pharmaceutical composition comprising the nucleic acid of any of claims 1 - 3 or 8 or 9 and a pharmaceutically acceptable excipient.

36. A pharmaceutical composition comprising the polypeptide of claim 13 and a pharmaceutically acceptable excipient.

37. A pharmaceutical composition comprising the antibody or antigen-binding fragment or derivative thereof of claim 15 and a pharmaceutically acceptable excipient.

38. A pharmaceutical composition comprising the agonist of claim 20 and a pharmaceutically acceptable excipient.

39. A pharmaceutical composition comprising the antagonist of claim 21 and a pharmaceutically acceptable excipient.

40. Nucleic acid of any one of claims 1 - 3 or 8 or 9 for use in therapy.

41. Use of the nucleic acid of claims 1 - 3 or 8 or 9 for the manufacture of a medicament for the treatment or prevention of a disorder associated with decreased expression or activity of human HTPL.

42. Polypeptide of claim 13 for use in therapy.

43. Use of the polypeptide of claim 13 for the manufacture of a medicament for the treatment or prevention of a disorder associated with decreased expression or activity of human HTPL.

44. Use of the agonist of claim 20 for the manufacture of a medicament for the treatment or prevention of a disorder associated with decreased expression or activity of human HTPL.

45. Antibody or antigen-binding fragment or derivative thereof of claim 15 for use in therapy.

46. Use of the antibody or antigen-binding fragment or derivative thereof of claim 15 for the manufacture of a medicament for the treatment or prevention of a disorder associated with increased expression or activity of human HTPL.

47. Use of the antagonist of claim 21 for the manufacture of a medicament for the treatment or prevention of a disorder associated with increased expression or activity of human HTPL.

48. A method of modulating the expression of a nucleic acid according to any of claims 1 - 3 or 8 or 9, the method comprising:
administering an effective amount of an agent which modulates the expression of a nucleic acid according to any of claims 1 - 3 or 8 or 9.

49. A method of modulating at least one activity of a polypeptide according to claim 13, the method comprising:
administering an effective amount of an agent which modulates at least one activity of a polypeptide according to claim 13.
